# EUROPEAN PATENT APPLICATION

(11) **EP 4 592 310 A2**
(43) Date of publication of application: **30.07.2025**
(21) Application number: 25170593.5
(22) Date of filing: 16.03.2021
(51) Int. Cl.: C07K 14/725

(54) **NOVEL ANTIGEN BINDING DOMAINS AND SYNTHETIC ANTIGEN RECEPTORS INCORPORATING THE SAME**

(30) Priority: 16.03.2020 US 202062990396 P
(62) Divisional of application: 21771595.2
(71) Applicant: Angeles Therapeutics, Inc., Toluca Lake, CA 91602 (US)
(72) Inventor: CHAUDHARY, Preet M., Toluca Lake, 91602 (US)
(74) Representative: Gill Jennings & Every LLP

(57) **Abstract**

The disclosure relates to compositions and methods of generating synthetic antigen receptors or SAR (*e.g.,* SIR, zSIR, cTCR, ab-TCRs, AABD-TCRs, TFP, TACs *etc*.) and antibodies (*e.g.,* bispecific antibodies, DARTs *etc*.) comprising one or more novel antigen binding domains. SARs as described comprise single chain immune receptors (*e.g.,* 1^{st}, 2^{nd} and 3^{rd} generation chimeric antigen receptors, TFPs, Tri-TAC and the like) and multiple chain immune receptors (*e.g.,* SIR, zSIR, cTCR, ab-TCR, AABD-TCR, αβTFP, γdTFP, recombinant TCRs *etc*.). SARs are able to redirect immune cell specificity and reactivity toward one or more selected targets exploiting the antigen-binding domain properties.

## Description

### CROSS REFERENCE TO RELATED APPLICATIONS

This application claims priority under 35 U.S.C. §119 from U.S. Provisional Application Serial No. 62/990,396, filed March 16, 2020, the disclosures of which are incorporated herein by reference.

### FIELD OF THE INVENTION

The disclosure relates to compositions and methods of generating synthetic antigen receptors or SAR *(e.g.,* SIR, zSIR, cTCR, ab-TCRs, AABD-TCRs, TFP, TACs *etc.)* and antibodies *(e.g.,* bispecific antibodies, DARTs *etc.)* comprising one or more novel antigen binding domains. SARs as described comprise single chain immune receptors (*e.g*., 1^{st}, 2^{nd} and 3^{rd} generation chimeric antigen receptors, TFPs, Tri-TAC and the like) and multiple chain immune receptors (*e.g*., SIR, zSIR, cTCR, ab-TCR, AABD-TCR, αβTFP, γdTFP, recombinant TCRs *etc.).* SARs are able to redirect immune cell specificity and reactivity toward one or more selected targets exploiting the antigen-binding domain properties. The SARs, as described herein, when expressed in immune cells, confer on such the ability to recognize a target antigen in an MHC-dependent or an MHC-independent manner. The disclosure describes a useful configuration of a SAR with two or more antigen binding domains. The disclosure describes useful antigen binding domains, a useful configurations of such antigen binding domains and vector constructs for construction of unispecific, bispecific and/or multispecific SARs. The disclosure also describes novel methods for selection of optimal antigen binding domains for incorporation into SARs. The disclosure further relates to improving the quality of SAR expressing T cells by expanding them in the presence of a SMAC mimetic and/or NIK agonist. The disclosure also described methods of manufacturing SARs using a device or a container containing a gas-permeable membrane under normoxic and hypoxic conditions. An exemplary device with gas permeable memebrane is a GRex flask. The disclosure also describes novel tags that can be added on the SAR construct and used to identify, isolate and eliminate SAR-expressing cells. The disclosure also provides use of thermostable luciferases (*e.g*., Photinus pyralis (lucPpy) and Photuris pennsylvanica (lucPpe) luciferase) for *in vivo* bioluminescence imaging applications. The disclosure also provides novel antigen binding domains and SARs incorporating them.

### INCORPORATION BY REFERENCE OF SEQUENCE LISTING

Accompanying this filing is a Sequence Listing entitled "Sequence-Listing_ST25.txt", created on March 16, 2021 and having 101,792,917 bytes of data, machine formatted on IBM-PC, MS-Windows operating system. The sequence listing is hereby incorporated herein by reference in its entirety for all purposes.

### BACKGROUND

CARs are synthetic immune-receptors, which can redirect T cells to selectively kill tumor cells. Unlike the physiologic T-cell receptor (TCR), which engages HLA-peptide complexes, CARs engage molecules that do not require peptide processing or HLA expression to be recognized. Initial first-generation CARs were constructed through the fusion of a scFv (single chain fragment variable)-based antigen binding domain to an inert CD8 transmembrane domain, linked to a cytoplasmic signaling domain derived from the CD3-ζ or Fc receptor γ chains. To overcome the lack of T-cell co-stimulation, first generation CARs were further modified by incorporating the cytoplasmic signaling domains of T-cell costimulatory receptors.

Despite the success with CAR-T cells, there are several limitations to this approach, including toxicities such as "Cytokine release syndrome' (CRS) and neurotoxicities. The inclusion of costimulatory domain in the CAR construct results in non-physiological tonic signaling through the receptor, which in turn could contribute to their toxicity and lack of persistence. CAR-Ts that have scFv as their antigen binding domain can also form clusters on the membrane due to crosslinking of heavy and light chains of different SAR-Ts resulting in tonic signaling. Furthermore, scFv used in CAR-Ts are also usually derived from mouse mAbs and thus have potential immunogenicity issues. Without wishing to be bound by theory, these problems are likely to be compounded when scFvs are used to design CAR-Ts having bispecific, multispecific, bivalent or biparatopic antigen binding moieties.

To overcome some of the design limitation of conventional 2nd generation CARs, several alternative designs, collectively termed next generation CARs, have been described, including Ab-TCR (WO 2017/070608 A1 incorporated herein by reference), TCR receptor fusion proteins or TFP (WO 2016/187349 A1 incorporated herein by reference), Synthetic Immune Receptors (SIRs) (see, WO 2018/102795 A1, incorporated herein by reference), Trifunctional T cell antigen coupler (Tri-TAC) (see, WO 2015/117229 A1, incorporated herein by reference) and zSIR (see WO2019232503, incorporated herein by reference). AABD-TCR platform is described in this disclosure. These alternative CAR designs, in general, lack a co-stimulatory domain. These alternative CAR designs may comprise of a single chain *(e.g.,* single chain SIR, or εTFP, γTFP and δTFP) or more than one chain (*e.g.*, double chain SIR, cTCR, zSIR, αβTFP and γδTFP). However, the problem of non-specific aggregation of scFv chains and resulting tonic signaling also affects the next generation CAR designs that comprise of scFv chain(s) for antigen binding. This problem is further compounded when scFv fragment are used to design multi-chain synthetic antigen receptors, such as SIR, cTCRs, Ab-TCRs having bispecific, bivalent, biparatopic, multispecific, multivalent or multiparatopic antigen binding moieties.

The present disclosure provides a solution to the above problems through the design of Synthetic Antigen Receptors (SAR). The term Synthetic Antigen Receptor (SAR) as described herein comprise receptors with the backbone of single chain immune receptors *(e.g.,* 1^{st}, 2^{nd} and 3^{rd} generation chimeric antigen receptors, TFPs, Tri-TAC and the like) and multiple chain immune receptors *(e.g.,* SIR, zSIR, cTCR, ab-TCR, AABD-TCR, αβTFP, γdTFP, recombinant TCR, and HLA-independent TCR *etc.).*

### SUMMARY

The disclosure describes compositions and configuration for making a Synthetic Antigen Receptor (SAR). In one embodiment, a SAR comprises a polypeptide comprising two or more antigen binding domains. In another embodiment, a SAR comprises a polypeptide that binds to two or more antigens. In yet another embodiment, a SAR comprises a polypeptide that binds to two or more epitopes of one or more antigens.

In an embodiment, the disclosure provides a SAR polynucleotide that encodes for a polypeptide which comprises an autonomous antigen binding domain (AABD) or a fragment thereof that is joined in frame to a vL, vH, Va, Vb, Vg, Vd, Ig domain, Ig like domain or a combination thereof via one or more optional linkers. In an embodiment, an AABD is any domain that can bind an antigen in an autonomous manner, *i.e.,* in the absence of another domain. In an embodiment, an AABD is a non-scFv domain. In an embodiment, an AABD is does not comprise a vL and vH fragment that form a Fv domain. In an exemplary embodiment, an AABD is a vHH, single variable domain antibody, FHVH (fully human vH domain), SVH (single vH domain), SVL (single vL domain), non immunoglobulin antigen binding scaffold *(e.g.,* centyrin, affibody, DARPIN, D domain *etc.),* ligand binding domain of a receptor, receptor binding domain of a ligand, autoantigen, adaptor binding domain, epitope, mimotope, a single variable domain of a T cell receptor (svd-TCR) or a fragment thereof.

In an embodiment, the disclosure provides a SAR polynucleotide that encodes for a polypeptide which comprises at least one autonomous antigen binding domain or a fragment thereof that is joined in frame to a vL, vH, Va, Vb, Vg, Vd, Ig domain, Ig like domain or a combination thereof via one or more optional linkers. In an embodiment, a SAR polynucleotide encodes for a polypeptide which comprises more than one autonomous antigen binding domains that are linked in frame via optional linkers.

In an embodiment, a SAR polynucleotide encodes for at least one polypeptide which comprises one or more autonomous antigen binding domains or fragments thereof that are joined in frame to a first module comprising vL, vH, Va, Vb, Vg, Vd, Ig domain, or Ig like domain or a combination thereof via one or more optional domains, and where the first module is connected via an optional linker to a second module that comprises a transmembrane domain. In an embodiment, the optional domain is a linker domain. In an embodiment, the second module comprises a connecting peptide, a transmembrane domain and an intracellular domain. In an embodiment, the second module comprises a TCR constant chain or a fragment thereof. In an embodiment, the second module comprises a constant chain of TCRα, TCRβ1, TCRβ2, TCRγ, TCRδ, or pre-TCRα or a fragment thereof.

In an embodiment, a SAR polynucleotide encodes for a polypeptide which comprises an autonomous antigen binding domain that is joined in frame to a polypeptide that comprises a transmembrane domain via an optional domain. In an embodiment, the optional domain is a connecting peptide. In an embodiment, the transmembrane domain belongs to a T cell receptor constant chain *(e.g.*, constant chain of TCRα, TCRβ1, TCRβ2, TCRγ, TCRδ, pr*etc.*R-α *etc.).* In an embodiment, the connectin peptide belongs to a T cell receptor constant chain *(e.g.,* connecting peptide of TCRα, TCRβ1, TCRβ2, TCRγ, TCRδ, pr*etc.*R-α *etc.).*

In an embodiment, a SAR polynucleotide encodes for a polypeptide which comprises an autonomous antigen binding domain that is joined in frame to a polypeptide that comprises a connecting peptide and a transmembrane domain. In an embodiment, the transmembrane domain belongs to a T cell receptor constant chain (*e.g.*, constant chain of TCRα, TCRβ1, TCRβ2, TCRγ, TCRδ, pr*etc.*R-α *etc.).* In an embodiment, the connectin peptide belongs to a T cell receptor constant chain (*e.g*., connecting peptide of TCRα, TCRβ1, TCRβ2, TCRγ, TCRδ, pr*etc.*R-α *etc.).*

In an embodiment, a SAR polynucleotide encodes for a polypeptide which comprises an autonomous antigen binding domain or a fragment thereof that is joined in frame to the N-terminus or near the N-terminus of a vL, vH, Va, Vb, Vg, Vd, Ig domain, Ig like domain or a combination thereof via one or more optional domains. In an embodiment, the optional domains are linker domains.

In an embodiment, a SAR polynucleotide encodes for a polypeptide which comprises one or more autonomous antigen binding domains or fragments thereof that are joined in frame to the N-terminus or near the N-terminus of a module comprising vL, vH, Va, Vb, Vg, Vd, Ig domain, Ig like domain or a combination thereof via one or more optional domains. In an embodiment, the optional domain is a linker domain.

In an embodiment, a SAR polynucleotide encodes for at least one polypeptide which comprises one or more autonomous antigen binding domains or fragments thereof that are joined in frame to the N-terminus or near the N-terminus of a first module comprising vL, vH, Va, Vb, Vg, Vd, Ig domain, or Ig like domain or a combination thereof via one or more optional domains and where the first module is connected via an optional linker to a second module that comprises a transmembrane domain. In an embodiment, the optional domain is a linker domain. In an embodiment, the second module comprises a connecting peptide, a transmembrane domain and an intracellular domain. In an embodiment, the second module comprises a TCR constant chain or a fragment thereof. In an embodiment, the second module comprises a constant chain of TCRα, TCRβ1, TCRβ2, TCRγ, TCRδ, or pre-TCRα or a fragment thereof.

In an embodiment, a SAR polynucleotide encodes for at two polypeptide chains with each chain comprising 0, 1, 2 or more autonomous antigen binding domains or fragments thereof that are joined in frame to the N-terminus or near the N-terminus of a first module comprising vL, vH, Va, Vb, Vg, Vd, Ig domain, or Ig like domain or a combination thereof via one or more optional domains and where the first module is connected via an optional linker to a second module that comprises a connecting peptide, a transmembrane domain and an optional intracellular domain and where the first polypeptide chain and the second polypeptide chain form a T cell receptor module (TCRM) that is capable of recruiting at least one TCR-associated signaling module when expressed in a T cell.

In an embodiment, a SAR polynucleotide encodes for a polypeptide which comprises an autonomous antigen binding domain that is joined in frame to a polypeptide that comprises a hinge domain and a transmembrane domain.

In an embodiment, a SAR polynucleotide encodes for a polypeptide which comprises an autonomous antigen binding domain or a fragment thereof that is joined in frame to a polypeptide that comprises a hinge domain, a transmembrane domain and one or more intracellular signaling domains. In an embodiment, the intracellular signaling domain comprises a primary activation domain. In an embodiment, the intracellular signaling domain comprises a primary activation domain and one or more co-stimulatory domains. In an embodiment, the intracellular signaling domain comprises one or more co-stimulatory domains. In an embodiment, the intracellular signaling domain lacks an activation domain. In an embodiment, the intracellular signaling domain comprises an activation domain containing one or more ITAM motifs.

The disclosure also provides polynucleotides encoding any of the SARs of the disclosure along with any accessory module. The nucleic acid sequences of the exemplary polynucleotides encoding SARs are provided in **(Tables 25-36 and 41-50).** In some embodiments, the SAR components and any accessory modules (*e.g*., therapeutic controls) are encoded by a single polynucleotide molecule. In some embodiments, the two or more SAR components (*e.g*., two chains of a double chain SAR) and the accessory modules are encoded by separate polynucleotides.

In various embodiments, the polynucleotide molecule(s) encoding a SAR described herein comprises one or more antigen specific coding domains that encode one or more antigen specific domains. In some embodiments, the antigen specific coding domain(s) of a SAR comprises one or more V_{L} (or vL) fragments. In some embodiments, the antigen specific coding domain(s) of a SAR comprises one or more V_{H} (or vH) fragments. In some embodiments, the antigen specific coding domain(s) encodes one or more scFVs (or scFvs) specific to antigens on target cells such as, for example, cancer cells. In some embodiments, the antigen specific coding domain(s) encodes one or more Fv fragments. In some embodiments, the antigen specific coding domain encodes one or more Fab fragments. In some embodiments, the antigen specific coding domain(s) of a SAR comprises one or more (Fab')2 fragments. In some embodiments, the antigen specific coding domain of a SAR polynucleotide encodes an autonomous antigen binding domain (AABD). In some embodiment, the antigen specific coding domain(s) of a SAR polynucleotide encodes one or more single domain antibodies (SDAB) or antibody fragments such as, for example, single vH domain (SVH) or single vL domain (SVL). In some embodiment, the antigen specific coding domain(s) of a SAR polynucleotide encodes single vH domains that are fully human in origin *(i.e.,* FHVH). In some embodiments, the antigen specific coding domain of a SAR polynucleotide encodes one or more camelid V_{HH} (vHH) domains. In some embodiments, the antigen specific coding domain(s) of a SAR polynucleotide encodes vHH domains that are humanized. In some embodiment, the antigen specific coding domain(s) of a SAR polynucleotide encodes variable domains derived from T cell receptors (e.g., Va/Vα, Vb/Vβ, Vg/Vγ and Vd/Vδ). In some embodiment, the antigen specific coding domain(s) of a SAR polynucleotide encodes a single chain TCR (scTCR). In some embodiment, the antigen specific coding domain(s) of a SAR polynucleotide encodes a single varianble domain TCR (svd-TCR). In some embodiments, the antigen specific coding domain(s) of a SAR polynucleotide encodes one or more ligand binding domains of receptors. In some embodiments, the antigen specific coding domain encodes one or more non-immunoglobulin scaffold such as, for example, a DARPIN, an affibody, an affilin, an adnectin, an affitin, an obodies, a repebody, a fynomer, an alphabody, an avimer, an atrimer, a centyrin, a pronectin, an anticalin, a kunitz domain, an Armadillo repeat protein, or a D domain (α3D domain) *etc.* In some embodiments, the antigen specific coding domain(s) of a SAR polynucleotide encodes one or more adaptor binding domains (*e.g.*, RZIP, EZIP, E4, K4, NKG2D-AF, NKG2D-YA *etc.).* In some embodiments, the antigen specific coding domain(s) of a SAR polynucleotide encodes one or more Fc binding domains *(e.g*., Fc binding region of CD16, CD32, or CD64 *etc.).* In some embodiments, the antigen specific coding domain(s) of a SAR polynucleotide encodes one or more autoantigens. In some embodiments, the antigen specific coding domain(s) of a SAR polynucleotide encodes one or more epitope tags or mimotopes. In some embodiments, the antigen specific coding domain(s) of a SAR polynucleotide encodes one or more ligand-binding domains of receptors. In some embodiments, the antigen specific coding domain(s) of a SAR polynucleotide encodes one or more receptor-binding domains of ligands. In some embodiments, the antigen specific coding domains of a SAR comprises at least one Fv *(e.g.,* vL and vH fragments) and one or more AABD *(e.g.,* SVH, SVL, vHH, svd-TCR, DARPIN, affibody, Centyrin, D domain, epitope, mimotope, EZIP, RZIP, E4, K4 *etc.).* In some embodiments, the antigen specific coding domains of a SAR comprises at least one TCR variable domain (*e.g*., Va/Vb or Vg/Vd fragments of a TCR) and one or more AABD (*e.g*., SVH, SVL, FHVH, vHH, svd-TCR, DARPIN, affibody, Centyrin, D domain, epitope, mimotope, EZIP, RZIP, E4, K4 *etc.).* In an embodiment, the antigen specific coding domains of a SAR comprises one or more than one *(e.g.,* 2, 3, 4, 5, 6 or more) AABD (*e.g*., SVH, SVL, vHH, FHVH, svd-TCR, DARPIN, affibody, Centyrin, D domain, epitope, mimotope, EZIP, RZIP, E4, K4 *etc.).* In an embodiment, a SAR comprises AABDs that are of the same type *(e.g.,* two vHH, three DARPINs, two Centyrins *etc.).* In an embodiment, a SAR polynucleotide(s) encodes for AABDs that are of different types *(e.g.,* one vHH and one DARPIN; one DARPIN, one vHH domain and one Centyrin *etc.).* In an embodiment, a SAR polynucleotide(s) encode AABDs that are fully human, humanized, chimeric or non-human in origin. In an embodiment, a SAR polynucleotide(s) encodes one or more peptide linkers. In an embodiment, a SAR polynucleotide(s) encodes for one for more flexible linkers (*e.g*., Gly-Ser linker). In an embodiment, a SAR polynucleotide(s) encodes for one for more protease cleavable linkers (*e.g.*, linkers that are cleaved by cellular proteases, *e.g.,* MMP14, *e.g.,* linker encoded by SEQ ID NO:1218). In an embodiment, a SAR polynucleotide(s) encodes for one or more Ig linkers or Ig like linkers. In an embodiment, a SAR polynucleotide encodes for one or more than one SAR chain. In an embodiment, the two or more SAR chains are encoded by one or more polynucleotides. In an embodiment, the two or more SAR chains are encoded by polynucleotides that are separated by cleavable linkers *(e.g.,* P2A, T2A, F2A *etc.)* that are optionally preceded by nucleic acid sequences encoding for a Furine cleavage site. In an embodiment, a SAR polynucleotide(s) are partially or fully codon-optimized.

In an embodiment, the disclosure provides SAR polypeptide encoded by any of the SAR polynucleotides described in the preceding sections.

In some embodiments, provided herein are polypeptides comprising any of the SARs of the disclosure along with any accessory module. The amino acid sequences of the exemplary SAR polypeptides are provided in **Tables 25-36 and 41-50.** In some embodiments, the two or more SAR components and any accessory modules (*e.g*., therapeutic controls) comprise a single polypeptide molecule. In some embodiments, the two or more SAR components *(e.g*., two chains of a double chain SAR) and the accessory modules comprises separate polypeptides. In various embodiments, a SAR polypeptide comprises one or more antigen specific domains. In some embodiments, the antigen specific domain(s) of a SAR comprise one or more V_{L} (or vL) fragments. In some embodiments, the antigen specific domain(s) of a SAR comprise one or more V_{H} (or vH) fragments. In some embodiments, the antigen specific domain(s) comprise one or more scFVs (or scFvs) specific to the antigens on target cells such as, for example, cancer cells. In some embodiments, the antigen specific domain(s) comprise one or more Fv fragments. In some embodiments, the antigen specific domain comprises one or more Fab fragments. In some embodiments, the antigen specific domain(s) of a SAR comprise one or more (Fab')2 fragments. In some embodiments, the antigen specific domain of a SAR polypeptide comprises an autonomous antigen binding domain (AABD). In some embodiment, the antigen specific domain(s) of a SAR polypeptide comprise one or more single domain antibodies (SDAB) or antibody fragments such as single vH domain (SVH) or single vL domain (SVL). In some embodiment, the antigen specific domain(s) of a SAR polypeptide comprise single vH domains that are fully human in origin *(i.e.,* FHVH). In some embodiments, the antigen specific domain of a SAR polypeptide comprises one or more camelid V_{HH} (vHH) domains. In some embodiments, the antigen specific domain(s) of a SAR polypeptide comprise vHH domains that are humanized. In some embodiment, the antigen specific domain(s) of a SAR polypeptide comprise variable domains derived from T cell receptors *(e.g*., Va/Vα, Vb/Vβ, Vg/Vγ and Vd/Vδ). In some embodiment, the antigen specific domain(s) of a SAR polypeptide comprise single chain TCR (scTCR). In some embodiment, the antigen specific domain(s) of a SAR polypeptide comprise single varianble domain TCR (svd-TCR). In some embodiments, the antigen specific domain(s) of a SAR polypeptide comprises one or more ligand binding domains of receptors. In some embodiments, the antigen specific domain comprises one or more non-immunoglobulin scaffold such as a DARPIN, an affibody, an affilin, an adnectin, an affitin, an obodies, a repebody, a fynomer, an alphabody, an avimer, an atrimer, a centyrin, a pronectin, an anticalin, a kunitz domain, an Armadillo repeat protein, or a D domain (α3D domain) *etc.* In some embodiments, the antigen specific domain(s) of a SAR polypeptide comprise one or more adaptor binding domains (e.g., RZIP, EZIP, E4, K4, NKG2D-AF, NKG2D-YA *etc.).* In some embodiments, the antigen specific domain(s) of a SAR polypeptide comprise one or more Fc binding domains (*e.g*., Fc binding region of CD16, CD32, or CD64 *etc.).* In some embodiments, the antigen specific domain(s) of a SAR polypeptide comprises one or more an autoantigen. In some embodiments, the antigen specific domain(s) of a SAR polypeptide comprises one or more an epitope tag or mimotope. In some embodiments, the antigen specific domain(s) of a SAR polypeptide comprises one or more ligand-binding domains of receptors. In some embodiments, the antigen specific domain(s) of a SAR polypeptide comprises one or more receptor-binding domains of ligands. In some embodiments, the antigen specific domains of a SAR comprise at least one Fv *(e.g.,* vL and vH fragments) and one or more AABD (*e.g*., SVH, SVL, vHH, svd-TCR, DARPIN, affibody, Centyrin, D domain, epitope, mimotope, EZIP, RZIP, E4, K4 *etc.).* In some embodiments, the antigen specific domains of a SAR comprise at least one TCR variable domain *(e.g.,* Va/Vb or Vg/Vd fragments of a TCR) and one or more AABD *(e.g.,* SVH, SVL, FHVH, vHH, svd-TCR, DARPIN, affibody, Centyrin, D domain, epitope, mimotope, EZIP, RZIP, E4, K4 *etc.).* In an embodiment, the antigen specific domains of a SAR comprise one or more than one *(e.g.,* 2, 3, 4, 5, 6 or more) AABD *(e.g.,* SVH, SVL, vHH, FHVH, svd-TCR, DARPIN, affibody, Centyrin, D domain, epitope, mimotope, EZIP, RZIP, E4, K4 *etc.).* In an embodiment, a SAR comprises AABDs that are of the same type *(e.g.,* two vHH, three DARPINs, two Centyrins *etc.).* In an embodiment, a SAR comprises AABDs that are of different types *(e.g.,* one vHH and one DARPIN; one DARPIN, one vHH domain and one Centyrin *etc.).* In an embodiment, a SAR polypeptide comprises AABDs that are fully human, humanized, chimeric or non-human in origin. In an embodiment, a SAR polypeptide comprises one or more linkers. In an embodiment, a SAR polypeptide comprises one for more flexible linkers (*e.g*., Gly-Ser linker). In an embodiment, a SAR polypeptide comprises one for more protease cleavable linkers (*e.g*., linkers that are cleaved by cellular proteases, *e.g.,* MMP14, *e.g.,* linker encoded by SEQ ID NO:1218). In an embodiment, a SAR polypeptide comprises one or more Ig linkers or Ig like linkers. In an embodiment, a SAR comprises one or more than one chain. In an embodiment, the two or more chains of a SAR comprise one or more polypeptides. In an embodiment, the two or more chains of a SAR are comprisespolypeptides that are separated by cleavable linkers *(e.g*., P2A, T2A, F2A *etc.)* that are optionally preceded by nucleic acid sequences encoding for a Furine cleavage site.

The disclosure provides SAR polynucleotides and polypeptides with the composition of exemplary SARs provided in **Tables 25-36 and 41-50.** As SARs are modular in design, additional SARs with novel compositions can be generated by those skilled in the art by substituting the different modules and tested using the assays provided in this disclosure. The exemplary component modules of the SAR are provided in **Tables 2-24.**

The disclosure also provides useful configurations for making a SAR of the disclosure. The disclosure provides SAR polynucleotides and polypeptides with the modular domain structure and architecture of exemplary SARs provided in **Tables 25-36 and 41-50.** In an embodiment, the disclosure provides at least one recombinant polynucleotide encoding at least one synthetic antigen receptor (SAR), the at least one SAR comprising: a) a first polypeptide chain comprising a vH, Va, Vg or an Ig linker domain fragment operably linked via an optional linker to a first T cell receptor constant chain fragment comprising a first transmembrane domain of a first TCR subunit; and b) a second polypeptide chain comprising a vL, Vb, Vd or an Ig linker domain operably linked via an optional linker to a second T cell receptor constant chain fragment comprising a second transmembrane domain of a second TCR subunit; and wherein one or more polypeptide chains comprise one or more autonomous antigen binding domains (AABD) selected from the group of a single vH domain (SVH); a single vL domain (SVL); a vHH domain; a single domain antibody; a svd-TCR, a non-immunoglobulin antigen binding scaffold such as DARPIN, affibody, affilis, adnectin, affitin, obody, repebody, fynomer, alphabody, avimer, atrimer, centyrin, pronecti, anticalins, kunitz domain, Armadillo repeat protein and a D domain; the ligand binding domain of a receptor; the receptor-binding domain of a ligand; an autoantigen; an adaptor binding domain, an Fc binding domain, an epitope tag, a mimotope, or an equivalent is/are operably linked to or near the N-terminus of the vH, vL, Va, Vb, Vg, Vd or an Ig linker domain via an optional linker; and wherein the first TCR constant chain fragment and the second TCR constant chain fragment form a T cell receptor module (TCRM) that is capable of recruiting at least one TCR-associated signaling module when expressed in a T cells.

In an embodiment, the disclosure provides that a useful configuration for making a bispecific or multispecific CAR involves attachment of one or more AABDs *(e.g*., non-scFV based antigen binding domains) to the N-terminus or near the N-terminus of the vL and/or vH domains comprising the scFv of such a CAR via an optional linker.

In an embodiment, the disclosure provides a useful configurations for a SAR *(e.g.,* a bispecific or a multispecific SAR) comprising one or more AABDs that are operably linked to the N-terminus or near the N-terminus of the vL, vH, scFv, vHH, FHVH, Va, Vb, Vd, Vg fragments comprising a single chain CAR *(e.g.,* a 2nd-generation CAR, a 3^{rd} generation CAR), a single chain TFP *(e.g.,* TFPε, TFPγ, TFPδ), a SIR, a cTCR, an Ab-TCR, an αβTFP, a γδ TFP or a TCR via an optional linker. In another embodiment, the disclosure provides a usefula useful configuration for making a SAR *(e.g.,* a bispecific or a multispecific SAR) with the backbone of an AABD-TCR comprises one or more AABD that are operably linked to TCR signaling modules via intervening Ig linker domains.

The disclosure describes that a useful configuration for making a bispecific or multispecific SIR, Ab-TCR, TFPαβ, TFPγδ or TCR involves attachment of one or more AABDs (e.g., non-scFV based antigen binding domains) to the N-terminus or near the N-terminus of the vL and/or vH domains comprising the Fv of such a SIR via an optional linker.

The disclosure further provides one or more vectors comprising nucleic acids encoding any of the SAR polypeptides and accessory modules described in the preceding sections. The SARs of the disclosure may be encoded by a single vector or more than one vector.

The disclosure further provides genetically modified cells comprising vectors comprising polynucleotides that encode the SARs and accessory modules of the disclosure.

The disclosure further provides methods of treatment using the genetically modified cells comprising vectors comprising polynucleotides that encode the SARs and accessory modules of the disclosure.

The disclosure also provides novel antibodies, antibody fragments, vHH, and single human vH domains (e.g., FHVH and chVH) capable of binding different antigens. The target antigens, names and SEQ ID NOs of these novel antigen binding domains, along with SEQ ID NOs of their CDR1-CDR3, are presented in **Table 39.** These novel antigen binding domains can be used for construction of SARs and/or SAR adaptors of the disclosure. Furthermore, these novel antigen binding domains can be used for the generation of other therapeutic and diagnostic biologicals and cell-based therapies including antibodies (*e.g*., bispecific and trispecific antibodies, antibody drug conjugates, radiolabelled antibodies, flurochromoe labelled antibodies, scFv, bispecific and trispecific T/NK cell engagers *etc.)* using methods described herein and using methods known in the art. The disclosure provides polynucleotide, polypeptides, vector, pharmaceutical compositions, cells and ktis comprising and/or expressing the novel antigen binding domains of the disclosure.

The disclosure further relates to polynucleotides encoding a SAR, SAR polypeptides, vectors comprising polynucleotides encoding SAR polypeptides and isolated cells expressing a SAR of the disclosure. The SARs of the disclosure may be expressed in immune cells *(e.g.,* T cells, NK cell *etc.)* or stem cells *(e.g.,* hematopoietic stem cells or induced pluripotent stem cells iPSC) that can give rise to immune cells. The SARs may be expressed in autologous or allogeneic stem cells. The cells expressing the SARs may have reduced or eliminated expression of one or more components of a TCR/CD3 signaling complex or their downstream signaling mediators. The cells expressing the SARs may have reduced or eliminated expression of HLA molecules, such as via downregulation or knockout of beta2 macroglobulin. The cells for the expression of SARs of the disclosure may be obtained from an autologous or an allogeneic donor. The cells for the expression of SARs of the disclosure may be obtained from an autologous or an allogeneic donor who has been administered a mobilizing agent *(e.g.,* a CXCR4 antagonist, G-CSF, GM-CSF *etc.).* The cells of the disclosure may express one SAR or more than one SAR. In an exemplary embodiment, one of the SARs may result in immune cell effector functions (*e.g*., cytotoxicity) while the other SAR may provide co-stimulation. The SARs expressing cells may target a single antigen or more than one antigen. The SAR-expressing cells may target the same epitope or different epitopes of a single antigen. In one aspect the SAR expressing cells may recognize an antigen preferentially or exclusively expressed on hematopoietic lineage cells. Exemplary antigens that are preferentially or exclusively expressed on hematopoietic lineage cells are CD19, CD20, CD22, BCMA, CS1, CD30, CD33, MPL, CD138, CD38, CD79b, BAFF-R, CD123 and the like. In one aspect the SAR-expressing cells may recognize an antigen preferentially or exclusively expressed on non-hematopoietic lineage cells. Exemplary antigens that are preferentially or exclusively expressed on non-hematopoietic lineage cells are Mesothelin (MSLN), Her2, EGFR, EGFRviii, Muc16, PSMA, IL13Ra2 and the like. In one aspect the SAR expressing cells recognize two or more novel antigen where at least one of the antigens is preferentially or exclusively expressed on hematopoietic lineage cells and at least one of the antigens is expressed on non-hematopoietic lineage cells. The SARs expressing cells of the disclosure can be used in therapy, in particular for the treatment of cancer. Methods for treating disease, for example cancer, are also within the scope of the invention.

In one embodiment, provided herein are isolated nucleic acid encoding unispecific, bispecific and multispecific SARs, wherein the antigen specific domain of the SAR targets BCMA. In exemplary embodiments, the sequences of isolated nucleic acid fragments targeting BCMA are set forth in SEQ ID NOs: 7409-8374. In exemplary embodiments, the sequences of isolated polypeptide targeting BCMA are set forth in SEQ ID NOs: 18099-19064 **(Table 36).** In some embodiments, the vL, vH and scFv fragments targeting BCMA are described in **Table 3** and set forth in SEQ ID NOs: 235-248, 477-490 and SEQ ID NOs: 719-732, respectively. The amino acid SEQ ID NO of the vL, vH and scFv fragments targeting BCMA are also described in **Table 3** and set forth in SEQ ID Nos:10925-10938, 10978-11180, and 11220-11422, respectively. In some embodiments, the SVH and vHH fragments targeting BCMA are described in **Table 4** and set forth in SEQ ID NOs: 852-858, 888-891, 893, 895, 901-902. In some embodiments, a Centyrin targeting BCMA is described in **Table 7** and set forth in SEQ ID NO: 983. Exemplary unispecific, bispecific and multispecific SARs incorporating AABD comprising SVH, vHH and Centyrins are provided in Tables 25-36. Further provided herein are vectors encoding nucleic acids encoding SAR, wherein the antigen specific domain of the SAR targets BCMA. Also provided herein are genetically engineered cells (such as T cells, NK cells) comprising vectors comprising nucleic acids encoding a SAR, wherein the antigen specific domain of the SAR targets BCMA.

In one embodiment, provided herein are isolated nucleic acid encoding unispecific, bispecific and multispecific SARs, wherein the antigen specific domain of the SAR targets PSMA (prostate specific membrane antigen). In exemplary embodiments, the sequences of isolated nucleic acid fragments targeting PSMA are set forth in SEQ ID NOs: 9686-10030. In exemplary embodiments, the sequences of isolated polypeptide targeting PSMA are set forth in SEQ ID NOs: 20376-20720 **(Table 36).** In some embodiments, the vL, vH and scFv fragments targeting PSMA are described in **Table 3** and set forth in SEQ ID NOs: 268-272, 510-514 and SEQ ID NOs: 752-756, respectively. The amino acid SEQ ID NO of the vL, vH and scFv fragments targeting PSMA are also described in **Table 3** and set forth in SEQ ID Nos: 10958-10962, 11200-11204, and 11442-11446, respectively. In some embodiments, the SVH fragments targeting PSMA are described in **Table 4** and set forth in SEQ ID NOs: 830-833. In some embodiments, Centyrins targeting PSMA is described in **Table** 7 and set forth in SEQ ID NO: 977-979. Exemplary unispecific, bispecific and multispecific SARs incorporating AABD comprising SVH, vHH and Centyrins are provided in **Tables 25-36.** Further provided herein are vectors comprising nucleic acids encoding a SAR, wherein the antigen specific domain of the SAR targets PSMA. Also provided herein are genetically engineered cells (such as T cells, NK cells) comprising vectors comprising nucleic acids encoding SAR, wherein the antigen specific domain of the SAR targets PSMA. Also provided are SAR that target PSMA with optimized expression and affinity so as to target cancer cells that express high levels of PSMA (*e.g*., more than 1.5-fold higher than normal cells) and spare normal healthy cells that express low to moderate level of PSMA. In an exemplary embodiment are provided SAR that respond to cancer cells that express PSMA at level at least 1.5-fold higher than the PSMA level observed in normal healthy prostate epithelium or immortalized cell lines derived from normal prostate epithelial cells. The expression level of PSMA can be measured by techniques known in the art, including but not limited to, immunohistochemistry, western blotting, northern blot and quantitative PCR *etc.* In one embodiment, the disclosure provides novel SARs targeting cells expressing high levels of PSMA and do not target cells expressing low levels of PSMA. A reference cell expressing high level of PSMA is LNCaP cell line and a cell expressing low levels of PSMA is PC3 and/or Huh-7 cell line. In an embodiment, the disclosure provides SARs that show 2-fold or higher *(e.g.,* 3 fold, 4 fold, 5 fold, 10 fold *etc.)* GFP induction in the Jurkat NFAT-GFP assay when expressed in JNG cells and co-cultured with LNCaP cells as compared to the GFP induction observed when co-cultured with PC3 or Huh-7 cell lines. In an embodiment, the disclosure provides SARs that show greater than 10% *(e.g.,* 20%, 30%, 40% or 50% *etc.)* GFP induction in the Jurkat NFAT-GFP assay when expressed in JNG cells and co-cultured with LNCaP cells as compared to the GFP induction observed when co-cultured with PC3 or Huh-7 cell lines.

In one embodiment, provided herein are isolated nucleic acid encoding unispecific, bispecific and multispecific SARs, wherein the antigen specific domain of the SAR targets Mesothelin (MSLN). In exemplary embodiments, the sequences of isolated nucleic acid fragments targeting MSLN are set forth in SEQ ID NOs: 10307-10720. In exemplary embodiments, the sequences of isolated polypeptide targeting MSLN are set forth in SEQ ID NOs: 20997-21410 **(Table 36).** In some embodiments, the vL, vH and scFv fragments targeting MSLN are described in **Table 3** and set forth in SEQ ID NOs: 277-282, 519-524 and 761-766, respectively. The amino acid SEQ ID NO of the vL, vH and scFv fragments targeting MSLN are also described in **Table 3** and set forth in SEQ ID Nos: 10967-10972, 11209-11214, and 11451-11456, respectively. In some embodiments, the vHH fragments targeting MSLN are described in **Table 4** and set forth in SEQ ID NOs: 876-877. Exemplary unispecific, bispecific and multispecific SARs incorporating vL, vH, scFv, vHH targeting MSLN are provided in Tables 25-36. Further provided herein are vectors comprising nucleic acids encoding a SAR, wherein the antigen specific domain of the SAR targets MSLN. Also provided herein are genetically engineered cells (such as T cells, NK cells) comprising vectors comprising nucleic acids encoding a SAR, wherein the antigen specific domain of the SAR targets MSLN. Also provided are SAR that target MSLN with optimized expression and affinity so as to target cancer cells that express high levels of MSLN *(e.g.,* more than 1.5-fold higher than normal cells) and spare normal healthy cells that express low to moderate level of MSLN. In an exemplary embodiment are provided SAR that respond to cancer cells that express MSLN at level at least 1.5 fold higher than the MSLN level observed in normal healthy peritoneal epithelium cells or immortalized cell lines derived from normal healthy peritoneal epithelial cells. The expression level of MSLN can be measured by techniques known in the art, including but not limited to, immunohistochemistry, western blotting, northern blot and quantitative PCR *etc.* In one embodiment, the disclosure provides novel SARs targeting cells expressing high levels of MSLN and do not target cells expressing low levels of MSLN. A reference cell expressing high level of MSLN is SKOV-3 cell line and a cell expressing low levels of MSLN is MCF-7 or LNCaP cell line. In an embodiment, the disclosure provides SARs that show 2 fold or higher *(e.g.,* 3 fold, 4 fold, 5 fold, 10 fold *etc.)* GFP induction in the Jurkat NFAT-GFP assay when expressed in JNG cells and co-cultured with SKOV-3 cells as compared to the GFP induction observed when co-cultured with MCF-7 or LNCaP cell line. In an embodiment, the disclosure provides SARs that show greater than 10% *(e.g.,* 20%, 30%, 40% or 50% *etc.)* GFP induction in the Jurkat NFAT-GFP assay when expressed in JNG cells and co-cultured with SKOV-3 cells as compared to the GFP induction observed when co-cultured with MCF-7 or LNCaP cell lines.

In one embodiment, provided herein are isolated nucleic acid encoding unispecific, bispecific and multispecific SARs, wherein the antigen specific domain of the SAR targets Her2. In exemplary embodiments, the sequences of isolated nucleic acid fragments targeting Her2 are set forth in SEQ ID NOs: 8858-8995. In exemplary embodiments, the sequences of isolated polypeptide targeting HER2 are set forth in SEQ ID NOs: 19548-19616 **(Table 36).** In some embodiments, the vL, vH and scFv fragments targeting HER2 are described in **Table 3** and set forth in SEQ ID NOs: 256-257, 498-499 and 740-741, respectively. The amino acid SEQ ID NO of the vL, vH and scFv fragments targeting HER2 are also described in **Table 3** and set forth in SEQ ID Nos: 10946-10947, 11188-11189, and 11430-11431, respectively. In some embodiments, the vHH fragments targeting HER2 are described in **Table 4** and set forth in SEQ ID NOs: 864-866. In some embodiments, the DARPINS fragments targeting HER2 are described in **Table 4** and set forth in SEQ ID NOs: 972-973. Exemplary unispecific, bispecific and multispecific SARs incorporating vL, vH, scFv, vHH and DARPINS targeting HER2 are provided in Tables 25-36. Further provided herein are vectors comprising nucleic acids encoding a SAR, wherein the antigen specific domain of the SAR targets HER2. Also provided herein are genetically engineered cells (such as T cells, NK cells) comprising vectors comprising nucleic acids encoding a SAR, wherein the antigen specific domain of the SAR targets HER2. Also provided are SAR that target Her2 with optimized expression and affinity so as to target cancer cells that express high levels of Her2 *(e.g.,* more than 1.5-fold higher than normal cells) and spare normal healthy cells that express low to moderate level of Her2. In an exemplary embodiment are provided SAR that respond to cancer cells that express Her2 at level at least 1.5-fold higher than the Her2 level observed in normal healthy breast epithelium cells or immortalized cell lines derived from normal healthy breast epithelium. The expression level of Her2 can be measured by techniques known in the art, including but not limited to, immunohistochemistry, western blotting, northern blot and quantitative PCR *etc.* In one embodiment, the disclosure provides novel SARs targeting cells expressing high levels of Her2 and do not target cells expressing low levels of Her2. A reference cell expressing high level of Her2 is SKOV-3 cell line and a cell expressing low levels of Her2 is MBA-MD-231 cell line. In an embodiment, the disclosure provides SARs that show 2-fold or higher *(e.g.,* 3-fold, 4 fold, 5 fold, 10 fold *etc.)* GFP induction in the Jurkat NFAT-GFP assay when expressed in JNG cells and co-cultured with SKOV-3 cells as compared to the GFP induction observed when co-cultured with MBA-MD-231 cell line. In an embodiment, the disclosure provides SARs that show greater than 10% *(e.g.,* 20%, 30%, 40% or 50% *etc.)* GFP induction in the Jurkat NFAT-GFP assay when expressed in JNG cells and co-cultured with SKOV-3 cells as compared to the GFP induction observed when co-cultured with MBA-MD-231 cell line.

In one embodiment, provided herein are isolated nucleic acid encoding unispecific, bispecific and multispecific SARs, wherein the antigen specific domain of the SAR targets CD229. In exemplary embodiments, the sequences of isolated nucleic acid fragments targeting CD229 are set forth in SEQ ID NOs: 9134-9409. In exemplary embodiments, the sequences of isolated polypeptide targeting CD229 are set forth in SEQ ID NOs: 19824-20099 **(Table 36).** In some embodiments, the vL, vH and scFv fragments targeting CD229 are described in **Table 3** and set forth in SEQ ID NOs: 260-263, 502-505 and 744-747, respectively. The amino acid SEQ ID NO of the vL, vH and scFv fragments targeting CD229 are also described in **Table 3** and set forth in SEQ ID Nos: 10950-10953, 11192-11195, and 11434-11437, respectively. Exemplary unispecific, bispecific and multispecific SARs incorporating comprising vL, vH, and scFv targeting CD229 are provided in Tables 25-36. Further provided herein are vectors comprising nucleic acids encoding a SAR, wherein the antigen specific domain of the SAR targets CD229. Also provided herein are genetically engineered cells (such as T cells, NK cells) comprising vectors comprising nucleic acids encoding a SAR, wherein the antigen specific domain of the SAR targets CD229.

In one embodiment, provided herein are isolated nucleic acid encoding unispecific, bispecific and multispecific SARs, wherein the antigen specific domain of the SAR targets ROR1. In exemplary embodiments, the sequences of isolated nucleic acid fragments targeting ROR1 are set forth in SEQ ID NOs: 8375-8581. In exemplary embodiments, the sequences of isolated polypeptide targeting ROR1 are set forth in SEQ ID NOs: 19065-19133 **(Table 36).** The amino acid SEQ ID NO of the vL, vH and scFv fragments targeting CD229 are also described in **Table 3.** Further provided herein are vectors comprising nucleic acids encoding a SAR, wherein the antigen specific domain of the SAR targets ROR1. Also provided herein are genetically engineered cells (such as T cells, NK cells) comprising vectors comprising nucleic acids encoding a SAR, wherein the antigen specific domain of the SAR targets ROR1.

In one embodiment, provided herein are isolated nucleic acid encoding unispecific, bispecific and multispecific SARs, wherein the antigen specific domain of the SAR targets CEA. In exemplary embodiments, the sequences of isolated nucleic acid fragments targeting CEA are set forth in SEQ ID NOs: 8720-8857. In exemplary embodiments, the sequences of isolated polypeptide targeting CEA are set forth in SEQ ID NOs: 19410-19547 **(Table 36).** In some embodiments, the vL, vH and scFv fragments targeting CEA are listed in **Table 3.** The amino acid SEQ ID NO of the vL, vH and scFv fragments targeting CEA are also described in **Table 3.** Further provided herein are vectors comprising nucleic acids encoding a SAR, wherein the antigen specific domain of the SAR targets CEA. Also provided herein are genetically engineered cells (such as T cells, NK cells) comprising vectors comprising nucleic acids encoding a SAR, wherein the antigen specific domain of the SAR targets CEA.

In one embodiment, provided herein are isolated nucleic acid encoding unispecific, bispecific and multispecific SARs, wherein the antigen specific domain of the SAR targets Toso. In exemplary embodiments, the sequences of isolated nucleic acid fragments targeting Toso are set forth in SEQ ID NOs: 8996-9064. In exemplary embodiments, the sequences of isolated polypeptide targeting Toso are set forth in SEQ ID NOs: 19686-19747 **(Table 36).** In some embodiments, the vL, vH and scFv fragments targeting Toso are listed in **Table 3.** The amino acid SEQ ID NO of the vL, vH and scFv fragments targeting Toso are also described in **Table 3.** Further provided herein are vectors comprising nucleic acids encoding a SAR, wherein the antigen specific domain of the SAR targets Toso. Also provided herein are genetically engineered cells (such as T cells, NK cells) comprising vectors comprising nucleic acids encoding a SAR, wherein the antigen specific domain of the SAR targets Toso.

In one embodiment, provided herein are isolated nucleic acid encoding unispecific, bispecific and multispecific SARs, wherein the antigen specific domain of the SAR targets EBV encoded gp350 protein. In exemplary embodiments, the sequences of isolated nucleic acid fragments targeting EBV gp350 are set forth in SEQ ID NOs: 9410-9547. In exemplary embodiments, the sequences of isolated polypeptide targeting EBV gp350 are set forth in SEQ ID NOs: 20169-20237 **(Table 36).** In some embodiments, the vL, vH and scFv fragments targeting EBV gp350 are listed in **Table 3.** The amino acid SEQ ID NO of the vL, vH and scFv fragments targeting EBV gp350 are also described in **Table 3.** Further provided herein are vectors comprising nucleic acids encoding a SAR, wherein the antigen specific domain of the SAR targets EBV gp350. Also provided herein are genetically engineered cells (such as T cells, NK cells) comprising vectors comprising nucleic acids encoding a SAR, wherein the antigen specific domain of the SAR targets EBV gp350. These SARs are useful for the treatment of disorders associated with infection with EBV.

In one embodiment, provided herein are isolated nucleic acid encoding unispecific, bispecific and multispecific SARs, wherein the antigen specific domain of the SAR targets EBV encoded LMP1 protein. In exemplary embodiments, the sequences of isolated nucleic acid fragments targeting EBV LMP1 are set forth in SEQ ID NOs: 9617-9685. In exemplary embodiments, the sequences of isolated polypeptide targeting EBV LMP1 are set forth in SEQ ID NOs: 20307-20375 **(Table 36).** In some embodiments, the vL, vH and scFv fragments targeting EBV LMP1 are listed in **Table 3.** The amino acid SEQ ID NO of the vL, vH and scFv fragments targeting EBV LMP1 are also described in **Table 3.** Further provided herein are vectors comprising nucleic acids encoding a SAR, wherein the antigen specific domain of the SAR targets EBV LMP1. Also provided herein are genetically engineered cells (such as T cells, NK cells) comprising vectors comprising nucleic acids encoding a SAR, wherein the antigen specific domain of the SAR targets EBV LMP1. These SARs are useful for the treatment of disorders associated with infection with EBV.

In one embodiment, provided herein are isolated nucleic acid encoding unispecific, bispecific and multispecific SARs, wherein the antigen specific domain of the SAR targets Influenza A Neuramidase (NA) protein. In exemplary embodiments, the sequences of isolated nucleic acid fragments targeting Influenza A NA are set forth in Table 36. In exemplary embodiments, the sequences of isolated polypeptide targeting Influenza A NA are set forth in **(Table 36).** In some embodiments, the vL, vH and scFv fragments targeting Influenza A NA are listed in **Table 3.** The amino acid SEQ ID NO of the vL, vH and scFv fragments targeting Influenza A NA are also described in **Table 3.** Further provided herein are vectors comprising nucleic acids encoding a SAR, wherein the antigen specific domain of the SAR targets Influenza A NA. Also provided herein are genetically engineered cells (such as T cells, NK cells) comprising vectors comprising nucleic acids encoding a SAR, wherein the antigen specific domain of the SAR targets Influenza A NA. These SARs are useful for the treatment of disorders associated with infection with Influenza A.

In one embodiment, provided herein are isolated nucleic acid encoding SARs, wherein the antigen specific domain of the SAR targets receptor binding domain of SARS-CoV2 spike glycoprotein (S-RBD). In exemplary embodiments, the sequences of isolated nucleic acid fragments targeting S-RBD are set forth in SEQ ID NO: 25-26. In exemplary embodiments, the sequences of isolated polypeptide targeting S-RBD are set forth in SEQ ID NO: 23318-19. Further provided herein are vectors comprising nucleic acids encoding a SAR, wherein the antigen specific domain of the SAR targets S-RBD. Also provided herein are genetically engineered cells (such as T cells, NK cells) comprising vectors comprising nucleic acids encoding a SAR, wherein the antigen specific domain of the SAR targets S-RBD. These SARs are useful for the treatment of disorders associated with infection with S-RBD.

In one embodiment, provided herein are isolated nucleic acid encoding unispecific, bispecific and multispecific SARs, wherein the antigen specific domain of the SAR targets an antigen listed in **Tables 25-36.** In exemplary embodiments, the sequences of isolated nucleic acid fragments targeting the antigens are set forth in SEQ ID NOs: 1330-1332, 1848-10720. In exemplary embodiments, the sequences of isolated polypeptide targeting the different antigens are set forth in SEQ ID NOs:12020-12022, 12539-21410 **(Table 36).** In some embodiments, the vL, vH and scFv fragments targeting the different antigens are described in **Table 3** and set forth in SEQ ID NOs: 46-282, 288-524 and 530-766, respectively. The amino acid SEQ ID NO of the vL, vH and scFv fragments targeting various antigens are also described in **Table 3** and set forth in SEQ ID Nos: 10736-10972, 10978-11214, and 11220-11456, respectively. In some embodiments, the exemplary AABD *(e.g.,* vHH, SVH, DARPIN, CENTYRIN, AFFIBODY, ligands, receptors, Zip domains, protein tags *etc.)* targeting various antigens are described in **Table 5, 7-9** and set forth in SEQ ID NOs: 830-902; 972-1023. Exemplary unispecific, bispecific and multispecific SARs incorporating vL, vH, scFv, and AABD are provided in Tables 25-36 and 41-50. Further provided herein are vectors comprising nucleic acids encoding a SAR, wherein the antigen specific domain(s) of the SAR targets different antigens. Also provided herein are genetically engineered cells (such as T cells, NK cells) comprising vectors comprising nucleic acids encoding a SAR, wherein the antigen specific domain(s) of the SAR targets different antigens.

The disclosure further relates to improving the quality of SAR expressing T cells by expanding them in the presence of a SMAC mimetic.

The disclosure further relates to improving the quality of SAR expressing T cells by expanding them in the presence of a NIK (NF-κB inducing kinase) agonist.

The SARs of the disclosure may be manufactured using an open, semi-close or close manufacturing system.

The SARs of the disclosure may be manufactured using a device or a container containing a gas-permeable membrane. An exemplary device with gas permeable membrane is a GRex flask.

The disclosure further teaches a method of manufacturing SARs under hypoxic conditions.

The disclosure further teaches a method of manufacturing SARs under hypoxic conditions using a device or a container containing a gas-permeable membrane. An exemplary device with gas permeable membrane is a GRex flask.

The disclosure also teaches a method of controlling the activity a SAR by incorporating in them at least one module that binds to albumin via a protease cleavable linker. An exemplary module that binds to albumin is a vH or vH domain targeting albumin *(e.g.,* SEQ ID NO (DNA): 878 and SEQ ID NO (PRT): 11568) that can be linked to a SAR via a MMP9 target sequence *(e.g.,* SEQ ID NO: 11911-11912 and 11921).

The disclosure further relates to polynucleotides encoding a SVH of the disclosure, vectors encoding such polypeptides, isolated cells expressing the SVH and isolated polypeptides encoding SVH of the disclosure. Such cells and polypeptides can be used in therapy, in particular for the treatment of cancer, autoimmune and infectious diseases. Methods for treating disease, for example cancer, are also within the scope of the disclosure.

In another aspect, the disclosure relates to an isolated SAR comprising two or more antigen binding domains, and two transmembrane domains wherein said antigen binding domain comprises one or more, for example at least two, human variable heavy chain (V_{H}) domains and is devoid of light chains.

In some embodiments, a SAR comprises two or more sets of two or more polypeptides. The polypeptides of each set of SARs are contiguous with each other (functional polypeptide unit 1) but are not contiguous with the polypeptides of the other set (functional polypeptide unit 2).

In another aspect, the disclosure relates to a method, for example an *ex vivo* method, for generating a cell or cell population for use in adaptive immunotherapy comprising transforming said cell or cell population with a SAR of the disclosure.

In another aspect, the disclosure relates to a method of generating SAR-expressing T cells *(e.g.,* CAR-T or SIR-T or TCR-T or ab-TCR-T, AABD-TCR-T, or TFP-T, TCR-T cells *etc.)* by expanding them in the presence of SMAC mimetics. In some embodiments, the SAR-T cells are expanded *ex vivo* in the presence of a SMAC mimetic for between 2-50 days.

In another aspect, the disclosure relates to a method of improving the efficacy of SAR-expressing T cells *(e.g.,* CAR-T or SIR-T or TCR-T or ab-TCR-T, AABD-TCR-T, or TFP-T, TCR-T cells *etc.)* by expanding them in the presence of SMAC mimetics. In some embodiments, the SAR-T cells are expanded *ex vivo* in the presence of a SMAC mimetic for between 2-50 days.

In another aspect, the disclosure relates to a method of improving the cytotoxicity of SAR-expressing T cells *(e.g.,* CAR-T or SIR-T or TCR-T or ab-TCR-T, AABD-TCR-T, or TFP-T, TCR-T cells *etc.)* by expanding them in the presence of SMAC mimetics. In some embodiments, the SAR-T cells are expanded *ex vivo* in the presence of a SMAC mimetic for between 2-50 days.

In another aspect, the disclosure relates to a method of generating SAR-expressing T cells *(e.g.,* CAR-T or SIR-T or TCR-T or ab-TCR-T, or TFP-T cells *etc.)* by expanding them in the presence of NIK agonist. In some embodiments, the SAR-T cells are expanded ex *vivo* in the presence of a NIK agonist for between 2-50 days.

In another aspect, the disclosure relates to a method of improving the efficacy of SAR-expressing T cells *(e.g.,* CAR-T or SIR-T or TCR-T or ab-TCR-T, AABD-TCR-T, or TFP-T, TCR-T cells *etc.)* by expanding them in the presence of NIK agonist. In some embodiments, the SAR-T cells are expanded *ex vivo* in the presence of NIK agonist for between 2-50 days.

In another aspect, the disclosure relates to a method of improving the cytotoxicity of SAR-expressing T cells *(e.g.,* CAR-T or SIR-T or TCR-T or ab-TCR-T, AABD-TCR-T, or TFP-T, TCR-T cells *etc.)* by expanding them in the presence of NIK agonist. In some embodiments, the SAR-T cells are expanded *ex vivo* in the presence of a NIK agonist for between 2-50 days.

In another aspect, the disclosure relates to a pharmaceutical composition comprising a cell or cell population of the disclosure.

Bioluminescence imaging is frequently used for monitoring the *in vivo* activity of cellular therapy products. The disclosure describes the use of thermostable luciferases (*e.g*., LucPPE and its engineered variants such as LucPPe-146-1H2 (SEQ ID NO (DNA): 17 and SEQ ID NO (PRT): 11997), LucPPe-133-1B2 (SEQ ID NO (DNA): 18), LucPPe-78-0B10 (SEQ ID NO: 19), LucPPe49-7C6A (SEQ ID NO: 20), LucPpL-81-6G1 (SEQ ID NO: 21) for *in vivo* bioluminescence imaging.

The disclosure also provides novel vL, vH and scFv fragments that can be used in the construction of single and double chain SARs, including 2^{nd} generation CARs, single and double chain SIRs, single and double chain cTCRs, Ab-TCR, AABD-TCR, TFPs, TAC and the like. Exemplary vL, vH and scFv fragments and their target antigens are listed in Table 3. Exemplary unispecific, bispecific and multispecific SARs based on the vL, vH and scFv fragments of hu-mROO5-1 are provided in Table 35. As the SARs are modular, the vL, vH and scFv fragments of hu-mROO5-1 can be replaced by vL, vH and scFv fragments targeting other antigen to develop SARs targeting those antigens. Table 36 provides the name of the first construct and SEQ ID NO of other constructs of the series in which vL, vH and scFv fragments of hu-mROO5-1 have been replaced by other vL, vH and scFv fragments. The order of the construct in each series is the same as the order of the constructs of the hu-mROO5-1 series listed in Table 35 and therefore the name and SEQ ID NO of a construct of a particular series can be determined by reference to Tables 35 and 36. For example, Table 35 shows that the last construct in the hu-mROO5-1 series (Series 1) is CD8SP-CD38-USC1-FHVH-32184-G4Sx3-R1-hu-mROO5-1-vL-[hTCRb-S57C]-F-P2A-IgSP-Apa-CD20-USC1-vHH-2HCD26-G4Sx3v2-Bst-hu-mROO5-1-vH-[hTCRa-T48C] and has the nucleic acid and amino acid SEQ ID Nos of 7408 and 18098. Table 36 shows that in the series 5, the hu-mROO5-1-vL is replaced by BCMA-huUSC76-vL and hu-mROO5-1-vH is replaced by BCMA-huUSC76-I58S-vH. The nucleic acid SEQ ID Nos of the different constructs of this series range from 7616-7684 and the amino acid SEQ ID Nos of the constructs in this series range from 18306-18374. As the order of the different constructs in series 5 is the same as the order of the constructs of series 1 shown in **Table** 35, the nucleic acid and amino acid SEQ ID Nos of the construct CD8SP-CD38-USC1-FHVH-32184-G4Sx3-R1-BCMA-huUSC76-vL-[hTCRb-S57C]-F-P2A-IgSP-Apa-CD20-USC1-vHH-2HCD26-G4Sx3v2-Bst-BCMA-huUSC76-I58S-vH-[hTCRa-T48C] can be determined to be 7684 and 18374, respectively. The same method can be used to determine the SEQ ID NO of any SAR of the series listed in **Table** 36.

In an embodiment, the disclosure provides at least one recombinant polynucleotide encoding at least one synthetic antigen receptor (SAR) polypeptide, the at least one SAR polypeptide comprising: (A) a first module comprising one or more autonomous antigen binding domains (AABDs) or fragments thereof selected from the group of: a single vH domain (SVH) or a fragment thereof; a single vL domain (SVL) or a fragment thereof; a vHH domain or a fragment thereof; a single domain antibody or a fragment thereof; a single variable domain of a TCR (svd-TCR) or a fragment thereof; a non-immunoglobulin antigen binding scaffold selected from a DARPIN, an affibody, an affilis, an adnectin, an affitin, an obody, an repebody, an fynomer, an alphabody, an avimer, an atrimer, an centyrin, an pronecti, an anticalins, an kunitz domain, an Armadillo repeat protein and a D domain, or a fragment of any of the foregoing; a ligand-binding domain of a receptor or a fragment thereof; a receptor-binding domain of a ligand; an autoantigen or a fragment thereof; an adaptor binding domain or a fragment thereof; and an Fc binding domain or a fragment thereof; and (B) a second module comprising vL (V_{L}), vH (V_{H}), Va/Vα, Vb1 (Vβ1), Vb2 (Vβ2), Vg (Vγ), Vd (Vδ), scFv, scTCR, Ig linker domain or a combination thereof; (C) an optional third module comprising an extracellular domain, a connecting peptide or a hinge domain; (D) a fourth module that comprises a transmembrane domain; and (E) an optional fifth module comprising one or more intracellular signaling domains; where the first, second, optional third, fourth and the optional fifth modules are operationally linked via one or more optional linkers.

The disclosure also provides a recombinant polynucleotide encoding a SAR polypeptide, where the one or more AABDs are attached via an optional linker to the N-terminus or near the N-terminus of A) a first polypeptide chain comprising a vH, Va, Vg or an Ig linker domain fragment operably linked via an optional linker to a first T cell receptor constant chain fragment comprising a first connecting peptide operably linked to a first transmembrane domain of a first TCR subunit; and (B) a second polypeptide chain comprising a vL, Vb, Vd or an Ig linker domain operably linked via an optional linker to a second T cell receptor constant chain fragment comprising a second connecting peptide operably linked to a second transmembrane domain of a second TCR subunit; wherein the first TCR constant chain fragment and the second TCR constant chain fragment form a T cell receptor module (TCRM) that is capable of recruiting at least one TCR-associated signaling module when expressed in a T cell.

In one embodiment, the disclosure provides at least one recombinant polynucleotide encoding at least one SAR polypeptide, wherein the encoded vH, Va, or Vg of the first antigen-binding domain and the vL, Vb or Vd of the second antigen-binding domain form an antigen-binding module that specifically binds to a target antigen.

In one embodiment, the disclosure provides at least one recombinant polynucleotide encoding at least one SAR polypeptide, wherein the AABD, vL, vH, Va, Vb, Vg, Vd and/or Ig linker domain is a fully human, humanized, chimeric or a non-human domain.

In one embodiment, the disclosure provides at least one recombinant polynucleotide encoding at least one SAR polypeptide, wherein the encoded Ig linker domain comprises a polypeptide with SEQ ID NO: 11832-11865 or a fragment or variant thereof with at least 70% sequence homology to a polypeptide having the sequence of SEQ ID NO: 11832-11865. In one embodiment, the disclosure provides at least one recombinant polynucleotide encoding at least one SAR polypeptide, wherein the encoded optional linker domain comprises a polypeptide with SEQ ID NO: 11832-11865, 11714-11730 or a fragment or a variant thereof with at least 70% sequence homology to a polypeptide having the sequence of SEQ ID NO: 11832-11865, 11714-11730 or a domain that is between 25 to 500 amino acids in length. In one embodiment, the disclosure provides at least one recombinant polynucleotide encoding at least one SAR polypeptide comprising a partial or entire extacellular, transmbrane and intracellular domains of a polypeptide capable of being recruited to a T cell receptor module (TCRM). In one embodiment, the disclosure provides at least one recombinant polynucleotide encoding at least one SAR polypeptide comprising the extacellular, transmbrane and intracellular domains of a polypeptide selected from the group of CD3δ, CD3ε, CD3γ and CD3ζ or a fragment thereof with at leasst 70% sequence homology to a polypeptide having the sequence of any one of SEQ ID NO: 11903 to 11906. In an embodiment, the disclosure provides at least one recombinant polynucleotide encoding at least one SAR polypeptide wherein the encoded T cell receptor constant chain fragment comprises a polypeptide or a fragment thereof having at least 70% sequence homology to a polypeptide having the sequence of any one of SEQ ID NO: 11732-11830. In one embodiment, the disclosure provides at least one recombinant polynucleotide encoding at least one SAR, wherein the encoded T cell receptor constant chain comprises a connecting peptide or a fragment thereof having at least 70% sequence homology to a connecting peptide having the sequence of any one of SEQ ID NO: 11867-11875. In one embodiment, the disclosure provides at least one recombinant polynucleotide encoding at least one SAR polypeptide comprising a transmembrane domain or a fragment thereof having at least 70% sequence homology to a transmembrane domain having a sequence of any one of SEQ ID NO: 11877-11881 or 23332-23334. In one embodiment, the disclosure provides at least one recombinant polynucleotide encoding at least one SAR polypeptide, wherein the intracellular domain contains a cytosolic domain or a fragment thereof with at least 70% sequence homology to a polypeptide having the sequence of any one of SEQ ID NO: 11883-11886, 11785, or 23335-23337.

In one embodiment, the disclosure provides at least one recombinant polynucleotide encoding at least one SAR polypeptide which has the backbone of a first generation CAR, a second generation CAR, a third generation CAR, a single chain SIR, a one and a half chain SIR, a double chain SIR, a zSIR, a single chain cTCR, a one and a half chain cTCR, a double chain cTCR, an Ab-TCR, an AABD-TCR, a TFPε, a TFPγ, a TFPδ, a TFPαβ, a TFPγδ, a Tri-TAC, a single chain TCR, a double chain TCR or an HLA-independent TCR.

In one embodiment, the disclosure provides the at least one recombinant polynucleotide encoding at least one SAR polypetpide, wherein the SAR can bind to: at least one antigen; and/or at least one epitope of one or more than antigen(s). In one embodiment, the disclosure provides at least one recombinant polynucleotide encoding at least one SAR polypeptide of disclosure, where the encoded two or more AABDs bind to: at least one antigen; and/or at least one epitope of one or more than antigen(s).

In one embodiment, the disclosure provides the at least one recombinant polynucleotide encoding at least one SAR polypeptide wherein the encoded one or more antigen binding domains bind to at least one antigen selected from the antigens listed in **Table B.**

In one embodiment, the disclosure provides at least one recombinant polynucleotide encoding at least one SAR polypeptide wherein the encoded SAR polypeptide comprises one or more antigen binding domains selected from the group consisting of: (i) a heavy chain variable region (vH) comprising a sequence as set forth in any of SEQ ID Nos: 10978-11214 or sequences with at least 70% identity thereto or sequences with at least 70% identity in the three complementarity determining regions (CDRs) to the sequences set forth in any one or more of SEQ ID NOS: 10978-11214 and 23148-23161 or sequences with less than 3 substitutions in the three CDRs of the sequences set forth in any one or more of SEQ ID NOS: 10978-11214 and 23148-23161 or sequences that bind to the same target antigens or the same epitopes on the target antigens as the sequences set forth in any one or more of SEQ ID NOS: 10978-11214 and 23148-23161 and which encodes a polypeptide that binds to its antigen; (ii) a light chain variable region (vL) comprising a sequence as set forth in any one of SEQ ID NO: 10736-10972 or sequences with at least 70% identity to sequences set forth in any one or more of SEQ ID NOS: 10736-10972 and 23136-23147 or sequences with at least 70% identity in the three complementarity determining regions (CDRs) to the sequences set forth in any one or more of SEQ ID NOS: 10736-10972 and 23136-23147 or sequences with less than 3 substitutions in the three CDRs of the sequences set forth in any one or more of SEQ ID NOS: 10736-10972 and 23136-23147 or sequences that bind to the same target antigens or the same epitopes on the target antigens as the sequences set forth in any one or more of SEQ ID NOS: 10736-10972 and 23136-23147 and which encodes a polypeptide that binds to its antigen; (iii) a single chain variable fragment (scFv) comprising a sequence as set forth in any one SEQ ID NO: 11220-11456 or sequences with at least 70% identity thereto and which encodes a polypeptide that binds to its antigen; (iv) a camelid VHH domain comprising a sequence as set forth in any one of SEQ ID NO: SEQ ID NOS: 11524-11525, 11530-11531, 11549-11575, 11576-11592 and 23163-23173 or sequences with at least 70% identity to sequences set forth in any one or more of SEQ ID NOS: 11520-11592 and 23163-23173 and or sequences with at least 70% identity in the three complementarity determining regions (CDRs) to the sequences set forth in any one or more of SEQ ID NOS: 11524-11525, 11530-11531, 11549-11575, 11576-11592 and 23163-23173 or sequences with less than 3 substitutions in the three CDRs of the sequences set forth in any one or more of SEQ ID NOS: 11524-11525, 11530-11531, 11549-11575, 11576-11592 and 23163-23173 or sequences that bind to the same target antigens or the same epitopes on the target antigens as the sequences set forth in any one or more of SEQ ID NOS: 11524-11525, 11530-11531, 11549-11575, 11576-11592 and 23163-23173 and which encodes a polypeptide that binds to its antigen; (v) a non-immunoglobulin scaffold encoded by a polynucleotide of any one of SEQ ID NOS: 11662-11673 or sequences with at least 70% identity to sequences set forth in any one or more of SEQ ID NOS: 11662-11673 or sequences that bind to the same target antigens or the same epitopes on the target antigens as the sequences set forth in any one or more of SEQ ID NOS: 11662-11673; (vi) the ligand binding domain of a receptor comprising a sequence as set forth in any one of SEQ ID NO:11674-11691 or sequences with at least 70% identity thereto and which encodes a polypeptide that binds to its cognate; (vii) the receptor binding domain of a ligand comprising a sequence as set forth in any one of SEQ ID NO 11692 to 11702, 22391-22392, 22402-22404 or sequences with at least 70% identity thereto and which encodes a polypeptide that binds to its cognate; (viii) a single vH domain comprising a sequence as set forth in any one of SEQ ID NO: SEQ ID NOS: 11519-11523, 11526-11529, 11532-11548, 11644-11645 and 23174 or sequences with at least 70% identity to sequences set forth in any one or more of SEQ ID NOS: 11519-11523, 11526-11529, 11532-11548, 11644-11645 and 23174 and or sequences with 70% identity in the three complementarity determining regions (CDRs) to the sequences set forth in any one or more of SEQ ID NOS: 11519-11523, 11526-11529, 11532-11548, 11644-11645 and 23174 or sequences with less than 3 substitutions in the three CDRs of the sequences set forth in any one or more of SEQ ID NOS: 11519-11523, 11526-11529, 11532-11548, 11644-11645 and 23174 or sequences that bind to the same target antigens or the same epitopes on the target antigens as the sequences set forth in any one or more of SEQ ID NOS: 11519-11523, 11526-11529, 11532-11548, 11644-11645 and 23174 and which encodes a polypeptide that binds to its antigen; (ix) an adaptor binding domain comprising a sequence as set forth in any one of SEQ ID NO 11704 to 11712, or 22383 or sequences with at least 70% identity thereto and which encodes a polypeptide that binds to its adaptor; (x) an autoantigen comprising a sequence as set forth in any one of SEQ ID NO 11687, 22406-22407 to 11712, or 22383 or sequences with at least 70% identity thereto and which encodes a polypeptide that binds to its autoantibody or autoantibody producing cells; (xi) a TCR variable region (Va, Vb, Vg or Vd) comprising a sequence as set forth in any of SEQ ID NOs: 22396-2239 and 11653-11660 or sequences with at least 70% identity thereto or sequences with at least 70% identity in the three complementarity determining regions (CDRs) to the sequences set forth in any one or more of SEQ ID NOS: 22396-22397 and 11653-11660 or sequences with less than 3 substitutions in the three CDRs of the sequences set forth in any one or more of SEQ ID NOS: 22396-22397 and 11653-11660 or sequences that bind to the same target antigens or the same epitopes on the target antigens as the sequences set forth in any one or more of SEQ ID NOS: 22396-22397 and 11653-11660 and which encodes a polypeptide that binds to its antigen; and (xii) a single variable TCR domain (svd-TCR) comprising a sequence as set forth in any of SEQ ID NOs: 22399-22400 or sequences with at least 70% identity thereto or sequences with 70-99% identity in the three complementarity determining regions (CDRs) to the sequences set forth in any one or more of SEQ ID NOS: 22399-22400 or sequences with less than 3 substitutions in the three CDRs of the sequences set forth in any one or more of SEQ ID NOS: 22399-22400 or sequences that bind to the same target antigens or the same epitopes on the target antigens as the sequences set forth in any one or more of SEQ ID NOS: 22399-22400 and which encodes a polypeptide that binds to its antigen.

In one embodiment, the disclosure provides at least one recombinant polynucleotide encoding at least one SAR polypeptide wherein at least one of the target antigens is expressed on blood lineage cells while at least one of the target antigens is expressed on solid tumor cells.

In one embodiment, the disclosure provides a recombinant expression system comprising the recombinant polynucleotide of of the disclosure which is co-expressed with a therapeutic control, wherein the therapeutic control is selected from the group consisting of a truncated epidermal growth factor receptor (tEGFR), truncated epidermal growth factor receptor viii (tEGFRviii), truncated CD30 (tCD30), truncated BCMA (tBCMA), truncated CD19 (tCD19), CD34, thymidine kinase, cytosine deaminase, nitroreductase, xanthine-guanine phosphoribosyl transferase, human caspase 8, human caspase 9, inducible caspase 9 (icaspase9), purine nucleoside phosphorylase, linamarase/linamarin/glucose oxidase, deoxyribonucleoside kinase, horseradish peroxidase (HRP)/indole-3-acetic (IAA), Gamma-glutamylcysteine synthetase, CD20/alphaCD20, CD34/thymidine kinase chimera, dox-dependent caspase-2, mutant thymidine kinase (HSV-TKSR39), AP1903/Fas system, a chimeric cytokine receptor (CCR), 41BBL, CD40L, K13, MC159, cFLIP-L/MRITα, cFLIP-p22, HTLV1 Tax, HTLV2 Tax, HTLV2 Tax-RS mutant, FKBPx2-K13, FKBPx2-HTLV2-Tax, FKBPx2-HTLV2-Tax-RS, IL6R-304-vHH-Alb8-vHH, IL12f, PD1-4H1 scFV, PD1-5C4 scFV, PD1-4H1-Alb8-vHH, PD1-5C4-Alb8-vHH, CTLA4-Ipilimumab-scFv, CTLA4-Ipilimumab-Alb8-vHH, IL6-19A-scFV, IL6-19A-scFV-Alb8-vHH, sHVEM, sHVEM-Alb8-vHH, hTERT, Fx06, CD3z, CD3z-GGGS-41BB, CD3-BBz, CD3-CD28z, CD3-CD28-Lck fusion protein, shRNA targeting Brd4, chimeric antigen receptor (CAR), hTERT, heparinase, a CAR, an inhibitory CAR and combination thereof and combinations thereof.

In one embodiment, the disclosure provides at least one vector comprising the recombinant polynucleotide comprising a SAR, wherein the vector is selected from the group consisting of a DNA vector, an RNA vector, a plasmid, a lentivirus vector, an adenoviral vector, a retrovirus vector, a baculovirus vector, a sleeping beauty transposon vector, and a piggybac transposon vector.

In one embodiment, the disclosure provides at least one SAR polypeptide encoded by the at least one recombinant polynucleotide comprising a SAR. In one embodiment, the disclosure provides a recombinant cell or a cell population that expresses the at least one recombinant polynucleotide encoding a SAR of the disclosure. In another embodiment, the disclosure provides a cell or cell population expressing a SAR wherein the cell is an immune effector cell or a stem cell that can give rise to an immune effector cell, or an induced pluripotent stem cell (iPSC) that can give rise to an immune effector cell. The cell or cell population of can be an autologous cell or an allogeneic cell.

In one embodiment, the disclosure provides a cell or cell population with impaired or abolished functional expression of an endogenous TCR and with functional expression of a SAR polypeptide. The disclosure also provides a cell or cell population where the SAR mRNA and the translated polypeptide is expressed from an expression cassette placed in the locus of an endogenous T cell gene. The disclosure also provides a cell population where the at least one polynucleotide is under the control of the promoter and/or regulatory elements for an endogenous T cell gene. The disclosure provides a cell or cell population where the endogenous T cell gene locus is TRAC locus, TRBC locus, TRGC locus and/or TRDC locus. In one embodiment, the disclosure provides a cell or cell population wherein the placement of the SAR polynucleotide disrupts or abolishes the endogenous expression of a TCR comprising an endogenous TCRα chain and/or an endogenous TCRβ chain, or an endogenous TCRγ chain and/or endogenous TCRδ chain in the T cell. In one embodiment, the disclosure provides a cell or cell population wherein the disruption or abolished expression of an endogenous TCR results in enhanced expression and/or activity of the non-naturally occurring immune receptor as compared to its expression and/or activity in T cells with wild-type endogenous TCR and wherein the SAR has the backbone of one or more of a SIR, a cTCR and/or an Ab-TCR.

In one embodiment, the disclosure provides a cell or cell population wherein the SAR polypeptide has the backbone of a TFP and the disruption or abolished expression of an endogenous TCR results in impaired expression and/or activity of the SAR as compared to its expression and/or activity in T cells with wild-type endogenous TCR.

In one embodiment, the disclosure provides a cell or cell population wherein the SAR polypeptide has the backbone of a TFP and the cell or cell population further expresses a sequence encoding a TCR constant chain, wherein the TCR constant chain is i) a TCR alpha constant chain or fragment thereof, or ii) a TCR beta constant chain or a fragment thereof, or iii) a TCR gamma constant chain or fragment thereof, or iv) a TCR delta constant chain or a fragment thereof, or a combination of i) and ii) or iii) and iv), and wherein the expression of a sequence encoding a TCR constant chain restores the expression and/or activity of the SAR polypeptide.

In one embodiment, the disclosure provides a method of making a SAR-expressing immune effector cell, comprising introducing at least one vector comprising a SAR or at least one recombinant polynucleotide comprising a SAR into an immune effector cell or a hematopoietic stem cell or progenitor cell that can give rise to an immune effector cell, under conditions such that the SAR polypeptide is expressed.

In one embodiment, the disclosure provides a method of expanding the SAR-expressing immune effector cells in a gas permeable flask under normoxic or hypoxic conditions.

In one embodiment, the disclosure provides a method where the SAR-expressing immune effector cells are expanded in the presence of a SMAC mimetic or a NIK agonist compound.

In one embodiment, the disclosure provides a method of providing anti-disease immunity in a subject comprising administering to the subject an effective amount of the immune effector cell or a stem cell that can give rise to an immune effector cell, wherein the cell is an autologous T cell or an allogeneic T cell, or an autologous NKT cell or an allogeneic NKT cell or an autologous or an allogeneic hematopoietic stem cell or an autologous or an allogeneic iPSC that can give rise to an immune effector cell.

In one embodiment, the disclosure provides a method of treating or preventing a disease associated with expression of a disease-associated antigen in a subject, comprising administering to the subject an effective amount of an immune effector cell comprising a synthetic antigen receptor (SAR) polypeptide, wherein the SAR polypeptide bind to one or more disease-associated antigens selected from antigens listed in **Table B** and wherein the disease associated with expression of the disease associated antigen is selected from the group consisting of a proliferative disease, a precancerous condition, a cancer, and a non-cancer related indication associated with expression of the disease-associated antigen.

In one embodiment, the disclosure provides a recombinant polynucleotide encoding a SAR polypeptide, the polynucleotide comprising a sequence selected from the group consisting of SEQ ID NO: 1849-10720, 21609-21625, 21626-21659, 21662-21792. 21808, 21813-21840, 21843-21879, 21882- 21892, 21894-21899, 21901-21947, 21949-21963, 21967-21972, 21985-22085, 22087-22099, 22104-22207, 22209-22250, 22252-22254, 22256-22262, 22264-22273, 22275-22284, 22286-22296 and 22307-22312 or a sequence with at least 75% identity to the nucleotide sequence of the above. In one embodiment, the disclosure provides an amino acid sequence encoding a synthetic antigen receptor (SAR) polypeptide selected from the group consisting of SEQ ID NO: 12539-21410, 22445-22460, 22462-22495, 22498-22614, 22624, 22626-22655, 22658-22685, 22687-22694, 22697-22707, 22709-22714, 22716-22762, 22764-22778, 22782-22787, 22800-22900, 22902-22914, 22919-23022, 23024-23065, 23067-23069, 23071-23077, 23090-23099, 23101-23111 and 23122-23127 or a sequence with at least 75% identity to an amino acid sequences of the forgoing.

In one embodiment, the disclosure provides a composition comprising at least one SAR polynucleotide, a SAR polypeptide molecule, a SAR vector or a SAR-expressing cell and a pharmaceutically acceptable excipient. In one embodiment, the disclosure provides a kit comprising least one SAR polynucleotide, a SAR polypeptide molecule, a SAR vector or a SAR-expressing cell.

### BRIEF DESCRIPTION OF THE FIGURES

The disclosure is further described in the following non-limiting figures.
**Figure 1** shows a schematic representation of different double chain uni-specific, bispecific and multispecific SARs.
**Figure 2** shows a schematic representation of different double chain uni-specific, bispecific and multispecific SARs comprising different forms of AABD (e.g., vHH, SVH, aVH, affibody, Centyrin etc.).

### DETAILED DESCRIPTION

The invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Unless stated otherwise, or implicit from context, the following terms and phrases include the meanings provided below. Unless explicitly stated otherwise, or apparent from context, the terms and phrases below do not exclude the meaning that the term or phrase has acquired in the art to which it pertains. The definitions are provided to aid in describing particular embodiments, and are not intended to limit the claimed invention, because the scope of the invention is limited only by the claims.

Unless otherwise defined, all technical and scientific terms used herein have the same meaning as commonly understood by one of ordinary skill in the art to which this invention belongs.

As used herein the term "comprising" or "comprises" is used in reference to compositions, methods, and respective component(s) thereof, that are useful to an embodiment, yet open to the inclusion of unspecified elements, whether useful or not. It will be understood by those within the art that, in general, terms used herein are generally intended as "open" terms *(e.g.,* the term "including" should be interpreted as "including but not limited to," the term "having" should be interpreted as "having at least," the term "includes" should be interpreted as "includes but is not limited to," *etc.).*

Generally, nomenclatures used in connection with, and techniques of, cell and tissue culture, pathology, oncology, molecular biology, immunology, microbiology, genetics and protein and nucleic acid chemistry and hybridization described herein are those well-known and commonly used in the art. The methods and techniques of the present disclosure are generally performed according to conventional methods well-known in the art and as described in various general and more specific references that are cited and discussed throughout the present specification unless otherwise indicated. See, *e.g.,* Sambrook et al., Molecular Cloning: A Laboratory Manual (4th ed., Cold Spring Harbor Laboratory Press, Cold Spring Harbor, N.Y. (2013)). Enzymatic reactions and purification techniques are performed according to manufacturer's specifications, as commonly accomplished in the art or as described herein. The nomenclatures used in connection with, and the laboratory procedures and techniques of, immunology, molecular biology, analytical chemistry, synthetic organic chemistry, and medicinal and pharmaceutical chemistry described herein are those well-known and commonly used in the art. Standard techniques are used for chemical syntheses, chemical analyses, pharmaceutical preparation, formulation, and delivery, and treatment of patients.

The term "about" when referring to a measurable value such as an amount, a temporal duration, and the like, is meant to encompass variations of ±20% or in some instances ±10%, or in some instances ±5%, or in some instances ±1%, or in some instances ±0.1% from the specified value, as such variations are appropriate to perform the disclosed methods or describe the compositions herein. Moreover, any value or range *(e.g.,* less than 20 or similar terminology) explicitly includes any integer between such values or up to the value. Thus, for example, "one to five mutations" explicitly includes 1, 2, 3, 4, and/or 5 mutations. The term "at least" refers to a minimum value in a range. Where "at least" precedes a percentage, the incremental values contemplate in the range are 0.1% up to, for example, 100% (in the case of identity/homology).

An "antigen binding domain" or "antigen binding module" or "antigen binding segment" or "antigen specific domain" (ASD) refers to a polypeptide or peptide that due to its primary, secondary or tertiary sequence, post-translational modifications and/or charge binds to an antigen with a high degree of specificity. The antigen binding domain may be derived from different sources, for example, an antibody (full length heavy chain, Fab fragments, single chain Fv (scFv) fragments, divalent single chain antibodies or diabodies), a non-immunoglobulin binding protein, a ligand or a receptor. There are, however, numerous alternatives, such as linked cytokines (which leads to recognition of cells bearing the cytokine receptor), affibodies, ligand binding domains from naturally occurring receptors, soluble protein/peptide ligand for a receptor (for example on a tumor cell), peptides, and vaccines to prompt an immune response, which may each be used in various embodiments of the disclosure. In some embodiments, almost any molecule that binds a given cognate or antigen with high affinity can be used as an ASD, as will be appreciated by those of skill in the art. In some embodiments, the antigen binding domain comprises T cell receptors (TCRs) or portions thereof. In exemplary embodiments, the target antigens and SEQ ID Nos of various antigen binding domains are set forth herein in **Tables 3-7.** In exemplary embodiments, the target antigen and SEQ ID NOs of vL, vH, scFVs, and their CDR regions are set forth herein in **Tables 6A-C** of patent application PCT/US18/53247 and in **Tables 3-4** of patent application PCT/US19/035096, which are incorporated in their entirety by reference herein. The vL, vH and scFv that can be used in the construction of the SARs of the disclosure may be "fully human", "humanized", "chimeric" or non-human in origin. Exemplary fully human vL, vH and scFv are represented by SEQ ID NO: 10854, 11096 and 11338, respectively. Exemplary humanized vL, vH and scFv are represented by SEQ ID NO: 10839, 11082 and 11323, respectively.

The term "autonomous antigen binding domain" or "AABD" as used herein refers to an antigen binding domain that can bind to an antigen autonomously, *i.e.,* in the absence of another antigen binding domain. An exemplary AABD is a single vH domain or an autonomous vH domain (aVH), typically a single human vH domain (SVH) that can bind an antigen in the absence of a vL domain. An exemplary AABD is a single vL domain or an autonomous vL domain, typically a single human vL domain (SVL) that can bind an antigen in the absence of a vH domain. Another exemplary AABD is a fully human vH domain (FHVH). AABD also refers to other antigen binding domains that can bind an antigen autonomously. In an embodiment, the AABD is a non-scFv antigen binding domain. Exemplary non-scFV based autonomous antigen binding domains include but are not limited to a vHH domain, a humanized vHH domain, a svd-TCR, and non-immunoglobulin antigen binding scaffold such as a DARPIN, an affibody, a ZIP domain *(e.g.,* RZIP, EZIP, E4, R4 *etc.),* an affilin, an adnectin, an affitin, an obodies, a repebody, a fynomer, an alphabody, an avimer, an atrimer, a centyrin, a pronectin, an anticalin, a kunitz domain, an Armadillo repeat protein or a fragment thereof; the ligand binding domain of a receptor *(e.g.,* CD16-V158A, NKG2D) or a fragment thereof, the receptor binding domain of a ligand *(e.g.,* APRIL, Thrombopoietin *etc.)* or a fragment thereof, an adaptor *(e.g.,* RZIP, EZIP, E4, K4, NKG2D-YA, NKG2D-AF *etc.)* or a fragment thereof, an adatptor binding protein *(e.g.* ULBP2R, ULBP2-S3 *etc.)* or a fragment thereof, an epitope or a tag *(e.g.,* Streptag, FLAG tag *etc.),* an autoantigen or a fragment thereof and the like.

The disclosure described the use of AABD, such as human VH domains, typically multiple human VH domains, as building blocks to make unispecific, bispecific and multispecific SARs. In an embodiment, the disclosure describes the use of AABD, such as human VH domains, typically multiple human VH domains, as building blocks to make unispecific, bispecific and multispecific CAR, SIR, cTCR, Ab-TCR, AABD-TCR, TFPs and recombinant TCRs.

The term "ABR" or "Antigen Binding Receptor" as described herein refers to any receptor that has an antigen binding domain. The antigen binding domain of an ABR may comprise of a scFv, a vL, vH, VHH, antibody, antibody fragment (e.g., Fab), antibody like moiety, Vα, Vβ, cytokine, receptor *etc.* In one embodiment, an ABR has a transmembrane or membrane anchoring domain that allows it to be expressed on the cell surface. Exemplary ABR include a 1^{st} generation CAR, a 2^{nd} generation CAR, a TFP, a TRI-TAC or TAC *etc.* Antigen masking receptors, as described herein, are also examples of ABR.

The term "Ab-TCR" or "AbTCR" refers to a next generation CAR platform as described in WO 2017/070608 A1 which is incorporated herein by reference. In an embodiment, an Ab-TCR comprises an antibody moiety that specifically binds to a target antigen fused to a TCR module capable of recruiting at least one TCR signaling module. Exemplary TCR modules that can be used in the construction of Ab-TCR are provided in SEQ ID NO:6009-6014 **(Table 6)** and in WO 2017/070608 A1 which is incorporated herein by reference. This disclosure provides bispecific, biparatopic and mutlispecific SARs with the backbone of Ab-TCR comprising one or more AABDs. The AABD domains of the SARs of the disclosure with the backbone of Ab-TCR can be fully human, humanized or non-human. In an embodiment, the disclosure provides Ab-TCR comprising one or more fully human vH domains. In an embodiment, the disclosure provides Ab-TCR comprising one or more fully human vL domains.

The term "AABD-TCR" refers to a novel double chain SAR platform described in this disclosure in which one or more AABDs are operably linked in frame to a TCR module capable without the presence of an intervening vL, vH, Va, Vb, Vg and/or Vd chain. In a preferred embodiment of the disclosure, an AABD-TCR comprises an Ig like linker domain that is present between the AABD and the TCR modules. The TCR module of the AABD-TCR is capable of recruiting at least one TCR signalign module. Exemplary AABD-TCRs are represented by SEQ ID NO (DNA): 6521 and 6530. Additional AABD-TCRs are presented in **Tables 33** and **34.** The AABD domains of AABD-TCR can be fully human, humanized or non-human. In an embodiment, the disclosure provides AABD-TCR comprising one or more fully human vH domains. In an embodiment, the disclosure provides AABD-TCR comprising one or more fully human vL domains.

The term "accessory module" refers to any one or more of PDL1, PDL2, CD80, CD86, crmA, p35, hNEMO-K277A (or NEMO-K277A), hNEMO-K277A-delta-V249-K555, mNEMO-K270A, K13-opt, IKK2-S177E-S181E (or IKK2-SS/EE), IKK1-S176E-S180E (or IKK1-SS/EE), MyD88-L265P, TCL-1a, MTCP-1, CMV-141, 41BBL, CD40L, vFLIP-K13, MC159, cFLIP-L/MRITα, cFLIP-p22, HTLV1 Tax, HTLV2 Tax, HTLV2 Tax-RS mutant, FKBPx2-K13, FKBPx2-HTLV2-Tax, FKBPx2-HTLV2-Tax-RS, IL6R-304-vHH-Alb8-vHH, IL12f, PD1-4H1 scFV, PD1-5C4 scFV, PD1-4H1-Alb8-vHH, PD1-5C4-Alb8-vHH, CTLA4-Ipilimumab-scFv, CTLA4-Ipilimumab-Alb8-vHH, IL6-19A-scFV, IL6-19A-scFV-Alb8-vHH, sHVEM, sHVEM-Alb8-vHH, hTERT, Fx06, shRNA targeting Brd4, IgSP-[hTRAC-opt2], IgSP-[hTRBC-opt2] and combination thereof that is expressed in an immune cell *(e.g.,* T cell, *e.g.,* CAR-T cell or TCR-T cell) to decrease, regulate or modify the activity of the immune cell. In some embodiments, the accessory module is co-expressed with an immune receptor such as a CAR or a TCR to increase, decrease, regulate or modify the expression or activity of a CAR or a TCR or a CAR-expressing or a TCR-expressing cell. The accessory module can be co-expressed with a CAR or a TCR using a single vector or using two or more different vectors. In a further embodiment, the accessory module comprises an FKBP (FK506 binding protein)-fusion protein, such as FKBPx2-NEMO, whose activity can be controlled by the administration of a dimerizer molecule. In some embodiments, the accessory module is expressed in an antigen presenting cell, *e.g.,* a dendritic cell.

The term "affibody" as used here refers to antibody mimetic molecules that are composed of alpha helices and lack disulfide bonds. An exemplary Affibody targeting Her3 is represented by SEQ ID NO: 11664. Other exemplary affibodies are known in the art.

As used herein "affinity" is meant to describe a measure of binding strength. Affinity, in some instances, depends on the closeness of stereochemical fit between a binding agent and its target (e.g., between an antibody and antigen including epitopes specific for the binding domain), on the size of the area of contact between them, and on the distribution of charged and hydrophobic groups. Affinity generally refers to the "ability" of the binding agent to bind its target. There are numerous ways used in the art to measure "affinity". For example, methods for calculating the affinity of an antibody for an antigen are known in the art, including use of binding experiments to calculate affinity. Binding affinity may be determined using various techniques known in the art, for example, surface plasmon resonance, bio-layer interferometry, dual polarization interferometry, static light scattering, dynamic light scattering, isothermal titration calorimetry, ELISA, analytical ultracentrifugation, and flow cytometry. An exemplary method for determining binding affinity employs surface plasmon resonance. Surface plasmon resonance is an optical phenomenon that allows for the analysis of real-time biospecific interactions by detection of alterations in protein concentrations within a biosensor matrix, for example using the BIAcore system (Pharmacia Biosensor AB, Uppsala, Sweden and Piscataway, N.J.). As used herein, the term "specific binding" means the contact between an antibody and an antigen with a binding affinity of at least 10⁻⁶ M. In certain aspects, antibodies bind with affinities of at least about 10⁻⁷M, and typically 10⁻⁸ M, 10⁻⁹ M, 1⁻¹⁰ M, 10⁻¹¹ M, or 10⁻¹² M.

The term "antibody," as used herein, refers to a protein, or polypeptide sequence derived from an immunoglobulin molecule which specifically binds with an antigen. Antibodies can be monoclonal, or polyclonal, multiple or single chain, or intact immunoglobulins, and may be derived from natural sources or from recombinant sources. Antibodies can be tetramers of immunoglobulin molecules. The antibody may be "fully human", 'humanized', 'chimeric' or non-human.

As used herein, "humanized" antibody refers to forms of non-human ( e.g . murine) antibodies that are chimeric immunoglobulins, immunoglobulin chains, or fragments thereof (such as Fv, Fab, Fab', F(ab')2 or other antigen binding subsequences of antibodies) that contain minimal sequence derived from non-human immunoglobulin.

As used herein, "human antibody" or "fully human antibody" means an antibody or an antibody fragment having an amino acid sequence corresponding to that of an antibody produced by a human and/or which has been made using any of the techniques for making human antibodies known to those skilled in the art or disclosed herein. This definition of a human antibody includes antibodies comprising at least one human heavy chain polypeptide or at least one human light chain polypeptide.

The term "chimeric antibody" is intended to refer to antibodies in which the variable region sequences are derived from one species and the constant region sequences are derived from another species.

The term "antibody fragment" refers to at least one portion of an antibody, that retains the ability to specifically interact with (e.g., by binding, steric hindrance, stabilizing/destabilizing, spatial distribution) an epitope of an antigen. Examples of antibody fragments include, but are not limited to, Fab, Fab', Fv fragments, scFv antibody fragments, disulfide-linked Fvs (sdFv), a Fd fragment consisting of the VH and CHl domains, linear antibodies, single domain antibodies (sdAb) such as either vL or vH, camelid vHH domains, multi-specific antibodies formed from antibody fragments such as a bivalent fragment comprising two Fab fragments linked by a disulfide bridge at the hinge region, and an isolated CDR or other epitope binding fragments of an antibody. An antigen binding fragment can also be incorporated into single domain antibodies, maxibodies, minibodies, nanobodies, intrabodies, diabodies, triabodies, tetrabodies, v-NAR and bis-scFv (see, *e.g.,* Hollinger and Hudson, Nature Biotechnology 23:1126-1136, 2005). Antigen binding fragments can also be grafted into scaffolds based on polypeptides such as a fibronectin type III (Fn3) (see U.S. Patent No.: 6,703,199, which describes fibronectin polypeptide minibodies).

The term "antibody heavy chain," refers to the larger of the two types of polypeptide chains present in antibody molecules in their naturally occurring conformations, and which normally determines the class to which the antibody belongs.

The term "antibody light chain," refers to the smaller of the two types of polypeptide chains present in antibody molecules in their naturally occurring conformations. Kappa (κ) and lambda (λ) light chains refer to the two major antibody light chain isotypes.

"Anticancer agent" refers to agents that inhibit aberrant cellular division and growth, inhibit migration of neoplastic cells, inhibit invasiveness or prevent cancer growth and metastasis. The term includes chemotherapeutic agents, biological agent (e.g., siRNA, viral vectors such as engineered MLV, adenoviruses, herpes virus that deliver cytotoxic genes), antibodies and the like.

The term "anticancer effect" refers to a biological effect which can be manifested by various means, including but not limited to, a decrease in tumor volume, a decrease in the number of cancer cells, a decrease in the number of metastases, an increase in life expectancy, decrease in cancer cell proliferation, decrease in cancer cell survival, or amelioration of various physiological symptoms associated with the cancerous condition. An "anticancer effect" can also be manifested by the ability of the SARs in prevention of the occurrence of cancer in the first place.

The term "antigen" or "Ag" refers to a molecule that provokes an immune response. This immune response may involve either antibody production, or the activation of specific immunologically-competent cells, or both. The skilled artisan will understand that any macromolecule, including virtually all proteins or peptides, can serve as an antigen. Furthermore, antigens can be derived from recombinant or genomic DNA. A skilled artisan will understand that any DNA, which comprises a nucleotide sequences or a partial nucleotide sequence encoding a protein that elicits an immune response therefore encodes an "antigen" as that term is used herein. Furthermore, one skilled in the art will understand that an antigen need not be encoded solely by a full-length nucleotide sequence of a gene. The disclosure includes, but is not limited to, the use of partial nucleotide sequences of more than one gene and that these nucleotide sequences are arranged in various combinations to encode polypeptides that elicit the desired immune response. Moreover, a skilled artisan will understand that an antigen need not be encoded by a "gene" at all. It is readily apparent that an antigen can be generated synthesized or can be derived from a biological sample, or might be macromolecule besides a polypeptide. Such a biological sample can include, but is not limited to a tissue sample, a tumor sample, a cell or a fluid with other biological components.

The term "antigen presenting cell" or "APC" refers to an immune system cell such as an accessory cell *(e.g.,* a B-cell, a dendritic cell, and the like) that displays a foreign antigen complexed with major histocompatibility complexes (MHC's) on its surface. T-cells may recognize these complexes using their T-cell receptors (TCRs). APCs process antigens and present them to T-cells.

The term "anti-infection effect" refers to a biological effect that can be manifested by various means, including but not limited to, *e.g.,* decrease in the titer of the infectious agent, a decrease in colony counts of the infectious agent, amelioration of various physiological symptoms associated with the infectious condition. An "anti-infectious effect" can also be manifested by the ability of the peptides, polynucleotides, cells and antibodies in prevention of the occurrence of infection in the first place.

The term "antitumor effect" or "anti-cancer effect" refers to a biological effect which can be manifested by various means, including but not limited to, *e.g.,* a decrease in tumor volume, a decrease in the number of tumor cells, a decrease in tumor cell proliferation, inhibition of metastasis, or a decrease in tumor cell survival.

The term "Association constant (Ka)" is defined as the equilibrium constant of the association of a receptor and ligand.

"Autoantibody" refers to an antibody that is produced by a B-cell specific for an autoantigen.

The term "autoantigen" refers to an endogenous antigen that stimulates production of an autoimmune response, such as production of autoantibodies. Autoantigen also includes a self-antigen or antigen from a normal tissue that is the target of a cell mediated or an antibody-mediated immune response that may result in the development of an autoimmune disease. Examples of autoantigens include, but are not limited to, desmoglein 1, desmoglein 3, and fragments thereof.

"Avidity" refers to the strength of the interaction between a binding agent and its target *(e.g.,* the strength of the interaction between an antibody and its antigen target, a receptor and its cognate and the like). The avidity can be weak or strong. Methods for calculating the affinity of an antibody for an antigen are known in the art, including use of binding experiments to calculate affinity. Antibody activity in functional assays (e.g., flow cytometry assay or Malibu-Glo assay) is also reflective of antibody affinity.

As used herein, the term "backbone" or "architecture" refers to the configuration of the different components (*e.g*., antigen binding domains, hinge domains, transmembrane domains, signaling domains) that comprise different SAR *(e.g.,* CAR, SIR, cTCR, Ab-TCR, TFP *etc.)* and/or any accessory module which is generally optional. In exemplary embodiment, a SAR can have the backbone of a 1^{st} generation CAR, a 2^{nd} generation CAR, a double chain SIR, a one and half chain SIR, a double chain cTCR, a one and a half chain cTCR, a zSIR, an Ab-TCR, an AABD-TCR, a εTFP, a γTFP, a δTFP, an αβTFP, an γδTFP, a TCR *etc.* A SAR with a particular backbone may have further subtypes, each representing a different backbone. Thus, a SAR with the backbone of a double chain SIR may have further subtypes based on the number (unispecific, bispecific, multispecific *etc.)* and nature *(e.g.,* vL, vH, scFv, vHH, FHVH, DARPIN *etc.)* of its antigen binding domains. Each of the above subtypes can be considered a separate backbone. In an exemplary embodiment, a SAR on any of the backbone may further co-express an accessory module *(e.g.,* PAC, K13-opt, MC159, icaspase 9 *etc.).* Thus, a SAR with the backbone of a double chain SIR may be coexpressed with K13-opt. In one embodiment, the SAR and the accessory module are encoded by a single nucleic acid molecule. In another embodiment, the SAR is encoded by the first nucleic acid molecule and the accessory module is encoded by a second nucleic acid molecule. In some embodiments, the accessory module is encoded by more than one nucleic acid molecule, depending on the number of components in the accessory modules. The two or more components of the SAR may be separated by a cleavable linker such as a 2A ribosomal skip sequence *(e.g.,* P2A, T2A, F2A *etc.).*

**Table A1-1:** SAR architectures/backbones. First generation conventional CARs (Conventional CAR I) have an activation domain (AD) domain (*e.g*., CD3z) and no costimulatory domain (CD). The TCR fusion proteins (TFP) are another example of conventional CAR 1. Second generation conventional CARs (Conventional CAR 2 or CAR II) have one costimulatory domain (*e.g*., 4-1BB or CD28) and an intracellular activation domain (AD) domain (*e.g*., CD3z). Third generation conventional CARs (Conventional CAR 3 or CAR III) have two costimulatory domains (*e.g*., 4-1BB and CD28) and an intracellular activation domain (e.g., CD3z). Ab-TCRs are dual chain receptors incorporating a vL-IgCL-linker-TCR domain (TCRD and a vH-Ig-CH1-linker-TCR domain (TCRD) and have been described in PCT/US2016/058305. AABD-TCRs are dual chain receptors described in this disclosure and generally incorporate an AABD-IgCL-TCRD module and an IgCH1-TCRD module or an IgCL-TCRD module and an AABD-IgCH1-TCRD module, where TCRD are complementary in nature *(i.e.,* TCRα and TCRβ or TCRγ and TCRδ). Bispecific and multispecific AABD-TCRs are also described in this disclosure and generally incorporate an (AABD)n-IgCL-TCRD module and an (AABD)n-IgCH1-TCRD module, where TCRD are complementary in nature *(i.e.,* TCRα and TCRβ or TCRγ and TCRδ). cTCRs are single chain, one-and-half, or double chain receptors consisting of antigen binding domain derived from a vL and vH fragment that are fused to one or more TCR constant chain (TCR-C) comprising their wild-type nucleic acid and amino acid sequences and result in activation of T cell signaling. Different configurations of cTCR are described in PCT/US2017/064379, WO 2018/102795 A1 and Gross et al, Proc. Natl. Acad. Sci USA (1989) 86:10024-26. Synthetic immune receptors are next generation CARs and are described in PCT/US2017/064379 or WO 2018/102795 A1. zSIRs are double chain receptors comprising two CD3z chains or fragments thereof with optional linkers and are described in PCT/US2019/035096.

| **Table A1-1 Exemplary Unispecific SAR Architectures (Backbones)** | | | | | | | |
|---|---|---|---|---|---|---|---|
| 1 | CAR 1 or CAR I **(including TFP)** | ASD | HR | TM | AD | | |
| 2 | CAR 2 (CAR II) | ASD | HR | TM | CD | AD | |
| 3 | CAR 3 (CAR III) | ASD | HR | TM | CD-I | CSD-II | AD |
| 4 | Ab-TCR | vL-Ig-CL | TCRD(1) | 2A | vH-Ig-CH1 | TCRD (II) | |
| 5 | Double Chain cTCR/SIR | vL | TCR-C(1) | 2A | vH | TCR-C (II) | |
| 6 | Double Chain zSIR | vL-linker | CD3z | 2A | vH-linker | CD3z | |
| 7 | One & Half Chain cTCR/SIR | | TCR-C(1) | 2A | ASD | TCR-C (II) | |
| 8 | AABD-TCR | AABD-Ig-CL | TCRD(1) | 2A | Ig-CH | TCRD(1) | |

TABLES A1-1 to A1-9 provide exemplary architectures of unispecific, bispecific and multispecific SARs of this disclosure. The abbreviations used are: SP (signal peptide); AADB (autonomous antigen binding domain); L (optional linker); LL (Long linker), (AABD-L)n (n copies of AABD with optional linker where n = 0, 1, 2, 3, 4 or more), AABD1-4 (different AABD targeting one or more antigens), V1 (vL, vH, Va, Vb, Vg or Vd chains), Ig (Ig linker), ConP (connecting peptide), TM (transmembrane domain), IC (intracellular domain), Ca (Constant chain of TCRα), Cb (constant chain of TCRβ), Cg (constant chain of TCRγ), Cd (constant chain of TCRδ), scFv (single chain fragment variable), scTFv (single chain fragment comprising two variable fragments of a TCR, *e.g.,* Va and Vb), dCa/dCb/dCg/dCd (N-terminallly deleted constant chain of TCRα, β, γ or δ lacking their Ig linker domain), TCR-ConP (connecting peptide of TCRα, β, γ or δ constant chain), Ca-ConP (connecting peptide of TCRα constant chain), IgCL (Ig linker from immunoglobulin light chain), IgCH1 (Ig liker from immunoglobulin heavy chain ), CD3εγδ ECD (extracellular domain of CD3ε, γ or δ chains), CD (costimulatory domain), 4-1BB (costimulatory domain of 4-1BB), CD28 (costimulatory domain of CD28), CD3z (activation domain of CD3z).

| **TABLE A1-2 EXEMPLARY BISPECIFIC AND MULTISPECIFIC SARS ARCHITECTURES** | | | | | | | | | | | | | | | | |
|---|---|---|---|---|---|---|---|---|---|---|---|---|---|---|---|---|
| **SAR Class** | | | | | | | | | | | | | | | | |
| 1 | SP | L | (AABD -L)n | V 1 | L | Ig | ConP | TM | IC | | | | | | | |
| 2 | SP | L | (AABD -L)n | V 1 | L | L L | ConP | TM | IC | | | | | | | |
| 3 | SP | L | AABD 1 | L | V1 | L | Ig | Con P | TM | IC | | | | | | |
| 4 | SP | L | AABD 1 | L | AABD 2 | L | V1 | L | Ig | Co nP | T M | IC | | | | |
| 5 | SP | L | AABD 1 | L | AABD 2 | L | AAB D3 | L | AA BD4 | L | V1 | L | I g | Con P | T M | I C |
| 6 | SP | L | AABD 1 | L | V1 | L | LL | Con P | TM | IC | | | | | | |
| 7 | SP | L | AABD 1 | L | AABD 2 | L | V1 | L | LL | Co nP | T M | IC | | | | |
| 8 | SP | L | AABD 1 | L | AABD 2 | L | AAB D3 | L | AA BD4 | L | V1 | L | L L | Con P | T M | I C |

**TABLE A1-3 EXEMPLARY BISPECIFIC AND MULTISPECIFIC SARS WITH THE BACKBONE OF DC-SIR OR TCR (INCLUDING HLA-INDEPENDENT TCR)**

| **SAR** | | | | | | |
|---|---|---|---|---|---|---|
| **Class** | | | | | | |
| 1 | SP | L | (AABD-L)n | vL | L | C-abgd (TCRα/β/γ/δ constant chain) |
| | SP | L | (AABD-L)n | vH | L | C-abgd (TCRα/β/γ/δ constant chain) |
| 2 | SP | L | (AABD-L)n | vL | L | Cb (TCRβ-constant chain) |
| | SP | L | (AABD-L)n | vH | L | Ca (TCRα-constant chain) |
| 3 | SP | L | (AABD-L)n | vL | L | Ca (TCRα-constant chain) |
| | SP | L | (AABD-L)n | vH | L | Cb (TCRβ-constant chain) |
| 4 | SP | L | (AABD-L)n | vL | L | Cg (TCRγ-constant chain) |
| | SP | L | (AABD-L)n | vH | L | Cd (TCRδ-constant chain) |
| 5 | SP | L | (AABD-L)n | vL | L | Cd (TCRδ-constant chain) |
| | SP | L | (AABD-L)n | vH | L | Cg (TCRγ-constant chain) |
| 6 | SP | L | (AABD-L)n | Va | L | Cb (TCRβ-constant chain) |
| | SP | L | (AABD-L)n | Vb | L | Ca (TCRα-constant chain) |
| 7 | SP | L | (AABD-L)n | Va | L | Ca (TCRα-constant chain) |
| | SP | L | (AABD-L)n | Vb | L | Cb (TCRβ-constant chain) |
| 8 | SP | L | (AABD-L)n | Vg | L | Cg (TCRγ-constant chain) |
| | SP | L | (AABD-L)n | Vd | L | Cd (TCRδ-constant chain) |
| 9 | SP | L | (AABD-L)n | Vd | L | Cd (TCRδ-constant chain) |
| | SP | L | (AABD-L)n | Vg | L | Cg (TCRγ-constant chain) |

**TABLE A1-4 EXEMPLARY BISPECIFIC AND MULTI-SPECIFIC SARS WITH A HYBRID BACKBONE**

| **SAR** | | | | | | |
|---|---|---|---|---|---|---|
| **Class** | | | | | | |
| 1 | SP | L | (AABD-L)n | vL/vH | L | C-abgd (TCRα/β/γ/δ constant chain) |
| | SP | L | (AABD-L)n | Va/b/c/d | L | C-abgd (TCRα/β/γ/δ constant chain) |
| 2 | SP | L | (AABD-L)n | vL | L | Cb (TCRβ-constant chain) |
| | SP | L | (AABD-L)n | Vb | L | Ca (TCRα-constant chain) |
| 3 | SP | L | (AABD-L)n | vH | L | Ca (TCRα-constant chain) |
| | SP | L | (AABD-L)n | Va | L | Cb (TCRβ-constant chain) |
| 4 | SP | L | (AABD-L)n | vH | L | Cg (TCRγ-constant chain) |
| | SP | L | (AABD-L)n | Vb | L | Cd (TCRδ-constant chain) |
| 5 | SP | L | (AABD-L)n | vL | L | Cd (TCRδ-constant chain) |
| | SP | L | (AABD-L)n | Vg | L | Cg (TCRγ-constant chain) |
| 6 | SP | L | (AABD-L)n | vL | L | Cb (TCRβ-constant chain) |
| | SP | L | (AABD-L)n | Vd | L | Ca (TCRα-constant chain) |
| 7 | SP | L | (AABD-L)n | vH | L | Ca (TCRα-constant chain) |
| | SP | L | (AABD-L)n | Vg | L | Cb (TCRβ-constant chain) |
| 8 | SP | L | (AABD-L)n | vH | L | Cg (TCRγ-constant chain) |
| | SP | L | (AABD-L)n | Vd | L | Cd (TCRδ-constant chain) |
| 9 | SP | L | (AABD-L)n | Va | L | Cd (TCRδ-constant chain) |
| | SP | L | (AABD-L)n | Vg | L | Cg (TCRγ-constant chain) |

| **TABLE A1-5: EXEMPLARY BISPECIFIC AND MULTISPECIFIC SARS WITH BACKBONE OF ONE AND HALF CHAIN-SIR** | | | | | | |
|---|---|---|---|---|---|---|
| **SAR Class** | | | | | | |
| 1 | SP | L | (AABD-L)n | scFv | L | C-abgd (TCRα/β/γ/δ constant chain) |
| | SP | L | (AABD-L)n | | L | C-abgd (TCRα/β/γ/δ constant chain) |
| 2 | SP | L | (AABD-L)n | scFv | L | Cb (TCRβ-constant chain) |
| | SP | L | (AABD-L)n | | L | Ca (TCRα-constant chain) |
| 3 | SP | L | (AABD-L)n | scFv | L | Ca (TCRα-constant chain) |
| | SP | L | (AABD-L)n | | L | Cb (TCRβ-constant chain) |
| 4 | SP | L | (AABD-L)n | scFv | L | Cg (TCRγ-constant chain) |
| | SP | L | (AABD-L)n | | L | Cd (TCRδ-constant chain) |
| 5 | SP | L | (AABD-L)n | scFv | L | Cd (TCRδ-constant chain) |
| | SP | L | (AABD-L)n | | L | Cg (TCRγ-constant chain) |

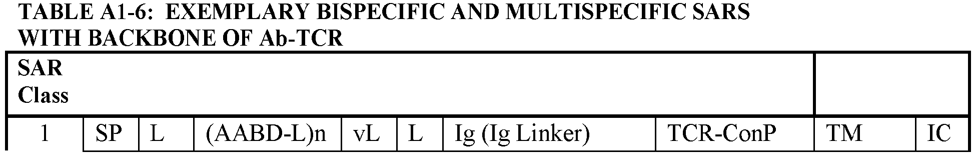

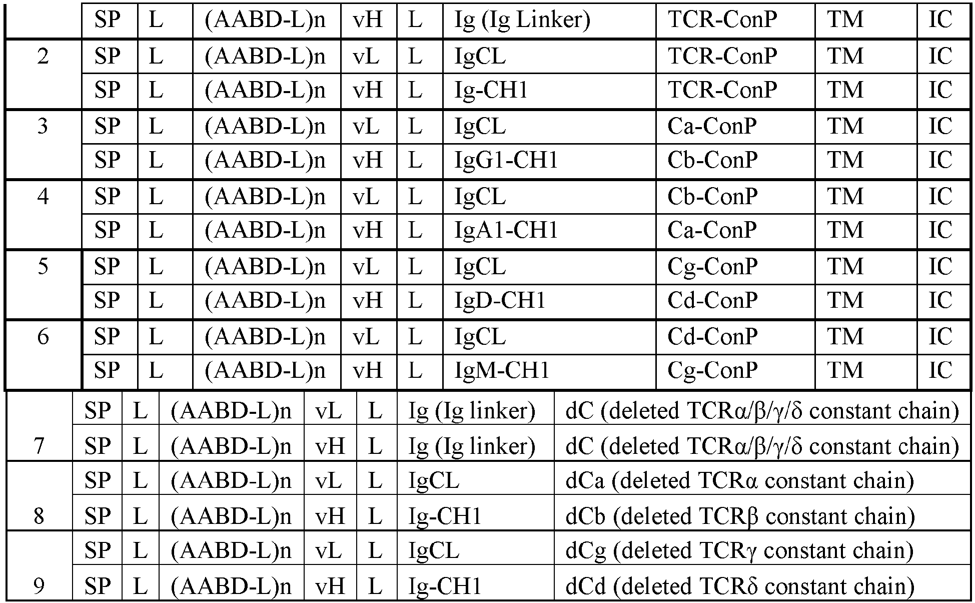

| **TABLE A1-7: EXEMPLARY BISPECIFIC AND MULTISPECIFIC SARS WITH BACKBONE OF AABD-TCR** | | | | | | | | | | | | | | |
|---|---|---|---|---|---|---|---|---|---|---|---|---|---|---|
| **SAR Class** | | | | | | | | | | | | | | |
| 1 | S P | L | (AABD-L)n | L | Ig | Con P | TM | IC | | | | | | |
| 2 | S P | L | AABD1 | L | Ig | Con P | TM | IC | | | | | | |
| 3 | S P | L | AABD1 | L | AABD 2 | L | Ig | Con P | TM | IC | | | | |
| 4 | S P | L | AABD1 | L | AABD 2 | L | AAB D3 | L | Ig | Con P | T M | IC | | |
| 5 | S P | L | AABD1 | L | AABD 2 | L | AAB D3 | L | AA BD4 | L | Ig | Con P | T M | I C |
| 6 | S P | L | (AABD-L)n | L | LL | Con P | TM | IC | | | | | | |
| 7 | S P | L | AABD1 | L | LL | Con P | TM | IC | | | | | | |
| 8 | S P | L | AABD1 | L | AABD 2 | L | LL | Con P | TM | IC | | | | |
| 9 | S P | L | AABD1 | L | AABD 2 | L | AAB D3 | L | LL | Con P | T M | IC | | |
| 10 | S P | L | AABD1 | L | AABD 2 | L | AAB D3 | L | AA BD4 | L | LL | Con P | T M | I C |

**TABLE A1-8: EXEMPLARY BISPECIFIC AND MULTISPECIFIC SARS WITH BACKBONE OF AABD-TCR**

| **SAR** | | | | | | | | |
|---|---|---|---|---|---|---|---|---|
| **Class** | | | | | | | | |
| 1 | SP | L | (AABD-L)n | L | Ig (Ig linker) | TCR-ConP | TM | IC |
| | SP | L | (AABD-L)n | L | Ig (Ig linker) | TCR-ConP | TM | IC |
| 2 | SP | L | (AABD-L)n | L | IgCL | TCR-ConP | TM | IC |
| | SP | L | (AABD-L)n | L | Ig-CH1 | TCR-ConP | TM | IC |
| 3 | SP | L | (AABD-L)n | L | IgCL | Ca-ConP | TM | IC |
| | SP | L | (AABD-L)n | L | IgG1-CH1 | Cb-ConP | TM | IC |
| 4 | SP | L | (AABD-L)n | L | IgCL | Cb-ConP | TM | IC |
| | SP | L | (AABD-L)n | L | IgA1-CH1 | Ca-ConP | TM | IC |
| 5 | SP | L | (AABD-L)n | L | IgCL | Cg-ConP | TM | IC |
| | SP | L | (AABD-L)n | L | IgD-CH1 | Cd-ConP | TM | IC |
| 6 | SP | L | (AABD-L)n | L | IgCL | Cd-ConP | TM | IC |
| | SP | L | (AABD-L)n | L | IgM-CH1 | Cg-ConP | TM | IC |
| 7 | SP | L | (AABD-L)n | L | Ig (Ig linker) | dC (deleted TCRα/β/γ/δ constant chain) | | |
| | SP | L | (AABD-L)n | L | Ig (Ig linker) | dC (deleted TCRα/β/γ/δ constant chain) | | |
| 8 | SP | L | (AABD-L)n | L | IgCL | dCa (deleted TCRα constant chain) | | |
| | SP | L | (AABD-L)n | L | Ig-CH1 | dCb (deleted TCRβ constant chain) | | |
| 9 | SP | L | (AABD-L)n | L | IgCL | dCg (deleted TCRγ constant chain) | | |
| | SP | L | (AABD-L)n | L | Ig-CH1 | dCd (deleted TCRδ constant chain) | | |

| | **TABLE A1-9 EXEMPLARY BISPECIFIC AND MULTISPECIFIC SARS WITH BACKBONE OF SINGLE CHAIN CAR, TFP and scTFv-CAR** | | | | | | | | | |
|---|---|---|---|---|---|---|---|---|---|---|
| **SAR Class** | | | | | | | | | | |
| 1 | SP | L | (AABD-L)n | scFv | L | LL | TM | AD | | |
| 2 | SP | L | (AABD-L)n | scFv | L | Hinge | TM | AD | | |
| 3 | SP | L | (AABD-L)n | scFv | L | CD3εγδ ECD | TM | AD | | |
| 4 | SP | L | (AABD-L)n | scFv | L | LL | TM | (CD)n | AD | |
| 5 | SP | L | (AABD-L)n | scFv | L | Hinge | TM | (CD)n | AD | |
| 6 | SP | L | (AABD-L)n | scFv | L | CD3εγδ ECD | TM | (CD)n | AD | |
| 7 | SP | L | (AABD-L)n | scFv | L | LL | TM | CD1 | CD2 | AD |
| 8 | SP | L | (AABD-L)n | scFv | L | Hinge | TM | CD1 | CD2 | AD |
| 9 | SP | L | (AABD-L)n | scFv | L | CD3εγδ ECD | TM | CD1 | CD2 | AD |
| 10 | SP | L | (AABD-L)n | scFv | L | CD8-Hinge | CD8-TM | 4-1BB | CD3z | |
| 11 | SP | L | (AABD-L)n | scFv | L | CD28-Hinge | CD28-TM | CD28 | CD3z | |
| 12 | SP | L | (AABD-L)n | scFv | L | CD3ε-ECD | CD3e-TM | 4-1BB | CD3z | |
| 13 | SP | L | (AABD-L)n | scTFv | L | LL | TM | AD | | |
| 14 | SP | L | (AABD-L)n | scTFv | L | Hinge | TM | AD | | |
| 15 | SP | L | (AABD-L)n | scTFv | L | CD3εγδ ECD | TM | AD | | |
| 16 | SP | L | (AABD-L)n | scTFv | L | LL | TM | (CD)n | AD | |
| 17 | SP | L | (AABD-L)n | scTFv | L | Hinge | TM | (CD)n | AD | |
| 18 | SP | L | (AABD-L)n | scTFv | L | CD3εγδ ECD | TM | (CD)n | AD | |
| 19 | SP | L | (AABD-L)n | scTFv | L | LL | TM | CD1 | CD2 | AD |
| 20 | SP | L | (AABD-L)n | scTFv | L | Hinge | TM | CD1 | CD2 | AD |
| 21 | SP | L | (AABD-L)n | scTFv | L | CD3εγδ ECD | TM | CD1 | CD2 | AD |
| 22 | SP | L | (AABD-L)n | scTFv | L | CD8-Hinge | CD8-TM | 4-1BB | CD3z | |
| 23 | SP | L | (AABD-L)n | scTFv | L | CD28-Hinge | CD28-TM | CD28 | CD3z | |
| 24 | SP | L | (AABD-L)n | scTFv | L | CD3ε-ECD | CD3e-TM | 4-1BB | CD3z | |

As used herein "beneficial results" may include, but are not limited to, lessening or alleviating the severity of the disease condition, preventing the disease condition from worsening, curing the disease condition, preventing the disease condition from developing, lowering the chances of a patient developing the disease condition and prolonging a patient's life or life expectancy.

In another embodiment, an antibody molecule is a multispecific antibody molecule, *e.g.,* it comprises a plurality of immunoglobulin variable domain sequences, wherein a first immunoglobulin variable domain sequence of the plurality has binding specificity for a first epitope and a second immunoglobulin variable domain sequence of the plurality has binding specificity for a second epitope. In another embodiment, a multispecific antibody molecule is a bispecific antibody molecule. A bispecific antibody has specificity for two antigens. A bispecific antibody molecule is characterized by a first immunoglobulin variable domain sequence which has binding specificity for a first epitope and a second immunoglobulin variable domain sequence that has binding specificity for a second epitope. A bispecific molecule may be a bispecific T cell engaging antibody in which first antigen binding domain binds to an antigen *(e.g.,* CD3ε) expressed on T cells and the second antigen binding domain binds to an antigen expressed on a disease causing or disease associated cell *(e.g.,* a cancer cell). The bispecific antibodies can be used for inducing T cell mediated cytotoxicity against cells expressing the target antigen recognized by their second antigen binding domain. The novel antigen binding domains described in this disclosure can be used to construct bispecific T cell engagers.

"Binds the same epitope as" means the ability of an antibody, scFv, or other antigen binding domain to bind to a target antigen and having the same epitope as an exemplified antibody, scFv, or other antigen binding domain. As an example, the epitopes of the exemplified antibody, scFv, or other binding agent and other antibodies can be determined using standard epitope mapping techniques. Epitope mapping techniques, well known in the art include Epitope Mapping Protocols in Methods in Molecular Biology, Vol. 66 (Glenn E. Morris, Ed., 1996) Humana Press, Totowa, New Jersey. For example, linear epitopes may be determined by, *e.g*., concurrently synthesizing large numbers of peptides on solid supports, the peptides corresponding to portions of the protein molecule, and reacting the peptides with antibodies while the peptides are still operably linked to the supports. Such techniques are known in the art and described in, *e.g.,* U.S. Patent No. 4,708,871; Geysen et al, (1984) Proc. Natl. Acad. Sci. USA 8:3998-4002; Geysen et al, (1985) Proc. Natl. Acad. Sci. USA 82:78-182; Geysen et al, (1986) Mol. lmmunol. 23: 709-715. The epitope bound by the antigen binding domain of a CAR can be also determined by the Epitope Binning assay. Epitope binning is a competitive immunoassay used to characterize and then sort a library of monoclonal antibodies against a target protein. Antibodies against a similar target are tested against all other antibodies in the library in a pairwise fashion to see if antibodies block one another's binding to the epitope of an antigen. After each antibody has a profile created against all of the other antibodies in the library, a competitive blocking profile is created for each antibody relative to the others in the library. Closely related binning profiles indicate that the antibodies have the same or a closely related epitope and are "binned" together. Similarly, conformational epitopes are readily identified by determining spatial conformation of amino acids such as by, *e.g*., hydrogen/deuterium exchange, x-ray crystallography and two-dimensional nuclear magnetic resonance. See, *e.g.,* Epitope Mapping Protocols, supra. Antigenic regions of proteins can also be identified using standard antigenicity and hydropathy plots, such as those calculated using, *e.g.,* the Omiga version 1.0 software program available from the Oxford Molecular Group. This computer program employs the Hopp/Woods method, Hopp et al, (1981) Proc. Natl. Acad. Sci USA 78:3824-3828; for determining antigenicity profiles, and the Kyte-Doolittle technique, Kyte et al, (1982) J.Mol. Bioi. 157: 1 05-132; for hydropathy plots. To determine if selected monoclonal antibodies against a target *(e.g.,* CD19) bind to unique epitopes, each antibody can be biotinylated using commercially available reagents (Pierce, Rockford, Ill.). Competition studies using unlabeled monoclonal antibodies and biotinylated monoclonal antibodies can be performed using CD19-extracellualr domain coated-ELISA plates. Biotinylated mAb binding can be detected with a strepavidin-alkaline phosphatase probe. Exemplary epitopes of human CD20 antigen bound by scFv, SARs, antibodies and other immunotherapeutics of the current disclosure are provided in SEQ ID NO: 15149-15154 of patent application PCT/US18/53247, which is incorporated in its entirety by reference herein. Exemplary epitopes of human BCMA bound by scFv, SARs, antibodies and other immunotherapeutics of the current disclosure are provided in SEQ ID NO: 15155-15159 of patent application PCT/US18/53247, which is incorporated in its entirety by reference herein. An exemplary epitope of human MPL antigen bound by scFv, SARs, and antibodies of the current disclosure is provided in SEQ ID NO: 15160 of patent application PCT/US18/53247, which is incorporated in its entirety by reference herein.

As used herein, the term "biological equivalent thereof" or "variant" or "functional variant" is intended to be synonymous with "equivalent thereof" when referring to a reference protein, antibody or fragment thereof, polypeptide or nucleic acid, intends those having minimal homology while still maintaining desired structure or functionality. Unless specifically recited herein, it is contemplated that any of the above also includes equivalents thereof. For example, an equivalent intends at least about 70% homology or identity, or at least 80% homology or identity and alternatively, or at least about 85%, or alternatively at least about 90%, or alternatively at least about 95%, or alternatively at least 98% percent homology or identity and exhibits substantially equivalent biological activity to the reference protein, polypeptide, antibody or fragment thereof or nucleic acid. Alternatively, when referring to polynucleotides, an equivalent thereof is a polynucleotide that hybridizes under stringent conditions to the reference polynucleotide or its complement. Alternatively, when referring to polypeptides or proteins, an equivalent thereof is an expressed polypeptide or protein from a polynucleotide that hybridizes under stringent conditions to the polynucleotide or its complement that encodes the reference polypeptide or protein.

As used herein, the term "Bispecific" refers to an agent (*e.g*., antibody, antibody fragment, SAR, CAR *etc.)* that can bind to two antigens.

As used herein, the term "Biparatopic" refers to an agent *(e.g*., antibody, antibody fragment, SAR, CAR *etc.)* that can bind to two epitopes of an antigen.

As used herein, the term *"*multispecific" refers to an agent *(e.g*., antibody, antibody fragment, SAR, CAR *etc.)* that can bind to more than two antigens.

As used herein, the term "CDR" or "complementarity determining region" is intended to mean the non-contiguous antigen combining sites found within the variable region of both heavy and light chain polypeptides. These particular regions have been described by Kabat et al., J. Bioi. Chern. 252:6609-6616 (1977); Kabat et al., U.S. Dept. of Health and Human Services, "Sequences of proteins of immunological interest" (1991); Chothia et al., J. Mol. Bioi. 196:901-917 (1987); and MacCallum et al., J. Mol. Bioi. 25 262:732-745 (1996), where the definitions include overlapping or subsets of amino acid residues when compared against each other. Nevertheless, application of either definition to refer to a CDR of an antibody or grafted antibodies or variants thereof is intended to be within the scope of the term as defined and used herein. As used herein, the different CDRs of an antibody could be also defined by a combination of the different definitions. For example, VHCDR1 could be defined based on Kabat and VHCDR2 could be defined based on Chothia. The amino acid residues which encompass the CDRs as defined by each of the above cited references are as follows:

### CDR DEFINITIONS

| | **Kabat** | **Chothia** | **MacCallum** |
|---|---|---|---|
| VHCDR1 | 31-35 | 26-32 | 30-35 |
| VHCDR2 | 50-65 | 53-55 | 47-58 |
| VHCDR3 | 95-102 | 96-10 | 193-101 |
| VLCDR1 | 24-34 | 26-32 | 30-36 |
| VLCDR2 | 50-56 | 50-52 | 46-55 |
| VLCDR3 | 89-97 | 91-96 | 89-96 |

| | | | |
|---|---|---|---|
| (Residue Numbers correspond to the identified reference). | | | |

The SEQ IDs of the CDRs of the different vL and vH segments that can make up antigen binding domains of scFv, CARs, AMR, antibodies and other immunotherapeutics of the current disclosure are provided in SEQ ID NO: 13204-14121 and SEQ ID NO: 14122-15039, respectively **(Tables 6A, B)** of PCT/US18/53247 and in **Tables 5-6** in PCT/US2017/064379, which are incorporated herein by reference.

In some embodiments, reference to an antigen-binding module (such as a Fab-like or Fv-like antigen-binding module) that specifically binds to a target antigen means that the antigen-binding module binds to the target antigen with (a) an affinity that is at least about 10 *(e.g.,* about 10, 20, 30, 40, 50, 75, 100, 200, 300, 400, 500, 750, 1000 or more) times its binding affinity for other molecules; or (b) a K_{d} no more than about 1/10 *(e.g.,* 1/10, 1/20, 1/30, 1/40, 1/50, 1175, 1/100, 1/200, 1/300, 1/400, 1/500, 1/750, 1/1000 or less) times its K_{d} for binding to other molecules. Binding affinity can be determined by methods known in the art, such as ELISA, fluorescence activated cell sorting (FACS) analysis, Malibu-Glo assay, Topanga Assay, or radioimmunoprecipitation assay (RIA). K_{d} can be determined by methods known in the art, such as surface plasmon resonance (SPR) assay utilizing, for example, Biacore instruments, or kinetic exclusion assay (KinExA) utilizing, for example, Sapidyne instruments.

"Cancer" and "cancerous" refer to or describe the physiological condition in mammals that is typically characterized by unregulated cell growth. Examples of cancer include, but are not limited to B-cell lymphomas (Hodgkin's lymphomas and/or non-Hodgkins lymphomas), T cell lymphomas, myeloma, myelodysplastic syndrome, myeloproliferative disorders (*e.g*., polycythemia vera, myelofibrosis, essential thrombocythemia *etc.),* skin cancer, brain tumor, breast cancer, colon cancer, rectal cancer, esophageal cancer, anal cancer, cancer of unknown primary site, endocrine cancer, testicular cancer, lung cancer, hepatocellular cancer, gastric cancer, pancreatic cancer, cervical cancer, ovarian cancer, liver cancer, bladder cancer, cancer of the urinary tract, cancer of reproductive organs thyroid cancer, renal cancer, carcinoma, melanoma, head and neck cancer, brain cancer (*e.g*., glioblastoma multiforme), prostate cancer, including but not limited to androgen-dependent prostate cancer and androgen-independent prostate cancer, and leukemia. Other cancer and cell proliferative disorders will be readily recognized in the art. The terms "tumor" and "cancer" are used interchangeably herein, e.g., both terms encompass solid and liquid, *e.g*., diffuse or circulating, tumors. As used herein, the term "cancer" or "tumor" includes premalignant, as well as malignant cancers and tumors. The term "cancer" is meant to include all types of cancerous growths or oncogenic processes, metastatic tissues or malignantly transformed cells, tissues, or organs, irrespective of histopathologic type or stage of invasiveness.

"Cell therapy" or "Cell-based therapy" or "Immune cell therapy" or Immune effector cell therapy" or "adoptive cell therapy" refers to a therapy that involves the use of cells for the prevention or treatment of a disease. Non-limiting examples of cell therapy include CAR-T cell therapy, NK- cell therapy, recombinant TCR-T cell therapy, TIL (tumor infiltrating lymphocytes). Biological agents, such as antibodies (e.g., Bispecific T cell engagers and DARTs *etc.)* which mediate their effect by binding to and/or activating immune cells (e.g, T cells and NK cells) are other examples of cell therapies. Stem cell and organ transplants, including autologous and allogeneic blood and marrow transplants, are also examples of cell therapies.

The term "Centyrins" as used here refers to small, engineered proteins derived from a human protein, Tenascin C, which can bind to an antigen of interest with high affinity and specificity. Centyrins have highly stable fibronectin type III (FN3) domain. Exemplary Centyrins are provided in Table 7.

"Chemotherapeutic agents" are compounds that are known to be of use in chemotherapy for cancer. Non-limiting examples of chemotherapeutic agents can include alkylating agents such as thiotepa and CYTOXAN^{®} cyclosphosphamide; alkyl sulfonates such as busulfan, improsulfan and piposulfan; a camptothecin (including the synthetic analogue topotecan); bryostatin;nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (*e.g*., calicheamicin, especially calicheamicin gammalI and calicheamicin omegaI1 (see, *e.g*., Agnew, Chem. Intl. Ed. Engl., 33: 183-186 (1994)); dynemicin, including dynemicin A; bisphosphonates, such as clodronate; an esperamicin; as well as neocarzinostatin chromophore and related chromoprotein enediyne antiobiotic chromophores), aclacinomysins, actinomycin, authramycin, azaserine, bleomycins, cactinomycin, carabicin, caminomycin, carzinophilin, chromomycinis, dactinomycin, daunorubicin, detorubicin, 6-diazo-5-oxo-L-norleucine, ADRIAMYCIN^{®} doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK^{®} polysaccharide complex (JHS Natural Products, Eugene, Oreg.); razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, *e.g*., TAXOL^{®} paclitaxel (Bristol-Myers Squibb Oncology, Princeton, N.J.), ABRAXANE^{®} Cremophor-free, albumin-engineered nanoparticle formulation of paclitaxel (American Pharmaceutical Partners, Schaumberg, Ill.), and TAXOTERE^{®} doxetaxel (Rhone-Poulenc Rorer, Antony, France); chloranbucil; GEMZAR^{®} gemcitabine; 6-thioguanine; mercaptopurine; methotrexate; platinum analogs such as *cis*platin, oxaliplatin and carboplatin; vinblastine; platinum; etoposide (VP-16); ifosfamide; mitoxantrone; vincristine; NAVELBINE; vinorelbine; novantrone; teniposide; edatrexate; daunomycin; aminopterin; xeloda; ibandronate; irinotecan (Camptosar, CPT-11); topoisomerase inhibitor RFS 2000; difluoromethylornithine (DMFO); retinoids such as retinoic acid; capecitabine; combretastatin; leucovorin (LV); oxaliplatin, lapatinib (Tykerb); inhibitors of PKC-alpha, Raf, H-Ras, EGFR (*e.g*., erlotinib (Tarceva^{®})) and VEGF-A that reduce cell proliferation and pharmaceutically acceptable salts, acids or derivatives of any of the above or combinations thereof.

"Chimeric antigen receptors" (CARs) are artificial (non-naturally occurring) immune cell (*e.g.,* T cell) receptors contemplated for use as a therapy for cancer, using a technique called adoptive cell transfer. CARs are also known as artificial T-cell receptors, chimeric T-cell receptors or chimeric immunoreceptors. CARs are constructed specifically to stimulate T cell activation and proliferation in response to a specific antigen to which the CAR binds. Generally, a CAR refers to a set of polypeptides, typically two in the simplest embodiments, which when expressed in an immune effector cell, provides the cell with specificity for a target cell, typically a cancer cell, and with intracellular signal generation. In some embodiments, a CAR comprises at least an extracellular antigen binding domain, a transmembrane domain and a cytoplasmic signaling domain (also referred to herein as "an intracellular signaling domain") comprising a functional signaling domain derived from a stimulatory molecule and/or costimulatory molecule. In some aspects, the set of polypeptides are contiguous with each other. In one aspect, the stimulatory molecule is the zeta chain associated with the T cell receptor complex. In one aspect, the cytoplasmic signaling domain further comprises one or more functional signaling domains derived from at least one costimulatory molecule as defined below. In one embodiment, the costimulatory molecule is chosen from the costimulatory molecules described herein, *e.g.,* 4-lBB (*i.e.,* CD137), CD27 and/or CD28. In one embodiment, the CAR comprises an optional leader sequence at the amino-terminus (N-ter) of the CAR fusion protein. In one embodiment, the CAR further comprises a leader sequence at the N-terminus of the extracellular antigen binding domain, wherein the leader sequence is optionally cleaved from the antigen binding domain (*e.g.,* a scFv) during cellular processing and localization of the CAR to the cellular membrane. In various embodiments, CARs are recombinant polypeptides comprising an antigen-specific domain (ASD), a hinge region (HR), a transmembrane domain (TMD), an optional co-stimulatory domain (CSD) and an intracellular signaling domain (ISD). The optional costimulatory domain is generally absent in the 1^{st} generation CAR constructs. The nucleic acid sequences of several exemplary 2nd generation CARs comprising the different antigen binding domains (*e.g.,* vL and vH fragments, vHH, ligands and receptors *etc.*) and incorporating the 41BB costimulatory domain are presented in SEQ ID NO: 1455-1703 (**Table 8**) of PCT/US2020/014237. The corresponding amino acid sequences are provided in SEQ ID NO: 7341-7589 of PCT/US2020/014237. In an embodiment, the disclosure provides bispecific, biparatopic and multispecific CARs.

The term SAR, as used herein, the comprises CARs and also encompasses newer approaches to conferring antigen specificity onto cells, such as Antibody-TCR chimeric molecules or Ab-TCR (WO 2017/070608 A1 incorporated herein by reference), TCR receptor fusion proteins or TFP (WO 2016/187349 A1 incorporated herein by reference), Synthetic Immune Receptors (SIRs) (see, WO 2018/102795 A1, incorporated herein by reference), Tri-functional T cell antigen coupler (Tri-TAC or TAC) (see, WO 2015/117229 A1, incorporated herein by reference) and zSIR (see, PCT/US2019/035096, incorporated herein by reference). The nucleic acid sequences of several exemplary TFPs comprising the different antigen binding domains (*e.g*., vL and vH fragments, vHH, ligands and receptors *etc.*) and based on CD3ε, CD3δ, CD3γ and CD3ζ chains and co-expressing the optional accessory module NEMO-K277A are presented in SEQ ID NO:1900-2205, 2206-2511, 2512-2817, 2818-3123, respectively (**Table 13**) of PCT/US18/53247, which is incorporated in its entirety by reference herein. The order of the antigen binding domains contained in the construct of different CAR architectures and BiTE listed in **Table 13** of PCT/US18/53247, which is incorporated in its entirety by reference herein is the same as the order of the constructs on the zCAR-K277A architecture presented in **Table 12** of PCT/US18/53247, which is incorporated in its entirety by reference herein. Typically, the term "SAR-T cell" is used, to refer to T-cells that have been engineered to express a Synthetic antigen receptor. Thus, T lymphocytes bearing such SARs are generally referred to as SAR-T lymphocytes. If the SAR is a CAR, then the T cells are referred to as CAR-T cells. SARs can be also expressed in cells other than T cells, such as hematopoietic stem cells, induced pluripotent stem cells (iPSC), NK cells and macrophage. This disclosure provides bispecific, biparatopic and mutlispecific SARs with the backbone of CAR, SIR, zSIR, cTCR, Ab-TCR, AABD-TCR, TFP, and TCR *etc.* comprising one or more AABDs. The AABD domains of the SARs on any of the above backbones can be fully human, humanized or non-human. In an embodiment, the disclosure provides SARs on any of the above backbones comprising one or more fully human vH domains. In an embodiment, the disclosure provides SARs on any of the above backbones comprising one or more fully human vL domains.

"Codon optimization" or "controlling for species codon bias" refers to the preferred codon usage of a particular host cell. As will be understood by those of skill in the art, it can be advantageous to modify a coding sequence to enhance its expression in a particular host.

As used herein, "co-express" refers to expression of two or more polynucleotides or genes. Genes may be nucleic acids encoding, for example, a single protein or a chimeric protein as a single polypeptide chain. A SAR or a TCR described herein may be encoded by a single polynucleotide chain and expressed as single polypeptide chain, which is subsequently cleaved into different polypeptides, each representing a distinct functional unit. In some embodiments, where the SAR or a TCR consists of two or more functional polypeptide units, the different functional units are coexpressed using one or more polynucleotide chains. In one embodiment, costimulation is provided by an accessory module that is co-expressed with the SAR or a TCR but is not an integral part of the SAR or TCR polypeptide. In another embodiment, the different polynucleotide chains are linked by nucleic acid sequences that encode for cleavable linkers (*e.g.,* T2A, F2A, P2A, E2A *etc.*) **(Table 20).** In another embodiment, a Ser-Gly-Ser-Gly (SGSG) motif (SEQ ID NO: 1239 and 11929) is also added upstream of the cleavable linker sequences to enhance the efficiency of cleavage. The polynucleotides encoding the different units of a SAR or a TCR may be linked by IRES (Internal Ribosomal Entry Site) sequences. Alternately, the different functional units of a SAR or TCR are encoded by two different polynucleotides that are not linked via a linker but are instead encoded by, for example, two different vectors. The nucleic acid and amino acid sequences of exemplary cleavable linkers and Furine cleavage sites are provided in **Table 20.**

A "conservative substitution" or "conservative sequence modifications" refers to amino acid modifications that do not significantly affect or alter the binding characteristics or function of the encoded protein. For example, "conservative sequence modifications" refers to amino acid modifications that do not significantly affect or alter the binding characteristics or function of a SAR construct of the disclosure (*e.g*., a conservative change in the constant chain, antibody, antibody fragment, or non-immunoglobulin binding domains). Such conservative modifications include amino acid substitutions, additions and deletions. Modifications can be introduced by standard techniques known in the art, such as site-directed mutagenesis and PCR-mediated mutagenesis. Conservative amino acid substitutions are ones in which the amino acid residue is replaced with an amino acid residue having a similar side chain. Families of amino acid residues having similar side chains have been defined in the art. These families include amino acids with basic side chains (*e.g*., lysine, arginine, histidine), acidic side chains (*e.g*., aspartic acid, glutamic acid), uncharged polar side chains (*e.g*., glycine, asparagine, glutamine, serine, threonine, tyrosine, cysteine, tryptophan), nonpolar side chains (*e.g*., alanine, valine, leucine, isoleucine, proline, phenylalanine, methionine), beta-branched side chains (*e.g*., threonine, valine, isoleucine) and aromatic side chains (*e.g*., tyrosine, phenylalanine, tryptophan, histidine). Thus, one or more amino acid residues within a SAR of the disclosure can be replaced with other amino acid residues from the same side chain family and the altered SAR can be tested using the binding and/or functional assays described herein.

The term "TCR constant chain" or "constant region of T cell receptor" is defined as the constant chain of TCRα/TCRa, TCRβ1/TCRb1, TCRβ2/TCRb2, TCRγ/TCRd, TCRδ/TCRd and pre-TCRα. Exemplary TCR constant chains are listed in **Table 12.** A TCR constant chain can be divided into several subdomains such as Ig like C1 domain (*e.g*., SEQ ID NO: 11848-11865; Table 13), connecting peptide (*e.g.,* SEQ ID NO: 11867-11875; Table 14), transmembrane domain (SEQ ID NO:11877-11880; Table 15), and cytosolic domain (*e.g.,* SEQ ID NO: 11883-11885; **Table 16**). The cytosolic domains of TCRα, TCRβ1/β2, TCRγ and TCRδ chains are short and generally not believed to play any significant role in their signaling activities. The disclosure also provides deletion mutants and variants of the TCR chains listed in **Table 12** as long as they retain one or more of the functional and biological properties of the TCR chains such as the ability to pair with the complementary TCR chain, the ability to assemble with the TCR/CD3 complex and the ability to transmit a T cell signal (*e.g*., activate NFAT pathway) when engaged by target antigen expressing cells.

The term "constant region of T cell receptor-alpha" or "constant chain of T cell receptor-alpha" or "TCRα" or "Cα" is defined as the protein provided as SEQ ID NO: 11735 or 11733 or the equivalent residues (*i.e.,* a homolog or variant) from a non-human species, *e.g*., mouse, rodent, monkey, ape and the like. The term also covers any deletion or point mutant and variant of TCRα that retains the biological and functional properties of TCRα, such as such as the ability to pair with the complementary TCRβ chain, the ability to assemble with the TCR/CD3 complex and the ability to transmit a T cell signal (*e.g*., activate NFAT pathway) when engaged by target antigen expressing cells. The disclosure also provides certain mutations to TCRα polypeptides which can be used in the construction of SIRs and Ab-TCR (**Tables 12**). For example, sites of mutation in Cα that demonstrate increased expression and decreased mispairing are located at positions 91, 92, 93, and 94 of SEQ ID NO 11735. A TCR polypeptide with a Thr 48 Cys (T48C) mutation in Cα and a Ser-57-Cys (S57C) mutation in Cβ1 or Cβ2 chain (described more fully elsewhere herein) results in an additional disulfide bond between the two TCR constant chains (α and β). This, in turn, results in reduced mispairing with endogenous TCR chains in an immune cell and enhanced functionality. Similarly, a SIR with a Ser 61 Arg (S61R) mutation in Cα and an Arg 79 Gly (R79G) mutation in Cβ1 or Cβ2 chain (described more fully elsewhere herein) results in reduced mispairing with the endogenous TCR chains and enhanced functionality due to a "knob and hole" design for pairing. The disclosure provides Cα polypeptides having one or more or all of the mutations according to **Table A-8** below and **Table 12** which can be used in the construction of SIRs and Ab-TCR.

| **Table A-8: Mutations according to the disclosure in the human constant TCR-alpha region (Cα)** | | | |
|---|---|---|---|
| **Position (SEQ ID NO: 11735)** | **Amino acid in wild-type** | **Mutation** | **TYPE** |
| 10 | Y | C | disulfide bond |
| 15 | S | C | disulfide bond |
| 45 | T | C | disulfide bond |
| 48 | T | C | disulfide bond |
| 61 | S | R | Knob into Hole |
| 91 | P | S | Murinization |
| 92 | E | D | Murinization |
| 93 | S | V | Murinization |
| 94 | S | P | Murinization |

The human genome encodes for two highly homologous TCR beta constant chains; TCR beta1 (TCRβ1 or TCRb1 or cβ1) and TCR beta 2 (TCRβ2 or TCRb2 or cβ2). The SARs (*e.g.,* SIR, Ab-TCR or TFP) of the disclosure can comprise either of these two chains. Similarly, either TCR beta1 or TCR beta2 chains of other mammalian species can be used in the methods of the disclosure.

The term "constant chain of T cell receptor-beta 1" or "constant region of T cell receptor-beta 1" (TCR-beta1 or TCRβ1 or TCRb1 or hTCR-beta1 or Cβ1) is defined as a protein provided as SEQ ID NO: 11746 or the equivalent residues (*i.e*., a homolog) from a non-human species, *e.g*., mouse, rodent, monkey, ape and the like. The term also covers any deletion or point mutant and variant of TCRβ1 that retains the biological and functional properties of TCRβ1, such as such as the ability to pair with the complementary TCRα chain, the ability to assemble with the TCR/CD3 complex and the ability to transmit a T cell signal (*e.g*., activate NFAT pathway) when engaged by target antigen expressing cells. The disclosure also provides certain mutations to TCRβ1 polypeptides which can be used in the construction of SARs (*e.g*., SIRs and Ab-TCR) (**Table 12**). The disclosure also provides several deletion mutants of TCRβ1 that can be used in the construction of SARs (**Table 12**).

The term "constant chain of T cell receptor-beta 2" or "constant region of T cell receptor-beta 2" (TCR-beta2 or TCRβ2 or TCRb2 or Cβ2) is defined as the protein provided as SEQ ID NO: 11747 or the equivalent residues (*i.e*., a homolog) from a non-human species, *e.g*., mouse, rodent, monkey, ape and the like. The term also covers any deletion or point mutant and variant of TCRβ2 that retains the biological and functional properties of TCRβ2, such as such as the ability to pair with the complementary TCRα chain, the ability to assemble with the TCR/CD3 complex and the ability to transmit a T cell signal (*e.g*., activate NFAT pathway) when engaged by target antigen expressing cells. The disclosure also provides certain mutations to TCRβ2 polypeptides which can be used in the construction of SARs (*e.g*., SIRs and Ab-TCR) (**Table 12**). The disclosure also provides several deletion mutants of TCRβ2 that can be used in the construction of SARs (**Table 12)**.

The protein sequences for both Cβ1 (SEQ ID NO: 11746) and Cβ2 (SEQ ID NO: 11747) are known (**Table 6**). Differences between the sequences of Cβ2 and Cβ1 are easily identified by alignment of the sequences using typical and ordinary skill in the art. In general, Cβ1 and Cβ2 chains are highly homologous. Therefore, unless specified otherwise, the term constant chain of TCRβ, TCR-beta or TCRb refers to constant chain of either TCRβ1 or TCRβ2. Similarly, unless specified otherwise, the term Cβ applies to either Cβ1 or Cβ2. The disclosure also provides certain mutations to TCRβ chains that can be used in the construction of SIRs and Ab-TCRs. For example, sites of mutation in Cβs that demonstrate increased expression and decreased mispairing with the endogenous TCRα chains are provided herein. These mutation sites in Cβ1 and Cβ2 are located at positions 18, 22, 57, 79 133, 136, and 139 of SEQ ID NOs: 11746 and 11747 and are summarized in the **Tables A-9** and **A10** below. The mutation sites in Cβ1 and Cβ2 are identical in their positions. The only difference between the two sequences is that a mutation at position 136. At this position, a glutamic acid (E) is present in Cβ2, whereas a valine is present in Cβ1.

| **Table A-9: Mutations according to the disclosure in the human constant TCR-beta region1 (Cβ1)** | | | |
|---|---|---|---|
| **Position (SEQ ID NO: 11746)** | **Amino acid in wild-type** | **Mutation** | **TYPE** |
| 15 | E | C | disulfide bond |
| 17 | S | C | disulfide bond |
| 18 | E | K or R | Murinization |

| | | | |
|---|---|---|---|
| 22 | S | A | Murinization |
| 57 | S | C | disulfide bond |
| 59 | D | C | disulfide bond |
| 77 | S | C | disulfide bond |
| 79 | R | G | Knob into Hole |
| 133 | F | I | Murinization |
| 136 | V | A | Murinization |
| 139 | Q | H | Murinization |

| **Table A-10: Mutations according to the disclosure in the human constant TCR-beta region2 (Cβ2)** | | | |
|---|---|---|---|
| **Position (SEQ ID NO: 11747)** | **Amino acid in wild-type** | **Mutation** | **TYPE** |
| 15 | E | C | disulfide bond |
| 17 | S | C | disulfide bond |
| 18 | E | K or R | Murinization |
| 22 | S | A | Murinization |
| 57 | S | C | disulfide bond |
| 59 | D | C | disulfide bond |
| 77 | S | C | disulfide bond |
| 79 | R | G | Knob into Hole |
| 133 | F | I | Murinization |
| 136 | E | A | Murinization |
| 139 | Q | H | Murinization |

The term "constant chain of TCR-gamma" or "constant region of TCR-gamma" (TCR-gamma or TCRγ or TCRg or TCR-gamma1 or TCRγ1 or TCRg1 or Cγ) is defined as the protein provided as SEQ ID NO: 11771 or the equivalent residues (*i.e.,* a homolog) from a non-human species, *e.g*., mouse, rodent, monkey, ape and the like. The term also covers any deletion or point mutant and variant of TCRγ that retains the biological and functional properties of TCRγ, such as such as the ability to pair with the complementary TCR (*e.g.,* TCRδ) chain, the ability to assemble with the TCR/CD3 complex and the ability to transmit a T cell signal (*e.g*., activate NFAT pathway) when engaged by target antigen expressing cells. The disclosure also provides certain mutations to TCRγ polypeptides which can be used in the construction of SARs (*e.g*., SIRs and Ab-TCR) (**Table 12**). The disclosure also provides several deletion mutants of TCRβ that can be used in the construction of SARs (**Table 12**).

The term "constant chain of TCR-delta" or "constant region of TCR-delta" (TCR-delta or TCRδ or TCRd or Cδ) is defined as the proteins provided as SEQ ID NO: 5982 or the equivalent residues (*i.e.,* a homolog) from a non-human species, *e.g.,* mouse, rodent, monkey, ape and the like. The term also covers any deletion or point mutant and variant of TCRδ that retains the biological and functional properties of TCRδ, such as such as the ability to pair with the complementary TCR (*e.g.,* TCRγ) chain, the ability to assemble with the TCR/CD3 complex and the ability to transmit a T cell signal (*e.g*., activate NFAT pathway) when engaged by target antigen expressing cells. The disclosure also provides certain mutations to TCRδ polypeptides which can be used in the construction of SARs (*e.g.,* SIRs and Ab-TCR) (**Table 12**). The disclosure also provides several deletion mutants of TCRβ that can be used in the construction of SARs (**Table 12**).

The term "constant chain of pr*etc*.R-α" or "constant region of pr*etc.*R*"* (pr*etc*.Rα or pr*etc*.R-α or preCα) is defined as the protein provided as SEQ ID NO: 11769 or the equivalent residues (*i.e.,* a homolog) from a non-human species, *e.g.,* mouse, rodent, monkey, ape and the like. The term also covers any deletion or point mutant and variant of pr*etc*.Rα that retains the biological and functional properties of pr*etc*.Rα, such as such as the ability to pair with the complementary TCR (*e.g.,* TCRβ) chain, the ability to assemble with the TCR/CD3 complex and the ability to transmit a T cell signal (*e.g*., activate NFAT pathway) when engaged by target antigen expressing cells. The disclosure also provides certain mutations to pr*etc*.Rα polypeptides which can be used in the construction of SARs (*e.g.,* SIRs and Ab-TCR) (**Table 12**). The disclosure also provides several deletion mutants of pr*etc*.Rα that can be used in the construction of SARs (**Table 12**).

It will be recognized that proteins can have identity or homology to one another and retain similar or identical functions. The disclosure includes TCR constant regions that have 70%, 80%, 85%, 90%, 95%, 97%, 98%, 98.5%, 99% or 99.9% identity to any of the sequences described herein while retaining the biological activity.

Accordingly, the disclosure provides SARs (*e.g.,* SIR, cTCR, zSIR, Ab-TCR, TFP and the like) having a T-cell receptor constant chain having a sequence selected from the group consisting of: (a) an amino acid sequence that is at least 70% identical to any of the SEQ ID NO: 11733-11742, 11744, 11745, 11793-11803 and which can optionally have one or more mutations at positions 61, 91, 92, 93, 94, 120, 127 and/or 129; (b) an amino acid sequence that is at least 75% identical to any of the SEQ ID NO:11746- 11766, 11804-11817 and which can optionally have one or more mutations at positions 18, 22, 57, 79, 133, 136 and/or 139; c) an amino acid sequence that is at least 70% identical to any of the SEQ ID NO:11818-11823; (d) an amino acid sequence that is at least 70% identical to any of the SEQ ID NO:11824-11830; and (e) an amino acid sequence that is at least 70% identical to any of the SEQ ID NO:11769-11770; and The T-cell receptor constant chains of any of (a)-(d) retain at least one biological activity of the wild-type T-cell receptor constant chain to which it has identity or homology.

In one embodiment, the disclosure provides a modified TCR is selected from the group consisting of a wild-type TCR, a high affinity TCR, and a chimeric TCR. In another embodiment, the modified TCR comprises at least one extra disulfide bond. In yet another embodiment, the modified TCR comprises a TCR alpha chain and TCR beta chain.

The term "constitutively active" refers to a molecule, *e.g.,* a protein that has signaling activity without the need of a stimulus. Exemplary constitutive active proteins are NEMO-K277A and vFLIP K13 as they can activate NF-κB signaling when expressed in a suitable cell without the need of an additional stimulus.

The term a "costimulatory molecule" or a "costimulatory receptor" refers to a cognate binding partner on a T cell that specifically binds with a costimulatory ligand, thereby mediating a costimulatory response by the T cell, such as, but not limited to, proliferation. Costimulatory extracellular molecules are cell surface molecules other than antigen receptors or their ligands that contribute to an efficient immune response. Costimulatory molecules include, but are not limited to, an MHC class I molecule, BTLA and a Toll ligand receptor, as well as OX40, CD28, 2B4 and 4-1BB (CD137). A co-stimulatory receptor may be expressed on cells other T cells, such as NK cells or macrophages.

A "costimulatory intracellular signaling domain" or "costimulatory domain" (CSD) can be the intracellular portion of a costimulatory receptor. A costimulatory molecule can be represented in the following protein families: TNF receptor proteins, Immunoglobulin-like proteins, cytokine receptors, integrins, signaling lymphocytic activation molecules (SLAM proteins), and activating NK cell receptors. Examples of such molecules include CD27, CD28, 4-1BB (CD137), OX40, GITR, CD30, 2B4, CD40, ICOS and the like. The intracellular signaling domain can comprise the entire intracellular portion, or the entire native intracellular signaling domain, of the molecule from which it is derived, or a functional fragment or derivative thereof. The SARs of the disclosure may comprise one or more co-stimulatory domains.

The term "cTCR" refers to a wild-type TCR nucleic acid coding sequence and the corresponding wild-type TCR protein linked to an antigen binding domain that is not derived from a TCR. The TCR chains of a cTCR are not codon optimized and lack mutations that can enhance their expression or reduce pairing with endogenous TCR chains. In exemplary embodiment, the antigen binding domaino of a cTCR may comprise of a vL, vH or scFv fragments. cTCR have been described in (Gross, Waks & Eshhar, Proc. Natl. Acad. Sci. USA, 1989). cTCRs are used in some embodiments and as reference controls. For example, a cTCR having a CD19 binding domain and a CD19-SIR (comprising a mutant TCR chain or codon optimized TCR chain and CD19 binding domain) will have different expression and/or difference binding affinities to the target antigen. This disclosure provides bispecific, biparatopic and mutlispecific SARs with the backbone of a cTCR comprising one or more AABDs. The AABD domains of the SARs of the disclosure with the backbone of a cTCR can be fully human, humanized or non-human. In an embodiment, the disclosure provides a cTCR comprising one or more fully human vH domains. In an embodiment, the disclosure provides a cTCR comprising one or more fully human vL domains.

The term "cytosolic" or "cytoplasmic" refers to an agent, *e.g.,* a protein that is situated in the cytoplasm of a cell in its mature form. A cytosolic protein can translocate into the nucleus but is not a transmembrane protein and is not secreted outside the cell. An exemplary cytosolic protein is MC159 and K13.

Cytokine Release Syndrome (CRS) is a complication of cell therapies (*e.g*., SAR-T, bispecific T cell engaging antibodies *etc.*) that manifests itself with a constellation of signs and symptoms such as fever, hypotension, shortness of breath, renal dysfunction, pulmonary dysfunction and/or capillary leak syndrome.

DARPIN or DARPins (Designed ankyrin repeat proteins) are genetically engineered antibody mimetic proteins typically exhibiting highly specific and high-affinity target protein binding. An exemplary DARPINs are presented in **Table 7.**

The term "degenerative disorders" refers to a disease that is the result of a continuous process based on degenerative cell changes, affecting tissues or organs, which will increasingly deteriorate over time, whether due to normal bodily wear or lifestyle choices such as exercise or eating habits. Exemplary degenerative diseases include Alzheimer's disease, Creutzfeldt-Jakob disease, Diabetes mellitus (type II), and Atherosclerosis.

"Derived from" as that term is used herein, indicates a relationship between a first and a second molecule. It generally refers to structural similarity between the first molecule and a second molecule and does not connotate or include a process or source limitation on a first molecule that is derived from a second molecule. For example, in the case of an antigen binding domain that is derived from an antibody molecule, the antigen binding domain retains sufficient antibody structure such that is has the required function, namely, the ability to bind to an antigen. It does not connotate or include a limitation to a particular process of producing the antibody, *e.g*., it does not mean that, to provide the antigen binding domain, one must start with an antibody sequence and delete unwanted sequence, or impose mutations, to arrive at the antigen binding domain.

"Dimerization molecule," as that term is used herein refers to a molecule that promotes the association of a first switch domain with a second switch domain.

The phrase "disease associated with expression of a target antigen" or "disease associated antigen as described herein" includes, but is not limited to, a disease associated with expression of a target antigen as described herein or condition associated with cells which express a target antigen as described herein including, *e.g*., proliferative diseases such as a cancer or malignancy or a precancerous condition such as a myelodysplasia, a myelodysplastic syndrome or myeloproliferative disorder or a pre leukemia; or a noncancer related indication associated with cells which express a target antigen as described herein. In one aspect, a cancer associated with expression of a tumor antigen as described herein is a hematological cancer. In one aspect, a cancer associated with expression of a tumor antigen as described herein is a solid cancer. Further diseases associated with expression of a tumor antigen described herein include, but are not limited to, atypical and/or non-classical cancers, malignancies, precancerous conditions or proliferative diseases associated with expression of a tumor antigen as described herein. Non-cancer related indications associated with expression of a target antigen as described herein include, but are not limited to, *e.g.,* autoimmune disease, (*e.g*., lupus), inflammatory disorders (allergy and asthma) and transplantation. In some embodiments, the target antigen-expressing cells express, or at any time expressed, mRNA encoding the target antigen. In another embodiment, the target antigen -expressing cells produce the target antigen protein (*e.g*., wild-type or mutant), and the target antigen protein may be present at normal levels or reduced levels. In another embodiment, the target antigen -expressing cells produced detectable levels of a target antigen protein at one point, and subsequently produced substantially no detectable target antigen protein.

"Disease targeted by genetically modified cells" as used herein encompasses the targeting of any cell involved in any manner in any disease by the genetically modified cells of the disclosure, irrespective of whether the genetically modified cells target diseased cells or healthy cells to effectuate a therapeutically beneficial result. The genetically modified cells include, but are not limited to, genetically modified T-cells, NK cells, hematopoietic stem cells, pluripotent embryonic stem cells, induced pluripotent stem cells (iPSC) or embryonic stem cells. The genetically modified cells express the conventional SARs and novel backbones containing conventional SARs with accessory modules of the disclosure, which SARs may target any of the antigens expressed on the surface of target cells. Examples of antigens which may be targeted include, but are not limited to, antigens expressed on B-cells; antigens expressed on Carcinomas, sarcomas, lymphomas, leukemia, germ cell tumors, and blastomas; antigens expressed on various immune cells; and antigens expressed on cells associated with various hematologic diseases, autoimmune diseases, and/or inflammatory diseases. Other antigens that may be targeted will be apparent to those of skill in the art and may be targeted by the SARs of the disclosure in connection with alternate embodiments thereof.

The term "Dissociation constant (Kd)" is defined as the equilibrium constant of the dissociation of a receptor-ligand (*e.g*., binding domain - cognate) interaction.

As used herein a "diverse set of non-naturally occurring immune receptors" or "diverse set of SARs" or "diverse set of SARs" refers to a plurality of non-naturally occurring immune receptors having the same or different binding domains linked to a diverse set of signaling chains or "backbones" wherein each construct comprising a binding domain and a different signaling chain or "backbone" provide a diverse range of binding to a target antigen and/or varied expression levels. For example, depending upon the mutation composition of the constant domain (*e.g*., mutant TCRa+TCRb), the binding affinity of the binding domain to its target varies. In some embodiments, a bispecific and/or multispecific SAR with the backbone of a SIR, Ab-TCR or TFP of the disclosure comprises a binding affinity that is lower than a 2^{nd} generation CAR but greater than a wild-type TCR *(e.g.,* cTCR) with the same binding domain(s).

As used herein, an "epitope" is defined to be the portion of an antigen capable of eliciting an immune response, or the portion of an antigen that binds to an antibody or antibody fragment. Epitopes can be a protein sequence or subsequence.

The term "expression vector" refers to a vector comprising a recombinant polynucleotide comprising expression control sequences operatively linked to a nucleotide sequence to be expressed. Expression vectors include all those known in the art, including cosmids, plasmids (*e.g.,* naked or contained in liposomes) and viruses (*e.g.,* lentiviruses, retroviruses, adenoviruses, and adena-associated viruses) that incorporate the recombinant polynucleotide.

As used in the art, "Fc receptor" and "FcR" describe a receptor that binds to the Fc region of an antibody. In some embodiments, the FcR is a native sequence human FcR.

The term "functional portion" when used in reference to a SAR refers to any part or fragment of the SAR, which part or fragment retains the biological activity of the SAR of which it is a part (the parent SAR). Functional portions encompass, for example, those parts of a SAR that retain the ability to recognize target cells, or detect, treat, or prevent a disease, to a similar extent, the same extent, or to a higher extent, as the parent SAR. In reference to the parent SAR, the functional portion can comprise, for instance, about 10%, 25%, 30%, 50%, 68%, 80%, 90%, 95%, or more, of the parent SAR.

"Genetically modified cells", "redirected cells", "genetically engineered cells" or "modified cells" as used herein refer to cells that express a SAR of the disclosure. In some embodiments, the genetically modified cells comprise vectors that encode a SAR. In some embodiments, the genetically modified cells comprise vectors that encode a SAR and one or more accessory molecules (*e.g.,* PDL1, PDL2, crmA, MC159 *etc.*)*.*

"Hinge region" (HR) as used herein refers to the hydrophilic region which is between the antigen binding domain and the transmembrane domain of a SAR. The hinge regions include but are not limited to Fc fragments of antibodies or fragments or derivatives thereof, hinge regions of antibodies or fragments or derivatives thereof, CH2 regions of antibodies, CH3 regions of antibodies, artificial spacer sequences or combinations thereof. Examples of hinge regions include but are not limited to CD8a hinge, and artificial spacers made of polypeptides which may be as small as, for example, Gly3 or CH1 and CH3 domains of IgGs (such as human IgG4). Exemplary HR are provided in **Table 17** (SEQ ID NO: 1198-1204).

"Immune cell" as used herein refers to the cells of the mammalian immune system including but not limited to antigen presenting cells, B-cells, basophils, cytotoxic T-cells, dendritic cells, eosinophils, granulocytes, helper T-cells, leukocytes, lymphocytes, macrophages, mast cells, memory cells, monocytes, natural killer cells, neutrophils, phagocytes, plasma cells and T-cells.

The term "immune disorder" refers to a disease characterized by dysfunction of immune system. An autoimmune disease is a condition arising from an abnormal immune response to a normal body part. There are at least 80 types of autoimmune diseases.

"Immune effector cell," as that term is used herein, refers to a cell that is involved in an immune response, *e.g*., in the promotion of an immune effector response. Examples of immune effector cells include T cells, *e.g*., alpha/beta T cells and gamma/delta T cells, B cells, natural killer (NK) cells, natural killer T (NKT) cells, mast cells, and myeloic-derived phagocytes.

"Immune effector function" or "immune effector response," "effector function" refers to the specialized function of a differentiated cell. Effector function of a T-cell, for example, may be cytolytic activity or helper activity including the secretion of cytokines. For example, an immune effector function or response refers a property of a T or NK cell that promotes killing or the inhibition of growth or proliferation, of a target cell. In the case of a T cell, primary stimulation and co-stimulation are examples of immune effector function or response. In case of antigen presenting cells (*e.g*., dendritic cells) antigen presentation and cytokine secretion are examples of effector functions.

"Immune response" as used herein refers to immunities including but not limited to innate immunity, humoral immunity, cellular immunity, immunity, inflammatory response, acquired (adaptive) immunity, autoimmunity and/or overactive immunity.

An "intracellular signaling domain," (ISD) or "cytoplasmic domain" or "primary intracellular signaling domain" or "activation domain" as the term is used herein, refers to an intracellular signaling portion of a molecule. The intracellular signaling domain generates a signal that promotes an immune effector function of the cell. Examples of immune effector function include cytolytic activity and helper activity, including the secretion of cytokines. A primary intracellular signaling domain can comprise a signaling motif which is known as an immunoreceptor tyrosine-based activation motif or ITAM. Examples of ITAM containing primary cytoplasmic signaling sequences include, but are not limited to, those derived from CD3 zeta, common FcR gamma (FCER1G), Fc gamma RIIa, FcR beta (Fc Epsilon R1b), CD3 gamma, CD3 delta, CD3 epsilon, CD79a, CD79b, DAPl0, and DAP12.

The term "isolated" as used herein refers to molecules or biologicals or cellular materials being substantially free from other materials. In one aspect, the term "isolated" refers to nucleic acid, such as DNA or RNA, or protein or polypeptide (*e.g*., an antibody or derivative thereof), or cell or cellular organelle, or tissue or organ, separated from other DNAs or RNAs, or proteins or polypeptides, or cells or cellular organelles, or tissues or organs, respectively, that are present in the natural source. The term "isolated" also refers to a nucleic acid or peptide that is substantially free of cellular material, viral material, or culture medium when produced by recombinant DNA techniques, or chemical precursors or other chemicals when chemically synthesized. Moreover, an "isolated nucleic acid" is meant to include nucleic acid fragments which are not naturally occurring as fragments and would not be found in the natural state. The term "isolated" is also used herein to refer to polypeptides which are isolated from other cellular proteins and is meant to encompass both purified and recombinant polypeptides. The term "isolated" is also used herein to refer to cells or tissues that are isolated from other cells or tissues and is meant to encompass both, cultured and engineered cells or tissues.

As used herein, the term "linker" (also "linker domain" or "linker region") refers to an oligo or a polypeptide (or an oligo encoding the polypeptide) that joins together two or more domains or regions of a SAR polynucleotide or polypeptide, respectively, disclosed herein. The linker can be anywhere from 1 to 500 amino acids in length or 3 to 1500 nucleotide in length. In an embodiment, the linker is 1 or more amino acids (*e.g.,* 1, 2, 3, 4, 5, 6, 7, 8, 9, 10, 15, 20, 25, 30, 35, 40, 45, 50, 55, 60, 65, 70, 75, 80, 85, 90, 95, 100, 105, 110, 115, 120, 125, 130, 135, 140, 145, 150, 160, 170, 180, 190, 200, 210, 220, 230, 250, 275, 300, 325, 350, 375, 400, 450, 500 amino acids and any number in between) in length. In an embodiment, the linker is between 1 and 100, between 1 and 125, between 1 and 150, between 1 and 200, between 1 and 250, between 1 and 300, between 1 and 350, between 1 and 400, between 1 and 450, between 3 and 500 amino acids in length. In an embodiment, the SARs of the disclosure may comprise one or more than one linker (*e.g.,* 2, 3, 4 or more).

A "long linker" or "long linker domain" is a linker that is between 25 to 500 amino acid in length. In an embodiment, a long linker is about 25, 30, 35, 40, 45, 50, 55, 60, 65, 70, 75, 80, 85, 90, 95, 100, 105, 110, 115, 120, 125, 130, 135, 140, 145, 150, 160, 170, 180, 190, 200, 210, 220, 230, 250, 275, 300, 325, 350, 375, 400, 450, 500 amino acids and any number in between in length. In an embodiment, a long linker is between 25 and 125 amino acids in length. In an embodiment, a long linker is between 50 and 150 amino acids in length. In an embodiment, a long linker is between 75 and 175 amino acids in length. In an embodiment, a long linke is between 100 and 200 amino acids in length. In an embodiment, a long linker is between 120 and 220 amino acids in length. In an embodiment, a long linker is between 100 and 300 amino acids in length.

In an embodiment, the linker encodes for or comprises of an immunoglobulin (Ig) domain or an Ig like domain or a fragment thereof. The terms "Ig domain", "Ig linker domain", "Ig like domains" or "Ig like linker domains" are used interchangeably in this disclosure. The immunoglobulin domain is a type of protein domain that consists of a 2-layer sandwich of 7-9 antiparallel β-strands arranged in two β-sheets with a Greek key topology, consisting of about 125 amino acids. The Ig domains can be classified as IgV, IgC1, IgC2, or IgI. IgV domains with 9 beta strands are generally longer than IgC domains with 7 beta strands. In an embodiment, the linker comprises an IgV domain or a fragment thereof. In an embodiment, the linker comprises an IgC domain or a fragment thereof. Ig domains are found in immunoglobulins, T cell receptor chains, class I MHC, class II MHC, β2 microglobulin, coreceptors (*e.g.,* CD4, CD8, CD19 *etc.*)*,* antigen receptor accessory molecules (*e.g.,* CD3γ, CD3δ, CD3ε, CD79a, CD79b), costimulatory or inhibitory molecules (*e.g*., CD28, CD80, CD86), NK cell receptors (*e.g*., KIR), Leukocyte immunologlobulin like receptor (LILR), IgSF CAMs (*e.g.,* NCAM, ICAM, CD2 *etc.*), cytokine receptors (*e.g.,* IL-1R, CSF-1R *etc.*), growth factor receptors (*e.g*., PDGFR), Receptor tyrosine kinases and phosphatases, Ig binding receptors, cytoskeleton proteins (*e.g.,* titin, palladin *etc.*) and other proteins (*e.g.,* CD147, CD90 *etc.*)*.* Exemplary Ig linker domains are IgCL (SEQ ID NO: 1142) and IgG1-CH1 (SEQ ID NO: 1143). Additional exemplary Ig linkers are presented in **Table 13** (SEQ ID NO (PRT): 11832-11865). In an embodiment, the linker possesses an E set domain. An E set domain is an "Early" Ig-like fold families possibly related to the immunoglobulin and/or fibronectin type III superfamilies. In an embodiment, the linker possesses a Fibronectin type III domain.

The linker can be a flexible linker. The term "flexible polypeptide linker" as used herein refers to a peptide linker that consists of or consists mostly of amino acids such as glycine and/or serine residues used alone or in combination, to link polypeptide chains together (*e.g*., variable heavy and variable light chain regions together). In one embodiment, the flexible polypeptide linker is a Gly/Ser linker and comprises the amino acid sequence (Gly-Gly-Gly-Ser)ₙ, (*e.g.,* SEQ ID NO:11715) where n is a positive integer equal to or greater than 1. For example, n=l, n=2, n=3. n=4, n=5 and n=6, n=7, n=8, n=9 and n=l0. In one embodiment, the flexible polypeptide linkers include, but are not limited to, (Gly₄Ser)₄ or (Gly₄Ser)₃.

In some embodiments the "linker" is cleavable or non-cleavable. Unless specified otherwise, the term "linker" used herein means a non-cleavable linker. Said non-cleavable linkers may be composed of flexible residues which allow freedom of motion of adjacent protein doamins relative to one another. Non-limiting examples of such residues include glycine and serine. In some embodiments, linkers include non-flexible residues. Examples of cleavable linkers include 2A linkers (for example T2A), 2A-like linkers or functional equivalents thereof and combinations thereof. In some embodiments, the linkers include the picornaviral 2A-like linker, CHYSEL sequences of porcine teschovirus (P2A), *Thosea asigna* virus (T2A) or combinations, variants and functional equivalents thereof. In some embodiments, the linker sequences may comprise a motif that results in cleavage between the 2A glycine and the 2B proline (see, *e.g.,* T2A sequence). The nucleic sequences of several exemplary cleavable linkers are provided in SEQ ID NO: 1233 to SEQ ID NO: 1238 and amino acid sequences of several exemplary linkers are provided in SEQ ID NO: 11923 to SEQ ID NO: 11928. Other cleavable linkers that may be used herein are readily appreciated by those of skill in the art.

In an embodiment, a Ser-Gly-Ser-Gly (SGSG) motif (SEQ ID NOs: 11929) is also added upstream of the cleavable linker sequences to enhance the efficiency of cleavage. A potential drawback of the cleavable linkers is the possibility that the small 2A tag left at the end of the N-terminal protein may affect protein function or contribute to the antigenicity of the proteins. To overcome this limitation, in some embodiments, a furine cleavage site (RAKR) (SEQ ID NO: 11931) is added upstream of the SGSG motifs to facilitate cleavage of the residual 2A peptide following translation.

A linker may be a protease cleavable linker. Exemplary protease cleavable linkers are presented in **Table 19.**

The term "lentivirus" refers to a genus of the Retroviridae family. Lentiviruses are unique among the retroviruses in being able to infect non-dividing cells; they can deliver a significant amount of genetic information into the DNA of the host cell, so they are one of the most efficient methods of a gene delivery vector. HIV, SIV, and FIV are all examples of lenti viruses.

The term "lentiviral vector" refers to a vector derived from at least a portion of a lentivirus genome, including especially a self-inactivating lentiviral vector as provided in Milone et al., Mol. Ther. 17(8): 1453-1464 (2009). Other examples of lentivirus vectors that may be used in the clinic include but are not limited to, *e.g.,* the LENTIVECTOR^{®} gene delivery technology from Oxford BioMedica, the LENTIMAX^{™} vector system from Lentigen and the like. Nonclinical types of lentiviral vectors are also available and would be known to one skilled in the art. Other examples of lentivirus vectors are pLENTI-EF1α (SEQ ID NO: 1), pLENTI-EF1α-DWPRE (SEQ ID NO: 2), pCCLc-MNDU3-WPRE (SEQ ID NO: 4) and pCCLc-MNDU3-Eco-Nhe-Sal-WPRE (SEQ ID NO: 5). In an exemplary embodiment, the nucleic acid fragment encoding a SAR, or SAR plus accessory module(s), or the accessory module(s) can be cloned between the *Nhe* I and *Sal* I sites present in the pLENTI-EF1α and the pCCLc-MNDU3-Eco-Nhe-Sal-WPRE vectors using methods known in the art.

"Mammal" as used herein refers to any member of the class *Mammalia,* including, without limitation, humans and nonhuman primates such as chimpanzees and other apes and monkey species; farm animals such as cattle, sheep, pigs, goats and horses; domestic mammals such as dogs and cats; laboratory animals including rodents such as mice, rats and guinea pigs, and the like.

"Native" or "Naturally occurring" or "endogenous" as used herein refers to a gene, protein, nucleic acid (*e.g.,* DNA, RNA *etc.*) or fragment thereof that is native to a cell or is naturally expressed in a cell. Thus, a native or endogenous TCRα chain polypeptide of a T cell consists of a variable domain (Vα) joined to a TCRα constant chain. The native or endogenous TCRα chain precursor polypeptide also consists of an amino-terminal signal peptide that is cleaved from the mature polypeptide.

The term "NIK agonist" as used herein refers to an agent that activates the activity of NF-κB inducing kinase. In an embodiment, a NIK agonist is a SMAC mimetic.

The term "SMAC mimetic" as used herein refers to an agent that mimics the actaivity of SMAC/DIABLO protein.

The term "near the N-terminus" as used herein means within the N-terminal 30 amino acids. For example, the term "an AABD operably linked to the N-terminus or near the N-terminus of a vL and/or vH domain", mean an AABD that is operably linked at the N-terminus of a vL or a vH fragment or operably linked to the N-terminal 2, 3, 4, 5, 6, 7, 8, 9, 10, 11, 12, 13, 14, 15, 16, 17, 18, 19, 25 or 30 amino acid comprising the vL or the vH domain. Similarly, the term "an AABD operably linked to the N-terminus or near the N-terminus of a Va and/or Vb domain", mean an AABD that is operably linked at the N-terminus of a Va or a Vb fragment or operably linked to the N-terminal 2, 3, 4, 5, 6, 7, 8, 9, 10, 11, 12, 13, 14, 15, 16, 17, 18, 19 , 20, 21, 22, 23, 24, 25 or 30 amino acid comprising the Va or the Vb domain. An AABD of the disclosure may be operably linked to or near the N-terminus of another domain either directly or via an intervening linker sequence.

As used herein a "non-naturally occurring agent" or "non-native" or "exogenous" refers to an agent that is not naturally expressed in a cell. Stated another way, the non-naturally occurring agent is "engineered" to be expressed in a cell. A non-naturally occurring agent may be a cloned version of a naturally occurring agent. Exemplary non-naturally occurring agents include SARs (*e.g*., CAR, SIRs, Ab-TCRs, TFPs, recombinant TCR, NEMO-K277A, vFLIP-K13 and K13-opt). A non-naturally occurring agent may be expressed into a cell using techniques of gene transfer known in the art, such as lentiviral or retroviral mediated gene transfer. A non-naturally occurring agent may be expressed in an immune cell using an exogenous promoter (*e.g*., EF1α promoter) or an endogenous promoter (*e.g.,* TCRα promoter).

As used herein a "non-naturally occurring immune receptor" or "exogenous immune receptor" refers to an immune receptor that is not naturally expressed in an immune cell. Stated another way, the non-naturally occurring immune receptor is "engineered" to be expressed in an immune cell. A non-naturally occurring immune receptor may be a cloned version of a naturally occurring immune receptor. Alternatively, a non-naturally occurring immune receptor may be a chimeric receptor that is produced using recombinant molecular biology techniques. Exemplary non-naturally occurring immune receptors include SARs, SIR, Ab-TCRs, TFPs and recombinant TCR. A non-naturally occurring immune receptor may be introduced into an immune cell using techniques of gene transfer known in the art, such as lentiviral or retroviral mediated gene transfer. A non-naturally occurring immune receptor may be expressed in an immune cell using an exogenous promoter (*e.g.,* EF1α promoter) or an endogenous promoter (*e.g*., TCRα promoter).

As used herein a "non-scFv antigen binding domain" or "non-scFv based antigen binding domain" refers to an antigen binding domain that is not comprised of a single chain variable fragment (*i.e.,* vL-linker-vH or vH-linker-vL). Exemplary non-scFv based antigen binding domains include but are not limied to vHH, FHVH, SVL, non-immunoglobulin antigen binding scaffolds (*e.g.,* DARPIN, Centyrin, affibodies *etc.*) ligand-binding domain of a receptor, receptor binding domain of a ligand, adaptor binding domains (*e.g.,* RZIP, EZIP, E4, K4, D domains, NKG2D-YA, NKG2D-AF, CD16A-V158, CD32 or CD64 *etc.*) and auto-antigens. AABD are non-scFv based antigen binding domains.

As used herein a "non-naturally occurring TCR antigen binding domain" or "exogenous TCR antigen binding domain" refers to a binding domain operably linked to a TCR constant region that is chimeric and non-naturally occurring with respect to a TCR present in nature. Stated another way, the non-naturally occurring TCR antigen binding domain is "engineered" using recombinant molecular biology techniques to be operably linked to a TCR and moreover, that the antigen binding domain is obtain or derived from a molecule that is distinct from a TCR found in nature. An antigen binding domain that is distinct from a TCR in nature includes antibody vH and vL fragments, humanized antibody fragments, chimeric antibody fragments, receptor ligands, and the like.

The term "NKT cells" as used here refers to a subset of T cells that coexpress an αβ T-cell receptor, but also express a variety of molecular markers that are typically associated with NK cells, such as NK1.1. The best-known NKT cells differ from conventional αβ T cells in that their T-cell receptors are far more limited in diversity ('invariant' or 'type 1' NKT). They and other CD1d-restricted T cells ('type 2' NKT) recognize lipids and glycolipids presented by CD1d molecules, a member of the CD1 family of antigen-presenting molecules, rather than peptide-major histocompatibility complexes (MHCs).

The term "operably linked" or "functionally linked" or "operably joined" or "operably attached" refers to functional linkage or association between a first component and a second component such that each component can be functional. For example, operably linked includes the association between a regulatory sequence and a heterologous nucleic acid sequence resulting in expression of the latter. For example, a first nucleic acid sequence is operably linked with a second nucleic acid sequence when the first nucleic acid sequence is placed in a functional relationship with the second nucleic acid sequence. In the context of two different modules of a molecule, the first module is operably linked to the second module when their nucleotide sequences are joined in frame. In the context of two polypeptides that are operably linked a first polypeptide functions in the manner it would independent of any linkage and the second polypeptide functions as it would absent a linkage between the two. The terms "operably linked" or "operably attached" are used interchangeably with the terms "linked", "attached" or "joined".

"Percent identity" in the context of two or more nucleic acids or polypeptide sequences, refers to two or more sequences that are the same. Two sequences are "substantially identical" if two sequences have a specified percentage of amino acid residues or nucleotides that are the same (*e.g.,* 60% identity, optionally 70%, 71%. 72%. 73%, 74%, 75%, 76%, 77%, 78%, 79%, 80%,81%, 82%, 83%, 84%, 85%, 86%, 87%, 88%, 89%, 90%, 91%, 92%, 93%, 94%, 95%, 96%, 97%, 98%, 99% identity over a specified region, or, when not specified, over the entire sequence), when compared and aligned for maximum correspondence over a comparison window, or designated region as measured using one of the following sequence comparison algorithms or by manual alignment and visual inspection. Optionally, the identity exists over a region that is at least about 50 nucleotides (or 10 amino acids) in length, or more typically over a region that is 100 to 500 or 1000 or more nucleotides (or 20, 50, 200 or more amino acids) in length.

For sequence comparison, generally one sequence acts as a reference sequence, to which test sequences are compared. Two examples of algorithms that can be used for determining percent sequence identity and sequence similarity are the BLAST and BLAST 2.0 algorithms, which are described in Altschul et al., (1977) Nuc. Acids Res. 25:3389-3402; and Altschul et al., (1990) J. Mol. Bioi. 215:403-410, respectively. Software for performing BLAST analyses is publicly available through the National Center for Biotechnology Information.

The term "retrovirus vector" refers to a vector derived from at least a portion of a retrovirus genome. Examples of retrovirus vector include MSCVneo, MSCV-pac (or MSCV-puro), MSCV-hygro as available from Addgene or Clontech.

The term "Sleeping Beauty Transposon" or "Sleeping Beauty Transposon Vector" refers to a vector derived from at least a portion of a Sleeping Beauty Transposon genome.

The term "single chain variable region" or "scFv" refers to a fusion protein comprising at least one antibody fragment comprising a variable region of a light chain and at least one antibody fragment comprising a variable region of a heavy chain, wherein the light and heavy chain variable regions are contiguously linked, *e.g.,* via a synthetic linker, *e.g.,* a short flexible polypeptide linker, and capable of being expressed as a single chain polypeptide, and wherein the scFv retains the specificity of the intact antibody from which it is derived. Unless specified, as used herein an scFv may have the vL and vH variable regions in either order, *e.g*., with respect to the N-terminal and C-terminal ends of the polypeptide, the scFv may comprise vL-linker-vH or may comprise vH-linker-vL. In this disclosure, a scFv is also described as vL-Gly-Ser-Linker-vH. Alternatively, a scFv is also described as (vL+vH) or (vH+vL).

The term "signaling domain" refers to the functional region of a protein which transmits information within the cell to regulate cellular activity via defined signaling pathways by generating second messengers or functioning as effectors by responding to such messengers.

The term "Synthetic Antigen Receptor" or "SAR" refers to a non-natural polypeptide, which when expressed in an effector cell, provides the cell with specificity for a target cell, typically a cancer cell. SARs are engineered receptors that graft an antigen specificity onto cells (*e.g.,* T cells, NK cells, NKT cells, monocytes/macrophages, B lymphocytes or combination thereof) thus combining the antigen binding properties of the antigen binding domain with the effector function of the cells. Exemplary effector functions for T and NK cells may include target-cell lysis, cytokine production and self renewal. For monocytes/macrophages, effector function may include phagocytosis of the target cell. SAR, as the term is defined herein, covers first generation CARs, 2^{nd} generation CARs, 3^{rd} generation CARs, and next generation CARs, such as Synthetic immune receptors (SIR), cTCR, Ab-TCR, AABD-TCR, TFP (*e.g.,* TFPε, TFPγ, TFPδ), TAC, recombinant TCR and the like. A SAR can be single chain, double chain or one and half chain. A SAR can be unispecific, bispecific or multispecific. A SAR may have one or more antigen binding domains. A SAR may have the backbone of a CAR (*e.g.,* a 2^{nd} generation CAR), SIR, cTCR, Ab-TCR, AABD-TCR, TFP, TAC or a TCR. SARs, as referred to herein, encompass artificial T cell receptors, chimeric T cell receptors or chimeric immunoreceptors. However, the term SAR is not limited to a T cell receptor or to an immunoreceptor. A SAR may or may not have a signaling domain of its own. An exemplary SAR that lacks a signaling domain of its own is a SAR with the backbone of an SIR or a TCR, which transmit a signal via recruitment of other signaling proteins that possess a signaling domain. A SAR can be any non-natural antigen binding receptor (ABR). The term "antigen binding domain or "antigen-specific targeting domain" as used herein refers to the region of the SAR which targets and binds to specific antigens. The antigen binding domain of a SAR may comprise of an antibody or antibody fragment (*e.g*., vL, vH, Fv, Fab, scFv, vHH, single domain antibody *etc.*)*,* a T cell receptor (TCR) or a fragment of a TCR (*e.g.,* Va, Vb, Vg, Vd or a single variable domain TCR *etc.*). The antigen binding domain(s) of a SAR may comprise of one or more autonomous antigen binding domains (AABD) such as a non-immunologlobulin antigen binding scaffold (*e.g.,* DARPIN, Centyrin, affibody, D domain *etc.*), a receptor binding domain of a ligand, the ligand binding domain of a receptor, an autoantigen, an adaptor binding domain (*e.g.,* RZIP, EZIP, E4, K4, NKG2D-AF *etc.*), an Fc binding domain of a receptor (*e.g.,* Fc binding region of CD16A-V158, CD32, CD64 *etc.*), or combination thereof. A SAR may have one or more than one antigen binding domains. The different modules and domains of a SAR may be connected by one or more linkers. The SAR may also comprise one or more epitope tags or mimotopes, which may be used to detect the expression of the SAR, to isolate and purify SAR expressing cells, to monitor persistence of SAR-expressing cells and to deplete SAR-expressing cells. In an embodiment, the epitope and/or mimotops are targeted by an antibody, an antibody fragment, an antibody derivative (*e.g*., antibody drug conjugate) to serve as a "suicide switch" to diminish or eliminate SAR-expressing cells in case of toxicity. When a SAR is expressed in a host cell, this domain forms the extracellular domain (ectodomain).

The term SVH domain as used herein refers to a single human V_{H} domain antibody (V_{H} sdAb). These terms are thus used interchangeably. The term SVH is also used interchangeably with independent vH domains. A SVH is an example of an autonomous antigen binding domain (AABD). An exemplary SVH is a fully human vH domain (FHVH) presented in SEQ ID NO (DNA): 827-828 and SEQ ID NO (PRT): 11517-11518. Another exemplary SVH is a chVH domain presented in SEQ ID NO (DNA): 830-831 and SEQ ID NO (PRT): 11520-11521. Another exemplary SVH is an aVH domain presented in SEQ ID NO (DNA): 850-851 and SEQ ID NO (PRT): 11540-11541. The SEQ ID numbers of other exemplary SVH domains are presented in Table 5. Additional SVH domains that can be used in the construction of the SARs of the disclosure are provided in WO2016062988, WO2016113556, WO2017191476, WO2018039180, WO2019006072, WO2018237037, WO2018119215, WO2019126756, WO2019055689 and WO2020018922, which are incorporated in their entirety by reference herein.

The term SVL domain as used herein refers to a single human vL domain antibody (vL sdAb). These terms are thus used interchangeably. The term SVL is also used interchangeably with independent vL domains. A SVL is an example of an autonomous antigen binding domain (AABD).

As used herein, "single variable domain T cell receptor" or "svd-TCR" refer to the variable domain of a T cell receptor capable of specifically binding to an epitope in the absence of a second TCR variable domain. For example, an svd-TCR comprising a Vb variable domain is capable of binding to an epitope independent of and/or in the absence of a Va variable domain, and an svd-TCR comprising a Va variable domain is capable of binding to an epitope independent of and/or in the absence of a nb variable domain. Illustrative epitopes recognized by svd-TCRs include peptide:MHC complexes (pMHC complexes).

The term "Synthetic Immune Receptor" or alternatively a "SIR" refers to a set of polypeptides, typically two in some embodiments, which when expressed in an effector cell, provides the cell with specificity for a target cell, typically a cancer cell, and with intracellular signal generation. SIRs represent next generation CAR platforms that are described in WO 2018/102795 A1 which is incorporated herein by reference. In a typical embodiment, a SIR comprises one or more antigen binding domains (*e.g*., antibody or antibody fragment, a ligand or a receptor) that bind to antigens as described herein, and are joined to one or more T cell receptor constant chains or regions via an optional linker. In some embodiments, the set of polypeptides are contiguous with each other. In some embodiments, a SIR comprises two or more sets of two or more polypeptides. The polypeptides of each set of SIR are contiguous with each other (functional polypeptide unit 1) but are not contiguous with the polypeptides of the other set (functional polypeptide unit 2). In some embodiments, the T cell receptor constant chains (or regions) of the SIR is chosen from the constant chain of human T cell receptor-alpha (TCR-alpha or TCRα or TCRa or hTCR-alpha or hTCRα or hTCRa or Cα), human T cell receptor-beta1(TCR-beta1 or TCRβ1 or TCRb1 or hTCR-beta1 or hTCRβ1 or hTCRb1 or Cβ1), human T cell receptor-beta 2 (TCR-beta2 or TCRβ2 or TCRb2 or hTCR-beta2 or hTCRβ2 or hTCRb2 or Cβ2 also designated TCR-beta, TCRβ or TCRb or Cβ), human Pre-T cell receptor alpha ((pr*etc*.R-alpha or pr*etc*.Rα or pr*etc*.Ra or preCα), human T cell receptor-gamma (TCR-gamma or TCRγ or TCRg or or hTCR-gamma or hTCRγ or hTCRg or hTCRγ1 or hTCRgamma1, or Cγ), or human T cell receptor-delta (TCR-delta or TCRd or TCRδ or hTCR-delta or hTCRd or hTCRδ or Cδ). In some embodiments, the TCR constant chains of SIR are encoded by their wild-type nucleotide sequences while in other aspects the TCR constant chains of SIR are encoded by the nucleotide sequences that are not wild-type. In some embodiments, the TCR constant chains of SIR are encoded by their codon optimized sequences. In some embodiments, the TCR constant chains of SIR encode for the wild-type polypeptide sequences while in other embodiments the TCR constant chains of SIR encoded for polypeptides that carry one or more mutations. In some embodiments, the TCR constant chains of SIR are encoded by their codon optimized sequences that carry one or more mutations. The disclosure also covers deletion mutants of TCR constant chains that retain at least one of the biological and functional properties of the corresponding full-length TCR chain. A SIR that comprises an antigen binding domain (*e.g.,* a scFv, or vHH) that targets a specific tumor maker "X", such as those described herein, is also referred to as X-SIR or XSIR. For example, a SIR that comprises an antigen binding domain that targets CD19 is referred to as CD19-SIR or CD19SIR. The TCR constant chain/domain of a SIR can be derived from the same species in which the SIR will ultimately be used. For example, for use in humans, it may be beneficial for the TCR constant chain of the SIR to be derived from or comprised of human TCR constant chains. However, in some instances, it is beneficial for the TCR constant chain to be derived from the same species in which the SIR will ultimately be used in, but modified to carry amino acid substitutions that enhance the expression of the TCR constant chains. For example, for use in humans, it may be beneficial for the TCR constant chain of the SIR to be derived from or comprised of human TCR constant chains but in which certain amino acids are replaced by the corresponding amino acids from the murine TCR constant chains. Such murinized TCR constant chains provide increased expression of the SIR. The SIR or functional portion thereof, can include additional amino acids at the amino or carboxy terminus, or at both termini, which additional amino acids are not found in the amino acid sequence of the TCR or antigen binding domain which make up the SIR. Desirably, the additional amino acids do not interfere with the biological function of the SIR or functional portion, *e.g.,* recognize target cells, detect cancer, treat or prevent cancer, *etc.* More desirably, the additional amino acids enhance the biological activity, as compared to the biological activity of the parent SIR. This disclosure provides bispecific, biparatopic and mutlispecific SARs with the backbone of SIR comprising one or more AABDs. The AABD domains of the SARs of the disclosure with the backbone of SIR can be fully human, humanized or non-human. In an embodiment, the disclosure provides SIR comprising one or more fully human vH domains. In an embodiment, the disclosure provides Ab-TCR comprising one or more fully human vL domains.

The term "stimulation," refers to a primary response induced by binding of a stimulatory molecule (*e.g.,* a TCR/CD3 complex) with its cognate ligand (or target antigen) thereby mediating a signal transduction event, such as, but not limited to, signal transduction via the TCR/CD3. Stimulation can mediate altered expression of certain molecules.

The term "stimulatory molecule," refers to a molecule expressed by an immune cell (*e.g.,* T cell, NK cell, B cell) that provides the cytoplasmic signaling sequence(s) that regulate activation of the immune cell in a stimulatory way for at least some aspect of the immune cell signaling pathway.

The term "subject" is intended to include living organisms in which an immune response can be elicited (*e.g.,* any domesticated mammals or a human). The terms "subject" or "individual" or "animal" or "patient" are used interchangeably herein to refer to any subject, particularly a mammalian subject, for whom administration of a composition or pharmaceutical composition of the disclosure is desired. Mammalian subjects include humans, non-human primates, dogs, cats, guinea pigs, rabbits, rats, mice, horses, cattle, cows, and the like, with humans being preferred.

"Switch domain," or a "dimerization domain" as used herein, typically refers to a polypeptide-based entity that, in the presence of a dimerization molecule, associates with another switch domain. The association results in a functional coupling of a first entity linked to, *e.g.,* fused to, a first switch domain, and a second entity linked to, *e.g.,* fused to, a second switch domain. A first and second switch domain are collectively referred to as a dimerization switch.

The term "target antigen" refers to an antigen that is bound by an antigen binding agent (*e.g.,* an antibody, antibody fragment, SAR-T cell, SAR adaptor *etc.*)*.* The term "target antigen expressing cell" refers to a cell, *i.e.,* a target cell that expresses an antigen bound by an antigen binding agent. In an embodiment, a cell expressing a SAR of the disclosure undergoes activation, proliferation and induces effector functions (*e.g*., cytokine production, cytotoxicity *etc.*) when it binds its target antigen expressing cell either directly or indirectly via a SAR adaptor. Non-limiting examples of target antigens are listed in **Table B.** A SAR of the disclosure may bind one or more (*e.g.,* 2, 3, 4, 5 or more) target antigens listed in **Table B** either directly or via SAR adaptors described herein.

**TABLE B**

| **TABLE B: Exemplary Antigens Targeted by Antibodies, antibody fragments (*e.g*., scFv), AABD (*e.g.,* FHVH, vHH, DARPIN, Centryin, D domains, Adaptors *etc.*) and SARS of the disclosure** |
|---|
| CD5; CD19; CD123; CD22; CD30; CD171; CS1 (also referred to as CD2 subset 1, CRACC, SLAMF7, CD319, and 19A24); C-type lectin-like molecule-1 (CLL-1 or CLECL1); CD33; epidermal growth factor receptor variant III (EGFRviii); ganglioside G2 (GD2); ganglioside GD3 (aNeu5Ac(2-8)aNeu5Ac(2-3)bDGalp(l-4 )bDGlcp(l-l)Cer); TNF receptor family member B cell maturation (BCMA); Tn antigen ((Tn Ag) or (GalNAcα-Ser/Thr)); prostate-specific membrane antigen (PSMA); Receptor tyrosine kinase-like orphan receptor 1 (ROR1); Fms Like Tyrosine Kinase 3 (FLT3); Tumor-associated glycoprotein 72 (TAG72); CD38; CD44v6; a glycosylated CD43 epitope expressed on acute leukemia or lymphoma but not on hematopoietic progenitors, a glycosylated CD43 epitope expressed on non-hematopoietic cancers, Carcinoembryonic antigen (CEA); Epithelial cell adhesion molecule (EPCAM); B7H3 (CD276); KIT (CD117); Interleukin-13 receptor subunit alpha-2 (IL-13Ra2 or CD213A2); Mesothelin; Interleukin 11 receptor alpha (IL-llRa); prostate stem cell antigen (PSCA); Protease Serine 21 (Testisin or PRSS21); vascular endothelial growth factor receptor 2 (VEGFR2); Lewis(Y) antigen; CD24; Platelet-derived growth factor receptor beta (PDGFR-beta); Stage-specific embryonic antigen-4 (SSEA-4); CD20; Folate receptor alpha (FRa or FR1); Folate receptor beta (FRb); Receptor tyrosine-protein kinase ERBB2 (Her2/neu); Mucin 1, cell surface associated (MUC1); epidermal growth factor receptor (EGFR); neural cell adhesion molecule (NCAM); Prostase; prostatic acid phosphatase (PAP); elongation factor 2 mutated (ELF2M); Ephrin B2; fibroblast activation protein alpha (FAP); insulin-like growth factor 1 receptor (IGF-I receptor), carbonic anhydrase IX (CAlX); Proteasome (Prosome, Macropain) Subunit, Beta Type, 9 (LMP2); glycoprotein 100 (gpl00); oncogene fusion protein consisting of breakpoint cluster region (BCR) and Abelson murine leukemia viral oncogene homolog 1 (Abl) (bcr-abl); tyrosinase; ephrin type-A receptor 2 (EphA2); sialyl Lewis adhesion molecule (sLe); ganglioside GM3 (aNeu5Ac(2-3)bDClalp(l- 4)bDGlcp(l-1)Cer); transglutaminase 5 (TGS5); high molecular weight-melanoma associated antigen (HMWMAA); o-acetyl-GD2 ganglioside (OAcGD2); tumor endothelial marker 1 (TEM1/CD248); tumor endothelial marker 7-related (TEM7R); claudin 6 (CLDN6); thyroid stimulating hormone receptor (TSHR); G protein coupled receptor class C group 5, member D (GPRC5D); chromosome X open reading frame 61 (CXORF61); CD97; CD179a; anaplastic lymphoma kinase (ALK); Polysialic acid; placenta-specific 1 (PLAC1); hexasaccharide portion of globoH glycoceramide (GloboH); mammary gland differentiation antigen (NY-BR-1); uroplakin 2 (UPK2); Hepatitis A virus cellular receptor 1 |
| (HAVCR1); adrenoceptor beta 3 (ADRB3); pannexin 3 (PANX3); G protein-coupled receptor 20 (GPR20); lymphocyte antigen 6 complex, locus K 9 (LY6K); Olfactory receptor 51E2 (OR51E2); TCR Gamma Alternate Reading Frame Protein (TARP); Wilms tumor protein (WT1); Cancer/testis antigen 1 (NY-ESO-1); Cancer/testis antigen 2 (LAGE-1a); Melanoma-associated antigen 1 (MAGE-A1); ETS translocation-variant gene 6, located on chromosome 12p (ETV6-AML); sperm protein 17 (SPA17); X Antigen Family, Member 1A (XAGEl); angiopoietin-binding cell surface receptor 2 (Tie 2); melanoma cancer testis antigen-1 (MAD-CT-1); melanoma cancer testis antigen-2 (MAD-CT-2); Fos-related antigen 1; tumor protein p53 (p53); p53 mutant; prostein; survivin; telomerase; prostate carcinoma tumor antigen-1 (PCT A-1 or Galectin 8), melanoma antigen recognized by T cells 1 (MelanA or MARTI); Rat sarcoma (Ras) mutant; human Telomerase reverse transcriptase (hTERT); sarcoma translocation breakpoints; melanoma inhibitor of apoptosis (ML-IAP); ERG (transmembrane protease, serine 2 (TMPRSS2) ETS fusion gene); N-Acetyl glucosaminyl-transferase V (NA17); paired box protein Pax-3 (PAX3); Androgen receptor; Cyclin Bl; v-myc avian myelocytomatosis viral oncogene neuroblastoma derived homolog (MYCN); Ras Homolog Family Member C (RhoC); Tyrosinase-related protein 2 (TRP-2); Cytochrome P450 lB 1 (CYPlB 1); CCCTC-Binding Factor (Zinc Finger Protein)-Like (BORIS or Brother of the Regulator oflmprinted Sites), Squamous Cell Carcinoma Antigen Recognized By T Cells 3 (SART3); Paired box protein Pax-5 (PAX5); proacrosin binding protein sp32 (OY-TESl); lymphocyte-specific protein tyrosine kinase (LCK); A kinase anchor protein 4 (AKAP-4); synovial sarcoma, X breakpoint 2 (SSX2); Receptor for Advanced Glycation Endproducts (RAGE-1); renal ubiquitous 1 (RUl); renal ubiquitous 2 (RU2); legumain; human papilloma virus E6 (HPV E6); human papilloma virus E7 (HPV E7); intestinal carboxyl esterase; heat shock protein 70-2 mutated (mut hsp70-2); CD79a; CD79b; CD72; Leukocyte-associated immunoglobulin-like receptor 1 (LAIRl); Fc fragment of IgA receptor (FCAR or CD89); Leukocyte immunoglobulin-like receptor subfamily A member 2 (LILRA2); CD300 molecule-like family member f (CD300LF); C-type lectin domain family 12 member A (CLEC12A); bone marrow stromal cell antigen 2 (BST2); EGF-like module-containing mucin-like hormone receptor-like 2 (EMR2); lymphocyte antigen 75 (LY75); Glypican-3 (GPC3); Fc receptor-like 5 (FCRL5); and immunoglobulin lambda-like polypeptide 1 (IGLLl), MPL, Biotin, c-MYC epitope Tag, CD34, LAMP1 TROP2, GFRalpha4, CDH17, CDH6, NYBR1, CDH19, CD200R, Slea (CA19.9; Sialyl Lewis Antigen); Fucosyl-GM1, PTK7, gpNMB, CDH1-CD324, DLL3, CD276/B7H3, IL11Ra, IL13Ra2, CD179b-IGLl1, TCRgamma-delta, NKG2D, CD32 (FCGR2A), Tn ag, Tim1-/HVCR1, CSF2RA (GM-CSFR-alpha), TGFbetaR2, Lews Ag, TCR-beta1 chain, TCR-beta2 chain, TCR-gamma chain, TCR-delta chain, FITC, Leutenizing hormone receptor (LHR), Follicle stimulating hormone receptor (FSHR), Gonadotropin Hormone receptor (CGHR or GR), CCR4, GD3, SLAMF6, SLAMF4, HIV1 envelope glycoprotein, HTLV1-Tax, CMV pp65, EBV-EBNA3c, KSHV K8.1, KSHV-gH, influenza A hemagglutinin (HA), GAD, PDL1, Guanylyl cyclase C (GCC), auto antibody to desmoglein 3 (Dsg3), auto antibody to desmoglein 1 (Dsg1), HLA, HLA-A, HLA-A2, HLA-B, HLA-C, HLA-DP, HLA-DM, HLA-DOA, HLA-DOB, HLA-DQ, HLA-DR, HLA-G, IgE, CD99, Ras G12V, Tissue Factor 1 (TF1), AFP, GPRC5D, Claudin18.2 (CLD18A2 or CLDN18A.2), P-glycoprotein, STEAP1, Liv1, Nectin-4, Cripto, gpA33, BST1/CD157, low conductance chloride channel (LCCC), TAJ/TROY, MPL (TPO-R), KIR3DL2, CD32b, CD229, Toso and BAFF-R. |

The terms "T-cell" and "T-lymphocyte" are interchangeable and used synonymously herein. Examples include but are not limited to naïve T cells ("lymphocyte progenitors"), central memory T cells, effector memory T cells, stem memory T cells (T_{scm}), iPSC-derived T cells, synthetic T cells or combinations thereof.

The term "T/NK cell activating antibody therapy" as used herein refers to an antibody therapy that activates the T and/or NK cells. Examples of T/NK cell activating antibody therapy include bispecific T cell engaging antibody (*e.g*., Blinatumomab) or bispecific NK cell engaging antibody.

TCRs are described using the International Immunogenetics (IMGT) TCR nomenclature, and links to the IMGT public database of TCR sequences. Native alpha-beta heterodimeric TCRs have an alpha chain (TCRα or TCRa) and a beta chain (TCRβ or TCRb). Gamma-delta heterodimeric TCRs have a gamma (TCRg) and TCR-delta (TCRd) chain. Broadly, each chain comprises variable, joining and constant regions, and the beta chain also usually contains a short diversity region between the variable and joining regions, but this diversity region is often considered as part of the joining region. Each variable region comprises three CDRs (Complementarity Determining Regions) embedded in a framework sequence, one being the hypervariable region named CDR3. There are several types of alpha chain variable (Vα or Va) regions and several types of beta chain variable (Vβ or Vb) regions distinguished by their framework, CDR1 and CDR2 sequences, and by a partly defined CDR3 sequence. The Vα/Va types are referred to in IMGT nomenclature by a unique TRAV number. Thus "TRAV21" defines a TCR Vα/Va region having unique framework and CDR1 and CDR2 sequences, and a CDR3 sequence which is partly defined by an amino acid sequence which is preserved from TCR to TCR but which also includes an amino acid sequence which varies from TCR to TCR. In the same way, "TRBV5-1" defines a TCR Vβ/Vb region having unique framework and CDR1 and CDR2 sequences, but with only a partly defined CDR3 sequence. The variable regions of TCRγ (TCRg) and TCRδ (TCRd) are referred to as Vg and Vd, respectively. The variable regions of a TCR (*e.g.,* Vα, Vβ, Vγ, Vδ) may bind to an antigen in an MHC (or HLA) dependent manner. The variable regions of a TCR (*e.g.,* Vα, Vβ, Vγ, Vδ) may bind to an antigen in an MHC (or HLA) independent manner. In an exemplary embodiment, variable regions of a TCR (*e.g.,* Vα, Vβ, Vγ, Vδ) may bind to extracellular antigens (*e.g.,* CD19, CD20, CD22, Mesothelin *etc.*) in an MHC (or HLA)-independent manner. Such TCRs are designated HLA-independent TCR. In an embodiment, the SARs of the present disclosure may comprise of HLA-dependent and/or HLA-independent TCR variable regions. In another exemplary embodiment, SARs of the disclosure may comprise of one variable region of TCR and one variable region of an antibody. Thus, a double chain SAR may comprise of a Va fragment attached to TCRα constant chain and vH (or vL) fragment attached to TCRβ constant chain. An AABD may be attached to one or both the chains.

The α and β chains of αβ TCRs are generally regarded as each having two "domains", namely variable and constant domains. The variable domain consists of a concatenation of variable region and joining region. In the present specification and claims, the term "TCR alpha variable domain or Va or Vα" therefore refers to the concatenation of TRAV and TRAJ regions, and the term TCR alpha constant domain (Cα) refers to the extracellular TRAC region, or to a C-terminal truncated TRAC sequence. Likewise, the term "TCR beta variable domain or Vβ or Vb" refers to the concatenation of TRBV and TRBD/TRBJ regions, and the term TCR beta constant domain (Cβ) refers to the extracellular TRBC region, or to a C-terminal truncated TRBC sequence.

TCRs of the disclosure may be non-naturally occurring and/or purified and/or engineered. TCRs of the disclosure may have more than one mutation present in the alpha chain variable domain and/or the beta chain variable domain relative to the parental TCR. "Engineered TCR" and "mutant TCR" are used synonymously herein and generally mean a TCR which has one or more mutations introduced relative to the parental TCR, in particular in the Va and/or Vb or Vg and/or Vd domain thereof. An engineered TCR may bind to an antigen in an HLA-dependent or HLA-independent manner.

An "HLA-independent TCR" or an "MHC-independent TCR" as defined herein is a TCR that can recognize an antigen independent of MHC restriction. In an exemplary embodiment, an HLA-independent TCR may bind to an antigen on the cell surface that is not presented by the MHC complex. In an embodiment, an HLA-independent TCR may bind to an antigen that is expressed on the cell surface independent of presentation by the MHC complex. An HLA-indendent TCR may be a naturally occurring TCR. In an exemplary embodiment, an HLA-independent TCR is MC.7.G5 (MC7G5) that recognizes MR1, a ubiquitously expressed, monomorphic antigen presenting molecule. An HLA-independent TCR may be an engineered or recombinant TCR. In an exemplary embodiment, an HLA-indepenent TCR is an engineered TCR that may bind to proteins that are expressed on cell surface such as CD19, CD20, Mesothelin, PSMS or BCMA. Methods to engineer the variable domains of a TCR (*e.g.,* CDR grafting *etc.*) are known in the art and can be used to generate HLA-independent TCR that can bind to proteins (*e.g.,* CD19, MSLN, PSMA *etc.*) or protein epitopes expressed extracellularly independent of the MHC complex. This disclosure provides bispecific, biparatopic and mutlispecific SARs with the backbone of a TCR, including HLA-independent TCR, comprising one or more AABDs. The AABD domains of the SARs of the disclosure with the backbone of a TCR (*e.g.,* HLA independent TCR) can be fully human, humanized or non-human. In an embodiment, the disclosure provides TCR (*e.g.,* HLA independent TCR) comprising one or more fully human vH domains. In an embodiment, the disclosure provides TCR (*e.g.,* HLA independent TCR) comprising one or more fully human vL domains.

An ""HLA-independent TCR variable domain" as defined herein is the variable domain of a TCR that can bind to an antigen in an HLA-independent manner. An HLA independent variable domain may be the variable domain of an HLA independent TCRα, TCRβ, TCRγ, TCRδ or pre-TCRα. An HLA independent TCR variable domain may be a single variable domain TCR (*i.e.,* svd-TCR). An HLA independent TCR variable domain may be a naturally occurring HLA-independent variable domain or an engineered HLA-independent variable domain. In an exemplary embodiment, an engineered HLA-independent variable domain can be generated against the extracellular domain of a protein (*e.g.,* CD19, CD22, BCMA, MSLN, PSMA) using techniques known in the art (*e.g.,* CDR grafting, screening phage display libraries *etc.*)*.*

The term "T cell receptor module," or "TCRM," refers to a heterodimer comprising sequences derived from a T cell receptor. The TCRM comprises T cell receptor transmembrane domains and may further comprise all or a portion of T cell receptor connecting peptides and/or intracellular domains.

The term "TCR-associated signaling module" refers to a molecule having a cytoplasmic immunoreceptor tyrosine-based activation motif (ITAM) that is part of the TCR-CD3 complex. TCR-associated signaling modules include CDγε, CDδε and CD3ζζ.

"Therapeutic agents" as used herein refers to agents that are used to, for example, treat, inhibit, prevent, mitigate the effects of, reduce the severity of, reduce the likelihood of developing, slow the progression of and/or cure, a disease. Diseases targeted by the therapeutic agents include but are not limited to infectious diseases, Carcinomas, sarcomas, lymphomas, leukemia, germ cell tumors, blastomas, antigens expressed on various immune cells, and antigens expressed on cells associated with various hematologic diseases, and/or inflammatory diseases.

"Therapeutic Controls" as used herein refers to an element used for controlling the activity of a SAR (including next generation CAR) expressing cell. In some embodiments, therapeutic controls for controlling the activity of the SAR expressing cells of the disclosure comprise any one or more of truncated epidermal growth factor receptor (tEGFR), truncated epidermal growth factor receptor viii (tEGFRviii), truncated CD30 (tCD30), truncated BCMA (tBCMA), truncated CD19 (tCD19), thymidine kinase, cytosine deaminase, nitroreductase, xanthine-guanine phosphoribosyl transferase, human caspase 8, human caspase 9, inducible caspase 9, purine nucleoside phosphorylase, linamarase/linamarin/glucose oxidase, deoxyribonucleoside kinase, horseradish peroxidase (HRP)/indole-3-acetic (IAA), Gamma-glutamylcysteine synthetase, CD20/alphaCD20, CD34/thymidine kinase chimera, dox-dependent caspase-2, mutant thymidine kinase (HSV-TKSR39), AP1903/Fas system, a chimeric cytokine receptor (CCR), a selection marker, and combinations thereof.

The term "therapeutic effect" refers to a biological effect which can be manifested by various means, including but not limited to, *e.g.,* decrease in tumor volume, a decrease in the number of cancer cells, a decrease in the number of metastases, an increase in life expectancy, decrease in cancer cell proliferation, decrease in cancer cell survival, decrease in the titer of the infectious agent, a decrease in colony counts of the infectious agent, amelioration of various physiological symptoms associated with a disease condition. A "therapeutic effect" can also be manifested by the ability of the peptides, polynucleotides, cells and antibodies in prevention of the occurrence of disease in the first place or in the prevention of relapse of the disease.

The term "therapeutically effective amount" as used herein refers to the amount of a pharmaceutical composition comprising one or more peptides as disclosed herein or a mutant, variant, analog or derivative thereof, to decrease at least one or more symptom of the disease or disorder, and relates to a sufficient amount of pharmacological composition to provide the desired effect. The phrase "therapeutically effective amount" as used herein means a sufficient amount of the composition to treat a disorder, at a reasonable benefit/risk ratio applicable to any medical treatment.

A therapeutically or prophylactically significant reduction in a symptom is, *e.g.,* at least about 10%, at least about 20%, at least about 30%, at least about 40%, at least about 50%, at least about 60%, at least about 70%, at least about 80%, at least about 90%, at least about 100%, at least about 125%, at least about 150% or more in a measured parameter as compared to a control or non-treated subject or the state of the subject prior to administering the oligopeptides described herein.

The term "TCR receptor fusion proteins" or "TFP" refers to a next generation SAR platform as described in WO 2016/187349 A1 which is incorporated herein by reference. In an embodiment, a TFP comprises an antibody moiety that specifically binds to a target antigen fused to a TCR chain such as CD3ε, CD3γ, CD3δ, TCRα or TCRβ. Exemplary TCR chains that can be used in the construction of TFP are represented by SEQ ID NOs: 11903-11906 of this disclosure and are provided in WO 2017/070608 A1 which is incorporated herein by reference. A TFP incorporating CD3ε chain is referred to as a CD3ε TFP or TFPε. A TFP incorporating CD3γ chain is referred to as a CD3γ TFP or TFPγ. A TFP incorporating CD3δ chain is referred to as a CD3δ TFP or TFPδ. The TFP incorporating CD3ε, CD3γ or CD3δ chains are collectively referred to as CD3ε/γ/δ TFP or TFPε/γ/δ. This disclosure provides bispecific, biparatopic and mutlispecific SARs with the backbone of a TFP *(e.g.,* TFPε), comprising one or more AABDs. The AABD domains of the SARs of the disclosure with the backbone of a TFP can be fully human, humanized or non-human. In an embodiment, the disclosure provides TFP comprising one or more fully human vH domains. In an embodiment, the disclosure provides TFP comprising one or more fully human vL domains. In an embodiment, the disclosure provides TFP comprising a non-immunoglobulin antigen binding scaffold (*e.g*., Centyrin or DARPIN). In an embodiment, the disclosure provides TFP comprising an adaptor binding domain *(*e*.g.,* leucine zipper domain, *e.g.,* RZip, Ezip, E4, K4 *etc.*)*.*

The term "transfer vector" refers to a composition of matter which comprises an isolated nucleic acid and which can be used to deliver the isolated nucleic acid to the interior of a cell. Examples of viral transfer vectors include, but are not limited to, adenoviral vectors, adena-associated virus vectors, retroviral vectors, lentiviral vectors, and the like.

"Transmembrane domain" (TMD) as used herein refers to the region of the SAR which crosses the plasma membrane. The transmembrane domain of the SAR of the disclosure is the transmembrane region of a transmembrane protein (for example Type I transmembrane proteins), an artificial hydrophobic sequence or a combination thereof. Other transmembrane domains will be apparent to those of skill in the art and may be used in connection with alternate embodiments of the disclosure. In some embodiments, the TMD encoded SAR comprises a transmembrane domain selected from the transmembrane domain of TCRα, TCRβ. TCRγ, TCRδ chain of a T-cell receptor, CD3γ, CD3ε, CD3δ, CD3ζ, FcRγ, CD28, CD45, CD4, CD5, CD8, NKp44, NKp30, NKp46, NKG2D, and/or NKG2C.

As used herein "Tri-functional T cell antigen coupler" or "Tri-TAC" or "TAC" refer to a next generation SAR platform described in WO 2015/117229 A1, which is incorporated herein by reference. Tri-TAC targeting different antigens can be constructed using the antigen binding domains (*e.g*., vL and vH fragments, scFv, vHH, ligands and receptors *etc.*) described in this disclosure using techniques known in the art.

As used herein, the terms "treat," "treatment," "treating," or "amelioration" refer to therapeutic treatments, wherein the object is to reverse, alleviate, ameliorate, inhibit, slow down or stop the progression or severity of a condition associated with, a disease or disorder.

"Tumor," as used herein refers to all neoplastic cell growth and proliferation, whether malignant or benign, and all pre-cancerous and cancerous cells and tissues.

"Vector", "cloning vector" and "expression vector" as used herein refer to the vehicle by which a polynucleotide sequence (*e.g.,* a foreign gene) can be introduced into a host cell, so as to transform the host and promote expression (*e.g*., transcription and translation) of the introduced sequence. Vectors include plasmids, phages, viruses, *etc.*

The term "viral vector" refers to a vector obtained or derived from a virus. Typically, the virus is a retrovirus including, but not limited to, lentiviruses and gamma retroviruses. The viral vector of the disclosure may be a retroviral vector, such as a gamma-retroviral vector. The viral vector of the disclosure may be a lentiviral vector.

The term "zeta" or alternatively "zeta chain", "CD3-zeta" or "TCR-zeta" is defined as the protein provided as GenBank Acession No. BAG36664.1, or the equivalent residues from a non-human species.

The binding domain of a SAR binds to a desired epitope or antigen. For example, the epitope recognized by a SAR is determined from the epitope recognized by the scFv used as the binding domain of the SAR. For example, since the antigen specific domain of the SAR CD8SP-hu-mROO5-1-vL-Gly-Ser-Linker-hu-mROO5-1-vH-Myc-CD8TM-BBz-T2A-PAC (SEQ ID NO: 7340) targeting CD19 is comprised of humanized scFv (SEQ ID NO: 11323), it is expected that the SAR targets the same epitope as the scFv and/or the parental antibody from which the scFv is derived. The epitopes recognized by several scFv and/or their parental antibodies used in the construction of the SARs and backbones of this disclosure are known in the art. Alternatively, the epitope targeted by an AMR or a SAR can be determined by generating a series of mutants of its target antigen and testing the ability of the mutants to bind to the SAR-expressing cells using techniques known in the art, for example, using the Topanga Assay. As an example, the epitope recognized by the SAR CD8SP-hu-mROO5-1-vL-Gly-Ser-Linker-hu-mROO5-1-vH-Myc-CD8TM-BBz-T2A-PAC (SEQ ID NO: 7340) targeting CD19 can be determined by generating a panel of deletion and point mutants of the CD19-ECD-GGSG-NLuc-4xFlag-2xStreptag-8xHis-T2A-Pac (DNA SEQ ID NO: 1282 and PRT SEQ ID NO: 11972). The mutant constructs would be transfected into a suitable cell line (*e.g.,* 293FT cells) and the supernatant containing the fusion protein collected and assayed for NLuc activity to assure that the different mutant CD19-ECD-GGSG-NLuc-4xFlag-2xStreptag-8xHis fusion proteins are being secreted in the supernatant. Subsequently, the fusion proteins would be tested for their ability to bind to cells (*e.g.,* Jurkat cells or T cells) expressing the SAR (SEQ ID NO: 7340) construct. The mutant that fails to bind to the SAR-expressing cells is a candidate for containing the epitope targeted by the CD19-specific SAR. An alternate approach to determine the epitope recognized by a particular SAR could include a functional competitive assay with different test antibodies. For example, T cells expressing the SAR (SEQ ID NO: 7340) could be co-cultured with a cell line expressing CD19 (*e.g.,* RAJI cells) in the absence and presence of increasing concentrations of different test CD19 antibodies. In case the epitope recognized by a test CD19 antibody overlaps with the epitope recognized by the SAR ((SEQ ID NO: 7340), then the test antibody would be expected to block target-cell killing and cytokine production induced by T cells expressing the SAR (SEQ ID NO: 7340) in a dose-dependent manner. A non-specific antibody of the same isotype as the test antibody would be included as a control and would be expected to have no effect on the target-cell killing and cytokine production induced by T cells expressing the SAR. Similarly, a specific SAR can be expressed in Jurkat-NFAT-EGFP cells and the ability of a test antibody to block EGFP induction by the SAR-expressing Jurkat-NFAT-GFP cells upon coculture with a target cell line can be used to determine whether the epitope recognized by the test antibody overlaps with the epitope recognized by the said SAR.

**TABLE 1**

| **SEQ ID NO** | **Name of vector or component** | **SEQ ID NO** | |
|---|---|---|---|
| 1 | pLenti-EF1a | 17 | LucPPe-146-1H2 |
| 2 | pLenti-EF1a-DWPRE | 18 | LucPPe-133-1B2 |
| 3 | MSCV-Bgl2-AvrII-EcoR1B1-Mlu-Sal-ClaI | 19 | LucPPe-78-0B10 |
| 4 | pCCLc-MNDU3-WPRE | 20 | LucPPe49-7C6A |
| 5 | pCCLc-MNDU3-Eco-Nhe-Sal-WPRE | 21 | LucPpL-81-6G1 |
| 6 | pCCLc-MNDU3 -delta-WPRE | 22 | CD8SP-StreptagII-R1-S-Protein-RBD- Mlu-mCD8-hinge-TM |
| 7 | (EF1a)_Promoter_Variant | 23 | TRAC-5-SA-F2A-CD8SP-CD19- vHH-048-G4Sx3-R1-hu-mROO5-1- vL-[hTCRb-S57C]-F-P2A-IgSP-Apa- CD20-vHH-2HCD25-G4Sx3v2-hu- mROO5-1-vH-[hTCRa-T48C]-STOP- pA-Del-Exon1-TRAC-3 |
| 8 | pSBbi-Pur | 24 | MNDU3-promoter |
| 9 | MSCVhygro-GLuc-HA | 25 | DNA barcode 9 |
| 10 | MSCVpac-GLUC-R03 | 26 | DNA barcode 10 |
| 11 | pLENTI-NLuc-AcV5-Blast | 27 | DNA barcode 11 |
| 12 | pLENTI-Gluc-Flag-blast | 28 | DNA barcode 12 |
| 13 | PolyA | 29 | DNA barcode 13 |
| 14 | PolyA | 30 | DNA barcode 13 |
| 15 | PolyA | | |
| 16 | polyA | | |

**Table 2**

| NAME | SEQ ID NO (DNA) | SEQ ID NO (PRT) | NAME | SEQ ID NO (DNA) | SEQ ID NO (PRT) |
|---|---|---|---|---|---|
| CD8_Signal_Peptide | 31 | 10721 | (Gly4Ser)x3_Linker | 37 | 10727 |
| CD8-SIGNAL-PEPTIDE | 32 | 10722 | (Gly4Ser)x3_Linker | 38 | 10728 |
| IgH_Signal_Peptide | 33 | 10723 | (GGGGS)x3-Linker | 39 | 10729 |
| IgH_Signal_Peptide | 34 | 10724 | (GGGGS)x3-Linker | 40 | 10730 |
| TCRa-SP | 35 | 10725 | (GGSG)7_Linker | 41 | 10731 |
| TCRb-SP | 36 | 10726 | (GGSG)7_Linker_2 | 42 | 10732 |
| (GGGGS)x3_LINKER | 37 | 10727 | DDAKK_linker | 43 | 10733 |

**Table 3**

| TARGET ANTIGEN | vL NAME | SEQ ID (DNA) | SEQ ID NO (PRT) | vH NAME | SEQ ID (DNA) | SEQ ID (PRT) | scFv-DNA SEQ ID | scFv-PRT SEQ ID |
|---|---|---|---|---|---|---|---|---|
| ALK | Alk-48 | 46 | 10736 | Alk-48 | 288 | 10978 | 530 | 11220 |
| ALK | Alk-58 | 47 | 10737 | Alk-58 | 289 | 10979 | 531 | 11221 |
| BCMA | BCMA-huC12A3 | 48 | 10738 | BCMA-HuC12A3 | 290 | 10980 | 532 | 11222 |
| BCMA | BCMA-J6M0 | 49 | 10739 | BCMA-J6M0 | 291 | 10981 | 533 | 11223 |
| CD123 | CD123-CSL362 | 50 | 10740 | CD123-CSL362 | 292 | 10982 | 534 | 11224 |
| CD19 | CD138 | 51 | 10741 | CD138 | 293 | 10983 | 535 | 11225 |
| CD19 | CD179b | 52 | 10742 | CD179b | 294 | 10984 | 536 | 11226 |
| CD19 | CD19Bu12 | 53 | 10743 | CD19Bu12 | 295 | 10985 | 537 | 11227 |
| CD19 | FMC63 | 54 | 10744 | FMC63 | 296 | 10986 | 538 | 11228 |
| CD19 | huFMC63-11 | 55 | 10745 | huFMC63-11 | 297 | 10987 | 539 | 11229 |
| CD20 | CD20-2F2 | 56 | 10746 | CD20-2F2 | 298 | 10988 | 540 | 11230 |
| CD20 | CD20-GA101 | 57 | 10747 | CD20-GA101 | 299 | 10989 | 541 | 11231 |
| CD22 | CD22-h10F4 | 58 | 10748 | CD22-h10F4 | 300 | 10990 | 542 | 11232 |
| CD276 | CD276-17 | 59 | 10749 | CD276-17 | 301 | 10991 | 543 | 11233 |
| CD30 | CD30-5F11 | 60 | 10750 | CD30-5F11 | 302 | 10992 | 544 | 11234 |
| CD30 | CD30-Ac10 | 61 | 10751 | CD30-Ac10 | 303 | 10993 | 545 | 11235 |
| CD32 | CD32-Med39 | 62 | 10752 | CD32-Med39 | 304 | 10994 | 546 | 11236 |
| CD324 | CD324-hSC10-7 | 63 | 10753 | CD324-hSC10-7 | 305 | 10995 | 547 | 11237 |
| CD324 | CD324-SC10-6 | 64 | 10754 | CD324-SC10-6 | 306 | 10996 | 548 | 11238 |
| CD33b | CD33-huMvc9 | 65 | 10755 | CD33-huMvc9 | 307 | 10997 | 549 | 11239 |
| CD33 | CD33-AF5 | 66 | 10756 | CD33-AF5 | 308 | 10998 | 550 | 11240 |
| CD34 | CD34-hu4C7 | 67 | 10757 | CD34-hu4C7 | 309 | 10999 | 551 | 11241 |
| CD5 | CD5-18 | 68 | 10758 | CD5-18 | 310 | 11000 | 552 | 11242 |
| CD5 | CD5-9 | 69 | 10759 | CD5-9 | 311 | 11001 | 553 | 11243 |
| CD70 | CD70-h1F6 | 70 | 10760 | CD70-h1F6 | 312 | 11002 | 554 | 11244 |
| CD79b | CD79b-2F2 | 71 | 10761 | CD79b-2F2 | 313 | 11003 | 555 | 11245 |
| CD79b | huMA79bv28 | 72 | 10762 | huMA79bv28 | 314 | 11004 | 556 | 11246 |
| CDH17 | CDH17-PTA001A4 | 73 | 10763 | CDH17-PTA001A4 | 315 | 11005 | 557 | 11247 |
| CDH19 | CDH19-16A4 | 74 | 10764 | CDH19-16A4 | 316 | 11006 | 558 | 11248 |
| CDH6 | CDH6-NOV710 | 75 | 10765 | CDH6-NOV710 | 317 | 11007 | 559 | 11249 |
| CDH6 | CDH6-NOV712 | 76 | 10766 | CDH6-NOV712 | 318 | 11008 | 560 | 11250 |
| CLEC5A | CLEC5A-3E12A2 | 77 | 10767 | CLEC5A-3E12A2 | 319 | 11009 | 561 | 11251 |
| CLEC5A | CLEC5A-8H8F5 | 78 | 10768 | CLEC5A-8H8F5 | 320 | 11010 | 562 | 11252 |
| CLL1 | CLL1-M26 | 79 | 10769 | CLL1-M26 | 321 | 11011 | 563 | 11253 |
| CLL1 | CLL1-M32 | 80 | 10770 | CLL1-M32 | 322 | 11012 | 564 | 11254 |
| CS1/SLA MF7 | HuLuc64[2] | 81 | 10771 | HuLuc64 | 323 | 11013 | 565 | 11255 |
| CS1/SLA MF7 | huLuc90 | 82 | 10772 | huLuc90 | 324 | 11014 | 566 | 11256 |
| CSF2RA | CSF2RA-Ab1 | 83 | 10773 | CSF2RA-Ab1 | 325 | 11015 | 567 | 11257 |
| CSF2RA | CSF2RA-Ab6 | 84 | 10774 | CSF2RA-Ab6 | 326 | 11016 | 568 | 11258 |
| DLL3 | DLL3-hSC16-13 | 85 | 10775 | DLL3-hSC16-13 | 327 | 11017 | 569 | 11259 |
| DLL3 | DLL3-hSC16-56 | 86 | 10776 | DLL3-hSC16-56 | 328 | 11018 | 570 | 11260 |
| EGFR | Cetuximab | 87 | 10777 | Cetuximab | 329 | 11019 | 571 | 11261 |
| EGFRviii | EGFRviii-2173 | 88 | 10778 | EGFRviii-2173 | 330 | 11020 | 572 | 11262 |
| EpCAM | EpCam1-D5K5 | 89 | 10779 | EpCam1-D5K5 | 331 | 11021 | 573 | 11263 |
| EpCAM | EpCam1-MM1 | 90 | 10780 | EpCam1-MM1 | 332 | 11022 | 574 | 11264 |
| FLT3 | FLT3-NC7 | 91 | 10781 | FLT3-NC7 | 333 | 11023 | 575 | 11265 |
| HIV1-gp | HIV1-N6 | 92 | 10782 | HIV1-N6 | 334 | 11024 | 576 | 11266 |
| Folate Receptor-1 | FR1-huMov19 | 93 | 10783 | FR1-huMov19 | 335 | 11025 | 577 | 11267 |
| GD2 | GD2-hu14-18 | 94 | 10784 | GD2-hu14-18 | 336 | 11026 | 578 | 11268 |
| GD2 | GD2-hu3F8 | 95 | 10785 | GD2-hu3F8 | 337 | 11027 | 579 | 11269 |
| GD3 | GD3-KM-641 | 96 | 10786 | GD3-KM-641 | 338 | 11028 | 580 | 11270 |
| GFR4 | GFRAlpha4-P4-6 | 97 | 10787 | GFRAlpha4-P4-6 | 339 | 11029 | 581 | 11271 |
| GM1 | GM1-5B2 | 98 | 10788 | GM1-5B2 | 340 | 11030 | 582 | 11272 |
| GPRC5D | GPRC5D-ET150-18 | 99 | 10789 | GPRC5D-ET150-18 | 341 | 11031 | 583 | 11273 |
| GPRC5D | GPRC5D-ET150-5 | 100 | 10790 | GPRC5D-ET150-5 | 342 | 11032 | 584 | 11274 |
| Her2 | Her2-Hu4D5 | 101 | 10791 | Her2-Hu4D5 | 343 | 11033 | 585 | 11275 |
| HIV1-gp100 | HIV1-PGT-128 | 102 | 10792 | HIV1-PGT-128 | 344 | 11034 | 586 | 11276 |
| HIV1-gp100 | HIV1-X5 | 103 | 10793 | HIV1-X5 | 345 | 11035 | 587 | 11277 |
| IL11Ra | IL11Ra-8E2 | 104 | 10794 | IL11Ra-8E2 | 346 | 11036 | 588 | 11278 |
| IL13Ra2 | IL13Ra2-hu107 | 105 | 10795 | IL13Ra2-hu107 | 347 | 11037 | 589 | 11279 |
| IL13Ra2 | IL13Ra2-hu108 | 106 | 10796 | IL13Ra2-hu108 | 348 | 11038 | 590 | 11280 |
| L1CAM | L1CAM-9-3-HU3 | 107 | 10797 | L1CAM-9-3-HU3 | 349 | 11039 | 591 | 11281 |
| LAMP1 | LAMP1-humab1-2 | 108 | 10798 | LAMP1-humab1-2 | 350 | 11040 | 592 | 11282 |
| LAMP1 | LAMP1-Mb4 | 109 | 10799 | LAMP1-Mb4 | 351 | 11041 | 593 | 11283 |
| Lym1 | Lym1 | 110 | 10800 | Lym1 | 352 | 11042 | 594 | 11284 |
| Lym2 | Lym2 | 111 | 10801 | Lym2 | 353 | 11043 | 595 | 11285 |
| MPL/TPO -R | MPL-111 | 112 | 10802 | MPL-111 | 354 | 11044 | 596 | 11286 |
| MPL/TPO -R | MPL-161-HL | 113 | 10803 | MPL-161-HL | 355 | 11045 | 597 | 11287 |
| MPL/TPO -R | MPL-161 | 114 | 10804 | MPL-161 | 356 | 11046 | 598 | 11288 |
| MPL/TPO -R | MPL-175 | 115 | 10805 | MPL-175 | 357 | 11047 | 599 | 11289 |
| TCRB1 | TCRB1-E09 | 116 | 10806 | TCRB1-E09 | 358 | 11048 | 600 | 11290 |
| TCRB1 | TCRB1-Jovi1 | 117 | 10807 | TCRB1-Jovi1 | 359 | 11049 | 601 | 11291 |
| TCRB2 | TCRB2-CP01-D05 | 118 | 10808 | TCRB2-CP01-D05 | 360 | 11050 | 602 | 11292 |
| TCRB2 | TCRB2-CP01-E05 | 119 | 10809 | TCRB2-CP01-E05 | 361 | 11051 | 603 | 11293 |
| TCRgd | TCRgd-G5-4 | 120 | 10810 | TCRgd-G5-4 | 362 | 11052 | 604 | 11294 |
| TnAg | TnAg | 121 | 10811 | TnAg | 363 | 11053 | 605 | 11295 |
| Tn-Muc1 | Tn-Muc1-hu5E5 | 122 | 10812 | Tn-Muc1-hu5E5 | 364 | 11054 | 606 | 11296 |
| TROP2 | TROP2-ARA47-HV3KV3 | 123 | 10813 | TROP2-ARA47-HV3KV3 | 365 | 11055 | 607 | 11297 |
| WT1/HLA -A2 | WT1-Ab13 | 124 | 10814 | WT1-Ab13 | 366 | 11056 | 608 | 11298 |
| WT1/HLA -A2 | WT1-Ab15 | 125 | 10815 | WT1-Ab15 | 367 | 11057 | 609 | 11299 |
| WT1/HLA -A2 | WT1-Ab5 | 126 | 10816 | WT1-Ab5[2] | 368 | 11058 | 610 | 11300 |
| CD123 | CD123-1172 | 127 | 10817 | CD123-1172 | 369 | 11059 | 611 | 11301 |
| CDH19 | CDH19-4B10 | 128 | 10818 | CDH19-4B10 | 370 | 11060 | 612 | 11302 |
| Folate Receptor-beta | FRbeta-m923 | 129 | 10819 | FRbeta-m923 | 371 | 11061 | 613 | 11303 |
| B7J4 | B7H4-hu22C10 | 130 | 10820 | B7H4-hu22C10 | 372 | 11062 | 614 | 11304 |
| B7H4 | B7H4-hu1D11 | 131 | 10821 | B7H4-hu1D11 | 373 | 11063 | 615 | 11305 |
| CD23 | CD23-p5E8 | 132 | 10822 | CD23-p5E8 | 374 | 11064 | 616 | 11306 |
| GCC | GCC-Ab229 | 133 | 10823 | GCC-Ab229 | 375 | 11065 | 617 | 11307 |
| CD200R | CD200R-huDx182 | 134 | 10824 | CD200R-huDx182 | 376 | 11066 | 618 | 11308 |
| AFP/HLA -A2 | AFP-76 | 135 | 10825 | AFP-76 | 377 | 11067 | 619 | 11309 |
| AFP/HLA -A2 | AFP-79 | 136 | 10826 | AFP-79 | 378 | 11068 | 620 | 11310 |
| BCMA | BCMA-ET-03 | 137 | 10827 | BCMA-ET-03 | 379 | 11069 | 621 | 11311 |
| BCMA | BCMA-huC11.D5.3L1H3 | 138 | 10828 | BCMA-huC11.D5.3L1H3 | 380 | 11070 | 622 | 11312 |
| BCMA | BCMA-huC13-F12 | 139 | 10829 | BCMA-huC13-F12 | 381 | 11071 | 623 | 11313 |
| CD123 | CD123-DART-1 | 140 | 10830 | CD123-DART-1 | 382 | 11072 | 624 | 11314 |
| CD123 | CD123-DART-2 | 141 | 10831 | CD123-DART-2 | 383 | 11073 | 625 | 11315 |
| CD123 | CD123-1176 | 142 | 10832 | CD123-1176 | 384 | 11074 | 626 | 11316 |
| CD123 | CD123-2B8 | 143 | 10833 | CD123-2B8 | 385 | 11075 | 627 | 11317 |
| CD123 | CD123-9D7 | 144 | 10834 | CD123-9D7 | 386 | 11076 | 628 | 11318 |
| CD123 | CD123-3B10 | 145 | 10835 | CD123-3B10 | 387 | 11077 | 629 | 11319 |
| CD19 | CD19-MEDI-3649 | 146 | 10836 | CD19-MEDI-3649 | 388 | 11078 | 630 | 11320 |
| CD19 | CD19-Medrex-24D1 | 147 | 10837 | CD19-Medrex-24D1 | 389 | 11079 | 631 | 11321 |
| CD19 | CD19-MOR0028 | 148 | 10838 | CD19-MOR0028 | 390 | 11080 | 632 | 11322 |
| CD19 | CD19-humROO5-1 | 149 | 10839 | CD19-humROO5-1 | 391 | 11081 | 633 | 11323 |
| CD20 | CD20-Ubli-v4 | 150 | 10840 | CD20-Ubli-v4 | 392 | 11082 | 634 | 11324 |
| CD20 | CD20-7D8 | 151 | 10841 | CD20-7D8 | 393 | 11083 | 635 | 11325 |
| CD33 | CD33-SGNh2H12 | 152 | 10842 | CD33-SGNh2H12 | 394 | 11084 | 636 | 11326 |
| CD99 | CD99-hu12E7 | 153 | 10843 | CD99-hu12E7 | 395 | 11085 | 637 | 11327 |
| CLL1 | CLL1-21C9-L2H3 | 154 | 10844 | CLL1-21C9-L2H3 | 396 | 11086 | 638 | 11328 |
| CLL1 | CLL1-6E7L4H1e | 155 | 10845 | CLL1-6E7L4H1e | 397 | 11087 | 639 | 11329 |
| CLL1 | CLL1-hu1075-v1 | 156 | 10846 | CLL1-hu1075-v1 | 398 | 11088 | 640 | 11330 |
| CLL1 | CLL1-hu1075-v2 | 157 | 10847 | CLL1-hu1075-v2 | 399 | 11089 | 641 | 11331 |
| FITC | FITC-4M-53 | 158 | 10848 | FITC-4M-53 | 400 | 11090 | 642 | 11332 |
| FITC | FITC-E2 | 159 | 10849 | FITC-E2 | 401 | 11091 | 643 | 11333 |
| GPRC5D | GPRC5D-ET150-1 | 160 | 10850 | GPRC5D-ET150-1 | 402 | 11092 | 644 | 11334 |
| GPRC5D | GPRC5D-ET150-2 | 161 | 10851 | GPRC5D-ET150-2 | 403 | 11093 | 645 | 11335 |
| HLA-A2 | HLA-A2-3PB2 | 162 | 10852 | HLA-A2-3PB2 | 404 | 11094 | 646 | 11336 |
| Kappa-Light-chain | Kappa-LC1 | 163 | 10853 | Kappa-LC1 | 405 | 11095 | 647 | 11337 |
| CD19 | hCD19-EUK5-13 | 164 | 10854 | hCD19-EUK5-13 | 406 | 11096 | 648 | 11338 |
| Streptag | Streptag | 165 | 10855 | Streptag | 407 | 11097 | 649 | 11339 |
| BCMA | BCMA-huC13-F12-L1H22 | 166 | 10856 | BCMA-huC13-F12-L1H22 | 408 | 11098 | 650 | 11340 |
| BCMA | BCMA-huC12A3-L3H32 | 167 | 10857 | BCMA-huC12A3-L3H32 | 409 | 11099 | 651 | 11341 |
| MPL/TPO -R | Hu-161-2 | 168 | 10858 | Hu-161-2 | 410 | 11100 | 652 | 11342 |
| CD22 | CD22-5 | 169 | 10859 | CD22-5 | 411 | 11101 | 653 | 11343 |
| CD22 | CD22-10 | 170 | 10860 | CD22-10 | 412 | 11102 | 654 | 11344 |
| CD22 | CD22-31 | 171 | 10861 | CD22-31 | 413 | 11103 | 655 | 11345 |
| CD22 | CD22-53 | 172 | 10862 | CD22-53 | 414 | 11104 | 656 | 11346 |
| CD22 | CD22-65 | 173 | 10863 | CD22-65 | 415 | 11105 | 657 | 11347 |
| MPL/TPO -R | MPL-hu-175-2 | 174 | 10864 | MPL-hu-175-2 | 416 | 11106 | 658 | 11348 |
| MPL/TPO -R | MPL-hu-111-2 | 175 | 10865 | MPL-hu-111-2 | 417 | 11107 | 659 | 11349 |
| CD 179a | CD179a-2460-B04 | 176 | 10866 | CD179a-2460-B04 | 418 | 11108 | 660 | 11350 |
| CD179a | CD179a-2462-E07 | 177 | 10867 | CD179a-2462-E07 | 419 | 11109 | 661 | 11351 |
| CD22 | CD22-HA22 | 178 | 10868 | CD22-HA22 | 420 | 11110 | 662 | 11352 |
| STEAP1 | STEAP1-hu120 | 179 | 10869 | STEAP1-hu120 | 421 | 11111 | 663 | 11353 |
| Liv1 | hLiv1-mAb2 | 180 | 10870 | hLiv1-mAb2 | 422 | 11112 | 664 | 11354 |
| Nectin4 | hu-Nectin4-mAb1 | 181 | 10871 | hu-Nectin4-mAb1 | 423 | 11113 | 665 | 11355 |
| CRIPTO | hu-Cripto-L1H2 | 182 | 10872 | hu-Cripto-L1H2 | 424 | 11114 | 666 | 11356 |
| gpA33 | hu-gpA33 | 183 | 10873 | hu-gpA33 | 425 | 11115 | 667 | 11357 |
| ROR1 | ROR1-DART4 | 184 | 10874 | ROR1-DART4 | 426 | 11116 | 668 | 11358 |
| BCMA | BCMA-BB-CAR02 | 185 | 10875 | BCMA-BB-CAR02 | 427 | 11117 | 669 | 11359 |
| CLL1 | CLL1-24C8 | 186 | 10876 | CLL1-24C8 | 428 | 11118 | 670 | 11360 |
| CLL1 | CLL1-24C1 | 187 | 10877 | CLL1-24C1 | 429 | 11119 | 671 | 11361 |
| FLT3 | FLT3-10E3 | 188 | 10878 | FLT3-10E3 | 430 | 11120 | 672 | 11362 |
| FLT3 | FLT3-8B5 | 189 | 10879 | FLT3-8B5 | 431 | 11121 | 673 | 11363 |
| IL1RAP | IL1RAP-IAPB57 | 190 | 10880 | IL1RAP-IAPB57 | 432 | 11122 | 674 | 11364 |
| IL1RAP | IL1RAP-IAPB63 | 191 | 10881 | IL1RAP-IAPB63 | 433 | 11123 | 675 | 11365 |
| IL1RAP | hu-IL1RAP-CANO4 | 192 | 10882 | hu-IL1RAP-CANO4 | 434 | 11124 | 676 | 11366 |
| MSLN | MSLN-7D9-v3 | 193 | 10883 | MSLN-7D9-v3 | 435 | 11125 | 677 | 11367 |
| MSLN | MSLN-hu22A10 | 194 | 10884 | MSLN-hu22A10 | 436 | 11126 | 678 | 11368 |
| BST1 | hu1-A1 | 195 | 10885 | hu1-A1 | 437 | 11127 | 679 | 11369 |
| BST1 | hu1-A2 | 196 | 10886 | hu1-A2 | 438 | 11128 | 680 | 11370 |
| BST1 | hu1-A3 | 197 | 10887 | hu1-A3 | 439 | 11129 | 681 | 11371 |
| CD19 | CAT 17-HL | 198 | 10888 | CAT 17-HL | 440 | 11130 | 682 | 11372 |
| CD22 | hu-HA22-1 | 199 | 10889 | hu-HA22-1 | 441 | 11131 | 683 | 11373 |
| CD70 | CD70-HL-AM13 | 200 | 10890 | CD70-HL-AM13 | 442 | 11132 | 684 | 11374 |
| BCMA | BCMA-BB-CAR02 | 201 | 10891 | BCMA-BB-CAR02 | 443 | 11133 | 685 | 11375 |
| Her2 | Her2-169 | 202 | 10892 | Her2-169 | 444 | 11134 | 686 | 11376 |
| Her2 | Her2-XMT-1520 | 203 | 10893 | Her2-XMT-1520 | 445 | 11135 | 687 | 11377 |
| Her2 | Her2-XMT-1518 | 204 | 10894 | Her2-XMT-1518 | 446 | 11136 | 688 | 11378 |
| Her2 | Her2-huMab4D5-D98W | 205 | 10895 | Her2-huMab4D5-D98W | 447 | 11137 | 689 | 11379 |
| MSLN | MSLN-3-HL-AM | 206 | 10896 | MSLN-3-HL-AM | 448 | 11138 | 690 | 11380 |
| MSLN | MSLN-5-HL | 207 | 10897 | MSLN-5-HL | 449 | 11139 | 691 | 11381 |
| EGFRviii | EGFRviii-2AM-HL | 208 | 10898 | EGFRviii-2AM-HL | 450 | 11140 | 692 | 11382 |
| EGFRviii | EGFRviii-H2M1863N2-HL | 209 | 10899 | EGFRviii-H2M1863N2-HL | 451 | 11141 | 693 | 11383 |
| DLL3 | DLL3-AM6-HL | 210 | 10900 | DLL3-AM6-HL | 452 | 11142 | 694 | 11384 |
| DLL3 | DLL3-AM14-HL | 211 | 10901 | DLL3-AM14-HL | 453 | 11143 | 695 | 11385 |
| Nectin4 | Nectin4-66-HL | 212 | 10902 | Nectin4-66-HL | 454 | 11144 | 696 | 11386 |
| MSLN | MSLN-237-HL | 213 | 10903 | MSLN-237-HL | 455 | 11145 | 697 | 11387 |
| MSLN | MSLN-HuAM15 | 214 | 10904 | MSLN-HuAM15 | 456 | 11146 | 698 | 11388 |
| MSLN | MSLN76923-HL | 215 | 10905 | MSLN76923-HL | 457 | 11147 | 699 | 11389 |
| PRLR | PRLR-CN | 216 | 10906 | PRLR-CN | 458 | 11148 | 700 | 11390 |
| EMR2 | EMR2-USC2-V4 | 217 | 10907 | EMR2-USC2-V4 | 459 | 11149 | 701 | 11391 |
| CEA | CEA-USC1-HL4 | 218 | 10908 | CEA-USC1-HL4 | 460 | 11150 | 702 | 11392 |
| Her3 | Her3-USC1-HL14 | 219 | 10909 | Her3-USC1-HL14 | 461 | 11151 | 703 | 11393 |
| FOLR1 | FOLR1-USC1-HL4 | 220 | 10910 | FOLR1-USC1-HL4 | 462 | 11152 | 704 | 11394 |
| FOLR1 | FOLR1-USC2-HL4 | 221 | 10911 | FOLR1-USC2-HL4 | 463 | 11153 | 705 | 11395 |
| CLDN6 | CLDN6-USC1-LH4 | 222 | 10912 | CLDN6-USC1-LH4 | 464 | 11154 | 706 | 11396 |
| CLDN6 | CLDN6-USC2-LH4 | 223 | 10913 | CLDN6-USC2-LH4 | 465 | 11155 | 707 | 11397 |
| SLC34A2 | HuMX35-LH4 | 224 | 10914 | HuMX35-LH4 | 466 | 11156 | 708 | 11398 |
| CD22 | CD22-INO | 225 | 10915 | CD22-INO | 467 | 11157 | 709 | 11399 |
| CD22 | CD22-hu-RFB4 | 226 | 10916 | hu-RFB4 | 468 | 11158 | 710 | 11400 |
| BCMA | BCMA-mJ22-9 | 227 | 10917 | BCMA-mJ22-9 | 469 | 11159 | 711 | 11401 |
| CD22 | CD22-hu-HA22-2 | 228 | 10918 | CD22-hu-HA22-2 | 470 | 11160 | 712 | 11402 |
| CD19 | huCD19-USC3 | 229 | 10919 | huCD19-USC3 | 471 | 11161 | 713 | 11403 |
| BCMA | BCMA-hu72 | 230 | 10920 | BCMA-hu72 | 472 | 11162 | 714 | 11404 |
| MPL/TPO -R | hu-161-3 | 231 | 10921 | hu-161-3 | 473 | 11163 | 715 | 11405 |
| BAFFR | hu-BAFFR-USC90 | 232 | 10922 | hu-BAFFR-USC90 | 474 | 11164 | 716 | 11406 |
| BAFFR | hu-BAFFR-USC55 | 233 | 10923 | hu-BAFFR-USC55 | 475 | 11165 | 717 | 11407 |
| BAFFR | hu-BAFFR-MOR-6654 | 234 | 10924 | hu-BAFFR-MOR-6654 | 476 | 11166 | 718 | 11408 |
| BCMA | BCMA-hu-USC73 | 235 | 10925 | BCMA-hu-USC73-G32S | 477 | 11167 | 719 | 11409 |
| BCMA | BCMA-hu-USC74 | 236 | 10926 | BCMA-hu-USC74-F35Y | 478 | 11168 | 720 | 11410 |
| BCMA | BCMA-hu-USC75 | 237 | 10927 | BCMA-hu-USC74-V104T | 479 | 11169 | 721 | 11411 |
| BCMA | BCMA-hu-USC76 | 238 | 10928 | BCMA-hu-USC76-158S | 480 | 11170 | 722 | 11412 |
| BCMA | BCMA-hu-USC77 | 239 | 10929 | BCMA-huUSC77-T59D | 481 | 11171 | 723 | 11413 |
| BCMA | BCMA-hu-USC78 | 240 | 10930 | BCMA-huUSC78-S64G | 482 | 11172 | 724 | 11414 |
| BCMA | BCMA-hu-USC79 | 241 | 10931 | BCMA-huUSC79-S64T | 483 | 11173 | 725 | 11415 |
| BCMA | BCMA-hu-USC80-N25K | 242 | 10932 | BCMA-huUSC80 | 484 | 11174 | 726 | 11416 |
| BCMA | BCMA-hu-USC81-127L | 243 | 10933 | BCMA-huUSC81 | 485 | 11175 | 727 | 11417 |
| BCMA | BCMA-hu-USC82-S29G | 244 | 10934 | BCMA-huUSC82 | 486 | 11176 | 728 | 11418 |
| BCMA | BCMA-hu-USC83-S31T | 245 | 10935 | BCMA-huUSC83 | 487 | 11177 | 729 | 11419 |
| BCMA | BCMA-hu-USC80-N25K | 246 | 10936 | BCMA-hu-USC73-G32S | 488 | 11178 | 730 | 11420 |
| BCMA | BCMA-hu-USC81-I27L | 247 | 10937 | BCMA-hu-USC731-G32S | 489 | 11179 | 731 | 11421 |
| BCMA | BCMA-hu-USC82-S29G | 248 | 10938 | BCMA-hu-USC73-G32S | 490 | 11180 | 732 | 11422 |
| ROR1 | ROR1-JJ-67 | 249 | 10939 | ROR1-JJ-67 | 491 | 11181 | 733 | 11423 |
| ROR1 | ROR1-JJ-78 | 250 | 10940 | ROR1-JJ-78 | 492 | 11182 | 734 | 11424 |
| ROR1 | ROR1-JJ-76 | 251 | 10941 | ROR1-JJ-76 | 493 | 11183 | 735 | 11425 |
| CD20 | CD20-Rituximab | 252 | 10942 | CD20-Rituximab | 494 | 11184 | 736 | 11426 |
| Her2 | Her2-FRP5-HL | 253 | 10943 | Her2-FRP5-HL | 495 | 11185 | 737 | 11427 |
| CD19 | CEA-huMN14 | 254 | 10944 | CEA-huMN14 | 496 | 11186 | 738 | 11428 |
| CEA | CEA-BW431-26 | 255 | 10945 | CEA-BW431-26 | 497 | 11187 | 739 | 11429 |
| Her2 | Her2-huMab4D5-H91A | 256 | 10946 | Her2-huMab4D5-D98A-F100A-Y102V | 498 | 11188 | 740 | 11430 |
| Her2 | Her22-USC-1516 | 257 | 10947 | Her22-USC-1516 | 499 | 11189 | 741 | 11431 |
| TOSO | TOSO-6B10 | 258 | 10948 | TOSO-6B10 | 500 | 11190 | 742 | 11432 |
| CD30 | CD30-HRS3 | 259 | 10949 | CD30-HRS3 | 501 | 11191 | 743 | 11433 |
| CD229 | CD229-USC1-2D4 | 260 | 10950 | CD229-USC1-2D4 | 502 | 11192 | 744 | 11434 |
| CD229 | CD229-2A2 | 261 | 10951 | CD229-2A2 | 503 | 11193 | 745 | 11435 |
| CD229 | CD229-USC3-2D5 | 262 | 10952 | CD229-USC3-2D5 | 504 | 11194 | 746 | 11436 |
| CD229 | CD229-USC2-2D4 | 263 | 10953 | CD229-USC2-2D4 | 505 | 11195 | 747 | 11437 |
| EBV-gp350 | EBV-gp350-7A1 | 264 | 10954 | EBV-gp350-7A1 | 506 | 11196 | 748 | 11438 |
| EBV-gp350 | EBV-gp350-6G4 | 265 | 10955 | EBV-gp350-6G4 | 507 | 11197 | 749 | 11439 |
| INFLUEN ZA-A-Neuramida se | INFL-NA-1E01 | 266 | 10956 | INFL-NA-1E01 | 508 | 11198 | 750 | 11440 |
| EBV-LMP1 | EBV-LMP1-HLEA | 267 | 10957 | EBV-LMP1-HLEA | 509 | 11199 | 751 | 11441 |
| PSMA | PSMA-J591-ds75 | 268 | 10958 | PSMA-J591-ds75 | 510 | 11200 | 752 | 11442 |
| PSMA | hu-PSMA-J591 | 269 | 10959 | hu-PSMA-J591 | 511 | 11201 | 753 | 11443 |
| PSMA | hu-PSMA-2-J591 | 270 | 10960 | hu-PSMA-2-J591 | 512 | 11202 | 754 | 11444 |
| PSMA | hu-PSMA-J591 | 271 | 10961 | hu-PSMA-J591-ds75 | 513 | 11203 | 755 | 11445 |
| PSMA | PSMA-J591-ds75 | 272 | 10962 | hu-PSMA-J591 | 514 | 11204 | 756 | 11446 |
| MUC1 | hu-MUC1-star2-MN-E6 | 273 | 10963 | hu-MUC1-star2-MN-E6 | 515 | 11205 | 757 | 11447 |
| MUC1 | MUC1-start-MN-E6 | 274 | 10964 | MUC1-start-MN-E6 | 516 | 11206 | 758 | 11448 |
| MUC1 | hu-MUC1-star2-MN-C2 | 275 | 10965 | hu-MUC1-star2-MN-C2 | 517 | 11207 | 759 | 11449 |
| gpA33 | hu-gp33-C825 | 276 | 10966 | hu-gp33-C825 | 518 | 11208 | 760 | 11450 |
| MSLN | MSLN-7D10 | 277 | 10967 | MSLN-7D10 | 519 | 11209 | 761 | 11451 |
| MSLN | MSLN-7D9-HL | 278 | 10968 | MSLN-7D9-HL-Y27F | 520 | 11210 | 762 | 11452 |
| MSLN | MSLN-7D9-HL-V29L | 279 | 10969 | MSLN-7D9-HL | 521 | 11211 | 763 | 11453 |
| MSLN | MSLN-hu22A10 | 280 | 10970 | MSLN-hu22A10-F27Y | 522 | 11212 | 764 | 11454 |
| MSLN | MSLN-hu22A10-N31S | 281 | 10971 | MSLN-hu22A10 | 523 | 11213 | 765 | 11455 |
| MSLN | MSLN-hu22A10-Y96W | 282 | 10972 | MSLN-hu22A10 | 524 | 11214 | 766 | 11456 |

**Table 4**

| **TARGET ANTIGEN** | **Va/Vd NAME** | **SEQ ID NO (DNA)** | **SEQ ID NO (PRT)** | **Vb/Vg NAME** | **SEQ ID NO (DNA)** | **SEQ ID NO (PRT)** |
|---|---|---|---|---|---|---|
| MR1 | MC7G5-TCRa-VA | 963 | 11653 | MC7G5-TCRa-Vb | 964 | 11654 |
| NY-ESO-1/HLA-A201 | NY-ESO-1-IG4-Va | 965 | 11655 | NY-ESO-1-IG4-Vb | 966 | 11656 |
| CMV-pp65/HLA-A2 | CMV-pp65-TRC1-Va | 967 | 11657 | CMV-pp65-TRC1-Vb | 968 | 11658 |
| (E)-4-hydroxy-3-methyl-but-2-enyl pyrophosphate (HMB-PP) | TCR-Vd2 | 969 | 11659 | TCR-Vg9 | 970 | 11660 |
| NY-ESO-1/HLA-A02:01 | NY-ESO1-svd-TCR | 21563 | 22399 | | | |
| MAGE-A3-1/HLA-A02:01 | MAGE-A3-sVb-7 | 21564 | 22400 | | | |

**Table 5**

| **Target Antigen** | **Name of Fragment** | **SEQ ID NO (DNA)** | **SEQ ID NO (PRT)** |
|---|---|---|---|
| CEA and CEA | CD8SP-CEA-300-aVH-G3Sx2-CEA-3001- aVH | 816 | 11506 |
| CEA | CD8SP-CEA-300-aVH | 817 | 11507 |
| CD20 and CD22 | CD8SP-CD20-C07-vHH-G4S-CD22- FHVH-158 | 818 | 11508 |
| CD20 and CD22 | CD8SP-CD20-C07-vHH | 819 | 11509 |
| PSMA and PSMA | CD8SP-PSMA71-chVH-G4Sx3-PSMA28- chVH | 820 | 11510 |
| PSMA | CD8SP-PSMA71-chVH | 821 | 11511 |
| PSMA and PSMA | IgHSP-PSMA28v2-chVH-G4Sx2- PSMA71v2-chVH | 822 | 11512 |
| PSMA | IgHSP-PSMA28v2-chVH | 823 | 11513 |
| CD38 | CD8SP-CD38-309407-FHVH | 824 | 11514 |
| CD38 and CD38 | IgHSP-CD38-663-FHVH-SG4x2-CD38- 986-FHVH | 825 | 11515 |
| CD38 | IgHSP-CD38-663-FHVH | 826 | 11516 |
| BCMA | CD8SP-BCMA-FHVH-33 | 827 | 11517 |
| BCMA | CD8SP-BCMA-FHVH-74 | 828 | 11518 |
| CD22 and BCMA | CD8SP-CD22-24-FHVH-G4S-BCMA-93- FHVH | 829 | 11519 |
| PSMA | PSMA71-chVH | 830 | 11520 |
| PSMA | PSMA28-chVH | 831 | 11521 |
| PSMA | PSMA28v2-chVH | 832 | 11522 |
| PSMA | PSMA71v2-chVH | 833 | 11523 |
| CD19 | CD19-vHH-048 | 834 | 11524 |
| CD20 | CD20-vHH-2HCD25 | 835 | 11525 |
| CD19 | CD19-FHVH-354 | 836 | 11526 |
| CD19 | CD19-FHVH-635 | 837 | 11527 |
| CD19 | CD19-USC1-FHVH-355 | 838 | 11528 |
| CD19 | CD19-USC2-FHVH-636 | 839 | 11529 |
| CD20 | CD20-vHH-C07 | 840 | 11530 |
| CD20 | CD20-VHH-USC1 | 841 | 11531 |
| CD22 | CD22-FHVH-158 | 842 | 11532 |
| CD22 | CD22-FHVH-24 | 843 | 11533 |
| CD22 | CD22-USC1-FHVH-160 | 844 | 11534 |
| CD38 | CD38-FHVH-309407 | 845 | 11535 |
| CD38 | CD38-FHVH-309201 | 846 | 11536 |
| CD38 | CD38-FHVH-663 | 847 | 11537 |
| CD38 | CD38-FHVH-986 | 848 | 11538 |
| CD38 | CD38-USC1-FHVH-32184 | 849 | 11539 |
| CEA | CEA-aVH-300 | 850 | 11540 |
| CEA | CEA-aVH-3001 | 851 | 11541 |
| BCMA | BCMA-USC1-FHVH-94 | 852 | 11542 |
| BCMA | BCMA-USC2-FHVH-95 | 853 | 11543 |
| BCMA | BCMA-USC3-FHVH-96 | 854 | 11544 |
| BCMA | BCMA-USC4-FHVH-34 | 855 | 11545 |
| BCMA | BCMA-FHVH-33 | 856 | 11546 |
| BCMA | BCMA-FHVH-74 | 857 | 11547 |
| BCMA | BCMA-FHVH-93 | 858 | 11548 |
| Muc16 | MUC16-VHH-R3MU29 | 859 | 11549 |
| Muc16 | MUC16-vHH-R3MU5-USC1 | 860 | 11550 |
| Muc16 | MUC16-USC2-huR3MU30 | 861 | 11551 |
| IL13Ra2 | IL13Ra2-vHH-USC2-Cl3 | 862 | 11552 |
| IL13Ra2 | IL13Ra2-huvHH-USC1-C1 | 863 | 11553 |
| Her2 | Her2-2D3-vHH | 864 | 11554 |
| Her2 | Her2-5F7-vHH | 865 | 11555 |
| Her2 | Her2-47D5-vHH | 866 | 11556 |
| Her3 | Her3-17B05So-vHH | 867 | 11557 |
| Her3 | Her3-21F06-vHH | 868 | 11558 |
| CEA | CEA1-vHH | 869 | 11559 |
| CEA | CEA5-vHH | 870 | 11560 |
| EGFR | EGFR-vHH | 871 | 11561 |
| EGFR | EGFR33-vHH | 872 | 11562 |
| cMet | cMET-171-vHH | 873 | 11563 |
| CXCR4 | CXCR4-2-vHH | 874 | 11564 |
| CXCR4 | CXCR4-1-vHH | 875 | 11565 |
| MSLN | SD1-vHH | 876 | 11566 |
| MSLN | SD2-vHH | 877 | 11567 |
| Albumin | Alb8-vHH | 878 | 11568 |
| CD123 | CD123-1-vHH | 879 | 11569 |
| CD123 | CD123-2-vHH | 880 | 11570 |
| IL6R | IL6R-304-vHH | 881 | 11571 |
| EGFR and CEA | EGFR-vHH-GS-CEA1-vHH | 882 | 11572 |
| EGFR and CEA | EGFR33-vHH-GS-CEA5-vHH | 883 | 11573 |
| Her2 and Her2 | Her2-5F7-vHH-GS-Linker-Her2-47D5- vHH | 884 | 11574 |
| Her2 and Her2 | Her2-Hu4D5-vL-GS-Linker-Her2-Hu4D5- vH | 885 | 11575 |
| Her3 and Her2 | Her3-17B05So-vHH-GS-Her2-2D3-vHH | 886 | 11576 |
| cMET and Her3 | cMET-171-vHH-GS-Her3-21F06-vHH | 887 | 11577 |
| MSLN | SD1-vHH-GS-SD2-vHH | 888 | 11578 |
| BCMA | BCMA-vHH-353 | 889 | 11579 |
| BCMA | BCMA-vHH-917 | 890 | 11580 |
| BCMA and BCMA | BCMA353--vHH-Gly-Ser-Linker-BCMA917-vHH | 891 | 11581 |
| CD38 | CD38-vHH-717 | 892 | 11582 |
| BCMA | BCMA-346-vHH-(BCMA-vHH-346) | 893 | 11583 |
| CD38 and BCMA | CD38-717-vHH-Ecoil-BCMA-346-vHH | 894 | 11584 |
| BCMA | BCMA-vHH-348 | 895 | 11585 |
| CD38 | CD38-vHH-331 | 896 | 11586 |
| BCMA and CD38 | BCMA-348-vHH-Ecoil-CD38-331-vHH | 897 | 11587 |
| CD19 | CD19-vHH | 898 | 11588 |
| CD20 | CD20-vHH | 899 | 11589 |
| CD19 and CD20 | CD19-vHH-Gly-Ser-Linker-CD20-vHH | 900 | 11590 |
| BCMA | BCMA-vHH-948 | 901 | 11591 |
| BCMA | BCMA-vHH-972 | 902 | 11592 |

**Table 6**

| **Target Antigen** | **Name of fragment** | **SEQ ID NO (DNA)** | **SEQ ID NO (PRT)** |
|---|---|---|---|
| CEA | CEA-300-aVH | 954 | 11644 |
| CEA | CEA-301-aVH | 955 | 11645 |

**Table 7**

| Target Antige n | Name of fragment | SEQ ID NO (DN A) | SEQ ID NO (PRT) | Target Antigen | Name of fragment | SEQ ID NO (DN A) | SEQ ID NO (PRT) |
|---|---|---|---|---|---|---|---|
| Her2 | Her2-DARPIN-1 | 972 | 11662 | PSMA | PSMA-Centyrin-2 | 978 | 11668 |
| Her2 | Her2-DARPIN-2 | 973 | 11663 | PSMA | PSMA-Centyrin-3 | 979 | 11669 |
| Her3 | Her3-affi | 974 | 11664 | EGFR | EGFR-Centyrin | 980 | 11670 |
| Her2 | Her2-affi | 975 | 11665 | cMET | cMET-Centyrin | 981 | 11671 |
| EGFR | EGFR-affi | 976 | 11666 | EGFR and cMET | EGFR-cMET-Cent | 982 | 11672 |
| PSMA | PSMA-Centyrin-1 | 977 | 11667 | BCMA | BCMA-Centyrin | 983 | 11673 |

**Table 8**

| **Name of fragment** | **SEQ ID NO (DNA)** | **SEQ ID NO (PRT)** |
|---|---|---|
| ACE2-ECD | 984 | 11674 |
| hCD19-Extracellular-Domain-minus-signal-peptide | 985 | 11675 |
| hMPL-Extracellular-Domain-with-signal-peptide | 986 | 11676 |
| CD8-SP-PD1-opt-ECD | 987 | 11677 |
| PD1-opt-ECD-minus-signal-peptide | 988 | 11678 |
| PD1-ECD-with-native-Signal-Peptide | 989 | 11679 |
| CTLA4-opt-ECD-with-signal-peptide | 990 | 11680 |
| NKG2D-ECD-minus-signal-peptide | 991 | 11681 |
| CD4-ECD-with-signal-peptide | 992 | 11682 |
| DC-SIGN-minus-signal-peptide | 993 | 11683 |
| CD16A-V158-ECD-v1-minus-signal-peptide | 994 | 11684 |
| CD16A-V158-ECD-v2-minus-signal-peptide | 995 | 11685 |
| dc-Avidin-minus-signal-peptide | 996 | 11686 |
| Desmoglein-3-(Dsg3)-ECD | 997 | 11687 |
| CD8SP-NKG2D | 998 | 11688 |
| CD8SP-NKG2D-GS-NKG2D-(Dimer) | 999 | 11689 |
| NKG2D | 1000 | 11690 |
| COVID-19-Spike-gp-RBD | 1001 | 11691 |

**TABLE 9**

| **Name of fragment** | **SEQ ID NO (DNA)** | **SEQ ID NO (PRT)** |
|---|---|---|
| LH-beta-with-Signal-Peptide | 1002 | 11692 |
| mTPO(1-187) | 1003 | 11693 |
| CGH-alpha-minus-Signal-Peptide | 1004 | 11694 |
| CGH-beta-with-Signal-Peptide | 1005 | 11695 |
| FSH-beta-minus-Signal-Peptide | 1006 | 11696 |
| LH-beta-with-Signal-Peptide | 1007 | 11697 |
| TSH-beta-with-Signal-Peptide | 1008 | 11698 |
| SP-CGHb-Gly-Ser-Linker-CGHa | 1009 | 11699 |
| CD8SP-FSHb-Gly-Ser-Linker-CGHa | 1010 | 11700 |
| SP-LHb-Gly-Ser-Linker-CGHa | 1011 | 11701 |
| SP-TSHb-Gly-Ser-Linker-CGHa | 1012 | 11702 |

**TABLE 10**

| **Name of fragment** | **SEQ ID NO (DNA)** | **SEQ ID NO (PRT)** |
|---|---|---|
| CD8SP-BCMA-FHVH93-GS-ULBP2R | 1013 | 11703 |
| RZIP | 1014 | 11704 |
| EZIP | 1015 | 11705 |
| E4 | 1016 | 11706 |
| K4 | 1017 | 11707 |
| NKG2D-AF | 1018 | 11708 |
| NKG2D-YA | 1019 | 11709 |
| NKG2D-AF-G4Sx3-NKG2D-AF | 1020 | 11710 |
| NKG2D-YA-G4Sx3-NKG2D-YA | 1021 | 11711 |
| ULBP2R | 1022 | 11712 |
| ULBP2-S3 | 1023 | 11713 |
| CD8SP-BCMA-FHVH93-GS-ULBP2-S3 | 1041 | 11731 |

**TABLE 11: Exemplary Linkers**

| **Name of fragment** | **SEQ ID NO (DNA)** | **SEQ ID NO (PRT)** |
|---|---|---|
| G4Sx3 | 1024 | 11714 |
| G4S | 1025 | 11715 |
| G3Sx2 | 1026 | 11716 |
| G4Sx2 | 1027 | 11717 |
| G4Sx3 | 1028 | 11718 |
| Myc-(P)-TAG | 1029 | 11719 |
| MYC2-TAG | 1030 | 11720 |
| MYC4-TAG | 1031 | 11721 |
| V5-TAG | 1032 | 11722 |
| HA-TAG | 1033 | 11723 |
| HIS-TAG | 1034 | 11724 |
| AVI-TAG-delta-GSG | 1035 | 11725 |
| G4Sx2-TAG | 1036 | 11726 |
| G4Sx2-TAG | 1037 | 11727 |
| StrepTagII | 1038 | 11728 |
| StrepTagII | 1039 | 11729 |
| FLAG-TAG | 1040 | 11730 |
| G4Sx3 | 1041 | 11731 |

**TABLE 12**

| **Name of fragment** | **SEQ ID NO (DNA)** | **SEQ ID NO (PRT)** |
|---|---|---|
| hTCR-alpha-constant | 1042 | 11732 |
| hTCRa-WT | 1043 | 11733 |
| hTCRa-CSDVP | 1044 | 11734 |
| hTCRa-opt2 | 1045 | 11735 |
| hTCRa-opt3 | 1046 | 11736 |
| hTCRa-T48C-opt (or hTCRa-T48C or hTCRα-T48C) | 1047 | 11737 |
| hTCRa-T48C-opt1 | 1048 | 11738 |
| hTCRa-SDVP | 1049 | 11739 |
| hTCRa-S61R | 1050 | 11740 |
| hTCRa-SDVPR | 1051 | 11741 |
| hTCRa-SD | 1052 | 11742 |
| hTCRaECD-CD3zECDTMCP-opt2 | 1053 | 11743 |
| mTCRa-opt | 1054 | 11744 |
| canin*etc*.Ra-opt (cTCRa-opt) | 1055 | 11745 |
| hTCR-b1-constant-region | 1056 | 11746 |
| hTCR-b2-constant-region | 1057 | 11747 |
| hTCRb-WT | 1058 | 11748 |
| hTCRb-S57C-opt1 | 1059 | 11749 |
| hTCRb-KACIAH | 1060 | 11750 |
| hTCRb-opt2 | 1061 | 11751 |
| hTCRb-opt2-deltaE | 1062 | 11752 |
| hTCRb-opt3 | 1063 | 11753 |
| hTCRb-opt4 | 1064 | 11754 |
| hTCRb-KAIAH | 1065 | 11755 |
| hTCRb-K18A22 | 1066 | 11756 |
| hTCRb-K18I133 | 1067 | 11757 |
| hTCRb-K18A136 | 1068 | 11758 |
| hTCRb-K18H139 | 1069 | 11759 |
| hTCRb-R18A22 | 1070 | 11760 |
| hTCRb-R18 | 1071 | 11761 |
| hTCRb-KAIAHG | 1072 | 11762 |
| hTCRb-KAG | 1073 | 11763 |
| hTCRb-R79G | 1074 | 11764 |
| mTCRb-opt | 1075 | 11765 |
| canin*etc*.Rb-opt (cTCRb-opt) | 1076 | 11766 |
| hTCRbECD-CD3zECDTMCP-opt | 1077 | 11767 |
| pr*etc*.Ra_gb_U38996.1 | 1078 | 11768 |
| *pretc.Ra* | 1079 | 11769 |
| pr*etc*.Ra-del48 | 1080 | 11770 |
| hTCR-gamma | 1081 | 11771 |
| hTCR-Gamma-Opt | 1082 | 11772 |
| hTCR-Delta | 1083 | 11773 |
| hTCR-Delta-Opt | 1084 | 11774 |
| hTCRa-opt2-Del | 1085 | 11775 |
| hTCRb-RC | 1086 | 11776 |
| hTCRb-RAC | 1087 | 11777 |
| CD3zECDTM-opt | 1088 | 11778 |
| CD3zECDTMCP-opt | 1089 | 11779 |
| CD3zECDTM-28z-opt | 1099 | 11789 |
| CD3zECDTM-BBz-opt | 1100 | 11790 |
| CD3zECDTM-28z-opt2 | 1101 | 11791 |
| CD3zECDTM-BBz-opt2 | 1102 | 11792 |
| hTCRa-T48C-opt-d4 | 1103 | 11793 |
| hTCRa-T48C-opt-d6 | 1104 | 11794 |
| hTCRa-T48C-opt-d10 | 1105 | 11795 |
| hTCRa-T48C-opt-d13 | 1106 | 11796 |
| hTCRa-T48C-opt-d17 | 1107 | 11797 |
| hTCRa-T48C-opt-d26 | 1108 | 11798 |
| hTCRa-T48C-opt-d32 | 1109 | 11799 |
| hTCRa-T48C-opt-d46 | 1110 | 11800 |
| hTCRa-wt2-opt-d52 | 1111 | 11801 |
| TCRa-wt2-opt-6MD | 1112 | 11802 |
| TCRa-wt2-opt-7MD | 1113 | 11803 |
| hTCRb-S57C-opt1 (or hTCRb-S57C or hTCRβ-S57C) | 1114 | 11804 |
| hTCRb-S57C-opt1-d2 (hTCRb-d2) | 1115 | 11805 |
| hTCRb-S57C-opt1-d4 (hTCRb-d4) | 1116 | 11806 |
| hTCRb-S57C-opt1-d6 (hTCRb-d6) | 1117 | 11807 |
| hTCRb-S57C-opt1-d9 (hTCRb-d9) | 1118 | 11808 |
| hTCRb-S57C-opt1-d14 (hTCRb-d14) | 1119 | 11809 |
| hTCRb-S57C-opt1-d17 (hTCRb-d17) | 1120 | 11810 |
| hTCRb-S57C-opt1-d29 (hTCRb-d29) | 1121 | 11811 |
| hTCRb-S57C-opt1-d41(hTCRb-d41) | 1122 | 11812 |
| hTCRb-S57C-opt1-d50 (hTCRb-d50) | 1123 | 11813 |
| hTCRb-S57C-opt1-d55 (hTCRb-d55) | 1124 | 11814 |
| hTCRb-S57C-opt1-d63 (hTCRb-d63) | 1125 | 11815 |
| TCRb-wt2-opt-6MD | 1126 | 11816 |
| TCRb-wt2-opt-7MD | 1127 | 11817 |
| hTCRg-Opt | 1128 | 11818 |
| hTCRg-Opt-d5 | 1129 | 11819 |
| hTCRg-Opt-d7 | 1130 | 11820 |
| hTCRg-Opt-d12 | 1131 | 11821 |
| TCRg-6MD | 1132 | 11822 |
| TCRg-7MD | 1133 | 11823 |
| hTCRd-Opt | 1134 | 11824 |
| hTCRd-opt-d2 | 1135 | 11825 |
| hTCRd-opt-d6 | 1136 | 11826 |
| hTCRd-opt-d8 | 1137 | 11827 |
| hTCRd-opt-d13 | 1138 | 11828 |
| TCRd-6MD | 1139 | 11829 |
| TCRd-7MD | 1140 | 11830 |

**TABLE 13: Ig Linker Domain derived from antibodies and TCR**

| **Name of fragment** | **SEQ ID NO (DNA)** | **SEQ ID NO (PRT)** |
|---|---|---|
| IgCL | 1142 | 11832 |
| IgG1-CH1 | 1143 | 11833 |
| IgG1-CH1-DeltaC | 1144 | 11834 |
| IgG1-CH1-Hinge | 1145 | 11835 |
| IgG1-CH1-v2 | 1146 | 11836 |
| IgG1-CH1-DeltaC-v2 | 1147 | 11837 |
| IgG1-CH1-Hinge-v2 | 1148 | 11838 |
| IgG2-0C-CH1 | 1149 | 11839 |
| IgG2-IC-CHI1 | 1150 | 11840 |
| IgG3-CHI1 | 1151 | 11841 |
| IgG4-CHI1 | 1152 | 11842 |
| IgAI-CHI1 | 1153 | 11843 |
| IgA2-CHI1 | 1154 | 11844 |
| IgD-CHI1 | 1155 | 11845 |
| IgE-CHI1 | 1156 | 11846 |
| IgM-CHI1 | 1157 | 11847 |
| TCRa-wt-opt-6ECD | 1158 | 11848 |
| TCRa-wt-opt-7ECD | 1159 | 11849 |
| TCRb-wt-opt-6ECD | 1160 | 11850 |
| TCRb-wt-opt-ECD-7ECD | 1161 | 11851 |
| TCRg-opt-6ECD | 1162 | 11852 |
| TCRg-opt-7ECD | 1163 | 11853 |
| TCRd-opt-6ECD | 1164 | 11854 |
| TCRd-opt-ECD-7ECD | 1165 | 11855 |
| TCRb-wt-opt-8ECD | 1166 | 11856 |
| TCRa-wt-opt-8ECD | 1167 | 11857 |
| TCRa-Ig-Like-C1-Domain-6MD | 1168 | 11858 |
| TCRa-Ig-Like-C1-Domain | 1169 | 11859 |
| TCRb-Ig-Like-C1-Domain-6MD | 1170 | 11860 |
| TCRb-Ig-Like-C1-Domain | 1171 | 11861 |
| TCRg-Ig-Like-C1-Domain-6MD | 1172 | 11862 |
| TCRg-Ig-Like-C1-Domain | 1173 | 11863 |
| TCRd-Ig-Like-C1-Domain | 1174 | 11864 |
| TCRd-Ig-Like-C1-Domain-6MD | 1175 | 11865 |

**TABLE 14: Exemplary Connecting Peptides**

| **Name of fragment** | **SEQ ID NO (DNA)** | **SEQ ID NO (PRT)** |
|---|---|---|
| TCRa-Connecting-Peptide-MD | 1177 | 11867 |
| TCRa-Connecting-Peptide | 1178 | 11868 |
| TCRb-Connecting-Peptide-MD | 1179 | 11869 |
| TCRb-Connecting-Peptide | 1180 | 11870 |
| TCRg-Connecting-Peptide-MD | 1181 | 11871 |
| TCRg-Connecting-Peptide | 1182 | 11872 |
| TCRd-Connecting-Peptide-NM | 1183 | 11873 |
| TCRd-Connecting-Peptide | 1184 | 11874 |
| CD3z-connecting-peptide | 1185 | 11875 |

**TABLE 15: Exemplary TCR Transmembrane Domains**

| **Name of fragment** | **SEQ ID NO (DNA)** | **SEQ ID NO (PRT)** |
|---|---|---|
| TCRa-Transmembrane-Domain | 1187 | 11877 |
| TCRb-Transmembrane-Domain | 1188 | 11878 |
| TCRg-Transmembrane-Domain | 1189 | 11879 |
| TCRd-Transmembrane-domain | 1190 | 11880 |
| CD3z-transmembrane-domain | 1191 | 11881 |

**TABLE 16: TCR Cytosolic Domains**

| **Name of fragment** | **SEQ ID NO (DNA)** | **SEQ ID NO (PRT)** |
|---|---|---|
| TCRa-Cytosolic-Domain | 1193 | 11883 |
| TCRb-Cytosolic-Domain | 1194 | 11884 |
| TCRg-Cytosolic-Domain | 1195 | 11885 |
| CD3z-Cytosolic-Domain | 1196 | 11886 |

**TABLE 17**

| **Name of fragment** | **SEQ ID NO (DNA)** | **SEQ ID NO (PRT)** |
|---|---|---|
| IgG1-Hinge | 1198 | 11888 |
| IgG2-Hinge | 1199 | 11889 |
| IgG1-CD28-spacer | 1200 | 11890 |
| lgG1-4-1BB-spacer | 1201 | 11891 |
| IgG4-(Hi-CH2-CH3)-spacer | 1202 | 11892 |
| lgG4-(Hi-CH3)-spacer | 1203 | 11893 |
| IgG4(Hi)-spacer | 1204 | 11894 |

**TABLE 18: Miscellaneous Domains**

| **Name of fragment** | **SEQ ID NO (DNA)** | **SEQ ID NO (PRT)** | **Name of fragment** | **SEQ ID NO (DNA)** | **SEQ ID NO (PRT)** |
|---|---|---|---|---|---|
| CD3z-cytosolic-domain | 1206 | 11896 | CD3zECDTM-opt2 | 1094 | 11784 |
| CD3z-cytosolic-domain | 1207 | 11897 | CD3zCP-opt2 | 1095 | 11785 |
| 4-1BB-cytosolic-domain | 1208 | 11898 | CD3zECDTMCP-opt2 | 1096 | 11786 |
| hCD8-Hinge-TM | 1209 | 11899 | CD28-CP-opt2 | 1097 | 11787 |
| hCD8-Hinge-TM-BBz | 1210 | 11900 | 41BB-CP-opt2 | 1098 | 11788 |
| hCD8TM-Hinge-BB | 1211 | 11901 | CD3e-ECD-opt2 | 23320 | 23329 |
| CD28-HingeTMCP | 1212 | 11902 | CD3d-ECD-opt2 | 23321 | 23330 |
| CD3d-ECDTMCP-opt2 | 1213 | 11903 | CD3g-ECD-opt2 | 23322 | 23331 |
| CD3eECDTMCP-opt2 | 1214 | 11904 | CD3e-TM-opt2 | 23323 | 23332 |
| CD3g-ECDTMCP-opt2 | 1215 | 11905 | CD3d-TM-opt2 | 23324 | 23333 |
| CD3zECDTMCP-opt2 | 1216 | 11906 | CD3g-TM-opt2 | 23325 | 23334 |
| CD3zECDTMCP-opt | 1090 | 11780 | CD3e-CP-opt2 | 23326 | 23335 |
| CD28-CP-opt | 1091 | 11781 | CD3d-CP-opt2 | 23327 | 23336 |
| 41BB-CP-opt | 1092 | 11782 | CD3g-CP-opt2 | 23328 | 23337 |
| CD3e-CP-opt | 1093 | 11783 | | | |

**TABLE 19: Protease Cleavable Linkers**

| **Name of fragment** | **SEQ ID NO (DNA)** | **SEQ ID NO (PRT)** |
|---|---|---|
| MMP14 | 1218 | 11908 |
| MMP14 | 1219 | 11909 |
| MMP14 | 1220 | 11910 |
| MMP2-or-MMP9 | 1221 | 11911 |
| MMP2-or-MMP9 | 1222 | 11912 |
| MMP1-or-MMP8 | 1223 | 11913 |
| MMP1-or-MMP8 | 1224 | 11914 |
| MMP1-or-MMP8 | 1225 | 11915 |
| MMP1-or-MMP8 | 1226 | 11916 |
| MMP3 | 1227 | 11917 |
| MMP3 | 1228 | 11918 |
| MMP7 | 1229 | 11919 |
| MMP7 | 1230 | 11920 |
| MMP9 | 1231 | 11921 |

**TABLE 20: Self Cleavable Linkers**

| **Name of fragment** | **SEQ ID NO (DNA)** | **SEQ ID NO (PRT)** |
|---|---|---|
| F2A | 1233 | 11923 |
| T2A | 1234 | 11924 |
| T2A | 1235 | 11925 |
| P2A | 1236 | 11926 |
| P2a-variant | 1237 | 11927 |
| E2A | 1238 | 11928 |
| SGSG | 1239 | 11929 |
| SGSG | 1240 | 11930 |
| FURINE-CLEAVAGE-SITE | 1241 | 11931 |
| FURINE-CLEAVAGE-SITE | 1242 | 11932 |
| FURINE-Cleavage-Site | 1243 | 11933 |

**TABLE 21: scFv-Nluc (Malibu Glo Reagents)**

| **Name of fragment** | **SEQ ID NO (DNA)** | **SEQ ID NO (PRT)** |
|---|---|---|
| CD8SP-FMC63-(vL-vH)-GGSG-NLuc-4xFLAG-x2STREP-8xHis-T2A-PAC | 1245 | 11935 |
| CD8SP-huFMC63-11-(vL-vH)-GGSG-NLuc-4xFLAG-x2STREP-8xHis-T2A-PAC | 1246 | 11936 |
| CD8SP-BCMA-J6M0-(vL-vH)-GGSG-NLuc-4xFLAG-x2STREP-8xHis-T2A-PAC | 1247 | 11937 |
| CD8SP-BCMA-huC12A3-L3H3-(vL-vH)-GGSG-NLuc-4xFLAG-x2STREP-8xHis-T2A-PAC | 1248 | 11938 |
| CD8SP-CD20-2F2-(vL-vH)-GGSG-NLuc-4xFLAG-x2STREP-8xHis-T2A-PAC | 1249 | 11939 |

**TABLE 22: SVH-N-Luc**

| **Target** | **Name of fragment** | **SEQ ID NO (DNA)** | **SEQ ID NO (PRT)** |
|---|---|---|---|
| CEA | CD8SP-CEA-300-aVH-G3Sx2-CEA-3001- aVH-NLuc-4xFlag-2xStreptag-8xHis-T2A-Pac | 1265 | 11955 |
| CEA | CD8SP-CEA-300-aVH-GGSG-NLuc-4xFlag- 2xStreptag-8xHis-T2A-Pac | 1266 | 11956 |
| CD20 | CD8SP-CD20-C07-vHH-G4S-CD22-FHVH- 158-NLuc-4xFlag-2xStreptag-8xHis-T2A-Pac | 1267 | 11957 |
| CD20 | CD8SP-CD20-C07-VH-GGSG-NLuc-4xFlag- 2xStreptag-8xHis-T2A-Pac | 1268 | 11958 |
| PSMA | CD8SP-PSMA71-chVH-G4Sx3-PSMA28- chVH-NLuc-4xFlag-2xStreptag-8xHis-T2A-Pac | 1269 | 11959 |
| PSMA | CD8SP-PSMA71-chVH-GGSG-NLuc-4xFlag- 2xStreptag-8xHis-T2A-Pac | 1270 | 11960 |
| PSMA | IgHSP-PSMA28v2-chVH-G4Sx2-PSMA71v2- chVH-NLuc-4xFlag-2xStreptag-8xHis-T2A-Pac | 1271 | 11961 |
| PSMA | IgHSP-PSMA28v2-chVH-GGSG-NLuc-4xFlag- 2xStreptag-8xHis-T2A-Pac | 1272 | 11962 |
| CD38 | CD8SP-CD38-309407-FHVH-GGSG-NLuc- 4xFlag-2xStreptag-8xHis-T2A-Pac | 1273 | 11963 |
| CD38 | IgHSP-CD38-663-FHVH-SG4x2-CD38-986- FHVH-NLuc-4xFlag-2xStreptag-8xHis-T2A- Pac | 1274 | 11964 |
| CD38 | IgHSP-CD38-663-FHVH-GGSG-NLuc-4xFlag- 2xStreptag-8xHis-T2A-Pac | 1275 | 11965 |
| BCMA | CD8SP-BCMA-33-FHVH-GGSG-NLuc- 4xFlag-2xStreptag-8xHis-T2A-Pac | 1276 | 11966 |
| BCMA | CD8SP-BCMA-74-FHVH-GGSG-NLuc- 4xFlag-2xStreptag-8xHis-T2A-Pac | 1277 | 11967 |
| CD22 | CD8SP-CD22-24-FHVH-G4S1-BCMA-93- FHVH-NLuc-4xFlag-2xStreptag-8xHis-T2A- Pac | 1278 | 11968 |

**TABLE 23: Extracellular-Domain-Nluc (ECD-Nluc)**

| Antigen | **Name of fragment** | **SEQ ID NO (DNA)** | **SEQ ID NO (PRT)** |
|---|---|---|---|
| MPL | MPL-ECD-GGSG-Nluc-AcV5 | 1280 | 11970 |
| CD19 | FLAG-CD19-ECD-GGSG-NLuc-AcV5 | 1281 | 11971 |
| CD19 | CD19-ECD-GGSG-NLuc-4xFlag-2xStreptag-8xHis-T2A-Pac | 1282 | 11972 |
| CD33 | CD33-ECD-GGSG-NLuc-4xFlag-2xStreptag-8xHis-T2A-Pac | 1283 | 11973 |
| CD33 | CD33-ECD-GGSG-Tluc16-4xFlag-2xStreptag-8xHis-T2A-Pac | 1284 | 11974 |
| CD33 | CD33-ECD-PaLuc1-HA-Streptag-3xHA-8xHis-T2A-pac | 1285 | 11975 |
| CD33 | CD33-ECD-Htanneri-Luc-x3Flag | 1286 | 11976 |
| CD138 | CD138-ECD-GGSG-NLuc-4xFlag-2xStreptag-8xHis-T2A-Pac | 1287 | 11977 |
| CD123 | CD123-ECD-GGSG-NLuc-4xFlag-2xStreptag-8xHis-T2A-Pac | 1288 | 11978 |
| CDH1 | CDH1-ECD-GGSG-NLuc-4xFlag-2xStreptag-8xHis-T2A-Pac | 1289 | 11979 |
| CD200R | CD200R-ECD-GGSG-NLuc-4xFlag-2xStreptag-8xHis-T2A-Pac | 1290 | 11980 |
| GPNMB | GPNMB-ECD-GGSG-NLuc-4xFlag-2xStreptag-8xHis-T2A-Pac | 1291 | 11981 |
| PTK7 | PTK7-ECD-GGSG-NLuc-4xFlag-2xStreptag-8xHis-T2A-Pac | 1292 | 11982 |
| CD34 | CD34-ECD-GGSG-NLuc-4xFlag-2xStreptag-8xHis-T2A-Pac | 1293 | 11983 |
| EpCAM | EpCAM-ECD-GGSG-NLuc-4xFlag-2xStreptag-8xHis-T2A-Pac | 1294 | 11984 |
| CLEC12A | CLEC12A-ECD-GGSG-NLuc-4xFlag-2xStreptag-8xHis-T2A- Pac | 1295 | 11985 |
| CD20 | CD20-ECx2-ECD-GGSG-TLuc16-4xFlag-2xStreptag-8xHis- T2A-Pac | 1296 | 11986 |
| CD20 | CD20-ECx1-ECD-GGSG-TurboLuc16-4xFlag-2xStreptag- 8xHis-T2A-Pac | 1297 | 11987 |
| CD22 | CD22v5-ECD-GGSG-NLuc-4xFlag-2xStreptag-8xHis-T2A-Pac | 1298 | 11988 |
| TSHR | TSHR-ECD-GGSG-NLuc-4xFlag-2xStreptag-8xHis-T2A-Pac | 1299 | 11989 |
| EGFRviii | EGFRviii-ECD-GGSG-NLuc-4xFlag-2xStreptag-8xHis-T2A- Pac | 1300 | 11990 |
| SLAMF7 | SLAMF7-CS1-ECD-GGSG-NLuc-4xFlag-2xStreptag-8xHis- T2A-Pac | 1301 | 11991 |
| PD1 | PD1-ECD-GGSG-NLuc-4xFlag-2xStreptag-8xHis-T2A-Pac | 1302 | 11992 |
| CTLA4 | CTLA4-opt-ECD-NLuc-4xFLAG-x2STREP-8xHis-T2A-PAC | 1303 | 11993 |
| NKG2D | CD8SP-NKG2D-ECD-4xFLAG-x2STREP-8xHis-T2A-PAC | 1304 | 11994 |
| PSMA | CD8SP-PSMA-ECD-4xFLAG-x2STREP-8xHis-T2A-PAC | 1305 | 11995 |
| BCMA | CD8SP-BCMA-ECD-GGSG-NLuc-4xFlag-2xStreptag-8xHis- T2A-Pac | 1306 | 11996 |

**TABLE 24: Therapeutic Controls**

| **Name of fragment** | **SEQ ID NO (DNA)** | **SEQ ID NO (PRT)** |
|---|---|---|
| PuroR_Variant-(PAC) | 1308 | 11998 |
| BlastR | 1309 | 11999 |
| CNB30 | 1310 | 12000 |
| GMCSF-SP-tEGFR | 1311 | 12001 |
| tEGFRviii | 1312 | 12002 |
| tCD19 | 1313 | 12003 |
| tBCMA | 1314 | 12004 |
| K13 | 1315 | 12005 |
| MC159 | 1316 | 12006 |
| K13-opt | 1317 | 12007 |
| icaspase-9 | 1318 | 12008 |
| IGHSP2-IL6R-304-VHH-ALB8-VHH | 1319 | 12009 |
| CD8SP2-PD1-4H1-scFv | 1320 | 12010 |
| CD8SP2-PD1-5C4-scFv | 1321 | 12011 |
| CD8SP2-CTLA4-Ipilimumab-scFv | 1322 | 12012 |
| CD8SP2-PD1-4H1-Alb8-vHH | 1323 | 12013 |
| CD8SP2-PD1-5C4-Alb8-vHH | 1324 | 12014 |
| CD8SP2-CTLA4-Ipilimumab-Alb8-vHH | 1325 | 12015 |
| IgSP-IL6-19A-scFV | 1326 | 12016 |
| IgSP-Fx06 | 1327 | 12017 |
| FKBP-K13 | 1328 | 12018 |
| FKBPX2-K13 | 1329 | 12019 |

**TABLE 25: Bispecific SIR Constructs Targeting CD19 and a second antigen targeted by SIR**

| **SIR TARGET** | **Name of fragment** | **SEQ ID NO (DNA)** | **SEQ ID NO (PRT)** |
|---|---|---|---|
| CD19 | CD8SP-CD19-FHVH-354-G4S3-FMC63-vL-[hTCRb-S57C- opt]-F-P2A-SP-FMC63-vH-[hTCRa-T48C-opt] | 1849 | 12539 |
| CD19 | CD8SP-CD19-FHVH-354-G4S3-huFMC63-11-vL-[hTCRb- S57C-opt]-F-P2A-SP-huFMC63-11-vH-[hTCRa-T48C-opt] | 1850 | 12540 |
| CD19 | CD8SP-CD19-FHVH-354-G4S3-CD19Bu12-vL-[hTCRb- S57C-opt]-F-P2A-SP-CD19Bu12-vH-[hTCRa-T48C-opt] | 1851 | 12541 |
| HIV1-gp100 | CD8SP-CD19-FHVH-354-G4S3-HIV1-N6-vL-[hTCRb-S57C- opt]-F-P2A-SP-HIV1-N6-vH-[hTCRa-T48C-opt] | 1852 | 12542 |
| ALK | CD8SP-CD19-FHVH-354-G4S3-Alk-48-vL-[hTCRb-S57C- opt]-F-P2A-SP-Alk-48-vH-[hTCRa-T48C-opt] | 1853 | 12543 |
| ALK | CD8SP-CD19-FHVH-354-G4S3-Alk-58-vL-[hTCRb-S57C- opt]-F-P2A-SP-Alk-58-vH-[hTCRa-T48C-opt] | 1854 | 12544 |
| CD45 | CD8SP-CD19-FHVH-354-G4S3-BC8-CD45-vL-[hTCRb- S57C-opt]-F-P2A-SP-BC8-CD45-vH-[hTCRa-T48C-opt] | 1855 | 12545 |
| BCMA | CD8SP-CD19-FHVH-354-G4S3-BCMA-J6M0-vL-[hTCRb- S57C-opt]-F-P2A-SP-BCMA-J6M0-vH-[hTCRa-T48C-opt] | 1856 | 12546 |
| BCMA | CD8SP-CD19-FHVH-354-G4S3-BCMA-huC12A3-L3H3-vL- [hTCRb-S57C-opt]-F-P2A-SP-BCMA-huC12A3-L3H3-vH- [hTCRa-T48C-opt] | 1857 | 12547 |
| BCMA | CD8SP-CD19-FHVH-354-G4S3-BCMA-ET-40-vL-[hTCRb- S57C-opt]-F-P2A-SP-BCMA-ET-40-vH-[hTCRa-T48C-opt] | 1858 | 12548 |
| BCMA | CD8SP-CD19-FHVH-354-G4S3-BCMA-ET-54-vL-[hTCRb- S57C-opt]-F-P2A-SP-BCMA-ET-54-vH-[hTCRa-T48C-opt] | 1859 | 12549 |
| CCR4 | CD8SP-CD19-FHVH-354-G4S3-CCR4-humAb1567-vL- [hTCRb-S57C-opt]-F-P2A-SP-CCR4-humAb1567-vH- [hTCRa-T48C-opt] | 1860 | 12550 |
| CD5 | CD8SP-CD19-FHVH-354-G4S3-CD5-9-vL-[hTCRb-S57C- opt]-F-P2A-SP-CD5-9-vH-[hTCRa-T48C-opt] | 1861 | 12551 |
| CD5 | CD8SP-CD19-FHVH-354-G4S3-CD5-18-vL-[hTCRb-S57C- opt]-F-P2A-SP-CD5-18-vH-[hTCRa-T48C-opt] | 1862 | 12552 |
| CD20 | CD8SP-CD19-FHVH-354-G4S3-CD20-2F2-vL-[hTCRb- S57C-opt]-F-P2A-SP-CD20-2F2-vH-[hTCRa-T48C-opt] | 1863 | 12553 |
| CD20 | CD8SP-CD19-FHVH-354-G4S3-CD20-GA101-vL-[hTCRb- S57C-opt]-F-P2A-SP-CD20-GA101-vH-[hTCRa-T48C-opt] | 1864 | 12554 |
| CD22 | CD8SP-CD19-FHVH-354-G4S3-CD22-h10F4v2-vL-[hTCRb- S57C-opt]-F-P2A-SP-CD22-h10F4v2-vH-[hTCRa-T48C-opt] | 1865 | 12555 |
| CD276 | CD8SP-CD19-FHVH-354-G4S3-CD22-m971-vL-[hTCRb- S57C-opt]-F-P2A-SP-CD22-m971-vH-[hTCRa-T48C-opt] | 1866 | 12556 |
| CD30 | CD8SP-CD19-FHVH-354-G4S3-CD30-5F11-vL-[hTCRb- S57C-opt]-F-P2A-SP-CD30-5F11-vH-[hTCRa-T48C-opt] | 1867 | 12557 |
| CD30 | CD8SP-CD19-FHVH-354-G4S3-CD30-Ac10-vL-[hTCRb- S57C-opt]-F-P2A-SP-CD30-Ac10-vH-[hTCRa-T48C-opt] | 1868 | 12558 |
| CD32 | CD8SP-CD19-FHVH-354-G4S3-CD32-Med9-vL-[hTCRb- S57C-opt]-F-P2A-SP-CD32-Med9-vH-[hTCRa-T48C-opt] | 1869 | 12559 |
| CD33 | CD8SP-CD19-FHVH-354-G4S3-CD33-AF5-vL-[hTCRb- S57C-opt]-F-P2A-SP-CD33-AF5-vH-[hTCRa-T48C-opt] | 1870 | 12560 |
| CD33 | CD8SP-CD19-FHVH-354-G4S3-CD33-huMyc9-vL-[hTCRb- S57C-opt]-F-P2A-SP-CD33-huMyc9-vH-[hTCRa-T48C-opt] | 1871 | 12561 |
| CD34 | CD8SP-CD19-FHVH-354-G4S3-CD34-hu4C7-vL-[hTCRb- S57C-opt]-F-P2A-SP-CD34-hu4C7-vH-[hTCRa-T48C-opt] | 1872 | 12562 |
| CD44v6 | CD8SP-CD19-FHVH-354-G4S3-CD44v6-Biwa8-vL-[hTCRb- S57C-opt]-F-P2A-SP-CD44v6-Biwa8-vH-[hTCRa-T48C-opt] | 1873 | 12563 |
| CD70 | CD8SP-CD19-FHVH-354-G4S3-CD70-h1F6-vL-[hTCRb- S57C-opt]-F-P2A-SP-CD70-h1F6-vH-[hTCRa-T48C-opt] | 1874 | 12564 |
| CD79b | CD8SP-CD19-FHVH-354-G4S3-CD79b-2F2-vL-[hTCRb- S57C-opt]-F-P2A-SP-CD79b-2F2-vH-[hTCRa-T48C-opt] | 1875 | 12565 |
| CD123 | CD8SP-CD19-FHVH-354-G4S3-CD123-CSL362-vL- [hTCRb-S57C-opt]-F-P2A-SP-CD123-CSL362-vH-[hTCRa- T48C-opt] | 1876 | 12566 |
| CD138 | CD8SP-CD19-FHVH-354-G4S3-CD138-vL-[hTCRb-S57C- opt]-F-P2A-SP-CD138-vH-[hTCRa-T48C-opt] | 1877 | 12567 |
| CD179b | CD8SP-CD19-FHVH-354-G4S3-CD179b-vL-[hTCRb-S57C- opt]-F-P2A-SP-CD179b-vH-[hTCRa-T48C-opt] | 1878 | 12568 |
| CD276 | CD8SP-CD19-FHVH-354-G4S3-CD276-17-vL-[hTCRb- S57C-opt]-F-P2A-SP-CD276-17-vH-[hTCRa-T48C-opt] | 1879 | 12569 |
| CD324 | CD8SP-CD19-FHVH-354-G4S3-CD324-SC10-6-vL-[hTCRb- S57C-opt]-F-P2A-SP-CD324-SC10-6-vH-[hTCRa-T48C-opt] | 1880 | 12570 |
| CD324 | CD8SP-CD19-FHVH-354-G4S3-CD324-hSC10-17-vL- [hTCRb-S57C-opt]-F-P2A-SP-CD324-hSC10-17-vH-[hTCRa- T48C-opt] | 1881 | 12571 |
| CDH6 | CD8SP-CD19-FHVH-354-G4S3-CDH6-NOV710-vL- [hTCRb-S57C-opt]-F-P2A-SP-CDH6-NOV710-vH-[hTCRa- T48C-opt] | 1882 | 12572 |
| CDH6 | CD8SP-CD19-FHVH-354-G4S3-CDH6-NOV712-vL- [hTCRb-S57C-opt]-F-P2A-SP-CDH6-NOV712-vH-[hTCRa- T48C-opt] | 1883 | 12573 |
| CDH17 | CD8SP-CD19-FHVH-354-G4S3-CDH17-PTA001A4-vL- [hTCRb-S57C-opt]-F-P2A-SP-CDH17-PTA001A4-vH- [hTCRa-T48C-opt] | 1884 | 12574 |
| CDH19 | CD8SP-CD19-FHVH-354-G4S3-CDH19-16A4-vL-[hTCRb- S57C-opt]-F-P2A-SP-CDH19-16A4-vH-[hTCRa-T48C-opt] | 1885 | 12575 |
| EGFR | CD8SP-CD19-FHVH-354-G4S3-Cetuximab-vL-[hTCRb- S57C-opt]-F-P2A-SP-Cetuximab-vH-[hTCRa-T48C-opt] | 1886 | 12576 |
| CLEC5A | CD8SP-CD19-FHVH-354-G4S3-CLEC5A-8H8F5-vL- [hTCRb-S57C-opt]-F-P2A-SP-CLEC5A-8H8F5-vH-[hTCRa- T48C-opt] | 1887 | 12577 |
| CLEC5A | CD8SP-CD19-FHVH-354-G4S3-CLEC5A-3E12A2-vL- [hTCRb-S57C-opt]-F-P2A-SP-CLEC5A-3E12A2-vH- [hTCRa-T48C-opt] | 1888 | 12578 |
| CLL1 | CD8SP-CD19-FHVH-354-G4S3-CLL1-M26-vL-[hTCRb- S57C-opt]-F-P2A-SP-CLL1-M26-vH-[hTCRa-T48C-opt] | 1889 | 12579 |
| CSF2RA | CD8SP-CD19-FHVH-354-G4S3-CLL1-M32-vL-[hTCRb- S57C-opt]-F-P2A-SP-CLL1-M32-vH-[hTCRa-T48C-opt] | 1890 | 12580 |
| CS1 | CD8SP-CD19-FHVH-354-G4S3-HuLuc64-vL-[hTCRb-S57C- opt]-F-P2A-SP-HuLuc64-vH-[hTCRa-T48C-opt] | 1891 | 12581 |
| CS1 | CD8SP-CD19-FHVH-354-G4S3-CS1-huLuc90-vL-[hTCRb- S57C-opt]-F-P2A-SP-huLuc90-vH-[hTCRa-T48C-opt] | 1892 | 12582 |
| CSF2RA | CD8SP-CD19-FHVH-354-G4S3-CSF2RA-Ab6-vL-[hTCRb- S57C-opt]-F-P2A-SP-CSF2RA-Ab6-vH-[hTCRa-T48C-opt] | 1893 | 12583 |
| CSF2RA | CD8SP-CD19-FHVH-354-G4S3-CSF2RA-Ab1-vL-[hTCRb- S57C-opt]-F-P2A-SP-CSF2RA-Ab1-vH-[hTCRa-T48C-opt] | 1894 | 12584 |
| EGFRviii | CD8SP-CD19-FHVH-354-G4S3-EGFRvIII-139-vL-[hTCRb- S57C-opt]-F-P2A-SP-EGFRvIII-139-vH-[hTCRa-T48C-opt] | 1895 | 12585 |
| EGFRviii | CD8SP-CD19-FHVH-354-G4S3-EGFRvIII-2173-vH- [hTCRb-S57C-opt]-F-P2A-SP-EGFRvIII-2173-vH-[hTCRa- T48C-opt] | 1896 | 12586 |
| EpCAM | CD8SP-CD19-FHVH-354-G4S3-Epcam1-MM1-vL-[hTCRb- S57C-opt]-F-P2A-SP-Epcam1-MM1-vH-[hTCRa-T48C-opt] | 1897 | 12587 |
| EpCAM | CD8SP-CD19-FHVH-354-G4S3-Epcam1-D5K5-vL-[hTCRb- S57C-opt]-F-P2A-SP-Epcam1-D5K5-vH-[hTCRa-T48C-opt] | 1898 | 12588 |
| FLT3 | CD8SP-CD19-FHVH-354-G4S3-FLT3-NC7-vL-[hTCRb- S57C-opt]-F-P2A-SP-FLT3-NC7-vH-[hTCRa-T48C-opt] | 1899 | 12589 |
| Folate Receptor-1 | CD8SP-CD19-FHVH-354-G4S3-FR1-huMov19-vL-[hTCRb- S57C-opt]-F-P2A-SP-FR1-huMov19-vH-[hTCRa-T48C-opt] | 1900 | 12590 |
| GD2 | CD8SP-CD19-FHVH-354-G4S3-GD2-hu14-18-vL-[hTCRb- S57C-opt]-F-P2A-SP-GD2-hu14-18-vH-[hTCRa-T48C-opt] | 1901 | 12591 |
| GD2 | CD8SP-CD19-FHVH-354-G4S3-GD2-hu3F8-vL-[hTCRb- S57C-opt]-F-P2A-SP-GD2-hu3F8-vH-[hTCRa-T48C-opt] | 1902 | 12592 |
| GD3 | CD8SP-CD19-FHVH-354-G4S3-GD3-KM-641-vL-[hTCRb- S57C-opt]-F-P2A-SP-GD3-KM-641-vH-[hTCRa-T48C-opt] | 1903 | 12593 |
| GFR4 | CD8SP-CD19-FHVH-354-G4S3-GFRAlpha4-P4-6-vL- [hTCRb-S57C-opt]-F-P2A-SP-GFRAlpha4-P4-6-vH-[hTCRa- T48C-opt] | 1904 | 12594 |
| GFR4 | CD8SP-CD19-FHVH-354-G4S3-GFRa4-P4-10-vL-[hTCRb- S57C-opt]-F-P2A-SP-GFRa4-P4-10-vH-[hTCRa-T48C-opt] | 1905 | 12595 |
| GM1 | CD8SP-CD19-FHVH-354-G4S3-GM1-5B2-vL-[hTCRb- S57C-opt]-F-P2A-SP-GM1-5B2-vH-[hTCRa-T48C-opt] | 1906 | 12596 |
| GM1 | CD8SP-CD19-FHVH-354-G4S3-GM1-7E5-vL-[hTCRb- S57C-opt]-F-P2A-SP-GM1-7E5-vH-[hTCRa-T48C-opt] | 1907 | 12597 |
| GRPC5D | CD8SP-CD19-FHVH-354-G4S3-GPRC5D-ET150-5-vL- [hTCRb-S57C-opt]-F-P2A-SP-GPRC5D-ET150-5-vH- [hTCRa-T48C-opt] | 1908 | 12598 |
| GRPC5D | CD8SP-CD19-FHVH-354-G4S3-GPRC5D-ET150-18-vL- [hTCRb-S57C-opt]-F-P2A-SP-GPRC5D-ET150-18-vH- [hTCRa-T48C-opt] | 1909 | 12599 |
| GPC3 | CD8SP-CD19-FHVH-354-G4S3-GPC3-4E5-vL-[hTCRb- S57C-opt]-F-P2A-SP-GPC3-4E5-vH-[hTCRa-T48C-opt] | 1910 | 12600 |
| gpNMB | CD8SP-CD19-FHVH-354-G4S3-gpNMB-115-vL-[hTCRb- S57C-opt]-F-P2A-SP-gpNMB-115-vH-[hTCRa-T48C-opt] | 1911 | 12601 |
| GRP78 | CD8SP-CD19-FHVH-354-G4S3-GRP78-GC18-vL-[hTCRb- S57C-opt]-F-P2A-SP-GRP78-GC18-vH-[hTCRa-T48C-opt] | 1912 | 12602 |
| Her2 | CD8SP-CD19-FHVH-354-G4S3-Her2-Hu4D5-vL-[hTCRb- S57C-opt]-F-P2A-SP-Her2-Hu4D5-vH-[hTCRa-T48C-opt] | 1913 | 12603 |
| HIV1-gp100 | CD8SP-CD19-FHVH-354-G4S3-HIV1-E5-vL-[hTCRb-S57C- opt]-F-P2A-SP-HIV1-E5-vH-[hTCRa-T48C-opt] | 1914 | 12604 |
| HIV1-gp100 | CD8SP-CD19-FHVH-354-G4S3-HIV1-3BNC117-vL- [hTCRb-S57C-opt]-F-P2A-SP-HIV1-3BNC117-vH-[hTCRa- T48C-opt] | 1915 | 12605 |
| HIV1-gp100 | CD8SP-CD19-FHVH-354-G4S3-HIV1-PGT-128-vL-[hTCRb- S57C-opt]-F-P2A-SP-vH-[hTCRa-T48C-opt] | 1916 | 12606 |
| HIV1-gp100 | CD8SP-CD19-FHVH-354-G4S3-HIV1-VR-C01-vL-[hTCRb- S57C-opt]-F-P2A-SP-HIV1-VR-C01-vH-[hTCRa-T48C-opt] | 1917 | 12607 |
| HIV1-gp100 | CD8SP-CD19-FHVH-354-G4S3-HIV1-X5-vL-[hTCRb-S57C- opt]-F-P2A-SP-HIV1-X5-vH-[hTCRa-T48C-opt] | 1918 | 12608 |
| IL11Ra | CD8SP-CD19-FHVH-354-G4S3-IL11Ra-8E2-Ts107-vL- [hTCRb-S57C-opt]-F-P2A-SP-IL11Ra-8E2-Ts107-vH- [hTCRa-T48C-opt] | 1919 | 12609 |
| IL13Ra2 | CD8SP-CD19-FHVH-354-G4S3-IL13Ra2-hu107-vL-[hTCRb- S57C-opt]-F-P2A-SP-IL13Ra2-hu107vH-[hTCRa-T48C-opt] | 1920 | 12610 |
| IL13Ra2 | CD8SP-CD19-FHVH-354-G4S3-IL13Ra2-Hu108-vL- [hTCRb-S57C-opt]-F-P2A-SP-IL13Ra2-Hu108-vH-[hTCRa- T48C-opt] | 1921 | 12611 |
| LAMP1 | CD8SP-CD19-FHVH-354-G4S3-LAMP1-humab1-2-vL- [hTCRb-S57C-opt]-F-P2A-SP-LAMP1-humab1-2vH-[hTCRa- T48C-opt] | 1922 | 12612 |
| LAMP1 | CD8SP-CD19-FHVH-354-G4S3-LAMP1-Mb4-vL-[hTCRb- S57C-opt]-F-P2A-SP-LAMP1-Mb4-vH-[hTCRa-T48C-opt] | 1923 | 12613 |
| L1CAM | CD8SP-CD19-FHVH-354-G4S3-L1CAM-9-3-HU3-vL- [hTCRb-S57C-opt]-F-P2A-SP-L1CAM-9-3-HU3-vH-[hTCRa- T48C-opt] | 1924 | 12614 |
| Lym1 | CD8SP-CD19-FHVH-354-G4S3-Lym1-vL-[hTCRb-S57C- opt]-F-P2A-SP-Lym1-vH-[hTCRa-T48C-opt] | 1925 | 12615 |
| Lym2 | CD8SP-CD19-FHVH-354-G4S3-Lym2-vL-[hTCRb-S57C- opt]-F-P2A-SP-Lym2-vH-[hTCRa-T48C-opt] | 1926 | 12616 |
| MPL/TPO -R | CD8SP-CD19-FHVH-354-G4S3-MPL-175-vL-[hTCRb- S57C-opt]-F-P2A-SP-175-vH-[hTCRa-T48C-opt] | 1927 | 12617 |
| MPL/TPO -R | CD8SP-CD19-FHVH-354-G4S3-MPL-161-vL-[hTCRb- S57C-opt]-F-P2A-SP-161-vH-[hTCRa-T48C-opt] | 1928 | 12618 |
| TCRB1 | CD8SP-CD19-FHVH-354-G4S3-TCRB1-CP01-E09-vL- [hTCRb-S57C-opt]-F-P2A-SP-TCRB1-CP01-E09-vH- [hTCRa-T48C-opt] | 1929 | 12619 |
| TCRB1 | CD8SP-CD19-FHVH-354-G4S3-TCRB1-Jovi1-vL-[hTCRb- S57C-opt]-F-P2A-SP-TCRB1-Jovi1-vH-[hTCRa-T48C-opt] | 1930 | 12620 |
| TCRB2 | CD8SP-CD19-FHVH-354-G4S3-TCRB2-CP01-D05-vL- [hTCRb-S57C-opt]-F-P2A-SP-TCRB2-CP01-D05-vH- [hTCRa-T48C-opt] | 1931 | 12621 |
| TCRB2 | CD8SP-CD19-FHVH-354-G4S3-TCRB2-CP01-E05-vL- [hTCRb-S57C-opt]-F-P2A-SP-TCRB2-CP01-E05-vH- [hTCRa-T48C-opt] | 1932 | 12622 |
| TCRgd | CD8SP-CD19-FHVH-354-G4S3-TCRgd-G5-4-vL-[hTCRb- S57C-opt]-F-P2A-SP-TCRgd-G5-4-vH-[hTCRa-T48C-opt] | 1933 | 12623 |
| TGFBR2 | CD8SP-CD19-FHVH-354-G4S3-TGFBR2-Ab1-vL-[hTCRb- S57C-opt]-F-P2A-SP-TGFBR2-Ab1-vH-[hTCRa-T48C-opt] | 1934 | 12624 |
| TIM1 | CD8SP-CD19-FHVH-354-G4S3-TIM1-HVCR1-270-2-vL- [hTCRb-S57C-opt]-F-P2A-SP-TIM1-HVCR1-270-2-vH- [hTCRa-T48C-opt] | 1935 | 12625 |
| TIM1 | CD8SP-CD19-FHVH-354-G4S3-TIM1-HVCR1-ARD5-vL- [hTCRb-S57C-opt]-F-P2A-SP-TIM1-HVCR1-ARD5vH- [hTCRa-T48C-opt] | 1936 | 12626 |
| TnAg | CD8SP-CD19-FHVH-354-G4S3-TnAg-vL-[hTCRb-S57C- opt]-F-P2A-SP-TnAg-vH-[hTCRa-T48C-opt] | 1937 | 12627 |
| Tn-Muc1 | CD8SP-CD19-FHVH-354-G4S3-TnMuc1-hu5E5-RHA8- RKA-2-vL-[hTCRb-S57C-opt]-F-P2A-SP-TnMuc1-hu5E5- RHA8-RKA-2vH-[hTCRa-T48C-opt] | 1938 | 12628 |
| TROP2 | CD8SP-CD19-FHVH-354-G4S3-TROP2-ARA47-HV3KV3- vL-[hTCRb-S57C-opt]-F-P2A-SP-TROP2-ARA47-HV3KV3- vH-[hTCRa-T48C-opt] | 1939 | 12629 |
| TROP2 | CD8SP-CD19-FHVH-354-G4S3-TROP2-h7E6-SVG-vL- [hTCRb-S57C-opt]-F-P2A-SP-TROP2-h7E6-SVG-vH- [hTCRa-T48C-opt] | 1940 | 12630 |
| TSLPR | CD8SP-CD19-FHVH-354-G4S3-TSLPR-vL-[hTCRb-S57C- opt]-F-P2A-SP-TSLPR-vH-[hTCRa-T48C-opt] | 1941 | 12631 |
| Tyrosinase | CD8SP-CD19-FHVH-354-G4S3-Tyros-B2-vL-[hTCRb-S57C- opt]-F-P2A-SP-Tyros-B2-vH-[hTCRa-T48C-opt] | 1942 | 12632 |
| Tyrosinase | CD8SP-CD19-FHVH-354-G4S3-Tyros-MC1-vL-[hTCRb- S57C-opt]-F-P2A-SP-Tyros-MC1-vH-[hTCRa-T48C-opt] | 1943 | 12633 |
| VGFR3 | CD8SP-CD19-FHVH-354-G4S3-VEGFR3-Ab1-vL-[hTCRb- S57C-opt]-F-P2A-SP-VEGFR3-Ab1-vH-[hTCRa-T48C-opt] | 1944 | 12634 |
| WT1/HLA -A2 | CD8SP-CD19-FHVH-354-G4S3-WT1-Ab5-vL-[hTCRb- S57C-opt]-F-P2A-SP-WT1-Ab5-vH-[hTCRa-T48C-opt] | 1945 | 12635 |
| WT1/HLA -A2 | CD8SP-CD19-FHVH-354-G4S3-MYC3-WT1-Ab13-vL- [hTCRb-S57C-opt]-F-P2A-SP-WT1-Ab13-vH-[hTCRa-T48C- opt] | 1946 | 12636 |
| WT1/HLA -A2 | CD8SP-CD19-FHVH-354-G4S3-MYC3-WT1-Ab15-vL- [hTCRb-S57C-opt]-F-P2A-SP-WT1-Ab15-vH-[hTCRa-T48C- opt] | 1947 | 12637 |
| CD123 | CD8SP-CD19-FHVH-354-G4S3-CD123-1172-vL-[hTCRb- S57C-opt]-F-P2A-SP-CD123-1172-vH-[hTCRa-T48C-opt] | 1948 | 12638 |
| CDH19 | CD8SP-CD19-FHVH-354-G4S3-CDH19-4B10-vL-[hTCRb- S57C-opt]-F-P2A-SP-CDH19-4B10-vH-[hTCRa-T48C-opt] | 1949 | 12639 |
| Folate Receptor-beta | CD8SP-CD19-FHVH-354-G4S3-FRbeta-m923-vL-[hTCRb- S57C-opt]-F-P2A-SP-FRbeta-m923-vH-[hTCRa-T48C-opt] | 1950 | 12640 |
| LHR | CD8SP-CD19-FHVH-354-G4S3-LHR-8B7-vL-[hTCRb- S57C-opt]-F-P2A-SP-LHR-8B7-vH-[hTCRa-T48C-opt] | 1951 | 12641 |
| LHR | CD8SP-CD19-FHVH-354-G4S3-LHR-5F4-21-vL-[hTCRb- S57C-opt]-F-P2A-SP-LHR-5F4-21-vH-[hTCRa-T48C-opt] | 1952 | 12642 |
| B7H4 | CD8SP-CD19-FHVH-354-G4S3-B7H4-hu22Cl0-vL-[hTCRb- S57C-opt]-F-P2A-SP-B7H4-hu22Cl0-vH-[hTCRa-T48C-opt] | 1953 | 12643 |
| B7H4 | CD8SP-CD19-FHVH-354-G4S3-B7H4-hu1D11-vL-[hTCRb- S57C-opt]-F-P2A-SP-B7H4-hu1D11-vH-[hTCRa-T48C-opt] | 1954 | 12644 |
| CD23 | CD8SP-CD19-FHVH-354-G4S3-CD23-p5E8-vL-[hTCRb- S57C-opt]-F-P2A-SP-CD23-p5E8-vH-[hTCRa-T48C-opt] | 1955 | 12645 |
| GCC | CD8SP-CD19-FHVH-354-G4S3-GCC-5F9-vL-[hTCRb- S57C-opt]-F-P2A-SP-GCC-5F9-vH-[hTCRa-T48C-opt] | 1956 | 12646 |
| GCC | CD8SP-CD19-FHVH-354-G4S3-GCC-Ab229-vL-[hTCRb- S57C-opt]-F-P2A-SP-GCC-Ab229-vH-[hTCRa-T48C-opt] | 1957 | 12647 |
| CD200R | CD8SP-CD19-FHVH-354-G4S3-CD200R-huDx182-vL- [hTCRb-S57C-opt]-F-P2A-SP-CD200R-huDx182-vH- [hTCRa-T48C-opt] | 1958 | 12648 |
| AFP | CD8SP-CD19-FHVH-354-G4S3-AFP-76-vL-[hTCRb-S57C- opt]-F-P2A-SP-AFP-76-vH-[hTCRa-T48C-opt] | 1959 | 12649 |
| AFP | CD8SP-CD19-FHVH-354-G4S3-AFP-79-vL-[hTCRb-S57C- opt]-F-P2A-SP-AFP-79-vH-[hTCRa-T48C-opt] | 1960 | 12650 |
| BCMA | CD8SP-CD19-FHVH-354-G4S3-BCMA-ET-03-vL-[hTCRb- S57C-opt]-F-P2A-SP-BCMA-ET-03-vH-[hTCRa-T48C-opt] | 1961 | 12651 |
| CD123 | CD8SP-CD19-FHVH-354-G4S3-CD123-DART-1-vL- [hTCRb-S57C-opt]-F-P2A-SP-CD123-DART-1-vH-[hTCRa- T48C-opt] | 1962 | 12652 |
| CD123 | CD8SP-CD19-FHVH-354-G4S3-CD123-DART-2-vL- [hTCRb-S57C-opt]-F-P2A-SP-CD123-DART-2-vH-[hTCRa- T48C-opt] | 1963 | 12653 |
| CD123 | CD8SP-CD19-FHVH-354-G4S3-CD123-1176-vL-[hTCRb- S57C-opt]-F-P2A-SP-CD123-1176-vH-[hTCRa-T48C-opt] | 1964 | 12654 |
| CD123 | CD8SP-CD19-FHVH-354-G4S3-CD123-2B8-vL-[hTCRb- S57C-opt]-F-P2A-SP-CD123-2B8-vH-[hTCRa-T48C-opt] | 1965 | 12655 |
| CD123 | CD8SP-CD19-FHVH-354-G4S3-CD123-9D7-vL-[hTCRb- S57C-opt]-F-P2A-SP-CD123-9D7-vH-[hTCRa-T48C-opt] | 1966 | 12656 |
| CD123 | CD8SP-CD19-FHVH-354-G4S3-CD123-3B10-vL-[hTCRb- S57C-opt]-F-P2A-SP-CD123-3B10-vH-[hTCRa-T48C-opt] | 1967 | 12657 |
| CD19 | CD8SP-CD19-FHVH-354-G4S3-CD19-MOR0028-vL- [hTCRb-S57C-opt]-F-P2A-SP-CD19-MOR0028-vH-[hTCRa- T48C-opt] | 1968 | 12658 |
| CD19 | CD8SP-CD19-FHVH-354-G4S3-CD19-hu-mROO5-1-vL- [hTCRb-S57C-opt]-F-P2A-SP-CD19-hu-mROO5-1-vH- [hTCRa-T48C-opt] | 1969 | 12659 |
| CD20 | CD8SP-CD19-FHVH-354-G4S3-CD20-Ubli-v4-vL-[hTCRb- S57C-opt]-F-P2A-SP-CD20-Ubli-v4-vH-[hTCRa-T48C-opt] | 1970 | 12660 |
| CD20 | CD8SP-CD19-FHVH-354-G4S3-CD20-7D8-vL-[hTCRb- S57C-opt]-F-P2A-SP-CD20-7D8-vH-[hTCRa-T48C-opt] | 1971 | 12661 |
| CD33 | CD8SP-CD19-FHVH-354-G4S3-CD33-SGNh2H12-vL- [hTCRb-S57C-opt]-F-P2A-SP-CD33-SGNh2H12-vH- [hTCRa-T48C-opt] | 1972 | 12662 |
| CD99 | CD8SP-CD19-FHVH-354-G4S3-CD99-hu12E7-vL-[hTCRb- S57C-opt]-F-P2A-SP-CD99-hu12E7-vH-[hTCRa-T48C-opt] | 1973 | 12663 |
| CLL1 | CD8SP-CD19-FHVH-354-G4S3-CLL1-21C9-L2H3-vL- [hTCRb-S57C-opt]-F-P2A-SP-CLL1-21C9-L2H3-vH- [hTCRa-T48C-opt] | 1974 | 12664 |
| CLL1 | CD8SP-CD19-FHVH-354-G4S3-CLL1-6E7L4H1e-vL- [hTCRb-S57C-opt]-F-P2A-SP-CLL1-6E7L4H1e-vH-[hTCRa- T48C-opt] | 1975 | 12665 |
| CLL1 | CD8SP-CD19-FHVH-354-G4S3-CLL1-hu1075-v1-vL- [hTCRb-S57C-opt]-F-P2A-SP-CLL1-hu1075-v1-vH-[hTCRa- T48C-opt] | 1976 | 12666 |
| CLL1 | CD8SP-CD19-FHVH-354-G4S3-CLL1-hu1075-v2-vL- [hTCRb-S57C-opt]-F-P2A-SP-CLL1-hu1075-v2-vH-[hTCRa- T48C-opt] | 1977 | 12667 |
| FITC | CD8SP-CD19-FHVH-354-G4S3-FITC-4M-53-vL-[hTCRb- S57C-opt]-F-P2A-SP-FITC-4M-53-vH-[hTCRa-T48C-opt] | 1978 | 12668 |
| FITC | CD8SP-CD19-FHVH-354-G4S3-FITC-E2-vH-[hTCRb-S57C- opt]-F-P2A-SP-FITC-E2-vL-[hTCRa-T48C-opt] | 1979 | 12669 |
| HLA-A2 | CD8SP-CD19-FHVH-354-G4S3-HLA-A2-3PB2-vL-[hTCRb- S57C-opt]-F-P2A-SP-HLA-A2-3PB2-vH-[hTCRa-T48C-opt] | 1980 | 12670 |
| HPV16 | CD8SP-CD19-FHVH-354-G4S3-HPV16-7-8-vL-[hTCRb- S57C-opt]-F-P2A-SP-HPV16-7-8-vH-[hTCRa-T48C-opt] | 1981 | 12671 |
| HPV16 | CD8SP-CD19-FHVH-354-G4S3-HPV16-2-vL-[hTCRb-S57C- opt]-F-P2A-SP-HPV16-2-vH-[hTCRa-T48C-opt] | 1982 | 12672 |
| CD22 | CD8SP-CD19-FHVH-354-G4S3-CD22-5-vH-[hTCRb-S57C- opt]-F-P2A-SP-CD22-5-vL-[hTCRa-T48C-opt] | 1983 | 12673 |
| CD22 | CD8SP-CD19-FHVH-354-G4S3-CD22-10-vH-[hTCRb-S57C- opt]-F-P2A-SP-CD22-10-vL-[hTCRa-T48C-opt] | 1984 | 12674 |
| CD22 | CD8SP-CD19-FHVH-354-G4S3-CD22-31-vH-[hTCRb-S57C- opt]-F-P2A-SP-CD22-31-vL-[hTCRa-T48C-opt] | 1985 | 12675 |
| CD22 | CD8SP-CD19-FHVH-354-G4S3-CD22-53-vH-[hTCRb-S57C- opt]-F-P2A-SP-CD22-53-vL-[hTCRa-T48C-opt] | 1986 | 12676 |
| CD22 | CD8SP-CD19-FHVH-354-G4S3-CD22-65-vH-[hTCRb-S57C- opt]-F-P2A-SP-CD22-65-vL-[hTCRa-T48C-opt] | 1987 | 12677 |
| Kappa-Light Chain | CD8SP-CD19-FHVH-354-G4S3-Kappa-LC1-vL-[hTCRb- S57C-opt]-F-P2A-SP-Kappa-LC1-vH-[hTCRa-T48C-opt] | 1988 | 12678 |
| PTK7 | CD8SP-CD19-FHVH-354-G4S3-PTK7-7C8-vL-[hTCRb- S57C-opt]-F-P2A-SP-PTK7-7C8-vH-[hTCRa-T48C-opt] | 1989 | 12679 |
| PTK7 | CD8SP-CD19-FHVH-354-G4S3-PTK7-12C6a-vL-[hTCRb- S57C-opt]-F-P2A-SP-PTK7-12C6a-vH-[hTCRa-T48C-opt] | 1990 | 12680 |
| CD19 | CD8SP-CD19-FHVH-354-G4S3-hCD19-EUK5-13-vL- [hTCRb-S57C-opt]-F-P2A-SP-hCD19-EUK5-13-vH-[hTCRa- T48C-opt] | 1991 | 12681 |
| Streptag | CD8SP-CD19-FHVH-354-G4S3-Streptag-vL-[hTCRb-S57C- opt]-F-P2A-SP-Streptag-vH-[hTCRa-T48C-opt] | 1992 | 12682 |
| MPL | CD8SP-CD19-FHVH-354-G4S3-MPL-hu-161-2-vL-[hTCRb- S57C-opt]-F-P2A-SP-MPL-hu-161-2-vH-[hTCRa-T48C-opt] | 1993 | 12683 |
| BCMA | CD8SP-CD19-FHVH-354-G4S3-BCMA-huC12A3-L3H3- vL2-[hTCRb-S57C-opt]-F-P2A-SP-BCMA-huC12A3-L3H3- vH2-[hTCRa-T48C-opt] | 1994 | 12684 |
| BCMA | CD8SP-CD19-FHVH-354-G4S3-BCMA-huC13-F12-L1H2- vL2-[hTCRb-S57C-opt]-F-P2A-SP-BCMA-huC13-F12-L1H2- vH2-[hTCRa-T48C-opt] | 1995 | 12685 |
| CD179a | CD8SP-CD19-FHVH-354-G4S3-CD179a-2460-B04-vL- [hTCRb-S57C-opt]-F-P2A-SP-CD179a-2460-B04-vH- [hTCRa-T48C-opt] | 1996 | 12686 |
| CD179a | CD8SP-CD19-FHVH-354-G4S3-CD179a-2462-E07-vL- [hTCRb-S57C-opt]-F-P2A-SP-CD179a-2462-E07-vH- [hTCRa-T48C-opt] | 1997 | 12687 |
| MPL | CD8SP-CD19-FHVH-354-G4S3-MPL-hu-175-2-vL-[hTCRb- S57C-opt]-F-P2A-SP-MPL-hu-175-2-vH-[hTCRa-T48C-opt] | 1998 | 12688 |
| MPL | CD8SP-CD19-FHVH-354-G4S3-MPL-hu-111-2-vL-[hTCRb- S57C-opt]-F-P2A-SP-MPL-hu-111-2-vH-[hTCRa-T48C-opt] | 1999 | 12689 |
| CD22 | CD8SP-CD19-FHVH-354-G4S3-CD22-HA22-vL-[hTCRb- S57C-opt]-F-P2A-SP-CD22-HA22-vH-[hTCRa-T48C-opt] | 2000 | 12690 |
| STEAP1 | CD8SP-CD19-FHVH-354-G4S3-STEAP1-hu120-vL-[hTCRb- S57C-opt]-F-P2A-SP-STEAP1-hu120-vH-[hTCRa-T48C-opt] | 2001 | 12691 |
| hLiv1 | CD8SP-CD19-FHVH-354-G4S3-hLiv1-mAb2-vL-[hTCRb- S57C-opt]-F-P2A-SP-hLiv1-mAb2-vH-[hTCRa-T48C-opt] | 2002 | 12692 |
| Nectin4 | CD8SP-CD19-FHVH-354-G4S3-hu-Nectin4-mAb1-vL- [hTCRb-S57C-opt]-F-P2A-SP-hu-Nectin4-mAb1-vH-[hTCRa- T48C-opt] | 2003 | 12693 |
| Cripto | CD8SP-CD19-FHVH-354-G4S3-hu-Cripto-L1H2-vL- [hTCRb-S57C-opt]-F-P2A-SP-hu-Cripto-L1H2-vH-[hTCRa- T48C-opt] | 2004 | 12694 |
| gpA33 | CD8SP-CD19-FHVH-354-G4S3-hu-gpA33-vL-[hTCRb- S57C-opt]-F-P2A-SP-hu-gpA33-vH-[hTCRa-T48C-opt] | 2005 | 12695 |
| ROR1 | CD8SP-CD19-FHVH-354-G4S3-ROR1-DART4-vL-[hTCRb- S57C-opt]-F-P2A-SP-ROR1-DART4-vH-[hTCRa-T48C-opt] | 2006 | 12696 |
| FLT3 | CD8SP-CD19-FHVH-354-G4S3-FLT3-8B5-vL-[hTCRb- S57C-opt]-F-P2A-SP-FLT3-8B5-vH-[hTCRa-T48C-opt] | 2007 | 12697 |
| FLT3 | CD8SP-CD19-FHVH-354-G4S3-FLT3-10E3-vL-[hTCRb- S57C-opt]-F-P2A-SP-FLT3-10E3-vH-[hTCRa-T48C-opt] | 2008 | 12698 |
| BCMA | CD8SP-CD19-FHVH-354-G4S3-BCMA-BB-CAR02-vL- [hTCRb-S57C-opt]-F-P2A-SP-BCMA-BB-CAR02-vH- [hTCRa-T48C-opt] | 2009 | 12699 |
| CLL1 | CD8SP-CD19-FHVH-354-G4S3-CLL1-24C1-vL-[hTCRb- S57C-opt]-F-P2A-SP-CLL1-24C1-vH-[hTCRa-T48C-opt] | 2010 | 12700 |
| CLL1 | CD8SP-CD19-FHVH-354-G4S3-CLL1-24C8-vL-[hTCRb- S57C-opt]-F-P2A-SP-CLL1-24C8-vH-[hTCRa-T48C-opt] | 2011 | 12701 |
| MSLN | CD8SP-CD19-FHVH-354-G4S3-MSLN-7D9-v3-vL-[hTCRb- S57C-opt]-F-P2A-SP-MSLN-7D9-v3-vH-[hTCRa-T48C-opt] | 2012 | 12702 |
| MSLN | CD8SP-CD19-FHVH-354-G4S3-MSLN-hu22A10-vL- [hTCRb-S57C-opt]-F-P2A-SP-MSLN-hu22A10-vH-[hTCRa- T48C-opt] | 2013 | 12703 |

**TABLE 26. Bispecifics and Trispecific Constructs on different backbones targeting different antigens. These constructs were generated by changing the [hTCRb-S57C-opt] and [hTCRa-T48C-opt] modules of the construct in Table 25 with the modules shown in the first construct of each series in the Table below. The order of the vL/vH fragments of the constructs of each series in Table 26 is the same as the order of vL/vH chains of the constructs of series 1 whose details are provided in Table 25.**

| **TABLE 26: Bispecifics and Trispecific Constructs Targeting Different Antigens** | | | | | | |
|---|---|---|---|---|---|---|
| **1^{st} Target** | **2^{nd} target** | **3^{rd} targe t** | **Cons truct Type** | **Name of first construct in the series** | **SEQ ID NO of Series (DNA)** | **SEQ ID NO of Series (PRT)** |
| Variable | CD19 | None | Bi-specif ic | CD8SP-CD19-FHVH-354-G4S3-FMC63- vL-[hTCRb-S57C-opt]-F-P2A-SP- FMC63-vH-[hTCRa-T48C-opt] | 1849-2013 | 12539-12703 |
| Variable | CD19 | None | Bi-specif ic | CD8SP-CD19-FHVH-354-G4S3- FMC63-vL-[hTCRa-T48C]-F-F2A-SP- FMC63-vH-[hTCRb-S57C] | 2014-2177 | 12704-12868 |
| Variable | CD19 | None | Bi-specif ic | CD8SP-CD19-FHVH-354-G4S3-FMC63- vL-[hTCRbECD-CD3zECDTMCP-opt]- F-P2A-SP-FMC63-vH-[hTCRaECD- CD3zECDTMCP-opt2] | 2178-2343 | 12869-13033 |
| Variable | CD19 | CD22 | Tri-specif ic | CD8SP-CD19-FHVH-354-G4S3-FMC63- vL-[hTCRb-S57C-opt]-F-P2A-SP-CD22- FHVH-24-FMC63-vH-[hTCRa-T48C- opt] | 2344-2508 | 13034-13198 |
| Variable | CD19 | CD22 | Tri-specif ic | CD8SP-CD19-FHVH-354-G4S3- FMC63-vL-[hTCRa-T48C]-F-F2A-SP- CD22-FHVH-24-FMC63-vH-[hTCRb- S57C] | 2509-2673 | 13199-13363 |
| Variable | CD19 | CD22 | Tri-specif ic |  CD8SP-CD19-FHVH-354-G4S3- FMC63-vL-[hTCRbECD- CD3zECDTMCP-opt]-F-P2A-SP-CD22- FHVH-24-FMC63-vH-[hTCRaECD- CD3zECDTMCP-opt2] | 2674-2838 | 13364-13528 |
| Variable | CD19 | None | Bi-specif ic | CD8SP-CD19-FHVH-354-G4S3-FMC63- vL-[IgCL-TCRg-6MD]-F-P2A-SP- FMC63-vH-[IgG1-CH1-TCRd-6MD] | 2839-3003 | 13529-13693 |
| Variable | CD19 | CD22 | Tri-specif ic | CD8SP-CD19-FHVH-354-G4S3-FMC63- vL-[IgCL-TCRg-6MD]-F-P2A-SP-CD22- FHVH-24-FMC63-vH-[IgG1-CH1- TCRd-6MD] | 3004-3168 | 13694-13858 |
| Variable | CD19 | None | Bi-specif ic | CD8SP-CD19-FHVH-354-G4S3-FMC63- vL-[IgCL-TCRb-wt-opt2-6MD]-F-F2A- SP-FMC63-vH-[IgG1-CH1-TCRa-wt- op2-6MD] | 3169-3333 | 13859-14023 |
| Variable | CD19 | CD22 | Tri-specif ic | CD8SP-CD19-FHVH-354-G4S3-FMC63- vL-[IgCL-TCRb-wt-opt2-6MD]-F-F2A- SP-CD22-FHVH-24-FMC63-vH-[IgG1- CH1-TCRa-wt-op2-6MD] | 3334-3498 | 14024-14188 |

**TABLE 27: Bispecific and Trispecific SARs Constructs targeting MIR1 and optionally targeting CD20 and/or CD19**

| **1st (TCR) Target** | **2nd target** | **3rd target BACKBONE** | | **SEQ ID NO (DNA)** | **SEQ ID NO (PRT)** |
|---|---|---|---|---|---|
| MIR1 | | CD20 | 2nd GEN CAR | 3499 | 14189 |
| MIR1 | | CD20 | alpha-beta-TCR | 3500 | 14190 |
| MIR1 | | CD20 | alpha-beta-TCR | 3501 | 14191 |
| MIR1 | | CD20 | alpha-beta-TCR | 3502 | 14192 |
| MIR1 | | CD20 | alpha-beta-TCR | 3503 | 14193 |
| MIR1 | | CD20 | alpha-beta-TCR | 3504 | 14194 |
| MIR1 | | CD20 | alpha-beta-TCR | 3505 | 14195 |
| MIR1 | | CD20 | alpha-beta-TCR | 3506 | 14196 |
| MIR1 | | CD20 | alpha-beta-TCR | 3507 | 14197 |
| MIR1 | | CD20 | alpha-beta-TCR | 3508 | 14198 |
| MIR1 | | CD20 | gamma-delta-TCR | 3509 | 14199 |
| MIR1 | | CD20 | gamma-delta-TCR | 3510 | 14200 |
| MIR1 | | CD20 | zSIR | 3511 | 14201 |
| MIR1 | | CD20 | SIR | 3512 | 14202 |
| MIR1 | | CD20 | SIR | 3513 | 14203 |
| MIR1 | | CD20 | SIR with one CD28 CSD | 3514 | 14204 |
| MIR1 | | CD20 | SIR with two CD28 CSD | 3515 | 14205 |
| MIR1 | | CD20 | SIR with one 4-1BB CSD | 3516 | 14206 |
| MIR1 | | CD20 | zSIR with two 4-1BB CSD | 3517 | 14207 |
| MIR1 | | CD20 | zSIR | 3518 | 14208 |
| MIR1 | | CD20 | Gamma-delta Ab-TCR | 3519 | 14209 |
| MIR1 | | CD20 | alpha-beta Ab-TCR | 3520 | 14210 |
| MIR1 | | CD20 | alpha-beta Ab-TCR | 3521 | 14211 |
| MIR1 | | CD20 | TFPe | 3522 | 14212 |
| MIR1 | | CD20 | TFPd | 3523 | 14213 |
| MIR1 | | CD20 | TFPg | 3524 | 14214 |
| MIR1 | | CD20 | TFPz | 3525 | 14215 |
| MIR1 | | CD20 | Single chain of TCR | 3526 | 14216 |
| MIR1 | | CD20 | Single chain of TCR | 3527 | 14217 |
| MIR1 | | CD20 | Single chain of TCR | 3528 | 14218 |
| MIR1 | | CD20 | Single chain of TCR | 3529 | 14219 |
| MIR1 | CD19 | | 2nd GEN CAR | 3530 | 14220 |
| MIR1 | CD19 | | 2nd GEN CAR | 3531 | 14221 |
| MIR1 | CD19 | | vFLIP-CAR | 3532 | 14222 |
| MIR1 | CD19 | | TCR | 3533 | 14223 |
| MIR1 | CD19 | | TCR | 3534 | 14224 |
| MIR1 | CD19 | | TCR | 3535 | 14225 |
| MIR1 | CD19 | | TCR | 3536 | 14226 |
| MIR1 | CD19 | | TCR | 3537 | 14227 |
| MIR1 | CD19 | | TCR | 3538 | 14228 |
| MIR1 | CD19 | | TCR | 3539 | 14229 |
| MIR1 | CD19 | | TCR | 3540 | 14230 |
| MIR1 | CD19 | | TCR | 3541 | 14231 |
| MIR1 | CD19 | | TCR | 3542 | 14232 |
| MIR1 | CD19 | | TCR | 3543 | 14233 |
| MIR1 | CD19 | | TCR | 3544 | 14234 |
| MIR1 | CD19 | | TCR | 3545 | 14235 |
| MIR1 | CD19 | | TCR | 3546 | 14236 |
| MIR1 | CD19 | | zSIR | 3547 | 14237 |
| MIR1 | CD19 | | SIR | 3548 | 14238 |
| MIR1 | CD19 | | SIR | 3549 | 14239 |
| MIR1 | CD19 | | SIR | 3550 | 14240 |
| MIR1 | CD19 | | SIR | 3551 | 14241 |
| MIR1 | CD19 | | SIR | 3552 | 14242 |
| MIR1 | CD19 | | SIR | 3553 | 14243 |
| MIR1 | CD19 | | zSIR | 3554 | 14244 |
| MIR1 | CD19 | | gamma-delta Ab-TCR | 3555 | 14245 |
| MIR1 | CD19 | | Alpha-beta Ab-TCR | 3556 | 14246 |
| MIR1 | CD19 | | Alpha-beta Ab-TCR | 3557 | 14247 |
| MIR1 | CD19 | | TFPe | 3558 | 14248 |
| MIR1 | CD19 | | TFPd | 3559 | 14249 |
| MIR1 | CD19 | | TFPg | 3560 | 14250 |
| MIR1 | CD19 | | TFPz | 3561 | 14251 |
| MIR1 | CD19 | | Single chain of TCR | 3562 | 14252 |
| MIR1 | CD19 | | Single chain of TCR | 3563 | 14253 |
| MIR1 | CD19 | | Single chain of TCR | 3564 | 14254 |
| MIR1 | CD19 | | Single chain of TCR | 3565 | 14255 |
| MIR1 | CD19 | | Single chain SIR | 3566 | 14256 |
| MIR1 | | | Single chain of TCR | 3567 | 14257 |
| MIR1 | | | Single chain of TCR | 3568 | 14258 |
| MIR1 | CD19 | | Single chain of TCR | 3569 | 14259 |
| MIR1 | CD19 | | Single chain of TCR | 3570 | 14260 |
| MIR1 | | | Single chain of TCR | 3571 | 14261 |
| MIR1 | | | Single chain of TCR | 3572 | 14262 |
| MIR1 | CD19 | | Single chain of TCR | 3573 | 14263 |
| MIR1 | CD19 | | Single chain of TCR | 3574 | 14264 |
| MIR1 | CD19 | CD20 | TCR | 3575 | 14265 |
| MIR1 | CD19 | CD20 | TCR | 3576 | 14266 |
| MIR1 | CD19 | CD20 | TCR | 3577 | 14267 |
| MIR1 | CD19 | CD20 | TCR | 3578 | 14268 |
| MIR1 | CD19 | CD20 | TCR | 3579 | 14269 |
| MIR1 | CD19 | CD20 | TCR | 3580 | 14270 |
| MIR1 | CD19 | CD20 | TCR | 3581 | 14271 |
| MIR1 | CD19 | CD20 | TCR | 3582 | 14272 |
| MIR1 | CD19 | CD20 | TCR | 3583 | 14273 |
| MIR1 | CD19 | CD20 | TCR | 3584 | 14274 |
| MIR1 | CD19 | CD20 | TCR | 3585 | 14275 |
| MIR1 | CD19 | CD20 | zSIR | 3586 | 14276 |
| MIR1 | CD19 | CD20 | SIR | 3587 | 14277 |
| MIR1 | CD19 | CD20 | SIR | 3588 | 14278 |
| MIR1 | CD19 | CD20 | SIR | 3589 | 14279 |
| MIR1 | CD19 | CD20 | SIR | 3590 | 14280 |
| MIR1 | CD19 | CD20 | SIR | 3591 | 14281 |
| MIR1 | CD19 | CD20 | SIR | 3592 | 14282 |
| MIR1 | CD19 | CD20 | zSIR | 3593 | 14283 |
| MIR1 | CD19 | CD20 | Gamma-delta Ab-TCR | 3594 | 14284 |
| MIR1 | CD19 | CD20 | alpha-beta Ab-TCR | 3595 | 14285 |
| MIR1 | CD19 | CD20 | alpha-beta Ab-TCR | 3596 | 14286 |

**TABLE 28: Bispecifics and Trispecific Constructs on the different backbones targeting different antigens. These constructs were generated by changing the CD20-vHH-2HCD25 module of the construct in Table 27 with the modules shown in the first construct of each series in the Table below. The order of the vL/vH fragments of the constructs of each series in Table 28 is the same as the order of vL/vH chains of the constructs of series 1 whose details are provided in Table 27.**

| **1st (TCR) Target** | **2nd target (option al)** | **3rd target (option al)** | **Name of first construct in the series** | **SEQ ID NO of Series (DNA)** | **SEQ ID NO of Series (PRT)** |
|---|---|---|---|---|---|
| MR1 | CD19 | CD20 | IgSP-Apa-CD20-vHH-2HCD25- G4Sx3v2-MC7G5-Va-Gly-Ser-Linker- Vb-Myc-CD8TM-BBz | 3499-3596 | 14189-14286 |
| MR1 | CD19 | CD20 | IgSP-NKG2D-G4Sx3-NKG2D- G4Sx3v2-MC7G5-Va-Gly-Ser-Linker- Vb-Myc-CD8TM-BBz | 3597-3694 | 14287-14384 |
| MR1 | CD19 | CD20 | IgSP-Apa-RZIP-G4Sx3v2-MC7G5-Va- Gly-Ser-Linker-Vb-Myc-CD8TM-BBz | 3695-3792 | 14385-14482 |
| MR1 | CD19 | CD20 | IgSP-Apa-CD16-V158-v2-G4Sx3v2- MC7G5-Va-Gly-Ser-Linker-Vb-Myc- CD8TM-BBz | 3793-3890 | 14483-14580 |
| CMV-pp65/HLA -A2 | CD19 | CD20 | IgSP-Apa-CD20-vHH-2HCD25- G4Sx3v2-CMV-pp65-TCR1-Va-Gly-Ser- Linker-Vb-Myc-CD8TM-BBz | 3891-3988 | 14581-14678 |
| NYESO-1/HLA-A2 | CD19 | CD20 | IgSP-Apa-CD20-vHH-2HCD25- G4Sx3v2-NYESO-IG4-Va-Gly-Ser- Linker-Vb-Myc-CD8TM-BBz | 3989-4086 | 14679-14776 |

**TABLE 29: Bispecific and Trispecific SARs Constructs targeting CD20 and a second antigen targeted by SIR based on hu-mROO5-1 vL/vH fragments (i.e. Fv)**

| **1st (SIR) Target** | **2nd target** | **3rd target** | **Name of fragment** | **SEQ ID NO (DNA)** | **SEQ ID NO (PRT)** |
|---|---|---|---|---|---|
| CD19 | CD20 | | IgSP-Apa-CD20-vHH-2HCD25-G4Sx3v2-hu- mROO5-1-vH-Gly-Ser-Linker-vL-Myc-CD8TM- BBz | 4087 | 14777 |
| CD19 | CD20 | | CD8SP-hu-mROO5-1-vL-[hTCRa-CSDVP]-F- F2A-IgSP-Apa-CD20-vHH-2HCD25-G4Sx3v2- hu-mROO5-1-vH-[hTCRb-KACIAH]-F-P2A- PAC | 4088 | 14778 |
| CD19 | CD20 | | CD8SP-hu-mROO5-1-vL-[hTCRb-KACIAH]-F- P2A-IgSP-Apa-CD20-vHH-2HCD25-G4Sx3v2- hu-mROO5-1-vH-[hTCRa-CSDVP]-F-F2A-PAC | 4089 | 14779 |
| CD19 | CD20 | | CD8SP-hu-mROO5-1-vL-[hTCRb-S57C]-F- P2A-IgSP-Apa-CD20-vHH-2HCD25-G4Sx3v2- hu-mROO5-1-vH-[hTCRa-T48C]-F-F2A-PAC | 4090 | 14780 |
| CD19 | CD20 | | CD8SP-hu-mROO5-1-vL-[hTCRb-S57C]-F- P2A-IgSP-Apa-CD20-vHH-2HCD25-G4Sx3v2- hu-mROO5-1-vH-[hTCRa-T48C] | 4091 | 14781 |
| CD19 | CD20 | | CD8SP-hu-mROO5-1-vL-[hTCRb-S57C]-F- P2A-IgSP-Apa-CD20-vHH-2HCD25-G4Sx3v2- hu-mROO5-1-vH-[hTCRa-T48C]-F-F2A-K13- opt | 4092 | 14782 |
| CD19 | CD20 | | CD8SP-hu-mROO5-1-vL-[hTCRa-T48C]-F- P2A-IgSP-Apa-CD20-vHH-2HCD25-G4Sx3v2- hu-mROO5-1-vH-[hTCRb-S57C] | 4093 | 14783 |
| CD19 | CD20 | | CD8SP-hu-mROO5-1-vL-[hTCRa-T48C]-F- P2A-IgSP-Apa-CD20-vHH-2HCD25-G4Sx3v2- hu-mROO5-1-vH-[hTCRb-S57C]-F-P2A-K13- opt | 4094 | 14784 |
| CD19 | CD20 | | CD8SP-hu-mROO5-1-vL-[hTCRb-opt2]-F-P2A- IgSP-Apa-CD20-vHH-2HCD25-G4Sx3v2-hu- mROO5-1-vH-[hTCRa-opt2]-F-F2A-PAC | 4095 | 14785 |
| CD19 | CD20 | | CD8SP-hu-mROO5-1-vL-[hTCRb-opt2]-F-P2A- IgSP-Apa-CD20-vHH-2HCD25-G4Sx3v2-hu- mROO5-1-vH-Myc-[pretc.Ra-Del48]-F-F2A- PAC | 4096 | 14786 |
| CD19 | CD20 | | CD8SP-hu-mROO5-1-vL-V5-[hTCRg1-opt]-F- P2A-IgSP-Apa-CD20-vHH-2HCD25-G4Sx3v2- hu-mROO5-1-vH-Myc-[hTCRd-opt]-F-F2A- PAC | 4097 | 14787 |
| CD19 | CD20 | | CD8SP-hu-mROO5-1-vL-[hTCRd-opt]-F-P2A- IgSP-Apa-CD20-vHH-2HCD25-G4Sx3v2-hu- mROO5-1-vH-[hTCRg1-opt] | 4098 | 14788 |
| CD19 | CD20 | | CD8-hu-mROO5-1-vL-IgCL-CD3zECDTMCP- opt-F-P2A-Spe-IgSP-Apa-CD20-vHH-2HCD25- G4Sx3v2-hu-mROO5-1-vH-IgG1-CH1- CD3zECDTMCP-opt2-F-F2A-PAC | 4099 | 14789 |
| CD19 | CD20 | | CD8SP-hu-mROO5-1-vL-[hTCRbECD- CD3zECDTMCP-opt]-F-P2A-IgSP-Apa-CD20- vHH-2HCD25-G4Sx3v2-hu-mROO5-1-vH- [hTCRaECD-CD3zECDTMCP-opt2] | 4100 | 14790 |
| CD19 | CD20 | | CD8SP-hu-mROO5-1-vL-[hTCRb-KAC-ECD- CD3zECDTMCP-opt]-F-P2A-IgSP-Apa-CD20- vHH-2HCD25-G4Sx3v2-hu-mROO5-1-vH- [hTCRa-CSDVP-ECD-CD3zECDTMCP-opt2] | 4101 | 14791 |
| CD19 | CD20 | | CD8SP-hu-mROO5-1-vL-V5-[hTCRbECD- CD3zECDTMCP-opt]-F-P2A-IgSP-Apa-CD20- vHH-2HCD25-G4Sx3v2-hu-mROO5-1-vH-Myc- [hTCRaECD-CD3zECDTM-28z-opt2] | 4102 | 14792 |
| CD19 | CD20 | | CD8SP-hu-mROO5-1-vL-V5-[hTCRbECD- CD3zECDTM-28z-opt]-F-P2A-IgSP-Apa-CD20- vHH-2HCD25-G4Sx3v2-hu-mROO5-1-vH-Myc- [hTCRaECD-CD3zECDTM-28z-opt2] | 4103 | 14793 |
| CD19 | CD20 | | CD8SP-hu-mROO5-1-vL-V5-[hTCRbECD- CD3zECDTMCP-opt]-F-P2A-IgSP-Apa-CD20- vHH-2HCD25-G4Sx3v2-hu-mROO5-1-vH- Myc4-[hTCRaECD-CD3zECDTM-BBz-opt2] | 4104 | 14794 |
| CD19 | CD20 | | CD8SP-hu-mROO5-1-vL-V5-[hTCRbECD- CD3zECDTM-BBz-opt]-F-P2A-IgSP-Apa- CD20-vHH-2HCD25-G4Sx3v2-hu-mROO5-1- vH-Myc4-[hTCRaECD-CD3zECDTM-BBz- opt2] | 4105 | 14795 |
| CD19 | CD20 | | CD8SP-hu-mROO5-1-vL-CD3zECDTMCP-opt- F-P2A-Spe-IgSP-Apa-CD20-vHH-2HCD25- G4Sx3v2-hu-mROO5-1-vH-Mlu- CD3zECDTMCP-opt2-F-F2A-PAC | 4106 | 14796 |
| CD19 | CD20 | | CD8SP-hu-mROO5-1-vL-[IgCL-TCRg-6MD]-F- P2A-IgSP-Apa-CD20-vHH-2HCD25-G4Sx3v2- hu-mROO5-1-vH-[IgG1-CH1-TCRd-6MD] | 4107 | 14797 |
| CD19 | CD20 | | CD8SP-hu-mROO5-1-vL-[IgCL-TCRb-IAH- 6MD]-F-P2A-IgSP-Apa-CD20-vHH-2HCD25- G4Sx3v2-hu-mROO5-1-vH-[IgG1-CH1-TCRa- SDVP-6MD] | 4108 | 14798 |
| CD19 | CD20 | | CD8SP-hu-mROO5-1-vL-[IgCL-TCRb-wt-opt2- 6MD]-F-P2A-IgSP-Apa-CD20-vHH-2HCD25- G4Sx3v2-hu-mROO5-1-vH-[IgG1-CH1-TCRa- wt-op2-6MD] | 4109 | 14799 |
| CD19 | CD20 | | IgSP-Apa-CD20-vHH-2HCD25-G4Sx3v2-hu- mROO5-1-(vH-vL)-G3S-CD3e-ECDTMCP-opt2 | 4110 | 14800 |
| CD19 | CD20 | | IgSP-Apa-CD20-vHH-2HCD25-G4Sx3v2-hu- mROO5-1-(vH-vL)-G3S-CD3d-ECDTMCP-opt2 | 4111 | 14801 |
| CD19 | CD20 | | IgSP-Apa-CD20-vHH-2HCD25-G4Sx3v2-hu- mROO5-1-(vH-vL)-G3S-CD3g-ECDTMCP-opt2 | 4112 | 14802 |
| CD19 | CD20 | | IgSP-Apa-CD20-vHH-2HCD25-G4Sx3v2-hu- mROO5-1-(vH-vL)-G3S-CD3z-ECDTMCP-opt2 | 4113 | 14803 |
| CD19 | CD20 | | IgSP-Apa-CD20-vHH-2HCD25-G4Sx3v2-hu- mROO5-1-vH-[hTCRa-opt2]-F-F2A-BlastR | 4114 | 14804 |
| CD19 | CD20 | | IgSP-Apa-CD20-vHH-2HCD25-G4Sx3v2-hu- mROO5-1-vH-[hTCRa-opt2] | 4115 | 14805 |
| CD19 | CD20 | | IgSP-Apa-CD20-vHH-2HCD25-G4Sx3v2-hu- mROO5-1-vH-Myc-[hTCRa-T48C-opt]-F-F2A- BlastR | 4116 | 14806 |
| CD19 | CD20 | | IgSP-Apa-CD20-vHH-2HCD25-G4Sx3v2-hu- mROO5-1-vH-Myc-[hTCRa-T48C-opt] | 4117 | 14807 |
| CD19 | | CD22 | CD8SP-CD22-FHVH-24-G4Sx3-R1-hu- mROO5-1-vL-Gly-Ser-Linker-hu-mROO5-1-vH- Myc-28z | 4118 | 14808 |
| CD19 | | CD22 | CD8SP-CD22-FHVH-24-G4Sx3-R1-hu- mROO5-1-vL-Gly-Ser-Linker-hu-mROO5-1-vH- Myc-CD8TM-BBz | 4119 | 14809 |
| CD19 | | CD22 | CD8SP-CD22-FHVH-24-G4Sx3-R1-hu- mROO5-1-vL-Gly-Ser-Linker-hu-mROO5-1-vH- Myc-CD8TM-z-P2A-K13-FLAG-T2A-PAC | 4120 | 14810 |
| CD19 | | CD22 | CD8SP-CD22-FHVH-24-G4Sx3-R1-hu- mROO5-1-vL-[hTCRa-CSDVP]-F-F2A-SP-hu- mROO5-1-vH-[hTCRb-KACIAH]-F-P2A-PAC | 4121 | 14811 |
| CD19 | | CD22 | CD8SP-CD22-FHVH-24-G4Sx3-R1-hu- mROO5-1-vL-[hTCRb-KACIAH]-F-P2A-SP-hu- mROO5-1-vH-[hTCRa-CSDVP]-F-F2A-PAC | 4122 | 14812 |
| CD19 | | CD22 | CD8SP-CD22-FHVH-24-G4Sx3-R1-hu- mROO5-1-vL-[hTCRb-S57C]-F-P2A-SP-hu- mROO5-1-vH-[hTCRa-T48C] | 4123 | 14813 |
| CD19 | | CD22 | CD8SP-CD22-FHVH-24-G4Sx3-R1-hu- mROO5-1-vL-[hTCRb-S57C]-F-P2A-SP-hu- mROO5-1-vH-[hTCRa-T48C]-F-F2A-K13-opt | 4124 | 14814 |
| CD19 | | CD22 | CD8SP-CD22-FHVH-24-G4Sx3-R1-hu- mROO5-1-vL-[hTCRa-T48C]-F-P2A-SP-hu- mROO5-1-vH-[hTCRb-S57C] | 4125 | 14815 |
| CD19 | | CD22 | CD8SP-CD22-FHVH-24-G4Sx3-R1-hu- mROO5-1-vL-[hTCRa-T48C]-F-P2A-SP-hu- mROO5-1-vH-[hTCRb-S57C]-F-P2A-K13-opt | 4126 | 14816 |
| CD19 | | CD22 | CD8SP-V5-[hTCRb-KACIAH]-F-P2A-CD8SP- CD22-FHVH-24-G4Sx3-R1-hu-mROO5-1-vL- Gly-Ser-Linker-hu-mROO5-1-vH-Myc-[hTCRa- CSDVP]-F-F2A-PAC | 4127 | 14817 |
| CD19 | | CD22 | CD8SP-MYC-[hTCRa-T48C-opt1]-F-F2A-SP- CD22-FHVH-24-G4Sx3-R1-hu-mROO5-1-vL- Gly-Ser-Linker-hu-mROO5-1-vH-V5-[hTCRb- S57C-opt1]-F-P2A-PAC | 4128 | 14818 |
| CD19 | | CD22 | CD8SP-CD22-FHVH-24-G4Sx3-R1-hu- mROO5-1-vL-[hTCRb-opt2]-F-P2A-SP-hu- mROO5-1-vH-[hTCRa-opt2]-F-F2A-PAC | 4129 | 14819 |
| CD19 | | CD22 | CD8SP-CD22-FHVH-24-G4Sx3-R1-hu- mROO5-1-vL-[hTCRb-opt2]-F-P2A-SP-hu- mROO5-1-vH-Myc-[pretc.Ra-Del48]-F-F2A- PAC | 4130 | 14820 |
| CD19 | | CD22 | CD8SP-[hTCRb-opt2]-F-P2A-CD8SP-CD22- FHVH-24-G4Sx3-R1-hu-mROO5-1-vL-Gly-Ser- Linker-hu-mROO5-1-vH-Myc4-[pr*etc.*Ra- Del48]-F-F2A-PAC | 4131 | 14821 |
| CD19 | | CD22 | CD8SP-CD22-FHVH-24-G4Sx3-R1-hu- mROO5-1-vL-V5-[hTCRg1-opt]-F-P2A-SP-hu- mROO5-1-vH-Myc-[hTCRd-opt]-F-F2A-PAC | 4132 | 14822 |
| CD19 | | CD22 | CD8SP-CD22-FHVH-24-G4Sx3-R1-hu- mROO5-1-vL-[hTCRd-opt]-F-P2A-SP-hu- mROO5-1-vH-[hTCRg1-opt] | 4133 | 14823 |
| CD19 | | CD22 | CD8SP-V5-[hTCRg1-opt]-F-P2A-CD8SP-CD22- FHVH-24-G4Sx3-R1-hu-mROO5-1-vL-Gly-Ser- Linker-hu-mROO5-1-vH-Myc-[hTCRd-opt]-F- F2A-PAC | 4134 | 14824 |
| CD19 | | CD22 | CD8-CD22-FHVH-24-G4Sx3-R1-hu-mROO5-1- vL-IgCL-CD3zECDTMCP-opt-F-P2A-Spe-SP- hu-mROO5-1-vH-IgG1-CH1-CD3zECDTMCP- opt2-F-F2A-PAC | 4135 | 14825 |
| CD19 | | CD22 | CD8SP-CD22-FHVH-24-G4Sx3-R1-hu- mROO5-1-vL-[hTCRbECD-CD3zECDTMCP- opt]-F-P2A-SP-hu-mROO5-1-vH-[hTCRaECD- CD3zECDTMCP-opt2] | 4136 | 14826 |
| CD19 | | CD22 | CD8SP-CD22-FHVH-24-G4Sx3-R1-hu- mROO5-1-vL-[hTCRb-KAC-ECD- CD3zECDTMCP-opt]-F-P2A-SP-hu-mROO5-1- vH-[hTCRa-CSDVP-ECD-CD3zECDTMCP- opt2] | 4137 | 14827 |
| CD19 | | CD22 | CD8SP-CD22-FHVH-24-G4Sx3-R1-hu- mROO5-1-vL-V5-[hTCRbECD- CD3zECDTMCP-opt]-F-P2A-SP-hu-mROO5-1- vH-Myc-[hTCRaECD-CD3zECDTM-28z-opt2] | 4138 | 14828 |
| CD19 | | CD22 | CD8SP-CD22-FHVH-24-G4Sx3-R1-hu- mROO5-1-vL-V5-[hTCRbECD-CD3zECDTM- | 4139 | 14829 |
| | | | 28z-opt]-F-P2A-SP-hu-mROO5-1-vH-Myc- [hTCRaECD-CD3zECDTM-28z-opt2] | | |
| CD19 | | CD22 | CD8SP-CD22-FHVH-24-G4Sx3-R1-hu- mROO5-1-vL-V5-[hTCRbECD- CD3zECDTMCP-opt]-F-P2A-SP-hu-mROO5-1- vH-Myc4-[hTCRaECD-CD3zECDTM-BBz- opt2] | 4140 | 14830 |
| CD19 | | CD22 | CD8SP-CD22-FHVH-24-G4Sx3-R1-hu- mROO5-1-vL-V5-[hTCRbECD-CD3zECDTM- BBz-opt]-F-P2A-SP-hu-mROO5-1-vH-Myc4- [hTCRaECD-CD3zECDTM-BBz-opt2] | 4141 | 14831 |
| CD19 | | CD22 | CD8-CD22-FHVH-24-G4Sx3-R1-hu-mROO5-1- vL-CD3zECDTMCP-opt-F-P2A-Spe-SP-hu- mROO5-1-vH-Mlu-CD3zECDTMCP-opt2-F- F2A-PAC | 4142 | 14832 |
| CD19 | | CD22 | CD8SP-CD22-FHVH-24-G4Sx3-R1-hu- mROO5-1-vL-[IgCL-TCRg-6MD]-F-P2A-SP- hu-mROO5-1-vH-[IgG1-CH1-TCRd-6MD] | 4143 | 14833 |
| CD19 | | CD22 | CD8SP-CD22-FHVH-24-G4Sx3-R1-hu- mROO5-1-vL-[IgCL-TCRb-IAH-6MD]-F-P2A- SP-hu-mROO5-1-vH-[IgG1-CH1-TCRa-SDVP- 6MD] | 4144 | 14834 |
| CD19 | | CD22 | CD8SP-CD22-FHVH-24-G4Sx3-R1-hu- mROO5-1-vL-[IgCL-TCRb-wt-opt2-6MD]-F- P2A-SP-hu-mROO5-1-vH-[IgG1-CH1-TCRa-wt- op2-6MD] | 4145 | 14835 |
| CD19 | | CD22 | CD8SP-CD22-FHVH-24-G4Sx3-R1-hu- mROO5-1-(vL-vH)-CD3e-ECDTMCP-opt2 | 4146 | 14836 |
| CD19 | | CD22 | CD8SP-CD22-FHVH-24-G4Sx3-R1-hu- mROO5-1-(vL-vH)-CD3d-ECDTMCP-opt2 | 4147 | 14837 |
| CD19 | | CD22 | CD8SP-CD22-FHVH-24-G4Sx3-R1-hu- mROO5-1-(vL-vH)-CD3g-ECDTMCP-opt2 | 4148 | 14838 |
| CD19 | | CD22 | CD8SP-CD22-FHVH-24-G4Sx3-R1-hu- mROO5-1-(vL-vH)-CD3z-ECDTMCP-opt2 | 4149 | 14839 |
| CD19 | | CD22 | CD8SP-CD22-FHVH-24-G4Sx3-R1-hu- mROO5-1-vL-Gly-Ser-Linker-hu-mROO5-1-vH- [hTCRa-opt2]-F-F2A-PAC | 4150 | 14840 |
| CD19 | | CD22 | CD8SP-CD22-FHVH-24-G4Sx3-R1-hu- mROO5-1-vL-Gly-Ser-Linker-hu-mROO5-1-vH- [hTCRb-opt2]-F-F2A-PAC | 4151 | 14841 |
| CD19 | | CD22 | CD8SP-CD22-FHVH-24-G4Sx3-R1-hu- mROO5-1-vL-Gly-Ser-Linker-hu-mROO5-1-vH- Myc4-[pretc.Ra-Del48]-F-F2A-PAC | 4152 | 14842 |
| CD19 | | CD22 | CD8SP-CD22-FHVH-24-G4Sx3-R1-hu- mROO5-1-vL-[hTCRb-opt2]-F-P2A-PAC | 4153 | 14843 |
| CD19 | | CD22 | CD8SP-CD22-FHVH-24-G4Sx3-R1-hu- mROO5-1-vL-[hTCRb-opt2] | 4154 | 14844 |
| CD19 | | CD22 | IgHSP-hu-mROO5-1-vH-[hTCRa-opt2]-F-F2A- BlastR | 4155 | 14845 |
| CD19 | | CD22 | IgHSP-hu-mROO5-1-vH-[hTCRa-opt2] | 4156 | 14846 |
| CD19 | | CD22 | CD8SP-CD22-FHVH-24-G4Sx3-R1-hu- mROO5-1-vL-V5-[hTCRb-S57C-opt]-F-P2A- PAC | 4157 | 14847 |
| CD19 | | CD22 | CD8SP-CD22-FHVH-24-G4Sx3-R1-hu- mROO5-1-vL-V5-[hTCRb-S57C-opt] | 4158 | 14848 |
| CD19 | | | IgHSP-hu-mROO5-1-vH-Myc-[hTCRa-T48C- opt]-F-F2A-BlastR | 4159 | 14849 |
| CD19 | | | IgHSP-hu-mROO5-1-vH-Myc-[hTCRa-T48C- opt] | 4160 | 14850 |
| CD19 | | CD22 | CD8SP-CD22-FHVH-24-G4Sx3-R1-hu- mROO5-1-vL-Gly-Ser-Linker-hu-mROO5-1-vH- [hTCRa-SDVP]-F-F2A-PAC | 4161 | 14851 |
| CD19 | | CD22 | CD8SP-CD22-FHVH-24-G4Sx3-R1-hu- mROO5-1-vL-Gly-Ser-Linker-hu-mROO5-1-vH- [hTCRb-KAIAH]-F-P2A-PAC | 4162 | 14852 |
| CD19 | CD20 | CD22 | CD8SP-CD22-FHVH-24-G4Sx3-R1-hu- mROO5-1-vL-[hTCRa-CSDVP]-F-F2A-IgSP- Apa-CD20-vHH-2HCD25-G4Sx3v2-hu- mROO5-1-vH-[hTCRb-KACIAH]-F-P2A-PAC | 4163 | 14853 |
| CD19 | CD20 | CD22 | CD8SP-CD22-FHVH-24-G4Sx3-R1-hu- mROO5-1-vL-[hTCRb-KACIAH]-F-P2A-IgSP- Apa-CD20-vHH-2HCD25-G4Sx3v2-hu- mROO5-1-vH-[hTCRa-CSDVP]-F-F2A-PAC | 4164 | 14854 |
| CD19 | CD20 | CD22 | CD8SP-CD22-FHVH-24-G4Sx3-R1-hu- mROO5-1-vL-[hTCRb-S57C]-F-P2A-IgSP-Apa- CD20-vHH-2HCD25-G4Sx3v2-hu-mROO5-1- vH-[hTCRa-T48C]-F-F2A-PAC | 4165 | 14855 |
| CD19 | CD20 | CD22 | CD8SP-CD22-FHVH-24-G4Sx3-R1-hu- mROO5-1-vL-[hTCRb-S57C]-F-P2A-IgSP-Apa- CD20-vHH-2HCD25-G4Sx3v2-hu-mROO5-1- vH-[hTCRa-T48C] | 4166 | 14856 |
| CD19 | CD20 | CD22 | CD8SP-CD22-FHVH-24-G4Sx3-R1-hu- mROO5-1-vL-[hTCRb-S57C]-F-P2A-IgSP-Apa- CD20-vHH-2HCD25-G4Sx3v2-hu-mROO5-1- vH-[hTCRa-T48C]-F-F2A-K13-opt | 4167 | 14857 |
| CD19 | CD20 | CD22 | CD8SP-CD22-FHVH-24-G4Sx3-R1-hu- mROO5-1-vL-[hTCRa-T48C]-F-P2A-IgSP-Apa- CD20-vHH-2HCD25-G4Sx3v2-hu-mROO5-1- vH-[hTCRb-S57C] | 4168 | 14858 |
| CD19 | CD20 | CD22 | CD8SP-CD22-FHVH-24-G4Sx3-R1-hu- mROO5-1-vL-[hTCRa-T48C]-F-P2A-IgSP-Apa- CD20-vHH-2HCD25-G4Sx3v2-hu-mROO5-1- vH-[hTCRb-S57C]-F-P2A-K13-opt | 4169 | 14859 |
| CD19 | CD20 | CD22 | CD8SP-CD22-FHVH-24-G4Sx3-R1-hu- mROO5-1-vL-[hTCRb-opt2]-F-P2A-IgSP-Apa- | 4170 | 14860 |
| | | | CD20-vHH-2HCD25-G4Sx3v2-hu-mROO5-1- vH-[hTCRa-opt2]-F-F2A-PAC | | |
| CD19 | CD20 | CD22 | CD8SP-CD22-FHVH-24-G4Sx3-R1-hu- mROO5-1-vL-[hTCRb-opt2]-F-P2A-IgSP-Apa- CD20-vHH-2HCD25-G4Sx3v2-hu-mROO5-1- vH-Myc-[pretc.Ra-Del48]-F-F2A-PAC | 4171 | 14861 |
| CD19 | CD20 | CD22 | CD8SP-CD22-FHVH-24-G4Sx3-R1-hu- mROO5-1-vL-V5-[hTCRg1-opt]-F-P2A-IgSP- Apa-CD20-vHH-2HCD25-G4Sx3v2-hu- mROO5-1-vH-Myc-[hTCRd-opt]-F-F2A-PAC | 4172 | 14862 |
| CD19 | CD20 | CD22 | CD8SP-CD22-FHVH-24-G4Sx3-R1-hu- mROO5-1-vL-[hTCRd-opt]-F-P2A-IgSP-Apa- CD20-vHH-2HCD25-G4Sx3v2-hu-mROO5-1- vH-[hTCRg1-opt] | 4173 | 14863 |
| CD19 | CD20 | CD22 | CD8SP-CD22-FHVH-24-G4Sx3-R1-hu- mROO5-1-vL-IgCL-CD3zECDTMCP-opt-F- P2A-Spe-IgSP-Apa-CD20-vHH-2HCD25- G4Sx3v2-hu-mROO5-1-vH-IgG1-CH1- CD3zECDTMCP-opt2-F-F2A-PAC | 4174 | 14864 |
| CD19 | CD20 | CD22 | CD8SP-CD22-FHVH-24-G4Sx3-R1-hu- mROO5-1-vL-[hTCRbECD-CD3zECDTMCP- opt]-F-P2A-IgSP-Apa-CD20-vHH-2HCD25- G4Sx3v2-hu-mROO5-1-vH-[hTCRaECD- CD3zECDTMCP-opt2] | 4175 | 14865 |
| CD19 | CD20 | CD22 | CD8SP-CD22-FHVH-24-G4Sx3-R1-hu- mROO5-1-vL-[hTCRb-KAC-ECD- CD3zECDTMCP-opt]-F-P2A-IgSP-Apa-CD20- vHH-2HCD25-G4Sx3v2-hu-mROO5-1-vH- [hTCRa-CSDVP-ECD-CD3zECDTMCP-opt2] | 4176 | 14866 |
| CD19 | CD20 | CD22 | CD8SP-CD22-FHVH-24-G4Sx3-R1-hu- mROO5-1-vL-V5-[hTCRbECD- CD3zECDTMCP-opt]-F-P2A-IgSP-Apa-CD20- vHH-2HCD25-G4Sx3v2-hu-mROO5-1-vH-Myc- [hTCRaECD-CD3zECDTM-28z-opt2] | 4177 | 14867 |
| CD19 | CD20 | CD22 | CD8SP-CD22-FHVH-24-G4Sx3-R1-hu- mROO5-1-vL-V5-[hTCRbECD-CD3zECDTM- 28z-opt]-F-P2A-IgSP-Apa-CD20-vHH-2HCD25- G4Sx3v2-hu-mROO5-1-vH-Myc-[hTCRaECD- CD3zECDTM-28z-opt2] | 4178 | 14868 |
| CD19 | CD20 | CD22 | CD8SP-CD22-FHVH-24-G4Sx3-R1-hu- mROO5-1-vL-V5-[hTCRbECD- CD3zECDTMCP-opt]-F-P2A-IgSP-Apa-CD20- vHH-2HCD25-G4Sx3v2-hu-mROO5-1-vH- Myc4-[hTCRaECD-CD3zECDTM-BBz-opt2] | 4179 | 14869 |
| CD19 | CD20 | CD22 | CD8SP-CD22-FHVH-24-G4Sx3-R1-hu- mROO5-1-vL-V5-[hTCRbECD-CD3zECDTM- BBz-opt]-F-P2A-IgSP-Apa-CD20-vHH- 2HCD25-G4Sx3v2-hu-mROO5-1-vH-Myc4- [hTCRaECD-CD3zECDTM-BBz-opt2] | 4180 | 14870 |
| CD19 | CD20 | CD22 | CD8SP-CD22-FHVH-24-G4Sx3-R1-hu- mROO5-1-vL-CD3zECDTMCP-opt-F-P2A-Spe- IgSP-Apa-CD20-vHH-2HCD25-G4Sx3v2-hu- mROO5-1-vH-Mlu-CD3zECDTMCP-opt2-F- F2A-PAC | 4181 | 14871 |
| CD19 | CD20 | CD22 | CD8SP-CD22-FHVH-24-G4Sx3-R1-hu- mROO5-1-vL-[IgCL-TCRg-6MD]-F-P2A-IgSP- Apa-CD20-vHH-2HCD25-G4Sx3v2-hu- mROO5-1-vH-[IgG1-CH1-TCRd-6MD] | 4182 | 14872 |
| CD19 | CD20 | CD22 | CD8SP-CD22-FHVH-24-G4Sx3-R1-hu- mROO5-1-vL-[IgCL-TCRb-IAH-6MD]-F-P2A- IgSP-Apa-CD20-vHH-2HCD25-G4Sx3v2-hu- mROO5-1-vH-[IgG1-CH1-TCRa-SDVP-6MD] | 4183 | 14873 |
| CD19 | CD20 | CD22 | CD8SP-CD22-FHVH-24-G4Sx3-R1-hu- mROO5-1-vL-[IgCL-TCRb-wt-opt2-6MD]-F- P2A-IgSP-Apa-CD20-vHH-2HCD25-G4Sx3v2- hu-mROO5-1-vH-[IgG1-CH1-TCRa-wt-op2- 6MD] | 4184 | 14874 |
| | CD20 | CD22 | CD8SP-CD22-FHVH-24-[IgCL-TCRb-wt-opt2- 6MD]-F-P2A-IgSP-Apa-CD20-2HCD25-VHH- [IgG1-CH1-TCRa-wt-opt2-6MD]-F-F2A-PAC | 4185 | 14875 |
| | CD20 | CD22 | CD8SP-CD22-FHVH-24-[IgCL-TCRb-wt-opt2- 6MD]-F-P2A-IgSP-Apa-CD20-2HCD25-VHH- [IgG1-CH1-TCRa-wt-opt2-6MD] | 4186 | 14876 |
| | CD20 | CD22 | CD8SP-CD22-FHVH-24-GS-[TCRb-wt-opt2- 6MD]-F-P2A-IgSP-Apa-CD20-2HCD25-VHH- GS-[TCRa-wt-opt2-6MD]-F-F2A-PAC | 4187 | 14877 |
| | CD20 | CD22 | CD8SP-CD22-FHVH-24-GS-[TCRb-wt-opt2- 6MD]-F-P2A-IgSP-Apa-CD20-2HCD25-VHH- GS-[TCRa-wt-opt2-6MD] | 4188 | 14878 |
| | CD20 | CD22 | CD8SP-CD22-FHVH-24-IgG1-Hinge-[TCRb-wt- opt2-6MD]-F-P2A-IgSP-Apa-CD20-2HCD25- VHH-GS-[TCRa-wt-opt2-6MD]-F-F2A-PAC | 4189 | 14879 |
| | CD20 | CD22 | CD8SP-CD22-FHVH-24-GS-[TCRb-wt-opt2- 6MD]-F-P2A-IgSP-Apa-CD20-2HCD25-VHH- IgG1-Hinge-v2-[TCRa-wt-opt2-6MD]-F-F2A- PAC | 4190 | 14880 |
| | CD20 | CD22 | CD8SP-CD22-FHVH-24-IgG1-Hinge-[TCRb-wt- opt2-6MD]-F-P2A-IgSP-Apa-CD20-2HCD25- VHH-IgG1-Hinge-v2-[TCRa-wt-opt2-6MD]-F- F2A-PAC | 4191 | 14881 |
| | CD20 | CD22 | CD8SP-CD22-FHVH-24-IgG1-Hinge-[TCRb-wt- opt2-6MD]-F-P2A-IgSP-Apa-CD20-2HCD25- VHH-IgG1-Hinge-v2-[TCRa-wt-opt2-6MD] | 4192 | 14882 |
| | CD20 | CD22 | CD8SP-CD22-FHVH-24-GS-[TCRb-wt-opt2- 6MD]-F-P2A-IgSP-Apa-CD20-2HCD25-VHH- IgG1-Hinge-v2-[TCRa-wt-opt2-6MD] | 4193 | 14883 |
| | CD20 | CD22 | CD8SP-CD22-FHVH-24-[IgCL-TCRg-6MD]-F- P2A-IgSP-Apa-CD20-2HCD25-VHH-[IgG1- CH1-TCRd-6MD]-F-F2A-PAC | 4194 | 14884 |
| | CD20 | CD22 | CD8SP-CD22-FHVH-24-[IgCL-TCRg-6MD]-F- P2A-IgSP-Apa-CD20-2HCD25-VHH-[IgG1- CH1-TCRd-6MD] | 4195 | 14885 |
| | CD20 | CD22 | CD8SP-CD22-FHVH-24-GS-[TCRg-6MD]-F- P2A-IgSP-Apa-CD20-2HCD25-VHH-GS- [TCRd-6MD]-F-F2A-PAC | 4196 | 14886 |
| | CD20 | CD22 | CD8SP-CD22-FHVH-24-GS-[TCRg-6MD]-F- P2A-IgSP-Apa-CD20-2HCD25-VHH-GS- [TCRd-6MD] | 4197 | 14887 |
| | CD20 | CD22 | CD8SP-CD22-FHVH-24-IgG1-Hinge-[TCRg- 6MD]-F-P2A-IgSP-Apa-CD20-2HCD25-VHH- GS-[TCRd-6MD]-F-F2A-PAC | 4198 | 14888 |
| | CD20 | CD22 | CD8SP-CD22-FHVH-24-GS-[TCRg-6MD]-F- P2A-IgSP-Apa-CD20-2HCD25-VHH-IgG1- Hinge-v2-[TCRd-6MD]-F-F2A-PAC | 4199 | 14889 |
| | CD20 | CD22 | CD8SP-CD22-FHVH-24-IgG1-Hinge-[TCRg- 6MD]-F-P2A-IgSP-Apa-CD20-2HCD25-VHH- IgG1-Hinge-v2-[TCRd-6MD]-F-F2A-PAC | 4200 | 14890 |
| | CD20 | CD22 | CD8SP-CD22-FHVH-24-IgG1-Hinge-[TCRg- 6MD]-F-P2A-IgSP-Apa-CD20-2HCD25-VHH- IgG1-Hinge-v2-[TCRd-6MD] | 4201 | 14891 |
| | CD20 | CD22 | CD8SP-CD22-FHVH-24-GS-[TCRg-6MD]-F- P2A-IgSP-Apa-CD20-2HCD25-VHH-IgG1- Hinge-v2-[TCRd-6MD] | 4202 | 14892 |
| | CD20 | CD22 | CD8SP-CD22-FHVH-24-GS-[TCRg-7MD]-F- P2A-IgSP-Apa-CD20-2HCD25-VHH-GS- [TCRd-6MD]-F-F2A-PAC | 4203 | 14893 |
| | CD20 | CD22 | CD8SP-CD22-FHVH-24-GS-[TCRg-6MD]-F- P2A-IgSP-Apa-CD20-2HCD25-VHH-GS- [TCRd-7MD]-F-F2A-PAC | 4204 | 14894 |
| | CD20 | CD22 | CD8SP-CD22-FHVH-24-GS-[TCRg-7MD]-F- P2A-IgSP-Apa-CD20-2HCD25-VHH-GS- [TCRd-7MD]-F-F2A-PAC | 4205 | 14895 |
| | CD20 | CD22 | CD8SP-CD22-FHVH-24-GS-[TCRg-7MD]-F- P2A-IgSP-Apa-CD20-2HCD25-VHH-GS- [TCRd-7MD] | 4206 | 14896 |
| | CD20 | CD22 | CD8SP-CD22-FHVH-24-IgG1-Hinge-[TCRg- 7MD]-F-P2A-IgSP-Apa-CD20-2HCD25-VHH- GS-[TCRd-7MD]-F-F2A-PAC | 4207 | 14897 |
| | CD20 | CD22 | CD8SP-CD22-FHVH-24-IgG1-Hinge-[TCRg- 7MD]-F-P2A-IgSP-Apa-CD20-2HCD25-VHH- IgG1-Hinge-v2-[TCRd-7MD]-F-F2A-PAC | 4208 | 14898 |
| | CD20 | CD22 | CD8SP-CD22-FHVH-24-IgG1-Hinge-[TCRg- 7MD]-F-P2A-IgSP-Apa-CD20-2HCD25-VHH- IgG1-Hinge-v2-[TCRd-7MD] | 4209 | 14899 |
| | | CD22 | CD8SP-CD22-FHVH-24-[IgCL-TCRb-6MD] | 4210 | 14900 |
| | CD20 | CD22 | IgSP-Apa-CD20-2HCD25-VHH-[IgG1-CH1- TCRa-wt-opt2-6MD] | 4211 | 14901 |
| | | CD22 | CD8SP-CD22-FHVH-24-[IgCL-TCRg-6MD] | 4212 | 14902 |
| | CD20 | CD22 | IgSP-Apa-CD20-2HCD25-VHH-[IgG1-CH1-TCRd-6MD] | 4213 | 14903 |

**TABLE 29: Bispecific and Trispecific SARs Constructs targeting CD20 and a second antigen targeted by a CAR or a SIR**

| **1st (SIR) Target** | **2nd target** | **3rd target** | **Name of fragment** | **SEQ ID NO (DNA)** | **SEQ ID NO (PRT)** |
|---|---|---|---|---|---|
| CD19 | CD20 | | IgSP-Apa-CD20-vHH-2HCD25-G4Sx3v2-hu- mROO5-1-vH-Gly-Ser-Linker-vL-Myc-CD8TM- BBz | 4087 | 14777 |
| CD19 | CD20 | | CD8SP-hu-mROO5-1-vL-[hTCRa-CSDVP]-F- F2A-IgSP-Apa-CD20-vHH-2HCD25-G4Sx3v2- hu-mROO5-1-vH-[hTCRb-KACIAH]-F-P2A- PAC | 4088 | 14778 |
| CD19 | CD20 | | CD8SP-hu-mROO5-1-vL-[hTCRb-KACIAH]-F- P2A-IgSP-Apa-CD20-vHH-2HCD25-G4Sx3v2- hu-mROO5-1-vH-[hTCRa-CSDVP]-F-F2A-PAC | 4089 | 14779 |
| CD19 | CD20 | | CD8SP-hu-mROO5-1-vL-[hTCRb-S57C]-F- P2A-IgSP-Apa-CD20-vHH-2HCD25-G4Sx3v2- hu-mROO5-1-vH-[hTCRa-T48C]-F-F2A-PAC | 4090 | 14780 |
| CD19 | CD20 | | CD8SP-hu-mROO5-1-vL-[hTCRb-S57C]-F- P2A-IgSP-Apa-CD20-vHH-2HCD25-G4Sx3v2- hu-mROO5-1-vH-[hTCRa-T48C] | 4091 | 14781 |
| CD19 | CD20 | | CD8SP-hu-mROO5-1-vL-[hTCRb-S57C]-F- P2A-IgSP-Apa-CD20-vHH-2HCD25-G4Sx3v2- hu-mROO5-1-vH-[hTCRa-T48C]-F-F2A-K13- opt | 4092 | 14782 |
| CD19 | CD20 | | CD8SP-hu-mROO5-1-vL-[hTCRa-T48C]-F- P2A-IgSP-Apa-CD20-vHH-2HCD25-G4Sx3v2- hu-mROO5-1-vH-[hTCRb-S57C] | 4093 | 14783 |
| CD19 | CD20 | | CD8SP-hu-mROO5-1-vL-[hTCRa-T48C]-F- P2A-IgSP-Apa-CD20-vHH-2HCD25-G4Sx3v2- hu-mROO5-1-vH-[hTCRb-S57C]-F-P2A-K13- opt | 4094 | 14784 |
| CD19 | CD20 | | CD8SP-hu-mROO5-1-vL-[hTCRb-opt2]-F-P2A- IgSP-Apa-CD20-vHH-2HCD25-G4Sx3v2-hu- mROO5-1-vH-[hTCRa-opt2]-F-F2A-PAC | 4095 | 14785 |
| CD19 | CD20 | | CD8SP-hu-mROO5-1-vL-[hTCRb-opt2]-F-P2A- IgSP-Apa-CD20-vHH-2HCD25-G4Sx3v2-hu- mROO5-1-vH-Myc-[pr*etc.*Ra-Del48]-F-F2A- PAC | 4096 | 14786 |
| CD19 | CD20 | | CD8SP-hu-mROO5-1-vL-V5-[hTCRg1-opt]-F- P2A-IgSP-Apa-CD20-vHH-2HCD25-G4Sx3v2- | 4097 | 14787 |
| | | | hu-mROO5-1-vH-Myc-[hTCRd-opt]-F-F2A- PAC | | |
| CD19 | CD20 | | CD8SP-hu-mROO5-1-vL-[hTCRd-opt]-F-P2A- IgSP-Apa-CD20-vHH-2HCD25-G4Sx3v2-hu- mROO5-1-vH-[hTCRg1-opt] | 4098 | 14788 |
| CD19 | CD20 | | CD8-hu-mROO5-1-vL-IgCL-CD3zECDTMCP- opt-F-P2A-Spe-IgSP-Apa-CD20-vHH-2HCD25- G4Sx3v2-hu-mROO5-1-vH-IgG1-CH1- CD3zECDTMCP-opt2-F-F2A-PAC | 4099 | 14789 |
| CD19 | CD20 | | CD8SP-hu-mROO5-1-vL-[hTCRbECD- CD3zECDTMCP-opt]-F-P2A-IgSP-Apa-CD20- vHH-2HCD25-G4Sx3v2-hu-mROO5-1-vH- [hTCRaECD-CD3zECDTMCP-opt2] | 4100 | 14790 |
| CD19 | CD20 | | CD8SP-hu-mROO5-1-vL-[hTCRb-KAC-ECD- CD3zECDTMCP-opt]-F-P2A-IgSP-Apa-CD20- vHH-2HCD25-G4Sx3v2-hu-mROO5-1-vH- [hTCRa-CSDVP-ECD-CD3zECDTMCP-opt2] | 4101 | 14791 |
| CD19 | CD20 | | CD8SP-hu-mROO5-1-vL-V5-[hTCRbECD- CD3zECDTMCP-opt]-F-P2A-IgSP-Apa-CD20- vHH-2HCD25-G4Sx3v2-hu-mROO5-1-vH-Myc- [hTCRaECD-CD3zECDTM-28z-opt2] | 4102 | 14792 |
| CD19 | CD20 | | CD8SP-hu-mROO5-1-vL-V5-[hTCRbECD- CD3zECDTM-28z-opt]-F-P2A-IgSP-Apa-CD20- vHH-2HCD25-G4Sx3v2-hu-mROO5-1-vH-Myc- [hTCRaECD-CD3zECDTM-28z-opt2] | 4103 | 14793 |
| CD19 | CD20 | | CD8SP-hu-mROO5-1-vL-V5-[hTCRbECD- CD3zECDTMCP-opt]-F-P2A-IgSP-Apa-CD20- vHH-2HCD25-G4Sx3v2-hu-mROO5-1-vH- Myc4-[hTCRaECD-CD3zECDTM-BBz-opt2] | 4104 | 14794 |
| CD19 | CD20 | | CD8SP-hu-mROO5-1-vL-V5-[hTCRbECD- CD3zECDTM-BBz-opt]-F-P2A-IgSP-Apa- CD20-vHH-2HCD25-G4Sx3v2-hu-mROO5-1- vH-Myc4-[hTCRaECD-CD3zECDTM-BBz- opt2] | 4105 | 14795 |
| CD19 | CD20 | | CD8SP-hu-mROO5-1-vL-CD3zECDTMCP-opt- F-P2A-Spe-IgSP-Apa-CD20-vHH-2HCD25- G4Sx3v2-hu-mROO5-1-vH-Mlu- CD3zECDTMCP-opt2-F-F2A-PAC | 4106 | 14796 |
| CD19 | CD20 | | CD8SP-hu-mROO5-1-vL-[IgCL-TCRg-6MD]-F- P2A-IgSP-Apa-CD20-vHH-2HCD25-G4Sx3v2- hu-mROO5-1-vH-[IgG1-CH1-TCRd-6MD] | 4107 | 14797 |
| CD19 | CD20 | | CD8SP-hu-mROO5-1-vL-[IgCL-TCRb-IAH- 6MD]-F-P2A-IgSP-Apa-CD20-vHH-2HCD25- G4Sx3v2-hu-mROO5-1-vH-[IgG1-CH1-TCRa- SDVP-6MD] | 4108 | 14798 |
| CD19 | CD20 | | CD8SP-hu-mROO5-1-vL-[IgCL-TCRb-wt-opt2- 6MD]-F-P2A-IgSP-Apa-CD20-vHH-2HCD25- | 4109 | 14799 |
| | | | G4Sx3v2-hu-mROO5-1-vH-[IgG1-CH1-TCRa- wt-op2-6MD] | | |
| CD19 | CD20 | | IgSP-Apa-CD20-vHH-2HCD25-G4Sx3v2-hu- mROO5-1-(vH-vL)-G3S-CD3e-ECDTMCP-opt2 | 4110 | 14800 |
| CD19 | CD20 | | IgSP-Apa-CD20-vHH-2HCD25-G4Sx3v2-hu- mROO5-1-(vH-vL)-G3S-CD3d-ECDTMCP-opt2 | 4111 | 14801 |
| CD19 | CD20 | | IgSP-Apa-CD20-vHH-2HCD25-G4Sx3v2-hu- mROO5-1-(vH-vL)-G3S-CD3g-ECDTMCP-opt2 | 4112 | 14802 |
| CD19 | CD20 | | IgSP-Apa-CD20-vHH-2HCD25-G4Sx3v2-hu- mROO5-1-(vH-vL)-G3S-CD3z-ECDTMCP-opt2 | 4113 | 14803 |
| CD19 | CD20 | | IgSP-Apa-CD20-vHH-2HCD25-G4Sx3v2-hu- mROO5-1-vH-[hTCRa-opt2]-F-F2A-BlastR | 4114 | 14804 |
| CD19 | CD20 | | IgSP-Apa-CD20-vHH-2HCD25-G4Sx3v2-hu- mROO5-1-vH-[hTCRa-opt2] | 4115 | 14805 |
| CD19 | CD20 | | IgSP-Apa-CD20-vHH-2HCD25-G4Sx3v2-hu- mROO5-1-vH-Myc-[hTCRa-T48C-opt]-F-F2A- BlastR | 4116 | 14806 |
| CD19 | CD20 | | IgSP-Apa-CD20-vHH-2HCD25-G4Sx3v2-hu- mROO5-1-vH-Myc-[hTCRa-T48C-opt] | 4117 | 14807 |
| CD19 | | CD22 | CD8SP-CD22-FHVH-24-G4Sx3-R1-hu- mROO5-1-vL-Gly-Ser-Linker-hu-mROO5-1-vH- Myc-28z | 4118 | 14808 |
| CD19 | | CD22 | CD8SP-CD22-FHVH-24-G4Sx3-R1-hu- mROO5-1-vL-Gly-Ser-Linker-hu-mROO5-1-vH- Myc-CD8TM-BBz | 4119 | 14809 |
| CD19 | | CD22 | CD8SP-CD22-FHVH-24-G4Sx3-R1-hu- mROO5-1-vL-Gly-Ser-Linker-hu-mROO5-1-vH- Myc-CD8TM-z-P2A-K13-FLAG-T2A-PAC | 4120 | 14810 |
| CD19 | | CD22 | CD8SP-CD22-FHVH-24-G4Sx3-R1-hu- mROO5-1-vL-[hTCRa-CSDVP]-F-F2A-SP-hu- mROO5-1-vH-[hTCRb-KACIAH]-F-P2A-PAC | 4121 | 14811 |
| CD19 | | CD22 | CD8SP-CD22-FHVH-24-G4Sx3-R1-hu- mROO5-1-vL-[hTCRb-KACIAH]-F-P2A-SP-hu- mROO5-1-vH-[hTCRa-CSDVP]-F-F2A-PAC | 4122 | 14812 |
| CD19 | | CD22 | CD8SP-CD22-FHVH-24-G4Sx3-R1-hu- mROO5-1-vL-[hTCRb-S57C]-F-P2A-SP-hu- mROO5-1-vH-[hTCRa-T48C] | 4123 | 14813 |
| CD19 | | CD22 | CD8SP-CD22-FHVH-24-G4Sx3-R1-hu- mROO5-1-vL-[hTCRb-S57C]-F-P2A-SP-hu- mROO5-1-vH-[hTCRa-T48C]-F-F2A-K13-opt | 4124 | 14814 |
| CD19 | | CD22 | CD8SP-CD22-FHVH-24-G4Sx3-R1-hu- mROO5-1-vL-[hTCRa-T48C]-F-P2A-SP-hu- mROO5-1-vH-[hTCRb-S57C] | 4125 | 14815 |
| CD19 | | CD22 | CD8SP-CD22-FHVH-24-G4Sx3-R1-hu- mROO5-1-vL-[hTCRa-T48C]-F-P2A-SP-hu- mROO5-1-vH-[hTCRb-S57C]-F-P2A-K13-opt | 4126 | 14816 |
| CD19 | | CD22 | CD8SP-V5-[hTCRb-KACIAH]-F-P2A-CD8SP- CD22-FHVH-24-G4Sx3-R1-hu-mROO5-1-vL- Gly-Ser-Linker-hu-mROO5-1-vH-Myc-[hTCRa- CSDVP]-F-F2A-PAC | 4127 | 14817 |
| CD19 | | CD22 | CD8SP-MYC-[hTCRa-T48C-opt1]-F-F2A-SP- CD22-FHVH-24-G4Sx3-R1-hu-mROO5-1-vL- Gly-Ser-Linker-hu-mROO5-1-vH-V5-[hTCRb- S57C-opt1]-F-P2A-PAC | 4128 | 14818 |
| CD19 | | CD22 | CD8SP-CD22-FHVH-24-G4Sx3-R1-hu- mROO5-1-vL-[hTCRb-opt2]-F-P2A-SP-hu- mROO5-1-vH-[hTCRa-opt2]-F-F2A-PAC | 4129 | 14819 |
| CD19 | | CD22 | CD8SP-CD22-FHVH-24-G4Sx3-R1-hu- mROO5-1-vL-[hTCRb-opt2]-F-P2A-SP-hu- mROO5-1-vH-Myc-[pretc.Ra-Del48]-F-F2A- PAC | 4130 | 14820 |
| CD19 | | CD22 | CD8SP-[hTCRb-opt2]-F-P2A-CD8SP-CD22- FHVH-24-G4Sx3-R1-hu-mROO5-1-vL-Gly-Ser- Linker-hu-mROO5-1-vH-Myc4-[pr*etc.*Ra- Del48]-F-F2A-PAC | 4131 | 14821 |
| CD19 | | CD22 | CD8SP-CD22-FHVH-24-G4Sx3-R1-hu- mROO5-1-vL-V5-[hTCRg1-opt]-F-P2A-SP-hu- mROO5-1-vH-Myc-[hTCRd-opt]-F-F2A-PAC | 4132 | 14822 |
| CD19 | | CD22 | CD8SP-CD22-FHVH-24-G4Sx3-R1-hu- mROO5-1-vL-[hTCRd-opt]-F-P2A-SP-hu- mROO5-1-vH-[hTCRg1-opt] | 4133 | 14823 |
| CD19 | | CD22 | CD8SP-V5-[hTCRg1-opt]-F-P2A-CD8SP-CD22- FHVH-24-G4Sx3-R1-hu-mROO5-1-vL-Gly-Ser- Linker-hu-mROO5-1-vH-Myc-[hTCRd-opt]-F- F2A-PAC | 4134 | 14824 |
| CD19 | | CD22 | CD8-CD22-FHVH-24-G4Sx3-R1-hu-mROO5-1- vL-IgCL-CD3zECDTMCP-opt-F-P2A-Spe-SP- hu-mROO5-1-vH-IgG1-CH1-CD3zECDTMCP- opt2-F-F2A-PAC | 4135 | 14825 |
| CD19 | | CD22 | CD8SP-CD22-FHVH-24-G4Sx3-R1-hu- mROO5-1-vL-[hTCRbECD-CD3zECDTMCP- opt]-F-P2A-SP-hu-mROO5-1-vH-[hTCRaECD- CD3zECDTMCP-opt2] | 4136 | 14826 |
| CD19 | | CD22 | CD8SP-CD22-FHVH-24-G4Sx3-R1-hu- mROO5-1-vL-[hTCRb-KAC-ECD- CD3zECDTMCP-opt]-F-P2A-SP-hu-mROO5-1- vH-[hTCRa-CSDVP-ECD-CD3zECDTMCP- opt2] | 4137 | 14827 |
| CD19 | | CD22 | CD8SP-CD22-FHVH-24-G4Sx3-R1-hu- mROO5-1-vL-V5-[hTCRbECD- CD3zECDTMCP-opt]-F-P2A-SP-hu-mROO5-1- vH-Myc-[hTCRaECD-CD3zECDTM-28z-opt2] | 4138 | 14828 |
| CD19 | | CD22 | CD8SP-CD22-FHVH-24-G4Sx3-R1-hu- mROO5-1-vL-V5-[hTCRbECD-CD3zECDTM- | 4139 | 14829 |
| | | | 28z-opt]-F-P2A-SP-hu-mROO5-1-vH-Myc- [hTCRaECD-CD3zECDTM-28z-opt2] | | |
| CD19 | | CD22 | CD8SP-CD22-FHVH-24-G4Sx3-R1-hu- mROO5-1-vL-V5-[hTCRbECD- CD3zECDTMCP-opt]-F-P2A-SP-hu-mROO5-1- vH-Myc4-[hTCRaECD-CD3zECDTM-BBz- opt2] | 4140 | 14830 |
| CD19 | | CD22 | CD8SP-CD22-FHVH-24-G4Sx3-R1-hu- mROO5-1-vL-V5-[hTCRbECD-CD3zECDTM- BBz-opt]-F-P2A-SP-hu-mROO5-1-vH-Myc4- [hTCRaECD-CD3zECDTM-BBz-opt2] | 4141 | 14831 |
| CD19 | | CD22 | CD8-CD22-FHVH-24-G4Sx3-R1-hu-mROO5-1- vL-CD3zECDTMCP-opt-F-P2A-Spe-SP-hu- mROO5-1-vH-Mlu-CD3zECDTMCP-opt2-F- F2A-PAC | 4142 | 14832 |
| CD19 | | CD22 | CD8SP-CD22-FHVH-24-G4Sx3-R1-hu- mROO5-1-vL-[IgCL-TCRg-6MD]-F-P2A-SP- hu-mROO5-1-vH-[IgG1-CH1-TCRd-6MD] | 4143 | 14833 |
| CD19 | | CD22 | CD8SP-CD22-FHVH-24-G4Sx3-R1-hu- mROO5-1-vL-[IgCL-TCRb-IAH-6MD]-F-P2A- SP-hu-mROO5-1-vH-[IgG1-CH1-TCRa-SDVP- 6MD] | 4144 | 14834 |
| CD19 | | CD22 | CD8SP-CD22-FHVH-24-G4Sx3-R1-hu- mROO5-1-vL-[IgCL-TCRb-wt-opt2-6MD]-F- P2A-SP-hu-mROO5-1-vH-[IgG1-CH1-TCRa-wt- op2-6MD] | 4145 | 14835 |
| CD19 | | CD22 | CD8SP-CD22-FHVH-24-G4Sx3-R1-hu- mROO5-1-(vL-vH)-CD3e-ECDTMCP-opt2 | 4146 | 14836 |
| CD19 | | CD22 | CD8SP-CD22-FHVH-24-G4Sx3-R1-hu- mROO5-1-(vL-vH)-CD3d-ECDTMCP-opt2 | 4147 | 14837 |
| CD19 | | CD22 | CD8SP-CD22-FHVH-24-G4Sx3-R1-hu- mROO5-1-(vL-vH)-CD3g-ECDTMCP-opt2 | 4148 | 14838 |
| CD19 | | CD22 | CD8SP-CD22-FHVH-24-G4Sx3-R1-hu- mROO5-1-(vL-vH)-CD3z-ECDTMCP-opt2 | 4149 | 14839 |
| CD19 | | CD22 | CD8SP-CD22-FHVH-24-G4Sx3-R1-hu- mROO5-1-vL-Gly-Ser-Linker-hu-mROO5-1-vH- [hTCRa-opt2]-F-F2A-PAC | 4150 | 14840 |
| CD19 | | CD22 | CD8SP-CD22-FHVH-24-G4Sx3-R1-hu- mROO5-1-vL-Gly-Ser-Linker-hu-mROO5-1-vH- [hTCRb-opt2]-F-F2A-PAC | 4151 | 14841 |
| CD19 | | CD22 | CD8SP-CD22-FHVH-24-G4Sx3-R1-hu- mROO5-1-vL-Gly-Ser-Linker-hu-mROO5-1-vH- Myc4-[pretc.Ra-Del48]-F-F2A-PAC | 4152 | 14842 |
| CD19 | | CD22 | CD8SP-CD22-FHVH-24-G4Sx3-R1-hu- mROO5-1-vL-[hTCRb-opt2]-F-P2A-PAC | 4153 | 14843 |
| CD19 | | CD22 | CD8SP-CD22-FHVH-24-G4Sx3-R1-hu- mROO5-1-vL-[hTCRb-opt2] | 4154 | 14844 |
| CD19 | | CD22 | IgHSP-hu-mROO5-1-vH-[hTCRa-opt2]-F-F2A- BlastR | 4155 | 14845 |
| CD19 | | CD22 | IgHSP-hu-mROO5-1-vH-[hTCRa-opt2] | 4156 | 14846 |
| CD19 | | CD22 | CD8SP-CD22-FHVH-24-G4Sx3-R1-hu- mROO5-1-vL-V5-[hTCRb-S57C-opt]-F-P2A- PAC | 4157 | 14847 |
| CD19 | | CD22 | CD8SP-CD22-FHVH-24-G4Sx3-R1-hu- mROO5-1-vL-V5-[hTCRb-S57C-opt] | 4158 | 14848 |
| CD19 | | | IgHSP-hu-mROO5-1-vH-Myc-[hTCRa-T48C- opt]-F-F2A-BlastR | 4159 | 14849 |
| CD19 | | | IgHSP-hu-mROO5-1-vH-Myc-[hTCRa-T48C- opt] | 4160 | 14850 |
| CD19 | | CD22 | CD8SP-CD22-FHVH-24-G4Sx3-R1-hu- mROO5-1-vL-Gly-Ser-Linker-hu-mROO5-1-vH- [hTCRa-SDVP]-F-F2A-PAC | 4161 | 14851 |
| CD19 | | CD22 | CD8SP-CD22-FHVH-24-G4Sx3-R1-hu- mROO5-1-vL-Gly-Ser-Linker-hu-mROO5-1-vH- [hTCRb-KAIAH]-F-P2A-PAC | 4162 | 14852 |
| CD19 | | CD22 | CD8SP-CD22-FHVH-24-G4Sx3-R1-hu- mROO5-1-vL-[hTCRa-CSDVP]-F-F2A-IgSP- Apa-CD20-vHH-2HCD25-G4Sx3v2-hu- mROO5-1-vH-[hTCRb-KACIAH]-F-P2A-PAC | 4163 | 14853 |
| CD19 | | CD22 | CD8SP-CD22-FHVH-24-G4Sx3-R1-hu- mROO5-1-vL-[hTCRb-KACIAH]-F-P2A-IgSP- Apa-CD20-vHH-2HCD25-G4Sx3v2-hu- mROO5-1-vH-[hTCRa-CSDVP]-F-F2A-PAC | 4164 | 14854 |
| CD19 | CD20 | CD22 | CD8SP-CD22-FHVH-24-G4Sx3-R1-hu- mROO5-1-vL-[hTCRb-S57C]-F-P2A-IgSP-Apa- CD20-vHH-2HCD25-G4Sx3v2-hu-mROO5-1- vH-[hTCRa-T48C]-F-F2A-PAC | 4165 | 14855 |
| CD19 | CD20 | CD22 | CD8SP-CD22-FHVH-24-G4Sx3-R1-hu- mROO5-1-vL-[hTCRb-S57C]-F-P2A-IgSP-Apa- CD20-vHH-2HCD25-G4Sx3v2-hu-mROO5-1- vH-[hTCRa-T48C] | 4166 | 14856 |
| CD19 | CD20 | CD22 | CD8SP-CD22-FHVH-24-G4Sx3-R1-hu- mROO5-1-vL-[hTCRb-S57C]-F-P2A-IgSP-Apa- CD20-vHH-2HCD25-G4Sx3v2-hu-mROO5-1- vH-[hTCRa-T48C]-F-F2A-K13-opt | 4167 | 14857 |
| CD19 | CD20 | CD22 | CD8SP-CD22-FHVH-24-G4Sx3-R1-hu- mROO5-1-vL-[hTCRa-T48C]-F-P2A-IgSP-Apa- CD20-vHH-2HCD25-G4Sx3v2-hu-mROO5-1- vH-[hTCRb-S57C] | 4168 | 14858 |
| CD19 | CD20 | CD22 | CD8SP-CD22-FHVH-24-G4Sx3-R1-hu- mROO5-1-vL-[hTCRa-T48C]-F-P2A-IgSP-Apa- CD20-vHH-2HCD25-G4Sx3v2-hu-mROO5-1- vH-[hTCRb-S57C]-F-P2A-K13-opt | 4169 | 14859 |
| CD19 | CD20 | CD22 | CD8SP-CD22-FHVH-24-G4Sx3-R1-hu- mROO5-1-vL-[hTCRb-opt2]-F-P2A-IgSP-Apa- | 4170 | 14860 |
| | | | CD20-vHH-2HCD25-G4Sx3v2-hu-mROO5-1- vH-[hTCRa-opt2]-F-F2A-PAC | | |
| CD19 | CD20 | CD22 | CD8SP-CD22-FHVH-24-G4Sx3-R1-hu- mROO5-1-vL-[hTCRb-opt2]-F-P2A-IgSP-Apa- CD20-vHH-2HCD25-G4Sx3v2-hu-mROO5-1- vH-Myc-[pretc.Ra-Del48]-F-F2A-PAC | 4171 | 14861 |
| CD19 | CD20 | CD22 | CD8SP-CD22-FHVH-24-G4Sx3-R1-hu- mROO5-1-vL-V5-[hTCRg1-opt]-F-P2A-IgSP- Apa-CD20-vHH-2HCD25-G4Sx3v2-hu- mROO5-1-vH-Myc-[hTCRd-opt]-F-F2A-PAC | 4172 | 14862 |
| CD19 | CD20 | CD22 | CD8SP-CD22-FHVH-24-G4Sx3-R1-hu- mROO5-1-vL-[hTCRd-opt]-F-P2A-IgSP-Apa- CD20-vHH-2HCD25-G4Sx3v2-hu-mROO5-1- vH-[hTCRg1-opt] | 4173 | 14863 |
| CD19 | CD20 | CD22 | CD8SP-CD22-FHVH-24-G4Sx3-R1-hu- mROO5-1-vL-IgCL-CD3zECDTMCP-opt-F- P2A-Spe-IgSP-Apa-CD20-vHH-2HCD25- G4Sx3v2-hu-mROO5-1-vH-IgG1-CH1- CD3zECDTMCP-opt2-F-F2A-PAC | 4174 | 14864 |
| CD19 | CD20 | CD22 | CD8SP-CD22-FHVH-24-G4Sx3-R1-hu- mROO5-1-vL-[hTCRbECD-CD3zECDTMCP- opt]-F-P2A-IgSP-Apa-CD20-vHH-2HCD25- G4Sx3v2-hu-mROO5-1-vH-[hTCRaECD- CD3zECDTMCP-opt2] | 4175 | 14865 |
| CD19 | CD20 | CD22 | CD8SP-CD22-FHVH-24-G4Sx3-R1-hu- mROO5-1-vL-[hTCRb-KAC-ECD- CD3zECDTMCP-opt]-F-P2A-IgSP-Apa-CD20- vHH-2HCD25-G4Sx3v2-hu-mROO5-1-vH- [hTCRa-CSDVP-ECD-CD3zECDTMCP-opt2] | 4176 | 14866 |
| CD19 | CD20 | CD22 | CD8SP-CD22-FHVH-24-G4Sx3-R1-hu- mROO5-1-vL-V5-[hTCRbECD- CD3zECDTMCP-opt]-F-P2A-IgSP-Apa-CD20- vHH-2HCD25-G4Sx3v2-hu-mROO5-1-vH-Myc- [hTCRaECD-CD3zECDTM-28z-opt2] | 4177 | 14867 |
| CD19 | CD20 | CD22 | CD8SP-CD22-FHVH-24-G4Sx3-R1-hu- mROO5-1-vL-V5-[hTCRbECD-CD3zECDTM- 28z-opt]-F-P2A-IgSP-Apa-CD20-vHH-2HCD25- G4Sx3v2-hu-mROO5-1-vH-Myc-[hTCRaECD- CD3zECDTM-28z-opt2] | 4178 | 14868 |
| CD19 | CD20 | CD22 | CD8SP-CD22-FHVH-24-G4Sx3-R1-hu- mROO5-1-vL-V5-[hTCRbECD- CD3zECDTMCP-opt]-F-P2A-IgSP-Apa-CD20- vHH-2HCD25-G4Sx3v2-hu-mROO5-1-vH- Myc4-[hTCRaECD-CD3zECDTM-BBz-opt2] | 4179 | 14869 |
| CD19 | CD20 | CD22 | CD8SP-CD22-FHVH-24-G4Sx3-R1-hu- mROO5-1-vL-V5-[hTCRbECD-CD3zECDTM- BBz-opt]-F-P2A-IgSP-Apa-CD20-vHH- 2HCD25-G4Sx3v2-hu-mROO5-1-vH-Myc4- [hTCRaECD-CD3zECDTM-BBz-opt2] | 4180 | 14870 |
| CD19 | CD20 | CD22 | CD8SP-CD22-FHVH-24-G4Sx3-R1-hu- mROO5-1-vL-CD3zECDTMCP-opt-F-P2A-Spe- IgSP-Apa-CD20-vHH-2HCD25-G4Sx3v2-hu- mROO5-1-vH-Mlu-CD3zECDTMCP-opt2-F- F2A-PAC | 4181 | 14871 |
| CD19 | CD20 | CD22 | CD8SP-CD22-FHVH-24-G4Sx3-R1-hu- mROO5-1-vL-[IgCL-TCRg-6MD]-F-P2A-IgSP- Apa-CD20-vHH-2HCD25-G4Sx3v2-hu- mROO5-1-vH-[IgG1-CH1-TCRd-6MD] | 4182 | 14872 |
| CD19 | CD20 | CD22 | CD8SP-CD22-FHVH-24-G4Sx3-R1-hu- mROO5-1-vL-[IgCL-TCRb-IAH-6MD]-F-P2A- IgSP-Apa-CD20-vHH-2HCD25-G4Sx3v2-hu- mROO5-1-vH-[IgG1-CH1-TCRa-SDVP-6MD] | 4183 | 14873 |
| CD19 | CD20 | CD22 | CD8SP-CD22-FHVH-24-G4Sx3-R1-hu- mROO5-1-vL-[IgCL-TCRb-wt-opt2-6MD]-F- P2A-IgSP-Apa-CD20-vHH-2HCD25-G4Sx3v2- hu-mROO5-1-vH-[IgG1-CH1-TCRa-wt-op2- 6MD] | 4184 | 14874 |
| | CD20 | CD22 | CD8SP-CD22-FHVH-24-[IgCL-TCRb-wt-opt2- 6MD]-F-P2A-IgSP-Apa-CD20-2HCD25-VHH- [IgG1-CH1-TCRa-wt-opt2-6MD]-F-F2A-PAC | 4185 | 14875 |
| | CD20 | CD22 | CD8SP-CD22-FHVH-24-[IgCL-TCRb-wt-opt2- 6MD]-F-P2A-IgSP-Apa-CD20-2HCD25-VHH- [IgG1-CH1-TCRa-wt-opt2-6MD] | 4186 | 14876 |
| | CD20 | CD22 | CD8SP-CD22-FHVH-24-GS-[TCRb-wt-opt2- 6MD]-F-P2A-IgSP-Apa-CD20-2HCD25-VHH- GS-[TCRa-wt-opt2-6MD]-F-F2A-PAC | 4187 | 14877 |
| | CD20 | CD22 | CD8SP-CD22-FHVH-24-GS-[TCRb-wt-opt2- 6MD]-F-P2A-IgSP-Apa-CD20-2HCD25-VHH- GS-[TCRa-wt-opt2-6MD] | 4188 | 14878 |
| | CD20 | CD22 | CD8SP-CD22-FHVH-24-IgG1-Hinge-[TCRb-wt- opt2-6MD]-F-P2A-IgSP-Apa-CD20-2HCD25- VHH-GS-[TCRa-wt-opt2-6MD]-F-F2A-PAC | 4189 | 14879 |
| | CD20 | CD22 | CD8SP-CD22-FHVH-24-GS-[TCRb-wt-opt2- 6MD]-F-P2A-IgSP-Apa-CD20-2HCD25-VHH- IgG1-Hinge-v2-[TCRa-wt-opt2-6MD]-F-F2A- PAC | 4190 | 14880 |
| | CD20 | CD22 | CD8SP-CD22-FHVH-24-IgG1-Hinge-[TCRb-wt- opt2-6MD]-F-P2A-IgSP-Apa-CD20-2HCD25- VHH-IgG1-Hinge-v2-[TCRa-wt-opt2-6MD]-F- F2A-PAC | 4191 | 14881 |
| | CD20 | CD22 | CD8SP-CD22-FHVH-24-IgG1-Hinge-[TCRb-wt- opt2-6MD]-F-P2A-IgSP-Apa-CD20-2HCD25- VHH-IgG1-Hinge-v2-[TCRa-wt-opt2-6MD] | 4192 | 14882 |
| | CD20 | CD22 | CD8SP-CD22-FHVH-24-GS-[TCRb-wt-opt2- 6MD]-F-P2A-IgSP-Apa-CD20-2HCD25-VHH- IgG1-Hinge-v2-[TCRa-wt-opt2-6MD] | 4193 | 14883 |
| | CD20 | CD22 | CD8SP-CD22-FHVH-24-[IgCL-TCRg-6MD]-F- P2A-IgSP-Apa-CD20-2HCD25-VHH-[IgG1- CH1-TCRd-6MD]-F-F2A-PAC | 4194 | 14884 |
| | CD20 | CD22 | CD8SP-CD22-FHVH-24-[IgCL-TCRg-6MD]-F- P2A-IgSP-Apa-CD20-2HCD25-VHH-[IgG1- CH1-TCRd-6MD] | 4195 | 14885 |
| | CD20 | CD22 | CD8SP-CD22-FHVH-24-GS-[TCRg-6MD]-F- P2A-IgSP-Apa-CD20-2HCD25-VHH-GS- [TCRd-6MD]-F-F2A-PAC | 4196 | 14886 |
| | CD20 | CD22 | CD8SP-CD22-FHVH-24-GS-[TCRg-6MD]-F- P2A-IgSP-Apa-CD20-2HCD25-VHH-GS- [TCRd-6MD] | 4197 | 14887 |
| | CD20 | CD22 | CD8SP-CD22-FHVH-24-IgG1-Hinge-[TCRg- 6MD]-F-P2A-IgSP-Apa-CD20-2HCD25-VHH- GS-[TCRd-6MD]-F-F2A-PAC | 4198 | 14888 |
| | CD20 | CD22 | CD8SP-CD22-FHVH-24-GS-[TCRg-6MD]-F- P2A-IgSP-Apa-CD20-2HCD25-VHH-IgG1- Hinge-v2-[TCRd-6MD]-F-F2A-PAC | 4199 | 14889 |
| | CD20 | CD22 | CD8SP-CD22-FHVH-24-IgG1-Hinge-[TCRg- 6MD]-F-P2A-IgSP-Apa-CD20-2HCD25-VHH- IgG1-Hinge-v2-[TCRd-6MD]-F-F2A-PAC | 4200 | 14890 |
| | CD20 | CD22 | CD8SP-CD22-FHVH-24-IgG1-Hinge-[TCRg- 6MD]-F-P2A-IgSP-Apa-CD20-2HCD25-VHH- IgG1-Hinge-v2-[TCRd-6MD] | 4201 | 14891 |
| | CD20 | CD22 | CD8SP-CD22-FHVH-24-GS-[TCRg-6MD]-F- P2A-IgSP-Apa-CD20-2HCD25-VHH-IgG1- Hinge-v2-[TCRd-6MD] | 4202 | 14892 |
| | CD20 | CD22 | CD8SP-CD22-FHVH-24-GS-[TCRg-7MD]-F- P2A-IgSP-Apa-CD20-2HCD25-VHH-GS- [TCRd-6MD]-F-F2A-PAC | 4203 | 14893 |
| | CD20 | CD22 | CD8SP-CD22-FHVH-24-GS-[TCRg-6MD]-F- P2A-IgSP-Apa-CD20-2HCD25-VHH-GS- [TCRd-7MD]-F-F2A-PAC | 4204 | 14894 |
| | CD20 | CD22 | CD8SP-CD22-FHVH-24-GS-[TCRg-7MD]-F- P2A-IgSP-Apa-CD20-2HCD25-VHH-GS- [TCRd-7MD]-F-F2A-PAC | 4205 | 14895 |
| | CD20 | CD22 | CD8SP-CD22-FHVH-24-GS-[TCRg-7MD]-F- P2A-IgSP-Apa-CD20-2HCD25-VHH-GS- [TCRd-7MD] | 4206 | 14896 |
| | CD20 | CD22 | CD8SP-CD22-FHVH-24-IgG1-Hinge-[TCRg- 7MD]-F-P2A-IgSP-Apa-CD20-2HCD25-VHH- GS-[TCRd-7MD]-F-F2A-PAC | 4207 | 14897 |
| | CD20 | CD22 | CD8SP-CD22-FHVH-24-IgG1-Hinge-[TCRg- 7MD]-F-P2A-IgSP-Apa-CD20-2HCD25-VHH- IgG1-Hinge-v2-[TCRd-7MD]-F-F2A-PAC | 4208 | 14898 |
| | CD20 | CD22 | CD8SP-CD22-FHVH-24-IgG1-Hinge-[TCRg- 7MD]-F-P2A-IgSP-Apa-CD20-2HCD25-VHH- IgG1-Hinge-v2-[TCRd-7MD] | 4209 | 14899 |
| | | CD22 | CD8SP-CD22-FHVH-24-[IgCL-TCRb-6MD] | 4210 | 14900 |
| | CD20 | CD22 | IgSP-Apa-CD20-2HCD25-VHH-[IgG1-CH1- TCRa-wt-opt2-6MD] | 4211 | 14901 |
| | | CD22 | CD8SP-CD22-FHVH-24-[IgCL-TCRg-6MD] | 4212 | 14902 |
| | CD20 | CD22 | IgSP-Apa-CD20-2HCD25-VHH-[IgG1-CH1- TCRd-6MD] | 4213 | 14903 |

**TABLE 31: Uni-Bi and Multispecific SAR Constructs**

| **TARGET** | | | | | | | |
|---|---|---|---|---|---|---|---|
| **1st** | **2nd** | **3rd** | **4th** | **5th** | **Name of fragment** | **SEQ ID NO (DNA)** | **SEQ ID NO (PRT)** |
| CD1 9 | CD1 9 | | | | CD8SP-CD19-vHH-048-G4Sx3-R1-hu- mROO5-1-vL-Gly-Ser-Linker-hu-mROO5- 1-vH-Myc-28z | 4976 | 15666 |
| CD1 9 | CD1 9 | | | | CD8SP-CD19-vHH-048-G4Sx3-R1-hu- mROO5-1-vL-Gly-Ser-Linker-hu-mROO5- 1-vH-Myc-CD8TM-BBz | 4977 | 15667 |
| CD1 9 | CD1 9 | | | | CD8SP-CD19-vHH-048-G4Sx3-R1-hu- mROO5-1-vL-Gly-Ser-Linker-hu-mROO5- 1-vH-Myc-CD8TM-z-P2A-K13-FLAG- T2A-PAC | 4978 | 15668 |
| CD1 9 | CD1 9 | | | | CD8SP-CD19-vHH-048-G4Sx3-R1-hu- mROO5-1-vL-[hTCRa-CSDVP]-F-F2A-SP- hu-mROO5-1-vH-[hTCRb-KACIAH]-F- P2A-PAC | 4979 | 15669 |
| CD1 9 | CD1 9 | | | | CD8SP-CD19-vHH-048-G4Sx3-R1-hu- mROO5-1-vL-[hTCRb-KACIAH]-F-P2A- SP-hu-mROO5-1-vH-[hTCRa-CSDVP]-F- F2A-PAC | 4980 | 15670 |
| CD1 9 | CD1 9 | | | | CD8SP-CD19-vHH-048-G4Sx3-R1-hu- mROO5-1-vL-[hTCRb-S57C]-F-P2A-SP- hu-mROO5-1-vH-[hTCRa-T48C] | 4981 | 15671 |
| CD1 9 | CD1 9 | | | | CD8SP-CD19-vHH-048-G4Sx3-R1-hu- mROO5-1-vL-[hTCRb-S57C]-F-P2A-SP- hu-mROO5-1-vH-[hTCRa-T48C]-F-F2A- K13-opt | 4982 | 15672 |
| CD1 9 | CD1 9 | | | | CD8SP-CD19-vHH-048-G4Sx3-R1-hu- mROO5-1-vL-[hTCRa-T48C]-F-P2A-SP- hu-mROO5-1-vH-[hTCRb-S57C] | 4983 | 15673 |
| CD1 9 | CD1 9 | | | | CD8SP-CD19-vHH-048-G4Sx3-R1-hu- mROO5-1-vL-[hTCRa-T48C]-F-P2A-SP- hu-mROO5-1-vH-[hTCRb-S57C]-F-P2A- K13-opt | 4984 | 15674 |
| CD1 9 | CD1 9 | | | | CD8SP-V5-[hTCRb-KACIAH]-F-P2A- CD8SP-CD19-vHH-048-G4Sx3-R1-hu- mROO5-1-vL-Gly-Ser-Linker-hu-mROO5- 1-vH-Myc-[hTCRa-CSDVP]-F-F2A-PAC | 4985 | 15675 |
| CD1 9 | CD1 9 | | | | CD8SP-MYC-[hTCRa-T48C-opt1]-F-F2A- SP-CD19-vHH-048-G4Sx3-R1-hu-mROO5- 1-vL-Gly-Ser-Linker-hu-mROO5-1-vH-V5- [hTCRb-S57C-opt1]-F-P2A-PAC | 4986 | 15676 |
| CD1 9 | CD1 9 | | | | CD8SP-CD19-vHH-048-G4Sx3-R1-hu- mROO5-1-vL-[hTCRb-opt2]-F-P2A-SP-hu- mROO5-1-vH-[hTCRa-opt2]-F-F2A-PAC | 4987 | 15677 |
| CD1 9 | CD1 9 | | | | CD8SP-CD19-vHH-048-G4Sx3-R1-hu- mROO5-1-vL-[hTCRb-opt2]-F-P2A-SP-hu- mROO5-1-vH-Myc-[pr*etc.*Ra-Del48]-F- F2A-PAC | 4988 | 15678 |
| CD1 9 | CD1 9 | | | | CD8SP-[hTCRb-opt2]-F-P2A-CD8SP- CD19-vHH-048-G4Sx3-R1-hu-mROO5-1- vL-Gly-Ser-Linker-hu-mROO5-1-vH-Myc4- [pretc.Ra-Del48]-F-F2A-PAC | 4989 | 15679 |
| CD1 9 | CD1 9 | | | | CD8SP-CD19-vHH-048-G4Sx3-R1-hu- mROO5-1-vL-V5-[hTCRg1-opt]-F-P2A-SP- hu-mROO5-1-vH-Myc-[hTCRd-opt]-F-F2A- PAC | 4990 | 15680 |
| CD1 9 | CD1 9 | | | | CD8SP-CD19-vHH-048-G4Sx3-R1-hu- mROO5-1-vL-[hTCRd-opt]-F-P2A-SP-hu- mROO5-1-vH-[hTCRg1-opt] | 4991 | 15681 |
| CD1 9 | CD1 9 | | | | CD8SP-V5-[hTCRg1-opt]-F-P2A-CD8SP- CD19-vHH-048-G4Sx3-R1-hu-mROO5-1- vL-Gly-Ser-Linker-hu-mROO5-1-vH-Myc- [hTCRd-opt]-F-F2A-PAC | 4992 | 15682 |
| CD1 9 | CD1 9 | | | | CD8SP-CD19-vHH-048-G4Sx3-R1-hu- mROO5-1-vL-IgCL-CD3zECDTMCP-opt- F-P2A-Spe-SP-hu-mROO5-1-vH-IgG1- CH1-CD3zECDTMCP-opt2-F-F2A-PAC | 4993 | 15683 |
| CD1 9 | CD1 9 | | | | CD8SP-CD19-vHH-048-G4Sx3-R1-hu- mROO5-1-vL-[hTCRbECD- CD3zECDTMCP-opt]-F-P2A-SP-hu- mROO5-1-vH-[hTCRaECD- CD3zECDTMCP-opt2] | 4994 | 15684 |
| CD1 9 | CD1 9 | | | | CD8SP-CD19-vHH-048-G4Sx3-R1-hu- mROO5-1-vL-[hTCRb-KAC-ECD- CD3zECDTMCP-opt]-F-P2A-SP-hu- mROO5-1-vH-[hTCRa-CSDVP-ECD- CD3zECDTMCP-opt2] | 4995 | 15685 |
| CD1 9 | CD1 9 | | | | CD8SP-CD19-vHH-048-G4Sx3-R1-hu- mROO5-1-vL-V5-[hTCRbECD- CD3zECDTMCP-opt]-F-P2A-SP-hu- mROO5-1-vH-Myc-[hTCRaECD- CD3zECDTM-28z-opt2] | 4996 | 15686 |
| CD1 9 | CD1 9 | | | | CD8SP-CD19-vHH-048-G4Sx3-R1-hu- mROO5-1-vL-V5-[hTCRbECD- CD3zECDTM-28z-opt]-F-P2A-SP-hu- mROO5-1-vH-Myc-[hTCRaECD- CD3zECDTM-28z-opt2] | 4997 | 15687 |
| CD1 9 | CD1 9 | | | | CD8SP-CD19-vHH-048-G4Sx3-R1-hu- mROO5-1-vL-V5-[hTCRbECD- CD3zECDTMCP-opt]-F-P2A-SP-hu- mROO5-1-vH-Myc4-[hTCRaECD- CD3zECDTM-BBz-opt2] | 4998 | 15688 |
| CD1 9 | CD1 9 | | | | CD8SP-CD19-vHH-048-G4Sx3-R1-hu- mROO5-1-vL-V5-[hTCRbECD- CD3zECDTM-BBz-opt]-F-P2A-SP-hu- mROO5-1-vH-Myc4-[hTCRaECD- CD3zECDTM-BBz-opt2] | 4999 | 15689 |
| CD1 9 | CD1 9 | | | | CD8SP-CD19-vHH-048-G4Sx3-R1-hu- mROO5-1-vL-CD3zECDTMCP-opt-F-P2A- Spe-SP-hu-mROO5-1-vH-Mlu- CD3zECDTMCP-opt2-F-F2A-PAC | 5000 | 15690 |
| CD1 9 | CD1 9 | | | | CD8SP-CD19-vHH-048-G4Sx3-R1-hu- mROO5-1-vL-[IgCL-TCRg-6MD]-F-P2A- SP-hu-mROO5-1-vH-[IgG1-CH1-TCRd- 6MD] | 5001 | 15691 |
| CD1 9 | CD1 9 | | | | CD8SP-CD19-vHH-048-G4Sx3-R1-hu- mROO5-1-vL-[IgCL-TCRb-IAH-6MD]-F- P2A-SP-hu-mROO5-1-vH-[IgG1-CH1- TCRa-SDVP-6MD] | 5002 | 15692 |
| CD1 9 | CD1 9 | | | | CD8SP-CD19-vHH-048-G4Sx3-R1-hu- mROO5-1-vL-[IgCL-TCRb-wt-opt2-6MD]- F-P2A-SP-hu-mROO5-1-vH-[IgG1-CH1- TCRa-wt-op2-6MD] | 5003 | 15693 |
| CD1 9 | CD1 9 | | | | CD8SP-CD19-vHH-048-G4Sx3-R1-hu- mROO5-1-(vL-vH)-CD3e-ECDTMCP-opt2 | 5004 | 15694 |
| CD1 9 | CD1 9 | | | | CD8SP-CD19-vHH-048-G4Sx3-R1-hu- mROO5-1-(vL-vH)-CD3d-ECDTMCP-opt2 | 5005 | 15695 |
| CD1 9 | CD1 9 | | | | CD8SP-CD19-vHH-048-G4Sx3-R1-hu- mROO5-1-(vL-vH)-CD3g-ECDTMCP-opt2 | 5006 | 15696 |
| CD1 9 | CD1 9 | | | | CD8SP-CD19-vHH-048-G4Sx3-R1-hu- mROO5-1-(vL-vH)-CD3z-ECDTMCP-opt2 | 5007 | 15697 |
| CD1 9 | CD1 9 | | | | CD8SP-CD19-vHH-048-G4Sx3-R1-hu- mROO5-1-vL-Gly-Ser-Linker-hu-mROO5- 1-vH-[hTCRa-opt2]-F-F2A-PAC | 5008 | 15698 |
| CD1 9 | CD1 9 | | | | CD8SP-CD19-vHH-048-G4Sx3-R1-hu- mROO5-1-vL-Gly-Ser-Linker-hu-mROO5- 1-vH-[hTCRb-opt2]-F-F2A-PAC | 5009 | 15699 |
| CD1 9 | CD1 9 | | | | CD8SP-CD19-vHH-048-G4Sx3-R1-hu- mROO5-1-vL-Gly-Ser-Linker-hu-mROO5- 1-vH-Myc4-[pretc.Ra-Del48]-F-F2A-PAC | 5010 | 15700 |
| CD1 9 | CD1 9 | | | | CD8SP-CD19-vHH-048-G4Sx3-R1-hu- mROO5-1-vL-[hTCRb-opt2]-F-P2A-PAC | 5011 | 15701 |
| CD1 9 | CD1 9 | | | | CD8SP-CD19-vHH-048-G4Sx3-R1-hu- mROO5-1-vL-[hTCRb-opt2] | 5012 | 15702 |
| CD1 9 | CD1 9 | | | | CD8SP-CD19-vHH-048-G4Sx3-R1-hu- mROO5-1-vL-V5-[hTCRb-S57C-opt]-F- P2A-PAC | 5013 | 15703 |
| CD1 9 | CD1 9 | | | | CD8SP-CD19-vHH-048-G4Sx3-R1-hu- mROO5-1-vL-V5-[hTCRb-S57C-opt] | 5014 | 15704 |
| CD1 9 | CD1 9 | | | | CD8SP-CD19-vHH-048-G4Sx3-R1-hu- mROO5-1-vL-Gly-Ser-Linker-hu-mROO5- 1-vH-[hTCRa-SDVP]-F-F2A-PAC | 5015 | 15705 |
| CD1 9 | CD1 9 | | | | CD8SP-CD19-vHH-048-G4Sx3-R1-hu- mROO5-1-vL-Gly-Ser-Linker-hu-mROO5- 1-vH-[hTCRb-KAIAH]-F-P2A-PAC | 5016 | 15706 |
| CD1 9 | | CD 20 | | | IgSP-Apa-CD20-vHH-2HCD25-G4Sx3v2- hu-mROO5-1-vH-Gly-Ser-Linker-vL-Myc- CD8TM-BBz | 5017 | 15707 |
| CD1 9 | | CD 20 | | | CD8SP-hu-mROO5-1-vL-[hTCRa-CSDVP]- F-F2A-IgSP-Apa-CD20-vHH-2HCD25- G4Sx3v2-hu-mROO5-1-vH-[hTCRb- KACIAH]-F-P2A-PAC | 5018 | 15708 |
| CD1 9 | | CD 20 | | | CD8SP-hu-mROO5-1-vL-[hTCRb- KACIAH]-F-P2A-IgSP-Apa-CD20-vHH- 2HCD25-G4Sx3v2-hu-mROO5-1-vH- [hTCRa-CSDVP]-F-F2A-PAC | 5019 | 15709 |
| CD1 9 | | CD 20 | | | CD8SP-hu-mROO5-1-vL-[hTCRb-S57C]-F- P2A-IgSP-Apa-CD20-vHH-2HCD25- G4Sx3v2-hu-mROO5-1-vH-[hTCRa-T48C]- F-F2A-PAC | 5020 | 15710 |
| CD1 9 | | CD 20 | | | CD8SP-hu-mROO5-1-vL-[hTCRb-S57C]-F- P2A-IgSP-Apa-CD20-vHH-2HCD25- G4Sx3v2-hu-mROO5-1-vH-[hTCRa-T48C] | 5021 | 15711 |
| CD1 9 | | CD 20 | | | CD8SP-hu-mROO5-1-vL-[hTCRb-S57C]-F- P2A-IgSP-Apa-CD20-vHH-2HCD25- G4Sx3v2-hu-mROO5-1-vH-[hTCRa-T48C]- F-F2A-K13-opt | 5022 | 15712 |
| CD1 9 | | CD 20 | | | CD8SP-hu-mROO5-1-vL-[hTCRa-T48C]-F- P2A-IgSP-Apa-CD20-vHH-2HCD25- G4Sx3v2-hu-mROO5-1-vH-[hTCRb-S57C] | 5023 | 15713 |
| CD1 9 | | CD 20 | | | CD8SP-hu-mROO5-1-vL-[hTCRa-T48C]-F- P2A-IgSP-Apa-CD20-vHH-2HCD25- G4Sx3v2-hu-mROO5-1-vH-[hTCRb-S57C]- F-P2A-K13-opt | 5024 | 15714 |
| CD1 9 | | CD 20 | | | CD8SP-hu-mROO5-1-vL-[hTCRb-opt2]-F- P2A-IgSP-Apa-CD20-vHH-2HCD25- G4Sx3v2-hu-mROO5-1-vH-[hTCRa-opt2]- F-F2A-PAC | 5025 | 15715 |
| CD1 9 | | CD 20 | | | CD8SP-hu-mROO5-1-vL-[hTCRb-opt2]-F- P2A-IgSP-Apa-CD20-vHH-2HCD25- G4Sx3v2-hu-mROO5-1-vH-Myc-[pretc.Ra- Del48]-F-F2A-PAC | 5026 | 15716 |
| CD1 9 | | CD 20 | | | CD8SP-hu-mROO5-1-vL-V5-[hTCRg1- opt]-F-P2A-IgSP-Apa-CD20-vHH-2HCD25- G4Sx3v2-hu-mROO5-1-vH-Myc-[hTCRd- opt]-F-F2A-PAC | 5027 | 15717 |
| CD1 9 | | CD 20 | | | CD8SP-hu-mROO5-1-vL-[hTCRd-opt]-F- P2A-IgSP-Apa-CD20-vHH-2HCD25- G4Sx3v2-hu-mROO5-1-vH-[hTCRg1-opt] | 5028 | 15718 |
| CD1 9 | | CD 20 | | | CD8SP-hu-mROO5-1-vL-IgCL- CD3zECDTMCP-opt-F-P2A-Spe-IgSP-Apa- CD20-vHH-2HCD25-G4Sx3v2-hu-mROO5- 1-vH-IgG1-CH1-CD3zECDTMCP-opt2-F- F2A-PAC | 5029 | 15719 |
| CD1 9 | | CD 20 | | | CD8SP-hu-mROO5-1-vL-[hTCRbECD- CD3zECDTMCP-opt]-F-P2A-IgSP-Apa- CD20-vHH-2HCD25-G4Sx3v2-hu-mROO5- 1-vH-[hTCRaECD-CD3zECDTMCP-opt2] | 5030 | 15720 |
| CD1 9 | | CD 20 | | | CD8SP-hu-mROO5-1-vL-[hTCRb-KAC- ECD-CD3zECDTMCP-opt]-F-P2A-IgSP- Apa-CD20-vHH-2HCD25-G4Sx3v2-hu- mROO5-1-vH-[hTCRa-CSDVP-ECD- CD3zECDTMCP-opt2] | 5031 | 15721 |
| CD1 9 | | CD 20 | | | CD8SP-hu-mROO5-1-vL-V5-[hTCRbECD- CD3zECDTMCP-opt]-F-P2A-IgSP-Apa- CD20-vHH-2HCD25-G4Sx3v2-hu-mROO5- 1-vH-Myc-[hTCRaECD-CD3zECDTM-28z- opt2] | 5032 | 15722 |
| CD1 9 | | CD 20 | | | CD8SP-hu-mROO5-1-vL-V5-[hTCRbECD- CD3zECDTM-28z-opt]-F-P2A-IgSP-Apa- CD20-vHH-2HCD25-G4Sx3v2-hu-mROO5- 1-vH-Myc-[hTCRaECD-CD3zECDTM-28z- opt2] | 5033 | 15723 |
| CD1 9 | | CD 20 | | | CD8SP-hu-mROO5-1-vL-V5-[hTCRbECD- CD3zECDTMCP-opt]-F-P2A-IgSP-Apa- CD20-vHH-2HCD25-G4Sx3v2-hu-mROO5- 1-vH-Myc4-[hTCRaECD-CD3zECDTM- BBz-opt2] | 5034 | 15724 |
| CD1 9 | | CD 20 | | | CD8SP-hu-mROO5-1-vL-V5-[hTCRbECD- CD3zECDTM-BBz-opt]-F-P2A-IgSP-Apa- CD20-vHH-2HCD25-G4Sx3v2-hu-mROO5- 1-vH-Myc4-[hTCRaECD-CD3zECDTM- BBz-opt2] | 5035 | 15725 |
| CD1 9 | | CD 20 | | | CD8SP-hu-mROO5-1-vL-CD3zECDTMCP- opt-F-P2A-Spe-IgSP-Apa-CD20-vHH- 2HCD25-G4Sx3v2-hu-mROO5-1-vH-Mlu- CD3zECDTMCP-opt2-F-F2A-PAC | 5036 | 15726 |
| CD1 9 | | CD 20 | | | CD8SP-hu-mROO5-1-vL-[IgCL-TCRg- 6MD]-F-P2A-IgSP-Apa-CD20-vHH- 2HCD25-G4Sx3v2-hu-mROO5-1-vH-[IgG1- CH1-TCRd-6MD] | 5037 | 15727 |
| CD1 9 | | CD 20 | | | CD8SP-hu-mROO5-1-vL-[IgCL-TCRb- IAH-6MD]-F-P2A-IgSP-Apa-CD20-vHH- 2HCD25-G4Sx3v2-hu-mROO5-1-vH-[IgG1- CH1-TCRa-SDVP-6MD] | 5038 | 15728 |
| CD1 9 | | CD 20 | | | CD8SP-hu-mROO5-1-vL-[IgCL-TCRb-wt- opt2-6MD]-F-P2A-IgSP-Apa-CD20-vHH- 2HCD25-G4Sx3v2-hu-mROO5-1-vH-[IgG1- CH1-TCRa-wt-op2-6MD] | 5039 | 15729 |
| CD1 9 | | CD 20 | | | IgSP-Apa-CD20-vHH-2HCD25-G4Sx3v2- hu-mROO5-1-(vH-vL)-G3S-CD3e- ECDTMCP-opt2 | 5040 | 15730 |
| CD1 9 | | CD 20 | | | IgSP-Apa-CD20-vHH-2HCD25-G4Sx3v2- hu-mROO5-1-(vH-vL)-G3S-CD3d- ECDTMCP-opt2 | 5041 | 15731 |
| CD1 9 | | CD 20 | | | IgSP-Apa-CD20-vHH-2HCD25-G4Sx3v2- hu-mROO5-1-(vH-vL)-G3S-CD3g- ECDTMCP-opt2 | 5042 | 15732 |
| CD1 9 | | CD 20 | | | IgSP-Apa-CD20-vHH-2HCD25-G4Sx3v2- hu-mROO5-1-(vH-vL)-G3S-CD3z- ECDTMCP-opt2 | 5043 | 15733 |
| CD1 9 | | CD 20 | | | IgSP-Apa-CD20-vHH-2HCD25-G4Sx3v2- hu-mROO5-1-vH-[hTCRa-opt2]-F-F2A- BlastR | 5044 | 15734 |
| CD1 9 | | CD 20 | | | IgSP-Apa-CD20-vHH-2HCD25-G4Sx3v2- hu-mROO5-1-vH-[hTCRa-opt2] | 5045 | 15735 |
| CD1 9 | | CD 20 | | | IgSP-Apa-CD20-vHH-2HCD25-G4Sx3v2- hu-mROO5-1-vH-Myc-[hTCRa-T48C-opt]- F-F2A-BlastR | 5046 | 15736 |
| CD1 9 | | CD 20 | | | IgSP-Apa-CD20-vHH-2HCD25-G4Sx3v2- hu-mROO5-1-vH-Myc-[hTCRa-T48C-opt] | 5047 | 15737 |
| CD1 9 | CD1 9 | CD 20 | | | CD8SP-CD19-vHH-048-G4Sx3-R1-hu- mROO5-1-vL-[hTCRa-CSDVP]-F-F2A- IgSP-Apa-CD20-vHH-2HCD25-G4Sx3v2- hu-mROO5-1-vH-[hTCRb-KACIAH]-F- P2A-PAC | 5048 | 15738 |
| CD1 9 | CD1 9 | CD 20 | | | CD8SP-CD19-vHH-048-G4Sx3-R1-hu- mROO5-1-vL-[hTCRb-KACIAH]-F-P2A- IgSP-Apa-CD20-vHH-2HCD25-G4Sx3v2- hu-mROO5-1-vH-[hTCRa-CSDVP]-F-F2A- PAC | 5049 | 15739 |
| CD1 9 | CD1 9 | CD 20 | | | CD8SP-CD19-vHH-048-G4Sx3-R1-hu- mROO5-1-vL-[hTCRb-S57C]-F-P2A-IgSP- Apa-CD20-vHH-2HCD25-G4Sx3v2-hu- mROO5-1-vH-[hTCRa-T48C]-F-F2A-PAC | 5050 | 15740 |
| CD1 9 | CD1 9 | CD 20 | | | CD8SP-CD19-vHH-048-G4Sx3-R1-hu- mROO5-1-vL-[hTCRb-S57C]-F-P2A-IgSP- Apa-CD20-vHH-2HCD25-G4Sx3v2-hu- mROO5-1-vH-[hTCRa-T48C] | 5051 | 15741 |
| CD1 9 | CD1 9 | CD 20 | | | CD8SP-CD19-vHH-048-G4Sx3-R1-hu- mROO5-1-vL-[hTCRb-S57C]-F-P2A-IgSP- Apa-CD20-vHH-2HCD25-G4Sx3v2-hu- mROO5-1-vH-[hTCRa-T48C]-F-F2A-K13- opt | 5052 | 15742 |
| CD1 9 | CD1 9 | CD 20 | | | CD8SP-CD19-vHH-048-G4Sx3-R1-hu- mROO5-1-vL-[hTCRa-T48C]-F-P2A-IgSP- Apa-CD20-vHH-2HCD25-G4Sx3v2-hu- mROO5-1-vH-[hTCRb-S57C] | 5053 | 15743 |
| CD1 9 | CD1 9 | CD 20 | | | CD8SP-CD19-vHH-048-G4Sx3-R1-hu- mROO5-1-vL-[hTCRa-T48C]-F-P2A-IgSP- Apa-CD20-vHH-2HCD25-G4Sx3v2-hu- mROO5-1-vH-[hTCRb-S57C]-F-P2A-K13- opt | 5054 | 15744 |
| CD1 9 | CD1 9 | CD 20 | | | CD8SP-CD19-vHH-048-G4Sx3-R1-hu- mROO5-1-vL-[hTCRb-opt2]-F-P2A-IgSP- Apa-CD20-vHH-2HCD25-G4Sx3v2-hu- mROO5-1-vH-[hTCRa-opt2]-F-F2A-PAC | 5055 | 15745 |
| CD1 9 | CD1 9 | CD 20 | | | CD8SP-CD19-vHH-048-G4Sx3-R1-hu- mROO5-1-vL-[hTCRb-opt2]-F-P2A-IgSP- Apa-CD20-vHH-2HCD25-G4Sx3v2-hu- mROO5-1-vH-Myc-[pr*etc.*Ra-Del48]-F- F2A-PAC | 5056 | 15746 |
| CD1 9 | CD1 9 | CD 20 | | | CD8SP-CD19-vHH-048-G4Sx3-R1-hu- mROO5-1-vL-V5-[hTCRg1-opt]-F-P2A- IgSP-Apa-CD20-vHH-2HCD25-G4Sx3v2- hu-mROO5-1-vH-Myc-[hTCRd-opt]-F-F2A- PAC | 5057 | 15747 |
| CD1 9 | CD1 9 | CD 20 | | | CD8SP-CD19-vHH-048-G4Sx3-R1-hu- mROO5-1-vL-[hTCRd-opt]-F-P2A-IgSP- Apa-CD20-vHH-2HCD25-G4Sx3v2-hu- mROO5-1-vH-[hTCRg1-opt] | 5058 | 15748 |
| CD1 9 | CD1 9 | CD 20 | | | CD8SP-CD19-vHH-048-G4Sx3-R1-hu- mROO5-1-vL-IgCL-CD3zECDTMCP-opt- F-P2A-Spe-IgSP-Apa-CD20-vHH-2HCD25- G4Sx3v2-hu-mROO5-1-vH-IgG1-CH1- CD3zECDTMCP-opt2-F-F2A-PAC | 5059 | 15749 |
| CD1 9 | CD1 9 | CD 20 | | | CD8SP-CD19-vHH-048-G4Sx3-R1-hu- mROO5-1-vL-[hTCRbECD- CD3zECDTMCP-opt]-F-P2A-IgSP-Apa- CD20-vHH-2HCD25-G4Sx3v2-hu-mROO5- 1-vH-[hTCRaECD-CD3zECDTMCP-opt2] | 5060 | 15750 |
| CD1 9 | CD1 9 | CD 20 | | | CD8SP-CD19-vHH-048-G4Sx3-R1-hu- mROO5-1-vL-[hTCRb-KAC-ECD- CD3zECDTMCP-opt]-F-P2A-IgSP-Apa- CD20-vHH-2HCD25-G4Sx3v2-hu-mROO5- 1-vH-[hTCRa-CSDVP-ECD- CD3zECDTMCP-opt2] | 5061 | 15751 |
| CD1 9 | CD1 9 | CD 20 | | | CD8SP-CD19-vHH-048-G4Sx3-R1-hu- mROO5-1-vL-V5-[hTCRbECD- CD3zECDTMCP-opt]-F-P2A-IgSP-Apa- CD20-vHH-2HCD25-G4Sx3v2-hu-mROO5- 1-vH-Myc-[hTCRaECD-CD3zECDTM-28z- opt2] | 5062 | 15752 |
| CD1 9 | CD1 9 | CD 20 | | | CD8SP-CD19-vHH-048-G4Sx3-R1-hu- mROO5-1-vL-V5-[hTCRbECD- CD3zECDTM-28z-opt]-F-P2A-IgSP-Apa- CD20-vHH-2HCD25-G4Sx3v2-hu-mROO5- 1-vH-Myc-[hTCRaECD-CD3zECDTM-28z- opt2] | 5063 | 15753 |
| CD1 9 | CD1 9 | CD 20 | | | CD8SP-CD19-vHH-048-G4Sx3-R1-hu- mROO5-1-vL-V5-[hTCRbECD- CD3zECDTMCP-opt]-F-P2A-IgSP-Apa- CD20-vHH-2HCD25-G4Sx3v2-hu-mROO5- 1-vH-Myc4-[hTCRaECD-CD3zECDTM- BBz-opt2] | 5064 | 15754 |
| CD1 9 | CD1 9 | CD 20 | | | CD8SP-CD19-vHH-048-G4Sx3-R1-hu- mROO5-1-vL-V5-[hTCRbECD- CD3zECDTM-BBz-opt]-F-P2A-IgSP-Apa- CD20-vHH-2HCD25-G4Sx3v2-hu-mROO5- 1-vH-Myc4-[hTCRaECD-CD3zECDTM- BBz-opt2] | 5065 | 15755 |
| CD1 9 | CD1 9 | CD 20 | | | CD8SP-CD19-vHH-048-G4Sx3-R1-hu- mROO5-1-vL-CD3zECDTMCP-opt-F-P2A- Spe-IgSP-Apa-CD20-vHH-2HCD25- G4Sx3v2-hu-mROO5-1-vH-Mlu- CD3zECDTMCP-opt2-F-F2A-PAC | 5066 | 15756 |
| CD1 9 | CD1 9 | CD 20 | | | CD8SP-CD19-vHH-048-G4Sx3-R1-hu- mROO5-1-vL-[IgCL-TCRg-6MD]-F-P2A- IgSP-Apa-CD20-vHH-2HCD25-G4Sx3v2- hu-mROO5-1-vH-[IgG1-CH1-TCRd-6MD] | 5067 | 15757 |
| CD1 9 | CD1 9 | CD 20 | | | CD8SP-CD19-vHH-048-G4Sx3-R1-hu- mROO5-1-vL-[IgCL-TCRb-IAH-6MD]-F- P2A-IgSP-Apa-CD20-vHH-2HCD25- G4Sx3v2-hu-mROO5-1-vH-[IgG1-CH1- TCRa-SDVP-6MD] | 5068 | 15758 |
| CD1 9 | CD1 9 | CD 20 | | | CD8SP-CD19-vHH-048-G4Sx3-R1-hu- mROO5-1-vL-[IgCL-TCRb-wt-opt2-6MD]- F-P2A-IgSP-Apa-CD20-vHH-2HCD25- G4Sx3v2-hu-mROO5-1-vH-[IgG1-CH1- TCRa-wt-op2-6MD] | 5069 | 15759 |
| CD1 9 | CD1 9 | | BC MA | | CD8SP-BCMA-FHVH-33-G3Sx2-CD19- vHH-048-G4Sx3-R1-hu-mROO5-1-vL-Gly- Ser-Linker-hu-mROO5-1-vH-Myc-28z | 5070 | 15760 |
| CD1 9 | CD1 9 | | BC MA | | CD8SP-BCMA-FHVH-33-G3Sx2-CD19- vHH-048-G4Sx3-R1-hu-mROO5-1-vL-Gly- Ser-Linker-hu-mROO5-1-vH-Myc-CD8TM- BBz | 5071 | 15761 |
| CD1 9 | CD1 9 | | BC MA | | CD8SP-BCMA-FHVH-33-G3Sx2-CD19- vHH-048-G4Sx3-R1-hu-mROO5-1-vL-Gly- Ser-Linker-hu-mROO5-1-vH-Myc-CD8TM- z-P2A-K13-FLAG-T2A-PAC | 5072 | 15762 |
| CD1 9 | CD1 9 | | BC MA | | CD8SP-BCMA-FHVH-33-G3Sx2-CD19- vHH-048-G4Sx3-R1-hu-mROO5-1-vL- [hTCRa-CSDVP]-F-F2A-SP-hu-mROO5-1- vH-[hTCRb-KACIAH]-F-P2A-PAC | 5073 | 15763 |
| CD1 9 | CD1 9 | | BC MA | | CD8SP-BCMA-FHVH-33-G3Sx2-CD19- vHH-048-G4Sx3-R1-hu-mROO5-1-vL- [hTCRb-KACIAH]-F-P2A-SP-hu-mROO5- 1-vH-[hTCRa-CSDVP]-F-F2A-PAC | 5074 | 15764 |
| CD1 9 | CD1 9 | | BC MA | | CD8SP-BCMA-FHVH-33-G3Sx2-CD19- vHH-048-G4Sx3-R1-hu-mROO5-1-vL- [hTCRb-S57C]-F-P2A-SP-hu-mROO5-1- vH-[hTCRa-T48C] | 5075 | 15765 |
| CD1 9 | CD1 9 | | BC MA | | CD8SP-BCMA-FHVH-33-G3Sx2-CD19- vHH-048-G4Sx3-R1-hu-mROO5-1-vL- [hTCRb-S57C]-F-P2A-SP-hu-mROO5-1- vH-[hTCRa-T48C]-F-F2A-K13-opt | 5076 | 15766 |
| CD1 9 | CD1 9 | | BC MA | | CD8SP-BCMA-FHVH-33-G3Sx2-CD19- vHH-048-G4Sx3-R1-hu-mROO5-1-vL- [hTCRa-T48C]-F-P2A-SP-hu-mROO5-1- vH-[hTCRb-S57C] | 5077 | 15767 |
| CD1 9 | CD1 9 | | BC MA | | CD8SP-BCMA-FHVH-33-G3Sx2-CD19- vHH-048-G4Sx3-R1-hu-mROO5-1-vL- [hTCRa-T48C]-F-P2A-SP-hu-mROO5-1- vH-[hTCRb-S57C]-F-P2A-K13-opt | 5078 | 15768 |
| CD1 9 | CD1 9 | | BC MA | | CD8SP-V5-[hTCRb-KACIAH]-F-P2A- CD8SP-BCMA-FHVH-33-G3Sx2-CD19- vHH-048-G4Sx3-R1-hu-mROO5-1-vL-Gly- Ser-Linker-hu-mROO5-1-vH-Myc-[hTCRa- CSDVP]-F-F2A-PAC | 5079 | 15769 |
| CD1 9 | CD1 9 | | BC MA | | CD8SP-MYC-[hTCRa-T48C-opt1]-F-F2A- SP-BCMA-FHVH-33-G3Sx2-CD19-vHH- 048-G4Sx3-R1-hu-mROO5-1-vL-Gly-Ser- Linker-hu-mROO5-1-vH-V5-[hTCRb-S57C- opt1]-F-P2A-PAC | 5080 | 15770 |
| CD1 9 | CD1 9 | | BC MA | | CD8SP-BCMA-FHVH-33-G3Sx2-CD19- vHH-048-G4Sx3-R1-hu-mROO5-1-vL- [hTCRb-opt2]-F-P2A-SP-hu-mROO5-1-vH- [hTCRa-opt2]-F-F2A-PAC | 5081 | 15771 |
| CD1 9 | CD1 9 | | BC MA | | CD8SP-BCMA-FHVH-33-G3Sx2-CD19- vHH-048-G4Sx3-R1-hu-mROO5-1-vL- [hTCRb-opt2]-F-P2A-SP-hu-mROO5-1-vH- Myc-[pretc.Ra-Del48]-F-F2A-PAC | 5082 | 15772 |
| CD1 9 | CD1 9 | | BC MA | | CD8SP-[hTCRb-opt2]-F-P2A-CD8SP- BCMA-FHVH-33-G3Sx2-CD19-vHH-048- G4Sx3-R1-hu-mROO5-1-vL-Gly-Ser- Linker-hu-mROO5-1-vH-Myc4-[pr*etc.*Ra- Del48]-F-F2A-PAC | 5083 | 15773 |
| CD1 9 | CD1 9 | | BC MA | | CD8SP-BCMA-FHVH-33-G3Sx2-CD19- vHH-048-G4Sx3-R1-hu-mROO5-1-vL-V5- [hTCRg1-opt]-F-P2A-SP-hu-mROO5-1-vH- Myc-[hTCRd-opt]-F-F2A-PAC | 5084 | 15774 |
| CD1 9 | CD1 9 | | BC MA | | CD8SP-BCMA-FHVH-33-G3Sx2-CD19- vHH-048-G4Sx3-R1-hu-mROO5-1-vL- | 5085 | 15775 |
| | | | | | [hTCRd-opt]-F-P2A-SP-hu-mROO5-1-vH- [hTCRg1-opt] | | |
| CD1 9 | CD1 9 | | BC MA | | CD8SP-V5-[hTCRg1-opt]-F-P2A-CD8SP- BCMA-FHVH-33-G3Sx2-CD19-vHH-048- G4Sx3-R1-hu-mROO5-1-vL-Gly-Ser- Linker-hu-mROO5-1-vH-Myc-[hTCRd-opt]- F-F2A-PAC | 5086 | 15776 |
| CD1 9 | CD1 9 | | BC MA | | CD8SP-BCMA-FHVH-33-G3Sx2-CD19- vHH-048-G4Sx3-R1-hu-mROO5-1-vL- IgCL-CD3zECDTMCP-opt-F-P2A-Spe-SP- hu-mROO5-1-vH-IgG1-CH1- CD3zECDTMCP-opt2-F-F2A-PAC | 5087 | 15777 |
| CD1 9 | CD1 9 | | BC MA | | CD8SP-BCMA-FHVH-33-G3Sx2-CD19- vHH-048-G4Sx3-R1-hu-mROO5-1-vL- [hTCRbECD-CD3zECDTMCP-opt]-F-P2A- SP-hu-mROO5-1-vH-[hTCRaECD- CD3zECDTMCP-opt2] | 5088 | 15778 |
| CD1 9 | CD1 9 | | BC MA | | CD8SP-BCMA-FHVH-33-G3Sx2-CD19- vHH-048-G4Sx3-R1-hu-mROO5-1-vL- [hTCRb-KAC-ECD-CD3zECDTMCP-opt]- F-P2A-SP-hu-mROO5-1-vH-[hTCRa- CSDVP-ECD-CD3zECDTMCP-opt2] | 5089 | 15779 |
| CD1 9 | CD1 9 | | BC MA | | CD8SP-BCMA-FHVH-33-G3Sx2-CD19- vHH-048-G4Sx3-R1-hu-mROO5-1-vL-V5- [hTCRbECD-CD3zECDTMCP-opt]-F-P2A- SP-hu-mROO5-1-vH-Myc-[hTCRaECD- CD3zECDTM-28z-opt2] | 5090 | 15780 |
| CD1 9 | CD1 9 | | BC MA | | CD8SP-BCMA-FHVH-33-G3Sx2-CD19- vHH-048-G4Sx3-R1-hu-mROO5-1-vL-V5- [hTCRbECD-CD3zECDTM-28z-opt]-F- P2A-SP-hu-mROO5-1-vH-Myc- [hTCRaECD-CD3zECDTM-28z-opt2] | 5091 | 15781 |
| CD1 9 | CD1 9 | | BC MA | | CD8SP-BCMA-FHVH-33-G3Sx2-CD19- vHH-048-G4Sx3-R1-hu-mROO5-1-vL-V5- [hTCRbECD-CD3zECDTMCP-opt]-F-P2A- SP-hu-mROO5-1-vH-Myc4-[hTCRaECD- CD3zECDTM-BBz-opt2] | 5092 | 15782 |
| CD1 9 | CD1 9 | | BC MA | | CD8SP-BCMA-FHVH-33-G3Sx2-CD19- vHH-048-G4Sx3-R1-hu-mROO5-1-vL-V5- [hTCRbECD-CD3zECDTM-BBz-opt]-F- P2A-SP-hu-mROO5-1-vH-Myc4- [hTCRaECD-CD3zECDTM-BBz-opt2] | 5093 | 15783 |
| CD1 9 | CD1 9 | | BC MA | | CD8SP-BCMA-FHVH-33-G3Sx2-CD19- vHH-048-G4Sx3-R1-hu-mROO5-1-vL- CD3zECDTMCP-opt-F-P2A-Spe-SP-hu- mROO5-1-vH-Mlu-CD3zECDTMCP-opt2- F-F2A-PAC | 5094 | 15784 |
| CD1 9 | CD1 9 | | BC MA | | CD8SP-BCMA-FHVH-33-G3Sx2-CD19- vHH-048-G4Sx3-R1-hu-mROO5-1-vL- [IgCL-TCRg-6MD]-F-P2A-SP-hu-mROO5- 1-vH-[IgG1-CH1-TCRd-6MD] | 5095 | 15785 |
| CD1 9 | CD1 9 | | BC MA | | CD8SP-BCMA-FHVH-33-G3Sx2-CD19- vHH-048-G4Sx3-R1-hu-mROO5-1-vL- [IgCL-TCRb-IAH-6MD]-F-P2A-SP-hu- mROO5-1-vH-[IgG1-CH1-TCRa-SDVP- 6MD] | 5096 | 15786 |
| CD1 9 | CD1 9 | | BC MA | | CD8SP-BCMA-FHVH-33-G3Sx2-CD19- vHH-048-G4Sx3-R1-hu-mROO5-1-vL- [IgCL-TCRb-wt-opt2-6MD]-F-P2A-SP-hu- mROO5-1-vH-[IgG1-CH1-TCRa-wt-op2- 6MD] | 5097 | 15787 |
| CD1 9 | CD1 9 | | BC MA | | CD8SP-BCMA-FHVH-33-G3Sx2-CD19- vHH-048-G4Sx3-R1-hu-mROO5-1-(vL- vH)-CD3e-ECDTMCP-opt2 | 5098 | 15788 |
| CD1 9 | CD1 9 | | BC MA | | CD8SP-BCMA-FHVH-33-G3Sx2-CD19- vHH-048-G4Sx3-R1-hu-mROO5-1-(vL- vH)-CD3d-ECDTMCP-opt2 | 5099 | 15789 |
| CD1 9 | CD1 9 | | BC MA | | CD8SP-BCMA-FHVH-33-G3Sx2-CD19- vHH-048-G4Sx3-R1-hu-mROO5-1-(vL- vH)-CD3g-ECDTMCP-opt2 | 5100 | 15790 |
| CD1 9 | CD1 9 | | BC MA | | CD8SP-BCMA-FHVH-33-G3Sx2-CD19- vHH-048-G4Sx3-R1-hu-mROO5-1-(vL- vH)-CD3z-ECDTMCP-opt2 | 5101 | 15791 |
| CD1 9 | CD1 9 | | BC MA | | CD8SP-BCMA-FHVH-33-G3Sx2-CD19- vHH-048-G4Sx3-R1-hu-mROO5-1-vL-Gly- Ser-Linker-hu-mROO5-1-vH-[hTCRa-opt2]- F-F2A-PAC | 5102 | 15792 |
| CD1 9 | CD1 9 | | BC MA | | CD8SP-BCMA-FHVH-33-G3Sx2-CD19- vHH-048-G4Sx3-R1-hu-mROO5-1-vL-Gly- Ser-Linker-hu-mROO5-1-vH-[hTCRb-opt2]- F-F2A-PAC | 5103 | 15793 |
| CD1 9 | CD1 9 | | BC MA | | CD8SP-BCMA-FHVH-33-G3Sx2-CD19- vHH-048-G4Sx3-R1-hu-mROO5-1-vL-Gly- Ser-Linker-hu-mROO5-1-vH-Myc4- [pretc.Ra-Del48]-F-F2A-PAC | 5104 | 15794 |
| CD1 9 | CD1 9 | | BC MA | | CD8SP-BCMA-FHVH-33-G3Sx2-CD19- vHH-048-G4Sx3-R1-hu-mROO5-1-vL- [hTCRb-opt2]-F-P2A-PAC | 5105 | 15795 |
| CD1 9 | CD1 9 | | BC MA | | CD8SP-BCMA-FHVH-33-G3Sx2-CD19- vHH-048-G4Sx3-R1-hu-mROO5-1-vL- [hTCRb-opt2] | 5106 | 15796 |
| CD1 9 | CD1 9 | | BC MA | | CD8SP-BCMA-FHVH-33-G3Sx2-CD19- vHH-048-G4Sx3-R1-hu-mROO5-1-vL-V5- [hTCRb-S57C-opt]-F-P2A-PAC | 5107 | 15797 |
| CD1 9 | CD1 9 | | BC MA | | CD8SP-BCMA-FHVH-33-G3Sx2-CD19- vHH-048-G4Sx3-R1-hu-mROO5-1-vL-V5- [hTCRb-S57C-opt] | 5108 | 15798 |
| CD1 9 | CD1 9 | | BC MA | | CD8SP-BCMA-FHVH-33-G3Sx2-CD19- vHH-048-G4Sx3-R1-hu-mROO5-1-vL-Gly- Ser-Linker-hu-mROO5-1-vH-[hTCRa- SDVP]-F-F2A-PAC | 5109 | 15799 |
| CD1 9 | CD1 9 | | BC MA | | CD8SP-BCMA-FHVH-33-G3Sx2-CD19- vHH-048-G4Sx3-R1-hu-mROO5-1-vL-Gly- Ser-Linker-hu-mROO5-1-vH-[hTCRb- KAIAH]-F-P2A-PAC | 5110 | 15800 |
| CD1 9 | CD1 9 | | BC MA | | CD8SP-BCMA-FHVH-33-G3Sx2-CD19- vHH-048-G4Sx3-R1-hu-mROO5-1-vL- [hTCRa-CSDVP]-F-F2A-IgSP-Apa-CD20- vHH-2HCD25-G4Sx3v2-hu-mROO5-1-vH- [hTCRb-KACIAH]-F-P2A-PAC | 5111 | 15801 |
| CD1 9 | CD1 9 | | BC MA | | CD8SP-BCMA-FHVH-33-G3Sx2-CD19- vHH-048-G4Sx3-R1-hu-mROO5-1-vL- [hTCRb-KACIAH]-F-P2A-IgSP-Apa-CD20- vHH-2HCD25-G4Sx3v2-hu-mROO5-1-vH- [hTCRa-CSDVP]-F-F2A-PAC | 5112 | 15802 |
| CD1 9 | CD1 9 | | BC MA | | CD8SP-BCMA-FHVH-33-G3Sx2-CD19- vHH-048-G4Sx3-R1-hu-mROO5-1-vL- [hTCRb-S57C]-F-P2A-IgSP-Apa-CD20- vHH-2HCD25-G4Sx3v2-hu-mROO5-1-vH- [hTCRa-T48C]-F-F2A-PAC | 5113 | 15803 |
| CD1 9 | CD1 9 | | BC MA | | CD8SP-BCMA-FHVH-33-G3Sx2-CD19- vHH-048-G4Sx3-R1-hu-mROO5-1-vL- [hTCRb-S57C]-F-P2A-IgSP-Apa-CD20- vHH-2HCD25-G4Sx3v2-hu-mROO5-1-vH- [hTCRa-T48C] | 5114 | 15804 |
| CD1 9 | CD1 9 | | BC MA | | CD8SP-BCMA-FHVH-33-G3Sx2-CD19- vHH-048-G4Sx3-R1-hu-mROO5-1-vL- [hTCRb-S57C]-F-P2A-IgSP-Apa-CD20- vHH-2HCD25-G4Sx3v2-hu-mROO5-1-vH- [hTCRa-T48C]-F-F2A-K13-opt | 5115 | 15805 |
| CD1 9 | CD1 9 | | BC MA | | CD8SP-BCMA-FHVH-33-G3Sx2-CD19- vHH-048-G4Sx3-R1-hu-mROO5-1-vL- [hTCRa-T48C]-F-P2A-IgSP-Apa-CD20- vHH-2HCD25-G4Sx3v2-hu-mROO5-1-vH- [hTCRb-S57C] | 5116 | 15806 |
| CD1 9 | CD1 9 | | BC MA | | CD8SP-BCMA-FHVH-33-G3Sx2-CD19- vHH-048-G4Sx3-R1-hu-mROO5-1-vL- [hTCRa-T48C]-F-P2A-IgSP-Apa-CD20- vHH-2HCD25-G4Sx3v2-hu-mROO5-1-vH- [hTCRb-S57C]-F-P2A-K13-opt | 5117 | 15807 |
| CD1 9 | CD1 9 | | BC MA | | CD8SP-BCMA-FHVH-33-G3Sx2-CD19- vHH-048-G4Sx3-R1-hu-mROO5-1-vL- [hTCRb-opt2]-F-P2A-IgSP-Apa-CD20- vHH-2HCD25-G4Sx3v2-hu-mROO5-1-vH- [hTCRa-opt2]-F-F2A-PAC | 5118 | 15808 |
| CD1 9 | CD1 9 | | BC MA | | CD8SP-BCMA-FHVH-33-G3Sx2-CD19- vHH-048-G4Sx3-R1-hu-mROO5-1-vL- [hTCRb-opt2]-F-P2A-IgSP-Apa-CD20- vHH-2HCD25-G4Sx3v2-hu-mROO5-1-vH- Myc-[pretc.Ra-Del48]-F-F2A-PAC | 5119 | 15809 |
| CD1 9 | CD1 9 | | BC MA | | CD8SP-BCMA-FHVH-33-G3Sx2-CD19- vHH-048-G4Sx3-R1-hu-mROO5-1-vL-V5- [hTCRg1-opt]-F-P2A-IgSP-Apa-CD20- vHH-2HCD25-G4Sx3v2-hu-mROO5-1-vH- Myc-[hTCRd-opt]-F-F2A-PAC | 5120 | 15810 |
| CD1 9 | CD1 9 | | BC MA | | CD8SP-BCMA-FHVH-33-G3Sx2-CD19- vHH-048-G4Sx3-R1-hu-mROO5-1-vL- [hTCRd-opt]-F-P2A-IgSP-Apa-CD20-vHH- 2HCD25-G4Sx3v2-hu-mROO5-1-vH- [hTCRg1-opt] | 5121 | 15811 |
| CD1 9 | CD1 9 | | BC MA | | CD8SP-BCMA-FHVH-33-G3Sx2-CD19- vHH-048-G4Sx3-R1-hu-mROO5-1-vL- IgCL-CD3zECDTMCP-opt-F-P2A-Spe- IgSP-Apa-CD20-vHH-2HCD25-G4Sx3v2- hu-mROO5-1-vH-IgG1-CH1- CD3zECDTMCP-opt2-F-F2A-PAC | 5122 | 15812 |
| CD1 9 | CD1 9 | | BC MA | | CD8SP-BCMA-FHVH-33-G3Sx2-CD19- vHH-048-G4Sx3-R1-hu-mROO5-1-vL- [hTCRbECD-CD3zECDTMCP-opt]-F-P2A- IgSP-Apa-CD20-vHH-2HCD25-G4Sx3v2- hu-mROO5-1-vH-[hTCRaECD- CD3zECDTMCP-opt2] | 5123 | 15813 |
| CD1 9 | CD1 9 | | BC MA | | CD8SP-BCMA-FHVH-33-G3Sx2-CD19- vHH-048-G4Sx3-R1-hu-mROO5-1-vL- [hTCRb-KAC-ECD-CD3zECDTMCP-opt]- F-P2A-IgSP-Apa-CD20-vHH-2HCD25- G4Sx3v2-hu-mROO5-1-vH-[hTCRa- CSDVP-ECD-CD3zECDTMCP-opt2] | 5124 | 15814 |
| CD1 9 | CD1 9 | | BC MA | | CD8SP-BCMA-FHVH-33-G3Sx2-CD19- vHH-048-G4Sx3-R1-hu-mROO5-1-vL-V5- [hTCRbECD-CD3zECDTMCP-opt]-F-P2A- IgSP-Apa-CD20-vHH-2HCD25-G4Sx3v2- hu-mROO5-1-vH-Myc-[hTCRaECD- CD3zECDTM-28z-opt2] | 5125 | 15815 |
| CD1 9 | CD1 9 | | BC MA | | CD8SP-BCMA-FHVH-33-G3Sx2-CD19- vHH-048-G4Sx3-R1-hu-mROO5-1-vL-V5- [hTCRbECD-CD3zECDTM-28z-opt]-F- P2A-IgSP-Apa-CD20-vHH-2HCD25- G4Sx3v2-hu-mROO5-1-vH-Myc- [hTCRaECD-CD3zECDTM-28z-opt2] | 5126 | 15816 |
| CD1 9 | CD1 9 | | BC MA | | CD8SP-BCMA-FHVH-33-G3Sx2-CD19- vHH-048-G4Sx3-R1-hu-mROO5-1-vL-V5- [hTCRbECD-CD3zECDTMCP-opt]-F-P2A- IgSP-Apa-CD20-vHH-2HCD25-G4Sx3v2- hu-mROO5-1-vH-Myc4-[hTCRaECD- CD3zECDTM-BBz-opt2] | 5127 | 15817 |
| CD1 9 | CD1 9 | | BC MA | | CD8SP-BCMA-FHVH-33-G3Sx2-CD19- vHH-048-G4Sx3-R1-hu-mROO5-1-vL-V5- [hTCRbECD-CD3zECDTM-BBz-opt]-F- P2A-IgSP-Apa-CD20-vHH-2HCD25- G4Sx3v2-hu-mROO5-1-vH-Myc4- [hTCRaECD-CD3zECDTM-BBz-opt2] | 5128 | 15818 |
| CD1 9 | CD1 9 | | BC MA | | CD8SP-BCMA-FHVH-33-G3Sx2-CD19- vHH-048-G4Sx3-R1-hu-mROO5-1-vL- CD3zECDTMCP-opt-F-P2A-Spe-IgSP-Apa- CD20-vHH-2HCD25-G4Sx3v2-hu-mROO5- 1-vH-Mlu-CD3zECDTMCP-opt2-F-F2A- PAC | 5129 | 15819 |
| CD1 9 | CD1 9 | | BC MA | | CD8SP-BCMA-FHVH-33-G3Sx2-CD19- vHH-048-G4Sx3-R1-hu-mROO5-1-vL- [IgCL-TCRg-6MD]-F-P2A-IgSP-Apa- CD20-vHH-2HCD25-G4Sx3v2-hu-mROO5- 1-vH-[IgG1-CH1-TCRd-6MD] | 5130 | 15820 |
| CD1 9 | CD1 9 | | BC MA | | CD8SP-BCMA-FHVH-33-G3Sx2-CD19- vHH-048-G4Sx3-R1-hu-mROO5-1-vL- [IgCL-TCRb-IAH-6MD]-F-P2A-IgSP-Apa- CD20-vHH-2HCD25-G4Sx3v2-hu-mROO5- 1-vH-[IgG1-CH1-TCRa-SDVP-6MD] | 5131 | 15821 |
| CD1 9 | CD1 9 | | BC MA | | CD8SP-BCMA-FHVH-33-G3Sx2-CD19- vHH-048-G4Sx3-R1-hu-mROO5-1-vL- [IgCL-TCRb-wt-opt2-6MD]-F-P2A-IgSP- Apa-CD20-vHH-2HCD25-G4Sx3v2-hu- mROO5-1-vH-[IgG1-CH1-TCRa-wt-op2- 6MD] | 5132 | 15822 |
| | CD1 9 | CD 20 | BC MA | | CD8SP-BCMA-FHVH-33-G3Sx2-CD19- vHH-048-[IgCL-TCRb-wt-opt2-6MD]-F- P2A-IgSP-Apa-CD20-2HCD25-VHH- [IgG1-CH1-TCRa-wt-opt2-6MD]-F-F2A- PAC | 5133 | 15823 |
| | CD1 9 | CD 20 | BC MA | | CD8SP-BCMA-FHVH-33-G3Sx2-CD19- vHH-048-[IgCL-TCRb-wt-opt2-6MD]-F- P2A-IgSP-Apa-CD20-2HCD25-VHH- [IgG1-CH1-TCRa-wt-opt2-6MD] | 5134 | 15824 |
| | CD1 9 | CD 20 | BC MA | | CD8SP-BCMA-FHVH-33-G3Sx2-CD19- vHH-048-GS-[TCRb-wt-opt2-6MD]-F-P2A- IgSP-Apa-CD20-2HCD25-VHH-GS-[TCRa- wt-opt2-6MD]-F-F2A-PAC | 5135 | 15825 |
| | CD1 9 | CD 20 | BC MA | | CD8SP-BCMA-FHVH-33-G3Sx2-CD19- vHH-048-GS-[TCRb-wt-opt2-6MD]-F-P2A- IgSP-Apa-CD20-2HCD25-VHH-GS-[TCRa- wt-opt2-6MD] | 5136 | 15826 |
| | CD1 9 | CD 20 | BC MA | | CD8SP-BCMA-FHVH-33-G3Sx2-CD19- vHH-048-IgG1-Hinge-[TCRb-wt-opt2- 6MD]-F-P2A-IgSP-Apa-CD20-2HCD25- VHH-GS-[TCRa-wt-opt2-6MD]-F-F2A- PAC | 5137 | 15827 |
| | CD1 9 | CD 20 | BC MA | | CD8SP-BCMA-FHVH-33-G3Sx2-CD19- vHH-048-GS-[TCRb-wt-opt2-6MD]-F-P2A- IgSP-Apa-CD20-2HCD25-VHH-IgG1- Hinge-v2-[TCRa-wt-opt2-6MD]-F-F2A- PAC | 5138 | 15828 |
| | CD1 9 | CD 20 | BC MA | | CD8SP-BCMA-FHVH-33-G3Sx2-CD19- vHH-048-IgG1-Hinge-[TCRb-wt-opt2- 6MD]-F-P2A-IgSP-Apa-CD20-2HCD25- VHH-IgG1-Hinge-v2-[TCRa-wt-opt2- 6MD]-F-F2A-PAC | 5139 | 15829 |
| | CD1 9 | CD 20 | BC MA | | CD8SP-BCMA-FHVH-33-G3Sx2-CD19- vHH-048-IgG1-Hinge-[TCRb-wt-opt2- 6MD]-F-P2A-IgSP-Apa-CD20-2HCD25- VHH-IgG1-Hinge-v2-[TCRa-wt-opt2-6MD] | 5140 | 15830 |
| | CD1 9 | CD 20 | BC MA | | CD8SP-BCMA-FHVH-33-G3Sx2-CD19- vHH-048-GS-[TCRb-wt-opt2-6MD]-F-P2A- IgSP-Apa-CD20-2HCD25-VHH-IgG1- Hinge-v2-[TCRa-wt-opt2-6MD] | 5141 | 15831 |
| | CD1 9 | CD 20 | BC MA | | CD8SP-BCMA-FHVH-33-G3Sx2-CD19- vHH-048-[IgCL-TCRg-6MD]-F-P2A-IgSP- Apa-CD20-2HCD25-VHH-[IgG1-CH1- TCRd-6MD]-F-F2A-PAC | 5142 | 15832 |
| | CD1 9 | CD 20 | BC MA | | CD8SP-BCMA-FHVH-33-G3Sx2-CD19- vHH-048-[IgCL-TCRg-6MD]-F-P2A-IgSP- Apa-CD20-2HCD25-VHH-[IgG1-CH1- TCRd-6MD] | 5143 | 15833 |
| | CD1 9 | CD 20 | BC MA | | CD8SP-BCMA-FHVH-33-G3Sx2-CD19- vHH-048-GS-[TCRg-6MD]-F-P2A-IgSP- Apa-CD20-2HCD25-VHH-GS-[TCRd- 6MD]-F-F2A-PAC | 5144 | 15834 |
| | CD1 9 | CD 20 | BC MA | | CD8SP-BCMA-FHVH-33-G3Sx2-CD19- vHH-048-GS-[TCRg-6MD]-F-P2A-IgSP- Apa-CD20-2HCD25-VHH-GS-[TCRd- 6MD] | 5145 | 15835 |
| | CD1 9 | CD 20 | BC MA | | CD8SP-BCMA-FHVH-33-G3Sx2-CD19- vHH-048-IgG1-Hinge-[TCRg-6MD]-F-P2A- IgSP-Apa-CD20-2HCD25-VHH-GS-[TCRd- 6MD]-F-F2A-PAC | 5146 | 15836 |
| | CD1 9 | CD 20 | BC MA | | CD8SP-BCMA-FHVH-33-G3Sx2-CD19- vHH-048-GS-[TCRg-6MD]-F-P2A-IgSP- Apa-CD20-2HCD25-VHH-IgG1-Hinge-v2- [TCRd-6MD]-F-F2A-PAC | 5147 | 15837 |
| | CD1 9 | CD 20 | BC MA | | CD8SP-BCMA-FHVH-33-G3Sx2-CD19- vHH-048-IgG1-Hinge-[TCRg-6MD]-F-P2A- IgSP-Apa-CD20-2HCD25-VHH-IgG1- Hinge-v2-[TCRd-6MD]-F-F2A-PAC | 5148 | 15838 |
| | CD1 9 | CD 20 | BC MA | | CD8SP-BCMA-FHVH-33-G3Sx2-CD19- vHH-048-IgG1-Hinge-[TCRg-6MD]-F-P2A- IgSP-Apa-CD20-2HCD25-VHH-IgG1- Hinge-v2-[TCRd-6MD] | 5149 | 15839 |
| | CD1 9 | CD 20 | BC MA | | CD8SP-BCMA-FHVH-33-G3Sx2-CD19- vHH-048-GS-[TCRg-6MD]-F-P2A-IgSP- Apa-CD20-2HCD25-VHH-IgG1-Hinge-v2- [TCRd-6MD] | 5150 | 15840 |
| | CD1 9 | CD 20 | BC MA | | CD8SP-BCMA-FHVH-33-G3Sx2-CD19- vHH-048-GS-[TCRg-7MD]-F-P2A-IgSP- Apa-CD20-2HCD25-VHH-GS-[TCRd- 6MD]-F-F2A-PAC | 5151 | 15841 |
| | CD1 9 | CD 20 | BC MA | | CD8SP-BCMA-FHVH-33-G3Sx2-CD19- vHH-048-GS-[TCRg-6MD]-F-P2A-IgSP- | 5152 | 15842 |
| | | | | | Apa-CD20-2HCD25-VHH-GS-[TCRd- 7MD]-F-F2A-PAC | | |
| | CD1 9 | | BC MA | | CD8SP-BCMA-FHVH-33-G3Sx2-CD19- vHH-048-[IgCL-TCRb-6MD] | 5153 | 15843 |
| | CD1 9 | | BC MA | | CD8SP-BCMA-FHVH-33-G3Sx2-CD19- vHH-048-[IgCL-TCRg-6MD] | 5154 | 15844 |
| CD1 9 | CD2 2 | CD 20 | | | IgSP-CD22-FHVH-158-G4Sx2-CD20-vHH- 2HCD25-G4Sx3v2-hu-mROO5-1-vH-Gly- Ser-Linker-vL-Myc-CD8TM-BBz | 5155 | 15845 |
| CD1 9 | CD2 2 | CD 20 | | | CD8SP-hu-mROO5-1-vL-[hTCRa-CSDVP]- F-F2A-IgSP-CD22-FHVH-158-G4Sx2- CD20-vHH-2HCD25-G4Sx3v2-hu-mROO5- 1-vH-[hTCRb-KACIAH]-F-P2A-PAC | 5156 | 15846 |
| CD1 9 | CD2 2 | CD 20 | | | CD8SP-hu-mROO5-1-vL-[hTCRb- KACIAH]-F-P2A-IgSP-CD22-FHVH-158- G4Sx2-CD20-vHH-2HCD25-G4Sx3v2-hu- mROO5-1-vH-[hTCRa-CSDVP]-F-F2A- PAC | 5157 | 15847 |
| CD1 9 | CD2 2 | CD 20 | | | CD8SP-hu-mROO5-1-vL-[hTCRb-S57C]-F- P2A-IgSP-CD22-FHVH-158-G4Sx2-CD20- vHH-2HCD25-G4Sx3v2-hu-mROO5-1-vH- [hTCRa-T48C]-F-F2A-PAC | 5158 | 15848 |
| CD1 9 | CD2 2 | CD 20 | | | CD8SP-hu-mROO5-1-vL-[hTCRb-S57C]-F- P2A-IgSP-CD22-FHVH-158-G4Sx2-CD20- vHH-2HCD25-G4Sx3v2-hu-mROO5-1-vH- [hTCRa-T48C] | 5159 | 15849 |
| CD1 9 | CD2 2 | CD 20 | | | CD8SP-hu-mROO5-1-vL-[hTCRb-S57C]-F- P2A-IgSP-CD22-FHVH-158-G4Sx2-CD20- vHH-2HCD25-G4Sx3v2-hu-mROO5-1-vH- [hTCRa-T48C]-F-F2A-K13-opt | 5160 | 15850 |
| CD1 9 | CD2 2 | CD 20 | | | CD8SP-hu-mROO5-1-vL-[hTCRa-T48C]-F- P2A-IgSP-CD22-FHVH-158-G4Sx2-CD20- vHH-2HCD25-G4Sx3v2-hu-mROO5-1-vH- [hTCRb-S57C] | 5161 | 15851 |
| CD1 9 | CD2 2 | CD 20 | | | CD8SP-hu-mROO5-1-vL-[hTCRa-T48C]-F- P2A-IgSP-CD22-FHVH-158-G4Sx2-CD20- vHH-2HCD25-G4Sx3v2-hu-mROO5-1-vH- [hTCRb-S57C]-F-P2A-K13-opt | 5162 | 15852 |
| CD1 9 | CD2 2 | CD 20 | | | CD8SP-hu-mROO5-1-vL-[hTCRb-opt2]-F- P2A-IgSP-CD22-FHVH-158-G4Sx2-CD20- vHH-2HCD25-G4Sx3v2-hu-mROO5-1-vH- [hTCRa-opt2]-F-F2A-PAC | 5163 | 15853 |
| CD1 9 | CD2 2 | CD 20 | | | CD8SP-hu-mROO5-1-vL-[hTCRb-opt2]-F- P2A-IgSP-CD22-FHVH-158-G4Sx2-CD20- vHH-2HCD25-G4Sx3v2-hu-mROO5-1-vH- Myc-[pretc.Ra-Del48]-F-F2A-PAC | 5164 | 15854 |
| CD1 9 | CD2 2 | CD 20 | | | CD8SP-hu-mROO5-1-vL-V5-[hTCRg1- opt]-F-P2A-IgSP-CD22-FHVH-158-G4Sx2- CD20-vHH-2HCD25-G4Sx3v2-hu-mROO5- 1-vH-Myc-[hTCRd-opt]-F-F2A-PAC | 5165 | 15855 |
| CD1 9 | CD2 2 | CD 20 | | | CD8SP-hu-mROO5-1-vL-[hTCRd-opt]-F- P2A-IgSP-CD22-FHVH-158-G4Sx2-CD20- vHH-2HCD25-G4Sx3v2-hu-mROO5-1-vH- [hTCRg1-opt] | 5166 | 15856 |
| CD1 9 | CD2 2 | CD 20 | | | CD8SP-hu-mROO5-1-vL-IgCL- CD3zECDTMCP-opt-F-P2A-Spe-IgSP- CD22-FHVH-158-G4Sx2-CD20-vHH- 2HCD25-G4Sx3v2-hu-mROO5-1-vH-IgG1- CH1-CD3zECDTMCP-opt2-F-F2A-PAC | 5167 | 15857 |
| CD1 9 | CD2 2 | CD 20 | | | CD8SP-hu-mROO5-1-vL-[hTCRbECD- CD3zECDTMCP-opt]-F-P2A-IgSP-CD22- FHVH-158-G4Sx2-CD20-vHH-2HCD25- G4Sx3v2-hu-mROO5-1-vH-[hTCRaECD- CD3zECDTMCP-opt2] | 5168 | 15858 |
| CD1 9 | CD2 2 | CD 20 | | | CD8SP-hu-mROO5-1-vL-[hTCRb-KAC- ECD-CD3zECDTMCP-opt]-F-P2A-IgSP- CD22-FHVH-158-G4Sx2-CD20-vHH- 2HCD25-G4Sx3v2-hu-mROO5-1-vH- [hTCRa-CSDVP-ECD-CD3zECDTMCP- opt2] | 5169 | 15859 |
| CD1 9 | CD2 2 | CD 20 | | | CD8SP-hu-mROO5-1-vL-V5-[hTCRbECD- CD3zECDTMCP-opt]-F-P2A-IgSP-CD22- FHVH-158-G4Sx2-CD20-vHH-2HCD25- G4Sx3v2-hu-mROO5-1-vH-Myc- [hTCRaECD-CD3zECDTM-28z-opt2] | 5170 | 15860 |
| CD1 9 | CD2 2 | CD 20 | | | CD8SP-hu-mROO5-1-vL-V5-[hTCRbECD- CD3zECDTM-28z-opt]-F-P2A-IgSP-CD22- FHVH-158-G4Sx2-CD20-vHH-2HCD25- G4Sx3v2-hu-mROO5-1-vH-Myc- [hTCRaECD-CD3zECDTM-28z-opt2] | 5171 | 15861 |
| CD1 9 | CD2 2 | CD 20 | | | CD8SP-hu-mROO5-1-vL-V5-[hTCRbECD- CD3zECDTMCP-opt]-F-P2A-IgSP-CD22- FHVH-158-G4Sx2-CD20-vHH-2HCD25- G4Sx3v2-hu-mROO5-1-vH-Myc4- [hTCRaECD-CD3zECDTM-BBz-opt2] | 5172 | 15862 |
| CD1 9 | CD2 2 | CD 20 | | | CD8SP-hu-mROO5-1-vL-V5-[hTCRbECD- CD3zECDTM-BBz-opt]-F-P2A-IgSP-CD22- FHVH-158-G4Sx2-CD20-vHH-2HCD25- G4Sx3v2-hu-mROO5-1-vH-Myc4- [hTCRaECD-CD3zECDTM-BBz-opt2] | 5173 | 15863 |
| CD1 9 | CD2 2 | CD 20 | | | CD8SP-hu-mROO5-1-vL-CD3zECDTMCP- opt-F-P2A-Spe-IgSP-CD22-FHVH-158- G4Sx2-CD20-vHH-2HCD25-G4Sx3v2-hu- mROO5-1-vH-Mlu-CD3zECDTMCP-opt2- F-F2A-PAC | 5174 | 15864 |
| CD1 9 | CD2 2 | CD 20 | | | CD8SP-hu-mROO5-1-vL-[IgCL-TCRg- 6MD]-F-P2A-IgSP-CD22-FHVH-158- G4Sx2-CD20-vHH-2HCD25-G4Sx3v2-hu- mROO5-1-vH-[IgG1-CH1-TCRd-6MD] | 5175 | 15865 |
| CD1 9 | CD2 2 | CD 20 | | | CD8SP-hu-mROO5-1-vL-[IgCL-TCRb- IAH-6MD]-F-P2A-IgSP-CD22-FHVH-158- G4Sx2-CD20-vHH-2HCD25-G4Sx3v2-hu- mROO5-1-vH-[IgG1-CH1-TCRa-SDVP- 6MD] | 5176 | 15866 |
| CD1 9 | CD2 2 | CD 20 | | | CD8SP-hu-mROO5-1-vL-[IgCL-TCRb-wt- opt2-6MD]-F-P2A-IgSP-CD22-FHVH-158- G4Sx2-CD20-vHH-2HCD25-G4Sx3v2-hu- mROO5-1-vH-[IgG1-CH1-TCRa-wt-op2- 6MD] | 5177 | 15867 |
| CD1 9 | CD2 2 | CD 20 | | | IgSP-CD22-FHVH-158-G4Sx2-CD20-vHH- 2HCD25-G4Sx3v2-hu-mROO5-1-(vH-vL)- G3S-CD3e-ECDTMCP-opt2 | 5178 | 15868 |
| CD1 9 | CD2 2 | CD 20 | | | IgSP-CD22-FHVH-158-G4Sx2-CD20-vHH- 2HCD25-G4Sx3v2-hu-mROO5-1-(vH-vL)- G3S-CD3d-ECDTMCP-opt2 | 5179 | 15869 |
| CD1 9 | CD2 2 | CD 20 | | | IgSP-CD22-FHVH-158-G4Sx2-CD20-vHH- 2HCD25-G4Sx3v2-hu-mROO5-1-(vH-vL)- G3S-CD3g-ECDTMCP-opt2 | 5180 | 15870 |
| CD1 9 | CD2 2 | CD 20 | | | IgSP-CD22-FHVH-158-G4Sx2-CD20-vHH- 2HCD25-G4Sx3v2-hu-mROO5-1-(vH-vL)- G3S-CD3z-ECDTMCP-opt2 | 5181 | 15871 |
| CD1 9 | CD2 2 | CD 20 | | | IgSP-CD22-FHVH-158-G4Sx2-CD20-vHH- 2HCD25-G4Sx3v2-hu-mROO5-1-vH- [hTCRa-opt2]-F-F2A-BlastR | 5182 | 15872 |
| CD1 9 | CD2 2 | CD 20 | | | IgSP-CD22-FHVH-158-G4Sx2-CD20-vHH- 2HCD25-G4Sx3v2-hu-mROO5-1-vH- [hTCRa-opt2] | 5183 | 15873 |
| CD1 9 | CD2 2 | CD 20 | | | IgSP-CD22-FHVH-158-G4Sx2-CD20-vHH- 2HCD25-G4Sx3v2-hu-mROO5-1-vH-Myc- [hTCRa-T48C-opt]-F-F2A-BlastR | 5184 | 15874 |
| CD1 9 | CD2 2 | CD 20 | | | IgSP-CD22-FHVH-158-G4Sx2-CD20-vHH- 2HCD25-G4Sx3v2-hu-mROO5-1-vH-Myc- [hTCRa-T48C-opt] | 5185 | 15875 |
| CD1 9 | CD1 9 | CD 22 | CD 20 | BC MA | CD8SP-BCMA-FHVH-33-G3Sx2-CD19- vHH-048-G4Sx3-R1-hu-mROO5-1-vL- [hTCRa-CSDVP]-F-F2A-IgSP-CD22- FHVH-158-G4Sx2-CD20-vHH-2HCD25- G4Sx3v2-hu-mROO5-1-vH-[hTCRb- KACIAH]-F-P2A-PAC | 5186 | 15876 |
| CD1 9 | CD1 9 | CD 22 | CD 20 | BC MA | CD8SP-BCMA-FHVH-33-G3Sx2-CD19- vHH-048-G4Sx3-R1-hu-mROO5-1-vL- [hTCRb-KACIAH]-F-P2A-IgSP-CD22- FHVH-158-G4Sx2-CD20-vHH-2HCD25- G4Sx3v2-hu-mROO5-1-vH-[hTCRa- CSDVP]-F-F2A-PAC | 5187 | 15877 |
| CD1 9 | CD1 9 | CD 22 | CD 20 | BC MA | CD8SP-BCMA-FHVH-33-G3Sx2-CD19- vHH-048-G4Sx3-R1-hu-mROO5-1-vL- [hTCRb-S57C]-F-P2A-IgSP-CD22-FHVH- 158-G4Sx2-CD20-vHH-2HCD25-G4Sx3v2- hu-mROO5-1-vH-[hTCRa-T48C]-F-F2A- PAC | 5188 | 15878 |
| CD1 9 | CD1 9 | CD 22 | CD 20 | BC MA | CD8SP-BCMA-FHVH-33-G3Sx2-CD19- vHH-048-G4Sx3-R1-hu-mROO5-1-vL- [hTCRb-S57C]-F-P2A-IgSP-CD22-FHVH- 158-G4Sx2-CD20-vHH-2HCD25-G4Sx3v2- hu-mROO5-1-vH-[hTCRa-T48C] | 5189 | 15879 |
| CD1 9 | CD1 9 | CD 22 | CD 20 | BC MA | CD8SP-BCMA-FHVH-33-G3Sx2-CD19- vHH-048-G4Sx3-R1-hu-mROO5-1-vL- [hTCRb-S57C]-F-P2A-IgSP-CD22-FHVH- 158-G4Sx2-CD20-vHH-2HCD25-G4Sx3v2- hu-mROO5-1-vH-[hTCRa-T48C]-F-F2A- K13-opt | 5190 | 15880 |
| CD1 9 | CD1 9 | CD 22 | CD 20 | BC MA | CD8SP-BCMA-FHVH-33-G3Sx2-CD19- vHH-048-G4Sx3-R1-hu-mROO5-1-vL- [hTCRa-T48C]-F-P2A-IgSP-CD22-FHVH- 158-G4Sx2-CD20-vHH-2HCD25-G4Sx3v2- hu-mROO5-1-vH-[hTCRb-S57C] | 5191 | 15881 |
| CD1 9 | CD1 9 | CD 22 | CD 20 | BC MA | CD8SP-BCMA-FHVH-33-G3Sx2-CD19- vHH-048-G4Sx3-R1-hu-mROO5-1-vL- [hTCRa-T48C]-F-P2A-IgSP-CD22-FHVH- 158-G4Sx2-CD20-vHH-2HCD25-G4Sx3v2- hu-mROO5-1-vH-[hTCRb-S57C]-F-P2A- K13-opt | 5192 | 15882 |
| CD1 9 | CD1 9 | CD 22 | CD 20 | BC MA | CD8SP-BCMA-FHVH-33-G3Sx2-CD19- vHH-048-G4Sx3-R1-hu-mROO5-1-vL- [hTCRb-opt2]-F-P2A-IgSP-CD22-FHVH- 158-G4Sx2-CD20-vHH-2HCD25-G4Sx3v2- | 5193 | 15883 |
| | | | | | hu-mROO5-1-vH-[hTCRa-opt2]-F-F2A- PAC | | |
| CD1 9 | CD1 9 | CD 22 | CD 20 | BC MA | CD8SP-BCMA-FHVH-33-G3Sx2-CD19- vHH-048-G4Sx3-R1-hu-mROO5-1-vL- [hTCRb-opt2]-F-P2A-IgSP-CD22-FHVH- 158-G4Sx2-CD20-vHH-2HCD25-G4Sx3v2- hu-mROO5-1-vH-Myc-[pretc.Ra-Del48]-F- F2A-PAC | 5194 | 15884 |
| CD1 9 | CD1 9 | CD 22 | CD 20 | BC MA | CD8SP-BCMA-FHVH-33-G3Sx2-CD19- vHH-048-G4Sx3-R1-hu-mROO5-1-vL-V5- [hTCRg1-opt]-F-P2A-IgSP-CD22-FHVH- 158-G4Sx2-CD20-vHH-2HCD25-G4Sx3v2- hu-mROO5-1-vH-Myc-[hTCRd-opt]-F-F2A- PAC | 5195 | 15885 |
| CD1 9 | CD1 9 | CD 22 | CD 20 | BC MA | CD8SP-BCMA-FHVH-33-G3Sx2-CD19- vHH-048-G4Sx3-R1-hu-mROO5-1-vL- [hTCRd-opt]-F-P2A-IgSP-CD22-FHVH- 158-G4Sx2-CD20-vHH-2HCD25-G4Sx3v2- hu-mROO5-1-vH-[hTCRg1-opt] | 5196 | 15886 |
| CD1 9 | CD1 9 | CD 22 | CD 20 | BC MA | CD8SP-BCMA-FHVH-33-G3Sx2-CD19- vHH-048-G4Sx3-R1-hu-mROO5-1-vL- IgCL-CD3zECDTMCP-opt-F-P2A-Spe- IgSP-CD22-FHVH-158-G4Sx2-CD20-vHH- 2HCD25-G4Sx3v2-hu-mROO5-1-vH-IgG1- CH1-CD3zECDTMCP-opt2-F-F2A-PAC | 5197 | 15887 |
| CD1 9 | CD1 9 | CD 22 | CD 20 | BC MA | CD8SP-BCMA-FHVH-33-G3Sx2-CD19- vHH-048-G4Sx3-R1-hu-mROO5-1-vL- [hTCRbECD-CD3zECDTMCP-opt]-F-P2A- IgSP-CD22-FHVH-158-G4Sx2-CD20-vHH- 2HCD25-G4Sx3v2-hu-mROO5-1-vH- [hTCRaECD-CD3zECDTMCP-opt2] | 5198 | 15888 |
| CD1 9 | CD1 9 | CD 22 | CD 20 | BC MA | CD8SP-BCMA-FHVH-33-G3Sx2-CD19- vHH-048-G4Sx3-R1-hu-mROO5-1-vL- [hTCRb-KAC-ECD-CD3zECDTMCP-opt]- F-P2A-IgSP-CD22-FHVH-158-G4Sx2- CD20-vHH-2HCD25-G4Sx3v2-hu-mROO5- 1-vH-[hTCRa-CSDVP-ECD- CD3zECDTMCP-opt2] | 5199 | 15889 |
| CD1 9 | CD1 9 | CD 22 | CD 20 | BC MA | CD8SP-BCMA-FHVH-33-G3Sx2-CD19- vHH-048-G4Sx3-R1-hu-mROO5-1-vL-V5- [hTCRbECD-CD3zECDTMCP-opt]-F-P2A- IgSP-CD22-FHVH-158-G4Sx2-CD20-vHH- 2HCD25-G4Sx3v2-hu-mROO5-1-vH-Myc- [hTCRaECD-CD3zECDTM-28z-opt2] | 5200 | 15890 |
| CD1 9 | CD1 9 | CD 22 | CD 20 | BC MA | CD8SP-BCMA-FHVH-33-G3Sx2-CD19- vHH-048-G4Sx3-R1-hu-mROO5-1-vL-V5- [hTCRbECD-CD3zECDTM-28z-opt]-F- P2A-IgSP-CD22-FHVH-158-G4Sx2-CD20- vHH-2HCD25-G4Sx3v2-hu-mROO5-1-vH- Myc-[hTCRaECD-CD3zECDTM-28z-opt2] | 5201 | 15891 |
| CD1 9 | CD1 9 | CD 22 | CD 20 | BC MA | CD8SP-BCMA-FHVH-33-G3Sx2-CD19- vHH-048-G4Sx3-R1-hu-mROO5-1-vL-V5- [hTCRbECD-CD3zECDTMCP-opt]-F-P2A- IgSP-CD22-FHVH-158-G4Sx2-CD20-vHH- 2HCD25-G4Sx3v2-hu-mROO5-1-vH-Myc4- [hTCRaECD-CD3zECDTM-BBz-opt2] | 5202 | 15892 |
| CD1 9 | CD1 9 | CD 22 | CD 20 | BC MA | CD8SP-BCMA-FHVH-33-G3Sx2-CD19- vHH-048-G4Sx3-R1-hu-mROO5-1-vL-V5- [hTCRbECD-CD3zECDTM-BBz-opt]-F- P2A-IgSP-CD22-FHVH-158-G4Sx2-CD20- vHH-2HCD25-G4Sx3v2-hu-mROO5-1-vH- Myc4-[hTCRaECD-CD3zECDTM-BBz- opt2] | 5203 | 15893 |
| CD1 9 | CD1 9 | CD 22 | CD 20 | BC MA | CD8SP-BCMA-FHVH-33-G3Sx2-CD19- vHH-048-G4Sx3-R1-hu-mROO5-1-vL- CD3zECDTMCP-opt-F-P2A-Spe-IgSP- CD22-FHVH-158-G4Sx2-CD20-vHH- 2HCD25-G4Sx3v2-hu-mROO5-1-vH-Mlu- CD3zECDTMCP-opt2-F-F2A-PAC | 5204 | 15894 |
| CD1 9 | CD1 9 | CD 22 | CD 20 | BC MA | CD8SP-BCMA-FHVH-33-G3Sx2-CD19- vHH-048-G4Sx3-R1-hu-mROO5-1-vL- [IgCL-TCRg-6MD]-F-P2A-IgSP-CD22- FHVH-158-G4Sx2-CD20-vHH-2HCD25- G4Sx3v2-hu-mROO5-1-vH-[IgG1-CH1- TCRd-6MD] | 5205 | 15895 |
| CD1 9 | CD1 9 | CD 22 | CD 20 | BC MA | CD8SP-BCMA-FHVH-33-G3Sx2-CD19- vHH-048-G4Sx3-R1-hu-mROO5-1-vL- [IgCL-TCRb-IAH-6MD]-F-P2A-IgSP- CD22-FHVH-158-G4Sx2-CD20-vHH- 2HCD25-G4Sx3v2-hu-mROO5-1-vH-[IgG1- CH1-TCRa-SDVP-6MD] | 5206 | 15896 |
| CD1 9 | CD1 9 | CD 22 | CD 20 | BC MA | CD8SP-BCMA-FHVH-33-G3Sx2-CD19- vHH-048-G4Sx3-R1-hu-mROO5-1-vL- [IgCL-TCRb-wt-opt2-6MD]-F-P2A-IgSP- CD22-FHVH-158-G4Sx2-CD20-vHH- 2HCD25-G4Sx3v2-hu-mROO5-1-vH-[IgG1- CH1-TCRa-wt-op2-6MD] | 5207 | 15897 |
| | CD1 9 | CD 22 | CD 20 | BC MA | CD8SP-BCMA-FHVH-33-G3Sx2-CD19- vHH-048-[IgCL-TCRb-wt-opt2-6MD]-F- P2A-IgSP-CD22-FHVH-158-G4Sx2-CD20- 2HCD25-VHH-[IgG1-CH1-TCRa-wt-opt2- 6MD]-F-F2A-PAC | 5208 | 15898 |
| | CD1 9 | CD 22 | CD 20 | BC MA | CD8SP-BCMA-FHVH-33-G3Sx2-CD19- vHH-048-[IgCL-TCRb-wt-opt2-6MD]-F- P2A-IgSP-CD22-FHVH-158-G4Sx2-CD20- 2HCD25-VHH-[IgG1-CH1-TCRa-wt-opt2- 6MD] | 5209 | 15899 |
| | CD1 9 | CD 22 | CD 20 | BC MA | CD8SP-BCMA-FHVH-33-G3Sx2-CD19- vHH-048-GS-[TCRb-wt-opt2-6MD]-F-P2A- IgSP-CD22-FHVH-158-G4Sx2-CD20- 2HCD25-VHH-GS-[TCRa-wt-opt2-6MD]-F- F2A-PAC | 5210 | 15900 |
| | CD1 9 | CD 22 | CD 20 | BC MA | CD8SP-BCMA-FHVH-33-G3Sx2-CD19- vHH-048-GS-[TCRb-wt-opt2-6MD]-F-P2A- IgSP-CD22-FHVH-158-G4Sx2-CD20- 2HCD25-VHH-GS-[TCRa-wt-opt2-6MD] | 5211 | 15901 |
| | CD1 9 | CD 22 | CD 20 | BC MA | CD8SP-BCMA-FHVH-33-G3Sx2-CD19- vHH-048-IgG1-Hinge-[TCRb-wt-opt2- 6MD]-F-P2A-IgSP-CD22-FHVH-158- G4Sx2-CD20-2HCD25-VHH-GS-[TCRa- wt-opt2-6MD]-F-F2A-PAC | 5212 | 15902 |
| | CD1 9 | CD 22 | CD 20 | BC MA | CD8SP-BCMA-FHVH-33-G3Sx2-CD19- vHH-048-GS-[TCRb-wt-opt2-6MD]-F-P2A- IgSP-CD22-FHVH-158-G4Sx2-CD20- 2HCD25-VHH-IgG1-Hinge-v2-[TCRa-wt- opt2-6MD]-F-F2A-PAC | 5213 | 15903 |
| | CD1 9 | CD 22 | CD 20 | BC MA | CD8SP-BCMA-FHVH-33-G3Sx2-CD19- vHH-048-IgG1-Hinge-[TCRb-wt-opt2- 6MD]-F-P2A-IgSP-CD22-FHVH-158- G4Sx2-CD20-2HCD25-VHH-IgG1-Hinge- v2-[TCRa-wt-opt2-6MD]-F-F2A-PAC | 5214 | 15904 |
| | CD1 9 | CD 22 | CD 20 | BC MA | CD8SP-BCMA-FHVH-33-G3Sx2-CD19- vHH-048-IgG1-Hinge-[TCRb-wt-opt2- 6MD]-F-P2A-IgSP-CD22-FHVH-158- G4Sx2-CD20-2HCD25-VHH-IgG1-Hinge- v2-[TCRa-wt-opt2-6MD] | 5215 | 15905 |
| | CD1 9 | CD 22 | CD 20 | BC MA | CD8SP-BCMA-FHVH-33-G3Sx2-CD19- vHH-048-GS-[TCRb-wt-opt2-6MD]-F-P2A- IgSP-CD22-FHVH-158-G4Sx2-CD20- 2HCD25-VHH-IgG1-Hinge-v2-[TCRa-wt- opt2-6MD] | 5216 | 15906 |
| | CD1 9 | CD 22 | CD 20 | BC MA | CD8SP-BCMA-FHVH-33-G3Sx2-CD19- vHH-048-[IgCL-TCRg-6MD]-F-P2A-IgSP- CD22-FHVH-158-G4Sx2-CD20-2HCD25- VHH-[IgG1-CH1-TCRd-6MD]-F-F2A-PAC | 5217 | 15907 |
| | CD1 9 | CD 22 | CD 20 | BC MA | CD8SP-BCMA-FHVH-33-G3Sx2-CD19- vHH-048-[IgCL-TCRg-6MD]-F-P2A-IgSP- CD22-FHVH-158-G4Sx2-CD20-2HCD25- VHH-[IgG1-CH1-TCRd-6MD] | 5218 | 15908 |
| | CD1 9 | CD 22 | CD 20 | BC MA | CD8SP-BCMA-FHVH-33-G3Sx2-CD19- vHH-048-GS-[TCRg-6MD]-F-P2A-IgSP- CD22-FHVH-158-G4Sx2-CD20-2HCD25- VHH-GS-[TCRd-6MD]-F-F2A-PAC | 5219 | 15909 |
| | CD1 9 | CD 22 | CD 20 | BC MA | CD8SP-BCMA-FHVH-33-G3Sx2-CD19- vHH-048-GS-[TCRg-6MD]-F-P2A-IgSP- CD22-FHVH-158-G4Sx2-CD20-2HCD25- VHH-GS-[TCRd-6MD] | 5220 | 15910 |
| | CD1 9 | CD 22 | CD 20 | BC MA | CD8SP-BCMA-FHVH-33-G3Sx2-CD19- vHH-048-IgG1-Hinge-[TCRg-6MD]-F-P2A- IgSP-CD22-FHVH-158-G4Sx2-CD20- 2HCD25-VHH-IgG1-Hinge-v2-[TCRd- 6MD] | 5221 | 15911 |
| | | CD 22 | CD 20 | | IgSP-CD22-FHVH-158-G4Sx2-CD20- 2HCD25-VHH-[IgG1-CH1-TCRa-wt-opt2- 6MD] | 5222 | 15912 |
| | | CD 22 | CD 20 | | IgSP-CD22-FHVH-158-G4Sx2-CD20- 2HCD25-VHH-[IgG1-CH1-TCRd-6MD] | 5223 | 15913 |

**TABLE 32: Bispecifics and Trispecific Constructs on different backbones targeting different antigens. These constructs were generated by changing the AABD modules of the construct in Table 31 with the modules shown in the first construct of each series in the Table below. The order of the TCR constant chain fragments of the constructs of each series in Table 32 is the same as the order of TCR constant chain fragments of the constructs of series 1 whose details are provided in Table 31.**

| **TARGET** | | | | | | | |
|---|---|---|---|---|---|---|---|
| **1st** | **2nd** | **3rd** | **4th** | **5th** | **Name of first construct in the series** | **SEQ ID NO Series (DNA)** | **SEQ ID NO Series (PRT)** |
| CD1 9 | CD1 9 | CD2 0 | CD 22 | BC MA | CD8SP-CD19-vHH-048-G4Sx3-R1-hu- mROO5-1-vL-Gly-Ser-Linker-hu-mROO5- 1-vH-Myc-28z | 4976-5223 | 15666-15913 |
| CD1 9 | CD3 8 | CD2 0 | CD 22 | BC MA | CD8SP-CD38-FHVH-309021-G4Sx3-R1- hu-mROO5-1-vL-Gly-Ser-Linker-hu- mROO5-1-vH-Myc-28z | 5224-5471 | 15914-16161 |
| CD1 9 | CD1 23 | CD2 0 | CD 22 | BC MA | CD8SP-CD123-vHH-2-309021-G4Sx3-R1- hu-mROO5-1-vL-Gly-Ser-Linker-hu- mROO5-1-vH-Myc-28z | 5472-5719 | 16162-16409 |
| CD1 9 | Her2 | Her3 | CD 22 | EGF R | CD8SP-Her2-vHH-2D3-309021-G4Sx3- R1-hu-mROO5-1-vL-Gly-Ser-Linker-hu- mROO5-1-vH-Myc-28z | 5720-5967 | 16410-16657 |
| CD1 9 | Her2 | IL13 Ra2 | CD 22 | BC MA | CD8SP-Her2-vHH-2D3-309021-G4Sx3- R1-hu-mROO5-1-vL-Gly-Ser-Linker-hu- mROO5-1-vH-Myc-28z | 5968-6215 | 16658-16905 |
| CD1 9 | CD3 8 | CD2 0 | CD 22 | BC MA | CD8SP-CD38-USC1-FHVH-32184-G4Sx3- R1-hu-mROO5-1-vL-Gly-Ser-Linker-hu- mROO5-1-vH-Myc-28z | 6216-6463 | 16906-17153 |

**TABLE: 33: Bispecific SAR Constructs targeting CD22 and/or PSMA**

| **Name of fragment** | **SEQ ID NO (DNA)** | **SEQ ID NO (PRT)** |
|---|---|---|
| CD8SP-CD22-FHVH-24-SG4S-PSMA-chVH-71v2-Myc-CD8TM-BBz- T2A-PAC | 6464 | 17154 |
| CD8SP-CD22-FHVH-24-SG4S-PSMA-chVH-71v2-Myc-28z-T2A- PAC | 6465 | 17155 |
| CD8SP-CD22-FHVH-24-SG4S-PSMA-chVH-71v2-Myc-28z | 6466 | 17156 |
| CD8SP-CD22-FHVH-24-SG4S-PSMA-chVH-71v2-Myc-CD8TM-BBz | 6467 | 17157 |
| CD8SP-PSMA-chVH-71v2-SG4S-CD22-FHVH-24-Myc-CD8TM-BBz | 6468 | 17158 |
| CD8SP-CD22-FHVH-24-SG4S-PSMA-chVH-71v2-Myc-CD8TM-z- P2A-K13-FLAG-T2A-PAC | 6469 | 17159 |
| CD8SP-CD22-FHVH-24-[hTCRa-CSDVP]-F-F2A-SP-PSMA-chVH- 71v2-[hTCRb-KACIAH]-F-P2A-PAC | 6470 | 17160 |
| CD8SP-CD22-FHVH-24-[hTCRb-KACIAH]-F-P2A-SP-PSMA-chVH- 71v2-[hTCRa-CSDVP]-F-F2A-PAC | 6471 | 17161 |
| CD8SP-CD22-FHVH-24-[hTCRb-S57C]-F-P2A-SP-PSMA-chVH- 71v2-[hTCRa-T48C] | 6472 | 17162 |
| CD8SP-CD22-FHVH-24-[hTCRb-S57C]-F-P2A-SP-PSMA-chVH- 71v2-[hTCRa-T48C]-F-F2A-K13-opt | 6473 | 17163 |
| CD8SP-CD22-FHVH-24-[hTCRa-T48C]-F-P2A-SP-PSMA-chVH- 71v2-[hTCRb-S57C] | 6474 | 17164 |
| CD8SP-CD22-FHVH-24-[hTCRa-T48C]-F-P2A-SP-PSMA-chVH- 71v2-[hTCRb-S57C]-F-P2A-K13-opt | 6475 | 17165 |
| CD8SP-V5-[hTCRb-KACIAH]-F-P2A-CD8SP-CD22-FHVH-24-SG4S- PSMA-chVH-71v2-Myc-[hTCRa-CSDVP]-F-F2A-PAC | 6476 | 17166 |
| CD8SP-MYC-[hTCRa-T48C-opt1]-F-F2A-SP-CD22-FHVH-24-SG4S- PSMA-chVH-71v2-V5-[hTCRb-S57C-opt1]-F-P2A-PAC | 6477 | 17167 |
| CD8SP-CD22-FHVH-24-[hTCRb-opt2]-F-P2A-SP-PSMA-chVH-71v2- [hTCRa-opt2]-F-F2A-PAC | 6478 | 17168 |
| CD8SP-CD22-FHVH-24-[hTCRb-opt2]-F-P2A-SP-PSMA-chVH-71v2- Myc-[preTCRa-Del48]-F-F2A-PAC | 6479 | 17169 |
| CD8SP-[hTCRb-opt2]-F-P2A-CD8SP-CD22-FHVH-24-SG4S-PSMA- chVH-71v2-Myc4-[pretc.Ra-Del48]-F-F2A-PAC | 6480 | 17170 |
| CD8SP-CD22-FHVH-24-V5-[hTCRg1-opt]-F-P2A-SP-PSMA-chVH- 71v2-Myc-[hTCRd-opt]-F-F2A-PAC | 6481 | 17171 |
| CD8SP-CD22-FHVH-24-[hTCRd-opt]-F-P2A-SP-PSMA-chVH-71v2- [hTCRg1-opt] | 6482 | 17172 |
| CD8SP-V5-[hTCRg1-opt]-F-P2A-CD8SP-CD22-FHVH-24-SG4S- PSMA-chVH-71v2-Myc-[hTCRd-opt]-F-F2A-PAC | 6483 | 17173 |
| CD8-CD22-FHVH-24-IgCL-CD3zECDTMCP-opt-F-P2A-Spe-SP- PSMA-chVH-71v2-IgG1-CH1-CD3zECDTMCP-opt2-F-F2A-PAC | 6484 | 17174 |
| CD8SP-CD22-FHVH-24-[hTCRbECD-CD3zECDTMCP-opt]-F-P2A- SP-PSMA-chVH-71v2-[hTCRaECD-CD3zECDTMCP-opt2] | 6485 | 17175 |
| CD8SP-CD22-FHVH-24-[hTCRb-KAC-ECD-CD3zECDTMCP-opt]-F- P2A-SP-PSMA-chVH-71v2-[hTCRa-CSDVP-ECD-CD3zECDTMCP- opt2] | 6486 | 17176 |
| CD8SP-CD22-FHVH-24-V5-[hTCRbECD-CD3zECDTMCP-opt]-F- P2A-SP-PSMA-chVH-71v2-Myc-[hTCRaECD-CD3zECDTM-28z- opt2] | 6487 | 17177 |
| CD8SP-CD22-FHVH-24-V5-[hTCRbECD-CD3zECDTM-28z-opt]-F- P2A-SP-PSMA-chVH-71v2-Myc-[hTCRaECD-CD3zECDTM-28z- opt2] | 6488 | 17178 |
| CD8SP-CD22-FHVH-24-V5-[hTCRbECD-CD3zECDTMCP-opt]-F- P2A-SP-PSMA-chVH-71v2-Myc4-[hTCRaECD-CD3zECDTM-BBz- opt2] | 6489 | 17179 |
| CD8SP-CD22-FHVH-24-V5-[hTCRbECD-CD3zECDTM-BBz-opt]-F- P2A-SP-PSMA-chVH-71v2-Myc4-[hTCRaECD-CD3zECDTM-BBz- opt2] | 6490 | 17180 |
| CD8-CD22-FHVH-24-CD3zECDTMCP-opt-F-P2A-Spe-SP-PSMA- chVH-71v2-Mlu-CD3zECDTMCP-opt2-F-F2A-PAC | 6491 | 17181 |
| CD8SP-CD22-FHVH-24-[IgCL-TCRg-6MD]-F-P2A-SP-PSMA-chVH- 71v2-[IgG1-CH1-TCRd-6MD] | 6492 | 17182 |
| CD8SP-CD22-FHVH-24-[IgCL-TCRb-IAH-6MD]-F-P2A-SP-PSMA- chVH-71v2-[IgG1-CH1-TCRa-SDVP-6MD] | 6493 | 17183 |
| CD8SP-CD22-FHVH-24-[IgCL-TCRb-wt-opt2-6MD]-F-P2A-SP- PSMA-chVH-71v2-[IgG1-CH1-TCRa-wt-op2-6MD] | 6494 | 17184 |
| CD8SP-CD22-FHVH-24-SG4S-PSMA-chVH-71v2-CD3e-ECDTMCP- opt2 | 6495 | 17185 |
| CD8SP-CD22-FHVH-24-SG4S-PSMA-chVH-71v2-CD3d-ECDTMCP- opt2 | 6496 | 17186 |
| CD8SP-CD22-FHVH-24-SG4S-PSMA-chVH-71v2-CD3g-ECDTMCP- opt2 | 6497 | 17187 |
| CD8SP-CD22-FHVH-24-SG4S-PSMA-chVH-71v2-CD3z-ECDTMCP- opt2 | 6498 | 17188 |
| CD8SP-PSMA-chVH-71v2-SG4S-CD22-FHVH-24-CD3e-ECDTMCP- opt2 | 6499 | 17189 |
| CD8SP-PSMA-chVH-71v2-SG4S-CD22-FHVH-24-CD3d-ECDTMCP- opt2 | 6500 | 17190 |
| CD8SP-PSMA-chVH-71v2-SG4S-CD22-FHVH-24-CD3g-ECDTMCP- opt2 | 6501 | 17191 |
| CD8SP-PSMA-chVH-71v2-SG4S-CD22-FHVH-24-CD3z-ECDTMCP- opt2 | 6502 | 17192 |
| CD8SP-CD22-FHVH-24-SG4S-PSMA-chVH-71v2-[hTCRa-opt2]-F- F2A-PAC | 6503 | 17193 |
| CD8SP-CD22-FHVH-24-SG4S-PSMA-chVH-71v2-[hTCRb-opt2]-F- F2A-PAC | 6504 | 17194 |
| CD8SP-CD22-FHVH-24-SG4S-PSMA-chVH-71v2-Myc4-[pr*etc.*Ra- Del48]-F-F2A-PAC | 6505 | 17195 |
| CD8SP-CD22-FHVH-24-[hTCRb-opt2]-F-P2A-PAC | 6506 | 17196 |
| CD8SP-CD22-FHVH-24-[hTCRb-opt2] | 6507 | 17197 |
| IgHSP-PSMA-chVH-71v2-[hTCRa-opt2]-F-F2A-BlastR | 6508 | 17198 |
| IgHSP-PSMA-chVH-71v2-[hTCRa-opt2] | 6509 | 17199 |
| CD8SP-CD22-FHVH-24-V5-[hTCRb-S57C-opt]-F-P2A-PAC | 6510 | 17200 |
| CD8SP-CD22-FHVH-24-V5-[hTCRb-S57C-opt] | 6511 | 17201 |
| IgHSP-PSMA-chVH-71v2-Myc-[hTCRa-T48C-opt]-F-F2A-BlastR | 6512 | 17202 |
| IgHSP-PSMA-chVH-71v2-Myc-[hTCRa-T48C-opt] | 6513 | 17203 |
| CD8SP-CD22-FHVH-24-SG4S-PSMA-chVH-71v2-[hTCRa-SDVP]-F- F2A-PAC | 6514 | 17204 |
| CD8SP-CD22-FHVH-24-SG4S-PSMA-chVH-71v2-[hTCRb-KAIAH]- F-P2A-PAC | 6515 | 17205 |
| CD8SP-CD22-FHVH-24-SG4S-PSMA-chVH-71v2-G4S-CD3e- ECDTMCP-opt2-F-F2A-PAC | 6516 | 17206 |
| CD8SP-CD22-FHVH-24-SG4S-PSMA-chVH-71v2-G4S-CD3d- ECDTMCP-opt2-F-F2A-PAC | 6517 | 17207 |
| CD8SP-CD22-FHVH-24-SG4S-PSMA-chVH-71v2-G4S-CD3g- ECDTMCP-opt2-F-F2A-PAC | 6518 | 17208 |
| CD8SP-CD22-FHVH-24-SG4S-PSMA-chVH-71v2-G4S-CD3z- ECDTMCP-opt2-F-F2A-PAC | 6519 | 17209 |
| CD8SP-CD22-FHVH-24-[IgCL-TCRb-wt-opt2-6MD]-F-P2A-SP- PSMA-chVH-71v2-[IgG1-CH1-TCRa-wt-opt2-6MD]-F-F2A-PAC | 6520 | 17210 |
| CD8SP-CD22-FHVH-24-[IgCL-TCRb-wt-opt2-6MD]-F-P2A-SP- PSMA-chVH-71v2-[IgG1-CH1-TCRa-wt-opt2-6MD] | 6521 | 17211 |
| CD8SP-CD22-FHVH-24-GS-[TCRb-wt-opt2-6MD]-F-P2A-SP-PSMA- chVH-71v2-GS-[TCRa-wt-opt2-6MD]-F-F2A-PAC | 6522 | 17212 |
| CD8SP-CD22-FHVH-24-GS-[TCRb-wt-opt2-6MD]-F-P2A-SP-PSMA- chVH-71v2-GS-[TCRa-wt-opt2-6MD] | 6523 | 17213 |
| CD8SP-CD22-FHVH-24-IgG1-Hinge-[TCRb-wt-opt2-6MD]-F-P2A- SP-PSMA-chVH-71v2-GS-[TCRa-wt-opt2-6MD]-F-F2A-PAC | 6524 | 17214 |
| CD8SP-CD22-FHVH-24-GS-[TCRb-wt-opt2-6MD]-F-P2A-SP-PSMA- chVH-71v2-IgG1-Hinge-v2-[TCRa-wt-opt2-6MD]-F-F2A-PAC | 6525 | 17215 |
| CD8SP-CD22-FHVH-24-IgG1-Hinge-[TCRb-wt-opt2-6MD]-F-P2A- SP-PSMA-chVH-71v2-IgG1-Hinge-v2-[TCRa-wt-opt2-6MD]-F-F2A- PAC | 6526 | 17216 |
| CD8SP-CD22-FHVH-24-IgG1-Hinge-[TCRb-wt-opt2-6MD]-F-P2A- SP-PSMA-chVH-71v2-IgG1-Hinge-v2-[TCRa-wt-opt2-6MD] | 6527 | 17217 |
| CD8SP-CD22-FHVH-24-GS-[TCRb-wt-opt2-6MD]-F-P2A-SP-PSMA- chVH-71v2-IgG1-Hinge-v2-[TCRa-wt-opt2-6MD] | 6528 | 17218 |
| CD8SP-CD22-FHVH-24-[IgCL-TCRg-6MD]-F-P2A-SP-PSMA-chVH- 71v2-[IgG1-CH1-TCRd-6MD]-F-F2A-PAC | 6529 | 17219 |
| CD8SP-CD22-FHVH-24-[IgCL-TCRg-6MD]-F-P2A-SP-PSMA-chVH- 71v2-[IgG1-CH1-TCRd-6MD] | 6530 | 17220 |
| CD8SP-CD22-FHVH-24-GS-[TCRg-6MD]-F-P2A-SP-PSMA-chVH- 71v2-GS-[TCRd-6MD]-F-F2A-PAC | 6531 | 17221 |
| CD8SP-CD22-FHVH-24-GS-[TCRg-6MD]-F-P2A-SP-PSMA-chVH- 71v2-GS-[TCRd-6MD] | 6532 | 17222 |
| CD8SP-CD22-FHVH-24-IgG1-Hinge-[TCRg-6MD]-F-P2A-SP-PSMA- chVH-71v2-GS-[TCRd-6MD]-F-F2A-PAC | 6533 | 17223 |
| CD8SP-CD22-FHVH-24-GS-[TCRg-6MD]-F-P2A-SP-PSMA-chVH- 71v2-IgG1-Hinge-v2-[TCRd-6MD]-F-F2A-PAC | 6534 | 17224 |
| CD8SP-CD22-FHVH-24-IgG1-Hinge-[TCRg-6MD]-F-P2A-SP-PSMA- chVH-71v2-IgG1-Hinge-v2-[TCRd-6MD]-F-F2A-PAC | 6535 | 17225 |
| CD8SP-CD22-FHVH-24-IgG1-Hinge-[TCRg-6MD]-F-P2A-SP-PSMA- chVH-71v2-IgG1-Hinge-v2-[TCRd-6MD] | 6536 | 17226 |

**TABLE 34: Bispecifics and Trispecific Constructs on different backbones targeting different antigens. These constructs were generated by changing the AABD modules of the construct in Table 33 with the modules shown in the first construct of each series in the Table below. The order of the backbone (e.g., TCR constant chain fragments) of the constructs of each series in Table 34 is the same as the order of the backbones (e.g., TCR constant chain fragments) of the constructs of series 1 whose details are provided in Table 33.**

| **Ist target** | **2nd target** | **Name of first construct in the series** | **SEQ ID NO of Series (DNA)** | **SEQ ID NO of Series (PRT)** |
|---|---|---|---|---|
| CD22 | BCMA | CD8SP-CD22-FHVH-24-SG4S-PSMA-chVH- 71v2-Myc-CD8TM-BBz-T2A-PAC | 6464-6536 | 17154-17226 |
| CD22 | PSMA | CD8SP-CD22-FHVH-24-SG4S-PSMA-chVH-28- Myc-CD8TM-BBz-T2A-PAC | 6537-6609 | 17227-17299 |
| CD22 | CD19 | CD8SP-CD22-FHVH-24-SG4S-CD19-FHVH- 354-Myc-CD8TM-BBz-T2A-PAC | 6610-6682 | 17300-17372 |
| CD22 | BCMA | CD8SP-CD22-FHVH-24-SG4S-BCMA-FHVH- 74-Myc-CD8TM-BBz-T2A-PAC | 6683-6755 | 17373-17445 |
| CD22 | BCMA | CD8SP-CD22-FHVH-24-SG4S-BCMA-FHVH- 93-Myc-CD8TM-BBz-T2A-PAC | 6756-6828 | 17446-17518 |
| CD22 | CEA | CD8SP-CD22-FHVH-24-SG4S-CEA-aVH-3001- Myc-CD8TM-BBz-T2A-PAC | 6829-6901 | 17519-17591 |
| CEA | BCMA | CD8SP-CEA-aVH-300-SG4S-BCMA-FHVH-93- Myc-CD8TM-BBz-T2A-PAC | 6902-6974 | 17592-17664 |
| BCMA | CD19 | CD8SP-BCMA-FHVH-93-SG4S-CD19-FHVH- 354-Myc-CD8TM-BBz-T2A-PAC | 6975-7047 | 17665-17737 |
| BCMA | CD38 | CD8SP-BCMA-FHVH-74-SG4S-CD38-FHVH- 309021-Myc-CD8TM-BBz-T2A-PAC | 7048-7120 | 17738-17810 |
| CD20 | CD22 | CD8SP-CD20-vHH-USC1-2HC2D6-SG4S- CD22-USC1-FHVH-160-Myc-CD8TM-BBz- T2A-PAC | 7121-7193 | 17811-17883 |
| BCMA | CD38 | CD8SP-BCMA-FHVH-74-SG4S-CD38-FHVH- USC1-32184-Myc-CD8TM-BBz-T2A-PAC | 7194-7266 | 17884-17956 |
| CD20 | CD19 | CD8SP-CD20-vHH-USC1-2HC2D6-SG4S- CD19-FHVH-354-Myc-CD8TM-BBz-T2A-PAC | 7267-7339 | 17957-18029 |

**TABLE 35: SARs based on hu-mROO5 vL and vH domains (Series 1)**

| **TYPE** | **Name of fragment** | **SEQ ID NO (DNA)** | **SEQ ID NO (PRT)** |
|---|---|---|---|
| 2nd Gen.CAR | CD8SP-hu-mROO5-1-vL-Gly-Ser-Linker-hu-mROO5-1- vH-Myc-CD8TM-BBz-T2A-PAC | 7340 | 18030 |
| 2nd Gen.CAR | CD8SP-hu-mROO5-1-vL-Gly-Ser-Linker-hu-mROO5-1- vH-Myc-28z-T2A-PAC | 7341 | 18031 |
| 2nd Gen.CAR | CD8SP-hu-mROO5-1-vL-Gly-Ser-Linker-hu-mROO5-1- vH-Myc-28z | 7342 | 18032 |
| 2nd Gen.CAR | CD8SP-hu-mROO5-1-vL-Gly-Ser-Linker-hu-mROO5-1- vH-Myc-CD8TM-BBz | 7343 | 18033 |
| 2nd Gen.CAR | CD8SP-hu-mROO5-1-vH-Gly-Ser-Linker-vL-Myc- CD8TM-BBz | 7344 | 18034 |
| 2nd Gen.CAR | CD8SP-hu-mROO5-1-vL-Gly-Ser-Linker-hu-mROO5-1- vH-Myc-CD8TM-z-P2A-K13-FLAG-T2A-PAC | 7345 | 18035 |
| DC SIR | CD8SP-hu-mROO5-1-vL-[hTCRa-CSDVP]-F-F2A-SP- hu-mROO5-1-vH-[hTCRb-KACIAH]-F-P2A-PAC | 7346 | 18036 |
| DC SIR | CD8SP-hu-mROO5-1-vL-[hTCRb-KACIAH]-F-P2A-SP- hu-mROO5-1-vH-[hTCRa-CSDVP]-F-F2A-PAC | 7347 | 18037 |
| DC SIR | CD8SP-hu-mROO5-1-vL-[hTCRb-S57C]-F-P2A-SP-hu- mROO5-1-vH-[hTCRa-T48C] | 7348 | 18038 |
| DC SIR | CD8SP-hu-mROO5-1-vL-[hTCRb-S57C]-F-P2A-SP-hu- mROO5-1-vH-[hTCRa-T48C]-F-F2A-K13-opt | 7349 | 18039 |
| DC SIR | CD8SP-hu-mROO5-1-vL-[hTCRa-T48C]-F-P2A-SP-hu- mROO5-1-vH-[hTCRb-S57C] | 7350 | 18040 |
| DC SIR | CD8SP-hu-mROO5-1-vL-[hTCRa-T48C]-F-P2A-SP-hu- mROO5-1-vH-[hTCRb-S57C]-F-P2A-K13-opt | 7351 | 18041 |
| OHC SIR | CD8SP-V5-[hTCRb-KACIAH]-F-P2A-CD8SP-hu- mROO5-1-vL-Gly-Ser-Linker-hu-mROO5-1-vH-Myc- [hTCRa-CSDVP]-F-F2A-PAC | 7352 | 18042 |
| OHC SIR | CD8SP-MYC-[hTCRa-T48C-opt1]-F-F2A-SP-hu- mROO5-1-vL-Gly-Ser-Linker-hu-mROO5-1-vH-V5- [hTCRb-S57C-opt1]-F-P2A-PAC | 7353 | 18043 |
| DC SIR | CD8SP-hu-mROO5-1-vL-[hTCRb-opt2]-F-P2A-SP-hu- mROO5-1-vH-[hTCRa-opt2]-F-F2A-PAC | 7354 | 18044 |
| DC SIR | CD8SP-hu-mROO5-1-vL-[hTCRb-opt2]-F-P2A-SP-hu- mROO5-1-vH-Myc-[pretc.Ra-Del48]-F-F2A-PAC | 7355 | 18045 |
| OHC SIR | CD8SP-[hTCRb-opt2]-F-P2A-CD8SP-hu-mROO5-1-vL- Gly-Ser-Linker-hu-mROO5-1-vH-Myc4-[pretc.Ra-Del48]- F-F2A-PAC | 7356 | 18046 |
| DC SIR | CD8SP-hu-mROO5-1-vL-V5-[hTCRg1-opt]-F-P2A-SP- hu-mROO5-1-vH-Myc-[hTCRd-opt]-F-F2A-PAC | 7357 | 18047 |
| DC SIR | CD8SP-hu-mROO5-1-vL-[hTCRd-opt]-F-P2A-SP-hu- mROO5-1-vH-[hTCRg1-opt] | 7358 | 18048 |
| OHC SIR | CD8SP-V5-[hTCRg1-opt]-F-P2A-CD8SP-hu-mROO5-1- vL-Gly-Ser-Linker-hu-mROO5-1-vH-Myc-[hTCRd-opt]- F-F2A-PAC | 7359 | 18049 |
| DC zSIR | CD8SP-hu-mROO5-1-vL-IgCL-CD3zECDTMCP-opt-F- P2A-Spe-SP-hu-mROO5-1-vH-IgG1-CH1- CD3zECDTMCP-opt2-F-F2A-PAC | 7360 | 18050 |
| DC SIR | CD8SP-hu-mROO5-1-vL-[hTCRbECD-CD3zECDTMCP- opt]-F-P2A-SP-hu-mROO5-1-vH-[hTCRaECD- CD3zECDTMCP-opt2] | 7361 | 18051 |
| DC SIR | CD8SP-hu-mROO5-1-vL-[hTCRb-KAC-ECD- CD3zECDTMCP-opt]-F-P2A-SP-hu-mROO5-1-vH- [hTCRa-CSDVP-ECD-CD3zECDTMCP-opt2] | 7362 | 18052 |
| DC SIR | CD8SP-hu-mROO5-1-vL-V5-[hTCRbECD- CD3zECDTMCP-opt]-F-P2A-SP-hu-mROO5-1-vH-Myc- [hTCRaECD-CD3zECDTM-28z-opt2] | 7363 | 18053 |
| DC SIR | CD8SP-hu-mROO5-1-vL-V5-[hTCRbECD- CD3zECDTM-28z-opt]-F-P2A-SP-hu-mROO5-1-vH- Myc-[hTCRaECD-CD3zECDTM-28z-opt2] | 7364 | 18054 |
| DC SIR | CD8SP-hu-mROO5-1-vL-V5-[hTCRbECD- CD3zECDTMCP-opt]-F-P2A-SP-hu-mROO5-1-vH- Myc4-[hTCRaECD-CD3zECDTM-BBz-opt2] | 7365 | 18055 |
| DC SIR | CD8SP-hu-mROO5-1-vL-V5-[hTCRbECD- CD3zECDTM-BBz-opt]-F-P2A-SP-hu-mROO5-1-vH- Myc4-[hTCRaECD-CD3zECDTM-BBz-opt2] | 7366 | 18056 |
| DC SIR | CD8SP-hu-mROO5-1-vL-CD3zECDTMCP-opt-F-P2A- Spe-SP-hu-mROO5-1-vH-Mlu-CD3zECDTMCP-opt2-F- F2A-PAC | 7367 | 18057 |
| DC Ab-TCR | CD8SP-hu-mROO5-1-vL-[IgCL-TCRg-6MD]-F-P2A-SP- hu-mROO5-1-vH-[IgG1-CH1-TCRd-6MD] | 7368 | 18058 |
| DC Ab-TCR | CD8SP-hu-mROO5-1-vL-[IgCL-TCRb-IAH-6MD]-F- P2A-SP-hu-mROO5-1-vH-[IgG1-CH1-TCRa-SDVP- 6MD] | 7369 | 18059 |
| DC Ab-TCR | CD8SP-hu-mROO5-1-vL-[IgCL-TCRb-wt-opt2-6MD]-F- P2A-SP-hu-mROO5-1-vH-[IgG1-CH1-TCRa-wt-op2- 6MD] | 7370 | 18060 |
| TFPe | CD8SP-hu-mROO5-1-scFv-CD3e-ECDTMCP-opt2 | 7371 | 18061 |
| TFPd | CD8SP-hu-mROO5-1-scFv-CD3d-ECDTMCP-opt2 | 7372 | 18062 |
| TFPg | CD8SP-hu-mROO5-1-scFv-CD3g-ECDTMCP-opt2 | 7373 | 18063 |
| TFPz | CD8SP-hu-mROO5-1-scFv-CD3z-ECDTMCP-opt2 | 7374 | 18064 |
| TFPe | CD8SP-hu-mROO5-1-scFv-CD3e-ECDTMCP-opt2 | 7375 | 18065 |
| TFPd | CD8SP-hu-mROO5-1-scFv-CD3d-ECDTMCP-opt2 | 7376 | 18066 |
| TFPg | CD8SP-hu-mROO5-1-scFv-CD3g-ECDTMCP-opt2 | 7377 | 18067 |
| TFPz | CD8SP-hu-mROO5-1-scFv-CD3z-ECDTMCP-opt2 | 7378 | 18068 |
| SIR Chain | CD8SP-hu-mROO5-1-vL-Gly-Ser-Linker-hu-mROO5-1- vH-[hTCRa-opt2]-F-F2A-PAC | 7379 | 18069 |
| SIR Chain | CD8SP-hu-mROO5-1-vL-Gly-Ser-Linker-hu-mROO5-1- vH-[hTCRb-opt2]-F-F2A-PAC | 7380 | 18070 |
| SIR Chain | CD8SP-hu-mROO5-1-vL-Gly-Ser-Linker-hu-mROO5-1- vH-Myc4-[pretc.Ra-Del48]-F-F2A-PAC | 7381 | 18071 |
| SIR Chain | CD8SP-hu-mROO5-1-vL-[hTCRb-opt2]-F-P2A-PAC | 7382 | 18072 |
| SIR Chain | CD8SP-hu-mROO5-1-vL-[hTCRb-opt2] | 7383 | 18073 |
| SIR Chain | IgHSP-hu-mROO5-1-vH-[hTCRa-opt2]-F-F2A-BlastR | 7384 | 18074 |
| SIR Chain | IgHSP-hu-mROO5-1-vH-[hTCRa-opt2] | 7385 | 18075 |
| SIR Chain | CD8SP-hu-mROO5-1-vL-V5-[hTCRb-S57C-opt]-F-P2A- PAC | 7386 | 18076 |
| SIR Chain | CD8SP-hu-mROO5-1-vL-V5-[hTCRb-S57C-opt] | 7387 | 18077 |
| SIR Chain | IgHSP-hu-mROO5-1-vH-Myc-[hTCRa-T48C-opt]-F-F2A- BlastR | 7388 | 18078 |
| SIR Chain | IgHSP-hu-mROO5-1-vH-Myc-[hTCRa-T48C-opt] | 7389 | 18079 |
| SIR Chain | CD8SP-hu-mROO5-1-vL-Gly-Ser-Linker-hu-mROO5-1- vH-[hTCRa-SDVP]-F-F2A-PAC | 7390 | 18080 |
| SIR Chain | CD8SP-hu-mROO5-1-vL-Gly-Ser-Linker-hu-mROO5-1- vH-[hTCRb-KAIAH]-F-P2A-PAC | 7391 | 18081 |
| TFPS-CD3e-ECDTMCP-opt2-F | CD8SP-hu-mROO5-1-scFv-G4S-CD3e-ECDTMCP-opt2- F-F2A-PAC | 7392 | 18082 |
| TFP-CD3d-ECDTMCP-opt2-F | CD8SP-hu-mROO5-1-scFv-G4S-CD3d-ECDTMCP-opt2- F-F2A-PAC | 7393 | 18083 |
| TFPS-CD3g-ECDTMCP-opt2-F | CD8SP-hu-mROO5-1-scFv-G4S-CD3g-ECDTMCP-opt2- F-F2A-PAC | 7394 | 18084 |
| TFPS-CD3z-ECDTMCP-opt2-F | CD8SP-hu-mROO5-1-scFv-G4S-CD3z-ECDTMCP-opt2- F-F2A-PAC | 7395 | 18085 |
| Bispecific DC SAR | CD8SP-CD19-FHVH-354-G4S3-CD19-hu-mROO5-1-vL- [hTCRb-S57C-opt]-F-P2A-SP-CD19-hu-mROO5-1-vH- [hTCRa-T48C-opt] | 7396 | 18086 |
| Bispecific DC SAR | CD8SP-CD19-FHVH-354-G4S3-CD19-hu-mROO5-1-vL- [hTCRa-T48C]-F-F2A-SP-CD19-hu-mROO5-1-vH- [hTCRb-S57C] | 7397 | 18087 |
| Bispecific DC SAR | CD8SP-CD19-FHVH-354-G4S3-CD19-hu-mROO5-1-vL- [hTCRbECD-CD3zECDTMCP-opt]-F-P2A-SP-CD19-hu- mROO5-1-vH-[hTCRaECD-CD3zECDTMCP-opt2] | 7398 | 18088 |
| Bispecific DC SAR | CD8SP-CD19-FHVH-354-G4S3-CD19-hu-mROO5-1-vL- [IgCL-TCRg-6MD]-F-P2A-SP-CD19-hu-mROO5-1-vH- [IgG1-CH1-TCRd-6MD] | 7399 | 18089 |
| Bispecific DC SAR | CD8SP-CD19-FHVH-354-G4S3-CD19-hu-mROO5-1-vL- [IgCL-TCRb-wt-opt2-6MD]-F-F2A-SP-CD19-hu- mROO5-1-vH-[IgG1-CH1-TCRa-wt-op2-6MD] | 7400 | 18090 |
| Trispecific DC SAR | CD8SP-CD19-FHVH-354-G4S3-CD19-hu-mROO5-1-vL- [hTCRa-T48C]-F-F2A-SP-CD22-FHVH-24-CD19-hu- mROO5-1-vH-[hTCRb-S57C] | 7401 | 18091 |
| Trispecific DC SAR | CD8SP-CD19-FHVH-354-G4S3-CD19-hu-mROO5-1-vL- [hTCRbECD-CD3zECDTMCP-opt]-F-P2A-SP-CD22- FHVH-24-CD19-hu-mROO5-1-vH-[hTCRaECD- CD3zECDTMCP-opt2] | 7402 | 18092 |
| Trispecific DC SAR | CD8SP-CD19-FHVH-354-G4S3-CD19-hu-mROO5-1-vL- [IgCL-TCRg-6MD]-F-P2A-SP-CD22-FHVH-24-CD19- hu-mROO5-1-vH-[IgG1-CH1-TCRd-6MD] | 7403 | 18093 |
| Trispecific DC SAR | CD8SP-CD19-FHVH-354-G4S3-CD19-hu-mROO5-1-vL- [IgCL-TCRb-wt-opt2-6MD]-F-F2A-SP-CD22-FHVH-24- CD19-hu-mROO5-1-vH-[IgG1-CH1-TCRa-wt-op2-6MD] | 7404 | 18094 |
| Bispecific DC SAR | CD8SP-CD38-USC1-FHVH-32184-G4Sx3-R1-hu- mROO5-1-vL-[hTCRb-S57C]-F-P2A-SP-hu-mROO5-1- vH-[hTCRa-T48C] | 7405 | 18095 |
| Bispecific DC SAR | CD8SP-hu-mROO5-1-vL-[hTCRb-S57C]-F-P2A-IgSP- Apa-CD20-USC1-vHH-2HCD26-G4Sx3v2-hu-mROO5-1- vH-[hTCRa-T48C] | 7406 | 18096 |
| Trispecific DC SAR | CD8SP-hu-mROO5-1-vL-[hTCRb-S57C]-F-P2A-IgSP- CD22-USC1-FHVH-160-G4Sx2-CD20-USC1-vHH- 2HCD26-G4Sx3v2-hu-mROO5-1-vH-[hTCRa-T48C] | 7407 | 18097 |
| Trispecific DC SAR | CD8SP-CD38-USC1-FHVH-32184-G4Sx3-R1-hu- mROO5-1-vL-[hTCRb-S57C]-F-P2A-IgSP-Apa-CD20- USC1-vHH-2HCD26-G4Sx3v2-hu-mROO5-1-vH- [hTCRa-T48C] | 7408 | 18098 |

**TABLE 36: Unispecific, Bispecifics and Trispecific Constructs on different backbones targeting different antigens. These constructs were generated by changing the vL/vH modules of the construct in Table 35 with the vL/vH shown in the first construct of each series in the Table below. The order of the TCR constant chain fragments of the constructs of each series in Table 36 is the same as the order of TCR constant chain fragments of the constructs of series 1 whose details are provided in Table 35.**

| **Series** | **Target** | **Name of first construct in the series** | **SEQ ID NO of Series (DNA)** | **SEQ ID NO of Series (PRT)** |
|---|---|---|---|---|
| 1 | CD19 | CD8SP-hu-mROO5-1-vL-G4Sx3-hu-mROO5-1-vH-Myc- CD8TM-BBz-T2A-PAC | 7340-7408 | 18030-18098 |
| 2 | BCMA | CD8SP-BCMA-hu-USC73-vL-G4Sx3-BCMA-hu- USC73-G32S-vH-Myc-CD8TM-BBz-T2A-PAC | 7409-7477 | 18099-18167 |
| 3 | BCMA | CD8SP-BCMA-hu-USC74-vL-G4Sx3-BCMA-hu- USC74-F35Y-vH-Myc-CD8TM-BBz-T2A-PAC | 7478-7546 | 18168-18236 |
| 4 | BCMA | CD8SP-BCMA-hu-USC75-vL-G4Sx3-BCMA-hu- USC75-V104T-vH-Myc-CD8TM-BBz-T2A-PAC | 7547-7615 | 18237-18305 |
| 5 | BCMA | CD8SP-BCMA-huUSC76-vL-G4Sx3-BCMA-huUSC76- I58S-vH-Myc-CD8TM-BBz-T2A-PAC | 7616-7684 | 18306-18374 |
| 6 | BCMA | CD8SP-BCMA-hu-USC77-vL-G4Sx3-BCMA-hu- USC77-T59S-vH-Myc-CD8TM-BBz-T2A-PAC | 7685-7753 | 18375-18443 |
| 9 | BCMA | CD8SP-BCMA-hu-USC80-N25K-vL-G4Sx3-BCMA- huUSC80-vH-Myc-CD8TM-BBz-T2A-PAC | 7892-7960 | 18582-18650 |
| 10 | BCMA | CD8SP-BCMA-huUSC81-I27L-vL-G4Sx3-BCMA- huUSC81-vH-Myc-CD8TM-BBz-T2A-PAC | 7961-8029 | 18651-18719 |
| 11 | BCMA | CD8SP-BCMA-hu-USC82-S29G-vL-G4Sx3-BCMA- huUSC82-vH-Myc-CD8TM-BBz-T2A-PAC | 8030-8098 | 18720-18788 |
| 12 | BCMA | CD8SP-BCMA-hu-USC83-S31T-vL-G4Sx3-BCMA- huUSC83-vH-Myc-CD8TM-BBz-T2A-PAC | 8099-8167 | 18789-18857 |
| 13 | BCMA | CD8SP-BCMA-hu-USC80-N25K-vL-G4Sx3-BCMA-hu- USC73-G32S-vH-Myc-CD8TM-BBz-T2A-PAC | 8168-8236 | 18858-18926 |
| 14 | BCMA | CD8SP-BCMA-hu-USC81-I27L-vL-G4Sx3-BCMA-hu- USC73-G32S-vH-Myc-CD8TM-BBz-T2A-PAC | 8237-8305 | 18927-18995 |
| 15 | BCMA | CD8SP-BCMA-hu-USC82-S29G-vL-G4Sx3-BCMA-hu- USC73-G32S-vH-Myc-CD8TM-BBz-T2A-PAC | 8306-8374 | 18996-19064 |
| 16 | ROR1 | CD8SP-ROR1-JJ-67-vL-G4Sx3-ROR1-JJ-67-vH-Myc- CD8TM-BBz-T2A-PAC | 8375-8443 | 19065-19133 |
| 17 | ROR1 | CD8SP-ROR1-JJ-78-vL-G4Sx3-ROR1-JJ-78-vH-Myc- CD8TM-BBz-T2A-PAC | 8444-8512 | 19134-19202 |
| 18 | ROR1 | CD8SP-ROR1-JJ-76-vL-G4Sx3-ROR1-JJ-76-vH-Myc- CD8TM-BBz-T2A-PAC | 8513-8581 | 19203-19271 |
| 19 | CD20 | CD8SP-CD20-Rituximab-vL-G4Sx3-CD20-Rituximab- vH-Myc-CD8TM-BBz-T2A-PAC | 8582-8650 | 19272-19340 |
| 20 | Her2 | CD8SP-Her2-FRP5-vL-G4Sx3-Her2-FRP5-vH-Myc- CD8TM-BBz-T2A-PAC | 8651-8719 | 19341-19409 |
| 21 | CEA | CD8SP-CEA-huMN14-vL-G4Sx3-CEA-huMN14-vH- Myc-CD8TM-BBz-T2A-PAC | 8720-8788 | 19410-19478 |
| 22 | CEA | CD8SP-CEA-BW431-26-vL-G4Sx3-CEA-BW431-26- vH-Myc-CD8TM-BBz-T2A-PAC | 8789-8857 | 19479-19547 |
| 23 | Her2 | CD8SP-Her2-huMab4D5-H91A-vL-G4Sx3-Her2- huMab4D5-D98A-F100A-Y102V-Myc-CD8TM-BBz- T2A-PAC | 8858-8926 | 19548-19616 |
| 24 | Her2 | CD8SP-Her2-USC-1516-vL-G4Sx3-Her2-USC-1516-vH- Myc-CD8TM-BBz-T2A-PAC | 8927-8995 | 19617-19685 |
| 25 | TOSO | CD8SP-TOSO-6B10-vL-G4Sx3-TOSO-6B10-vH-Myc- CD8TM-BBz-T2A-PAC | 8996-9064 | 19686-19754 |
| 26 | CD30 | CD8SP-CD30-HRS3-vL-G4Sx3-CD30-HRS3-vH-Myc- CD8TM-BBz-T2A-PAC | 9065-9133 | 19755-19823 |
| 27 | CD229 | CD8SP-CD229-USC1-2D4-vL-G4Sx3-CD229-USC1- 2D4-vH-Myc-CD8TM-BBz-T2A-PAC | 9134-9202 | 19824-19892 |
| 28 | CD229 | CD8SP-CD229-2A2-vL-G4Sx3-CD229-2A2-vH-Myc- CD8TM-BBz-T2A-PAC | 9203-9271 | 19893-19961 |
| 29 | CD229 | CD8SP-CD229-USC3-2D5-vL-G4Sx3-CD229-USC3- 2D5-vH-Myc-CD8TM-BBz-T2A-PAC | 9272-9340 | 19962-20030 |
| 30 | CD229 | CD8SP-CD229-USC2-2D4-vL-G4Sx3-CD229-USC2- 2D4-vH-Myc-CD8TM-BBz-T2A-PAC | 9341-9409 | 20031-20099 |
| 31 | EBV-gp350 | CD8SP-EBV-gp350-7A1-vL-G4Sx3-EBV-gp350-7A1- vH-Myc-CD8TM-BBz-T2A-PAC | 9410-9478 | 20100-20168 |
| 32 | EBV-gp350 | CD8SP-EBV-gp350-6G4-vL-G4Sx3-EBV-gp350-6G4- vH-Myc-CD8TM-BBz-T2A-PAC | 9479-9547 | 20169-20237 |
| 33 | INFLU ENZA-NA | CD8SP-INFL-NA-1E01-vL-G4Sx3-INFL-NA-1E01-vH- Myc-CD8TM-BBz-T2A-PAC | 9548-9616 | 20238-20306 |
| 34 | EBV-LMP1 | CD8SP-EBV-LMP1-HLEA-vL-G4Sx3-EBV-LMP1- HLEA-vH-Myc-CD8TM-BBz-T2A-PAC | 9617-9685 | 20307-20375 |
| 35 | PSMA | CD8SP-PSMA-J591-ds75-vL-G4Sx3-PSMA-J591-ds75- vH-Myc-CD8TM-BBz-T2A-PAC | 9686-9754 | 20376-20444 |
| 36 | PSMA | CD8SP-hu-PSMA-J591-vL-G4Sx3-hu-PSMA-J591-vH- Myc-CD8TM-BBz-T2A-PAC | 9755-9823 | 20445-20513 |
| 37 | PSMA | CD8SP-hu-PSMA-2-J591-vL-G4Sx3-hu-PSMA-2-J591- vH-Myc-CD8TM-BBz-T2A-PAC | 9824-9892 | 20514-20582 |
| 38 | PSMA | CD8SP-hu-PSMA-J591-vL-G4Sx3-PSMA-J591-ds75- vH-Myc-CD8TM-BBz-T2A-PAC | 9893-9961 | 20583-20651 |
| 39 | PSMA | CD8SP-PSMA-J591-ds75-vL-G4Sx3-hu-PSMA-J591- vH-Myc-CD8TM-BBz-T2A-PAC | 9962-10030 | 20652-20720 |
| 40 | MUC1 | CD8SP-hu-MUC1-star2-MN-E6-vL-G4Sx3-hu-MUC1- star2-MN-E6-vH-Myc-CD8TM-BBz-T2A-PAC | 10031-10099 | 20721-20789 |
| 41 | MUC1 | CD8SP-MUC1-star-MN-E6-vL-G4Sx3-MUC1-star-MN- E6-vH-Myc-CD8TM-BBz-T2A-PAC | 10100-10168 | 20790-20858 |
| 42 | MUC1 | CD8SP-hu-MUC1-star-MN-C2-vL-G4Sx3-hu-MUC1- star-MN-C2-vH-Myc-CD8TM-BBz-T2A-PAC | 10169-10237 | 20859-20927 |
| 43 | gpA33 | CD8SP-hu-gpA33-C825-vL-G4Sx3-hu-gpA33-C825-vH- Myc-CD8TM-BBz-T2A-PAC | 10238-10306 | 20928-20996 |
| 44 | MSLN | CD8SP-MSLN-7D10-vL-G4Sx3-MSLN-7D10-vH-Myc- CD8TM-BBz-T2A-PAC | 10307-10375 | 20997-21065 |
| 45 | MSLN | CD8SP-MSLN-7D9-HL-vL-G4Sx3-MSLN-7D9-HL-vH- Y27F-Myc-CD8TM-BBz-T2A-PAC | 10376-10444 | 21066-21134 |
| 46 | MSLN | CD8SP-MSLN-7D9-HL-vL-V29-G4Sx3-MSLN-7D9- HL-vH-Myc-CD8TM-BBz-T2A-PAC | 10445-10513 | 21135-21203 |
| 47 | MSLN | CD8SP-MSLN-hu22A10-vL-G4Sx3-MSLN-hu22A10- vH-F27Y-Myc-CD8TM-BBz-T2A-PAC | 10514-10582 | 21204-21272 |
| 48 | MSLN | CD8SP-MSLN-hu22A10 -vL-N31S-vH-G4Sx3-MSLN- hu22A10 -vH-Myc-CD8TM-BBz-T2A-PAC | 10583-10651 | 21273-21341 |
| 49 | MSLN | CD8SP-MSLN-hu22A10-vL-Y96W-G4Sx3-MSLN- hu22A10-vH-Myc-CD8TM-BBz-T2A-PAC | 10652-10720 | 21342-21410 |

**TABLE 37: EXEMPLARY EPITOPE TAGS**

| SEQ ID NO | NAME | SEQ ID NO: | NAME |
|---|---|---|---|
| 22411 | RITX | 22423 | Cituximab-mimo-1 |
| 22412 | Ritx2 | 22424 | Cituximab-mimotopx2 |
| 22413 | RitxGA | 22425 | Cituximab-mimotop |
| 22414 | RitxGA4 | 22426 | Panitumumab-tag 1 |
| 22415 | RituxGAtag | 22427 | Panitumumab-tag2 |
| 22416 | RitxGAx22-opt2 | 22428 | Panitumumab-tag3 |
| 22417 | RitxGAx2 | 22429 | Panitumumab-tag4 |
| 22418 | R15Cx2 | 22430 | Panitumumab-tag2x2 |
| 22419 | R5pLx2-Qbendx2CD28TM | 22431 | Panitumumab-tag2-v2 |
| 22420 | RituxGAtag-Herceptin-Mimotag | 22432 | Herceptin-Mimotag |
| 22421 | Cituxumab-Mimotope 1 | 22433 | HercepMimotopx3 |
| 22422 | Cituximab-mimo-1x2 | | |

**TABLE 38: MHC restricted Epitopes**

| **Name of fragment** | **SEQ ID NO** | **Name of fragment** | **SEQ ID NO** |
|---|---|---|---|
| gp100 | 21443 | EBNA-3c | 21455 |
| gp100 | 21444 | WT1 | 21456 |
| gp100 | 21445 | PR1 | 21457 |
| MUC1-A7(130-138) | 21446 | Ras9-G12V | 21458 |
| MUC1-D6(13-21) | 21447 | HPV16-E7 | 21459 |
| TAX(11-19) | 21448 | NY-ESO-1-(155-163) | 21460 |
| hTERT(540-548) | 21449 | NY-ESO-1-(157-165) | 21461 |
| hTERT(865-873) | 21450 | NY-ESO-1-(157-167) | 21462 |
| HIV1gag(77-85) | 21451 | Mesothelin | 21463 |
| CMV-pp65(495-503) | 21452 | MAGE-A3peptide | 21464 |
| MART(26-35) | 21453 | AFP-158 | 21465 |
| EBNA-3A(596-604) | 21454 | | |

**TABLE 39. NOVEL ANTIGEN BINDING DOMAINS WITH CDRs**

| **TARGET** | **Name of Fragmentt** | **SEQ ID NO** | **CDR1 SEQ ID NO** | **CDR2 SEQ ID NO** | **CDR3 SEQ ID NO** |
|---|---|---|---|---|---|
| MSLN | MSLN-7D10-vL | 23136 | 23181 | 23226 | 23271 |
| MSLN | MSLN-7D9-HL-vL | 23137 | 23182 | 23227 | 23272 |
| MSLN | MSLN-7D9-HL-V29L-vL | 23138 | 23183 | 23228 | 23273 |
| MSLN | MSLN-hu22A10-vL | 23139 | 23184 | 23229 | 23274 |
| MSLN | MSLN-hu22A10-N31S-vL | 23140 | 23185 | 23230 | 23275 |
| MSLN | MSLN-hu22A10-Y96W-vL | 23141 | 23186 | 23231 | 23276 |
| BCMA | BCMA-hu-USC79-vL | 23142 | 23187 | 23232 | 23277 |
| BCMA | BCMA-hu-USC80-N25K-vL | 23143 | 23188 | 23233 | 23278 |
| BCMA | BCMA-hu-USC81-I27L-vL | 23144 | 23189 | 23234 | 23279 |
| BCMA | BCMA-hu-USC82-S29G-vL | 23145 | 23190 | 23235 | 23280 |
| BCMA | BCMA-hu-USC83-S31T-vL | 23146 | 23191 | 23236 | 23281 |
| CS1 | hu-Luc64-2-vL | 23147 | 23192 | 23237 | 23282 |
| MSLN | MSLN-7D10-vH | 23148 | 23193 | 23238 | 23283 |
| MSLN | MSLN-7D9-HL-Y27F-vH | 23149 | 23194 | 23239 | 23284 |
| MSLN | MSLN-7D9-HL-vH | 23150 | 23195 | 23240 | 23285 |
| MSLN | MSLN-hu22A10-F27Y-vH | 23151 | 23196 | 23241 | 23286 |
| MSLN | MSLN-hu22A10-vH | 23152 | 23197 | 23242 | 23287 |
| MSLN | MSLN-hu22A10-vH | 23153 | 23198 | 23243 | 23288 |
| BCMA | BCMA-hu-USC73-G32S-vH | 23154 | 23199 | 23244 | 23289 |
| BCMA | BCMA-huUSC74-F35Y-vH | 23155 | 23200 | 23245 | 23290 |
| BCMA | BCMA-huUSC75-V104T-vH | 23156 | 23201 | 23246 | 23291 |
| BCMA | BCMA-huUSC76-I58S-vH | 23157 | 23202 | 23247 | 23292 |
| BCMA | BCMA-huUSC77-T59S-vH | 23158 | 23203 | 23248 | 23293 |
| BCMA | BCMA-huUSC78-S64G-vH | 23159 | 23204 | 23249 | 23294 |
| BCMA | BCMA-huUSC79-S64T-vH | 23160 | 23205 | 23250 | 23295 |
| CS1 | hu-Luc64-A60T-vH | 23161 | 23206 | 23251 | 23296 |
| CD20 | CD20-VHH-USC1 | 23162 | 23207 | 23252 | 23297 |
| MUC16 | MUC16-vHH-R3MU5-USC1 | 23163 | 23208 | 23253 | 23298 |
| MUC16 | MUC16-USC2-huR3MU30 | 23164 | 23209 | 23254 | 23299 |
| IL13Ra2 | IL13Ra2-vHH-USC2-Cl3 | 23165 | 23210 | 23255 | 23300 |
| IL13Ra2 | IL13Ra2-huvHH-USC1-C1 | 23166 | 23211 | 23256 | 23301 |
| BCMA | BCMA917-vHH-E59D | 23167 | 23212 | 23257 | 23302 |
| CD38 | CD38-331-vHH-D64E | 23168 | 23213 | 23258 | 23303 |
| CD38 | CD38-331-vHH-S53E | 23169 | 23214 | 23259 | 23304 |
| CD123 | CD123-USC-3-vHH | 23170 | 23215 | 23260 | 23305 |
| PD1 | PD1-vHH-USC-102C4 | 23171 | 23216 | 23261 | 23306 |
| PD1 | PD1-vHH-USC-707 | 23172 | 23217 | 23262 | 23307 |
| PD1 | PD1-vHH-C13 | 23173 | 23218 | 23263 | 23308 |
| CD22 | CD22-FHVH-162 | 23174 | 23219 | 23264 | 23309 |
| PSMA | PSMA-USC-29-chVH | 23175 | 23220 | 23265 | 23310 |
| PSMA | PSMA-USC-30-chVH | 23176 | 23221 | 23266 | 23311 |
| PSMA | PSMA-USC-31-chVH | 23177 | 23222 | 23267 | 23312 |
| PSMA | PSMA-USC76-chVH | 23178 | 23223 | 23268 | 23313 |
| PSMA | PSMA-USC101-chVH | 23179 | 23224 | 23269 | 23314 |
| PSMA | PSMA-USC104-chVH | 23180 | 23225 | 23270 | 23315 |

**Table 40: Exemplary diseases targeted by SARs.**

| **SAR/BiTE "X" TARGET** | **EXEMPLARY DISEASE TARGETED BY SARs (*i.e.,* conventional CARs and next generation CARs. *e.g.,* SIR, cTCR, Ab-TCR, AABD-TCR, TFP, Tri-TAC and TCR)** |
|---|---|
| CD19 | ALL, CLL, lymphoma, lymphoid blast crisis of CML, multiple myeloma, immune disorders |
| ALK | Non-small cell lung Cancer (NSCLC), ALCL (anaplastic large cell lymphoma), IMT (inflammatory myofibroblastic tumor), or neuroblastoma |
| CD45 | Blood cancers |
| BCMA | Myeloma, PEL, plasma cell leukemia, Waldenstrom's macroglobinemia |
| CD5 | Blood cancer, T cell leukemia, T cell lymphoma |
| CD20 | Blood cancers, Leukemia, ALL, CLL, lymphoma, immune disorders |
| CD22 | Blood cancers, Leukemia, ALL, CLL, lymphoma, lymphoid blast crisis of CML, immune disorders |
| CD23 | Blood cancers, Leukemia, ALL, CLL, lymphoma, autoimmune disorders |
| CD30 | Hodgkins's lymphoma, Cutaneous T cell lymphoma |
| CD32 | Solid tumors |
| CD33 | Blood cancers, AML, MDS |
| CD34 | Blood cancers, AML, MDS |
| CD44v6 | Blood cancers, AML, MDS |
| CD70 | Blood cancers, lymphoma, myeloma, Waldenstrom's macroglobulinemia, Kidney cancer |
| CD79b | Blood cancers, ALL, Lymphoma |
| CD123 | Blood cancers, AML, MDS |
| CD138 | Blood cancers, Myeloma, PEL, plasma cell leukemia, waldenstrom's macroglobulinemia |
| CD179b | Blood cancers, ALL, Lymphoma |
| CD276/B7-H3 | Ewing's sarcoma, neuroblastoma, rhabdomyosarcoma, ovarian, colorectal and lung cancers |
| CD324 | Solid tumors, esophageal, prostate, colorectal, breast, lung cancers |
| CDH6 | Solid tumors, renal, ovarian, thyroid cancers |
| CDH17 | Adenocarciniomas, gastrointestinal, lung, ovarian, endometrial cancers |
| CDH19 | Solid tumor, Melanoma |
| EGFR | Colon cancer, lung cancer |
| CLEC5A | Blood cancers, Leukemia, AML |
| GR/LHR | Prostate cancer, ovarian cancer or breast cancer |
| CLL1 | Blood cancer, Leukemia |
| CMVpp65 | CMV infection, CMV colitis, CMV pneumonitis |
| CS1/SLAMF7 | Blood cancers, myeloma, PEL, plasma cell leukemia, CLL, melanoma, lung cancer, ovarian cancer |
| CSF2RA | AML, CML, MDS |
| CD123 | Blood cancers, AML, MDS |
| DLL3 | Melanoma, lung cancer or ovarian cancer |
| EBNA3c/MHC I | Epstein Barr virus infection and related diseases including cancers |
| EBV-gp350 | Epstein Barr virus infection and related diseases |
| EGFR | Solid tumors, Colon cancer, lung cancer |
| EGFRvIII | Solid tumors, glioblastoma |
| EpCam1 | Gastrointestinal cancer |
| FLT3 | Blood cancers, AML, MDS, ALL |
| Folate Receptor alpha(FR1 or FOLR1) | Ovarian cancer, NSCLC, endometrial cancer, renal cancer, or other solid tumors |
| FSHR | Prostate cancer, ovarian cancer or breast cancer |
| GD2 | Neuroblastoma |
| GD3 | Melanoma |
| GFRa4 | Cancer, thyroid medullary cancer |
| Fucosyl-GM1(GM1) | Small cell lung cancer |
| GPRC5D | Myeloma, PEL, plasma cell leukemia, waldenstrom's macroglobulinemia |
| gp100 | Melanoma |
| GPC3 | Solid tumors, Lung cancer |
| gpNMB | Melanoma, brain tumors, gastric cancers |
| GRP78 | Myeloma |
| Her2 | Solid tumors, breast cancer, stomach cancer |
| Her3 | Colorectal, breast cancer |
| HMW-MAA | Melanoma |
| HTLV1-TAX/MHC I | HTLV1 infection associated diseases, Adult T cell leukemia-lymphoma |
| IL11Ra | Blood cancers, AML, ALL, CML, MDS, sarcomas |
| IL6Ra | Solid tumors, Liver cancer |
| IL13Ra2 | Glioblastomas |
| KSHV-K8.1 | Kaposi's sarcoma, PEL, Multicentric Castleman's disease |
| LAMP1 | Blood cancers, AML, ALL, MDS, CLL, CML |
| LewisY | Cancers |
| L1CAM | Solid tumors, ovarian, breast, endometrial cancers, melanoma |
| LHR | Prostate cancer, ovarian cancer or breast cancer |
| Lym1 | Blood cancer, Leukemia, Lymphoma |
| Lym2 | Blood cancer, Leukemia, Lymphoma |
| CD79b | Blood cancers, lymphoma |
| MART1/MHC I | Melanoma |
| Mesothelin | Mesothelioma, ovarian cancer, pancreatic cancer |
| Muc1/MHC I | Breast cancer, gastric cancer, colorectal cancer, lung cancer, or other solid tumors |
| Muc16 | Ovarian cancer |
| NKG2D | Leukemia, lymphoma or myeloma |
| NYBR1 | Breast cancer |
| PSCA | Prostate cancer |
| PR1/MHC I | Blood cancer, Leukemia |
| Prolactin Receptor | Breast cancer, chromophobe renal cell cancer |
| PSMA | Prostate cancer |
| PTK7 | Melanoma, lung cancer or ovarian cancer |
| ROR1 | Blood cancer, B cell malignancy, lymphoma, CLL |
| SLea | Pancreatic cancer, colon cancer |
| SSEA4 | Pancreatic cancer |
| Tyrosinase/MHC I | Melanoma |
| TCRB1 | T cell leukemias and lymphomas, autoimmune disorders |
| TCRB2 | T cell leukemias and lymphomas, autoimmune disorders |
| TCRgd | T cell leukemias and lymphomas, autoimmune disorders |
| hTERT | Solid tumors, blood cancers |
| TGFBR2 | Solid tumors, keloid |
| TIM1/HAVCR1 | Kidney cancer, liver cancer |
| TROP2 | Solid tumors, Breast cancer,prostate cancer |
| TSHR | Thyroid cancer, T cell leukemia, T cell Lymphoma |
| TSLPR | Blood cancers, Leukemias, AML, MDS |
| Tyrosinase/MHC I | Melanoma |
| VEGFR3 | Solid tumors |
| WT1/MHC I | Blood cancers, AML |
| Folate Receptorβ | AML, Myeloma |
| B7H4 | Breast cancer or ovarian cancer |
| CD23 | Blood cancers, Leukemias, CLL |
| GCC | Gastrointestinal cancer |
| CD200R | Blood cancers, AML, MDS |
| AFP/MHC I | Solid tumors, Liver cancer |
| CD99 | Liver cancer |
| GPRC5D | Myeloma, waldenstrom's macroglobinemia |
| HPV16-E7/MHC I | HPV16 associated cancers, cervical cancer, head and neck cancers |
| Tissue Factor 1 (TF1) | Solid tumors |
| Tn-Muc1 | Solid tumors and blood cancers |
| Igk-Light Chain | Myeloma, plasma cell leukemia |
| Ras G12V/ MHC I | Solid tumors and blood cancers |
| CLD 18A2 (Claudin 18.2) | Gastric, pancreatic, esophageal, ovarian, or lung cancer |
| CD43 | Blood cancers, AML |
| NY-ESO-1/MHC I | Myeloma |
| MPL/TPO-R | Blood cancer, AML, MDS, CML, ALL, Myeloproliferative disorders, Polycythemia vera, Myelofibrosis, Essential Polycythemia |
| P-glycoprotein (MDR1) | Renal cancer, liver cancer, Myeloma |
| CD179a | Blood cancers, Acute Leukemia, CLL, ALL, Lymphoma |
| STEAP1 | Gastric or prostate cancer, or lymphoma |
| Liv1 (SLC39A6) | Breast or prostate cancer |
| Nectin4 (PVRL4) | Bladder, renal, cervical, lung, head and neck or breast cancer |
| Cripto (TDGF 1) | Colorectal or endometrial or ovarian cancer |
| gpA33 | Colorectal or endometrial or ovarian cancer |
| FLT3 | Blood cancers, AML, ALL, MDS |
| BST1/CD157 | Blood cancers, AML, MDS |
| IL1RAP | Liver, colorectal, cervical, lung or ovarian cancer |
| Low Conductance Chloride Channel (LCCC) | Glioma |
| IgE | Allergy |
| HLA-A2 | Graft vs host disease, tissue rejection (SIR Expressed in regulatory T cells) |
| Amyloid | Amyloidoses, alzheimer's disease |
| HIV1-env | HIVI/AIDS and related conditions |
| HIV1-gag | HIV1/AIDS and related conditions |
| Influenza A HA | Influenza A infection |
| CD32b | Blood cancers, AML, CLL, ALL, CML, Myeloma, Lymphoma |
| CD96 | Blood cancers, AML, B-ALL, T cell lymphoma, T-ALL, melanoma |
| CD229 | Blood cancers, myeloma, lymphoma |

In one aspect, the disclosure provides novel compositions of synthetic antigen receptor (SARs). In another aspect, the disclosure provides novel configuration/architectures of SARs. In another aspect, the disclosure provides SARs with useful biological properties (*e.g.,* expression, binding affinity, effector functions *etc.*)*.* In another aspect, the disclosure provides SARs capable of binding to one or more than one antigen. In another aspect, the disclosure provides SARs capable of binding to one or more than one epitope of an antigen.

In one aspect, the disclosure provides a synthetic antigen receptor (SAR) comprising more than one (*i.e.,* 2, 3, 4, 5 or more) antigen binding domains. In another aspect, the disclosure provides a SAR capable of binding to and/or responding to more than one antigen or more than one epitope of an antigen. In another aspect, the disclosure provides a bispecific and/or a multispecific SAR capable of binding to and/or responding to more than one antigen or more than one epitope of an antigen. In another aspect, the disclosure provides useful antigen binding domains for construction of a bispecific and/or a multispecific SAR. In another aspect, the disclosure provides useful configurations (*i.e.,* the location of different domains) for a bispecific and/or a multispecific SAR. The bispecific and multispecific SAR of disclosure when expressed in an immune effector cell (*e.g.,* a T cell, NKT cell or NK cell *etc.*) confers on it the ability to bind to and/or respond to more than one antigen or more than one epitope of an antigen with nearly equal efficacy or greater efficacy as compared to two or more unispecific SAR targeting those same antigens or same epitopes of those antigens.

The presence of two or more antigen binding domains in a bispeficic or multi-specific SAR may result in steric hinderance, non-specific aggregation, poor expression, protein unfolding, and/or interference with antigen binding. In addition, the location of the antigen binding domain(s) relative to the transmebrane domain of SARs needs to be optimized in order to optimize signal transduction by the resulting receptor. Bispecific and multispecific CARs incorporating two or more scFv have been described in the art. However, the disclosure identifies that presence of more than one scFv (*i.e.,* 2, 3, 4 or more) in a SAR (*e.g.,* 2^{nd} generation CAR, SIR, Ab-TCR, zSIR, TFP or rTCR *etc.*) often results in steric hinderance, non-specific aggregation, tonic-signaling, poor expression, protein unfolding, and/or interference with antigen binding resulting in poor signaling and effector function (*e.g.,* cytokine production, cytotoxicity *etc*.)*.* Therefore, a major challenge in the generation of bispecific and multi-specific SARs comprising two or more antigen binding domains is to determine useful antigen binding domains (*e.g*., scFv, Fv, Fab, vHH, FHVH, Centyrin, affibody, cytokine, receptor, svd-TCR, *etc.*) that should be incorporated in such SARs so as to reduce steric hinderance, non-specific aggregation, tonic-signaling, poor expression, protein unfolding, and/or interference with antigen binding that can lead to poor signaling and effector function (*e.g.,* cytokine production, cytotoxicity *etc.*)*.*

A second challenge is to determine a useful configuration of the various antigen binding domains that comprise the bispecific and multi-specific SARs. For example, the optimal order of various antigen binding domains with respect to each other and with respect to other components of the SAR (*e.g.,* hinge domain, transmembrane domain *etc.*) needs to be determined to reduce non-specific aggregation, tonic-signaling, poor expression, protein unfolding, and/or interference with antigen binding resulting in poor signaling and effector function (*e.g.,* cytokine production, cytotoxicity *etc.*)*.* This is a significant challenge for all SARs and particularly for multichain SARs, such as SIR, Ab-TCR, zSIR, αβTFP or γδTFP whose antigen binding domain is composed of two different fragments (*e.g*., vL and vH, Va and Vb or Vg and Vd *etc.*)*.* For example, the attachment of a second antigen binding domain (*e.g.,* scFv or a vHH domain) to a double chain SIR (*e.g.,* CD8SP-hu-mROO5-1-vL-[hTCRb-S57C]-F-P2A-SP-hu-mROO5-1-vH-[hTCRa-T48C]; SEQ ID NO: 7348) which binds to CD19 through a vL and vH fragments that are operably linked to two separate TCR constant chains but join to form a Fv that binds to CD19 could potentially interfere with the interaction between the vL and vH fragments resulting in their inability to form a funcational Fv that can bind CD19.

The length of the hinge domain, which determines the distance between the antigen binding domain and the cell membrane, may influence the signaling via a chimeric antigen receptor. Therefore, another challenge in the field is that it is not known at the present whether attachment of multiple antigen binding domains may adversely affect the formation of an effective immunological synapse and signaling via a SAR by increasing the distance between the target antigen and the cell membrane.

Fusion of multiple antigen binding domains in a SAR could result in steric hinderance and improper folding. Another challenge in the field is that it is not known at the present whether linker domains are needed between the different antigen binding domains of a bispecific/multispecific SAR. The length and nature of the linker domains is also not known. This is of particular importance in case of double chain SAR (*e.g.,* double chain SIR, double chain zSIR, Ab-TCR, αβTFP or γδ TFP *etc.*) as the addition of an improper linker could potentially interfere with the interaction between the two chains or formation of a functional Fv. Additionally, linker(s) could adversely affect the formation of an effective immunological synapse and signaling via a SAR by increasing the distance between the target antigen and the cell membrane.

In one aspect, the present disclosure offers solution to the above problems.

In one aspect, the disclosure provides SARs with one or more antigen binding domains and one or more transmembrane domains. In an embodiment, the disclosure provides useful antigen binding domains for construction of bispecific and multispecific SARs.

The disclosure provides several exemplary SARs comprising different antigen binding domains, hinge domains, linker domains, connecting peptides, transmembrane domains, activation domains, costimulatory domains, accessory modules and therapeutic controls *etc.* The names and SEQ ID (DNA) and SEQ ID (PRT) of exemplary components that can be used in the construction of the SAR are provided in **Tables 1 to 20 and 24.** The names and SEQ ID (DNA) and SEQ ID (PRT) of exemplary SARs are provided in **Tables 25-36 and 41-50.** The target antigen(s), configuration and composition of the SARs can be deduced from their nucleic acid sequences and amino-acid sequences provided in this disclosure by performing sequence homology search for their component modules using programs such as BLAST. Alternatively, softwares, such as ApE (https://jorgensen.biology.utah.edu/wayned/ape/), can be used to determine the composition of the different SAR constructs whose nucleic acid sequences are provided in this disclosure. Finally, the configuration and composition of the different SARs of the disclosure can be deduced from their names by those skilled in the art.

In an embodiment, the disclosure provides a novel SAR with the architecture and/or configuration represented by any of the exemplary SARs provided in **Tables 25-36 and 41-50.** In an embodiment, the disclosure provides a novel SAR with the composition of any of the exemplary SARs provided in **Tables 25-36** and **41-50** or a functional variant thereof. In an embodiment, the disclosure provides a novel SAR with at least 70% homology (*e.g.,* 70%, 75%, 80%, 85%, 90%, 95%, 98%, 99% homology) to the amino acid sequence of any of the exemplary SARs provided in **Tables 25-36 and 41-50.** In an embodiment, the disclosure provides a novel SAR with at least 70% homology (*e.g.,* 70%, 75%, 80%, 85%, 90%, 95%, 98%, 99% homology) to the amino acid sequence of any of the exemplary SARs provided in **Tables 25-36** and **41-50** excluding the optional accessory modules. The nucleic acid and amino acid sequences of the SARs in Tables **25-36** and **41-50** include their signal peptides. However, as the signal peptides are removed from the mature SAR polypeptide chains, the sequences encoding the signal peptides are excluded for the purpose of determining the homology. Similarly, the sequence encoding the accessory modules and/or any cleavage linkers (*e.g.,* P2A, F2A *etc.*) or furine cleavage sites are excluded for the purpose of determining sequence homology. Thus, in an embodiment, the disclosure provides a novel SAR with at least 70% homology (*e.g.,* 70%, 75%, 80%, 85%, 90%, 95%, 98%, 99% homology) to the amino acid sequence of any of the exemplary SARs provided in **Tables 25-36 and 41-50** in the regions comprising their antigen binding domain(s) and signaling chain(s) (*e.g*., TCR constant chains).

In one aspect, the disclosure relates to the use of autonomous antigen binding domains (AABD) including human V_{H} domains, typically multiple human V_{H} domains, as building blocks to make SARs with advantageous antigen binding domains.

The present disclosure relates in one aspect to an autonomous antigen binding domain (AABD), methods of generating the same and uses of such domains for construction of synthetic antigen receptors and potentially antibody therapeutics. In one embodiment, the AABD domain has improved stability. In another embodiment, the AABD domain has improved thermal stability. In another embodiment, the AABD domain has improved solubility. In another embodiment, the AABD domain has less tendency for self-aggregation. In another embodiment, the AABD domain has improved ability to be secreted in the extracellular space when expessed in a mammalian cell with an N-terminal signal peptide.

In one aspect the AABD is a single domain antibody or antibody fragment. In one aspect, an AABD is a single variable heavy chain (VH or vH) domain **(SVH** domain) or a fragment thereof that is capable of binding the antigen in the absence of a variable light chain (VL or vL) domain. In another aspect, the AABD is a single variable heavy chain (VH) domain (or a SVH domain) of a fragment thereof that can be expressed as a soluble protein in the absence of a vL domain. In another aspect, the AABD is a single variable heavy chain (VH) domain (or a SVH domain) or a fragment thereof that can be expressed as a secreted protein in the absence of a vL domain when joined to an N-terminal secretory signal. Certain embodiments of present disclosure relate to a SAR comprising a first AABD, the first AABD specifically binding to an antigen in the absence of a second domain.

In one aspect the AABD is a single variable light chain (VL or vL) domain or a SVL domain or a fragment thereof that is capable of binding the antigen in the absence of a variable heavy chain (VH or vH) domain. In another aspect, the AABD is a single variable light chain (VL) domain (or a SVL domain) or a fragment thereof that can be expressed as a soluble protein in the absence of a vH domain. In another aspect, the AABD is a single variable light chain (VL) domain (or a SVL domain) or a fragment thereof that can be expressed as a secreted protein in the absence of a vH domain when joined to an N-terminal secretory signal.

In an embodiment, an AABD is a non-scFv based antigen binding domain or a fragment thereof.

In an embodiment, an AABD is a camelid vHH domain or a humanized vHH domain or a fragment thereof.

In an embodiment, an AABD is a non-immunoglobulin antigen binding scaffold or a fragment thereof.

In an embodiment, an AABD is a cytokine or a ligand or a fragment thereof.

In an embodiment, an AABD is the extracellular domain of a receptor or a fragment thereof.

In an embodiment, an AABD is a single variable domain T cell receptor (TCR) or a fragment thereof.

In an embodiment, an AABD is an autoantigen or a fragment thereof.

In an embodiment, an AABD is an adaptor domain, an adaptor binding domain or a fragment thereof. Exemaplary adaptors and adaptor binding domain include but are not limited to: RZIP, EZIP, E4, K4, NKG2D-YA, NKG2D-AF, D domains and the like.

The terms "single domain antibody, variable single domain or immunoglobulin single variable domain (ISV)" are all well known in the art and describe the single variable fragment of an antibody that binds to a target antigen. These terms are used interchangeably herein. As explained below, preferred embodiments of the various aspects of the disclosure relate to SARs comprising single heavy chain variable domain antibodies/immunoglobulin heavy chain single variable domains, designated SVH domains, which bind to different antigens, such as CD19, CD20, CD22, BCMA, CD38, MPL, CD123, CD33, Mesothelin, Her2, CS1/SLAMF7, CD30, GD2, GD3, FLT3, ROR1, CD79b, Lym1, Lym2, PSCA, PSMA, ALK, CD138, CEA, FAP, TAJ, CD229, IL13Ra2, CD32b, GPC3, Muc16 and KIR3DL2 in the absence of light chain. Human heavy chain single variable domain antibodies are particularly preferred.

Thus, in some preferred embodiments, the SARs of the disclosure comprise a binding domain that comprises or consist of a single domain antibody wherein said domain is a single human heavy chain variable domain (SVH). Thus, in a preferred aspect, the SARs of the disclosure comprise one or more binding domain that is devoid of V_{L} domains.

Thus, in some preferred embodiments, the SARs of the disclosure comprise a binding domain that comprises or consist of a single domain antibody wherein said domain is a camelid vHH (or VHH) domain or humanized vHH domain.

As used herein, the V_{H} domain is a human V_{H} domain or a non-human V_{H} domain. The term "a human V_{H} domain" includes a V_{H} domain that is derived from or based on a human V_{H} domain amino acid or nucleic acid sequence. Thus, the term includes variable heavy chain regions derived from human germline immunoglobulin sequences. As used herein, the term human V_{H} domain includes V_{H} domains that are isolated from transgenic mice expressing human immunoglobulin V genes, in particular in response to an immunisation with an antigen of interest, for example as described in WO 2016/062990. Such domains are typically fully human. In one embodiment, a human V_{H} domain can also include a V_{H} domain that is derived from or based on a human V_{H} domain amino acid or nucleic acid sequence encoding such V_{H} domain. Thus, the term includes variable heavy chain regions derived from or encoded by human germline immunoglobulin sequences. A substantially human V_{H} domain or V_{H} domain that is derived from or based on a human V_{H} domain may include amino acid residues not encoded by human germline immunoglobulin sequences (*e.g.,* mutations introduced *in vitro, e.g.,* by random or site-specific mutagenesis, or introduced by somatic mutation *in vivo*)*.* The term "human V_{H} domain" therefore also includes a substantially human V_{H} domain wherein one or more amino acid residue has been modified. For example, a substantially human V_{H} domain the V_{H} domain may include up to 10, for example 1, 2, 3, 4 or 5 amino acid modifications compared to a fully human sequence.

As used herein, the term VH, V_{H} or "variable domain" refers to immunoglobulin variable domains defined by Kabat et al., Sequences of Immunological Interest, 5th ed., U.S. Dept. Health & Human Services, Washington, D.C. (1991). The numbering and positioning of CDR amino acid residues within the variable domains is in accordance with the well-known Kabat numbering convention.

In one aspect, the novel AABD of a SAR is a single variable heavy chain (VH) domain or a **SVH domain.** The term SVH domain as used herein refers to a single human VH domain antibody (VH sdAb). These terms are thus used interchangeably. The term SVH is also used interchangeably with independent vH domains or autonomous vH domains. The disclosure relates to the use of human, typically multiple human SVH domains as building blocks to make SARs.

The SVH domains are small molecules of 12-14 kDa which can be combined into different formats to give multivalent or multispecific antigen binding domains of a SAR. SVH domains are robust and are characterised by high affinity and stability in serum. SVH domains are also characterised by high solubility in serum and lack of aggregation.

Each single V_{H} domain (SVH) antibody comprises three CDRs and four FRs, arranged from amino-terminus to carboxy-terminus in the following order: FR1-CDR1-FR2-CDR2-FR3-CDR3-FR4. Thus, in one embodiment of the invention, the domain is a human variable heavy chain (V_{H}) domain with the following formula FR1-CDR1-FR2-CDR2-FR3-CDR3-FR4.

In an embodiment, the present disclosure provides single-chain and multichain SARs (*e.g.,* SIRs, cTCRs Ab-TCR, TCR, αβTFP, or γδTFP *etc.*) that can be constructed using SVH domains having a W103R substitution according to Kabat system. An exemplary SVH targeting CD19 with W103R substitution is CD19-FHVH-354 and is represented by SEQ ID NO (DNA): 836 and SEQ ID NO (PRT): 11526 (Table 5). Additional exemplary AABD comprising a SVH with a W103R substitution are listed in Table 5.

In another embodiment, the disclosure provides multichain SARs (*e.g*., SIRs, cTCRs Ab-TCR, αβTFP, γδTFP or recombinant TCR *etc.*) having bispecific, bivalent or biparatopic antigen binding moieties that comprise SVH domains having a W103R substitution according to Kabat system. Exemplary SARs targeting different antigens based on the SIR backbone and comprising CD19-FHVH-354 as one of the antigen binding domains are presented in Table 25.

In another embodiment, the present disclosure provides multichain SARs (e.g, SIRs, cTCRs Ab-TCR, αβTFP, γδTFP or recombinant TCR *etc.*) having multispecific, multiivalent or multiparatopic antigen binding moieties that comprise SVH domains having a W103R substitution according to Kabat system. Exemplary bispecific and trispecific SARs targeting different antigens based on the SIR, zSIR and Ab-TCR backbones and comprising CD19-FHVH-354 as one of the antigen binding domains are presented in Table 26. As AABD are modular in nature, the CD19-FHVH-354 can be substituted by other AABD targeting different antigens to develop SARs targeting those antigens.

In another embodiment, the present disclosure provides single chain SARs (e.g, CARs, TFPs *etc.*) having bispecific, bivalent or biparatopic antigen binding moieties that comprise SVH having a W103R substitution according to Kabat system. An exemplary single chain bispecific SAR targeting CD22 and CD19 comprising CD22-FHVH-24 as one of the antigen binding domains is CD8SP-CD22-FHVH-24-G4Sx3-R1-hu-mROO5-1-vL-Gly-Ser-Linker-hu-mROO5-1-vH-Myc-28z (SEQ ID NO (DNA): 4118 and SEQ ID NO (PRT): 14808). In this SAR construct, the CD22-FHVH-24 is operably linked to the N-terminus of hu-mROO5-1 scFV via a Glycine-Serine (G4Sx3) linker. The scFV is fused in frame to a human CD28 hinge and transmembrane domain via a Myc linker. The SAR also carries a CD3z activation domain at its C-terminus. SARs with similar modular architecture are also within the scope of the present disclosure.

In another embodiment, the present disclosure provides single chain SARs (e.g, CARs, TFPs *etc.*) having multispecific, multiivalent or multiparatopic antigen binding moieties comprising SVH having a W103R substitution according to Kabat system.

In an embodiment, the present disclosure provides that single chain and multichain SARs can be constructed using SVH stabilized by the introduction of non-canonical cysteines, which are capable of forming disulfide bonds and/or form disulfide bridges under suitable conditions. An exemplary SVH comprising non-canonical cysteins is CEA-300-aVH and is provided in SEQ ID NO (DNA): 954 and SEQ ID NO (PRT): 11644. Additional exemplary such SVH are provided in WO2019149715, which is incorporated in its entirety by reference herein. An exemplary SAR incorporating CEA-300-aVH is represented by SEQ ID NO: 21956.

In one embodiment, the present disclosure is aimed at mitigating the shortcomings of existing adoptive cellular therapies by providing single chain SARs (*e.g*., CARs, TFPs *etc.*) comprising SVH where the SVH domains contains the substitution cysteines in positions (i) 52a and 71 or (ii) 33 and 52 according to Kabat numbering, wherein said cysteines are capable of forming a disulfide bond and/or form a disulfide bond under suitable conditions.

In an embodiment of the disclosure, the SVH domain used to make a SAR comprises a substitution selected from the group consisting of 44E, 45E, 45 R, (101-1)Y and 101D according to Kabat numbering. Particularly, the SVH comprises the substitutions 44E, 45E or 45R, (101-1)Y and 101D according to Kabat numbering. In an embodiment, the SVH domain comprises a substitution selected from the group consisting of G44E, T45E, T45 R, F(101-l)Y and A101D according to Kabat numbering. In an embodiment, the SVH domain comprises the substitutions G44E, T45E, T45 R, F(101-1)Y and A101D according to Kabat numbering.

In an embodiment of the disclosure, the SAR comprises an SVH with substitution selected from the group consisting of 44E, 45E and (101-l)Y according to Kabat numbering. In an embodiment, the SAR comprises an SVH domain with the substitutions 44E, 45E, and (101-1)Y according to Kabat numbering. In an embodiment, the SVH domain comprises a substitution selected from the group consisting of G44E, T45E and F(101-l)Y according to Kabat numbering, if present in the SVH domain. In an embodiment, the SAR comprises an SVH domain comprising the substitutions G44E, T45E, and F(101-1)Y according to Kabat numbering.

In an embodiment, the SVH domain of the SAR comprises a vH framework comprising a FR1, FR2, FR3 and FR4 with at least 85% sequence identity to the amino acid sequence of SEQ ID NOs: 21411, 21412, 21413 and 21414, respectively.

In an embodiment, the SVH domain of the SAR comprises a vH framework comprising a FR1, FR2, FR3 and FR4 with at least 85% sequence identity to the amino acid sequence of SEQ ID NO: 21415, 21416, 21417, and 21418, respectively.

In an embodiment, the SVH domain of the SAR comprises a vH framework comprising a FR1, FR2, FR3 and FR4 with at least 85% sequence identity to the amino acid sequence of SEQ ID NO: 21419, 21420, 21421, and 21422, respectively.

In an embodiment of the disclosure, the SVH domain of the SAR comprises a vH framework comprising a FR1, FR2, FR3 and FR4 with at least 85% sequence identity to the amino acid sequence of SEQ ID NO: 21423, 21424, 21425, and 21426, respectively.

In an embodiment of the disclosure, the SVH domain comprises a vH framework comprising a FR1, FR2, FR3 and FR4 with at least 85% sequence identity to the amino acid sequence of SEQ ID NO: 21427, 21428, 21429, and 21430, respectively.

In an embodiment of the disclosure, the SVH domain comprises a vH framework comprising a FR1, FR2, FR3 and FR4 with at least 85% sequence identity to the amino acid sequence of SEQ ID NO: 21431, 21432, 21433, and 21434, respectively.

In an embodiment, the SVH domain comprises a vH framework comprising a FR1, FR2, FR3 and FR4 with at least 85% sequence identity to the amino acid sequence of SEQ ID NO: 21435, 21436, 21437, and 21438, respectively.

In an embodiment, the SVH domain comprises a vH framework comprising a FR1, FR2, FR3 and FR4 with at least 85% sequence identity to the amino acid sequence of SEQ ID NO: 21439, 21440, 21441, and 21442, respectively.

The SVH domain is particularly useful for construction of a SAR, as FR1-4 according to SEQ ID NOs 21411-21442 are not immunogenic in humans.

The disclosure also relates to the use of human, typically multiple human SVH domains as building blocks to make SARs. The term SVH domain as used herein refers to a single human VH domain antibody (VH sdAb). These terms are thus used interchangeably. The term SVH is also used interchangeably with independent vH domains or autonomous vH domains. An SVH is a type of AABD.

The SVH domains are small molecules of 12-14 kDa which can be combined into different formats to give multivalent or multispecific antigen binding domains of a SAR. SVH domains are robust and are characterised by high affinity and stability in serum. SVH domains are also characterised by high solubility in serum and lack of aggregation.

In some embodiments, the SAR constructs described herein include a human SVH domain (typically multiple human SVH domains) that recognizes a target protein of interest, *e.g.,* a protein expressed on a tumor cell, such as an antigen.

In some embodiments, the SAR constructs described herein include a **human SVL** domain (typically multiple human SVL domains) that recognizes a target protein of interest, *e.g.,* a protein expressed on a tumor cell, such as an antigen. The term SVL domain as used herein refers to a single human VL domain antibody (VL sdAb). These terms are thus used interchangeably. The term SVL is also used interchangeably with independent vL domains or autonomous vL domains. An SVL is a type of AABD.

In one aspect, the AABD of a SAR is a camelid vHH domain. The disclosure also relates to a SAR comprising multiple vHH domains. The disclosure also relates to a SAR comprising humanized vHH domains. Exemplary vHH domains that can be used in the construction of the SAR of the disclosure and their target antigens are presented in **Table 5.**

In one aspect, the AABD of a SAR is a non-immunoglobulin antigen binding scaffold, such as DARPIN, an affibody, an affilin, an adnectin, an affitin, an obodies, a repebody, a fynomer, an alphabody, an avimer, an atrimer, a centyrin, a pronectin, an anticalin, a kunitz domain, an Armadillo repeat protein, D domain, or a fragment thereof. The disclosure also relates to a SAR comprising multiple non-immunoglobulin antigen binding scaffold. Exemplary non-immunoglobulin antigen binding scaffold that can be used in the construction of the SARs of the disclosure and their target antigens are presented in **Table 7-9.**

In one aspect, the AABD of a SAR is an adaptor binding domain (*e.g*., RZIP, EZIP, E4, K4, NKG2D-AF, NKG2D-YA, or D domain *etc.*)*.* The disclosure also incudes SARs that bind to multiple adaptors. In an embodiment, an adaptor binding domain is a leucine zipper domain. In one aspect, the AABD of a SAR binds to an adaptor (*e.g.,* RZIP, EZIP, E4, K4, D domain, Streptag, FITC, Biotin, ULBP2R, ULBP2-S3 *etc.).* It is understood by those skilled in the art that an adaptor and an adaptor binding protein can be substituted for each other. Thus, a SAR can comprise an RZIP module that binds to a SAR adaptor comprising an EZIP module. Alternatively, the SAR may comprise an EZIP module while the SAR adaptor may comprise an RZIP module. The disclosure also incudes SARs that bind to multiple adaptors. Exemplary adaptors and adaptor binding proteins are shown in **Table 10.**

In one aspect, the AABD of a SAR is the extracellular ligand-binding domain of a receptor or a fragment thereof. The disclosure also incudes SARs that comprise multiple extracellular ligand binding domains of receptors. Exemplary extracellular ligand-binding domains of receptors that can be used in the construction of a SAR are provided in **Table 8.**

In one aspect, the AABD of a SAR is the extracellular receptor-binding domain of a ligand or a cytokine or a fragment thereof. The disclosure also incudes SARs that comprise multiple extracellular receptor binding domains of ligands or cytokines. Exemplary extracellular receptor-binding domains of ligands or cytokines that can be used in the construction of a SAR are provided in **Table 9.**

In one aspect, the AABD of a SAR is an autoantigen. The disclosure also incudes SARs that comprise multiple auto-antigens. An exemplary auto-antigen that can be used in the construction of a SAR is Dsg3 (SEQ ID NO: 11687) or a fragment thereof.

In one aspect, the AABD of a SAR is a single variable domain of a T cell receptor (svd-TCR). The disclosure also incudes SARs that comprise multiple single variable domains of T cell receptors. Exemplary polynucleotides comprising svd-TCR domains are provided in SEQ ID NO (DNA): 21563-21564 and in WO2021030182 which is incorporated in its entirety by reference herein.

In one aspect, the AABD of a SAR is any protein that can bind to an antigen expressed on the surface of a cell.

The multiple AABD in a SAR could be present in different combinations (*e.g*., two Centyrins, one Centyrin and one vHH domain, vHH domain and a SVH domain and a Centyrin *etc.*)

In one aspect, the AABD of a SAR is a Centyrin. The disclosure also relates to a SAR comprising multiple Centyrins. In one aspect, the AABD of a SAR is a DARPINS. The present disclosure also relates to a SAR comprising multiple DARPINs. Similarly, the present disclosure relates to SARs containing multiple non-immunoglobulin antigen binding domains such as affibodies, affilins, adnectins, affitins, obodies, repebodies, fynomers, alphabodies, avimers, atrimers, pronectins, anticalins, kunitz domains, Armadillo repeat proteins.

In some embodiments, the SAR contains multiple AABDs. In an embodiment, the first AABD is linked to a second AABD, wherein the first and second AABD specifically bind antigens. In an embodiment, the antigens recognized by the SAR are peptide antigens that are bound to MHC complex. In some embodiments, the two or more AABD of a SAR recognize the same antigen. In other embodiments, the two or more AABD of the SAR recognize different antigens.

Whilst scFv typically used in SARs, such as 2^{nd} generation CARs, have the potential for unwanted aggregation, cluster formation and immunogenicity, the use of Autonomous Antigen Binding Domains (AABD) provides a stable format with substantially reduced potential for immunogenicity, non-specific-aggregation or unfolding. This is particularly useful when designing SARs having bispecific, bivalent or biparatopic antigen binding moieties. As demonstrated by the inventors herein, multiple AABD domains can readily be used in such mutimeric format thus facilitating the generation of multispecific SARs that enable simultaneous targeting of more than one target antigen or more than one epitope of an antigen.

In an embodiment, the disclosure provides a SAR that can target one or more than 1 antigen (*e.g*., 1, 2, 3, 4, 5, 6 or more antigens). In an embodiment, the disclosure provides a SAR that can target one or more than 1 epitope (*e.g.,* 1, 2, 3, 4, 5, 6 or more epitopes). In an embodiment, the disclosure provides a SAR that comprise one or more than 1 antigen binding domains (*e.g*., 1, 2, 3, 4, 5, 6 or more antigen-binding domains).

In an embodiment, the disclosure provides a SAR that comprises one or more than one chain with each chain comprising zero, one or more antigen binding domains operably linked to a transmembrane domain, an optional activation domain and an optioinal co-stimulatory domain. In an embodiment, the activation domain encodes for one or more ITAM motifs.

In an embodiment, the disclosure provides a single chain SAR that comprises one or more antigen binding domains operably linked to a transmembrane domain, an activation domain and an optioinal co-stimulatory domain. In an embodiment, the activation domain encodes for one or more ITAM motifs. An exemplary such a SAR is a SAR represented with the backbone of 1^{st} generation CAR, 2^{nd} generation CAR, 3^{rd} generation CAR, or εTFP, γTFP, δTFP, or ζTFP. Exemplary such SARs are represented by SEQ ID NO (DNA): 4087, 4110-4113, 4118-4120, SEQ ID NO (PRT): 14777, 14800-14803 and14808-14810.

The disclosure also provides single chain SARs based on the 2^{nd} generation CAR, K13-CAR and TFP (*e.g.,* TFPε, TFPγ, TFPδ) backbones **(Table 33 and 34)**. The disclosure provides single chain SARs with the modular domain structure and architecture of an exemplary SAR on the backbone of a 2^{nd} generation CAR targeting BCMA and CD19 represented by CD8SP-BCMA-FHVH-93-SG4S-CD19-FHVH-354-Myc-28z (SEQ ID NO: 17667). Another exemplary SAR on the backbone of a 2^{nd} generation CAR targeting BCMA and CD19 is CD8SP-CD19-FHVH-354-SG4S-BCMA-FHVH-93-Myc-CD8TM-BBz (SEQ ID NO: 17669). Exemplary SARs on the backbones of TFPs targeting BCMA and CD19 are CD8SP-BCMA-FHVH-93-SG4S-CD19-FHVH-354-CD3e-ECDTMCP-opt2 (SEQ ID NO: 17696), CD8SP-BCMA-FHVH-93-SG4S-CD19-FHVH-354-CD3d-ECDTMCP-opt2 (SEQ ID NO: 17697), and CD8SP-BCMA-FHVH-93-SG4S-CD19-FHVH-354-CD3g-ECDTMCP-opt2 (SEQ ID NO: 17698). The disclosure provides single chain SARs with the modular domain structure and architecture of additional exemplary SARs on the backbones of TFPs targeting BCMA and CD19 as represented by SEQ ID NO: 17700- 17703.

The disclosure provides single chain SARs with the modular domain structure and architecture of exemplary SARs on the backbone of 2^{nd} generation CARs targeting CD20 and CD22 represented by CD8SP-CD20-vHH-USC1-2HC2D6-SG4S-CD22-USC1-FHVH-160-Myc-28z and CD8SP-CD22-USC1-FHVH-160-SG4S--CD20-vHH-USC1-2HC2D6-Myc-CD8TM-BBz, which are represented by SEQ ID NO: 17813 and 17815. The disclosure provides SARs with the modular domain structure and architecture of exemplary SAR on the backbone of a 1st generation CAR co-expressing K13 and targeting CD20 and CD22 represented by CD8SP-CD20-vHH-USC1-2HC2D6-SG4S-CD22-USC1-FHVH-160-Myc-CD8TM-z-P2A-K13-FLAG-T2A-PAC (SEQ ID NO: 17816). The disclosure provides SARs with the modular domain structure and architecture of exemplary SARs on the backbone of TFPs targeting CD20 and CD22 as represented by SEQ ID NO: 17842- 17849.

Exemplary SARs on the backbone of 2^{nd} generation CARs targeting BCMA and CD38 are CD8SP-BCMA-FHVH-74-SG4S-CD38-FHVH-USC1-32184-Myc-28z and CD8SP-CD38-FHVH-USC1-32184-SG4S-BCMA-FHVH-74-Myc-CD8TM-BBz and are represented by SEQ ID NO: 17886 and 17888. An exemplary SAR on the backbone of a 1st generation CAR co-expressing K13 and targeting BCMA and CD38 is CD8SP-BCMA-FHVH-74-[hTCRb-S57C]-F-P2A-SP-CD38-FHVH-USC1-32184-[hTCRa-T48C]-F-F2A-K13-opt (SEQ ID NO: 17893). Exemplary SARs on the backbone of TFPs targeting BCMA and CD38 are represented by SEQ ID NO: 17915- 17922.

Exemplary SARs on the backbone of 2^{nd} generation CARs targeting CD20 and CD19 are CD8SP-CD20-vHH-USC1-2HC2D6-SG4S-CD19-FHVH-354-Myc-28z and CD8SP-CD20-vHH-USC1-2HC2D6-SG4S-CD19-FHVH-354-Myc-CD8TM-BBz and are represented by SEQ ID NO: 17959-17960. An exemplary SAR on the backbone of a 1st generation CAR co-expressing K13 and targeting CD20 and CD19 is CD8SP-CD20-vHH-USC1-2HC2D6-SG4S-CD19-FHVH-354-Myc-CD8TM-z-P2A-K13-FLAG-T2A-PAC (SEQ ID NO: 17962). Exemplary SARs on the backbone of TFPs targeting BCMA and CD38 are represented by SEQ ID NO: 17988- 17995.

Exemplary SARs on the backbone of 2^{nd} generation CARs targeting CD22 and CEA are CD8SP-CD22-FHVH-24-SG4S-CEA-aVH-3001-Myc-28z and CD8SP-CD22-FHVH-24-SG4S-CEA-aVH-3001-Myc-CD8TM-BBz and are represented by SEQ ID NO: 17521-17522. An exemplary 1st generation CAR co-expressing K13 and an optional PAC module and targeting CD22 and CEA is CD8SP-CD22-FHVH-24-SG4S-CEA-aVH-3001-Myc-CD8TM-z-P2A-K13-FLAG-T2A-PAC (SEQ ID NO: 17524). Exemplary SARs on the backbone of TFPs targeting CD22 and CEA are represented by SEQ ID NO: 17550- 17557.

An exemplary double chain SAR on the backbone of an AABD-TCR targeting CD20 and CD22 is CD8SP-CD20-vHH-USC1-2HC2D6-IgG1-Hinge-[TCRg-6MD]-F-P2ASP-CD22-USC1-FHVH-160-IgG1-Hinge-v2-[TCRd-6MD] and is represented by SEQ ID NO: 17883. Exemplary SARs on the backbone of AABD-TCRs targeting CD20 and CD22 are CD8SP-CD20-vHH-USC1-2HC2D6-[IgCL-TCRb-wt-opt2-6MD]-F-P2A-SP-CD22-USC1-FHVH-160-[IgG1-CH1-TCRa-wt-opt2-6MD] (SEQ ID NO: 17868) and CD8SP-CD20-vHH-USC1-2HC2D6-[IgCL-TCRg-6MD]-F-P2A-SP-CD22-USC1-FHVH-160-[IgG1-CH1-TCRd-6MD](SEQ ID NO: 17877). Exemplary SARs on the backbone of AABD-TCRs targeting BCMA and CD38 are CD8SP-BCMA-FHVH-74-[IgCL-TCRg-6MD]-F-P2A-SP-CD38-FHVH-USC1-32184-[IgG1-CH1-TCRd-6MD] and CD8SP-BCMA-FHVH-74-[IgCL-TCRb-wt-opt2-6MD]-F-P2A-SP-CD38-FHVH-USC1-32184-[IgG1-CH1-TCRa-wt-op2-6MD] and are represented by SEQ ID NO: 17912 and 17914. Exemplary SARs on the backbone of AABD-TCRs targeting CD20 and CD19 are CD8SP-CD20-vHH-USC1-2HC2D6-[IgCL-TCRb-wt-opt2-6MD]-F-P2A-SP-CD19-FHVH-354-[IgG1-CH1-TCRa-wt-opt2-6MD] and CD8SP-CD20-vHH-USC1-2HC2D6-[IgCL-TCRg-6MD]-F-P2A-SP-CD19-FHVH-354-[IgG1-CH1-TCRd-6MD] and are represented by SEQ ID Nos: 18016 and 18023, respectively. As SARs are modular in nature, the AABD of the above SARs can be substituted with AABD targeting different antigens to develop SARs targeting those antigens. **Table 33** provides the names, module composition and SEQ ID NOs of SARs targeting CD22 and PSMA. Table 34 provides the SEQ ID Nos of SARs generated from SARS listed in Table 33 by replacing the AABD targeting CD22 (*i.e.,* CD22-FHVH-24) and PSMA (*i.e.,* PSMA-chVH-71v2) with AABD targeting different antigens. Otherwise, the order of the constructs in **Table 34** is the same as the order of the constructs in Table 33.

In an embodiment, the disclosure provides a synthetic antigen receptor, comprising (a) one or more antigen-specific targeting regions, (b) at least one extracellular linker domain, (c) at least one transmembrane domain, (d) an optional co-stimulatory domain and (e) an optional intracellular signaling domain, wherein one antigen-specific targeting region comprises an antigen-specific single chain Fv (scFv) fragment, and a second antigen specific targeting domain comprises an AABD. In an exemplary embodiment, the AABD is a non-scFv antigen binding module (*e.g.,* a SVH, vHH, FHVH, SVL, svd-TCR, Centyrin, DARPIN, CD16A, CD64, CD32, NKG2D, NKG2D-AF, NKG2D-YA, RZIP, EZIP, E4, K4, D domain *etc.*)*.*

In an embodiment, the disclosure provides a bispecific or a multispecific synthetic antigen receptor comprising (a) at least two antigen-specific targeting regions, (b) at least one extracellular linker domain, (c) at least one transmembrane domain, (d) an optional co-stimulatory domain and (e) an optional intracellular signaling domain, wherein one antigen-specific targeting region comprises an antigen-specific single chain Fv (scFv) fragment, and a second antigen specific targeting domain comprises an AABD. In an exemplary embodiment, the AABD is a non-scFv antigen binding module (*e.g.,* a SVH, vHH, FHVH, SVL, svd-TCR, Centyrin, DARPIN, CD16A, CD64, CD32, NKG2D, NKG2D-AF, NKG2D-YA, RZIP, EZIP, E4, K4, D domain *etc.*). In an embodiment, the disclosure provides a bispecific or a multispecific synthetic antigen receptor having the general formula: (AABD)n-optional linker domain-scFv-hinge domain-transmembrane domain-optional one or more costimulatory domains-an activation domain; where n = 0, 1, 2, 3, 4, 5 or more and where the activation domain may contain one or more ITAM motifs. An exemplary such SAR is represented by IgSP-Apa-CD20-vHH-2HCD25-G4Sx3v2-hu-mROO5-1-vH-Gly-Ser-Linker-vL-Myc-CD8TM-BBz and has SEQ ID NO (DNA): 4087. This SAR has one antigen binding domain represented by humanized hu-mROO5-1 scFv that targets CD19 and a second antigen binding domain represented by CD20-vHH-2HCD25 that targets CD20. The two antigen binding domains are linked via a Gly-Ser (G4Sx3v2) flexible linker. This SAR construct also comprise a Myc tag, a human CD8 hinge and transmembrane domain, a 4-1BB costimulatory domain and a CD3z activation domain. Other exemplary such constructs are represented by SARs with the backbone of 1^{st} generation CAR, 2^{nd} generation CAR, 3^{rd} generation CAR, or εTFP, γTFP, δTFP, or ζTFP. Exemplary such SARs are represented by SEQ ID NO (DNA): 4087, 4110-4113, 4118-4120, SEQ ID NO (PRT): 14777, 14800-14803 and14808-14810.

In an embodiment, the disclosure provides a SAR that comprises one or more than one chain with each chain comprising zero, one, two or more antigen binding domains operably linked to a transmembrane domain but lacking an activation domain. Although such a SAR lacks an activation domain of its own, it is capable of signal transduction by recruitment of a signaling module comprising protein(s) that encode an activation domain. An exemplary such a SAR is a SAR based on the backbone of a SIR, cTCR, Ab-TCR, TCR, αβTFP or γδTFP. In an exemplary embodiment, the disclosure provides a SAR in which one or more AABD are attached to the N-terminus or near the N-terminus of one or both chains of a SIR, cTCR, Ab-TCR, TCR, αβTFP or γδTFP. In an exemplary embodiment, the disclosure provides a SAR in which one or more AABD are attached to the N-terminus or near the N-terminus of the vL or vH fragments comprising one or both chains of a SIR, cTCR, Ab-TCR, αβTFP or γδTFP. In an exemplary embodiment, the disclosure provides a SAR in which one or more AABD are attached to the N-terminus or near the N-terminus of the Va, Vb, Vg or Vd fragments comprising one or both chains of a TCR. Exemplary such SARs are represented by SEQ ID NO (DNA): 4088-4098, 4107-4109, 4166, and 3500-3510 and SEQ ID NO: (PRT): 14778-14788, 14797-14799, 14856 and 14190-14200. Additional exemplary such SARs are presented in **Tables 25-32**. In an exemplary embodiment, the AABD is a non-scFv antigen binding module (*e.g.,* a SVH, vHH, FHVH, SVL, svd-TCR, Centyrin, DARPIN, CD16A, CD64, CD32, NKG2D, NKG2D-AF, NKG2D-YA, RZIP, EZIP, E4, K4, D domain *etc.*)*.*

In an embodiment, the disclosure provides a single chain, one and a half chain or double chain SAR comprising at least one chain that has the general formula: (AABD)n-optional linker domain-scFv-optional linker-TCR constant chain; where n = 0, 1, 2, 3, 4, 5 or more and where TCR constant chain represents the constant chain of TCRα, TCRβ1, TCRβ2, TCRγ, TCRδ, pre-TCRα or a mutant or a variant thereof. Exemplary such SARs are represented by SEQ ID NO (DNA): 4127 and 4128 and SEQ ID NO (PRT): 14817-14818. In an embodiment, the scFv is replaced by a single chain soluble TCR comprising the Va and Vb fragments of an αβTCR joined by a flexible linker or Vg and Vd fragments derived from a γδTCR joined via a flexible linker.

In an embodiment, the disclosure provides a one and a half chain SAR or a double chain SAR comprising one chain that has the general formula: (AABD)n-optional linker domain-scFv-optional linker-TCR constant chain; and the second chain that has the general formula (AABD)n-optional linker domain-TCR constant chain where n = 0, 1, 2, 3, 4, 5 or more and where TCR constant chain represents the constant chain of TCRα (Cα), TCRβ1 (Cβ1), TCRβ2 (Cβ2), TCRγ (Cγ), TCRδ (Cδ) or pre-TCRα (pre-Cα) or a mutant or a variant thereof. In an embodiment, the two chains of the SAR comprise complementary TCR constant chains; *i.e.,* Cα and Cβ, pr*etc*.Rα and Cβ, or Cγ and Cδ. The Cβ chain could represent either Cβ1 or Cβ2. Exemplary such SARs are represented by SEQ ID NO (DNA): 4127 and 4128 and SEQ ID NO (PRT): 14817-14818. In an embodiment, the scFv is replaced by a single chain soluble TCR.

In an embodiment, the disclosure provides a one and a half chain synthetic antigen receptor comprising two TCR constant chains or fragment thereof where one of the chains comprises an AABD joined via an optional linker to an antigen-specific single chain Fv (scFv) fragment, which in turn is joined to a TCR constant chain or fragment thereof, and the second chain comprises a complementary TCR constant chain of a fragment thereof. In an exemplary embodiment, the AABD is a non-scFv antigen binding module *(e.g.,* a SVH, vHH, FHVH, SVL, svd-TCR, Centyrin, DARPIN, CD16A, CD64, CD32, NKG2D, NKG2D-AF, NKG2D-YA, RZIP, EZIP, E4, K4, D domain *etc.*)*.* An exemplary such SAR is represented by CD8SP-MYC-[hTCRa-T48C-opt1]-F-F2A-SP-CD22-FHVH-24-G4Sx3-R1-hu-mROO5-1-vL-Gly-Ser-Linker-hu-mROO5-1-vH-V5-[hTCRb-S57C-opt1]-F-P2A-PAC and has SEQ ID NO (DNA): 4128 and SEQ ID NO (PRT): 14818. This SAR has the backbone of a one and a half chain SIR (see WO2018102795) and comprises one chain encoding a MYC tag joined in frame to a mutated human TCRα constant chain (hTCRa-T48C-opt1; SEQ ID NO: 11738). The second chain of this SAR comprises one antigen binding domain represented by hu-mROO5-1 scFv (SEQ ID NO (DNA): 633 and SEQ ID NO (PRT): 11323) that targets CD19 and a second AABD represented by the fully-human vH domain CD22-FHVH-24 that targets CD22 (SEQ ID NO (DNA): 843 and SEQ ID NO (PRT): 11533). The two antigen binding domains are linked via a Gly-Ser (G4Sx3) flexible linker. The hu-mROO5-1 scFv is joined in frame to a mutated human TCRβ constant chain carrying a S57C mutation (hTCRb-S57C-opt1; SEQ ID NO (PRT): 11749) module via a V5 tag. The two chains are encoded by a single polynucleotide and are separated by a P2A cleavable linker. The SAR also encodes for a puromycin resistance gene (PAC), which is optional. Another such exemplary SAR construct is represented by CD8SP-V5-[hTCRb-KACIAH]-F-P2A-CD8SP-CD22-FHVH-24-G4Sx3-R1-hu-mROO5-1-vL-Gly-Ser-Linker-hu-mROO5-1-vH-Myc-[hTCRa-CSDVP]-F-F2A-PAC and is represented by SEQ ID NO (DNA): 4127 and SEQ ID NO (PRT): 14817. This SAR also has the backbone of a one and a half chain SIR (see WO2018102795) and comprises one chain encoding a V5 tag joined in frame to a mutated human TCRβ constant chain (hTCRb-KACIAH; SEQ ID NO: 11750). The second chain of this SAR comprises one antigen binding domain represented by hu-mROO5-1 scFv that targets CD19 and a second antigen binding domain represented by CD22-FHVH-24 that targets CD22. The two antigen binding domains are linked via a Gly-Ser (G4Sx3) flexible linker. The humanized hu-mROO5-1 scFv is joined in frame to a mutated human TCRα constant chain (hTCRa-CSDVP; SEQ ID NO: 11734) module via a Myc epitope tag.

In an embodiment, the disclosure provides a bispecific synthetic antigen receptor, comprising (a) at least two antigen-specific targeting regions, (b) at least one extracellular linker domain, (c) at least one transmembrane domain, (d) an optional co-stimulatory domain and (e) an optional intracellular signaling domain, wherein the antigen specific targeting domains comprise AABD. In an exemplary embodiment, both AABDs are non-scFv antigen binding modules (*e.g.,* a SVH, vHH, FHVH, SVL, svd-TCR, Centyrin, DARPIN, CD16A, CD64, CD32, NKG2D, NKG2D-AF, NKG2D-YA, RZIP, EZIP, E4, K4, and D domain *etc.*)*.* An exemplary such SAR is CD8SP-CD22-FHVH-24-SG4S-PSMA-chVH-71v2-Myc-CD8TM-BBz-T2A-PAC (SEQ ID NO: 6464) and comprises a CD8 signal peptide, a CD22 targeting fully human vH domain (FVHV), a Ser-Glyx4-Ser linker, a PSMA targeting single vH domain, a Myc tag, a CD8 hinge and transmembrane region, a 4-1BB costimulatory domain and a CD3 activation domain.

In an embodiment, the disclosure provides a single chain, one and a half chain or double chain SAR comprising at least one chain that has the general formula: (AABD)n-optional linker domain-vL or vH fragment-optional linker-TCR constant chain; where n = 0, 1, 2, 3, 4, 5 or more and where TCR constant chain represents the constant chain of TCRα, TCRβ1, TCRβ2, TCRγ, TCRδ or pre-TCRα or a mutant or a variant thereof.

In an embodiment, the disclosure provides a double chain SAR comprising one chain with the general formula: (AABD)n-optional linker domain-vL fragment-optional linker-TCR constant chain; and the second chain with the general formula; (AABD)n-optional linker domain-vH fragment-optional linker-TCR constant chain where n = 0, 1, 2, 3, 4, 5 or more and where TCR constant chain represents the constant chain of TCRα (Cα), TCRβ1 (Cβ1), TCRβ2 (Cβ2), TCRγ (Cγ), TCRδ (Cδ) or pre-TCRα (pre-Cα) or a mutant or a variant thereof. In an embodiment, the two chains of the SAR comprise complementary TCR constant chains; *i.e.,* Cα and Cβ, pr*etc*.Rα and Cβ, or Cγ and Cδ. The Cβ chain could represent either Cβ1 or Cβ2.

In an embodiment, the disclosure provides a double chain bispecific synthetic antigen receptor comprising two chains, each chain comprising (a) one or more antigen-specific targeting regions, (b) at least one extracellular linker domain, (c) at least one transmembrane domain, (d) an optional co-stimulatory domain and (e) an optional intracellular signaling domain, wherein one antigen-specific targeting region comprises a vL and/or a vH fragment that is capable of combining with the vH and/or vL fragment present on the second chain to create a fragment variable (Fv), and the second antigen specific targeting domain comprises an AABD (*e.g.,* a vHH, SVH, Centyrin, affibody *etc.*)*.* In one embodiment, the Fv binds an antigen. In another embodiment, the Fv does not bind an antigen. In an embodiment, the Fv serves as the scaffold for the attachment of the second antigen specific targeting domain comprising an AABD. In an embodiment, the AABD is a non-scFv antigen binding domain.

An exemplary double chain bispecific SAR comprising two chains is CD8SP-hu-mROO5-1-vL-[hTCRb-S57C]-F-P2A-IgSP-Apa-CD20-USC1-vHH-2HCD26-G4Sx3v2-hu-mROO5-1-vH-[hTCRa-T48C] and is represented by SEQ ID NO (DNA): 7406 and SEQ ID NO (PRT): 18096. This SAR construct has the backbone of a SIR, which has been described in WO2018102795 and is incorporated in its entirety by reference herein. One chain of the SAR construct comprises the humanized hu-mROO5-1 vL fragment fused to the constant chain of human TCRb carrying S57C mutation (hTCRb-S57C) and the other chain of the SAR comprises the humanized hu-mROO5-1 vH fragment fused to the constant chain of human TCRa carrying the T48C mutation (hTCRa-T48C). The hu-mROO5-1 vL and hu-mROO5-1 vH fragments of the SAR together form a Fv targeting CD19. A vHH fragment targeting CD20 (CD20-USC1-vHH-2HCD26; SEQ ID NO: 841) is fused to the N-terminus of the hu-mROO5-1 vH fragment via a Glycine-Serine linker. Thus, the SAR targets CD19 via the hu-mROO5-1 Fv and targets CD20 via CD20-USC1-vHH-2HCD26. It is to be noted that SARs are modular in format. Therefore, it is possible to replace one module of a SAR with another module. For example, the hu-mROO5-1 vL and hu-mROO5-1 vH fragments can be replaced with vL/vH fragment targeting a different antigen. **Table 25** provides the targeted antigens, name, nucleic acid SEQ ID NOs and amino acid SEQ ID NOs of several exemplary bispecific SARs on the SIR backbone comprising vL/vH fragments targeting different antigens and also comprising a SVH targeting CD19 (CD19-FHVH-354). In the SARs listed in **Table 25,** the vL fragment is operably linked to the hTCRb-S57C chain and the vH fragment is operably linked to the hTCRa-T48C chain. SARs can be constructed in which vL fragment is operably linked to the hTCRa-T48C chain hTCRb-S57C chain and the vH fragment is operably linked to the hTCRb-S57C chain. Exemplary such SAR constructs targeting different antigens are represented by SEQ ID NO (DNA): 2014-2177 and SEQ ID NO(PRT): 12704-12868. The order of vL/vH fragments of these constructs is the same as the order of the vL/vH fragments of the constructs shown in **Table 25.** Thus, the SAR construct with BCMA-J6M0 vL/vH fragments is the 8th construct in **Table 25.** Therefore, the nucleic acid and amino acid SEQ ID NO of the SAR construct CD8SP-CD19-FHVH-354-G4S3-BCMA-J6M0-vL-[hTCRa-T48C]-F-F2A-SP-BCMA-J6M0-vH-[hTCRb-S57C] with BCMA-J6M0 vL/vH fragments can be calculated to be 2021 (*i.e.,* 2014+8) and 12711 (*i.e.,* 12704+8), respectively. It is to be noted that since SARs are modular in format, the hTCRb-S57C chain can be replaced by a deletion mutant and/or variant as long as it retains its biological activity (*e.g*., ability to associate with the TCRa chain and/or the TCR/CD3 complex and/or induce antigen dependent T cell signaling). The nucleic acid and amino acid SEQ ID NOs of exemplary deletion mutants and variants of TCRb chain are listed in **Table 12** (*e.g.,* SEQ ID NO(DNA): 1056-1076 and SEQ ID NO(DNA):1114-1127). Similarly, the hTCRa-T48C chain can be replaced by a deletion mutant and/or variant as long as it retains its biological activity (*e.g*., ability to associate with the TCRb chain and/or the TCR/CD3 complex and/or induce antigen dependent T cell signaling). The nucleic acid and amino acid SEQ ID NOs of exemplary deletion mutants and variants of TCRa chain are listed in **Table 12** (*e.g.,* SEQ ID NO (DNA): 1042-1052 and SEQ ID NO (DNA):1103-1113).

The disclosure also describes novel deletion mutants of TCRa and TCRb chains that can be used in the construction of SIRs and bispecific/multispecific SARs. An exemplary SIR comprising the deletion mutants of TCRa and TCRb constant chain is CD8SP-hu-mROO5-1-vL-[hTCRb-S57C-opt-d17]-F-P2A-SP-hu-mROO5-1-vH-[hTCRa-T48C-opt-d17] and is represented by nucleic acid SEQ ID NO: 1331 and amino acid SEQ ID NO: 12021. The SIR/SAR constructs with deletion mutants of TCRa and TCRb chains are smaller in size and therefore have the advantage of increasing the titer of the lentiviral/retroviral vector encoding them since the titer of the lentiviral/retroviral vector is inversely proportion to the insert size.

The disclosure provides the targeted antigens, SAR name, module composition, DNA SEQ ID NOs and amino acid SEQ ID NOs of several exemplary bispecific and trispecific SARs on different backbones (*i.e.*, having different TCR constant chains) and comprising vL/vH fragments targeting different antigens in **Table 26.** These SAR constructs comprise a fully human single vH domain (CD19-FHVH-354) targeting CD19 and/or a fully human single vH domain (CD22-FHVH-24) targeting CD22. The name and composition of the first construct of each series is shown in **Table 26,** which can be used to determine the TCR constant chain modules present in that series. For example, it can be determined from **Table 26** that in the constructs represented by nucleic acid SEQ ID Nos: 2178-2343 the vL fragment is operably linked to the [hTCRbECD-CD3zECDTMCP-opt] module while the vH fragment is operably linked to the [hTCRaECD-CD3zECDTMCP-opt2] module. As the order of the different construct in this series is the same as the order of the construct of the series shown in **Table 25,** the vL/vH fragment of the different constructs in this series can be determined from the construct with the same vL/vH fragment in **Table 25.** A similar approach can be used to determine the SEQ ID NOs of other constructs carrying different vL/vH fragments of the series listed in **Table 26** by referring to **Table 25.** Other SARs that are based on the modular architecture of the SARs described in **Tables 25-26** are also within the scope of this disclosure.

The nucleic acid and amino acid SEQ IDs of exemplary bispecific and trispecific SARs based on the vL and vH fragments derived from FMC63 scFv and carrying different TCR constant chains are provided in **Tables 26.** The nucleic acid and amino acid SEQ IDs of exemplary bispecific and trispecific SARs based on the vL and vH fragments derived from other scFv fragments can be derived by replacing the vL and vH fragments of FMC63 scFv with the vL and vH fragments comprising the SARs listed in **Table 25.** The order of the SAR constructs comprising different vL/vH fragments in **Table 26** is same as the order of SARs shown in **Table 25** for SARs.

Another exemplary double chain bispecific SAR comprising two chains is CD8SP-hu-mROO5-1-vL-[IgCL-TCRg-6MD]-F-P2A-IgSP-Apa-CD20-vHH-2HCD25-G4Sx3v2-hu-mROO5-1-vH-[IgG1-CH1-TCRd-6MD] and represented by SEQ ID NO (DNA): 5037 and SEQ ID NO (PRT): 15727. In this SAR construct one chain of the SAR comprises the hu-mROO5-1 vL fragment fused to the truncated constant chain of human TCRg (TCRg-6MD) via an IgCL linker and the other chain of the SAR comprises hu-mROO5-1 vH fragment fused to the truncated constant chain of human TCRd (TCRd-6MD) via an IgG1-CH1 linker. The hu-mROO5-1 vL and hu-mROO5-1 vH fragments of the SAR collectively form a Fv targeting CD19. A vHH fragment targeting CD20 (CD20-vHH-2HCD25) is fused to the N-terminus of the hu-mROO5-1 vH fragment via a Glycine-Serine linker. Thus, the SAR targets CD19 via the hu-mROO5-1 Fv and targets CD20 via CD20-USC1-vHH-2HCD25. **Table 26** provides the nucleic acid SEQ ID (2839-3003) and amino acid SEQ ID Nos (13529-13693) of several exemplary bispecific SARs in which the vL fragment is operably linked to the [IgCL-TCRg-6MD] module and vH fragment is operably linked to the [IgG1-CH1-TCRd-6MD] modules and comprising vL/vH fragments targeting different antigens. These SAR constructs also comprise a SVH targeting CD19 (CD19-FHVH-354). As the order of the different construct in this series is the same as the order of the construct of the series shown in **Table 25,** the vL/vH fragment of the different constructs in this series can be determined from the construct with the identical vL/vH fragment listed in **Table 25.** As SARs are modular in format, it is possible to replace one module with another or its variant as long as the functional activity of SAR (*i.e*., ability to bind to its target antigen, ability to induce T cell signaling upon antigen binding, ability to assemble into TCR/CD3 complex *etc.*) is retained. Thus, in an exemplary embodiment, the vL fragment of the SAR is operably linked to [IgG1-CH1-TCRd-6MD] module or its variant and the vH fragment is operably linked to [IgCL-TCRg-6MD] module or its variants. The IgCL and IgG1-CH1 linkers can be replaced with other linkers as long as the functional activity of SAR (*i.e*., ability to bind to its target antigen, ability to induce T cell signaling upon antigen binding, ability to assemble into TCR/CD3 complex *etc.*) is retained. Exemplary such linkers are provided in **Table 13.** In the preferred embodiment, one chain of the bispecific SAR comprises IgCL (SEQ ID NO:11832) or its variant while the complementary chain comprises the linker represented by SEQ ID NOs:11833-11847. **Table 13** also lists the components of TCR constant chains that can serve as linkers in the construction of SAR. Exemplary linkers based on TCRa/TCRα constant chain are represented by SEQ ID NO: 11848-11849, 11857-11859. Exemplary linkers based on TCRb/TCRβ constant chain are represented by SEQ ID NO: 11850-11851, 11856, 11860-11861. Exemplary linkers based on TCRg/TCRγ constant chain are represented by SEQ ID NO: 11852-11853, 11862, 11863. Exemplary linkers based on TCRd/TCRδ constant chain are represented by SEQ ID NO: 11854-11855, 11862, 11864-11865. In an embodiment, one chain of a double chain SAR comprises a linker based on TCRa chain while the other chain comprises a linker based on TCRb chain. In another embodiment, one chain of a double chain SAR comprises a linker based on TCRg/TCRγ chain while the other chain comprises a linker based on TCRd/TCRδ chain. In an embodiment, one chain of a double chain SAR comprises a linker based on TCRa chain while the other chain comprises a linker based on IgCL (SEQ ID NO:11832). Other linkers based on immunoglobulin like modules are known in the art and can be used in alternate embodiment of the disclosure. Finally, in addition to the TCR chains and module, the CD20-vHH-2HCD25 fragment can be replaced with another AABD targeting a different antigen or a different epitope of CD20. Exemplary AABD are provided in **Tables 5, 7-10.** Additional AABD (*e.g.*, vHH, SVH, Centyrins *etc.*) targeting different antigens are known in the art.

An exemplary double chain bispecific SAR comprising two chains is CD8SP-hu-mROO5-1-vL-[IgCL-TCRb-wt-opt2-6MD]-F-P2A-IgSP-Apa-CD20-vHH-2HCD25-G4Sx3v2-hu-mROO5-1-vH-[IgG1-CH1-TCRa-wt-op2-6MD] and represented by DNA SEQ ID NO: 5039 and PRT SEQ ID NO: 15729. This SAR is similar to the SAR construct CD8SP-hu-mROO5-1-vL-[IgCL-TCRg-6MD]-F-P2A-IgSP-Apa-CD20-vHH-2HCD25-G4Sx3v2-hu-mROO5-1-vH-[IgG1-CH1-TCRd-6MD] represented by SEQ ID NO (DNA): 5037 and SEQ ID NO (PRT): 15727 except that TCRg-MD and TCRd-MD modules are replaced by (TCRb-wt-opt2-6MD; SEQ ID NO: 1126) and (TCRa-wt-op2-6MD; SEQ ID NO: 1112) modules, respectively. Thus, the SAR targets CD19 via the hu-mROO5-1 Fv and targets CD20 via CD20-USC1-vHH-2HCD25. **Table 26** provides the DNA SEQ ID (3169-3333) and amino acid SEQ ID NOs (13859-14023) of several exemplary bispecific SARs in which the vL fragment is operably linked to the [TCRb-wt-opt2-6MD] module and vH fragment is operably linked to the [TCRa-wt-op2-6MD] modules and comprising vL/vH fragments targeting different antigens. These SARs also comprise a SVH targeting CD19 (CD19-FHVH-354; SEQ ID NO: 836). As the order of the different construct in this series is the same as the order of the construct of the series shown in **Table 25,** the vL/vH fragment of the different constructs in this series can be determined from the construct with the same vL/vH fragment listed in **Table 25.** As SARs are modular in format, it is possible to replace one module with another or its variant as long as the functional activity of SAR is retained. Thus, in an exemplary embodiment, the vL fragment of the SAR is operably linked to [TCRa-wt-op2-6MD] module or its variant and the vH fragment is operably linked to [TCRb-wt-opt2-6MD] module or its variants. Similarly, the CD20-vHH-2HCD25 fragment can be replaced with another AABD targeting a different antigen or a different epitope of CD20. Exemplary AABD are provided in **Tables 5, 7-10.** Additional AABD (*e.g*., vHH, SVH, Centyrins *etc.*) targeting different antigens are known in the art.

Another exemplary double chain bispecific SAR comprising two chains is CD8SP-CD38-USC1-FHVH-32184-G4Sx3-R1-hu-mROO5-1-vL-[hTCRb-S57C]-F-P2ASP-hu-mROO5-1-vH-[hTCRa-T48C] and is represented by DNA SEQ ID NO: 7405 and PRT SEQ ID NO: 18095. This SAR construct has the backbone of a SIR. The hu-mROO5-1 vL and hu-mROO5-1 vH fragments of the SAR dimerize to form a Fv targeting CD19. An SVH fragment targeting CD38 (CD38-USC1-FHVH-32184) is fused to the N-terminus of the hu-mROO5-1 vL fragment via a Glycine-Serine linker. Thus, the SAR targets CD19 via the hu-mROO5-1 Fv and targets CD38 via CD38-USC1-FHVH-32184.

Another exemplary double chain trispecific SAR comprising two chains is CD8SP-CD38-USC1-FHVH-32184-G4Sx3-R1-hu-mROO5-1-vL-[hTCRb-S57C]-F-P2A-IgSP-Apa-CD20-USC1-vHH-2HCD26-G4Sx3v2-hu-mROO5-1-vH-[hTCRa-T48C] and is represented by DNA SEQ ID NO: 7408 and PRT SEQ ID NO: 18098. This SAR construct has the backbone of a SIR. The hu-mROO5-1 vL and hu-mROO5-1 vH fragments of the SIR dimerize to form a Fv targeting CD19. An SVH fragment targeting CD38 (CD38-USC1-FHVH-32184) is fused to the N-terminus of the hu-mROO5-1 vL fragment via a Glycine-Serine linker. A vHH fragment targeting CD20 (CD20-USC1-vHH-2HCD26) is fused to the N-terminus of the hu-mROO5-1 vH fragment via a Glycine-Serine linker. Thus, the SAR targets CD19 via the hu-mROO5-1 Fv and targets CD38 via CD38-USC1-FHVH-32184 and targets CD20 via CD20-USC1-vHH-2HCD26. Another exemplary trispecific SAR construct with a similar format is CD8SP-CD19-FHVH-354-G4S3-FMC63-vL-[hTCRb-S57C-opt]-F-P2A-SP-CD22-FHVH-24-FMC63-vH-[hTCRa-T48C-opt] and is represented by SEQ ID NO (DNA):2344 and SEQ ID NO (PRT): 13034, respectively. Additional SARs of this format in which the vL/vH of FMC63 are replaced by vL/vH fragments derived from other scFv and targeting different antigens are provided in **Table 26** (SEQ ID NO: 2344-2508 and 13034-13198). The vL/vH fragments of these constructs can be determined from their SEQ ID Nos. by examination of **Table 25** as the order of vL/vH fragments of these constructs is same as the order of the vL/vH fragments of constructs in **Table 25.** As discussed above, the TCR constant chains of these constructs can be replaced with other TCR constant chains described in **Table 12** or their deletion mutants and functional variants as long as the functional activity of SAR (*i.e*., ability to bind to its target antigen, ability to induce T cell signaling upon antigen binding, ability to assemble into TCR/CD3 complex *etc.*) is retained. An exemplary SAR containing deletion mutants of TCRa and TCRb constant chains is CD8SP-CD38-USC1-FHVH-32184-G4Sx3-R1-hu-mROO5-1-vL-[hTCRb-S57C-opt-del17]-F-P2A-IgSP-Apa-CD20-USC1-vHH-2HCD26-G4Sx3v2-hu-mROO5-1-vH-[hTCRa-T48C-opt-del17] and is represented by nucleic acid SEQ ID NO:1332 and amino acid SEQ ID NO: 12022. The SAR constructs with deletion mutants of TCRa and TCRb chains are smaller in size and therefore have the advantage of increasing the titer of the lentiviral/retroviral vector encoding them since the titer of the lentiviral/retroviral vector is inversely proportion to the insert size. Finally, in addition to the TCR chains and module, the CD38-USC1-FHVH-32184 and CD20-USC1-vHH-2HCD26 fragments can be replaced with other AABD targeting different antigens or epitope. Exemplary AABD are provided in **Tables 5, 7-10.** Additional AABD (*e.g.,* vHH, SVH, Centyrins *etc.*) targeting different antigens are known in the art.

An exemplary double chain trispecific SAR comprising two chains is CD8SP-hu-mROO5-1-vL-[hTCRb-S57C]-F-P2A-IgSP-CD22-USC1-FHVH-160-G4Sx2-CD20-USC1-vHH-2HCD26-G4Sx3v2-hu-mROO5-1-vH-[hTCRa-T48C] and is represented by DNA SEQ ID NO: 7407 and PRT SEQ ID NO: 18097. This SAR construct has the backbone of a SIR. The hu-mROO5-1 vL and hu-mROO5-1 vH fragments of the SIR dimerize to form a Fv targeting CD19. A vHH fragment targeting CD20 (CD20-USC1-vHH-2HCD26) is fused to the N-terminus of the hu-mROO5-1 vH fragment via a Glycine-Serine linker. Further, an SVH fragment targeting CD22 (CD22-USC1-FHVH-160) is fused to the N-terminus of the CD20 vHH fragment via another Glycine-Serine Linker. Thus, the SAR targets CD19 via the hu-mROO5-1 Fv, targets CD20 via CD22-USC1-FHVH-160 and targets CD20 via CD20-USC1-vHH-2HCD26. As SARs are modular in format, the TCR chains can be replaced by other TCR chains or their variants, AABD can be replaced by other AABDs or their variants and vL/vH fragments replaced by other vL/vH fragments or their variants.

An exemplary double chain tetra-specific SAR comprising two chains is CD8SP-BCMA-FHVH-33-G3Sx2-CD38-FHVH-309021-G4Sx3-R1-hu-mROO5-1-vL-[hTCRb-S57C]-F-P2A-IgSP-Apa-CD20-vHH-2HCD25-G4Sx3v2-hu-mROO5-1-vH-[hTCRa-T48C] and is represented by DNA SEQ ID NO: 5362 and PRT SEQ ID NO: 16052. This SAR construct has the backbone of a SIR. The hu-mROO5-1 vL and hu-mROO5-1 vH fragments of the SIR dimerize to form a Fv targeting CD19. A vHH fragment targeting CD20 (CD20-vHH-2HCD25; SEQ ID NO: 835) is fused to the N-terminus of the hu-mROO5-1 vH fragment via a Glycine-Serine linker. An SVH fragment targeting CD38 (CD38-FHVH-309021; SEQ ID NO: 846) is fused to the N-terminus of the hu-mROO5-1-vL fragment via a Glycine-Serine Linker. A SVH targeting BCMA (BCMA-FHVH-33; SEQ ID N O: 856) is fused to the N-terminus of the CD38-FHVH-309021 fragment via another Glycine-Serine Linker. Thus, the SAR targets CD19 via the hu-mROO5-1 Fv, targets CD20 via CD20-vHH-2HCD25, and targets CD38 via CD38-FHVH-309021 and targets BCMA via BCMA-FHVH-33. As SARs are modular in format, the TCR chains can be replaced by other TCR chains or their variants, AABD can be replaced by other AABDs or their variants and vL/vH fragments replaced by other vL/vH fragments or their variants.

An exemplary double chain penta-specific SAR comprising two chains is CD8SP-BCMA-FHVH-33-G3Sx2-CD38-FHVH-309021-G4Sx3-R1-hu-mROO5-1-vL-[hTCRb-S57C]-F-P2A-IgSP-CD22-FHVH-158-G4Sx2-CD20-vHH-2HCD25-G4Sx3v2-hu-mROO5-1-vH-[hTCRa-T48C] and is represented by DNA SEQ ID NO: 5437 and PRT SEQ ID NO: 16127. This SAR construct resembles the construct represented by DNA SEQ ID NO: 5362 and PRT SEQ ID NO: 16052 but has in addition an SVH fragment targeting CD22 (CD22-FHVH-158; SEQ ID NO: 848) fused to the N-terminus of the CD20-vHH-2HCD25 fragment via a Glycine-Serine Linker. Thus, the SAR targets CD19 via the hu-mROO5-1 Fv, targets CD20 via CD20-vHH-2HCD25, and targets CD38 via CD38-FHVH-309021 and targets BCMA via BCMA-FHVH-33. As SARs are modular in format, the TCR chains can be replaced by other TCR chains or their variants, AABD can be replaced by other AABDs or their variants and vL/vH fragments replaced by other vL/vH fragments or their variants. The vL/vH fragments comprising the SAR can be fully-human, humanized, chimeric or non-human in origin.

In the preceding examples, the bi-specific and multispecific SAR backbone comprises of vL and vH fragments to which one or more AABDs are operably linked. The disclosure also describes SARs whose backbone are comprised of variable fragments (Va/Vb or Vg/Vd) derived from TCRs.

In an embodiment, the disclosure provides a single chain, one and a half chain or double chain SAR comprising at least one chain that has the general formula: (AABD)n-optional linker domain-a TCR variable fragment-optional linker-TCR constant chain; where n = 0, 1, 2, 3, 4, 5 or more and where TCR variable fragment represents Va, Vb, Vg or Vd, and where TCR constant chain represents the constant chain of TCRα, TCRβ1, TCRβ2, TCRγ, TCRδ or pre-TCRα or a mutant or a variant thereof. In an exemplary embodiment, the AABD is a non-scFv antigen binding module (*e.g.*, a SVH, vHH, FHVH, SVL, svd-TCR, Centyrin, DARPIN, CD16A, CD64, CD32, NKG2D, NKG2D-AF, NKG2D-YA, RZIP, EZIP, E4, K4, D domain *etc.*)*.*

In an embodiment, the disclosure provides a double chain SAR comprising one chain with the general formula: (AABD)n-optional linker domain-Va fragment-optional linker-TCR constant chain; and the second chain with the general formula; (AABD)n-optional linker domain-Vb fragment-optional linker-TCR constant chain where n = 0, 1, 2, 3, 4, 5 or more and where TCR constant chain represents the constant chain of TCRα (Cα), TCRβ1 (Cβ1), TCRβ2 (Cβ2), TCRγ (Cγ), TCRδ (Cδ) or pre-TCRα (pre-Cα) or a mutant or a variant thereof. In an embodiment, the two chains of the SAR comprise complementary TCR constant chains; *i.e.*, Cα and Cβ, pr*etc*.Rα and Cβ, or Cγ and Cδ. The Cβ chain could represent either Cβ1 or Cβ2.

In an embodiment, the disclosure provides a double chain SAR comprising one chain with the general formula: (AABD)n-optional linker domain-Vg fragment-optional linker-TCR constant chain; and the second chain with the general formula; (AABD)n-optional linker domain-Vd fragment-optional linker-TCR constant chain where n = 0, 1, 2, 3, 4, 5 or more and where TCR constant chain represents the constant chain of TCRα (Cα), TCRβ1 (Cβ1), TCRβ2 (Cβ2), TCRγ (Cγ), TCRδ (Cδ) or pre-TCRα (pre-Cα) or a mutant or a variant thereof. In an embodiment, the two chains of the SAR comprise complementary TCR constant chains; *i.e.*, Cα and Cβ, pr*etc*.Rα and Cβ, or Cγ and Cδ. The Cβ chain could represent either Cβ1 or Cβ2.

An exemplary SAR in which the AABD is operably linked to the Va and/or Vb chain is CD8SP-MC7G5-Vb-[hTCRb-S57C]-F-P2A-IgSP-Apa-CD20-vHH-2HCD25-G4Sx3v2-MC7G5-Va-[hTCRa-T48C]-F-F2A-PAC and is represented by nucleic acid SEQ ID NO: 3502 and amino acid SEQ ID NO: 14192. This SAR construct has the MC7G5-Vb fragment fused to the constant chain of human TCRb carrying S57C mutation (hTCRb-S57C) and the MC7G5-Va fragment fused to the constant chain of human TCRa carrying the T48C mutation (hTCRa-T48C). The MC7G5-Va and MC7G5-Vb fragments of the SAR bind to MR1. A vHH fragment targeting CD20 (CD20-vHH-2HCD25) is fused to the N-terminus of the MC7G5-Va fragment via a Glycine-Serine linker (G4Sx3v2). Thus, the SAR targets MR1 via the MC7G5 Va/Vb fragments and targets CD20 via CD20-vHH-2HCD25.

An exemplary SAR based on the backbone of a γδ TCR is CD8SP-BCMA-FHVH-93-G4S-TCR-Vg9-F-P2A-IgHSP-CD20-vHH2-HCD25-G4S-TCR-Vd2-F-F2A-PAC (SEQ ID NO: 22201). This SAR has the backbone of a Vγ9/Vδ2 TCR. The Vγ9/Vδ2 T cells represent a minor and unconventional constituent of the leukocyte population in peripheral blood (0.5-5%), yet they are assumed to play an early and essential role in sensing 'danger' by invading pathogens. They recognize the same small microbial compound (E)-4-hydroxy-3-methyl-but-2-enyl pyrophosphate (HMB-PP), a natural intermediate of the non-mevalonate pathway of isopentenyl pyrophosphate (IPP) biosynthesis. The above SAR on Vγ9/Vδ2 backbone can also recognize BCMA through a BCMA-FHVH-93 that is attached to the N-terminus of Vg9 variable domain via a G4S linker and recognize CD20 through CD20-vHH2-HCD25 that is attached to the N-terminus of Vd2 variable domain. Additional SAR based on Vγ9/Vδ2 can be constructed by substituting the BCMA-FHVH-93 and CD20-vHH2-HCD25 with antigen binding domains (*e.g*., AABD) binding other antigens. Exemplary SAR based on Vγ9/Vδ2 can be also constructed by replacing the MC.7.G5 (MC7G5) Va and Vb domains of SARs with SEQ ID NO: 3500-3595 with Vγ9/Vδ2 vairable domains.

The disclosure also describes SARs in which the vL and/or vH fragments of the SARs described in the preceding section are replaced with the Va and/or Vb fragments derived from T cell receptors (TCR). Several exemplary bispecific and trispecific SARs based on Va and Vb fragments of a TCR (MC7G5) directed against MIR1, a monomorphic MHC class I-related protein (MR1), as the backbone and carrying AABD targeting CD19 and/or CD20 are provided in **Table 27.** The composition of these SARs can be determined from their name. Additional SARs based on TCRs are listed in **Table 28.** Other SARs that are based on the modular architecture of the SARs described in **Tables 27-28** are also within the scope of this disclosure.

The disclosure also provides exemplary SARs in which one or more AABDs are operably linked to the N-terminus or near the N-terminus of the variable fragment (*e.g*., Va, Vb, Vg or Vd) derived from a TCR. An exemplary such SAR construct is CD8SP-Sph-CD19-vHH-048-G4Sx3-R1-MC7G5-Vb-[hTCRb-S57C]-F-P2A-SP-MC7G5-Va-[hTCRa-T48C] and is represented by nucleic acid SEQ ID NO: 3535 and amino acid SEQ ID NO: 14225. An exemplary trispecific SAR based on MC7G5 backbone is CD8SP-Sph-CD19-vHH-048-G4Sx3-R1-MC7G5-Vb-[hTCRb-S57C]-F-P2A-IgSP-Apa-CD20-vHH-2HCD25-G4Sx3v2-MC7G5-Va-[hTCRa-T48C] and is represented by nucleic acid SEQ ID NO: 3578 and amino acid SEQ ID NO: 14268.

Several exemplary bispecific and trispecific SARs based on Va and Vb fragments of a TCR (MC7G5) directed against MIR1, a monomorphic MHC class I-related protein (MR1), as the backbone and carrying AABD targeting CD19 and/or CD20 are provided in **Table 27.** The compositions of these SARs can be determined from their name. It is possible to replace one module of these SARs with another module. The modules that can be replaced include the Va/Vb fragments, the TCR constant chains (*i.e.*, hTCRb-S57C and hTCRa-T48C) as well as the AABD (*i.e.*, CD20-vHH-2HCD25). Additional SARs based on different TCR variable domains, TCR constant chains and AABD are listed in **Table 28.** The SARs in which the Va/Vb fragment of the MC7G5-based SARs listed in **Table 27** are replaced by Va/Vb derived from a TCR targeting CMV-pp65-derived peptide (SEQ ID NO: 21452) in complex with HLA-A2 are represented by nucleic acid SEQ ID NO: 3891-3988 and amino acid SEQ ID NO: 14581-14678, respectively **(Table 28).** Similarly, the exemplary SARs in which the Va/Vb fragment of the MC7G5-based SARs listed in Table 27 are replaced by Va/Vb derived from a TCR targeting NYESO-1-derived peptide (SEQ ID NO: 21461) in complex with HLA-A2 are represented by nucleic acid SEQ ID NO: 3989-4086 and amino acid SEQ ID NO: 14679-14776, respectively **(Table 28).** The order of these constructs is similar to the order of the MC7G5 based constructs listed in **Table 27.**

The disclosur also provides SARs encoding SAR polypeptides in which one chain comprises a variable fragment derived from an antibody (*e.g*., vL or a vH) and the second chain comprises a variable fragment derived from a TCR (*e.g.*, Va, Vb, Vg or a Vd). Exemplary such SAR polypeptides are represented by SEQ ID NO (PRT): 23020 and 23021. The variable fragments derived from antibodies and TCRs in such a SAR may serve as scaffold for the attachment of one or more AABDs.

In an embodiment, the disclosure provides a single chain, one and a half chain or double chain SAR comprising at least one chain that has the general formula: (AABD)n-optional -linker-TCR constant chain; where n = 0, 1, 2, 3, 4, 5 or more and where TCR constant chain represents the constant chain of TCRα, TCRβ1, TCRβ2, TCRγ, TCRδ or pre-TCRα or a mutant or a variant thereof. In an embodiment, the linker is an Ig linker domain (*e.g.*, IgCL, IgG1-CH1 *etc.).* Exemplary Ig (or Ig like) linker domains are presented in **Table 13.** In an exemplary embodiment, the AABD is a non-scFv antigen binding module *(e.g.,* a SVH, vHH, FHVH, SVL, svd-TCR, Centyrin, DARPIN, CD16A, CD64, CD32, NKG2D, NKG2D-AF, NKG2D-YA, RZIP, EZIP, E4, K4, D domain *etc.*)*.* In an exemplary embodiment, the AABD is an adaptor binding domain.

In an embodiment, the disclosure provides a double chain SAR comprising one chain with the general formula: (AABD)n-Ig linker domain-TCR constant chain; and the second chain with the general formula; (AABD)n-Ig linker domains -TCR constant chain where n = 0, 1, 2, 3, 4, 5 or more and where TCR constant chain represents the constant chain of TCRα (Cα), TCRβ1 (Cβ1), TCRβ2 (Cβ2), TCRγ (Cγ), TCRδ (Cδ) or pre-TCRα (pre-Cα) or a mutant or a variant thereof. In an embodiment, the two chains of the SAR comprise complementary TCR constant chains; *e.g.*, Cα and Cβ, pretc.Rα and Cβ, or Cγ and Cδ. The Cβ chain could represent either Cβ1 or Cβ2. In an embodiment, the two chains of the SAR comprise complementary Ig linker domains; *e.g*.*,* IgCL and IgG1-CH1, IgCL and IgG2-0C-CH1, or IgCL (SEQ ID NO: 11832) and IgG4-CH1 (SEQ ID NO: 11842) *etc.* In an embodiment, one chain fo the SAR comprises IgCL linker domain (SEQ ID NO (DNA): 1142 and SEQ ID NO (PRT): 11832) and the second chain of the SAR comprises the IgG1-CH1 linker domain (SEQ ID NO (DNA): 1143 and SEQ ID NO (PRT): 11833). Additional Ig linker domains are presented in SEQ ID NO (DNA): 1149-1157 and SEQ ID NO (PRT): 11839-11847 **(Table 13)** and can substitute for IgG1-CHI linker domain. Finally, other Ig or Ig like linker domains are known in the art and can be used in alternate embodiments of the disclosure. In an exemplary embodiment, the AABD is a non-scFv antigen binding module (*e.g.*, a SVH, vHH, FHVH, SVL, svd-TCR, Centyrin, DARPIN, CD16A, CD64, CD32, NKG2D, NKG2D-AF, NKG2D-YA, RZIP, EZIP, E4, K4, D domain *etc.*)*.* In an exemplary embodiment, the AABD is an adaptor binding domain.

An exemplary SAR on the MC7G5 backbone in which the CD20-vHH-2HCD25 domain is replaced by the ligand binding domain of NKG2D (NKG2D-G4Sx3-NKG2D) is represented by CD8SP-MC7G5-Vb-[hTCRb-S57C]-F-P2A-IgSP-NKG2D-G4Sx3-NKG2D-G4Sx3v2-MC7G5-Va-[hTCRa-T48C] and has nucleic acid SEQ ID NO:3601 and amino acid SEQ ID NO:14291. Other exemplary SARs in which the CD20-vHH-2HCD25 domain of SARs listed in Table 27 is replaced by the ligand binding domain of NKG2D are represented by nucleic acid SEQ ID NO: 3597-3694 and amino acid SEQ ID NO: 14287-14384 (Table 28). The order of these constructs is similar to the order of the CD20-vHH-2HCD25 containing constructs listed in Table 27. The SAR constructs comprising the ligand binding domain of NKG2D are able to bind to and respond to cells expressing NKG2D ligands, such as MICA and MICB.

Described herein are methods and compositions that provide improved safety and efficacy of SAR cells (*e.g.*, SAR-T, SAR-NK, SAR-monocyte/macrophage cells *etc.*) by providing a universal approach for combinatorial, temporal, and logical control of multiple cell signaling pathways.

In one embodiment, specificity is enhanced by using SARs that can detect two or more antigens. The ability to sense more than two antigens can further improve tumor specificity. In addition, even when SAR T cells are on target, adverse side effects can still occur when over-activation of T cells leads to cytokine release syndrome. The disclosure provides useful designs and configuration for constructing SARs expressing Kill and ON switches to mitigate some of these side effects. Still, these switches serve mostly as master switches and consequentially lack fine control. Since control of natural immune cell activation is achieved through a balance of multiple co-stimulatory and co-inhibitory signaling pathways, strategies that provide tunable and temporal control over such signaling pathways are important for optimizing SAR-expressing cell (or SAR cell) performance. These challenges illustrate the need for intelligent controls of SAR cell response.

Universal CAR constructs comprising single chain chimeric receptors (*e.g.*, 2^{nd} generation CAR) have been described that do not directly recognize the target antigen, but instead make contact to adaptor molecules (CAR-adaptors), that in turn bind to the target antigen. Such universal CAR or modular-CARs have been proposed as a potential solution to the problems facing the conventional CARs, such as tumor heterogeneity and antigen escape, toxicity, tonic signaling leading to exhaustion, and immunogenicity. Thus, the activity of a universal CAR-T cells is strictly dependent on the presence of the CAR-adaptors enabling the formation of an immunological synapse. Consequently, the formation of the synapse depends on the respective characteristics of the CAR-adaptors; the affinity of the CAR-adaptors for the tumor antigens, the affinity towards the chosen extracellular domain (ECD) of the universal CAR, the level of expression of the CAR, its half-life as well as its bio-distribution, are all important. As the next generation CAR constructs (*e.g*., SIR, cTCR, Ab-TCR, TFP and/or TCR *etc.*) differ from the 2^{nd} generation CARs in their level of expression and affinity for the target, the appropriate composition and configuration for the incorporation of adaptors into next generation CAR constructs is not known at the present. Similarly, the appropriate composition and configuration for incorporation of adaptors into multichain constructs (*e.g*., double chain SIR, cTCR, Ab-TCR, αβTFP, γδTFP or TCR) is not known. Finally, the appropriate composition and configuration for the incorporation of adaptors into next generation CAR constructs (*e.g.*, SIR, cTCR, Ab-TCR, αβTFP, γδTFP or TCR *etc.*) with bispecific, biparatopic, multispecific and multi-paratopic properties is not known at the present.

Described herein are novel compositions, designs and configurations of universal SAR platforms with the ability to (a) serve as an ON/OFF switch, (b) sense multiple antigens and perform logic computations, and (c) independently regulate multiple signaling pathways, that will provide the necessary control for optimizing SAR cell therapy. The split, universal, programmable and reconfigurable UNI-SAR platforms described herein are used for this purpose. Importantly the UNI-SAR platforms can accommodate new targets without further manipulation to patients' immune cells. In an embodiment, the UNI-SAR platform is a two-component receptor system composed of a universal receptor expressed on the immune cells (*e.g.*, T cell, NK cells, NKT cells, monocytes *etc.*) and a tumor-targeting antigen binding adaptor (SAR-Adaptor). In an embodiment, the universal receptor is generated from the fusion of an AABD, scFv or Fv, comprising an adaptor binding domain to a signaling chain that is either capable of initiating a cell signal or recruits one or more signaling chains that are capable of initiating a cell signal. The SAR-adaptor molecule is generated from the fusion of an adaptor (*e.g.*, leucine zipper domain, *e.g.*, RZIP, EZIP *etc.*) to an antigen binding moiety (*e.g.*, scFv, vHH, FHVH, Centyrin *etc.*)*.* The antigen binding moiety of the SAR adaptor binds to the antigen and the adaptor (*e.g.*, leucine zipper domain, *e.g.*, RZIP) binds and activates the UNI-SAR on the immune cells. This system also functions as a tunable switch with the SAR adaptor as a titratable inducer. However, unlike other existing split CAR systems, described herein are orthogonal UNI-SAR/SAR adaptor pairs, which permit control of multiple signaling pathways independently and performance of logic operations.

One limitation of the split and universal CARs described in the art is the need for repeated and/or continuous administration of the SAR adaptor to activate the CAR expressing cells, which leads to inconvenience, increased cost, infectious and allergic complications. Therefore, in some situations, it would be desirable to have a SAR construct that can bind to and target cells with its built-in constitutive unispecific, bispecific and/or multispecific targeting modules but has the added flexibility of targeting additional antigens (*i.e.*, universal targeting capability) when used in combination with appropriate SAR adaptors comprising antigen binding domain(s) for those antigens.

The disclosure provides novel compositions, configurations and designs for a SAR, designated a UNICON-SAR, that has constitutive (or built-in) unispecific, bispecific and multi-specific targeting modules but in addition has one or more adaptor binding domains that also confer universal targeting properties when used in conjunction with an appropriate SAR-adaptor. In an exemplary embodiment, is provided a SAR that can constitutively bind and target cells expressing CD19, CD19 and CD20, CD19, CD20 and BCMA, or CD19, CD20 and PSMA but also comprises one or more adaptor binding domains that allows it to be redirected to Her2-expressing cell in the presence of a Her2 antibody (*e.g*., Herceptin) or another SAR adaptor comprising a Her2 binding domain. In another exemplary embodiment, is provided a SAR that can constitutively bind and target cells expressing CD19, CD19 and CD22, CD19, CD22 and BCMA, or CD19, CD22 and Her2 but also comprises one or more adaptor binding domains that allows it to be redirected to CD20-expressing cell in the presence of a CD20 antibody (*e.g*., Rituximab) or another SAR adaptor comprising a CD20 binding domain. Exemplary such SARs are provided in SEQ ID NO: 22173 and 22176. The advantage of such UNICON-SAR expressing cells is that they are active against their target antigen expressing cells (*e.g.*, cancer cells) constitutively through their built-in targeting modules but also have the flexibility of being redirected to additional antigens with the use of SAR adaptors, if and when needed. Thus UNICON-SAR design offers improved efficacy, multi-targeting, precision, flexibility, safety, convenience and economy. The UNISAR and UNICON-SAR platforms can be based on any of the adaptors described in this disclosure, including but not limited to leucine zippers (*e.g.*, RZIP and EZIP), E4, K4, D domains, non-natural NKG2D based receptor-ligand pairs (*e.g*., NKG2D-YA) and Fc binding modules.

In one embodiment, the UNI-SAR platform uses leucine zipper as the extracellular portion of the SAR and various signaling chains. The cognate leucine zipper is fused to an antigen binding moiety (*e.g.*, a specific scFv). The administration of the antibody/zipper fusion activates the T cells, and thus serves as an ON switch in a dose-dependent manner. Leucine zippers are a class of protein domain that can form heteromeric structures through charge interactions. Leucine zippers are beneficial for the UNI-SAR platform because many orthogonal pairs of leucine zippers are available, thus providing a large pool of candidates for design efforts. Leucine zipper domains can also be engineered to compete with each other for the same binding partner, thus allowing inhibition and "NOT" functionality. Moreover, we can utilize different affinities between leucine zipper pairs to engineer complex functions described in the art, such as OR, NEVIPLY, AND and XOR.

Each individual SAR can be readily paired to scFvs that target different antigens, thus allowing combinatorial and logical antigen sensing. *In vitro* characterization can map UNI-SAR and UNICON-SAR platforms responses (*e.g*., cytotoxicity, cytokine production, and memory T cell formation) to design parameters (*e.g*., zipSAR expression level, zipFv concentration, scFv affinity, and zipper affinity). The functionalities described herein can also be tested *in vivo* in mouse xenograft tumor models.

Current CAR designs have limited control and computing capabilities. These deficiencies render current CARs susceptible to dangerous over-activation and reduce CARs' ability to distinguish tumor cells from healthy tissues. Described herein are compositions and designs to address the control and computing capability by using, *e.g.*, the UNI-SAR and UNICON-SAR platforms to independently regulate multiple signaling pathways in T cells, which imparts logic and signaling mixer functions.

The methods and compositions provide advantages *e.g*., they provide the first function-rich, universal SAR platform featuring an ON/OFF switch, logical detection and integration, processing of >2 antigens, and independent regulation of different signaling pathways. These features have never been demonstrated together in a single system, and in particular, in the context of next generation CAR designs, such as SIR, Ab-TCR, and TFP. Further, such features have not been described for double chain next generation CARs, such as double chain SIR, double chain cTCR, double chain TCR, double chain Ab-TCR, double chain TCR and double chain AABD-TCR *etc.* In particular, provided herein is the first independent tuning of multiple signal pathways in T cells using SARs. The different parameters can affect a split, universal SAR system, which will be useful for designing titratable ON/OFF SAR switches. The UNISAR and UNICON-SAR designs described here can be used for the generation of the two-input Boolean logic gates with SARs, including OR, NDVIPLY, AND and XOR logic gates.

As used herein, "multi-component SAR" refers to a SAR comprising at least two separate polypeptides. In some embodiments, a multi-component SAR can comprise two, three, four, five, or more separate polypeptides. In some embodiments, the at least two separate polypeptides of a multi-component SAR each comprise a protein interaction domain that permits interaction, *e.g*., binding of the separate polypeptides. In some embodiments, at least one of the polypeptides of a multi-component SAR is an extracellular polypeptide, also known as SAR adaptor, having an antigen-binding domain but lacking a transmembrane domain and at least one of the polypeptides is a transmembrane polypeptide capable of transmitting a signal. In an embodiment, at least one of the transmembrane polypepides of a multi-component SAR either possesses an intracellular signaling domain or is capable of recruiting one or more proteins that possess an intracellular signaling domain. The transmembrane polypeptide of a multi-component SAR may have one or more antigen recognition domains at least one of which binds to the extracellular SAR adaptor that has an antigen-binding domain but lacking the transmembrane domain.

In one aspect of the embodiments, described herein is a multi-component chimeric antigen receptor (SAR); the multi-component SAR comprising: a) a first recognition polypeptide (or SAR adaptor) comprising 1) an antibody reagent specific for a first target antigen and 2) a protein interaction domain; and b) at least one signaling polypeptide comprising 1) an extracellular protein interaction domain that can bind specifically with the protein interaction domain of the first recognition polypeptide or the SAR adaptor and 2) at least one chain that either comprise an intracellular signaling domain or is capable of recruiting one or more proteins that comprise an intracellular signaling domain. In an embodiment, the intracellular signaling domain is an activation domain.

As used herein, "recognition polypeptide" or "SAR adaptor" or "adaptor" refers to an extracellular polypeptide having an antigen-binding domain. In some embodiments, the antigen-binding domain can be an antibody reagent (*e.g*., monoclonal antibody, scFv, camelid vHH domain, single domain antibody *etc.*) or a non-immunoglobulin antibody binding scaffold. In some embodiments, the recognition polypeptide can further comprise a protein interaction domain or an adaptor. Non-limiting examples of adaptors include RZIP, EZIP, E4, K4, D domains, NKG2D-YA, NKG2D-AF *etc.* In an embodiment, an antibody (*e.g.*, Rituximab) can serve as a SAR adaptor when used in combination with a SAR expressing an Fc binding domain (*e.g.*, CD16A, CD16-V158, CD32 or CD64 *etc.*)*.*

As used herein, "signaling polypeptide" refers to a transmembrane polypeptide that either comprise an intracellular signaling domain or is capable of recruiting one or more proteins with an intracellular signaling domain. In some embodiments, the signaling polypeptide can further comprise a protein interaction domain. In some embodiments, the signaling polypeptide can further comprise an extracellular protein interaction domain (or an adaptor binding domain) that binds to the SAR adaptor.

As used herein, "protein interaction domain" refers to a domain that permits specific binding of two separate polypeptides to each other. A number of exemplary protein interaction domains, as well as pairs of protein interaction domains are provided elsewhere herein. In some embodiments, the protein interaction domains of the polypeptides of a multi-component SAR can bind specifically, *e.g*., one of the protein interaction domains can bind specifically to a second protein interaction domain of the multi-component SAR. In some embodiments, specific binding can occur when two separate protein interaction domains are present. In some embodiments, specific binding can occur when three or more separate protein interaction domains are present. Exemplary protein interaction domains are known in the art and can be used in embodiments of the aspects described herein.

In some embodiments of any of the aspects described herein, the protein interaction domains can be leucine zipper domains. Leucine zipper domains are a type of protein-protein interaction domain commonly found in transcription factors characterized by leucine residues evenly spaced through a a-helix. Leucine zippers may form heterodimers or homodimers. A number of leucine zipper domains, as well as their ability to bind each other, are known in the art and discussed further, *e.g.*, in Reinke et al. JACS 2010 132:6025-31, Thomposon et al. ACS Synth Biol 2012 1: 118-129 and WO2017091546A1; each of which is incorporated by reference herein in its entirety. In some embodiments, one leucine zipper domain is RZIP and the second leucine zipper domain is EZIP.

In some embodiments, the protein interaction domain present on the extracellular protein is referred to as adaptor while the protein interaction domain present on the SAR is referred as adaptor binding domain. However, the two domains can be switched. Thus, in some embodiments, the extracellular polypeptide (i.e, SAR adaptor) may comprise an EZIP domain that interacts with the RZIP domain (*i.e.*, adaptor binding domain) present on the transmembrane domain comprising signaling chain of the SAR. In other embodiments, the extracellular polypeptide (i.e, SAR adaptor) may comprise a RZIP domain that interacts with the EZIP domain (*i.e.*, adaptor binding domain) present on the transmembrane domain comprising signaling chain of the SAR.

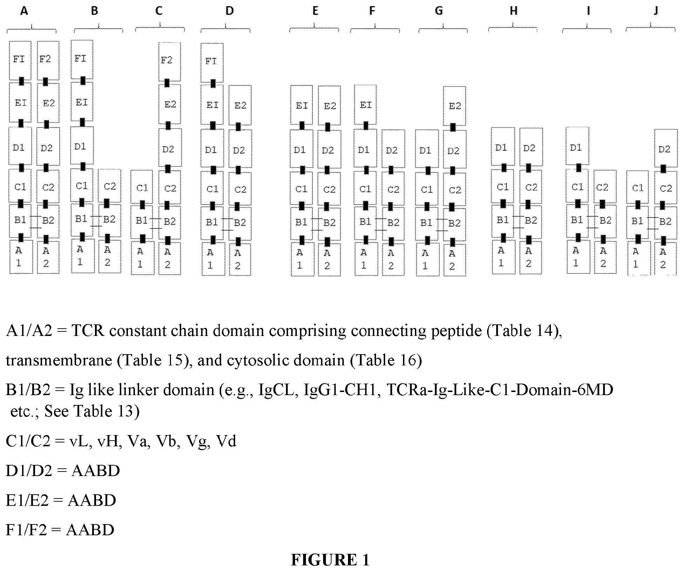

Further exemplary leucine zipper domains are described in Reinke et al. JACS 2010 132:6025-31; which is incorporated by reference herein in its entirety. For example, suitable leucine zipper domains can include SYNZIP 1 to SYNZIP 48, andBATF, FOS, ATF4, ATF3, BACH1, JUND, NFE2L3, and HEPTAD. Binding affinities of various combinations of these domains are described, *e.g.*, at Fig. 1 of Reinke *et al.* In some embodiments, a suitable pair of leucine zipper domains has a dissociation constant (Kd) of 1000 nM or less. In some embodiments, a suitable pair of leucine zipper domains has a dissociation constant (Kd) of 100 nM or less. In some embodiments, a suitable pair of leucine zipper domains has a dissociation constant (Kd) of 10 nM or less. In some embodiments, a suitable pair of leucine zipper domains has a dissociation constant (Kd) of 1 nM or less.

Further exemplary pairs of protein interaction domains can include a) PSD95-Dlgl-zo-1 (PDZ) domains; b) a streptavidin domain and a streptavidin binding protein (SBP) domain; and c) a PYL domain and an ABI domain.

In some embodiments of any of the aspects described herein, the protein interaction domains can be chemically-induced protein interaction domains, *e.g*., domains that will only bind specifically in the presence of a third molecule, *e.g.*, a small molecule or drug. Exemplary pairs of chemically-induced protein interaction domains can include: FKBP-binding domain of mTOR (FRB) and FK506 binding protein (FKBP) (binding of which is activated by tacrolimus, everolimus, or a rapalog); cyclophilin-Fas fusion protein (CyP-Fas) and FK506 binding protein (FKBP) (binding of which is activated by FKCsA); calcineurinA (CNA) and FK506 binding protein (FKBP) (binding of which is activated by FK506); gibberellin insensitive (GIA) and gibberellin insensitive dwarfl (GID1) (binding of which is activated by gibberellin); Snap-tag and Halo tag (binding of which is activated by HaXS); and T14-3-3-cdeltaC and C-Terminal peptides of PMA2 (CT52) (binding of which is activated by fusicoccin). Further description of chemically-induced protein interaction domains can be found in the art, *e.g.*, Miyamoto et al. Nat Chem Biol. 2012 Mar 25; 8(5): 465-470 and Belshaw et al. PNAS 1996 93 :4604-4607; each of which is incorporated herein by reference in its entirety.

In some embodiments of any of the aspects described herein, the protein interaction domains can comprise at least one nucleotide tag and at least one zinc finger domain. Zinc finger domains are characterized by the coordination of a zinc ion in order to stabilize their tertirary structure. The particular folds that appear in zinc fingers can vary. In some embodiments, a zinc finger domain can be a nucleotide-binding zinc finger domain. In some embodiments, a zinc finger domain can be a DNA-binding zinc finger domain. In some embodiments, the protein interaction domain of the recognition polypeptide is a nucleotide tag and the extracellular protein interaction domain of the signaling polypeptide is a zinc finger domain. In some embodiments, a nucleotide tag can be a DNA tag. In some embodiments, a nucleotide tag can be a dsDNA tag comprising the entire recognition sequence for the zinc finger domain being used. Exemplary zinc finger domains and their cognate nucleotide tags are described in the art, *e.g.*, Mali et al. Nature Methods 2013 10:403-406; which is incorporated by reference herein in its entirety. In some embodiments, a zinc finger domain can be sZF15 as described in Mali et al. Nature Methods 2013 10:403-406.

In some aspects, the SAR of the disclosure includes an adaptor binding domain (*e.g.*, a leucine zipper domain, *e.g.*, RZIP or EZIP *etc.*) that allows it to bind to an extracellular polypeptide or SAR adaptor. The immune cells expressing such SAR constructs can be redirected to different target cells via the use of different SAR adaptors (*i.e.*, different antigen binding domains (*e.g.*, antibodies, antibody fragments, vHH, FHVH *etc.*) fused to the adaptors). Exemplary adaptors and adaptor binding domains are provided in **Table 10.** In an exemplary embodiment, a RZIP encoding SAR can be used in conjunction with an EZIP encoding polypeptide containing an antigen binding domain targeting CD19 to target a CD19-expressing cell.

Similarly, a SAR comprising an antigen binding domain targeting Streptag (SEQ ID NO: 1192) or FITC (*e.g.*, SEQ ID NO: 1978-1979) can be used in combination with a Streptag-labelled or FITC-labelled antibody/antibody fragment to target an antigen bound by those antibodies/antibodies fragments. Other adaptors are known in the art (*e.g.*, WO2019099440, WO2016154621, WO2016168766, WO2016168773, WO2018075807 and Diana Darowski et al, MABS, 2019, 11, 4, 621-631) and can be used in alternate embodiment of the disclosure.

The disclosure provides useful configuration and location for incorporation of adaptors comprising leucine zipper domains (*e.g.*, RZIP, EZIP *etc.*) in a SAR. The disclosure provides useful configurations and locations for incorporation of multiple adaptors. The disclosure provides SAR constructs comprising 1, 2, 3 or more adaptors, *e.g*., adaptors comprising leucine zipper domains or other protein interaction domains described herein. In an embodiment, the SARs comprise two or more adaptors of the same type (*e.g.*, 2 RZIP domains or 2 E4 domains). In other embodiments, the SARs comprise two or more adaptors of different types (*e.g.*, 1 RZIP and 1 E4, or 1 EZIP and 1 D domain *etc.*)*.*

The disclosure provides several configurations and architectures for construction of SARs with AABDs comprising one or more adaptor binding domain. A number of these configurations have been described in the preceding sections in the description of useful configurations for designing SARs incorporating different AABD. In an embodiment, the disclosure provides that a useful location for attachment of an adaptor binding domain (*e.g*., RZIP, EZIP, K4, E4, NKG2D-YA, NKG2D-AF, D domain *etc.*) to a SAR is at or near the N-terminus of one or more fragments comprising its antigen binding domain (*e.g*., vL, vH, scFv, AABD, vHH, FHVH, SVH, SVL, non-immunolgobulin antigen binding scaffold, Va, Vb, Vg, Vd, svd-TCR *etc.*) via an optional linker. In an embodiment, the SAR is a single chain SAR. In another embodiment, the SAR is a multi-chain SAR. In an embodiment, an optional linker is present between the adaptor binding domain of the SAR and one or more fragments comprising its antigen binding domain. In an embodiment, the disclosure provides that a useful location for attachment of an adaptor binding domain (*e.g*., RZIP, EZIP, K4, E4, NKG2D-YA, NKG2D-AF, D domain *etc.*) to a SAR is at the N-terminus or near the N-terminus of an Ig linker, an Ig like linker or a long linker (*e.g.* linker more than 25 amino acids in length) which, in turn, is attached to a signaling chain (*e.g*., connecting peptide of a TCRα, TCRβ, TCRγ, TCRδ *etc.*)*.* The disclosure provides useful configuration and location for incorporation of adaptors comprising leucine zipper domains (*e.g.*, RZIP, EZIP *etc.*) in a SAR. The disclosure also provides useful configurations and locations for incorporation of multiple adaptors binding domains. The disclosure provides SAR constructs comprising 1, 2, 3 or more adaptor binding domains, *e.g*., adaptor binding domains comprising leucine zipper domains, D domains or other protein interaction domains described herein. In an embodiment, the SARs comprise two or more adaptor binding domains of the same type (*e.g.*, 2 RZIP domains or 2 E4 domains). In other embodiments, the SARs comprise two or more adaptor binding domains of different types (*e.g.*, 1 RZIP and 1 E4, or 1 EZIP and 1 D domain *etc.*)*.* In an embodiment, the two or more adaptor binding domains are located on the same polypeptide chain of the SAR. In an embodiment, the two or more adaptor binding domains are located on the same polypeptide chain of the SAR and are separated from each other by linkers. In exemplary embodiments, the linker can be a flexible linker (*e.g.*, SEQ ID NO: 1024-1028) or protease cleavable linker (see **Table 19).** In an embodiment, the two or more adaptor binding domains are located on different polypeptide chains of a multi-chain SAR (*e.g.*, a double chain SIR).

Exemplary SARs targeting EZIP and CD19 are represented by SEQ ID NO (DNA): 4341-4367. Exemplary SARs targeting EZIP, CD22 and CD19 are represented by SEQ ID NO (DNA): 4417-4463. These SAR constructs bind to EZIP domain via a RZIP domain (SEQ ID NO (DNA): 1014 and SEQ ID NO (PRT): 11704). The immune cells expressing these SARs can be used to target CD19 and/or CD22 expressing cells but can be redirected to target other antigens in combination with suitable antibody, antibody fragments and AABD (*e.g*., non-immunoglobulin antigen binding scaffolds) comprising a EZIP domain.

SARs can also be constructed by replacing the RZIP domain (SEQ ID NO (DNA): 1014 and SEQ ID NO (PRT): 11704) of the SARs in SEQ ID NO: 4341-4367 or 4417-4463 with an EZIP domain (SEQ ID NO (DNA): 1015 and SEQ ID NO (PRT): 11705). The immune cells expressing these SARs can be used to target CD19 and/or CD22 expressing cells but can be redirected to target other antigens in combination with suitable antibody, antibody fragments and AABD (*e.g*., non-immunoglobulin antigen binding scaffolds) comprising a RZIP domain.

An exemplary SAR on the MC7G5 backbone in which the CD20-vHH-2HCD25 domain is replaced by the adaptor-binding module RZIP is represented by CD8SP-MC7G5-Vb-[hTCRb-S57C]-F-P2A-IgSP-RZIP-G4Sx3v2-MC7G5-Va-[hTCRa-T48C] and has nucleic acid SEQ ID NO: 3699 and amino acid SEQ ID NO:14389. Other exemplary SARs in which the CD20-vHH-2HCD25 domain of SARs listed in **Table 27** is replaced by a RZIP are represented by nucleic acid SEQ ID NO: 3695-3792 and amino acid SEQ ID NO:14385-14482 **(Table 28).** The order of these constructs is similar to the order of the CD20-vHH-2HCD25 containing constructs listed in **Table 27.** The RZIP containing constructs can be used as universal SARs to target different antigens when combined with antigen binding domains (*e.g.*, scFv, FHVH, vHH, centyrins *etc.*) comprising EZIP modules that can bind to the RZIP module present on the SARs. Exemplary scFVs-EZIP fusion proteins targeting different antigens are represented by nucleic acid SEQ ID NO:1333-1521 and amino acid SEQ ID NO: 12023- 12111. These exemplary scFv-EZIP fusion proteins are based on scFv fragments presented by nucleic acid SEQ ID NO: 530-766 and amino acid SEQ ID NO: 11220-11456 **(Table 3)** and their target antigen can be determined from the target antigens of their scFvs as shown in **Table 3.** In an exemplary embodiment, the T cells expressing the SAR CD8SP-MC7G5-Vb-[hTCRb-S57C]-F-P2A-IgSP-Apa-RZIP-G4Sx3v2-MC7G5-Va-[hTCRa-T48C] can be directed to BCMA expressing myeloma cells by administering to the subject BCMA-J6M0-scFv-G4S-EZIP fusion protein represented by nucleic acid SEQ ID NO: 1336 and amino acid SEQ ID NO:12026. On the other hand, the T cells expressing the SAR CD8SP-MC7G5-Vb-[hTCRb-S57C]-F-P2A-IgSP-Apa-RZIP-G4Sx3v2-MC7G5-Va-[hTCRa-T48C] can be directed to CD19 expressing lymphoma cells by administering to the subject FMC63-scFv-G4S-EZIP fusion protein represented by nucleic acid SEQ ID NO: 1341 and amino acid SEQ ID NO:12031. Thus, T cells expressing SAR construct containing the RZIP module can serve as universal SAR-T cells that can be directed to different target antigens when combined with different scFv-EZIP fusion proteins targeting those antigens.

The RZIP containing SARs can be also used in combination with non-scFv based EZip fusion proteins. Exemplary non-scFv based EZip fusion proteins targeting different antigens are provided in nucleic acid SEQ ID NO:1522-1590 and amino acid SEQ ID NO:12212-12280. The antigen binding domains (*e.g.*, VHH, SVH, Centyrins *etc.)* of these EZip fusion proteins is based on the antigen binding domains represented by SEQ ID NO; 834-902 and amino acid SEQ ID NO:11524 -11592 as shown in **Table 5.** Additional EZIP comprising fusion proteins and their method of use to redirect immune cells expresing RZIP comprising constructs is described in Cho et al, Cell 173, 1426-1438, 2018, which is incorporated in its entirety by reference herein.

A number of additional adaptor and adaptor binding modules are known in the art. Exemplary adaptors and adaptor binding modules are listed in **Table 10.** The disclosure provides that SAR constructs can be constructed by replacing the RZIP modules of the SARs described in the preceding examples with E4 (SEQ ID NO: 1016), NKG2D-AF (SEQ ID NO:1018), NKG2D-YA (SEQ ID NO:1019), NKG2D-AF-G4Sx3-NKG2D-AF (SEQ ID NO:1020), NKG2D-YA-G4Sx3-or NKG2D-YA (SEQ ID NO:1021). Similarly, the EZIP modules in the EZip fusion proteins described in the preceding examples can be replaced by K4 (SEQ ID NO: 1017), ULBP2R (SEQ ID NO: 1022) and ULBP2-S3 (SEQ ID NO: 1023).

In an exemplary embodiment, the SAR constructs comprising E4 modules can be used in combination with K4 containing SAR adaptors to target cells expressing the antigen targeted by the antigen binding domain (*e.g.*, scFv, vHH, FHVH *etc.*) of the SAR adaptors. Exemplary SARs comprising E4 modules are presented in SEQ ID NO: 4468-4490 and 4544-4590. Exemplary SAR adaptors comprising scFv and vHH/FHVH attached to a K4 domain are presented in SEQ ID NO (PRT): 12281-12469 and 12470-12538, respectively and their target antigens can be determined from the target antigens of their scFvs and vHH/FHVH as shown in **Table 3 and Table 5,** respectively.

The location of the RZIP and EZIP modules can be switched so that the adaptor binding domain of the SAR may comprise an EZIP domain while the SAR adaptors may comprise a RZIP. Similarly, the location of the K4 and E4 domains can be switched between the SAR and the SAR adaptor.

The application WO2020159941, which is incorporated in its entirety by reference herein, describes modified, non-natural αl-α2 domains of NKG2D ligands (*e.g*., ULBP2R and ULBP2-S3) with attached polypeptides having specific target-binding properties, for example, antibodies or fragments of antibodies, that can be selectively delivered to CAR comprised of modified, non-natural NKG2D receptors (*e.g*., NKG2D-AF and NKG2D-YA) on engineered mammalian cells. However, the appropriate composition and configuration for the incorporation of non-natural NKG2D receptors into next generation CAR constructs (*e.g*., SIR, cTCR, Ab-TCR, TFP and/or TCR *etc.*) is not known.

In an embodiment, the disclosure provides a useful composition and configuration for SARs incorporating non-natural NKG2D receptors (*e.g*., NKG2D-AF and NKG2D-YA *etc.).* In an exemplary embodiment, the disclosure provides that the appropriate location for incorporation of a non-natural NKG2D receptor in a SAR is at or near the N-terminus of one or more fragments comprising its antigen binding domain (*e.g*., vL, vH, scFv, vHH, FHVH, SVH, SVL, non-immunolgobulin antigen binding scaffold, Va, Vb, Vg, Vd, svd-TCR *etc.*) via an optional linker. In an embodiment, the SAR has a single chain or more than one chain. In an embodiment, the disclosure provides that a useful location for the incorporation of a non-natural NKG2D receptor (NKG2D-YA, NKG2D-AF *etc.*) in a SAR is at the N-terminus or near the N-terminus of an Ig linker, an Ig like linker or a long linker (*e.g.*, linker more than 25 amino acids in length) that in turn is attached to a signaling chain (*e.g*., connecting peptide of a TCRα, TCRβ, TCRγ, TCRδ *etc.*)*.* The disclosure also provides useful configurations and locations for incorporation of multiple non-natural NKG2D receptors. The disclosure provides SAR constructs comprising 1, 2, 3 or more non-natural NKG2D receptor. In an embodiment, the SARs comprise two or more non-natural NKG2D receptor of the same type (*e.g.*, 2 NKG2D-YA domains or 2 NKG2D-AF domains). In other embodiments, the SARs comprise two or more non-natural NKG2D receptors of different types (*e.g.*, 1 NKG2D-AF and 1 NKG2D-AF *etc.*)*.* In an embodiment, the two or more non-natural NKG2D receptor are located on the same polypeptide chain of the SAR. In an embodiment, the two or more non-natural NKG2D receptor are located on the same polypeptide chain of the SAR and are separated by linkers. In exemplary embodiments, the linkers can be flexible linkers (*e.g.*, SEQ ID NO: 1024-1028) and/or protease cleavable linkers (see **Table 19).** In an embodiment, the two or more non-natural NKG2D receptor are located on different polypeptide chains of a multi-chain SAR (*e.g.*, a double chain SIR).

In an exemplary embodiment, the SAR constructs comprising NKG2D-AF or NKG2D-AF-G4Sx3-NKG2D-AF modules can be used in conjunction with ULBP2R fusion proteins to target cells expressing the antigen targeted by the antigen binding domain (*e.g*., scFv, vHH, FHVH *etc.*) of the latter. An exemplary ULBP2R fusion protein targeting CD8SP-BCMA-FHVH93-GS-ULBP2R is represented by SEQ ID NO (DNA): 1013 and SEQ ID NO (PRT): 11703 and can be used to redirect a SAR comprising a NKG2D-AF or a NKG2D-AF-G4Sx3-NKG2D-AF module to BCMA expressing cells.

In another exemplary embodiment, the SAR constructs comprising NKG2D-YA or NKG2D-YA-G4Sx3-NKG2D-YA modules can be used in conjunction with ULBP2-S3 fusion proteins to target cells expressing the antigen targeted by the antigen binding domain (*e.g.*, scFv, vHH, FHVH *etc.*) of the latter. An exemplary ULBP2-S3 fusion protein targeting CD8SP-BCMA-FHVH93-GS-ULBP2-S3 is represented by SEQ ID NO (DNA): 1041 and SEQ ID NO (PRT): 11731 and can be used to redirect a SAR comprising a NKG2D-YA or a NKG2D-YA-G4Sx3-NKG2D-YA module to BCMA expressing cells. In another exemplary embodiment, a SAR comprising NKG2D-YA is CD8SP-BCMA-FHVH93-G4S-EcoR1-humROO5-vL-IgCL-TCRb-wt2-opt-6MD-F-P2A-IgH-SP-NKG2D-YA-G4Sx3-NKG2D-YA-G4S-CD19-hu-mROO5-1-vH-IgG1-CH1-TCRa-wt2-opt-6MD-F-F2A-PAC (SEQ ID NO: 23102). The T cells expressing this SAR target BCMA and CD19 but can be directed to target PSMA expressing cells by exposure to the supernatant from cells expressing the construct CD8SP-PSMA-USC76-chVH-G4S-ULBP2-S3-G2S2-streptagII-F-P3A-EcoR1-hGlul-nonopt-T2A-eGFPter (SEQ ID NO: 23114) which comprises a PSMA targeting single chain vH in fusion with ULBP2. In an alternate embodiment, the disclosure provides a NKG2D-YA-G4Sx3-NKG2D-YA encoding SAR (*e.g.*, SEQ ID NO: 23102) that can be used in combination with a ULBP2-S3 encoding polypeptide (*e.g*., CD8SP-BCMA-FHVH93-GS-ULBP2-S3, SEQ ID NO: 11731) to target BCMA expressing cells. Methods to use NKG2D-AF, NKG2D-YA and other non-natural variants of NKG2D in combination with non-natutral NKG2D ligands (*e.g*., ULBP2R and ULBP2-S3) operably linked to heterologous proteins are described in WO2019191243 and WO2020159941, which are incorporated in their entirety by reference herein.

In an embodiment, the disclosure provides a useful composition and configuration for SAR incorporating D domains, which are derived from de novo-designed a-helical bundle, α3D. D domains are relatively small (approximately 1/3 the size of scFv), single-domain structures that lack disulfide bonds and N-linked glycosylation and can be engineered to minimize immunogenicity (Qin et al, Molecular Therapy 27, 7, 2019). In an embodiment, the disclosure provides that a useful location for attachment of a D domain to a SAR is at or near the N-terminus of one or more vL, vH, scFv, vHH, AABD, FHVH, SVH, SVL, non-immunolgobulin antigen binding scaffold, Va, Vb, Vg, Vd, svd-TCR fragments comprising the SAR via an optional linker. In an exemplary embodiment, the disclosure provides that the appropriate location for incorporation of a D domain in a SAR is at or near the N-terminus of one or more fragments comprising its antigen binding domain (*e.g*., vL, vH, scFv, vHH, FHVH, SVH, SVL, non-immunolgobulin antigen binding scaffold, Va, Vb, Vg, Vd, svd-TCR *etc.*) via an optional linker. In an embodiment, the SAR has a single chain or more than one chain. In an embodiment, the disclosure provides that a useful location for the attachment of a D domain followed by an optional linker in a SAR is at the N-terminus or near the N-terminus of an Ig linker, an Ig like linker or a long linker (*e.g*., linker more than 25 amino acids in length), which in turn is attached to a signaling chain (*e.g*., connecting peptide of a TCRα, TCRβ, TCRγ, TCRδ *etc.*)*.* The disclosure also provides useful configurations and locations for incorporation of multiple D domains. The disclosure provides SAR constructs comprising 1, 2, 3 or more D domains. In an embodiment, the SARs comprise two or more adaptor binding domains comprising of D domains. In other embodiments, the SARs comprise two or more adaptor binding domains of different types (*e.g.*, 1 D domain and 1 RZIP *etc.*)*.* In an embodiment, the two or more D domains are located on the same polypeptide chain of the SAR. In an embodiment, the two or more D domains are located on the same polypeptide chain of the SAR and are separated by linkers. In exemplary embodiments, the linkers can be flexible linkers (*e.g.*, SEQ ID NO: 1024-1028) and/or protease cleavable linkers (see **Table 19).** In an embodiment, the two or more D domains are located on different polypeptide chains of a multi-chain SAR (e*.g.*, a double chain SIR).

In an exemplary embodiment, SAR comprising a D domain targeting p26 protein is CD8SP-Af03-G4S-EcoR1-hu-mROO5-vL-[hTCRb-S57C]-F-P2A-SP-CD19-hu-mROO5-1-vH-[hTCRa-T48C-opt]-F-F2A-PAC (SEQ ID NO: 23107). T cells expressing this SAR can be directed by target CD123 expressing cells by exposure to the SAR adaptor CD8SP-bc40-p26-CD123-cg06-GGS-Nluc-FlagX4-Streptag-GGS-8XHis-T2A-PAC (SEQ ID NO: 23120), which expresses p26 in fusion with a D domain targeting CD123. Methods of using D domain containing proteins to target conventional CAR-T cells have been described in WO2019099440, which is incorporated in its entirety by reference herein.

Other SARs that are based on the modular architecture of the SARs comprising the adaptor binding domains described in the preceding sections are also within the scope of this disclosure.

In an alternate embodiment, the adaptor binding domain of a SAR may comprise of vL and vH fragments that combine to form an Fv. In an exemplary embodiment, a SAR targeting Streptag is represented by CD8SP-CD19-FHVH-354-G4S3-Streptag-vL-[hTCRb-S57C-opt]-F-P2A-SP-Streptag-vH-[hTCRa-T48C-opt] (SEQ ID NO (DNA: 1992 and SEQ ID NO: (PRT): 12682). This SAR construct has the backbone of a SIR and comprises a vL and vH fragments targeting Streptag attached to [hTCRb-S57C-opt] and [hTCRa-T48C-opt] modules, respectively. This SAR construct also comprises an AABD (CD19-FHVH-354) targeting CD19 that is attached to the N-terminus of Streptag vL fragment via a G4S3 flexible linker. Thus, this SAR can target CD19 via the CD19-FHVH-354 domain but, in addition, can be redirected to a different antigen when used in combination with a SAR adaptor comprising an antigen binding moiety (*e.g*., antibody, antibody fragment, vHH, FHVH, non-immunoglobulin antigen binding scaffold *etc.*) tarageting the said antigen and comprising a Streptag. Exemplary SAR adaptors (*e.g.*, scFv, vHH, *etc.)* targeting different antigens and comprising a Streptag are provided in **Tables 21 and 22.** In an exemplary embodiment, this SAR construct can also target BCMA expressing target cells in combination with a SAR adaptor targeting BCMA and carrying one or more Streptags. For example, a SAR adaptor targeting BCMA can be expressed in mammalian cells by the expression of constructs represented by SEQ ID NO: 1247, 1248, 1276 or 1277 and purified from the supernatant.

Exemplary SARs targeting FITC and CD19 are represented by SEQ ID NO: 1978-1979 **(Table 25).** The immune cells expressing these SARs can be used to target CD19 expressing cells but can, in addition, be directed to target other antigens by appropriate FITC-labelled antibodies, antibody fragments and AABDs (*e.g*., non-immunoglobulin antigen binding scaffolds).

SAR constructs can be constructed targeting GCN4 using vL, vH and scFv fragments targeting this protein as described in WO2018075807. Exemplary SAR constructs are provided in SEQ ID NO: 22603-22614. The immune cells expressing these SARs can be directed to target other antigens in combination with suitable antibodies or antibody fragments carrying GCN4 tags. Exemplary GCN4 tags are provided in SEQ ID NO (PRT): 21467-21470. Exemplary SAR adaptors comprising antigen binding moieties (*e.g*., scFv, vHH, FHVH *etc.*) comprising N-terminal GCN4 tags that can be used in conjunction with GCN4-targeted SARs to direct the SAR-expressing cells towards BCMA, PSMA, CD19, MUC16, IL13Ra2, CD19, CD123, MSLN, CD22, MPL (TPO-R), CD30, FLT3, CD33 and Her2 are provided in SEQ ID NO (PRT): 21471-21484, respectively. Additional exemplary antigen binding moieties comprising GCN4 tags and methods of their use are provided in WO2018075807, which is incorporated in its entirety by reference herein.

Other SARs that target Streptag, FITC, GCN4 or any other tag or label and are based on the modular architecture of the SARs described in the preceding section are also within the scope of this disclosure.

The disclosure also provides compositions and useful configurations for SAR that comprise an Fc binding module that is capable of binding to antibodies. An exemplary SAR on the MC7G5 backbone in which the CD20-vHH-2HCD25 domain is replaced by the Fc binding region of low affinity Fc receptor (CD16A-F158V or CD16A-V158) is represented by CD8SP-MC7G5-Vb-[hTCRb-S57C]-F-P2A-IgSP-Apa-CD16-V158-v2-G4Sx3v2-MC7G5-Va-[hTCRa-T48C] and has nucleic acid SEQ ID NO:3797 and amino acid SEQ ID NO: 14487. Other exemplary SARs in which the CD20-vHH-2HCD25 domain of SARs listed in **Table 27** is replaced by the Fc binding portion of CD16A or its mutant (*e.g*., CD16-V158-v2) are represented by nucleic acid SEQ ID NO: 3793-3890 and amino acid SEQ ID NO: 14483-14580 **(Table 28).** The order of these constructs is similar to the order of the CD20-vHH-2HCD25 containing constructs listed in **Table 27.** The CD16A or CD16A-F158V (CD16A-V158) containing constructs can be used as universal SARs to target different antigens when combined with antigen binding domains (*e.g*., antibodies, antibody fragments, bispecific antibodies *etc.*) comprising Fc modules that can bind to the CD16-V158 module present on the SARs. Thus, the T cells expressing the SAR CD8SP-MC7G5-Vb-[hTCRb-S57C]-F-P2A-IgSP-Apa-CD16-V158-v2-G4Sx3v2-MC7G5-Va-[hTCRa-T48C] can be directed to CD20 expressing lymphoma cells by administering to the subject the CD20 monoclonal antibody Rituximab. On the other hand, the T cells expressing the SAR CD8SP-MC7G5-Vb-[hTCRb-S57C]-F-P2A-IgSP-Apa-CD16-V158-v2-G4Sx3v2-MC7G5-Va-[hTCRa-T48C] can be directed to Her2 expressing cancer cells by administering to the subject Herceptin. Thus, T cells expressing SAR construct containing the CD16-V158 module can serve as universal SAR-T cells that can be directed to different target antigens when combined with Fc module containing different antibodies or antibody fragments targeting those antigens. In an embodiment, a SAR may comprise one or two Fc binding modules, *e.g*., one or two Fc binding domains of CD16. In an embodiment, each chain of a double chain SAR comprises a CD16-V158 module. Other SARs that comprise Fc binding domain of CD16 and are based on the modular architecture of the SARs described in the preceding sections are also within the scope of this disclosure.

The disclosure also provides SARs that comprise one or more extracellular Fc binding domains derived from CD32 and CD64. Thus, the CD16A module of the construct with SEQ ID NO: SEQ ID NO:3797 can be replaced by the Fc binding module derived from CD32 to generate SARs that can bind to Fc domain of antibodies, antibody fragments, including bispecific and multispecific antibodies. Other SARs that comprise Fc binding domain of CD32 or CD64 and are based on the modular architecture of the SARs described in the preceding sections are also within the scope of this disclosure.

In an embodiment, the disclosure provides single chain, one-and a half chain, and double chain SARs with the backbone of an AABD-TCR comprising one or more adaptor binding domains that are operably attached to TCR constant chains via intervening Ig linkers. An exemplary such construct is CD8SP-RZIP-[IgCL-TCRb-wt-opt2-6MD]-F-P2A-SP-PSMA-chVH-71v2-[IgG1-CH1-TCRa-wt-opt2-6MD] (SEQ ID NO: 21609) that comprises a RZIP domain operably attached to truncated TCRb chain via an IgCL linker and a PSMA-targeted single vH domain (PSMA-chVH-71v2) operably attached to a truncated TCRa chain via an IgG1-CH1 linker. In an exemplary embodiment, the T cells expressing this SAR construct can target PSMA expressing cells through PSMA-chVH-71v2 but can be redirected to also target BCMA expressing cells when exposed to BCMA-J6M0-scFv-G4S-EZIP fusion protein represented by nucleic acid SEQ ID NO: 1336 and amino acid SEQ ID NO:12026. In the construct CD8SP-RZIP-[IgCL-TCRb-wt-opt2-6MD]-F-P2A-SP-RZIP-[IgG1-CH1-TCRa-wt-opt2-6MD] (SEQ ID NO: 21610), the PSMA-chVH-71v2 module is replaced by a second RZIP module. The constructs with SEQ ID NO: 21611 and 21622 are similar to the constructs with SEQ ID NO: 21609 and 21610 except that the RZIP module(s) are replaced by E4 module(s). The T cells expressing these SAR constructs can be redirected to different antigens upon exposure to K4 fusion polypeptide comprising different antigen binding domains. Similarly, the constructs with SEQ ID NO: 21613 and 21614 are similar to constructs with with SEQ ID NO: 21609 and 21610 except that the RZIP module(s) are replaced by NKG2D-AF modules. The constructs with SEQ ID NO: 21615 and 21616 are similar to constructs with with SEQ ID NO: 21609 and 21610 except that the RZIP module(s) are replaced by CD16-V158 modules. The SAR constructs with SEQ ID NO: 21609 to 21616 are similar to the SAR constructs with SEQ ID NO: 21617 to 21624 except that the [IgCL-TCRb-wt-opt2-6MD] and [IgG1-CH1-TCRa-wt-opt2-6MD] modules are replaced by [IgCL-TCRg-6MD] and [IgCL-TCRd-6MD] modules. Other SARs that are based on the modular architecture of the AABD-TCR described in the preceding sections are also within the scope of this disclosure.

In an embodiment, the disclosure provides a double chain bispecific synthetic antigen receptor, comprising two chains each comprising (a) at least two antigen-specific targeting regions, (b) at least one extracellular linker domain, (c) at least one transmembrane domain, (d) an optional co-stimulatory domain and (e) an optional intracellular signaling domain, wherein one antigen-specific targeting region comprises a Va and/or a Vb fragment derived from a T cell receptor that is capable of dimerizing with the Vb and/or Va fragment present on the second chain, and the second antigen specific targeting domain comprises a AABD. In an embodiment, the AABD is a non-scFv antigen binding domain.

In an embodiment, the disclosure provides a double chain bispecific synthetic antigen receptor, comprising two chains each comprising (a) at least two antigen-specific targeting regions, (b) at least one extracellular linker domain, (c) at least one transmembrane domain, (d) an optional co-stimulatory domain and (e) an optional intracellular signaling domain, wherein one antigen-specific targeting region comprises a Vg and/or a Vd fragment derived from a T cell receptor that is capable of dimerizing with the Vg and/or Vd fragment present on the second chain, and the second antigen specific targeting domain comprises a AABD. In an embodiment, the AABD is a non-scFv antigen binding domain.

The disclosure also provides a useful configuration for making a SAR (*e.g.*, a bispecific or multispecific SIR). In an embodiment, the disclosure provides a SAR (*e.g.*, a bispecific or a multispecific SAR) comprising an AABD that is operably linked to the N-terminus or near the N-terminus of the vL and/or vH domains. In an embodiment, the disclosure provides a a SAR (*e.g.*, a bispecific or a multispecific SAR) comprising an AABD that is operably linked to the N-terminus or near the N-terminus of the Va and/or Vb domains. In an embodiment, the disclosure provides a SAR (*e.g.*, a bispecific or a multispecific SAR) comprising an AABD that is operably linked to the N-terminus or near the N-terminus of the Vg and/or Vd domains. In an embodiment, the disclosure provides a SAR (*e.g.*, a bispecific or a multispecific SAR) comprising an AABD that is operably linked to the N-terminus or near the N-terminus of the vL, vH, Va, Vb, Vd, Vg fragments comprising a CAR (*e.g.*, a 2nd-generation CAR, *e.g.*, a BBz CAR), a SIR, a cTCR, an Ab-TCR, an αβTFP, a γδ TFP or a TCR via an optional linker.

The disclosure describes that a useful configuration for making a bispecific or multispecific CAR involves attachment of one or more AABDs (*e.g*., non-scFV based antigen binding domains) to the N-terminus or near the N-terminus of the vL and/or vH domains comprising the scFv of such a CAR via an optional linker. An exemplary bispecific SAR based on CAR backbone is CD8SP-CD19-vHH-048-G4Sx3-R1-hu-mROO5-1-vL-Gly-Ser-Linker-hu-mROO5-1-vH-Myc-28z (SEQ ID NO: 4976).

The disclosure describes that a useful configuration for making a bispecific or multispecific SIR involves attachment of one or more AABDs (*e.g*., non-scFV based antigen binding domains) to the N-terminus or near the N-terminus of the vL and/or vH domains comprising the Fv of such a SIR via an optional linker. An exemplary bispecific SAR based on SIR backbone is CD8SP-CD19-vHH-048-G4Sx3-R1-hu-mROO5-1-vL-[hTCRb-S57C]-F-P2A-SP-hu-mROO5-1-vH-[hTCRa-T48C] (SEQ ID NO: 4981).

The disclosure describes that a useful configuration for making a bispecific or multispecific cTCR involves attachment of one or more AABDs (*e.g*., non-scFV based antigen binding domains) to the N-terminus or near the N-terminus of the vL and/or vH domains comprising the Fv of such a cTCR via an optional linker.

The disclosure describes that a useful configuration for making a bispecific or multispecific Ab-TCR involves attachment of one or more AABDs (*e.g*., non-scFV based antigen binding domains) to the N-terminus or near the N-terminus of the vL and/or vH domains comprising the Fv of such an Ab-TCR via an optional linker.

The disclosure describes that a useful configuration for making a bispecific or multispecific TFPε/TFPγ/TFPδ/TFPζ involves attachment of one or more AABDs (*e.g*., non-scFV based antigen binding domains) to the N-terminus or near the N-terminus of the vL and/or vH domains of such an TFP via an optional linker.

The disclosure also provides a useful configuration for making a SAR (*e.g.*, a bispecific or multispecific SAR) comprising more than one (*e.g.*, 2, 3, 4, 5 or more) AABDs. In an embodiment, the disclosure provides a SAR (*e.g.*, a bispecific or a multispecific SAR) comprising an AABD that is operably linked to the N-terminus or near the N-terminus of another AABD via an optional linker.

In an embodiment, the disclosure provides a useful configuration for making bispecific and multispecific SARs that have a single chain (*e.g.*, 1^{st} generation CAR, 2^{nd} generation CAR, vFLIP-CAR, TFPε, TFPγ, TFPδ or TFPζ *etc.*), double chains (*e.g.*, double chain SIR, double chain cTCR, double chain zSIR, double chain Ab-TCR, double chain TFP, double chain TCR *etc.*) or one and half chains (*e.g.*, one and half chain cTCR or one and a half chain SIR). The names and SEQ ID NOs of exemplary bispecific and multispecific SARs on various backbones and comprising hu-mROO5-1 vL and vH fragments, BCMA-FHVH-33, CD22-FHVH-158, and/or CD19-vHH-048 are provided in **Table 31.** Exemplary SAR constructs in which the BCMA-FHVH-33, CD22-FHVH-158, and/or CD19-vHH-048 modules of constructs in Table 31 are replaced by other AABD modules are provided in **Table 32.** The order of the backbones used in constructs in Table 32 is same as the order of the backbones used in constructs in Table 31. In an embodiment, the SAR has the backbone of a CAR, *e.g.*, a second generation CAR. An exemplary such single chain SAR with the backbone of a 2^{nd} generation CAR is CD8SP-BCMA-FHVH-33-G3Sx2-CD19-vHH-048-G4Sx3-R1-hu-mROO5-1-vL-Gly-Ser-Linker-hu-mROO5-1-vH-Myc-28z (SEQ ID NO: 5070). In an embodiment, the SAR has the backbone of a SIR, *e.g.*, a double chain SIR or a a one and half chain chain. An exemplary such SAR with the backbone of a double chain SIR is represented by CD8SP-BCMA-FHVH-33-G3Sx2-CD19-vHH-048-G4Sx3-R1-hu-mROO5-1-vL-[hTCRa-T48C]-F-P2A-SP-hu-mROO5-1-vH-[hTCRb-S57C] (SEQ ID NO: 5077). An exemplary such SAR on the backbone of a one and a half chain SIR is CD8SP-V5-[hTCRb-KACIAH]-F-P2A-CD8SP-BCMA-FHVH-33-G3Sx2-CD19-vHH-048-G4Sx3-R1-hu-mROO5-1-vL-Gly-Ser-Linker-hu-mROO5-1-vH-Myc-[hTCRa-CSDVP]-F-F2A-PAC (SEQ ID NO: 5079). In an embodiment, the SAR has the backbone of a cTCR. In an embodiment, the SAR has the backbone of an Ab-TCR. Exemplary such SARs with the backbone of an Ab-TCR are CD8SP-BCMA-FHVH-33-G3Sx2-CD19-vHH-048-G4Sx3-R1-hu-mROO5-1-vL-[IgCL-TCRg-6MD]-F-P2A-SP-hu-mROO5-1-vH-[IgG1-CH1-TCRd-6MD] (SEQ ID NO: 5095) and CD8SP-BCMA-FHVH-33-G3Sx2-CD19-vHH-048-G4Sx3-R1-hu-mROO5-1-vL-[IgCL-TCRg-6MD]-F-P2A-SP-hu-mROO5-1-vH-[IgG1-CH1-TCRd-6MD] (SEQ ID NO: 5097). In an embodiment, the SAR has the backbone of a TFP. Exemplary SARs with the backbone of a TFP are represented by CD8SP-BCMA-FHVH-33-G3Sx2-CD19-vHH-048-G4Sx3-R1-hu-mROO5-1-(vL-vH)-CD3e-ECDTMCP-opt2 (SEQ ID NO: 5098), CD8SP-BCMA-FHVH-33-G3Sx2-CD19-vHH-048-G4Sx3-R1-hu-mROO5-1-(vL-vH)-CD3d-ECDTMCP-opt2 (SEQ ID NO: 5099), and CD8SP-BCMA-FHVH-33-G3Sx2-CD19-vHH-048-G4Sx3-R1-hu-mROO5-1-(vL-vH)-CD3g-ECDTMCP-opt2 (SEQ ID NO: 5100). In an embodiment, the AABD is a non-scFV based antigen binding domain. In an exemplary embodiment, the optional linker is a protease cleavable linker. Exemplary protease cleavable linkers are provided in **Table 19.** Exemplary AABD include but are not limited to a SVH domain, a vHH domain, and non-immunoglobulin antigen binding scaffold such as a DARPIN, an affibody, a ZIP domain (*e.g.*, RZIP, EZIP, E4, R4 *etc.*), an affilin, an adnectin, an affitin, an obodies, a repebody, a fynomer, an alphabody, an avimer, an atrimer, a centyrin, a pronectin, an anticalin, a kunitz domain, an Armadillo repeat protein or a fragment thereof; a receptor (*e.g.*, CD16-V158A, NKG2D), a ligand (*e.g.*, APRIL, Thrombopoietin) and the like. In an exemplary embodiment, the AABD for incorporation into a SIR is a soluble domain, *i.e.*, does not aggregate on expression in mammalian cells. In an exemplary embodiment, the AABD for incorporation into a SIR is efficiently secreted in the supernatant when expressed in mammalian cells with an N-terminal signal peptide.

The disclosure describes that a useful configuration for making a bispecific or multispecific zSIR (see WO2019232503, incorporated herein by reference) involves attachment of AABD to the N-terminus or near the N-terminus of the vL and/or vH domain of such a zSIR via an optional linker. In an embodiment, the AABD is a non-scFV based antigen binding domain. An exemplary SAR based on a zSIR backbone is CD8SP-hu-mROO5-1-vL-IgCL-CD3zECDTMCP-opt-F-P2A-Spe-IgSP-Apa-CD20-vHH-2HCD25-G4Sx3v2-hu-mROO5-1-vH-IgG1-CH1-CD3zECDTMCP-opt2-F-F2A-PAC and is represented by nucleic acid SEQ ID NO: 5029 and amino acid SEQ ID NO: 15719. An exemplary multi-specific SAR based on zSIR backbone is CD8SP-BCMA-FHVH-33-G3Sx2-CD19-vHH-048-G4Sx3-R1-hu-mROO5-1-vL-IgCL-CD3zECDTMCP-opt-F-P2A-Spe-IgSP-Apa-CD20-vHH-2HCD25-G4Sx3v2-hu-mROO5-1-vH-IgG1-CH1-CD3zECDTMCP-opt2-F-F2A-PAC and targets BCMA, CD19 and CD20. This SAR is represented by nucleic acid SEQ ID NO: 5122 and amino acid SEQ ID NO: 15812. As SARs are modular in nature, the different modules of this SAR can be replaced to target other antigen(s). Thus, SARs based on zSIR backbone and containing different vL/vH fragments targeting various antigens can be constructed. Similarly, SARs based on zSIR backbone in which the CD20-vHH-2HCD25 module is replaced by other AABD can be constructed to target different antigens. Finally, the linker domains (*i.e.*, IgCL and IgG1-CH1) are optional and can be replaced with different linkers listed in **Table 13** as long as the resulting SAR retains its functional properties (*i.e.*, antigen binding, T cell signaling *etc.*)*.*

The disclosure describes that a useful configuration for making a bispecific or multispecific cTCR involves attachment of AABD (*e.g*., non-scFV based antigen binding domains) to the N-terminus or near the N-terminus of the vL and/or vH domain of such a cTCR via an optional linker. In an exemplary embodiment, the optional linker is a protease cleavable linker.

The disclosure describes that a useful configuration for making a bispecific or multispecific Ab-TCR involves attachment of AABD (*e.g*., non-scFV based antigen binding domains) to the N-terminus or near the N-terminus of the vL and/or vH domain of such Ab-TCR via an optional linker. In an exemplary embodiment, the optional linker is a protease cleavable linker.

The disclosure describes that a useful configuration for making a bispecific or multispecific αβTFP or γδ-TFP involves attachment of an AABD (*e.g*., non-scFV based antigen binding domains) to the N-terminus or near the N-terminus of the vL and/or vH domain of such a αβTFP or γδ-TFP via an optional linker.

The disclosure describes that a useful configuration for making a bispecific or multispecific single chain SAR (*e.g.*, 1st generation CAR, 2nd generation CAR, TAC, ζTFP, εTFP, δTFP, γTFP, single chain SIR, single chain cTCR, or a single chain cTCR *etc.*) involves attachment of AABD (*e.g*., non-scFV based antigen binding domains) to the N-terminus or near the N-terminus of the vL and/or vH domain of such a single chain CAR via an optional linker.

The disclosure describes that a useful configuration for making a bispecific or multispecific rTCR (RECOMBINANT TCR) involves attachment of an AABD to the N-terminus or near the N-terminus of the Va and/or Vb domain of such a rTCR via an optional linker. In an exemplary embodiment, the optional linker is a protease cleavable linker.

The disclosure also provides exemplary bispecific and multispecific SARs based on MC.7.G5 (or MC7G5), a monomorphic MHC class I-related protein (MR1)-restricted TCR.

The disclosure also provides exemplary bispecific and multispecific SARs based on IG4, a NY-ESO-1 restricted TCR that targets the NY-ESO1 peptide SLLMWITQC (SEQ ID NO: 21461) in complex with HLA-A2.

The disclosure also provides exemplary bispecific and multispecific SARs based on a CMV pp65-restricted TCR that targets the CMV pp65 peptide NLVPMVATV (SEQ ID NO: 21452) in complex with HLA-A2.

The exemplary SARs described in the preceding examples comprised of an AABD that is operably linked to the N-terminus or near the N-terminus of a vL, vH, Va, Vb, Vg or Vd chain comprising a SIR, cTCR, Ab-TCR, zSIR, αβTFP, γδTFP or TCR. The present disclosure also provides a novel platform of SAR, designated AABD-TCR, in which one or more AABDs are operably linked in frame to a TCR module without the presence of an intervening vL, vH, Va, Vb, Vg and/or Vd chain. In an exemplary AABD-TCR, one AABD module is operably linked to the connecting peptide of a first TCR module via a first intervening linker domain and the second AABD is operably linked to the connecting peptide of a second TCR module via a second intervening linker. In various embodiment, the linker is a flexible linker (*e.g*., Gly-Ser linker). In a preferred embodiment, the linker comprises an immunoglobulin (Ig) domain. In particular, the IgCL (SEQ ID NO (DNA): 1142 and SEQ ID NO (PRT): 3536) and IgCH domains (SEQ ID NO (DNA): 1143-1157 and SEQ ID NO (PRT): 3537-3551) derived from antibodies serve as useful linkers between the AABD and the TCR chain. Additional Ig-domains and/or Ig-like domains are known in the art (*e.g.*, **Table 13;** SEQ ID NO (DNA):1168- 1175 and SEQ ID NO (PRT):3562-3569) and can serve as useful linkers in alternate embodiment of the disclosure. The disclosure also provides novel deletion mutants of the extracellular Ig domains of the TCR constant chains (*e.g.*, Cα, Cβ, Cγ, Cδ) that can serve as linker in the construction of SARs (SEQ ID NO: 11848-11865) **(Table 13).** The extracellular Ig domains of the TCR constant chains (*e.g.*, Cα, Cβ, Cγ or Cδ) can serve as linkers between the antigen binding domain (*e.g*., vL, vH, vHH, DARPIN, FHVH *etc.*) and TCR connecting peptides (SEQ ID NO: 11867-11875) **(Table 14)** of the TCR modules in the construction of SARs with the backbone of AABD-TCR, SIR or cTCR. In an embodiment, the exemplary TCR module are represented by TCRa-wt2-opt-6MD (SEQ ID NOs: 1112), TCRb-wt2-opt-6MD (SEQ ID NO: 1126), TCRg-6MD (SEQ ID NO: 1132) and TCRd-6MD (SEQ ID NO: 1139) or functional variants thereof **(Table 12).** In an embodiment, the TCR modules are capable of recruiting at least one TCR signaling module.

In an embodiment, the disclosure provides a double chain bispecific synthetic antigen receptor where at least one chain comprises (a) one or more antigen-specific targeting domains, (b) at least one linker domain selected from SEQ ID NO: 11832-11865 or domains with at least 70% homology to SEQ ID NO: 11832-11865 (Table 13) or deletion mutants and functional variants thereof, (c) an optional hinge domain selected from SEQ ID NO:11888-11894 or domains with at least 70% homology to SEQ ID NO: 11832-11865 (Table 13) or deletion mutants and functional variants thereof; d) at least one connecting peptide selected from SEQ ID NO: 11867-11875 (Table 14) or domains with at least 70% homology to SEQ ID NO: 11867-11875 or deletion mutants and functional variants thereof, (e) at least one transmembrane domain selected from SEQ ID NO: 11877-11881 or domains with at least 70% homology to SEQ ID NO: 11867-11875 or deletion mutants and functional variants thereof, (f) an optional intracellular signaling domain selected from SEQ ID NO: 11883-11886, wherein the antigen-specific targeting region comprises an AABD. In an exemplary embodiment, the AABD is a non-scFv antigen binding module (*e.g.*, a SVH, vHH, Centyrin, svd-TCR *etc.*)

In an embodiment, the disclosure provides a double chain bispecific synthetic antigen receptor where each chain comprises (a) one or more antigen-specific targeting domain, (b) at least one linker domain selected from SEQ ID NO: 11832-11865 or domains with at least 70% homology to SEQ ID NO: 11832-11865 (Table 13) or deletion mutants and functional variants thereof, (c) an optional hinge domain selected from SEQ ID NO:11888-11894 or domains with at least 70% homology to SEQ ID NO: 11832-11865 (Table 13) or deletion mutants and functional variants thereof; d) at least one connecting peptide selected from SEQ ID NO: 11867-11875 (Table 14) or domains with at least 70% homology to SEQ ID NO: 11867-11875 or deletion mutants and functional variants thereof, (e) at least one transmembrane domain selected from SEQ ID NO: 11877-11881 or domains with at least 70% homology to SEQ ID NO: 11867-11875 or deletion mutants and functional variants thereof, (f) an optional intracellular signaling domain selected from SEQ ID NO: 11883-11886, wherein the antigen-specific targeting region comprises an AABD. In an exemplary embodiment, the AABD is a non-scFv antigen binding module (*e.g.*, a SVH, vHH, Centyrin *etc.*)*.*

An exemplary double chain bispecific SAR targeting CD22 and PSMA is CD8SP-CD22-FHVH-24-[IgCL-TCRg-6MD]-F-P2A-SP-PSMA-chVH-71v2-[IgG1-CH1-TCRd-6MD] and is represented by nucleic acid SEQ ID NO:6492 and amino acid SEQ ID NO: 17182. One of the chains of this construct comprises a SVH targeting CD22 (CD22-FHVH-24) that is fused via an IgCL linker (11832) to a TCRg-6MD module (SEQ ID NO: 11822) comprising the connecting peptide, transmembrane and cytosolic domain of human TCRg/TCRγ. The second chain of this construct comprises an SVH targeting PSMA (PSMA-chVH-71v2) that is fused via an IgG1-CH1 linker (11833) to a TCRd-6MD module (SEQ ID NO: 11829) comprising the connecting peptide, transmembrane and cytosolic domain of human TCRd/TCRd. The two chains of this SAR are separted via a Furine site (SEQ ID NO:11931) and P2A (SEQ ID NO (DNA):1236 and SEQ ID NO (PRT): 11926) self-cleavable linker.

Another exemplary double chain bispecific SAR targeting CD22 and PSMA is CD8SP-CD22-FHVH-24-[IgCL-TCRb-wt-opt2-6MD]-F-P2A-SP-PSMA-chVH-71v2-[IgG1-CH1-TCRa-wt-op2-6MD] (SEQ ID NO: 6521). This SAR resembles the SAR described in the preceding section except that the TCRg-6MD module is replaced by TCRb-wt-opt2-6MD module (SEQ ID NO (DNA): 1126 and SEQ ID NO (PRT): 11816) and the TCRd-6MD is replaced by TCRa-wt-opt2-6MD module (SEQ ID NO (DNA): 1112 and SEQ ID NO (PRT): 11802).

An exemplary double chain SAR on the backbone of an AABD-TCR targeting CD20 and CD22 is CD8SP-CD20-vHH-USC1-2HC2D6-IgG1-Hinge-[TCRg-6MD]-F-P2ASP-CD22-USC1-FHVH-160-IgG1-Hinge-v2-[TCRd-6MD] and is represented by SEQ ID NO: 17883. Exemplary SARs on the backbone of AABD-TCRs targeting CD20 and CD22 are CD8SP-CD20-vHH-USC1-2HC2D6-[IgCL-TCRb-wt-opt2-6MD]-F-P2A-SP-CD22-USC1-FHVH-160-[IgG1-CH1-TCRa-wt-opt2-6MD] (SEQ ID NO: 17868) and CD8SP-CD20-vHH-USC1-2HC2D6-[IgCL-TCRg-6MD]-F-P2A-SP-CD22-USC1-FHVH-160-[IgG1-CH1-TCRd-6MD](SEQ ID NO: 17877). Exemplary SARs on the backbone of AABD-TCRs targeting BCMA and CD38 are CD8SP-BCMA-FHVH-74-[IgCL-TCRg-6MD]-F-P2A-SP-CD38-FHVH-USC1-32184-[IgG1-CH1-TCRd-6MD] and CD8SP-BCMA-FHVH-74-[IgCL-TCRb-wt-opt2-6MD]-F-P2A-SP-CD38-FHVH-USC1-32184-[IgG1-CH1-TCRa-wt-op2-6MD] and are represented by SEQ ID NO: 17912 and 17914. Exemplary SARs on the backbone of AABD-TCRs targeting CD20 and CD19 are CD8SP-CD20-vHH-USC1-2HC2D6-[IgCL-TCRb-wt-opt2-6MD]-F-P2A-SP-CD19-FHVH-354-[IgG1-CH1-TCRa-wt-opt2-6MD] and CD8SP-CD20-vHH-USC1-2HC2D6-[IgCL-TCRg-6MD]-F-P2A-SP-CD19-FHVH-354-[IgG1-CH1-TCRd-6MD] and are represented by SEQ ID Nos: 18016 and 18023, respectively. As SARs are modular in nature, the AABD of the above SARs can be substituted with AABD targeting different antigens to develop SARs targeting those antigens. Exemplary AABDs are provided in **Tables 5, 7-10.** Similarly, the IgCL and IgG1-CH1 linkers in AABD-TCRs can be replaced by other linkers. Exemplary Ig-like linkers are provided in **Table 13.** Additoinal Ig like linker domains are known in the art and can be used in alternate embodiment of the disclosure. Finally, the TCRa-wt-op2-6MD, TCRb-wt-op2-6MD, TCRg-6MD, and TCRd-6MD modules can be replaced by their functional variants, including mutants and protein domains with at least 70% homology to the sequences of TCRa-wt-op2-6MD, TCRb-wt-op2-6MD, TCRg-6MD, and TCRd-6MD at the amino acid level.

**Table 33** provides the names and SEQ ID NOs of several single and double chain bispecific SAR constructs on the AABD-TCR backbone targeting CD22 and/or PSMA. **Table 34** provides the names and SEQ ID NOs of several single and double chain bispecific SAR constructs targeting different antigens which are derived by replacing the SVH targeting CD22 and/or PSMA from the construct listed in **Table** 13 with SVH targeting different antigens. The backbone (*i.e.*, framework minus the antigen binding domain) of the constructs in **Table 34** is the same as the backbones of the constructs listed in **Table 33.** Therefore, the SEQ ID of a construct with a particular backbone listed in **Table 34** can be determined by reference to **Table 33.**

In one aspect, the present disclosure describes a useful configuration of a SAR for targeting two or more antigens. In one aspect, the present disclosure describes a useful configuration of a SAR for targeting two or more epitopes of one or more antigens.

In some embodiments, a SAR may comprise or consist of a single antigen binding domain joined to a single T cell receptor constant chain. The single chain SAR may pair with a complementary endogenous TCR chain to assemble a TCR/CD3 complex which is capable of binding an antigen targeted by the antigen binding domain of the SAR and transducing a T cell signal. In some embodiments, a SAR may comprise or consist of more than one antigen binding fragments (*e.g.*, SVH, vHH, Centyrin *etc.*) that are joined via a linker and are in turn joined to a single T cell receptor constant chain. In another embodiments, a SAR comprises or consists of two antigen binding domains that are each joined in frame to a separate T cell receptor constant chain. For example, antigen binding domain 1 is joined to the constant chain of TCRα (Cα) to constitute functional unit 1 and antigen binding domain 2 is joined to the constant chain of TCRβ (Cβ) to constitute functional unit 2. The two functional units of such SAR are coexpressed in the same cell to become functionally active. In some embodiments, the two functional units of the SAR are coexpressed using a single vector, while in other embodiments the two functional units are coexpressed in the same cells using different vectors. In some embodiments, the two functional units of the SAR are coexpressed by transfection of a single mRNA sequence that encodes for both functional units, while in other embodiments the two functional units are coexpressed by transfection of two different mRNA sequences, each encoding for one functional unit.

In yet another embodiment, a SAR comprises or consists of an antigen binding domain that is joined to one T cell receptor constant chain (functional unit 1) but is coexpressed with a second T cell receptor constant chain. The purpose of the second T cell receptor constant chain in such a SAR is to facilitate the cell surface expression of the functional unit 1 (*e.g*., antigen binding domain 1 joined to a T cell receptor constant chain). As such, the second T cell receptor constant chain may be expressed by itself or expressed as a fusion protein carrying an epitope tag (*e.g.*, MYC, V5, AcV5, G4Sx2, StrepTagII *etc.*) or expressed as a fusion protein carrying any irrelevant protein fragment (*e.g*., vL or vH fragment) that does not interfere with the assembly and function of the functional unit 1. As an example, a SAR may comprise or consist of antigen binding domain 1 joined to Cα (constant chain of TCRα) and an empty (*i.e.*, lacking an antigen binding domain) Cβ (constant chain of TCRβ). The two functional units of such SAR are coexpressed in the same cell to become functionally active. In some embodiments, the two functional units of the SAR are coexpressed using a single vector, while in other embodiments the two functional units are coexpressed in the same cells using different vectors. In some embodiments, the two functional units of the SAR are coexpressed by transfection of a single mRNA sequence that encodes for both functional units, while in other embodiments the two functional units are coexpressed by transfection of two different mRNA sequences, each encoding for one functional unit.

In addition to AABD, the antigen binding domain(s) of single chain SARs (*e.g.*, 1st, 2nd and 3rd generation chimeric antigen receptors, ε/γ/δ/TFPs, TAC and the like) and multiple chain SARs (*e.g.*, SIR, zSIR, cTCR, ab-TCRs, αβTFP, or γdTFP *etc.*) can comprise of antibody or antibody fragments (vL, vH, Fv, Fab, or scFv *etc.).* In some SARs (*e.g.*, SIR, zSIR, cTCR, Ab-TCR *etc.*), the vL and vH fragments are joined to different TCR constant chains or fragments thereof at their C-termini via optional linkers but join together to form Fv that retains the antigen binding specificity of the parent antibody from which the vL and vH fragments are derived. In the case of bispecific and multispecific SARs, one or more AABD are operably linked at or near the N-termini of the vL and/or vH fragments.

In one embodiment, the antigen binding domain(s) of a SAR of the disclosure comprises a Fv (*e.g.*, a vL fragment and a vH fragment that are operably linked to different TCR constant chains and are not present in a single chain fragment variable format or an scFv format) and at least one AABD (*e.g.*, SVH, VHH, Centyrin *etc.*) that is specific for the same antigen as bound by the Fv, thus providing a bivalent binding molecule. In one embodiment, the antigen binding domain comprises a Fv and at least one AABD (*e.g*., SVH, VHH, Centyrin *etc.*) that is specific for the same antigen as the Fv but bind to different epitopes on said antigen. In other words, the antigen binding domain comprises a Fv that binds to a first epitope and a second AABD (*e.g.*, SVH, VHH, Centyrin *etc.*) that binds to a second epitope. The epitopes may be overlapping. Thus, the antigen binding domain is biparatopic and the scope of the disclosure includes a biparatopic SAR. In yet another embodiment, the antigen binding domain comprises a Fv and at least one AABD (*e.g.*, SVH, VHH, Centyrin *etc.*) that are specific for the same antigen and bind to the same epitopes on said antigen. In yet another embodiment, the antigen binding domain comprises a Fv and at least one AABD (*e.g*., SVH, VHH, Centyrin *etc.*) where the Fv fragment does not bind to any specific antigen with significant affinity or binds with insignificant affinity and merely serves as a scaffold for the attachment of the one or more AABD.

In addition to vH and SVH, the AABD comprising the antigen binding domain of SAR may comprise other domains, such as but not limited to a vHH domain, and non-immunoglobulin antigen binding scaffold such as a DARPIN, an affibody, a ZIP domain (*e.g.*, RZIP, EZIP, E4, R4 *etc.*), an affilin, an adnectin, an affitin, an obodies, a repebody, a fynomer, an alphabody, an avimer, an atrimer, a centyrin, a pronectin, an anticalin, a kunitz domain, an Armadillo repeat protein or a fragment thereof; a receptor (*e.g*., CD16-V158A, NKG2D), a ligand (*e.g*., APRIL, Thrombopoietin) and the like.

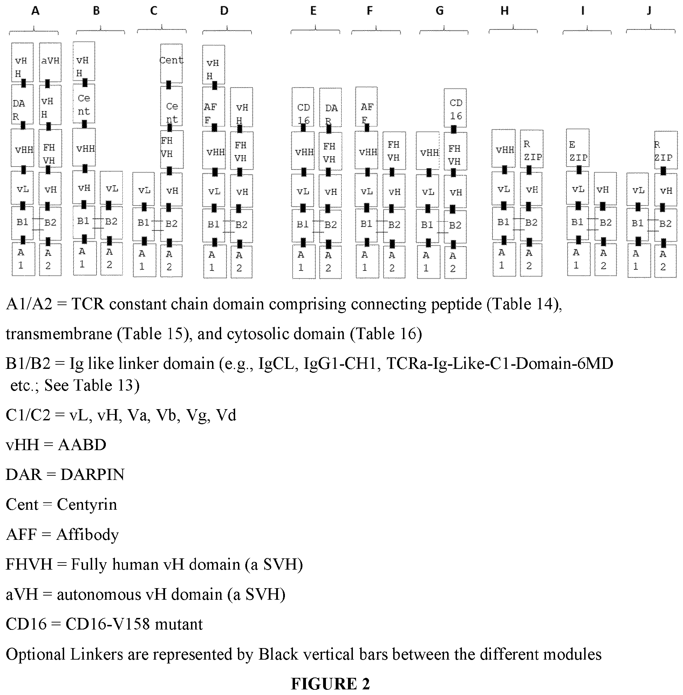

Exemplary configurations of SARs are schematically presented in **Figures 1-2** **and Tables A1-A7.**

In some aspects, the SAR comprises a vL and vH domain that combine to form a Fv targeting a specific antigen and one or more AABD.

In some aspects, the SAR of the disclosure thus comprises one, typically more than one V_{H} domain, *i.e.* one or more V_{H} single domain antibody, and is devoid of light chains. In a preferred embodiment, the AABD comprises at least two V_{H} single domain (SVH) antibodies.

In some aspects, the SAR of the disclosure thus comprises one, typically more than one VHH domain, *i.e.* one or more VHH single domain antibody, and is devoid of light chains. In a preferred embodiment, the AABD comprises at least two VHH single domains.

In some aspects, the SAR of the disclosure thus comprises one, typically more than one non-immunoglobulin antigen binding scaffold, *i.e.* one or more domains selected from a DARPIN, a affibody, a ZIP domain (*e.g.*, RZIP, EZIP, E4, R4 *etc.*), a affilin, an adnectin, an affitin, an obodies, a repebody, a fynomer, an alphabody, an avimer, an atrimer, a centyrin, a pronectin, an anticalin, a kunitz domain, an Armadillo repeat protein or a fragment thereof. In an embodiment, the SAR comprises two AABD. In an embodiment, the SAR comprises a Fv (i.e. vL/vH fragment that combine to form a Fv) and at least one AABD.

In some embodiments, a SAR of the disclosure comprises at least two AABD (*e.g.*, two SVH domains or two VHH domains or one SVH and one VHH domain *etc.*) which target one or more antigen.

In some embodiments, the SAR of the disclosure typically comprises at least two antigen binding domains which target one or more antigen.

In one embodiment, the antigen binding domains of a SAR of the disclosure comprises two or at least two AABD (*e.g.*, SVH, VHH, Centyrin *etc.*) that are specific for the same antigen, thus providing a bivalent binding molecule. In one embodiment, the antigen binding domain comprises two or at least two AABDs (*e.g.*, SVH, VHH, Centyrin *etc.*) that are specific for the same antigen but bind to different epitopes on said antigen. In other words, the antigen binding domain comprises a first AABD (*e.g.*, SVH, VHH, Centyrin *etc.*) that binds to a first epitope and a second AABD (*e.g.*, SVH, VHH, Centyrin *etc.)* that binds to a second epitope. The epitopes may be overlapping. Thus, the antigen binding domain is biparatopic and the scope of the disclosure includes a biparatopic SAR. In yet another embodiment, the antigen binding domain comprises two AABD (*e.g*., SVH, VHH, Centyrin *etc.*) that are specific for the same antigen and bind to the same epitopes on said antigen.

In one embodiment, the antigen binding domains of a SAR of the disclosure comprises a TCR Fv (*e.g.*, a Va/Vb fragment or Vg/Vd fragments that are operably linked to different TCR constant chains and are not present in a single chain TCR format or an scTCR format) and at least one AABD (*e.g.*, SVH, VHH, Centyrin *etc.*) that is specific for the same antigen as bound by the TCR Fv, thus providing a bivalent binding molecule. In one embodiment, the antigen binding domain comprises a TCR Fv (*e.g*., Va/Vb or Vg/Vd) and at least one AABD (*e.g.*, SVH, VHH, Centyrin *etc.*) that is specific for the same antigen as the TCR Fv but bind to different epitopes on said antigen. In other words, the antigen binding domain comprises a TCR Fv that binds to a first epitope and a second AABD (*e.g*., SVH, VHH, Centyrin *etc.*) that binds to a second epitope. The epitopes may be overlapping. Thus, the antigen binding domain is biparatopic and the scope of the disclosure includes a biparatopic SAR. In yet another embodiment, the antigen binding domain comprises a TCR Fv and at least one AABD (*e.g.*, SVH, VHH, Centyrin *etc.*) that are specific for the same antigen and bind to the same epitopes on said antigen. In yet another embodiment, the antigen binding domain comprises a TCR Fv and at least one AABD (*e.g.*, SVH, VHH, Centyrin *etc.*) where the TCR Fv fragment does not bind to any specific antigen with significant affinity or binds with insignificant affinity and merely serves as a scaffold for the attachment of the one or more AABD. In an embodiment, the variable fragments (*i.e.*, Va, Vb, Vd/Vg) are derived from an HLA-independent TCR, *i.e.*, a TCR that can bind to an antigen independent of an HLA molecule. An exemplary HLA-independent TCR comprises variable fragments that can bind to protein antigens that are expressed on cell surface (*e.g*., CD19, CD20, CD22, PSMA, Her2, Mesothelin, *etc.*)*.*

In another embodiment, the antigen binding domain comprises two AABD (*e.g.*, SVH, VHH, Centyrin *etc.*) that are specific for two different antigens, thus providing a bispecific antigen binding domain. In other words, the antigen binding domain comprises a first AABD (*e.g.*, SVH, VHH, Centyrin *etc.*) that binds to a first target and a second AABD (*e.g.*, SVH, VHH, Centyrin *etc.*) that binds to a second target. Thus, in certain embodiments, the disclosure relates to bispecific SARs.

As used herein, the term "bispecific SAR" or "bispecifc antigen binding domain" thus refers to a polypeptide that comprises a binding molecule as described herein which has a binding site that has binding specificity for a first target antigen, and a second polypeptide domain which has a binding site that has binding specificity for a second antigen target, *i.e.*, the bispecific binding molecule has specificity for two targets. The first target and the second target are not the same, *i.e.* are different targets, *e.g.*, proteins; both may be present on a cell surface. Accordingly, a bispecific binding molecule as described herein can selectively and specifically bind to a cell that expresses (or displays on its cell surface) the first target and the second target. In another embodiment, the binding molecule comprises more than two antigen-binding domains providing a multispecific binding molecule. A multispecific antigen-binding domain as described herein can in addition to binding a first target bind one or more additional targets, *i.e.*, a multispecific polypeptide can bind at least two, at least three, at least four, at least five, at least six, or more targets, wherein the multi specific polypeptide agent has at least two, at least, at least three, at least four, at least five, at least six, or more target binding sites respectively.

Antigen binding domains that comprise three or more AABD (*e.g*., SVH, VHH, Centyrin *etc.*) are therefore also within the scope of the invention.

Two or more AABD (*e.g.*, SVH, VHH, Centyrin *etc.)* may be connected by a linker, for example a polypeptide linker. A linker may be also present between the vL and/or vH domain comprising the Fv and the AABD. Suitable linkers, for example comprising linker include GS residues such as (Gly₄Ser)ₙ, where n=from 1 to 10, *e.g.*, 1 , 2, 3, 4, 5, 6, 7, 8, 9 or 10. Exemplary linkers are provided in SEQ ID NO (DNA): 1025-1028 and SEQ ID NO (PRT): 11715-11718 (Table 8).

A linker may be also present between the vL and vH domain comprising the Fv of a SAR and the TCR constant chain connecting peptide to which the vL and vH domains are operably linked. Exemplary such linkers are provided in Table 13. In a preferred embodiment, the vL fragment of the SAR is operably linked to the linker IgCL (SEQ ID NO: 11832) and the vH fragment is operably linked to the linker IgG1-CH1. An exemplary such SAR is CD8SP-CD19-FHVH-354-G4S3-CD19-hu-mROO5-1-vL-[IgCL-TCRg-6MD]-F-P2A-SP-CD22-FHVH-24-CD19-hu-mROO5-1-vH-[IgG1-CH1-TCRd-6MD] and is represented by SEQ ID NO: 18093 (Table 35). In another preferred embodiment, the vL fragment of the SAR is operably linked to the linker TCRa-wt-opt-6ECD (SEQ ID NO:11848) or the linker TCRa-Ig-Like-C1-Domain (SEQ ID NO: 11859) or their deletion mutant or variant and the complementary vH fragment is operably linked to the linker TCRb-wt-opt-6ECD (SEQ ID NO:11850) or the linker TCRb-Ig-Like-C1-Domain (SEQ ID NO: 11861) or their deletion mutant or variant. An exemplary such SAR is CD8SP-CD19-FHVH-354-G4S3-CD19-hu-mROO5-1-vL-[hTCRa-T48C]-F-F2A-SP-CD22-FHVH-24-CD19-hu-mROO5-1-vH-[hTCRb-S57C] and is represented by SEQ ID NO: 18091. In another preferred embodiment, the vL fragment of the SAR is operably linked to the linker TCRg-opt-6ECD ((SEQ ID NO: 11852) or TCRg-Ig-Like-C1-Domain (SEQ ID NO: 11863) or their deletion mutant or variant and the complementary vH fragment is operably linked to the linker TCRd-opt-6ECD (SEQ ID NO: 11854) or TCRd-Ig-Like-C1-Domain (SEQ ID NO: 11864) or their deletion mutant or variant. An exemplary such SAR is CD8SP-hu-mROO5-1-vL-[hTCRd-opt]-F-P2A-IgSP-Apa-CD20-vHH-2HCD25-G4Sx3v2-hu-mROO5-1-vH-[hTCRg1-opt] and is represented by SEQ ID NO: 14788. In some embodiments, potentially any domain can serve as a linker between the vL/vH fragments and the TCR constant chains that does not interfere with the ability of the vL/vH fragments to combine to form a Fv.

It is to be noted that the term TCR constant chain as used herein comprises the TCRb-wt-opt-6ECD or the TCR Ig like C1 domain, the TCR connecting peptide, the TCR transmembrane domain and the TCR cytosolic domain. The different TCR constant chains and their deletion and point mutants are presented in **Table 12.** Thus, the TCRb-wt-opt-6ECD and the TCRb-Ig-like-C1 domain represent the N-terminal portion of the TCRb constant chain. Similarly, TCRa-wt-opt-6ECD (SEQ ID NO:11848) or the linker TCRa-Ig-Like-C1-Domain (SEQ ID NO: 11859) domains represent the N-terminal portion of the TCRa constant chains. Therefore, stated in another way, in some SAR (*e.g.*, a double chain SIR and bispecific SAR incorporating such a SIR) the vL fragment is operably linked to the TCRb constant chain while the vH fragment is operably linked to the TCRa constant chain. Alternatively, in some SAR (*e.g.*, a double chain SIR or a bispecific SAR incorporating such as SIR) the vL fragment is operably linked to the TCRa constant chain while the vH fragment is operably linked to the TCRb constant chain. Similarly, in some SAR (*e.g.*, double chain SIR or a bispecific SAR incorporating such as SIR), the vL fragment is operably linked to the TCRγ constant chain while the vH fragment is operably linked to the TCRδ constant chain. Finally, in some SAR, (*e.g.*, double chain SIR or a bispecific SAR incorporating such as SIR), the vL fragment is operably linked to the TCRδ constant chain while the vH fragment is operably linked to the TCRγ constant chain.

In some embodiments, the one or more AABD comprising the antigen binding domain of the SAR are operably linked to the TCR constant chain connecting peptide **(*e.g.*,** SEQ ID NO (DNA): 1177-1185; **Table 14)** without the intervening vL/vH, Va/Vb or Vg/Vd fragments. In such SAR constructs, designated AABD-TCR, a linker is optionally and typically present between the AABD of the SAR and the TCR constant chain connecting peptide to which the AABD are operably linked. Exemplary such linkers are provided in **Table 13.** In a preferred embodiment, one of the AABDs of a double chain SAR is operably linked to the linker IgCL (SEQ ID NO: 11832) and the other AABD is operably linked to the linker IgG1-CH1 (SEQ ID NO: 11833). An exemplary such SAR with the backbone of an AABD-TCR is CD8SP-CD22-FHVH-24-[IgCL-TCRb-wt-opt2-6MD]-F-P2A-SP-PSMA-chVH-71v2-[IgG1-CH1-TCRa-wt-opt2-6MD] (SEQ ID NO: 6521). Another exemplary such SAR with the backbone of an AABD-TCR is CD8SP-CD22-FHVH-24-[IgCL-TCRg-6MD]-F-P2A-SP-PSMA-chVH-71v2-[IgG1-CH1-TCRd-6MD] (SEQ ID NO: 6530). Alternative Ig-CH1 linker are provided in SEQ ID NO: 11839-11847 (Table 13) and can be used in alternative embodiment of the disclosure.

The one or more AABD (*e.g.*, SVH, VHH, Centyrin *etc*.) which forms the antigen binding unit of the SAR of the invention, "binds" or is "capable of binding" an antigen of interest, *i.e.*, targets, antigen with sufficient affinity such the SAR is useful in therapy in targeting a cell or tissue expressing the antigen.

As used herein, the term "target" refers to a biological molecule (*e.g*., antigen, peptide, polypeptide, protein, lipid, carbohydrate) to which a polypeptide domain which has a binding site can selectively bind. The target can be, for example, an intracellular target (such as an intracellular protein target) or a cell-surface target (such as a membrane protein, *e.g.*, a receptor protein). Preferably, a target is a cell-surface target, such as a cell-surface protein.

In one embodiment, the target of the antigen binding domain of the SAR is a tumor antigen. In one embodiment, the tumor antigen is associated with a hematologic malignancy. In another embodiment, the tumor antigen is associated with a solid tumor. In yet another embodiment, the one or more antigens targeted by the SAR is/are selected from the antigens listed in antigen **Table B.** However, a skilled person would understand that other tumor antigens are also targets within the scope of the invention.

In some embodiments, the antigen binding domain of the SAR polypeptide molecule binds to an antigen in association with HLA-A2. Non-limiting examples of antigens that are recognized in association with HLA-A2 include TARP, WT1, hTERT, gp100, Tyrosinase, MART1, NY-ESO1, CMV pp65, EBV EBNA3c, HIV1 gag, HTLV1-Tax, PR1, CMV pp65, EBV-EBNA3c, Ras G12V mutant, and GAD.

In some embodiments, the antigen binding domain of the SAR polypeptide molecule comprises of an autoantigen or a fragment thereof that binds to an autoantibody. Non-limiting exampls of autoantigen include Dsg1 and Dsg3.

In some embodiments, the antigen binding domain of the SAR polypeptide molecule is derived from or comprises wild-type or non-wild-type sequence of an antibody, an antibody fragment, an scFv, a Fv, a Fab, a (Fab')2, a single domain antibody (SDAB ), a vH or vL domain, a camelid VHH domain, or a non-immunoglobulin scaffold such as a DARPIN, an affibody, an affilin, an adnectin, an affitin, an obodies, a repebody, a fynomer, an alphabody, an avimer, an atrimer, a centyrin, a pronectin, an anticalin, a kunitz domain, an Armadillo repeat protein, an autoantigen, a receptor or a ligand. In some embodiments, the encoded SAR polypeptide contains more than one antigen binding domains. In embodiments, the antigen binding domain is operably linked directly or via an optional linker to the NH2-terminal end of a TCR domain (*i.e.*, constant chains of TCR-alpha, TCR-beta1, TCR-beta2, pretc.R-alpha, pre-TCR-alpha-Del48, TCR-gamma, or TCR-delta). The nucleic acid and amino acid sequences of several exemplary linkers are provided in **Tables 11 and 13.**

In some embodiments, the antigen binding domain of a SAR polypeptide molecule is derived from or comprises of vL and vH domains of an antibody that are separately operably linked to the NH2-terminus of two constant chains of a T cell receptor (*i.e.* constant chains of TCR-alpha, TCR-beta1, TCR-beta2, pretc.R-alpha, pre-TCR-alpha-Del48, TCR-gamma, or TCR-delta, or mutants or variant thereof as described herein) to jointly constitute a Fragment variable (Fv) that binds to a specific antigen. An exemplary such SAR which targets CD19 is provided in CD8SP-hu-mROO5-1-vL-[hTCRb-S57C]-F-P2A-SP-hu-mROO5-1-vH-[hTCRa-T48C] (DNA SEQ ID NO:7348 and amino acid SEQ ID NO:18038). In this SAR, the vL fragment derived from humanized mROO5-1, a CD19 monoclonal antibody, is operably linked to constant region of a mutant human TCRb chain [hTCRb-S57C] while the vH fragment derived from the humanized mROO5-1 monoclonal antibody is operably linked to the constant region of a mutant human TCRα chain [hTCRa-T48C]. This SAR which has the backbone of a SIR can be used to make bispecific and multispecific SARs by attaching the different AABDs to the or near the N-termini of the vL and/or vH fragments. **Table 29** includes the SEQ ID Nos of several exemplary bispecific and trispecific SARs based on the CD8SP-hu-mROO5-1-vL-[hTCRb-S57C]-F-P2A-SP-hu-mROO5-1-vH-[hTCRa-T48C] (DNA SEQ ID NO:7348 and amino acid SEQ ID NO:18038).

In some embodiments, the SAR polypeptide has an antigen binding domain that is expressed as single chain variable fragments (scFv) and is joined to the NH2-terminus of one of the constant chains of a T cell receptor (*i.e.*, constant chains of TCR-alpha, TCR-beta1, TCR-beta2, pretc.R-alpha, pre-TCR-alpha-Del48, TCR-gamma, or TCR-delta, variants or mutants thereof). An exemplary such SAR (SIR) is CD8SP-V5-[hTCRb-KACIAH]-F-P2A-CD8SP-hu-mROO5-1-vL-Gly-Ser-Linker-hu-mROO5-1-vH-Myc-[hTCRa-CSDVP]-F-F2A-PAC (DNA SEQ ID NO: 7352 and PRT SEQ ID NO: 18042). This SAR which has the backbone of a SIR can be used to make bispecific and multispecific SARs by attaching the different AABDs to the or near the N-terminus of the scFv. **Table 29** includes the SEQ ID Nos of several exemplary bispecific and trispecific SARs based on the SAR CD8SP-V5-[hTCRb-KACIAH]-F-P2A-CD8SP-hu-mROO5-1-vL-Gly-Ser-Linker-hu-mROO5-1-vH-Myc-[hTCRa-CSDVP]-F-F2A-PAC (DNA SEQ ID NO: 7352 and PRT SEQ ID NO: 18042). An exemplary such bispecific SAR that targets CD19 and CD22 is CD8SP-V5-[hTCRb-KACIAH]-F-P2A-CD8SP-CD22-FHVH-24-G4Sx3-R1-hu-mROO5-1-vL-Gly-Ser-Linker-hu-mROO5-1-vH-Myc-[hTCRa-CSDVP]-F-F2A-PAC (DNA SEQ ID NO: 4127 and amino acid SEQ ID NO: 14817).

In certain embodiments, the AABD of the two polypeptide chains of a double chain SARs are similar in structure (*e.g*., both AABD are SVH or camelid VHH domain or affibodies or Centyrins). In one embodiment, the AABD of the two polypeptide chains of a double chain SARs are not similar in structure (*e.g*., the first antigen binding domain is a SVH and the second antigen binding domain is a camelid VHH).

In some embodiments, the antigen binding domain of the encoded SAR polypeptides is encoded by a codon optimized nucleotide sequence of the corresponding wild-type sequence or a non-wild-type sequence antibody, single domain antibodies (SDAB ), VH domains, VL domain, camelid VHH domains, or a non-immunoglobulin scaffolds such as DARPINs, affibodies, affilins, adnectins, affitins, obodies, repebodies, fynomers, alphabodies, avimers, atrimers, centyrins, pronectins, anticalins, kunitz domains, Armadillo repeat proteins, autoantigen, receptors or ligands.

In some embodiments, the encoded one or more antigen binding domains of the SAR polypeptide comprise any one or more of light chain variable domain (vL or VL) amino acid sequences of SEQ ID NO 10736 to 10972 wherein up to 20 amino acid residues but no more than 21 amino acids are replaced by any other amino acid residues, or sequences with 70-99.9% identity to amino acid sequences of SEQ ID NO 10736 to 10972, or sequences with 70-100% identity to the complementarity determining regions (CDR's) of SEQ ID NO: 10736 to 10972, or sequences with up to 3 amino acid substitution in each of the three complementarity determining regions of 10736 to 10972. **Table 3** shows the target antigens, names, SEQ ID NO (DNA), SEQ ID NO (PRT), SEQ ID NO (PRT) of scFv of the exemplary vL domains used in this disclosure. In an embodiment, the vL domains can be fully human, humanized, chimeric or non-human in origin.

In some embodiments, the encoded one or more antigen binding domains of the SAR polypeptide comprise any one or more of heavy chain variable domain (vH or VH) amino acid sequences of SEQ ID NO: 10978 to 11214 wherein up to 20 amino acid residues but no more than 21 amino acids are replaced by any other amino acid residues, or sequences with 70-99.9% identity to amino acid sequences of SEQ ID NO In some embodiments, the encoded one or more antigen binding domains of the SAR polypeptide comprise any one or more of heavy chain variable domain (vH or VH) amino acid sequences of SEQ ID NO: 10978 to 11214 wherein up to 20 amino acid residues but no more than 21 amino acids are replaced by any other amino acid residues, or sequences with 70-99.9% identity to amino acid sequences of SEQ ID NO: 10978 to 11214, or sequences with 70-100% identity to the complementarity determining regions (CDR's) of SEQ ID NO: 10978 to 11214, or sequences with up to 3 amino acid substitution in any of the three complementarity determining regions of SEQ ID NO: 10978 to 11214. Table 3 shows the target antigens, names, SEQ ID NO (DNA), SEQ ID NO (PRT), SEQ ID NO (PRT) of scFv of the exemplary vH domains used in this disclosure. In an embodiment, the vH domains can be fully human, humanized, chimeric or non-human in origin.

In some embodiments, the encoded one or more antigen binding domains of the SAR polypeptide comprise any one or more of camelid single domain antibody (vHH or VHH) amino acid sequences of SEQ ID NO: 11517-11592 wherein up to 20 amino acid residues but no more than 21 amino acids are replaced by any other amino acid residues, or sequences with 70-99.9% identity to amino acid sequences of SEQ ID NO 11517-11592, or sequences with up to 3 amino acid substitution in any of the three complementarity determining regions (CDR's) of SEQ I11517-11592. **Table 5** shows the target antigens, names, SEQ ID NO (DNA) and SEQ ID NO (PRT) of the Exemplary vHH domains used in this disclosure. In an exemplary embodiment, the vHH domains can be humanized, chimeric or non-human.

In some embodiments, the encoded one or more antigen binding domains of the SAR polypeptide comprise any one or more of non immunoglobulin antigen binding scaffold amino acid sequences of SEQ ID NO: 11662-11673 wherein up to 20 amino acid residues but no more than 21 amino acids are replaced by any other amino acid residues, or sequences with 70-99% identity to amino acid sequences of SEQ ID NO: 11662-11673. **Table 7** shows the target antigens, names, SEQ ID NO (DNA), SEQ ID NO (PRT), names of the exemplary non immunoglobulin antigen binding scaffold used in this disclosure.

In some embodiments, the encoded one or more antigen binding domains of the SAR polypeptide comprise any one or more of receptor amino acid sequences of SEQ ID NO 11674-11691 wherein up to 20 amino acids but no more than 21 amino acids are replaced by any other amino acid residues, or sequences with 70-99.9% identity to amino acid sequences of SEQ ID NO: 11674-11691. **Table 8** shows the SEQ ID NO (DNA), SEQ ID NO (PRT), and names of receptors that can be used in the construction of SARs.

In some embodiments, the encoded one or more antigen binding domains of the SAR polypeptide comprise an autoantigen amino acid sequences of SEQ ID NO:11687 wherein up to 19 amino acid residues but no more than 20 amino acids are replaced by any other amino acid residues, or sequences with 70-100% identity to amino acid sequences of SEQ ID NO 11687. **Table 8** shows the SEQ ID NO (DNA), SEQ ID NO (PRT), and name of an exemplary autoantigen.

In some embodiments, the encoded one or more antigen binding domains of the SAR molecule comprise any one or more of ligand amino acid sequences of SEQ ID NO:11692 to 11702 wherein up to 20 amino acid residues but no more than 21 amino acids are replaced by any other amino acid residues or sequences with 70-100% identity to amino acid sequences of SEQ ID NO:11692 to 11702. **Table 9** shows the SEQ ID NO (DNA), SEQ ID NO (PRT), and names of different exemplary ligands.

In some embodiments, the encoded one or more antigen binding domains of the SAR polypeptide comprise any one or more of scFv amino acid sequences of SEQ ID NO:11220-11456 wherein up to 40 amino acid residues but no more than 41 amino acids are replaced by any other amino acid residues, or sequences with 70-100% identity to amino acid sequences of SEQ ID NO 11220-11456 or sequences with 70-100% identity in the six complementarity determining regions (CDR's) in each of SEQ ID NO 11220-11456 or sequences with up to 3 substitution in any of the six complementarity determining regions (CDR's) in each of SEQ ID NO11220-11456. **Table 3** shows the target antigens, SEQ ID NO (DNA), SEQ ID NO (PRT), names and amino acid sequences of the exemplary scFVs used in this disclosure. In an embodiment, the scFv can be fully human, humanized, chimeric or non-human in origin.

In some embodiments, the encoded one or more antigen binding domains of the SAR polypeptide comprise any one or more of an antigen binding portion, *e.g*., CDRs, of vL and vH fragments targeting this antigen or domains with up to 3 amino acid substitutions in any of the CDRs of the vL and vH fragments listed in **Table 3.** The sequences of the CDR1-3 of the vL and vH fragments listed in Table 3 can be determined by methods known in the art.

In some embodiments, the encoded one or more antigen binding domains of the SAR polypeptide comprise any one or more of an antigen binding portion, *e.g*., CDRs, of vHH fragments targeting this antigen. The SEQ ID NO of the vHH fragments targeting different antigens are listed in **Table 5** and the sequences of their corresponding CDR1-3 can be determined by methods known in the art.

In one embodiment, an antigen binding domain of a SAR is an antigen binding portion of a receptor known to bind this target antigen.

In some embodiments, the encoded one or more antigen binding domains of the SAR polypeptide comprise any one or more of an antigen binding region of the receptor comprising the SAR polypeptide.

In some embodiments, the encoded one or more antigen binding domains of the SAR polypeptide comprise any one or more of an antigen binding region of the ligand comprising the SAR polypeptide.

In some embodiments, the encoded one or more antigen binding domains of the SAR polypeptide comprise any one or more of an antigen binding region of the non-immunoglobulin scaffold comprising the SAR polypeptide.

In another embodiment, the disclosure provides SARs that bind to the same epitope on the different targets described in **Tables 25-36** as any of the SARs of the disclosure (*i.e.*, SARs that have the ability to cross-compete for binding to the different targets with any of the SARs of the disclosure). In some embodiments, the antigen specific domains of these SARs could be determined from vL fragments, vH fragments and/or scFv fragments of the antibodies that were used as the component of the SAR. In some embodiments, the reference antibodies for cross-competition studies to determine the target-epitope recognized by a SAR of the disclosure described in **Tables 25-36** are vL, vH, scFvs, SVH, vHH, non-immunoglobulin antigen binding domains having SEQ ID Nos. as shown in **Tables 3-9.** In an exemplary embodiment, the reference scFv hu-mROO5-1 represented by SEQ ID NO:11323 can be used in cross-competition studies to determine the target-epitope recognized by hu-mROO5-1-based SARs of the disclosure described in **Tables 25-36.** In some embodiments, the reference AABD fragments for cross-competition studies to determine the target-epitope recognized by a SAR of the disclosure described in **Tables 25-36** are AABD fragments having SEQ ID NOs as shown in **Table 5.** In some embodiments, the reference non-immunoglobulin antigen binding scaffolds for cross-competition studies for cross-competition studies to determine the target-epitope recognized by a SAR of the disclosure described in **Tables 25-36** are non-immunoglobulin antigen binding scaffolds-based AABD having sequences as shown in **Table 7.** In some embodiments, the reference ligands for cross-competition studies to determine the target-epitope recognized by a SAR of the disclosure described in **Tables 25-36** are ligands having SEQ ID Nos listed in **Table 9.** In some embodiments, the reference SARs for cross-competition studies against SARs targeting different targets are SARs having SEQ ID Nos listed in **Tables 25-36.**

The SARs described herein may be encoded by a single polynucleotide chain and translated into a single polypeptide chain, which is subsequently cleaved into different proteins. The nucleic acid molecule encoding a SAR can comprises one or more leader sequences (also known as a signal peptide). In one embodiment, each functional unit (*e.g.*, an antigen binding domain joined to a T cell receptor constant chain plus Furine-SGSG-cleavable linker or a T cell receptor constant chain plus Furine-SGSG-cleavable linker) of a SAR can be preceded by a leader sequence encoding a signal peptide which directs the SAR to the cell surface as a type I transmembrane protein. In one embodiment, the antigen-binding domain of SAR is extracellular-facing. In some embodiments, short nucleic acid sequences (3-9 nucleic acids) comprising restriction enzyme sites are located between the different subunits of a SAR, *e.g*., between a signal sequence and the antigen binding domain of the SAR or between the antigen binding and the TCR chain.

In an embodiment, SARs can be generated with different TCR constant chains. The TCR constant chains may be encoded by their wild-type sequences, non-wild-type sequences or codon optimized sequences. In addition, the TCR constant chains may carry specific mutations (*e.g*., TCRβ constant chain with one or more mutation to enhance their cell surface expression and/or pairing with each other and to reduce pairing with endogenous TCR chains. Exemplary TCR chains that can be used in the construction of the SARs are provided in **Table 12.** The mutations in the TCR domain of a SAR modify the binding affinity and/or expression of the SAR to a target or cell, respectively. For example, the disclosure contemplates a diverse population of SARs against a particular antigen target that can be designed and screened based upon the nucleic acid sequence codon optimization and/or the mutation in the TCR chain to promote pairing or expression and/or the use of a linker between the binding domain and the TCR domain. In some embodiments, an immune effector cell expressing a SAR from the pool shows more than 2 fold, more than 5-fold, more than 10-fold, and even more than 100-fold difference in one or more of the characteristics selected from the group of antigen binding affinity, cell surface expression, cell signaling, NFAT reporter activity, cytotoxicity, cytokine secretion, proliferation, *in vivo* persistence, expression of exhaustion markers, and *in vivo* activity as compared to a comparable immune effector cell expressing another SAR from the pool containing the same binding domain, (*e.g.*, a binding domain derived from the same scFv as is present in the test SAR) when assayed under similar conditions. The disclosure contemplates a library of X-SAR molecules wherein X is the antigen binding domain target such that library or "pool" provides SARs with varied binding affinity, expression levels and functional characteristics (*e.g*., cytotoxicity, cytokine production and long-term persistence). In some embodiments, the a SAR in the pool have more than 2 fold, typically more than 5-fold, even more preferebaly more than 10-fold, and even more typically more than 100-fold difference in one or more of the characteristics selected from the group of antigen binding affinity, cell surface expression, cytotoxicity, cytokine secretion, T cell proliferation, T cell persistence, T cell exhaustion, and *in vivo* activity when expressed in an immune effector cell as compared to another SAR in the pool containing the same binding domain, (*e.g.*, a binding domain derived from the same scFv as is present in the test SAR) when assayed under similar conditions. The different SARs in the pool may be tagged with different DNA barcodes to allow their identification by next-genreation sequencing or other techniques known in the art. Exemplary barcodes are presented by SEQ ID NO: 25 to 30. The barcodes may be inserted in the vector encoding the SAR at a convenient location so that they do not interfere with the expression of the SAR. In an exemplary embodiment, the barcodes are inserted immediately downstream of the stop codon of the SAR. One of skill in the art can screen such pools to identify X-SIRs with a desired binding affinity, expression level or functional characteristics using any one or more of the assays described herein. Different SARs or different pools of SARs may be suitable for different diseases and disease conditions and may be combined to generate a diverse and polyclonal immune response. Thus, T cell expressing a SAR with higher affinity for the target may be more effective in killing a tumor cell in the short term but may exhaust quickly and/or have short term persistence *in vivo.* Such T cells expressing a high affinity SAR may be combined with T cells expressing a low affinity SAR that may not be as effective in killing a tumor cell in the short term but may not exhaust quickly and/or persist longer *in vivo.* The SARs of the disclosure, including the different pools of SARs, may be also combined with other genetically engineered T cells, such as SAR-NK cells, to generate a diverse immune response. Accordingy, the disclosure provides a library of X-SARs.

In an embodiment, one of the embodiments, the extracellular and transmembrane domain of one polypeptide chain comprises of the constant chain of TCRα or a fragment thereof while the extracellular and transmembrane domain of other polypeptide chain comprises of constant chain of TCRβ or a fragment thereof. In an embodiment, one of the embodiments, the extracellular and transmembrane domain of one polypeptide chain comprises of the constant chain of TCRγ or a fragment thereof while the extracellular and transmembrane domain of other polypeptide chain comprises of constant chain of TCRδ or a fragment thereof. In an embodiment, the extracellular and transmembrane domain of one polypeptide chain comprises of the constant chain of pretc.Rα while the extracellular and transmembrane domain of other polypeptide chain comprises of constant chain of TCRβ. In an embodiment, the linker domain of one of the polypeptides comprises of the linker listed with SEQ ID NOS: 11832 and the linker domain of the second polypeptide comprises of one of the linkers selected from SEQ ID NO: 11833-11847.

In some embodiments, the SAR of the disclosure comprises two polypeptide chains, one of the chains having a general formula:

AABD (n)-optional linker1-vL-optional linker2-extracellular domain-transmembrane domain- optional Intracellular signaling domain(n)

wherein n is 1 or more; and the second chain having the general formula:

AABD (n)-optional linker3-vH-optional linker4-extracellular domain-transmembrane domain- optional Intracellular signaling domain(n)

wherein n is 0 or more; In one embodiment, n is at least 1, for example 1, 2, 3, 4 or 5. In an embodiment, the vL of one polypeptide combines with vH of the other polypeptide to form a fragment variable (Fv) that binds to an antigen. In an embodiment, the vL of one polypeptide combines with vH of the other polypeptide to form a fragment variable (Fv) that does not bind to an antigen and serves only as the backbone or a scaffold for attachment of AABD. The AABD also forms the antigen binding domain and is located at the extracellular side when expressed in a cell. In an embodiment, the extracellular and transmembrane domain of one polypeptide chain comprises of the constant chain of TCRα or a fragment thereof while the extracellular and transmembrane domain of other polypeptide chain comprises of constant chain of TCRβ or a fragment thereof. In an embodiment, the extracellular and transmembrane domain of one polypeptide chain comprises of the constant chain of TCRγ or a fragment thereof while the extracellular and transmembrane domain of other polypeptide chain comprises of constant chain of TCRδ or a fragment thereof. In an embodiment, the extracellular and transmembrane domain of one polypeptide chain comprises of the constant chain of pretc.Rα while the extracellular and transmembrane domain of other polypeptide chain comprises of constant chain of TCRβ. In an embodiment, the linker2 domain of one of the polypeptides comprises of the linker listed with SEQ ID NOS: 11832 and the linker4 domain of the second polypeptide comprises of one of the linkers selected from SEQ ID NO: 11833-11847. In an embodiment the linker2 and linker4 are optional. In an embodiment, the linker1 and linker3 comprise of linkers with SEQ ID NO: 11715-11717. In an embodiment, the linker1 and linker3 are optional. Exemplary SARs are schematically presented in **Figures 1-2** **and Tables A1-A7.**

In some embodiments, the SAR of the disclosure comprises two polypeptide chains, one of the chains having a general formula:

AABD (n)-optional linker1-extracellular domain-transmembrane domain- optional Intracellular signaling domain(n)

wherein n is 1 or more; and the second chain having the general formula

AABD (n)-optional linker3-scFv-optional linker4-extracellular domain-transmembrane domain- optional Intracellular signaling domain(n)

wherein n is 0 or more; In one embodiment, n is at least 1, for example 1, 2, 3, 4 or 5. In an embodiment, the scFv binds to an antigen. The AABD also forms the antigen binding domain and is located at the extracellular side when expressed in a cell. In an embodiment, the extracellular and transmembrane domain of one polypeptide chain comprises of the constant chain of TCRα or a fragment thereof while the extracellular and transmembrane domain of other polypeptide chain comprises of constant chain of TCRβ or a fragment thereof. In an embodiment, the extracellular and transmembrane domain of one polypeptide chain comprises of the constant chain of TCRγ or a fragment thereof while the extracellular and transmembrane domain of other polypeptide chain comprises of constant chain of TCRδ or a fragment thereof. In an embodiment, the extracellular and transmembrane domain of one polypeptide chain comprises of the constant chain of pr*etc*.Rα while the extracellular and transmembrane domain of other polypeptide chain comprises of constant chain of TCRβ. In an embodiment, the linker2 domain of one of the polypeptide comprises of the linker listed with SEQ ID NOS: 11832 and the linker4 domain of the second polypeptide comprises of one of the linkers selected from SEQ ID NO: 11833-11847. In an embodiment the linker2 and linker4 are optional. In an embodiment, the linker1 and linker3 comprise of linkers with SEQ ID NO: 11715-11717. In an embodiment, the linker1 and linker3 are optional.

Exemplary SARs are schematically presented in **Tables A1-A7 and** **Figures 1-2****.**

In some embodiments, the SAR of the disclosure comprises two polypeptide chains, one of the chains having a general formula: AABD (n)-optional linker1-Va-optional linker2-extracellular domain-transmembrane domain- optional Intracellular signaling domain(n) wherein n is 1 or more; and the second chain having the general formula AABD (n)-optional linker3-Vb-optional linker4-extracellular domain-transmembrane domain- optional Intracellular signaling domain(n) wherein n is 0 or more; In one embodiment, n is at least 1, for example 1, 2, 3, 4 or 5. In an embodiment, the Va of one polypeptide combines with Vb of the other polypeptide to form a TCR fragment variable (TCR-Fv) that binds to an antigen. The AABD also forms the antigen binding domain and is located at the extracellular side when expressed in a cell. In an embodiment, the extracellular and transmembrane domain of one polypeptide chain comprises of the constant chain of TCRα or a fragment thereof while the extracellular and transmembrane domain of other polypeptide chain comprises of constant chain of TCRβ or a fragment thereof.

In some embodiments, the SAR of the disclosure comprises two polypeptide chains, one of the chains having a general formula: AABD (n)-optional linker1-Vg-optional linker2-extracellular domain-transmembrane domain- optional Intracellular signaling domain(n) wherein n is 1 or more; and the second chain having the general formula AABD (n)-optional linker3-Vd-optional linker4-extracellular domain-transmembrane domain- optional Intracellular signaling domain(n) wherein n is 0 or more; In one embodiment, n is at least 1, for example 1, 2, 3, 4 or 5. In an embodiment, the Vg of one polypeptide combines with Vd of the other polypeptide to form a TCR fragment variable (TCR-Fv) that binds to an antigen. The AABD also forms the antigen binding domain and is located at the extracellular side when expressed in a cell. In an embodiment, the extracellular and transmembrane domain of one polypeptide chain comprises of the constant chain of TCRγ or a fragment thereof while the extracellular and transmembrane domain of other polypeptide chain comprises of constant chain of TCRδ or a fragment thereof.

In some embodiments, the SAR polynucleotide and polypeptides of the disclosure have the backbone of a double chain SIR, a one and half chain SIR a double chain cTCR, a one and a half chain cTCR, a double chain ab-TCR, a one and a half chain ab-TCR, an AABD-TCR, a single chain TFP, a double chain TFP, a 1st generation CAR, a 2^{nd} generation CAR, a 3rd generation CAR, a TAC, a TCR and the like.

Bispecific and multispecific single chain chimeric antigen receptor (CAR) with the backbone of 2^{nd} generation CAR or 3^{rd} generation CARs have been described in the art. These CAR constructs generally comprise of two or more scFvs that are attached in tandem to a hinge, transmembrane, costimulatory and activation domain. A major problem with such bispecific and multispecific constructs is non-specific aggregatiton of the scFv domains, which results in poor expression, diminished binding to one or more target antigens, reduced signaling, reduced effector function, tonic signaing, early exhaustion and diminished *in vivo* efficacy. Thus, it was recently reported that a bispecific 2^{nd} generation CAR construct targeting both CD19 and CD20 showed lower efficacy was compared to a unispecific CD19 CAR construct in a clinical trial. Bispecific and multispecific constructs on the backbone of SIR have been described in WO2018102795. These bispecific and multispecific double chain SIR constructs comprised of two scFv or two vHH fragments targeting that were attached to two different TCR constant chains. Alternate configuration, such as one scFv attached to the first TCR constant chain and a non-scFV antigen binding domain (*e.g.*, vHH or DARPIN or affibody) attached to the second TCR constant chain have been also described. However, similar to the single chain bispecific/multispecific CAR constructs, these bispecific and multispecific SIR constructs suffered from poor expression, diminished binding to one or more target antigens, reduced signaling and reduced effector function. The disclosure provides a solution to the long-standing problem of generating bispecific and multispecific constructs by providing novel compositions of binding domains, optimal linkers between the different domains and novel configurations. The disclosure provides optimized unispecific, bispecific and multispecific SARs that have superior expression, binding activity, signaling, persistence, effector functions (*e*.*g*., cell activation, proliferation, cytokine production and cytotoxicity *etc.)* and *in vitro* and *in vitro* properties as compared to the constructs described in the art.

In an embodiment described herein, the bispecific or multispecific SAR of the disclosure shows at least 30% *(e.g.,* 40%, 50%, 60%, 70%, 80%, 90%, or 95%, 99% *etc.)* affinity for each of the target antigens as compared to the affinity of the each of the corresponding unispecific SARs when expressed in an effector cell and compared under similar conditions. The binding affinity can be measured using assays known in the art such as the Topanga Assay.

In other embodiments described herein, the bispecific SAR of the disclosure shows at least 30% *(e.g.,* 40%, 50%, 60%, 70%, 80%, 90%, or 95%, 99% *etc.)* signaling activity against each of the target antigen expressing cell as compared to the signaling activity of each of the corresponding unispecific SARs when expressed in an effector cell and compared under similar conditions. The signaling activity can be measured using methods known in the art, such as the Jurkat-NFAT-GFP cell assay.

In other embodiments described herein, the bispecific SAR of the disclosure shows at least 30% *(e.g.,* 40%, 50%, 60%, 70%, 80%, 90%, or 95%, 99% *etc.)* cytokine *(e.g.,* TNFα, IFNγ, IL-2 *etc.)* production against each of the target antigen expressing cell as compared to the cytokine production of each of the corresponding unispecific SARs when expressed in an effector cell and compared under similar conditions. The cytokine production *(e.g.,* TNFα, IFNγ, IL-2 *etc.)* can be measured using methods known in the art, such as ELISA.

In other embodiments described herein, the bispecific SAR of the disclosure shows at least 30% *(e.g.,* 40%, 50%, 60%, 70%, 80%, 90%, or 95%, 99% *etc.)* of the cytotoxic activity against each of the target antigen expressing cell as compared to the cytotoxic activity of each of the corresponding unispecific SARs when expressed in an effector cell and compared under similar conditions. The cytotoxic activity can be measured using methods known in the art, such as the Matador or radioactive chromium release assay.

In other embodiments described herein, the bispecific SAR of the disclosure shows at least 30% *(e.g.,* 40%, 50%, 60%, 70%, 80%, 90%, or 95%, 99% *etc.)* of the *in vivo* activity against each of the target antigen expressing cell as compared to the *in vivo* activity of each of the corresponding unispecific SARs when expressed in an effector cell and compared under similar conditions. In an embodiment, the *in vivo* activity is measured using xenograft model in immunodeficient mice.

In an embodiment described herein, the multi-specific SAR of the disclosure shows at least 30% *(e.g.,* 40%, 50%, 60%, 70%, 80%, 90%, or 95%, 99% *etc.)* affinity for one or more of the target antigens as compared to the affinity of the each of the corresponding unispecific SARs when expressed in an effector cell and compared under similar conditions. In an embodiment described herein, the multi-specific SAR of the disclosure shows at least 10% greater affinity *(e.g.,* 20%, 30%, 40%, 50%, 60%, 70%, 80%, 90%, 100%, 150% or 200% greater affinity *etc.)* for one or more of the target antigens as compared to the affinity of a corresponding construct comprising two or more scFv based antigen binding domains when expressed in an effector cell and compared under similar conditions. In an embodiment, the multispecific SAR of the disclosure shows superior binding affinity against more than one antigen as compared to a unispecific SAR. The binding affinity can be measured using assays known in the art such as the Topanga Assay. For example, T cells expressing the SAR CD8SP-CD22-FHVH-24-G4Sx3-R1-hu-mROO5-1-vL-[hTCRa-CSDVP]-F-F2A-IgSP-Apa-CD20-vHH-2HCD25-G4Sx3v2-hu-mROO5-1-vH-[hTCRb-KACIAH]-F-P2A-PAC (SEQ ID NO: 4163) or CD8SP-hu-mROO5-1-vL-[hTCRa-CSDVP]-F-F2A-IgSP-Apa-CD16-V158-v2-G4Sx3v2-hu-mROO5-1-vH-[hTCRb-KACIAH]-F-P2A-PAC (SEQ ID NO: 4723) show at least 30% binding affinity for CD19 as T cells expressing the unispecific SAR CD8SP-hu-mROO5-1-vL-[hTCRa-CSDVP]-F-F2A-SP-hu-mROO5-1-vH-[hTCRb-KACIAH]-F-P2A-PAC (SEQ ID NO: 7346) as measured by Topanga Assay using CD19-ECD-GGS-NLuc reagent (SEQ ID NO: 11971). Furthermore, T cells expressing the multispecific CD19xCD22xCD20 SAR with SEQ ID NO: 4163 show greater binding to CD22 and CD20 as compared with T cells expressing the unispecific CD19 SAR with SEQ ID NO: 7346. In another exemplary embodiment, T cells expressing the multispecific CD19xCD22xCD20 SAR with SEQ ID NO: 4163 show greater binding to CD22 and CD20 as compared with T cells expressing the CD22xCD20 bispecific construct CD8SP-CD22-FHVH-24-[hTCRa-CSDVP]-F-F2A-IgSP-Apa-CD20-vHH-2HCD25-[hTCRb-KACIAH]-F-P2A-PAC (SEQ ID NO: 21626) in which the CD22-FHVH-24 binding domain is directly attached to the TCRα chain (hTCRa-CSDVP) without an intervening vL region or Ig linker and CD20-vHH-2HCD25 binding domain is directly attached to TCRb chain (hTCRb-KACIAH) without an intervening vH or Ig linker.

In an embodiment described herein, the bispecific and/or multi-specific SAR of the disclosure shows at least 10% greater affinity *(e.g.,* 20%, 30%, 40%, 50%, 60%, 70%, 80%, 90%, 100%, 150% or 200% greater affinity *etc.)* for one or more of the target antigens as compared to the affinity of a corresponding bispecific/multispecific constructs comprising two or more scFv based antigen binding domains when expressed in an effector cell and compared under similar conditions. For example, T cells expressing the bispecific CD19xCD20 SAR CD8SP-hu-mROO5-1-vL-[hTCRa-CSDVP]-F-F2A-IgSP-Apa-CD20-vHH-2HCD25-G4Sx3v2-hu-mROO5-1-vH-[hTCRb-KACIAH]-F-P2A-PAC with SEQ ID NO: 4088 show at least 10% greater binding affinity to CD19 and/or CD20 as compared with T cells expressing the construct CD8SP-CD19-hu-mROO5-1-scFv-[hTCRa-CSDVP]-F-F2A-IgSP-CD20-7D8-scFv-[hTCRb-KACIAH]-F-P2A-PAC (SEQ ID NO: 21627).

In other embodiments described herein, the multi-specific SAR of the disclosure shows at least 30% *(e.g.,* 40%, 50%, 60%, 70%, 80%, 90%, or 95%, 99% *etc.)* of the signaling activity against one or more of the target antigen expressing cell as compared to the signaling activity of each of the corresponding unispecific SARs when expressed in an effector cell and compared under similar conditions. In an embodiment, the multispecific SAR of the disclosure shows superior signaling activity against more than one antigen-expressing cells as compared to a unispecific SAR. The signaling activity can be measured using methods known in the art, such as the Jurkat-NFAT-GFP (JNG) cell assay. In an exemplary embodiment, JNG cells expressing the SAR with SEQ ID NO: 4163 or SEQ ID NO: 4723 show at least 30% GFP induction as observed with JNG cells expressing the SAR with SEQ ID NO: 7346 when cultured under similar conditions with RAJI cells expressing the CD19 antigen. In another embodiment, JNG cells expressing the multispecific CD19xCD22xCD20 SAR with SEQ ID NO: 4163 show greater GFP induction when co-cultured with CD19-null *(e.g.,* CD19-KO) but CD22⁺ and CD20⁺ RAJI cells as compared with JNG cells expressing the CD19 unispecific SAR with SEQ ID NO: 7346. In another exemplary embodiment, JNG cells expressing the multispecific CD19xCD22xCD20 SAR with SEQ ID NO: 4163 show greater binding to CD22 and CD20 as compared with JNG cells expressing the CD22xCD20 bispecific construct.

In an embodiment described herein, the bispecific and/or multi-specific SAR of the disclosure shows at least 10% greater signalign activity *(e.g.,* 20%, 30%, 40%, 50%, 60%, 70%, 80%, 90%, 100%, 150% or 200% greater affinity *etc.)* against one or more of the target antigens expressing cells as compared to the signaling activity of the corresponding bispecific/multispecific constructs comprising two or more scFv based antigen binding domains when expressed in an effector cell and compared under similar conditions. For example, JNG cells expressing the bispecific CD19xCD20 SAR with SEQ ID NO: 4088 show at least 10% greater GFP induction when co-cultured for 24 hours with RAJI (CD19⁺/CD20⁺) and/or RAJI-CD19-KO (CD19⁻/CD20⁺) cells as compared with JNG cells expressing the construct CD8SP-CD19-hu-mROO5-1-scFv-[hTCRa-CSDVP]-F-F2A-IgSP-CD20-7D8-scFv-[hTCRb-KACIAH]-F-P2A-PAC (SEQ ID NO: 21627).

In other embodiments described herein, the multi-specific SAR of the disclosure shows at least 30% *(e.g.,* 40%, 50%, 60%, 70%, 80%, 90%, or 95%, 99% *etc.)* of the cytotoxic activity against each of the target antigen expressing cell as compared to the cytotoxic activity of each of the corresponding unispecific SARs when expressed in an effector cell and compared under similar conditions. The cytotoxic activity can be measured using methods known in the art, such as the Matador or radioactive chromium release assay. For example, T cells expressing the SAR with SEQ ID NO: 4163 or SEQ ID NO: 4723 show at least 30% cytotoxicity towards RAJI cells as observed with T cells expressing the SAR with SEQ ID NO: 7346 when assayed under similar conditions. In another embodiment, T cells expressing the multispecific CD19xCD22xCD20 SAR with SEQ ID NO: 4163 show greater cytotoxicity when co-cultured with CD19-null *(e.g.,* CD19-KO) but CD22⁺ and CD20⁺ RAJI cells as compared with T cells expressing the CD19 unispecific SAR with SEQ ID NO: 7346. In another exemplary embodiment, T cells expressing the multispecific CD19xCD22xCD20 SAR with SEQ ID NO: 4163 show greater cytotoxicity to CD19⁻/CD22⁺/CD20⁺ as compared with T cells expressing the CD22xCD20 bispecific construct with SEQ ID NO: 21626.

In an embodiment described herein, the bispecific and/or multi-specific SAR of the disclosure shows at least 10% greater cytotoxicity (*e*.*g*., 20%, 30%, 40%, 50%, 60%, 70%, 80%, 90%, 100%, 150% or 200% greater affinity *etc.)* against one or more of the target antigens expressing cells as compared to the cytotoxicity of the corresponding bispecific/multispecific constructs comprising two or more scFv based antigen binding domains when expressed in an effector cell and compared under similar conditions. For example, T cells expressing the bispecific CD19xCD20 SAR with SEQ ID NO: 4088 show at least 10% greater cytotoxicity when co-cultured for 24 hours with RAJI (CD19⁺/CD20⁺) and/or RAJI-CD19-KO (CD19⁻/CD20⁺) cells as compared with T cells expressing the construct CD8SP-CD19-hu-mROO5-1-scFv-[hTCRa-CSDVP]-F-F2A-IgSP-CD20-7D8-scFv-[hTCRb-KACIAH]-F-P2A-PAC.

In an embodiment described herein, the bispecific and/or multi-specific SAR of the disclosure comprising an adaptor binding domain attached to the N-terminus or near the N-terminus of a vL/vH fragment shows at least 10% greater cytotoxicity (*e*.*g*., 20%, 30%, 40%, 50%, 60%, 70%, 80%, 90%, 100%, 150% or 200% greater affinity *etc.)* against one or more of the target antigens expressing cells as compared to the cytotoxicity of the corresponding bispecific/multispecific constructs comprising one scFv and one adaptor binding domain that are individually attached to separate TCR constant chains when expressed in an effector cell and compared under similar conditions. For example, T cells expressing the SAR CD8SP-hu-mROO5-1-vL-[hTCRa-CSDVP]-F-F2A-IgSP-Apa-RZip-G4Sx3v2-hu-mROO5-1-vH-[hTCRb-KACIAH]-F-P2A-PAC with SEQ ID NO: 4342 show at least 10% greater cytotoxicity when co-cultured for 24 hours with SKOV3 cells (Her2+/CD19-) in the presence of EZip-tagged Herceptin or Her2-huMab4D5-D98W-scFv-G4S-EZIP (SEQ ID NO: 12182) as compared with T cells expressing the construct CD8SP-CD19-hu-mROO5-1-scFv-[hTCRa-CSDVP]-F-F2A-IgSP-RZIP-[hTCRb-KACIAH]-F-P2A-PAC (SEQ ID NO: 21628).

In other embodiments described herein, the multispecific SAR of the disclosure shows at least 30% (*e.g.,* more than 40%, 50%, 60%, 70%, 80%, 90%, or 95%, 99%, 100% *etc.)* cytokine (*e.g.,* TNFα, IFNγ, IL-2 *etc.*) production against each of the target antigen expressing cell as compared to the cytokine production of each of the corresponding unispecific SARs when expressed in an effector cell and compared under similar conditions. The cytokine production *(e.g.,* TNFα, IFNγ, IL-2 *etc.)* can be measured using methods known in the art, such as ELISA. For example, T cells expressing the SAR with SEQ ID NO: 4163 or SEQ ID NO: 4723 show at least 30% TNFα, IFNγ and/or IL2 production as observed with T cells expressing the SAR with SEQ ID NO: 7346 when co-cultured with RAJI cells under similar conditions. In another exemplary embodiment, T cells expressing the multispecific CD19xCD22xCD20 SAR with SEQ ID NO: 4163 show greater TNFα, IFNγ and IL2 production when co-cultured with CD19-null *(e.g.,* CD19-KO) but CD22⁺ and CD20⁺ RAJI cells as compared with T cells expressing the CD19 unispecific SAR with SEQ ID NO: 7346. In another exemplary embodiment, T cells expressing the multispecific CD19xCD22xCD20 SAR with SEQ ID NO: 4163 show greater TNFα, IFNγ and IL2 produciton when co-cultured with CD19⁻/CD22⁺/CD20⁺ as compared with T cells expressing the CD22xCD20 bispecific construct with SEQ ID NO: 21626.

In an embodiment described herein, the bispecific and/or multi-specific SAR of the disclosure shows at least 10% greater cytokine production *(e.g.,* 20%, 30%, 40%, 50%, 60%, 70%, 80%, 90%, 100%, 150% or 200% greater affinity *etc.)* against one or more of the target antigens expressing cells as compared to the cytokine production of the corresponding bispecific/multispecific constructs comprising two or more scFv based antigen binding domains when expressed in an effector cell and compared under similar conditions.

In other embodiments described herein, the multi-specific SAR of the disclosure shows at least 30% *(e.g.,* 40%, 50%, 60%, 70%, 80%, 90%, or 95%, 99% *etc.)* of the *in vivo* activity against each of the target antigen expressing cell as compared to the *in vivo* activity of each of the corresponding unispecific SARs when expressed in an effector cell and compared under similar conditions. In an embodiment, the *in vivo* activity is measured using xenograft model in immunodeficient mice. For example, T cells expressing the SAR with SEQ ID NO: 4163 or SEQ ID NO: 4723 show at least 30% tumor reduction as observed with T cells expressing the SAR with SEQ ID NO: 7346 when assayed using RAJI xenograft model in NSG immunodeficient mice. In another exemplary embodiment, T cells expressing the multispecific CD19xCD22xCD20 SAR with SEQ ID NO: 4163 show greater *in vivo* activity against with CD19-null *(e.g.,* CD19-KO) but CD22⁺ and CD20⁺ RAJI cells as compared with T cells expressing the CD19 unispecific SAR with SEQ ID NO: 7346. In another exemplary embodiment, T cells expressing the multispecific CD19xCD22xCD20 SAR with SEQ ID NO: 4163 show greater *in vivo* activity against CD19⁻/CD22⁺/CD20⁺ RAJI cells as compared with T cells expressing the CD22xCD20 bispecific construct with SEQ ID NO: 21626.

In an embodiment described herein, the bispecific and/or multi-specific SAR of the disclosure shows at least 10% greater *in vivo* anti-tumor activity *(e.g.,* 20%, 30%, 40%, 50%, 60%, 70%, 80%, 90%, 100%, 150% or 200% greater affinity *etc.)* against one or more of the target antigens expressing cells as compared to the *in vivo* anti-tumor activity of the corresponding bispecific/multispecific constructs comprising two or more scFv based antigen binding domains when expressed in an effector cell and compared under similar conditions.

In some embodiments, when expressed on the surface of a cell, binding of the antigen binding domain comprised by the Fv *(i.e.,* vL/vH fragments) of a bispecific SAR to its cognate antigen is not substantially reduced by one or more AABDs that are operably linked to the N-terminus region of the vL and/or vH fragment of the said SAR. In some embodiments, when expressed on the surface of a cell, binding of the antigen binding domain comprised by the Fv *(i.e.,* vL/vH fragments) of a bispecific/multispecific SAR comprising one or more AABDs that are operably linked to the N-terminus region of the vL and/or vH fragment of the said SAR to its cognate antigen is at least 30% *(e.g.,* 40%, 50%, 60%, 70%, 80%, 90%, or 95%, 99% *etc.)* of the binding affinity of a corresponding unispecific SAR lacking the AABD. In an embodiment, the SAR is a single chain SAR *(e.g.,* a 2^{nd} generation CAR or a single chain TFP or TAC). In an embodiment, the SAR is a double chain SAR *(e.g.,* a double chain SIR, a double chain cTCR, a double chain Ab-TCR, a double chain TFP, a double chain zSIR *etc.).* In an exemplary embodiment, the AABD is a SVH, SVL, FHVH, vHH, non-immunoglobulin antigen binding domain, adaptor binding domain, epitope tag, mimotope, auto-antigen, ligand binding domain of a receptor or receptor binding domain of a ligand, an Fc binding receptor *etc.*

In some embodiments, when present on the surface of a cell, the antigen binding affinity of the antigen binding domain comprised by the Fv *(i.e.* vL/vH fragments) of a bispecific SAR comprising one or more AABDs that are operably linked to the N-terminus region of the vL and/or vH fragment of the said SAR is at least 70%, 80%, 85%, 90%, 95%, 96%, 97%, 98% or 99% of antigen binding affinity of the antigen binding domain of a corresponding unispecific SAR in which one or more AABDs are not operably linked to the N-terminus region of the vL and/or vH fragment.

In some embodiments, binding of the antigen binding domain of said first chain of SAR to its cognate antigen in the presence of said second chain of SAR (or a CAR) is 70%, 80%, 85%, 90%, 95%, 96%, 97%, 98% or 99% of binding of the antigen binding domain of said first chain of SAR to its cognate antigen in the absence of said second chain of SAR (or a CAR) to its cognate antigen. For example, if a cell expresses a double chain SAR in which the first chain comprises of an scFV targeting CD19 joined to TCRα and the second chain comprises of a camelid vHH fragment targeting CD123 joined to TCRβ2, then the binding of the antigen binding domain of said first chain of SAR to its cognate antigen *(i.e.* CD19) in the presence of said second chain of SAR is 70%, 80%, 85%, 90%, 95%, 96%, 97%, 98% or 99% of binding of the antigen binding domain of said first chain of SAR to its cognate antigen *(i.e.* CD19) in the absence of said second chain of SAR to its cognate antigen *(i.e.* CD123). In another example, if a cell expresses a double chain SAR in which the first chain comprises of a vL fragment of FMC63 antibody targeting CD19 operationally linked to TCRα and the second chain comprises of the vH fragment of FMC63 antibody targeting CD19 operationally linked to TCRβ2, along with a CAR comprising vHH fragment targeting CD123, then the binding of the antigen binding domain of said first and second chains of the double chain SAR to their cognate antigen *(i.e.* CD19) in the presence of said CAR is 70%, 80%, 85%, 90%, 95%, 96%, 97%, 98% or 99% of binding of the antigen binding domain of said first and second chains of the double-chain of SAR to their cognate antigen (*i*.*e*. CD19) in the absence of said CAR to its cognate antigen *(i.e.* CD123).

In some embodiments, when present on the surface of a cell, the antigen binding domains of said first chain said second chain of a double chain SAR, associate with one another less than if both were scFv antigen binding domains. In some embodiments, the antigen binding domains of said first chain said second chain of a double chain SAR, associate with one another 5%, 10%, 20%, 30%, 40%, 50%, 60%, 70%, 80%, 90% or 99% less than if both were scFv antigen binding domains.

In some embodiments, the antigen specific domains of the SARs comprise one or more vL fragments. In exemplary embodiments, the one or more vL fragments are described in Table 3. In some embodiments, the polynucleotides encoding the one more vL fragments comprise, consist of or consist essentially of sequences set forth in any one or more of SEQ ID NOs: 46-282. In some embodiments, the polypeptides encoding the one more vL fragments comprise, consist of or consist essentially of sequences set forth in any one or more of SEQ ID NOS: 10736-10972 or sequences with 70-99% identity to sequences set forth in any one or more of SEQ ID NOS: 10736-10972 or sequences with 70-99% identity in the three complementarity determining regions (CDRs) to the sequences set forth in any one or more of SEQ ID NOS: 10736-10972 or sequences with less than 3 substitutions in the three CDRs of the sequences set forth in any one or more of SEQ ID NOS: 10736-10972 or sequences that bind to the same target antigens or the same epitopes on the target antigens as the sequences set forth in any one or more of SEQ ID NOS: 10736-10972. In some embodiments, the antigen specific domains of the SARs comprise one or more vH fragments. In exemplary embodiments, the one or more vH fragments are described in **Table 3.** In some embodiments, the polynucleotides encoding the one more vH fragments comprise, consist of or consist essentially of sequences set forth in any one or more of SEQ ID NOS: 288-524. In some embodiments, the polypeptides encoding the one more vH fragments comprise, consist of or consist essentially of sequences set forth in any one or more of SEQ ID NOS: 10978-11214 or sequences with 70-99% identity to sequences set forth in any one or more of SEQ ID NOS: 10978-11214 or sequences with 70-99% identity in the three complementarity determining regions (CDRs) to the sequences set forth in any one or more of SEQ ID NOS: 10978-11214 or sequences with less than 3 substitutions in the three CDRs of the sequences set forth in any one or more of SEQ ID NOS: 10978-11214 or sequences that bind to the same target antigens or the same epitopes on the target antigens as the sequences set forth in any one or more of SEQ ID NOS: 10978-11214.

In exemplary embodiments, the one or more AABD *(e.g.,* VHH (or vHH) or SVH) fragments are described in **Table 4.** In some embodiments, the polynucleotides encoding the one more AABD (*e*.*g*., VHH (or vHH) or SVH) comprise, consist of or consist essentially of sequences set forth in any one or more of SEQ ID NOS: 830-902. In some embodiments, the polypeptides encoding the one more AABD (*e*.*g*., VHH (or vHH) or SVH) comprise, consist of or consist essentially of sequences set forth in any one or more of SEQ ID NOS: 11520-11592 or sequences with 70-99% identity to sequences set forth in any one or more of SEQ ID NOS: 11520-11592 or sequences with 70-99% identity in the three complementarity determining regions (CDRs) to the sequences set forth in any one or more of SEQ ID NOS: 11520-11592 or sequences with less than 3 substitutions in the three CDRs of the sequences set forth in any one or more of SEQ ID NOS: 11520-11592 or sequences with less than 3 substitutions in the three CDRs of the sequences set forth in any one or more of SEQ ID NOS: 11520-11592 or sequences that bind to the same target antigens or the same epitopes on the target antigens as the sequences set forth in any one or more of SEQ ID NOS: 11520-11592.

In exemplary embodiments, the one or more AABD are non-immunoglobulin antigen binding scaffold as described in **Table 7.** In some embodiments, the polynucleotides encoding the one more non-immunoglobulin antigen binding scaffolds comprise, consist of or consist essentially of sequences set forth in any one or more of SEQ ID NOS: 972-983. In some embodiments, the polypeptides encoding the one more non-immunoglobulin antigen binding scaffolds comprise, consist of or consist essentially of sequences set forth in any one or more of SEQ ID NOS: 11662-11673 or sequences with 70-99% identity to sequences set forth in any one or more of SEQ ID NOS: 11662-11673 or sequences that bind to the same target antigens or the same epitopes on the target antigens as the sequences set forth in any one or more of SEQ ID NOS: 11662-11673.

In exemplary embodiments, the one or more AABD are ligand binding domains of receptors. Exemplary such ligand binding domains of receptors are described in **Table 8**. In exemplary embodiments, the one or more AABD are ligand binding domains of receptors with SEQ ID NO: 11674 to 11691 or sequences with 70-99% identity to sequences set forth in any one or more of SEQ ID NOS: 11674 to 11691.

In one embodiment, the V_{H} domain is selected from SEQ ID NOs. 11517-11592 having one or more amino acid substitutions, deletions, insertions or other modifications compared to SEQ ID NOs. 11517-11592, and which retains a biological function of the single domain antibody.

In one embodiment, the modification is a conservative sequence modification. Thus, one or more amino acid residues within the CDR regions of a single domain antibody of the disclosure can be replaced with other amino acid residues from the same side chain family and the altered antibody can be tested for retained function *(i.e.,* the functions set forth in (c) through (I) above) using the functional assays described herein.

In another embodiment, the vH (or V_{H}) domain is selected from one of the SEQ ID NOs. 11517-11592, but comprises one or more amino acid substitutions, for example 1 to 20, such as 1, 2, 3, 4, 5, 6, 7, 8, 9 or 10 amino acid substitutions. In one embodiment, the one or more amino acid substitution is in one or more of the framework areas. In another embodiment, the one or more amino acid substitution is in one or more of the CDRs. In one embodiment, the amino acid substitutions are in the framework and CDR sequences.

In one embodiment, the V_{H} single domain antibody that is a variant of a single domain antibody selected from those having SEQ ID NOs. 11517-11592 that comprises one or more sequence modification and has improvements in one or more of a property such as binding affinity, specificity, thermostability, expression level, effector function, glycosylation, reduced immunogenicity, or solubility as compared to the unmodified single domain antibody.

A skilled person will know that there are different ways to identify, obtain and optimise the antigen binding molecules as described herein, including *in vitro* and *in vivo* expression libraries. This is further described in the examples. Optimisation techniques known in the art, such as display (*e*.*g*., ribosome and/or phage display) and / or mutagenesis (*e*.*g*., error-prone mutagenesis) can be used. The disclosure therefore also comprises sequence optimised variants of the single domain antibodies described herein.

The disclosure also relates to the use of multiple human V_{H} domains in a SAR construct. In one embodiment, more than one, for example two or three V_{H} domains are selected from the V_{H} domains with SEQ ID NOs shown in **Table 5** or amino acid sequences with at least 70%, 80% or 90% homology thereto (with corresponding nucleic acid SEQ ID Nos shown in **Table 5).**

In one embodiment, the binding domain of the SAR of the disclosure provides biparatopic targeting to any one or more of tha antigens listed in **Table B.** Thus, the binding domain comprises a first AABD (*e*.*g*., V_{H} single domain antibody) that binds to a first epitope of one or more antigens listed in **Table B** and an AABD (*e.g*., V_{H} single domain antibody), an Fv or an scF that binds to a second epitope of that antigen. The first and second epitope may be overlapping.

In one embodiment, the binding domain of the SAR of the disclosure provides biparatopic targeting to BCMA. Thus, the binding domain comprises a first AABD *(e.g.,* V_{H} single domain antibody) that binds to a first epitope of BCMA and an AABD *(e.g.,* V_{H} single domain antibody), an Fv, or an scFv that binds to a second epitope of BCMA. The first and second epitope may be overlapping.

In one embodiment, the binding domain of the SAR of the disclosure provides biparatopic targeting to CD19. Thus, the binding domain comprises a first AABD *(e.g.,* V_{H} single domain antibody) that binds to a first epitope of CD19 and an AABD *(e.g.,* V_{H} single domain antibody), an Fv or an scF that binds to a second epitope of CD19. The first and second epitope may be overlapping.

In one embodiment, the binding domain of the SAR of the disclosure provides biparatopic targeting to MSLN. Thus, the binding domain comprises a first AABD *(e.g.,* V_{H} single domain antibody) that binds to a first epitope of MSLN and an AABD *(e.g.,* V_{H} single domain antibody), an Fv or an scF that binds to a second epitope of MSLN. The first and second epitope may be overlapping.

In one embodiment, the binding domain of the SAR of the disclosure provides biparatopic targeting to CD20. Thus, the binding domain comprises a first AABD *(e.g.,* V_{H} single domain antibody) that binds to a first epitope of CD20 and an AABD *(e.g.,* V_{H} single domain antibody), an Fv or an scF that binds to a second epitope of CD20. The first and second epitope may be overlapping.

In one embodiment, the binding domain of the SAR of the disclosure provides biparatopic targeting to BCMA. Thus, the binding domain comprises a first AABD *(e.g.,* V_{H} single domain antibody) that binds to a first epitope of BCMA and an AABD *(e.g.,* V_{H} single domain antibody), an Fv or an scF that binds to a second epitope of BCMA. The first and second epitope may be overlapping.

In one embodiment, the binding domain of the SAR of the disclosure provides biparatopic targeting to CD22. Thus, the binding domain comprises a first AABD *(e.g.,* V_{H} single domain antibody) that binds to a first epitope of CD22 and an AABD *(e.g.,* V_{H} single domain antibody), an Fv or an scF that binds to a second epitope of CD22. The first and second epitope may be overlapping.

In one embodiment, the binding domain of the SAR of the disclosure provides biparatopic targeting to PSMA. Thus, the binding domain comprises a first AABD *(e.g.,* V_{H} single domain antibody) that binds to a first epitope of PSMA and an AABD *(e.g.,* V_{H} single domain antibody), an Fv or an scF that binds to a second epitope of PSMA. The first and second epitope may be overlapping.

In one embodiment, the binding domain of the SAR of the disclosure provides biparatopic targeting to CD123. Thus, the binding domain comprises a first AABD (*e.g*., V_{H} single domain antibody) that binds to a first epitope of CD123 and an AABD *(e.g.,* V_{H} single domain antibody), an Fv or an scF that binds to a second epitope of CD123. The first and second epitope may be overlapping.

In one embodiment, the binding domain of the SAR of the disclosure provides biparatopic targeting to Her2. Thus, the binding domain comprises a first AABD (*e.g*., V_{H} single domain antibody) that binds to a first epitope of Her2 and an AABD (*e.g*., V_{H} single domain antibody), an Fv or an scF that binds to a second epitope of Her2. The first and second epitope may be overlapping.

In one embodiment, the binding domain of the SAR of the disclosure provides biparatopic targeting to CD123. Thus, the binding domain comprises a first vH single domain antibody that binds to a first epitope of CD123 and a second vH single domain antibody that binds to a second epitope of CD123. The first and second epitope may be overlapping.

In one embodiment, the binding domain of the SAR of the disclosure provides biparatopic targeting to Muc16. Thus, the binding domain comprises a first vH single domain antibody that binds to a first epitope of Muc16 and a second vH single domain antibody that binds to a second epitope of Muc16. The first and second epitope may be overlapping.

In one embodiment, the binding domain of the SAR of the disclosure provides bispecific targeting. Thus, the binding domain comprises a vH single domain antibody or vHH domain that binds BCMA and a second binding moiety that targets a second target. The vH single domain antibody that binds BCMA is for example selected from SEQ ID NOs. 11543-11548, 11579-11580, 11585-11586 or from a sequence with at least 60%, 70%, 80% or 90% homology thereto. The second binding moiety may be an antibody fragment, typically a V_{H} single domain antibody, a centyrin, an affibody or a vHH domain. The second target may be selected from CD19, CD20, CD22, BCMA, PSCA, CS1, GPC3, CSPG4, EGFR, 5T4, L1 CAM, MUC16, ROR1, cKit, ROR, 1mesothelin, IL3Ra, c-Met, EGFRvlll, GD-2, NY-ESO-1 TCR or MAGE A3 TCR, HER2, Wilm's tumor gene 1 (WT1), carcinoembryonic antigen (CEA), mucin 16, MUC1, an immuno checkpoint target or combinations thereof. However, a skilled person would understand that other tumor antigens are also potential combination targets within the scope of the invention. In the case of a single chain SAR, the two binding domains may be present in either order. For example, in the case of a bispecific single chain SAR targeting BCMA and CD38, the order of the vH domains may be BCMA-vH-linker-CD38-vH or CD38-vH-linker-BCMA-vH. Similarly, in the case of a double chain SAR, the two binding domains may be operably linked to either of the two constant chains. For example, in the case of a double chain SAR targeting BCMA and CD38, the configuration could be BCMA-vH-TCRα-2A-CD38-vH-TCRβ or CD38-vH-TCRα-2A-BCMA-TCRβ, or BCMA-vH-TCRβ-2A-CD38-vH-TCRα or CD38-vH-TCRβ-2A-BCMA-TCRα where 2A represents a cleavable linker such as P2A, T2A, F2A and the like. In another embodiment, in the case of a double chain SAR targeting BCMA and CD38, the configuration could be BCMA-vH-TCRγ-2A-CD38-vH-TCRδ or CD38-vH-TCRγ-2A-BCMA-TCRδ or BCMA-vH-TCRδ-2A-CD38-vH-TCRγ or CD38-vH-TCRδ-2A-BCMA-TCRγ, where 2A represents a cleavable linker such as P2A, T2A, F2A and the like.

In one embodiment, the fist binding domain of SAR comprises an AABD (*e.g.,* V_{H} single domain antibody or SVH, vHH or Centyrin *etc.)* that binds BCMA and a second binding moiety that targets CD38. The AABD *(e.g.,* SVH or vHH or Centryrin. *etc.*) that binds BCMA is for example selected from SEQ ID NOs. 11543-11548, 11579-11580, 11585-11586, 11673, 23167 or from a sequence with at least 60%, 70%, 80%, 90%, 98% homology thereto. The AABD (*e.g*., SVH, VHH or Centyrin) that binds CD38 is for example selected from SEQ ID NOs. 11535-11539, 11582, 11586, 23168 and 23169 or from a sequence with at least 60%, 70%, 80%, 90% or 98% homology thereto.

In an embodiment, the SAR further comprises vL/vH fragments that combine to form a Fv targeting a specific antigen. Exemplary vL/vH fragments and their target antigens are provided in **Table 3.** Additional exemplary vL/vH fragments, their LC-CDR1-3, HC-CDR1-3 and their target antigens are provided in **Table 39.** In an embodiment, the SAR further comprises Va/Vb or Vg/Vd fragments that combine to form a TCR-Fv targeting different antigens. Exemplary Va/Vb or Vg/Vd fragments and their target antigens are provided in **Table 4.**

In one embodiment, one binding domain of SAR comprises an AABD *(e.g.,* V_{H} single domain antibody or SVH, vHH or Centyrin *etc.)* that binds BCMA and a second binding moiety that targets CD19. The AABD (*e*.*g*., V_{H} single domain antibody or SVH, vHH or Centyrin *etc.)* that binds BCMA is for example selected from SEQ ID NOs. 11543-11548, 11579-11580, 11585-11586, 11673 or from a sequence with at least 60%, 70%, 80% or 90% homology thereto. The AABD that binds CD19 is for example selected from SEQ ID NOs. 11524, 11526-11529, 11588 or from a sequence with at least 60%, 70%, 80% or 90% homology thereto. In an embodiment, the SAR further comprises vL/vH fragments that combine to form a Fv targeting a specific antigen. Exemplary vL/vH fragments and their target antigens are provided in **Table 3.** In an embodiment, the SAR further comprises Va/Vb or Vg/Vd fragments that combine to form a TCR-Fv targeting different antigens. Exemplary Va/Vb or Vg/Vd fragments and their target antigens are provided in **Table 4.**

In one embodiment, the first binding domain of SAR comprises an AABD (*e.g.,* V_{H} single domain antibody or SVH, vHH or Centyrin *etc.)* that binds BCMA and a second binding moiety that targets CD22. The AABD that binds BCMA is for example selected from SEQ ID NOs. 11543-11548, 11579-11580, 11585-11586, 11673 or from a sequence with at least 60%, 70%, 80% or 90% homology thereto. The AABD that binds CD22 is for example selected from SEQ ID NOs. 11532-11534 or from a sequence with at least 60%, 70%, 80% or 90% homology thereto. In an embodiment, the SAR further comprises vL/vH fragments that combine to form a Fv targeting a specific antigen. Exemplary vL/vH fragments and their target antigens are provided in **Table 3.** In an embodiment, the SAR further comprises Va/Vb or Vg/Vd fragments that combine to form a TCR-Fv targeting different antigens. Exemplary Va/Vb or Vg/Vd fragments and their target antigens are provided in **Table 4.**

In one embodiment, the fist binding domain of SAR comprises an AABD (*e.g.,* V_{H} single domain antibody or SVH, vHH or Centyrin *etc.)* that binds BCMA and a second binding moiety that targets CD20. The V_{H} single domain antibody that binds BCMA is for example selected from SEQ ID NOs. 11543-11548, 11579-11580, 11585-11586, 11673 or from a sequence with at least 60%, 70%, 80% or 90% homology thereto. The AABD that binds CD2 is for example selected from SEQ ID NOs. 11530-31, 11589 or from a sequence with at least 60%, 70%, 80% or 90% homology thereto. In an embodiment, the SAR further comprises vL/vH fragments that combine to form a Fv targeting a specific antigen. Exemplary vL/vH fragments and their target antigens are provided in **Table 3.** In an embodiment, the SAR further comprises Va/Vb or Vg/Vd fragments that combine to form a TCR-Fv targeting different antigens. Exemplary Va/Vb or Vg/Vd fragments and their target antigens are provided in **Table 4.**

In one embodiment, the fist binding domain of SAR comprises an AABD that binds CD22 and a second binding moiety that targets CD20. The AABD that binds CD22 is for example selected from SEQ ID NOs. 11532-11534 or from a sequence with at least 60%, 70%, 80% or 90% homology thereto. The AABD that binds CD20 is for example selected from SEQ ID NOs. 11530-31, 11589 or from a sequence with at least 60%, 70%, 80% or 90% homology thereto. In an embodiment, the SAR further comprises vL/vH fragments that combine to form a Fv targeting a specific antigen. Exemplary vL/vH fragments and their target antigens are provided in **Table 3.** In an embodiment, the SAR further comprises Va/Vb or Vg/Vd fragments that combine to form a TCR-Fv targeting different antigens. Exemplary Va/Vb or Vg/Vd fragments and their target antigens are provided in **Table 4.**

In one embodiment, the fist binding domain of SAR comprises an AABD that binds CD22 and a second binding moiety that targets CD19. The AABD that binds CD22 is for example selected from SEQ ID NOs. 11532-11534 or from a sequence with at least 60%, 70%, 80% or 90% homology thereto. The AABD that binds CD19 is for example selected from SEQ ID NOs. 11524, 11526-11529, 11588 or from a sequence with at least 60%, 70%, 80% or 90% homology thereto. In an embodiment, the SAR further comprises vL/vH fragments that combine to form a Fv targeting a specific antigen. Exemplary vL/vH fragments and their target antigens are provided in **Table 3.** In an embodiment, the SAR further comprises Va/Vb or Vg/Vd fragments that combine to form a TCR-Fv targeting different antigens. Exemplary Va/Vb or Vg/Vd fragments and their target antigens are provided in **Table 4.**

In one embodiment, the fist binding domain of SAR comprises an AABD that binds CD19 and a second binding moiety that targets CD20. The AABD that binds CD19 is for example selected from SEQ ID NOs. 11524, 11526-11529, 11588 or from a sequence with at least 60%, 70%, 80% or 90% homology thereto. The V_{H} single domain antibody that binds CD20 is for example selected from SEQ ID NOs. 11530-31, 11589 or from a sequence with at least 60%, 70%, 80% or 90% homology thereto. In an embodiment, the SAR further comprises vL/vH fragments that combine to form a Fv targeting a specific antigen. Exemplary vL/vH fragments and their target antigens are provided in **Table 3.** In an embodiment, the SAR further comprises Va/Vb or Vg/Vd fragments that combine to form a TCR-Fv targeting different antigens. Exemplary Va/Vb or Vg/Vd fragments and their target antigens are provided in **Table 4.**

In one embodiment, the fist binding domain of SAR comprises an AABD that binds CD19 and a second binding moiety that targets CD38. The V_{H} single domain antibody that binds CD19 is for example selected from SEQ ID NOs. 11524, 11526-11529, 11588 or from a sequence with at least 60%, 70%, 80% or 90% homology thereto. The AABD that binds CD38 is for example selected from SEQ ID NOs. 11535-11539, 11582 and 11586 or from a sequence with at least 60%, 70%, 80% or 90% homology thereto. In an embodiment, the SAR further comprises vL/vH fragments that combine to form a Fv targeting a specific antigen. Exemplary vL/vH fragments and their target antigens are provided in **Table 3.** In an embodiment, the SAR further comprises Va/Vb or Vg/Vd fragments that combine to form a TCR-Fv targeting different antigens. Exemplary Va/Vb or Vg/Vd fragments and their target antigens are provided in **Table 4.**

In one embodiment, the fist binding domain of SAR comprises AABD that binds CD19 and a second binding moiety that targets CD123. The AABD that binds CD19 is for example selected from SEQ ID NOs. 11524, 11526-11529, 11588 or from a sequence with at least 60%, 70%, 80% or 90% homology thereto. The AABD that binds CD123 is for example selected from SEQ ID NOs. 11569-70 or from a sequence with at least 60%, 70%, 80% or 90% homology thereto. In an embodiment, the SAR further comprises vL/vH fragments that combine to form an Fv targeting a specific antigen. Exemplary vL/vH fragments and their target antigens are provided in **Table 3.** In an embodiment, the SAR further comprises Va/Vb or Vg/Vd fragments that combine to form a TCR-Fv targeting different antigens. Exemplary Va/Vb or Vg/Vd fragments and their target antigens are provided in **Table 4.**

In one embodiment, the fist binding domain of SAR comprises AABD that binds CD19 and a second binding moiety that targets BAFF-R. In an embodiment, the SAR further comprises vL/vH fragments that combine to form a Fv targeting a specific antigen. Exemplary vL/vH fragments and their target antigens are provided in **Table 3.** In an embodiment, the SAR further comprises Va/Vb or Vg/Vd fragments that combine to form a TCR-Fv targeting different antigens. Exemplary Va/Vb or Vg/Vd fragments and their target antigens are provided in **Table 4.**

In one embodiment, at least one binding domain of SAR comprises an AABD that binds to the spike glycoprotein of a virus. In an embodiment, the virus is coronavirus. In an embodiment, the at least one binding domain of SAR comprises an AABD that binds to the COVID-19 spike glycoprotein. In an embodiment, the at least one binding domain of SAR comprises an AABD that binds to the SARS spike glycoprotein. In an embodiment, the at least one binding domain of SAR comprises an AABD that binds to the MERS spike glycoprotein. The AABD that binds COVID-19 spike glycoprotein is for example the extracellular domain of ACE2 (Angiotensin-converting Enzyme 2; Genbank: AY623811) or a fragment thereof. The AABD that binds COVID-19 spike glycoprotein is for example a SVH, VHH, Centyrin, Affibody, vL, vH, Fv, scFv or a fragment thereof. The AABD that binds SARS spike glycoprotein is for example the extracellular domain of ACE2 receptor or a fragment thereof. The AABD that binds SARS spike glycoprotein is for example a SVH, VHH, Centyrin, Affibody, vL, vH, Fv, scFv or a fragment thereof.

In one embodiment, the fist binding domain of SAR comprises AABD that binds CD19 and a second binding moiety that targets CD79b. The V_{H} single domain antibody that binds CD19 is for example selected from SEQ ID NOs. 11524, 11526-11529, 11588 or from a sequence with at least 60%, 70%, 80% or 90% homology thereto. In an embodiment, the SAR further comprises vL/vH fragments that combine to form a Fv targeting a specific antigen. Exemplary vL/vH fragments and their target antigens are provided in **Table 3.** In an embodiment, the SAR further comprises Va/Vb or Vg/Vd fragments that combine to form a TCR-Fv targeting different antigens. Exemplary Va/Vb or Vg/Vd fragments and their target antigens are provided in **Table 4.**

In one embodiment, the binding domain of the SAR of the disclosure provides bispecific targeting. Thus, the binding domain comprises an AABD that binds PSMA and a second binding moiety that targets a second target. The AABD antibody that binds PSMA is for example selected from SEQ ID NOs. 11520-11523, 11667-11669 or from a sequence with at least 60%, 70%, 80% or 90% homology thereto. The second binding moiety may be an antibody fragment, typically a V_{H}single domain antibody. The second target may be selected from CD19, CD20, CD22, BCMA, PSCA, CS1, GPC3, CSPG4, EGFR, 5T4, L1 CAM, MUC16, ROR1, cKit, mesothelin, IL3Ra, c-Met, EGFRvlll, GD-2, NY-ESO-1 TCR or MAGE A3 TCR, HER2, Wilm's tumor gene 1 (WT1), carcinoembryonic antigen (CEA), mucin 16, MUC1, an immuno checkpoint target or combinations thereof. However, a skilled person would understand that other tumor antigens are also potential combination targets within the scope of the invention.

In one aspect the novel antigen binding domain of a SAR binds to an antigen preferentially or exclusively expressed on hematopoietic lineage cells. Exemplary antigens that are preferentially or exclusively expressed on hematopoietic lineage cells are CD19, CD20, CD22, BCMA, CS1, CD33, MPL, CD138, CD38 and CD123. In one aspect the novel antigen binding domain of a SAR binds to an antigen preferentially or exclusively expressed on non-hematopoietic lineage cells. Exemplary antigens that are preferentially or exclusively expressed on non-hematopoietic lineage cells are Mesothelin (MSLN), Her2, EGFR, PSMA, IL13Ra2 and the like. In one aspect the SAR expresses two or more novel antigen binding domains where at least one of the novel antigen binding domain binds to an antigen preferentially or exclusively expressed on hematopoietic lineage cells and at least one of the novel antigen domain binds to an antigen expressed on non-hematopoietic lineage cells.

In a preferred embodiment, a vH single domain antibody for use in a SAR as described herein is generated from human heavy chain only antibody produced in a transgenic rodent that expresses human heavy chain loci.

The mouse having functionally-silenced endogenous lambda and kappa L-chain loci may, for example, be made as disclosed in WO 2003/000737, which is hereby incorporated by reference in its entirety.

Alternative methods known in the art may be used for deletion or inactivation of endogenous mouse or rat immunoglobulin genes and introduction of human V_{H}, D and J genes in combination with mouse immunoglobulin constant region genes lacking C_{H}1 domains, mouse enhancer and regulatory regions.

The transgenic rodent described above produces human variable heavy chains which can be isolated and used for the generation of human V_{H} domains for use in the SARs of the invention, for example as described in WO 2016/062990 or WO 2016/113557.

Thus, the disclosure also relates to a SAR comprising a single V_{H} domain antibody generated from human variable heavy chains produced in a TKO mouse that expresses human heavy chain loci.

In a embodiment, a vL single domain antibody for use in a SAR as described herein is generated from human heavy chain only antibody produced in a transgenic rodent that expresses human light chain loci.

In addition to a binding domain as described in detail above, the SAR of the disclosure may further comprise one or more TCR constant chains or fragments and variants thereof. Exemplary TCR constant chains are provided in **Table 12.** The components of TCR constant chains, including TCR Ig-Like-C1-Domains (SEQ ID NO: 11848-11865), TCR connecting peptides (SEQ ID NO: 11867-11875), transmembrane domains (SEQ ID NO: 11877-11881), and TCR cytosolic domains (SEQ ID NO: 11883-11886) are provided in **Tables 13-16.** The TCR constant chains and fragments for the construction of the SARs are for example selected from SEQ ID NOs., 11848-11865, 11867-11875, 11877-11881, 11883-11886 or from a sequence with at least 60%, 70%, 80% or 90% homology thereto or functional variants thereof.

The vector encoding SAR generally have a limited capacity to encode a SAR. For example, the size of a SAR polynucleotide affects the titer of the lentiviral or retroviral vector. As such, a SAR that is small in size is desirable. In one aspect, the present disclosure describes a unispecific double chain SAR inclusive of two signal peptides and an intervening 2A linker that is less than 1765 nucleotide, less than 1770 nucleotide, less than 1780 nucleotide, less than 1790 nucleotide, less than 1800 nucleotide, less than 1820 nucleotide in size. In one aspect, the present disclosure describes a unispecific double chain SAR where one of the chain without the signal sequence is no longer than 815 nucleotide, 820 nucleotide, 825 nucleotides or 850 nucleotides and the second chain without the signal sequence is no longer than 790 nucleotides, 800 nucleotides, 810 nucleotides, 815 nucleotides, 820 nucleotides, 825 nucleotides or 850 nucleotides. In one aspect the SAR has the backbone of a SIR, cTCR, Ab-TCR, AABD-TCR, εTFP, γTFP, δTFP, αβTFP, γδTFP or a TCR. In one aspect the SAR has the backbone of a SIR. In one aspect the SAR has the backbone of a SIR, cTCR, Ab-TCR, AABD-TCR, αβTFP, γδTFP or a TCR with TCRα and TCRβ constant chains. In one aspect the SAR has the backbone of a SIR, cTCR, Ab-TCR, AABD-TCR, αβTFP, γδTFP or a TCR with TCRγ and TCRδ constant chains.

The disclosure also provides novel deletion mutants of the constant chains of TCRα (SEQ ID Nos: 11793-11803), TCRβ (SEQ ID NO: 11805-11817), TCRγ (SEQ ID NO: 11819-11823) and TCRδ (SEQ ID NO: 11825-11830) **(Table 12)** that can be used in the construction of SIRs and SARs based on the SIR backbones. Use of the deletion mutants of the constant chains of TCRα, TCRβ, TCRγ and TCRδ help to reduce the size of the SIR/SAR constructs, improve their packaging into viral vectors and thereby improve viral vector titer and transduction efficiency.

In one aspect, the present disclosure describes a double chain SAR where the TCRα constant chain fragment is less than 370, 380, 390, 400, 410 or 421 nucleotides in length and TCRβ constant chain fragment is less than 490 nucleotides, less than 500 nucleotides, less than 510 nucleotides, less than 520 nucleotides, less than 530 nucleotides or less than 540 nucleotides in length. In one aspect the SAR has the backbone of a SIR, cTCR, Ab-TCR, AABD-TCR, αβTFP, or a TCR. In one aspect the SAR has the backbone of a SIR. In one aspect the SAR has the backbone of a SIR with TCRα and TCRβ constant chains or TCRγ and TCRδ constant chains. In one aspect the SAR has the backbone of a cTCR with TCRα and TCRβ constant chains or TCRγ and TCRδ constant chains. In one aspect the TCRα and TCRβ constant chain fragments carry mutations that enhance their chain-pairing and reduce chain pairing with the endogenous TCRαβ chains. In one aspect the TCRα and TCRβ constant chain fragments carry mutations that result in an extra cysteine bond (double bond) between the two chains. Exemplary TCRα and TCRβ constant chains that can be used in the construction of the SARs of the disclosure are hTCRa-T48C-opt-d17 (SEQ ID NO: 11797) and hTCRb-S57C-opt1-d17 (SEQ ID NO: 11810) or TCRα and TCRβ chain variants comprising the above. Exemplary TCRα and TCRβ chain variants are provided in **Table 12.**

In one aspect the disclosure provides SARs comprising a TCRα constant chain fragment comprising hTCRa-T48C-opt-d17 (SEQ ID NO: 11797) or a variant with at least 70%, 75%, 80%, 85%, 90%, 95%, 98%, 99%, 99.9% homology to the sequence represented by hTCRa-T48C-opt-d17 (SEQ ID NO: 11797). In one aspect the disclosure provides SARs comprising a TCRα constant chain fragment comprising hTCRa-T48C-opt-d17 (SEQ ID NO: 11797) or its deletion mutant which retains the ability to pair with the complementary TCRβ constant chain. In one aspect the disclosure provides SARs comprising a TCRα constant chain fragment comprising hTCRa-T48C-opt-d17 (SEQ ID NO: 11797) or its deletion mutant which retains the ability to incorporate into the TCR/CD3 complex, recruit a TCR signaling module and/or induce T cell signaling upon engagement of target antigen. Additional TCRα constant chain and deletion mutants that can be used in the construction of the SARs are provided in **Table 12.**

In one aspect the disclosure provides SARs comprising a TCRβ constant chain fragment comprising hTCRb-S57C-opt1-d17 (SEQ ID NO: 11810) or a variant with at least 70%, 75%, 80%, 85%, 90%, 95%, 98%, 99%, 99.9% homology to the amino acid sequence represented by hTCRb-S57C-opt1-d17 (SEQ ID NO: 11810). In one aspect the disclosure provides SARs comprising a TCRβ constant chain fragment comprising hTCRb-S57C-opt1-d17 (SEQ ID NO: 11810) or its deletion mutant which retains the ability to pair with the complementary TCRα constant chain. In one aspect the disclosure provides SARs comprising a TCRβ constant chain fragment comprising hTCRb-S57C-opt1-d17 (SEQ ID NO: 11810) or its deletion mutant which retains the ability to incorporate into the TCR/CD3 complex, recruit a TCR signaling module and/or induce T cell signaling upon engagement of target antigen. Additional TCRα constant chain and deletion mutants that can be used in the construction of the SARs are provided in **Table 12.**

In one aspect the disclosure provides SARs comprising a TCRγ constant chain fragment comprising hTCRg-Opt-d12 (SEQ ID NO: 11821) or a variant with at least 70%, 75%, 80%, 85%, 90%, 95%, 98%, 99%, 99.9% homology to the sequence represented by hTCRg-Opt-d12 (SEQ ID NO: 11821). In one aspect the disclosure provides SARs comprising a TCRγ constant chain fragment comprising hTCRg-Opt-d12 (SEQ ID NO: 11821) or its deletion mutant which retains the ability to pair with the complementary TCRδ constant chain. In one aspect the disclosure provides SARs comprising a TCRg constant chain fragment comprising hTCRg-Opt-d12 (SEQ ID NO: 11821) or its deletion mutant which retains the ability to incorporate into the TCR/CD3 complex, recruit a TCR signaling module and/or induce T cell signaling upon engagement of target antigen.

In one aspect the disclosure provides SARs comprising a TCRγ constant chain fragment comprising hTCRg-Opt-d5 (SEQ ID NO (PRT): 11819) or a variant with at least 70%, 75%, 80%, 85%, 90%, 95%, 98%, 99%, 99.9% homology to the amino-acid sequence represented by hTCRg-Opt-d5 (SEQ ID NO (PRT): 11819). In one aspect the disclosure provides SARs comprising a TCRγ constant chain fragment comprising hTCRg-Opt-d5 (SEQ ID NO: 11819) or its deletion mutant which retains the ability to pair with the complementary TCRδ constant chain. In one aspect the disclosure provides SARs comprising a TCRγ constant chain fragment comprising hTCRg-Opt-d5 (SEQ ID NO: 11819) or its deletion mutant which retains the ability to incorporate into the TCR/CD3 complex, recruit a TCR signaling module and/or induce T cell signaling upon engagement of target antigen. Additional TCRγ constant chain and deletion mutants that can be used in the construction of the SARs are provided in **Table 12.**

In one aspect the disclosure provides SARs comprising a TCRδ constant chain fragment comprising hTCRd-opt-d13 (SEQ ID NO: 11828) or a variant with at least 70%, 75%, 80%, 85%, 90%, 95%, 98%, 99%, 99.9% homology to the sequence represented by hTCRd-opt-d13 (SEQ ID NO: 11828). In one aspect the disclosure provides SARs comprising a TCRδ constant chain fragment comprising hTCRd-opt-d13 (SEQ ID NO: 11828) or its deletion mutant which retains the ability to pair with the complementary TCRγ constant chain. In one aspect the disclosure provides SARs comprising a TCRδ constant chain fragment comprising hTCRd-opt-d13 (SEQ ID NO: 11828) or its deletion mutant which retains the ability to incorporate into the TCR/CD3 complex, recruit a TCR signaling module and/or induce T cell signaling upon engagement of target antigen.

In one aspect the disclosure provides SARs comprising a TCRδ constant chain fragment comprising hTCRd-opt-d2 (SEQ ID NO: 11825) or a variant with at least 70%, 75%, 80%, 85%, 90%, 95%, 98%, 99%, 99.9% homology to the sequence represented by hTCRd-opt-d5 (SEQ ID NO: 11828). In one aspect the disclosure provides SARs comprising a TCRδ constant chain fragment comprising hTCRd-opt-d5 (SEQ ID NO: 11825) or its deletion mutant which retains the ability to pair with the complementary TCRγ constant chain. In one aspect the disclosure provides SARs comprising a TCRδ constant chain fragment comprising hTCRd-opt-d5 (SEQ ID NO: 11825) or its deletion mutant which retains the ability to incorporate into the TCR/CD3 complex, recruit a TCR signaling module and/or induce T cell signaling upon engagement of target antigen. Additional TCRδ constant chain and deletion mutants that can be used in the construction of the SARs are provided in **Table 12.**

In one aspect, the disclosure relates to an isolated SAR comprising an antigen binding domain, a transmembrane domain and an intracellular signaling domain wherein said antigen binding domain comprises one or more, for example at least two, human variable heavy chain (VH) domains and is devoid of light chains.

In one embodiment, the SARs of the disclosure comprise one or more connecting peptides. Exemplary connecting peptides are represented by SEQ ID NO: 11867-11875 (Table 14). In one embodiment, the SARs of the disclosure comprise one or more TCR cytosolic domains. Exemplary TCR cytosolic domains are represented by SEQ ID NO: 11833-11886 (Table 16).

In one embodiment, the SARs of the disclosure are co-expressed with one or more accessory modules. Exemplary accessory modules include K13, Synth-K13, MC159, hNEMO-K277A, tBCMA *etc.* **(Table 24).**

In one embodiment, the SAR of the disclosure further comprises a hinge or spacer region which connects the extracellular antigen binding domain and the transmembrane domain. This hinge or spacer region can be used to achieve different lengths and flexibility of the resulting SAR. Examples of a hinge or spacer region that can be used according to the disclosure include, but are not limited to, Fc fragments of antibodies or fragments or derivatives thereof, hinge regions of antibodies, or fragments or derivatives thereof, C_{H}2 regions of antibodies, C_{H}3 regions of antibodies, artificial spacer sequences, for example peptide sequences, or combinations thereof. In one embodiment, the hinge is an lgG4 hinge or a CD8A hinge. Specifically, within the scope of disclosure are hinge sequences shown in **Table 17 and 18.**

In addition to a binding domain as described in detail above, the SAR of the disclosure further comprises one or two transmembrane domains. A "transmembrane domain" (TMD) as used herein refers to the region of the SAR which crosses the plasma membrane and is connected to the endoplasmic signaling domain and the antigen binding domain, in case of the latter optionally via a hinge. In one embodiment, the transmembrane domain of the SAR of the disclosure is the transmembrane region of a transmembrane protein (for example Type I transmembrane proteins), an artificial hydrophobic sequence or a combination thereof. In one embodiment, the transmembrane domain comprises the CD3zeta domain or CD28 transmembrane domain. Other transmembrane domains will be apparent to those of skill in the art and may be used in connection with alternate embodiments of the invention. Specifically, within the scope of disclosure are TMD sequences shown in **table 15** and 18.

The SAR of the disclosure may optionally further comprise an intracellular "activation domain" that transmits activation signals to T cells and directs the cell to perform its specialized function. Examples of domains that transduce the effector function signal and can be used accoridng to the disclosure include but are not limited to the ζ chain of the T-cell receptor complex or any of its homologs (*e*.*g*., η chain, FCER1G and β chains, MB 1 (Iga) chain, B29 (Ig ) chain, *etc.),* human CD3zeta chain, CD3 polypeptides (γ, δ and ε), syk family tyrosine kinases (Syk, ZAP 70, *etc.),* src family tyrosine kinases (Lck, Fyn, Lyn, *etc.).* Other activation domains will be apparent to those of skill in the art and may be used in connection with alternate embodiments of the invention. Specifically, within the scope of disclosure are intracellular activation domain sequence shown in SEQ ID NO: 11896 and 11783.

In one embodiment, the SAR of the disclosure further comprises one or more co- stimulatory domains to enhance SAR cell activity after antigen specific engagement. Inclusion of this domain in the SAR of the disclosure enhances the proliferation, survival and/or development of memory cells. Specifically, within the scope of disclosure are costimulatory domains sequences with SEQ ID NO: 11898 and 11781-82 as shown in Table 18.

In one embodiment, the SAR of the disclosure further comprises a "linker domain" or "linker region" that connects different domains of the SAR. This domain includes an oligo- or polypeptide region from about 1 to 500 amino acids in length. Suitable linkers will be apparent to those of skill in the art and may be used in connection with alternate embodiments of the invention. Specifically, within the scope of disclosure are linker domain sequences shown in **Table 11 and 13.** In an embodiment, the linker is a long linker and comprise a sequence between 25 and 500 amino acids in length. In particular, a long linker is used for connecting an AABD to a TCR connecting peptide which lack a vL, vH, Va, Vb, Vg, Vd or Ig linker domain.

In one embodiment, the SAR of the disclosure further comprises one or more "leader sequences" or signal peptides (SP). In one embodiment, the leader sequence is a CD8A signal peptide (SED ID NO: 31) or an IgH signal peptide (SEQ ID NO: 33). Specifically, within the scope of disclosure are leader sequences shown in **Table 2.** In an embodiment, each chain of a double chain SAR or one and a half chain SAR comprises a leader sequence or a signal peptide. The signal peptide is cleaved from the mature SAR polypeptide. Thus, although the sequences of SAR polypeptides are shown with their signal peptide (SP), it is understood by those skilled in the art that the signal peptide will not be present on the mature SAR polypeptide.

In some aspects, the SAR of the disclosure includes an antigen binding domain that transmits an inhibitory signal.

The SAR may further include a label, for example a label that facilitates imaging, such as a fluorescent label or other tag. This can, for example be used in methods for imaging tumor binding. The label may be conjugated to the antigen binding domain.

In one embodiment, the SARs of the disclosure are co-expressed with one or more accessory modules. Exemplary accessory modules include K13, Synth-K13, MC159, hNEMO-K277A, tBCMA *etc.* **(Table 24).**

In some embodiments, the extracellular domain of any one of the SARs disclosed herein may comprise one or more epitopes specific for *(e.g.,* specifically recognized by) an antigen binding agent, *e.g.,* a monoclonal antibody. These epitopes are also referred to herein as mAb-specific epitopes. Exemplary mAb-specific epitopes are disclosed in International Patent Publication No. WO 2016/120126, which is incorporated herein in its entirety. The disclosure provides new composition and locations for incorporation of epitopes in a SAR. In these embodiments, the extracellular domain comprises one or more antigen binding domains that bind to target antigens and one or more epitopes that bind to one or more antibodies (*e*.*g*., monoclonal antibody, bispecific antibody, antibody fragments, vHH, non-immunoglobulin antigen binding scaffold *etc.).* SARs comprising the epitopes can be single-chain or multi-chain.

The inclusion of eptiopes specific for monoclonal antibodies and other antigen binding agents *(e.g.,* vHH, non-immunoglobulin antigen binding domains *etc.)* in the extracellular domain of the SARs described herein allows sorting and depletion of engineered immune cells expressing the SARs. In some embodiments, this feature also promotes recovery of endogenous antigen-expressing cells that were depleted by administration of engineered immune cells expressing the SARs.

Accordingly, in some embodiments, the present disclosure relates to a method for sorting and/or depleting the engineered immune cells endowed with the SARs comprising antigen-binding agents (*e*.*g*., mAb)-specific epitopes and a method for promoting recovery of endogenous antigen-expressing cells *(i.e.,* normal healthy cells that express the antigen targeted by the SAR cells), such as bone marrow progenitor cells.

Several epitope-monoclonal antibody couples can be used to generate CARs comprising monoclonal antibody specific epitopes; in particular, those already approved for medical use, such as CD20 epitope/rituximab as a non-limiting example.

The disclosure also encompasses methods for sorting the engineered immune cells endowed with the antigen-specific SARs expressing the mAb-specific epitope(s) and therapeutic methods where the activation of the engineered immune cells endowed with these SARs is modulated by depleting the cells using an antibody that targets the external ligand binding domain of said CARs.

In some embodiments, the SAR-T cell comprises a polynucleotide encoding a suicide polypeptide, such as for example RQR8. See, *e*.*g*., WO2013153391 A, which is hereby incorporated by reference in its entirety. In SAR-T or SAR-NK cells comprising the polynucleotide, the suicide polypeptide is expressed at the surface of a SAR-T cell or SAR-NK cells. In some embodiments, the suicide polypeptide comprises the amino acid sequence shown in SEQ ID NO: 22419.

The suicide polypeptide may also comprise a signal peptide at the amino terminus, for example, CD8 signal peptide (SEQ ID NO: 10721).

When the suicide polypeptide is expressed at the surface of a CAR-T cell, binding of rituximab to the R epitopes of the polypeptide causes lysis of the cell. More than one molecule of rituximab may bind per polypeptide expressed at the cell surface. Each R epitope of the polypeptide may bind a separate molecule of rituximab. Deletion of antigen-specific SAR-T cells may occur *in vivo,* for example by administering rituximab to a patient. The decision to delete the transferred cells may arise from undesirable effects being detected in the patient which are attributable to the transferred cells, such as for example, when unacceptable levels of toxicity are detected.

In some embodiments, upon administration to a patient, engineered immune cells expressing at their cell surface any one of the antigen-specific SARs described herein may reduce, kill or lyse endogenous antigen-expressing cells of the patient. In one embodiment, a percentage reduction or lysis of antigen X-expressing endogenous cells or cells of a cell line expressing antigen X by engineered immune cells expressing any one of the antigen X-specific SARs described herein is at least about or greater than 10%, 15%, 20%, 25%, 30%, 35%, 40%, 45%, 50%, 55%, 60%, 65%, 70%, 75%, 80%, 85%, 90%, or 95%. In one embodiment, a percentage reduction or lysis of antigen X-expressing endogenous cells or cells of a cell line expressing antigen X by engineered immune cells expressing any one of the antigen X-specific SARs described herein is about 5% to about 95%, about 10% to about 95%, about 10% to about 90%, about 10% to about 80%, about 10% to about 70%, about 10% to about 60%, about 10% to about 50%, about 10% to about 40%, about 20% to about 90%, about 20% to about 80%, about 20% to about 70%, about 20% to about 60%, about 20% to about 50%, about 25% to about 75%, or about 25% to about 60%. In one embodiment, the endogenous antigen X-expressing cells are endogenous antige X-expressing bone marrow cells. In an exemplary embodiment, the endogenous antigen X is selected from the group of, but not limited to, CD19, CD33, CD34, CD123, MPL, CD70, CD20, CD22, CD30, BCMA, SLAMF7, CD138, CLL1, CD38 or a combination thereof.

In one embodiment, the percent reduction or lysis of target cells, *e.g.,* a cell line expressing antigen X, by engineered immune cells expressing at their cell surface membrane an antigen X-specific SAR of the disclosure can be measured using the assay disclosed herein (*e*.*g*., Flow cytometry).

In one aspect, provided are methods for *in vitro* sorting of a population of immune cells, wherein a subset of the population of immune cells comprises engineered immune cells expressing any one of the antigen X-specific SARs comprising epitopes specific for an antigen binding agent (*e*.*g*., monoclonal antibody, polyclonal antibody, vHH, DARPIN, Centyrin *etc.)* described herein. The method comprises contacting the population of immune cells with an antigen binding agent (*e*.*g*., monoclonal antibody) specific for the epitopes and selecting the immune cells that bind to the antigen binding agent *(e.g.,* monoclonal antibody) to obtain a population of cells enriched in engineered immune cells expressing antigen X-specific SAR.

In some embodiments, said antigen binding agent (*e*.*g*., monoclonal antibody) specific for said epitope is optionally conjugated to a fluorophore. In this embodiment, the step of selecting the cells that bind to the antigen binding agent (*e*.*g*., monoclonal antibody) can be done by Fluorescence Activated Cell Sorting (FACS). In some embodiments, said antigen binding agent (*e*.*g*., monoclonal antibody) specific for said epitope is optionally conjugated to a magnetic particle. In this embodiment, the step of selecting the cells that bind to the antigen binding agent (*e.g*., monoclonal antibody) can be done by Magnetic Activated Cell Sorting (MACS).

In some embodiments, the extracellular binding domain of the SAR comprises a mAb-specific epitope of one or more of SEQ ID NO: 22411-22432.

According to the disclosure, cells to be administered to the recipient may be enriched *in vitro* from the source population. Methods of expanding source populations are known in the art, and may include selecting cells that express an antigen such as CD34 antigen, using combinations of density centrifugation, immuno-magnetic bead purification, affinity chromatography, and fluorescent activated cell sorting, known to those skilled in the art.

In some embodiments, the method used for sorting immune cells expressing a SAR is the Magnetic- Activated Cell Sorting (MACS).

In some embodiments, the mAb used in the method for sorting immune cells expressing the SAR is chosen from alemtuzumab, ibritumomab tiuxetan, muromonab-CD3, tositumomab, abciximab, basiliximab, brentuximab vedotin, cetuximab, infliximab, rituximab, bevacizumab, certolizumab pegol, daclizumab, eculizumab, efalizumab, gemtuzumab, natalizumab, omalizumab, palivizumab, ranibizumab, tocilizumab, trastuzumab, vedolizumab, adalimumab, belimumab, canakinumab, denosumab, golimumab, ipilimumab, ofatumumab, panitumumab, QBEND-10 and/or ustekinumab. In some embodiments, said mAb is rituximab. In another embodiment, said mAb is QBEND-10.

In some embodiments, the SAR-T or SAR-NK cell comprises a selected epitope within the scFv having a specificity to be recognized by a specific antibody. See, *e.g.,* WO2016/120216, which is hereby incorporated by reference in its entirety. Such an epitope facilitates sorting and/or depleting the SAR-T or SAR-NK cells. The epitope can be selected from any number of epitopes known in the art. In some embodiments, the epitope can be a target of a monoclonal antibody approved for medical use, such as, for example without limitation, the CD20 epitope recognized by rituximab.

In some embodiments, the epitope is located within the SAR. In an embodiment, the epitope is attached near the N-terminus or at the N-terminus of vL and/or vH fragment comprising the antigen binding domain of a SAR (*e.g.,* 1^{st} generation CAR, 2^{nd} generation CAR, SIR, cTCR, Ab-TCR, zSIR, TFPε, TFPγ, TFPδ, αβTFP, γδTFP, TCR, HLA-independent TCR *etc.).* In an embodiment, the epitope is attached near the N-terminus or at the N-terminus of AABD fragment comprising the antigen binding domain of a SAR (*e.g.,* 1^{st} generation CAR, 2^{nd} generation CAR, SIR, cTCR, Ab-TCR, AABD-TCR, zSIR, TFPε, TFPγ, TFPδ, αβTFP, γδTFP, TCR, HLA-independent TCR *etc.).* In an embodiment, the epitope is separated from the antigen binding domaion of the SAR via a linker. In some embodiments, two instances of the same epitope, separated by linkers, may be used in the SAR.

In some embodiments, the epitope-specific antibody may be conjugated with a cytotoxic drug. It is also possible to promote CDC cytotoxicity by using engineered antibodies on which are grafted component(s) of the complement system. In some embodiments, activation of the CAR-T cells can be modulated by depleting the cells using an antibody which recognizes the epitope.

The SARs described herein may be synthesized as single polypeptide chains. In this embodiment, the antigen-specific targeting regions are at the N- terminus, arranged in tandem and are separated by a linker peptide.

In another aspect, the disclosure provides an isolated SAR polypeptide molecule comprising one or more antigen binding domains (*e*.*g*., antibody or antibody fragment, a ligand or a receptor) that bind to antigens as described herein and are jointed to one or more T cell receptor constant chains.

In some embodiments, a SAR may comprise or consist of a single polypeptide that contains a single antigen binding domain joined to the NH2-terminus of a single T cell receptor constant chain (Class 1).

In some embodiments, a SAR comprises or consists of two polypeptides that assemble to make a functional SAR (Class 2). Each of the polypeptides of such dual chain Class 2 SAR contains a T cell receptor constant chain and contains (as in Class 2A) or does not contain (as in Class 2B) one or more antigen binding domains. In Class 2A SARs, each of the antigen binding domains is joined to the N-terminus of a separate T cell receptor constant chain. For example, antigen binding domain 1 (*e.g.,* vL fragment of an antibody) is joined to the constant chain of T cell receptor beta (TCRβ) to constitute functional polypeptide unit 1 and antigen binding domain 2 (vH fragment of an antibody) is joined to the constant chain of T cell receptor α (TCRα) to constitute functional polypeptide unit 2. The two functional polypeptide units of such SAR are coexpressed in the same cell and pair with each other to become functionally active. It should be noted that each of the antigen binding domains may in turn be bispecific or multispecific, thereby allowing the Class 2 SARs to target more than 2 antigens.

In some embodiments, a dual polypeptides chain SAR comprises or consists of an antigen binding domain that is joined to the NH2-terminus of only one T cell receptor constant chain (functional polypeptide unit 1) but is coexpressed with a second T cell receptor constant chain. Such SARs are designated Class 2B. The purpose of the second T cell receptor constant chain in such Class 2B SARs is to facilitate the cell surface expression of the functional polypeptide unit 1 (*i.e.,* antigen binding domain 1 joined to a T cell receptor constant chain). As such, the second T cell receptor constant chain in Class 2B SARs may be expressed by itself or expressed as a fusion protein carrying an epitope tag *(e.g.,* MYC, V5, AcV5, G4Sx2, StrepTagII *etc.)* or expressed as a fusion protein carrying any irrelevant protein fragment (*e*.*g*., vL or vH fragment) so long as the irrelevant protein does not interfere with the assembly and function of the functional unit 1. As an example, a Class 2B SAR may comprise or consist of antigen binding domain 1 joined to the constant chain of T cell receptor alpha (TCRα) to constitute funcational polypeptide unit 1 and the empty *(i.e.,* lacking an antigen binding domain) constant chain of T cell receptor β (TCRβ) constituting the functional polypeptide unit 2.

In some embodiments, the two functional polypeptide units of Class 2 SARs are coexpressed in a cell using different vectors. In some embodiments, the two functional polypeptide units of the Class 2 SARs are coexpressed in a cell using a single vector which employs two separate regulatory elements (*e*.*g*., promoters) to encode for two polynucleotides encoding the two functional polypeptide units of Class 2 SARs. In some embodiments, the two functional polypeptide units of the Class 2 SARs are coexpressed in a cell using a single vector which employs a single promoter to express a polynucleotide containing an IRES sequence that separates the nucleotide fragments encoding the two polypeptides of the SAR. In some embodiments, the two functional polypeptide units of the Class 2 SARs are coexpressed in a cell using a single vector which employs a single promoter to express a polynucleotide encoding for a single polypeptide containing a cleavable linker *(e.g.,* F2A, T2A, E2A, P2A, Furine-SGSG-F2A, Furine-SGSG-T2A, Furine-SGSG-E2A, Furine-SGSG-P2A *etc.).* The resulting mRNA encodes a single polypeptide which subsequently generates the two functional polypeptide units of the SAR. In some embodiments, the two functional polypeptide units of the Class 2 SARs are coexpressed using transfection of a single mRNA sequence that encodes for both functional polypeptide units, while in other embodiments the two functional polypeptide units are coexpressed by transfection of two different mRNA sequences, each encoding for one functional polypeptide unit. In some embodiments, the vector or mRNA encoding the SAR may encode for additional genes/proteins (therapeutic controls, inhibitory molecules, accessory modules *etc.),* which may be separated from the SAR encoding sequences by IRES or cleavable linkers or combination thereof. In another embodiment, a therapeutic control or accessory module or both could be expressed in the cell in which SAR is expressed using a separate vector or mRNA. It is to be understood that the therapeutic controls or accessory modules are not essential for the function of a SAR and any of the SAR of the embodiment can be used without the therapeutic control or the accessory modules. For example, the antibiotic resistance cassette, such as PAC (puromycin resistance gene), can be removed from the SAR-encoding vectors of this disclosure without compromising the functionality of the SAR.

Also provided are functional variants of the SARs described herein, which have substantial or significant sequence identity or similarity to a parent SAR, which functional variant retains the biological activity of the SAR of which it is a variant. Functional variants encompass, for example, those variants of the SAR described herein (the parent SAR) that retain the ability to recognize target cells to a similar extent, the same extent, or to a higher extent, as the parent SAR. In reference to the parent SAR, the functional variant can, for instance, be at least about 30%, about 50%, about 75%, about 80%, about 85%, about 90%, about 91 %, about 92%, about 93%, about 94%, about 95%, about 96%), about 97%, about 98%, about 99% or more identical in amino acid sequence to the parent SAR.

A functional variant can, for example, comprise the amino acid sequence of the parent SAR with at least one conservative amino acid substitution. Alternatively, the functional variants can comprise the amino acid sequence of the parent SAR with at least one non-conservative amino acid substitution. In this case, it is preferable for the non-conservative amino acid substitution to not interfere with or inhibit the biological activity of the functional variant. The non-conservative amino acid substitution may enhance the biological activity of the functional variant, such that the biological activity of the functional variant is increased as compared to the parent CAR.

The SARs (including functional portions and functional variants) can be of any length, *i.e.,* can comprise any number of amino acids, provided that the SARs (or functional portions or functional variants thereof) retain their biological activity, *e*.*g*., the ability to specifically bind to antigen, detect diseased cells in a mammal, or treat or prevent disease in a mammal, *etc.* For example, the SAR can be about 300 to about 5000 amino acids long, such as 300, 400, 500, 600, 700, 800, 900, 1000 or more amino acids in length.

In another aspect, the disclosure relates to an isolated nucleic acid construct comprising at least one nucleic acid encoding a SAR as defined above. In one embodiment, the nucleic acid encodes a protein that targets one or more of the targets listed in **Table B.**

The nucleic acid and amino acid SEQ ID Nos of suitable SAR sequences within the scope of the disclosure are are listed in **Tables 25-36 and 41-50.** Also within the scope of the disclosure are sequences with at least 60%, 70%, 80% or 90% homology thereto.

The term "nucleic acid," "polynucleotide," or "nucleic acid molecule" refers to deoxyribonucleic acid (DNA) or ribonucleic acid (RNA), or a combination of a DNA or RNA. RNA includes *in vitro* transcribed RNA or synthetic RNA; an mRNA sequence encoding a SAR polypeptide as described herein). The nucleic acid may further comprise a suicide gene. The nucleic acid may further comprise a kill-switch. The nucleic acid may further comprise a therapeutic control or accessory module. The construct may be in the form of a plasmid, vector, transcription or expression cassette.

In one embodiment, the vector is an *in vitro* transcribed vector, *e.g.,* a vector that transcribes RNA of a nucleic acid molecule described herein. The expression vector may be provided to a cell in the form of a viral vector. Viral vector technology is well known in the art and is described, for example, in Sambrook et al. (Molecular Cloning: A Laboratory Manual, Cold Spring Harbor Laboratory, New York, 2013). A number of viral based systems have been developed for gene transfer into mammalian cells. For example, retroviruses such as, adenovirus vectors are used. In one embodiment, a lentivirus vector is used. This is demonstrated in the examples. The disclosure also relates to a virus comprising a SAR described above.

The disclosure also includes an RNA construct that can be directly transfected into a cell. A method for generating mRNA for use in transfection involves *in vitro* transcription (IVT) of a template with specially designed primers, followed by poly A addition, to produce a construct containing 3' and 5' untranslated sequence ("UTR") *(e.g.,* a 3' and/or 5' UTR described herein), a 5' cap *(e.g.,* a 5' cap described herein) and/or Internal Ribosome Entry Site (IRES) *(e.g.,* an IRES described herein), the nucleic acid to be expressed, and a poly A tail, typically 50-2000 bases in length (SEQ ID NO:13-16). RNA so produced can efficiently transfect different kinds of cells. In one embodiment, the template includes sequences for the SAR. In one embodiment, an RNA SAR vector is transduced into a cell, *e.g.,* a T cell or a NK cell, by electroporation. In another embodiment, an RNA SAR vector is transduced into a cell, *e.g.,* a T cell or a NK cell, by causing transient perturbations in cell membrane using a microfluid device. The different chains (or functional polypeptide units) of a SAR can be also introduced in a cell using one or more than one vector a combination of different vectors or techniques.

In another embodiment, one chain or functional polypeptide unit of SAR can be introduced using a retroviral vector while the other functional polypeptide unit is introduced using a lentiviral vector. In another aspect, one functional polypeptide unit is introduced using a lentiviral vector while the other functional polypeptide unit is introduced using a sleeping beauty transposon. In yet another aspect, one functional polypeptide unit is introduced using a lentiviral vector while the other functional polypeptide unit is introduced using RNA transfection. In yet another aspect, one functional polypeptide units is produced in a cell by genetic recombination at the endogeneous TCR chain loci using gene targeting techniques known in the art while the other functional polypeptide unit is introduced using a lentiviral or a retroviral vector.

RNA can be introduced into target cells using any of a number of different methods, which include, but are not limited to, electroporation, cationic liposome mediated transfection using lipofection, polymer encapsulation, peptide mediated transfection, or biolistic particle delivery systems such as "gene guns" (see, for example, Nishikawa, et al. Hum Gene Ther., 12(8):861-70 (2001) or by causing transient perturbations in cell membranes using a microfluidic device (see, for example, patent applications WO 2013/059343 A1 and PCT/US2012/060646).

In some embodiments, the non-viral method includes the use of a transposon (also called a transposable element). In some embodiments, a transposon is a piece of DNA that can insert itself at a location in a genome, for example, a piece of DNA that is capable of self-replicating and inserting its copy into a genome, or a piece of DNA that can be spliced out of a longer nucleic acid and inserted into another place in a genome. For example, a transposon comprises a DNA sequence made up of inverted repeats flanking genes for transposition. Exemplary methods of nucleic acid delivery using a transposon include a Sleeping Beauty transposon system (SBTS) and a piggyBac (PB) transposon system.

In some embodiments, cells, *e.g.,* T or NKT or stem cells or iPSC or synthetic T cell, are generated that express a SAR described herein by using a combination of gene insertion using the SBTS and genetic editing using a nuclease (*e*.*g*., Zinc finger nucleases (ZFNs), Transcription Activator-Like Effector Nucleases (TALENs), the CRISPR/Cas system, or engineered meganuclease reengineered homing endonucleases).

In some embodiments, use of a non-viral method of delivery permits reprogramming of cells, *e.g.,* T or NKT or stem cells or iPSC or synthetic T cell, and direct infusion of the cells into a subject. Advantages of non-viral vectors include but are not limited to the ease and relatively low cost of producing sufficient amounts required to meet a patient population, stability during storage, and lack of immunogenicity.

In a further aspect, the disclosure also relates to an isolated cell or cell population comprising one or more nucleic acid construct as described above. The cell has thus been genetically modified to express a SAR nucleic acid construct of the invention. Thus, the disclosure provides genetically engineered cells which comprise and stably express a SAR nucleic acid construct of the invention. In one embodiment, the cell is selected from the group consisting of a T cell, a NKT cell, a Natural Killer (NK) cell, a cytotoxic T lymphocyte (CTL), a regulatory T cell, hematopoietic stem cells and/or pluripotent embryonic/induced stem cells. T cells may be isolated from a patient for transfection with a SAR nucleic acid construct of the invention.

It is specifically contemplated herein that each of the individual embodiments described herein can be combined, *e.g*., in a single cell. By way of non-limiting example, a single cell could comprise a first complete multi-component SAR and a second partial multi-component SAR, wherein each multi-component SAR can be according to any of the embodiments described herein.

For example, cells can be transfected with the nucleic acid of the disclosure *ex vivo.* Various methods produce stable transfectants which express a SARs of the invention. In one embodiment, a method of stably transfecting and re-directing cells is by electroporation using naked DNA. Additional methods to genetically engineer cells using naked DNA encoding a SAR of the disclosure include but are not limited to chemical transformation methods (*e.g*., using calcium phosphate, dendrimers, liposomes and/or cationic polymers), non-chemical transformation methods (*e.g*., electroporation, optical transformation, gene electrotransfer and/or hydrodynamic delivery) and/or particle-based methods (*e.g*., impalefection, using a gene gun and/or magnetofection). The transfected cells demonstrating presence of an integrated un-rearranged vector and expression of the SAR may be expanded *ex vivo.* Viral transduction methods may also be used to generate redirected cells which express the SAR of the invention.

In some embodiments, a vector of the disclosure can further comprise a promoter. Non-limiting examples of a promoter include, for example, an EF1α promoter, an EF1α promoter variant (SEQ ID NO: 7), a CMV IE gene promoter, MNDU3 promoter (SEQ ID NO: 24), an ubiquitin C promoter, a core-promoter or a phosphoglycerate kinase (PGK) promoter. In some embodiments, the vector is an RNA nucleic acid. In some embodiments, the vector comprises a poly(A) tail. For example, contemplated herein is a poly(A) tail comprising about 150 adenosine bases (SEQ ID NO: 13-16). In some embodiments, the vector comprises a 3'UTR. In an embodiment, the SAR is expressed under the control of a synthetic notch (SynNotch) combinatorial antigen circuit (Dannenfelser et al., 2020, Cell Systems 11, 215-228).

In another aspect, the disclosure provides a method of making a cell *(e.g.,* an immune effector cell or population thereof) comprising introducing into (*e.g*., transducing) a cell, *e.g.,* a T cell, a NKT cell or a stem cell or a iPSC or a synthetic T cell described herein, with a vector comprising a nucleic acid encoding a SAR, *e.g.,* a SAR described herein; or a nucleic acid encoding a SAR molecule *e.g.,* a SAR described herein.

The cell can be an immune effector cell *(e.g.,* a T cell, a NKT cell, a NK cell, a monocyte/macrophage or a combination thereof) or a stem/progenitor cell that can give rise to an immune effector cell or a synthetic T cell. In some embodiments, the cell in the methods is diaglycerol kinase (DGK) and/or Ikaros deficient. In some embodiments, the cell in the methods is deficient in constant chains of endogenous T cell receptor α, β1, β2, pre-TCRα, γ or δ or combination thereof. In some embodiments, the cell in the methods is deficient in HLA antigens. In some embodiments, the cell in the methods is deficient in β2 microglobulin. In some embodiments, the cell in the methods is deficient in expression of the target antigen of SAR. For example, the SAR expressing T cell is deficient in endogenous CD5 in case the SAR is directed against CD5 or is deficient in TCR-beta1constant chain in case the SAR is directed against TCR-beta1 constant chain or is deficient in TCR-beta2 constant chain in case the SAR is directed against TCR-beta2 or is deficient in CS1 in case the SAR is directed against CS1.

In some embodiment, the introducing the nucleic acid molecule encoding a SAR comprises transducing a vector comprising the nucleic acid molecule encoding a SAR, or transfecting the nucleic acid molecule encoding a SAR, wherein the nucleic acid molecule is an *in vitro* transcribed RNA. In some embodiments, the nucleic acid molecule encodes two or more components of a SAR, are introduced by transducing a cell with more than one vector or transfecting with two or more nucleic acid molecules encoding the different subunits of a SAR. For example, a cell may be transduced with two separate vectors each encoding one of the two functional polypeptide units of a SAR. Similarly, a cell may be transduced with two separate *in vitro* transcribed RNAs each encoding one of the two functional polypeptide units of a SAR. In addition to the functional polypeptide units of the SAR, each of the RNAs may carry a different selection marker or reporter (*e.g.*, tEGFR or CD34 or CNB30 or mutant DHFR) that can be used to select the cells transduced with both the RNAs and thus expressing both the the functional polypeptide units of the SAR.

In some embodiments, the method further comprises: a) providing a population of immune effector cells *(e.g.,* T cells or NK cells); and b) removing T regulatory cells from the population, thereby providing a population of T regulatory-depleted cells; wherein steps a) and b) are performed before introducing the nucleic acid encoding the SAR to the population. In embodiments of the methods, the T regulatory cells comprise CD25⁺ T cells, and are removed from the cell population using a CD25 antibody, or fragment thereof. The CD25 antibody, or fragment thereof, can be operably linked to a substrate, *e.g*., a bead. In other embodiments, the population of immune effector cells that is depleted of T regulatory cells provided from step (b) contains less than 30%, 25%, 20%, 15%, 10%, 5%, 4%, 3%, 2%, 1% of CD25+ cells. In yet other embodiments, the method further comprises: removing cells from the population which express a disease-associated antigen that does not comprise CD25 to provide a population of T regulatory-depleted and disease-associated antigen depleted cells prior to introducing the nucleic acid encoding a SAR to the population. The disease-associated antigen can be selected from CD19, CD30, CD123, CD20, CD22, CD33, CD138, BCMA, Lym1, Lym2, CD79b, CD170, CD179b, CD14 or CD11b, or a combination thereof.

In other embodiments, the method further comprises depleting cells from the population which express a checkpoint inhibitor, to provide a population of T regulatory-depleted and inhibitory molecule depleted cells prior to introducing the nucleic acid encoding a SAR to the population. The checkpoint inhibitor can be chosen from CTLA-4, PD-1, LAG-3, TIM3, B7-H1, CD160, P1H, 2B4, CEACAM (*e.g*., CEACAM-1, CEACAM-3, and/or CEACAM-5), TIGIT, BTLA, and LAIRl.

The disclosure also provides recombinant cells, *e.g.,* an immune effector cell, *(e.g.,* a population of cells, *e.g.,* a population of immune effector cells) and/or a stem cell *(e.g.,* a hematopoietic stem cell, a peripheral blood stem cell, a bone marrow derived stem cell, an immune stem cell, an induced pluripotent stem cell or iPSC) comprising a nucleic acid molecule, a SAR polypeptide molecule, or a vector as described herein.

In some embodiments, the cell is an immune cell. Non-limiting examples of immune cells include T-cells, NKT cells, NK-cells and monocyte/macrophages. Further, non-limiting examples of T-cells include Tregs, CD8+ T cells, and CD4+ T cells. In one embodiment, the cell is a human T cell. In some embodiments, the cell is a human cell. In some embodiments, the cell is a dog cell.

In one embodiment, the human cell is a T cell, an NKT cell or an NK cell that expresses P-glycoprotein (P-gp or Pgp; MDR1, ABCB1, CD243). In one embodiment, the human cell is a T cell, an NKT cell or an NK cell that stains dull with dyes that are substrates of P-glycoprotein mediated efflux. In one embodiment, the human cell is a T cell, an NKT cell or an NK cell that is resistant to a drug transported by P-gp. In one embodiment, the cell is a T cell as described in application no. PCT/US2017/042248, which is incorporated herein by reference. In some embodiments cells which lack expression of P-gp or P-gp activity are removed from the population.

The single chain and double chain SARs of the disclosure can be expressed in T cells in which the expression of the endogenous TCRα (TRAC), TCRβ (TRBC), TCRγ (TRGC) and/or TCRδ (TRDC) chains has been reduced or eliminated using methods known in the art. Such T cells in which the expression of endogenous functional TRAC, TRBC, TRGC and/or TRDC chains has been reduced or eliminated can be used for the purpose of allogeneic cell therapy.

In one embodiment, the cell is a T cell and the T cell is deficient in one or more of endogenous T cell receptor chains. T cells stably lacking expression of a functional TCR according to the disclosure may be produced using a variety of approaches such as use of Zn finger nucleases (ZFN), CRISP/Cas9 and shRNA targeting the endogenous T cell receptor chains. A non-limiting exemplary method relating to shRNAs is described in US 2012/0321667A1, which is incorporated herein by reference. Another non-limitng expemplary method relating to eliminating endogenous TCR expression using ZFNs targeting constant regions of α and β chains of TCRs is described in Torikai H et al (Blood, 119(24), June, 14 2012). Methods to generate T cells with reduced or eliminated expression of one or more TCR chains using CRISP/Cas9 are also known in the art. It is to be noted that in some embodiments, the SARs of the disclosure comprise constant chains of TCRs that are codon-optimized or are designed to differ in nucleotide sequences from the endogenous TCR constant chains and therefore escape targeting by the CRISP/Cas9, ZFN and/or shRNAs targeting the endogenous TCR constant chains.

A T cell lacking a functional endogenous TCR can be, *e.g*., engineered such that it does not express any functional endogenous TCR on its surface, engineered such that it does not express one or more subunits (*e.g*., constant chains of endogenous TCRα, TCRβ1, TCRβ2, TCRγ, TCRδ or pre-TCRα) that comprise a functional endogenous TCR or engineered such that it produces very little functional endogenous TCR on its surface. Alternatively, the T cell can express a substantially impaired endogenous TCR, *e.g.,* by expression of mutated or truncated forms of one or more of the subunits of the TCR. The term "substantially impaired TCR" means that this TCR will not elicit an adverse immune reaction in a host. The unmodified TCRs are generally poorly expressed in primary human T cells when expressed ectopically (*e.g*., using retroviral or lentiviral vectors), suggesting that they compete inefficiently with endogenous TCR chains for cell surface expression. However, optimization of the TCR chains for efficient translation in human cells results in better expression of the introduced TCR. More importantly, ectopic expression of such dominant TCR prevented surface expression of a large proportion of the endogenous TCR repertoire in human T cells.

SARs with the backbones of TFPs comprising CD3ε, CD3γ, and CD3δ chains (designated as CD3ε/γ/δ TFPs) have poor expression and activity when expressed in αβ T cells that lack or have impaired functional endogenous or native TCRα or TCRβ chain polypeptide on their surface. For example, SARS represented by SEQ ID NO: 4110-4112 have impaired expression and activity *(e.g.,* T cell activation, proliferation, cytokine production and cytotoxicity *etc.)* in αβ T cell in which the endogenous TRAC genomic locus has been disrupted. The disclosure provides a solution to this problem by re-expressing a TCRα constant chain (TRAC chain) polypeptide or a fragment thereof in T cells in which the expression of native full length TCRα chain polypeptide has been reduced or eliminated as described in WO2019067805, which is incorporated in its entirety by reference herein. In an exemplary embodiment, the expression and signaling activity of SARs based on CD3ε/γ/δ backbone can be restored in αβ T cells in which the expression of native TCRα chain is reduced or eliminated by expressing into such T cells a nucleic acid construct encoding an exogenous TCRα constant chain (TRAC chain) *(e.g.,* SEQ ID NO (DNA): 1045 and SEQ ID NO (PRT): 11735) or a fragment thereof. In another exemplary embodiment, the expression and signaling activity of SARs based on CD3ε/γ/δ backbone can be restored in αβ T cells in which the expression of native TCRβ chain is reduced or eliminated by expressing into such T cells a nucleic acid construct encoding an exogenous TCRβ constant chain (TRBC chain) *(e.g.,* SEQ ID NO (DNA): 1061 and SEQ ID NO (PRT): 11751) or a fragment thereof. In another exemplary embodiment, the expression and signaling activity of SARs based on CD3ε/γ/δ backbone can be restored in γδ T cells in which the expression of native TCRγ chain is reduced or eliminated by expressing into such T cells a nucleic acid construct encoding an exogenous TCRγ constant chain (TRGC chain) *(e.g.,* SEQ ID NO (DNA): 1082 and SEQ ID NO (PRT): 11771) or a fragment thereof. In another exemplary embodiment, the expression and signaling activity of SARs based on CD3ε/γ/δ backbone can be restored in γδ T cells in which the expression of native TCRδ chain is reduced or eliminated by expressing into such T cells a nucleic acid construct encoding an exogenous TCRδ constant chain (TRDC chain) *(e.g.,* SEQ ID NO (DNA): 1084 and SEQ ID NO (PRT): 11774) or a fragment thereof.

In another exemplary embodiment, the expression and signaling activity of SARs based on CD3ε/γ/δ backbone can be restored in αβ T cells in which the expression of native TCRα and/or β chain is reduced or eliminated by expressing into such T cells a nucleic acid construct encoding an exogenous TCRγ constant chain (TRGC chain) *(e.g.,* SEQ ID NO (DNA): 1082 and SEQ ID NO (PRT): 11771) or a fragment thereof and/or an exogenous TCRδ constant chain (TRDC chain) *(e.g.,* SEQ ID NO (DNA): 1084 and SEQ ID NO (PRT): 11774) or a fragment thereof.

The disclosure further provides that the expression and activity of CD3ε/γ/δ based SARs can be restored in T cells with impaired or lack of expression native TCRα/β/γ or δ chains by coexpression of a SAR on the SIR, Ab-TCR, AABD-TCR, or TCR backbones comprising the missing TCRα/β/γ or δ constant chains. The disclosure provides that for combination therapies with allogeneic T cells involving two SARs, a CD3α/γ/δ TFP-based SAR is typically combined with a SAR based on SIR, Ab-TCR, AABD-TCR or TCR backbones which incorporate the TCR constant chain or TCR constant chain fragment whose expression is reduced or missing in the allogeneic T cells.

In an embodiment, the single chain and double chain SARs of the disclosure can be targeted to the TRAC, TRBC, TRGC and/or TRDC locus in T cells using methods as described in PCT/US2018/053247 and Eyquem J et al (Nature, 543(7643):113-117, 2017) which are incorporated in their entirety by reference herein. Such T cells in which the endogenous TRAC, TRBC, TRGC and/or TRDC loci are disrupted by insertion of SAR can be used for the purpose of allogeneic cell therapy.

In an embodiment, the SAR of the disclosure is expressed under the physiological regulatory mechanisms afforded by endogenous TCR genes. In an embodiment, the SAR is expressed under the promoter and 3' untranslated regulatory mechanisms afforded by endogenous TCR genes. In one embodiment, the expression cassette encoding a SAR is targeted to the endogenous TCRα, TCRβ1/β2, TCRγ or TCRδ gene locus.

In an embodiment, the SAR is targeted to the endogenous TCRα gene locus (TRAC) so that the TCRα constant chain of the SAR *(i.e.,* a SAR with the backbone of a SIR, cTCR, Ab-TCR, AABD-TCR, αβTFP, or TCR) is expressed wholly or in part from the endogenous native TCRα constant chain gene. In an embodiment, the SAR is targeted to the endogenous TCRα gene locus (TRAC) so that the TCRα constant chain of the SAR *(i.e., a* SAR with the backbone of a SIR, cTCR, Ab-TCR, AABD-TCR, αβTFP, or TCR) is encoded completely or in part by at least one of the exons of the endogenous TCRα constant chain gene. In an embodiment, the SAR is targeted to the endogenous TCRα gene locus (TRAC) so that the TCRα constant chain of the SAR *(i.e.,* a SAR with the backbone of a SIR, cTCR, Ab-TCR, AABD-TCR, αβTFP, or TCR) shares completely or in part the 3' untranslated region and polyadenylation sequence of the native/endogenous TCRα constant chain gene.

In an embodiment, the SAR is targeted to the endogenous TCRβ gene locus (TRBC) so that the TCRβ constant chain of the SAR *(i.e.,* a SAR with the backbone of a SIR, cTCR, Ab-TCR, AABD-TCR, αβTFP, or TCR) is expressed wholly or in part from the endogenous native TCRβ constant chain gene. In an embodiment, the SAR is targeted to the endogenous TCRβ gene locus (TRBC) so that the TCRβ constant chain of the SAR *(i.e., a* SAR with the backbone of a SIR, cTCR, Ab-TCR, AABD-TCR, αβTFP, or TCR) is encoded completely or in part by at least one of the exons of the endogenous TCRβ constant chain gene. In an embodiment, the SAR is targeted to the endogenous TCRβ gene locus (TRBC) so that the TCRα constant chain of the SAR *(i.e.,* a SAR with the backbone of a SIR, cTCR, Ab-TCR, AABD-TCR, αβTFP, or TCR) shares completely or in part the 3' untranslated region and polyadenylation sequence of the native/endogenous TCRβ constant chain gene.

In an embodiment, the SAR is targeted to the endogenous TCRγ gene locus (TRGC) so that the TCRγ constant chain of the SAR *(i.e.,* a SAR with the backbone of a SIR, cTCR, Ab-TCR, AABD-TCR, γδTFP, or γδTCR) is expressed wholly or in part from the endogenous native TCRγ constant chain gene. In an embodiment, the SAR is targeted to the endogenous TCRγ gene locus (TRGC) so that the TCRγ constant chain of the SAR *(i.e., a* SAR with the backbone of a SIR, cTCR, Ab-TCR, AABD-TCR, γδTFP, or γδTCR) is encoded completely or in part by at least one of the exons of the endogenous TCRγ constant chain gene. In an embodiment, the SAR is targeted to the endogenous TCRγ gene locus (TRGC) so that the TCRγ constant chain of the SAR *(i.e.,* a SAR with the backbone of a SIR, cTCR, Ab-TCR, AABD-TCR, γδTFP, or γδTCR) shares completely or in part the 3' untranslated region and polyadenylation sequence of the native/endogenous TCRγ constant chain gene.

In an embodiment, the SAR is targeted to the endogenous TCRδ gene locus (TRDC) so that the TCRδ constant chain of the SAR *(i.e.,* a SAR with the backbone of a SIR, cTCR, Ab-TCR, AABD-TCR, γδTFP, or γδTCR) is expressed wholly or in part from the endogenous native TCRδ constant chain gene. In an embodiment, the SAR is targeted to the endogenous TCRδ gene locus (TRDC) so that the TCRδ constant chain of the SAR *(i.e., a* SAR with the backbone of a SIR, cTCR, Ab-TCR, AABD-TCR, γδTFP, or γδTCR) is encoded completely or in part by at least one of the exons of the endogenous TCRδ constant chain gene. In an embodiment, the SAR is targeted to the endogenous TCRδ gene locus (TRGC) so that the TCRδ constant chain of the SAR *(i.e.,* a SAR with the backbone of a SIR, cTCR, Ab-TCR, AABD-TCR, γδTFP, or γδTCR) shares completely or in part the 3' untranslated region and polyadenylation sequence of the native/endogenous TCRδ constant chain gene.

The disclosure also provides compositions and methods for targeting bispecific and multispecific SARs to the TRAC and/or TRBC loci for the purpose of generating allogeneic SAR-T cells.

In one embodiment, the cell is a stem cell and the stem cell is deficient in one or more of endogenous T cell receptor chains. In another embodiment, the cell is a stem cell in which one or more target antigens *(e.g.,* MPL, CD33, CD123, CD19, *etc.)* of the SAR have been deleted or mutated to a form that is no longer recognized by the SAR. As an example, a SAR targeting CD19 is expressed in stem cells that have been made deficient in CD19 using CRISP/Cas9 or Zn finger nucleases so that the B cells produced by such stem cells are not eliminated by the T cells expressing the CD19-targeting SAR. Alternatively, a SAR targeting CD19 is expressed in stem cells in which the endogenous CD19 has been mutated to a form that is not targeted by SAR using CRISP/Cas9 or Zn finger nucleases so that the B cells produced by such stem cells are not eliminated by the T cells expressing the CD19-targeting SAR. In another embodiment, the SAR is expressed in immune effector cells and the stem cells from an autologous or an allogeneic donor are genetically engineered to either lack the expression of the SAR-target antigen or to express a mutated form of SAR target antigen which is not recognized by the SAR. For example, a SAR targeting CD19 is expressed in T cells that are infused into a patient along with autologous or allogeneic hematopoietic stem cells that have been made deficient in CD19 using CRISP/Cas9 or Zn finger nucleases so that the B cells produced by such stem cells are not eliminated by the T cells expressing the CD19-targeting SAR. Alternatively, a SAR targeting CD19 is expressed in T cells that are infused into a patient along with autologous or allogeneic hematopoietic stem cells in which the endogenous CD19 has been mutated to a form that is not targeted by SAR using CRISP/Cas9 or Zn finger nucleases so that the B cells produced by such stem cells are not eliminated by the T cells expressing the CD19-targeting SAR. A similar approach can be used to mutate or eliminate other endogenous antigens *(e.g.,* MPL, CD33, CD123 *etc.)* in stem cells using shRNA, CRISP/Cas9 or Zn finger nucleases in subjects receiving SAR-T cells targeting these antigens for the treatment of specific diseases in which these antigens are expressed on disease associated or disease-causing cells.

T cells or natural killer (NK) or stem cells can be obtained from a subject. The term "subject" is intended to include living organisms in which an immune response can be elicited (*e.g*., mammals). Examples of subjects include humans, monkeys, chimpanzees, dogs, cats, mice, rats, and transgenic species thereof. T cells can be obtained from a number of sources, including peripheral blood mononuclear cells, bone marrow, lymph node tissue, cord blood, thymus tissue, tissue from a site of infection, ascites, pleural effusion, spleen tissue, and tumors. T cells could be tissue resident gamma-delta T cells, which can be cultured and expanded *in vitro* prior to expression of the SAR.

In another aspect, the disclosure relates to a method for producing a genetically modified cell or cell population comprising expressing in said cell or cell population a SAR nucleic acid construct of the invention. The method may include introducing into the cell a nucleic acid as described herein *(e.g.,* an *in vitro* transcribed RNA or synthetic RNA; an mRNA sequence encoding a SAR polypeptide as described herein). In embodiments, the RNA expresses the SAR polypeptide transiently. In one embodiment, the cell is a cell as described herein, *e.g.,* an immune effector cell *(e.g.,* T cells or NK cells, or cell population). Cells produced by such methods are also within the scope of the invention.

In another aspect, the disclosure relates to an *ex vivo* method for generating a population of cells for use in adaptive immunotherapy comprising transforming said cell with a SAR of the invention.

In certain aspects of the disclosure, immune effector cells, *e.g.,* T cells, can be obtained from a unit of blood collected from a subject using any number of techniques known to the skilled artisan, such as Ficoll^{™} separation. In one preferred aspect, cells from the circulating blood of an individual are obtained by apheresis. In one aspect, the cells are collected from a subject in whom the T cells have been mobilized by administration of an agent. In some embodiments, the immune cells are collected from a donor who has been administered a CXCR4 antagonist *(e.g.,* Plerixafor), a cytokine *(e.g.,* G-CSF, GM-CSF or sargramostim, Neulasta or Pegfilgastrim), a beta2 agonist (*e.g*., epinephrine), a tyrosine kinase inhibitor *(e.g.,* dasatinib), chemotherapy drug(s) *(e.g.,* cyclophosphamide, doxorubicin *etc.)* either singly or in combination, prior to the collection of immune cells. In some embodiments, the donor is an autologous donor while in other embodiments, the donor is an allogeneic donor.

The apheresis product usually contains lymphocytes, including T cells, monocytes, granulocytes, B cells, other nucleated white blood cells, red blood cells, and platelets. In one aspect, the cells collected by apheresis may be washed to remove the plasma fraction and, optionally, to place the cells in an appropriate buffer or media for subsequent processing steps. In one embodiment, the cells are washed with phosphate buffered saline (PBS). In an alternative embodiment, the wash solution lacks calcium and may lack magnesium or may lack many if not all divalent cations.

It is recognized that the methods of the application can utilize culture media conditions comprising 5% or less, for example 2%, human AB serum, and employ known culture media conditions and compositions, for example those described in Smith et al., "Ex vivo expansion of human T cells for adoptive immunotherapy using the novel Xeno-free CTS Immune Cell Serum Replacement" Clinical & Translational Immunology (2015) 4, e31; doi: 10.1038/cti.2014.31.

In one aspect, T cells are isolated from peripheral blood lymphocytes by lysing the red blood cells and depleting the monocytes, for example, by by counterflow centrifugal elutriation or centrifugation through a PERCOLL^{™} gradient.

In another embodiment, a SAR-expressing effector cell described herein can further express an agent which enhances the activity of a SAR-expressing cell. In some embodiments, the agent is one that inhibits an inhibitory molecule. Non-limiting examples of inhibitory molecules include PD-1, PD-L1, CTLA-4, TIM-3, CEACAM (*e.g*., CEACAM-1, CEACAM-3 and/or CEACAM-5), LAG-3, VISTA, BTLA, TIGIT, LAIR1, CD160, 2B4 and TGFR beta. In one embodiment, the agent that inhibits an inhibitory molecule comprises a first polypeptide, *e.g.,* a scFv or VHH or a receptor or a ligand fragment that binds an inhibitory molecule, associated with a second polypeptide that provides a positive signal to the cell, *e.g.,* an intracellular signaling domain, such as 41BB, CD27, OX40, CD28, Dap10, CD2, *CD5,* ICAM-1, LFA-1, Lck, TNFR-1, TNFR-II, Fas, CD30, CD40 or combinations thereof) and/or a primary signaling domain *(e.g.,* a CD3 zeta signaling domain). In one embodiment, the agent that inhibits an inhibitory molecule comprises a first polypeptide, *e.g*., a scFv or VHH fragment or a receptor or a ligand fragment that binds an inhibitory molecule, associated with a T cell receptor constant chain described herein (*e.g*., constant chain of TCRa, TCRb1, TCRb2, pre-TCRa, pre-TCRa-Del48, TCR-gamma, or TCR-delta).

In another embodiment, the SAR-expressing cell described herein can further express an accessory module, *e.g.,* an agent which enhances the activity of a SAR-expressing cell. Several examples of accessory modules that comprise of agents that can enhance the activity of a SAR-expressing cell are provided in SEQ ID NO: 11998 to 12019 **(Table 24).** For example, in one embodiment, the agent can be an agent which increases the expression and/or activity of SAR chains *(e.g.,* CD3ζ, CD3δ, CD3ε, CD3γ or combination thereof). In another embodiment, the agent can be an agent *(e.g.,* vFLIP K13, vFLIP MC159, cFLIP-L, cFLIP-p22, HTLV1 Tax, HTLV2 Tax, 41BB or CD28) which provides costimulatory signal to SAR expressing cells. In another embodiment, the agent can be an agent (*e.g*., FKBPx2-K13, FKBPx2-MC159, FKBPx2-cFLIP, FKBPx2-cFLIP-L, FKBPx2-cFLIP-p22, FKBPx2-HTLV1 Tax, FKBPx2-HTLV2 Tax, FKBPx2-41BB or FKBPx2-CD28, Myr-MYD88-CD40-Fv'-Fv *etc.)* which provides costimulatory signal to SAR expressing cells in an inducible manner. In another embodiment, the agent can be a cytokine or a chemokine (*e.g*., CD40L, IL2, IL-7, IL-15, IL12f or IL-21) that promotes the prolilferation or persistence of SAR-expressing cells. In another embodiment, the agent can be a soluble receptor (*e.g*., sHVEM or sHVEM-Alb8-vHH) that promotes the activity of SAR expressing cells and/or synergizes with SAR-expressing cells. In another embodiment, the agent can be an agent that inhibits an inhibitory molecule. Inhibitory molecules, *e.g*., PD1, can, in some embodiments, decrease the ability of a SAR-expressing cell to mount an immune effector response. In another embodiment, the agent can be a scFV targeting PD1 or CTLA4. In one embodiment, the agent comprises a first polypeptide, *e.g.,* of an inhibitory molecule such as PD1, PD-L1, CTLA4, TIM3, CEACAM (*e.g*., CEACAM-1, CEACAM-3 and/or CEACAM-5), LAG3, VISTA, BTLA, TIGIT, LAIR1, CD160, 2B4 or TGFR beta, or a fragment of any of these *(e.g.,* at least a portion of an extracellular domain of any of these), and a second polypeptide which is an intracellular signaling domain described herein (*e.g.*, comprising a costimulatory domain *(e.g.,* 41BB, CD27 or CD28, *e.g.,* as described herein) and/or a primary signaling domain *(e.g.,* a CD3 zeta signaling domain described herein). In one embodiment, the agent comprises a first polypeptide of PD 1 or a fragment thereof *(e.g.,* at least a portion of an extracellular domain of PDl), and a second polypeptide of an intracellular signaling domain described herein *(e.g.,* a CD28 signaling domain described herein and/or a CD3 zeta signaling domain described herein).

In some embodiments, the immune cells are engineered to be resistant to one or more chemotherapy drugs. The chemotherapy drug can be, for example, a purine nucleotide analogue (PNA), thus making the immune cell suitable for cancer treatment combining adoptive immunotherapy and chemotherapy. Exemplary PNAs include, for example, clofarabine, fludarabine, cyclophosphamide, and cytarabine, alone or in combination. PNAs are metabolized by deoxycytidine kinase (dCK) into mono-, di-, and tri-phosphate PNA. Their tri-phosphate forms compete with ATP for DNA synthesis, act as pro-apoptotic agents, and are potent inhibitors of ribonucleotide reductase (RNR), which is involved in trinucleotide production. Provided herein are SAR-T cells comprising a disrupted or inactivated dCK gene. In some embodiments, the dCK knockout cells are made by transfection of T cells using polynucleotides encoding specific TAL-nulcease directed against dCK genes by, for example, electroporation of mRNA. The dCK knockout SAR-T cells can be resistant to PNAs, including for example clorofarabine and/or fludarabine, and maintain T cell cytotoxic activity toward cells expressing the SAR antigen(s).

In one embodiment, the SAR-expressing effector cell described herein can further comprise a second SAR that may include a different antigen binding domain to the same or a different target. In some embodiments, the second SAR may target the same or a different cell type from the first SAR.

In one embodiment, the SAR-expressing effector cell described herein can further comprise a second SAR with the same or a different antigen binding domain, optionally the same or a different target. In some embodiments, the second SAR may target the same or a different cell type from the first SAR. The two SARs may have the same backbone or different backbones. In an exemplary embodiment, the two SARs may have the backbone of 2^{nd} generation CARs. In another exemplary embodiment, one SAR may have the backbone of a SIR while the second SAR may have the backbone of a TFPε. In another exemplary embodiment, one SAR may have the backbone of an Ab-TCR while the second SAR may have the backbone of a SIR. The nucleic acid and amino acid sequences of several exemplary SARs on different backbones are presented in Tables 35 and 36. In one embodiment, the SAR includes an antigen binding domain to a target expressed on the same disease cell type *(e.g.,* cancer) as the disease associated antigen. In one embodiment, the SAR expressing cell comprises a first SAR that targets a first antigen, and a 2^{nd} SAR that targets a second, different, antigen and includes an intracellular signaling domain having no primary signaling domain but a costimulatory signaling domain. While not wishing to be bound by theory, placement of a costimulatory signaling domain, *e.g.*, 4-1BB, CD28, CD27 or OX-40, onto SAR *(e.g.,* a 2^{nd} generation CAR), can modulate the SAR activity to cells where both targets are expressed. In one embodiment, the SAR expressing cell comprises i) a first disease associated antigen SAR *(e.g.,* a SIR) that includes one or more antigen binding domains that bind a target antigen described herein, and one or two TCR constant chains, and ii) a 2^{nd} generation CAR or a TFPε that targets a different target antigen *(e.g.,* an antigen expressed on that same disease associated (*e.g*., cancer) cell type as the first target antigen) and includes an antigen binding domain, a transmembrane domain and a primary signaling domain and a costimulatory domain. In another embodiment, the SAR expressing cell comprises a i) a first SAR *(e.g.,* a SIR) that includes an antigen binding domain that binds a target antigen described herein, and one or two TCR constant chains and ii) a CAR that targets an antigen other than the first target antigen *(e.g.,* an antigen expressed on the same cancer cell type as the first target antigen) and includes an antigen binding domain specific to the antigen, a transmembrane domain and a costimulatory signaling domain. This CAR construct lacks the CD3z domain. In yet another embodiment, the SAR expressing cell comprises i) a first disease associated antigen SAR *(e.g.,* a SIR or an Ab-TCR) that includes one or more antigen binding domains that bind a target antigen described herein, and one or two TCR constant chains, and ii) a CAR that targets a different target antigen *(e.g.,* an antigen expressed on that same disease associated (*e.g*., cancer) cell type as the first target antigen) and includes an antigen binding domain, a transmembrane domain and a primary signaling domain but without a costimulatory domain.

In one embodiment, the CAR comprises the antigen binding domain, a transmembrane domain and an intracellular signaling domain (such as but not limited to one or more intracellular signaling domain from 41BB, CD27, OX40, CD28, Dap10, CD2, *CD5,* ICAM-1, LFA-1, Lck, TNFR-1, TNFR-II, Fas, CD30, CD40 or combinations thereof) and/or a primary signaling domain (such as but not limited to a CD3 zeta signaling domain).

In one embodiment, the SAR-expressing effector cell comprises a SAR described herein and an inhibitory CAR. In one embodiment, the inhibitory CAR comprises an antigen binding domain that binds an antigen found on normal cells but not cancer cells. In one embodiment, the inhibitory CAR comprises an antigen binding domain, a transmembrane domain and an intracellular domain of an inhibitory molecule. For example, the intracellular domain of the inhibitory CAR can be an intracellular domain of any one of PDl, PD-Ll, CTLA-4, TIM-3, CEACAM (*e.g*., CEACAM-1, CEACAM-3 and/or CEACAM-5), LAG-3, VISTA, BTLA, TIGIT, LAIR1, CD160, 2B4 or TGFR beta. An exemplary SAR poypeptide co-expressing an inhibitory CAR is presented in SEQ ID NO: 3220. The inhibitory CAR in this polypeptide expresses a vHH targeting CXCR4 fused to the transmembrane domain and the cytosoloic domain of LAIR1.

In certain embodiments, the antigen binding domain of the first SAR molecule *(e.g.,* a CAR) comprises a scFv and the antigen binding domain of the 2^{nd} SAR *(e.g.,* a SIR, Ab-TCR, or TFP) molecule does not comprise a scFv. For example, the antigen binding domain of the first SAR molecule comprises a scFv and the antigen binding domain of the 2^{nd} SAR molecule comprises a camelid VHH domain.

In one embodiment, the disclosure provides an immune effector cell *(e.g.,* T cell, NK cell) expressing a SAR comprising an antigen binding domain that binds to a tumor antigen as described herein, and a CAR comprising a PD 1 extracellular domain or a fragment thereof. In some embodiments, the cell further comprises an inhibitory molecule comprising an inhKIR cytoplasmic domain; a transmembrane domain, *e.g.,* a KIR transmembrane domain; and an inhibitor cytoplasmic domain, *e.g.,* an ITIM domain, *e.g.,* an inhKIR ITIM domain.

Cells that express a SAR of the disclosure are used in the treatment of disease.

The disclosure thus relates to methods for the prevention and/or treatment of cancer, comprising administering to a subject a cell or cell population comprising a SAR as described herein, said method comprising administering, to a subject in need thereof, a pharmaceutically active amount of a cell and/or of a pharmaceutical composition of the invention.

The disclosur ealso relates to a SAR, a cell or cell population comprising a SAR as described herein for use in therapy. The disclosure also relates to a SAR or a cell comprising a SAR as described herein for use in the treatment of cancer. The disclosure also relates to the use of a SAR or a cell comprising a SAR as described herein in the manufacture of a medicament for the treatment of cancer.

In another aspect, the disclosure relates to a method for stimulating a T cell-mediated immune response to a target cell population or tissue in a subject, the method comprising administering to a subject an effective amount of a cell or cell population that expresses a SAR of the invention, wherein the antigen binding domain is selected to specifically recognize the target cell population or tissue.

In another aspect, the disclosure relates to a method of providing an anti-tumor immunity in a subject, the method comprising administering to the mammal an effective amount of a cell or cell population genetically modified to express a SAR of the invention, thereby providing an anti- tumor immunity in the subject.

The disclosure also provides a method comprising administering a SAR molecule, a cell expressing a SAR molecule or a cell comprising a nucleic acid encoding a SAR molecule to a subject. In one embodiment, the subject has a disorder described herein, *e.g.,* the subject has cancer, infectious disease, allergic disease, degenerative disease or autoimmune disease, which expresses a target antigen described herein. In yet one embodiment, the subject has increased risk of a disorder described herein, *e.g.,* the subject has increased risk of cancer, infectious disease, allergic disease, degenerative disease or autoimmune disease, which expresses a target antigen described herein. In one embodiment, the subject is a human. In another embodiment, the subject is an animal. In yet another embodiment, the subject is a companion animal such as a dog.

In one embodiment, the disclosure provides methods of treating or preventing a disease by providing to the subject in need thereof immune effector cells *(e.g.,* T cells) or stem cells that can give rise to immune effector cells that are engineered to express an targeted X-SIR, wherein X represents a disease associated antigen as described herein, and wherein the disease causing or disease-associated cells express said X antigen. **Table 45** provides a list of different antigens and the exemplary diseases that can be prevented, inhibited or treated using immune effector cells expressing SARs targeting these antigens.

In another embodiment, the disclosure provides methods of treating or preventing cancer by providing to the subject in need thereof immune effector cells *(e.g.,* T cells) that are engineered to express a XSAR (or X-SAR) described herein, wherein the cancer cells express antigen target "X". In one embodiment, X is expressed on both normal cells and cancers cells, but is expressed at lower levels on normal cells. In one embodiment, the method further comprises selecting a SAR that binds X with an affinity that allows the XSAR to bind and kill the cancer cells expressing X but less than 30%, 25%, 20%, 15%, 10%, 5% or less of the normal cells expressing X are killed, *e.g.,* as determined by an assay described herein. For example, the Gluc release cytotoxicity assay described herein can be used to identify XSARs that target, *e.g.,* the cancer cells. In one embodiment, the selected SAR has an antigen binding domain that has a binding affinity KD of about 10⁻⁴ M to 10⁻⁸ M, more commonly about 10⁻⁵ M to 10⁻⁷ M, and typically about 10⁻⁶ M or 10⁻⁷ M, for the target antigen. In one embodiment, the selected antigen binding domain has a binding affinity that is at least two-fold, five-fold, 10-fold, 20-fold, 30-fold, 50-fold, 100-fold or 1,000-fold less than a reference antibody, *e.g*., an antibody described herein and from which the binding domain of the SAR is derived.

In another embodiment, the disclosure provides methods of treating or preventing cancer by providing to the subject in need thereof immune effector cells *(e.g.,* T cells) that are engineered to express a bispecific SAR (or AxB-SAR) described herein, wherein A and B represent the two different antigens targeted by the SAR. In an exemplary embodiment, antigen A is CD19 and antigen B is CD22 and the disease is B cell lymphoma or leukemia. In an exemplary embodiment, antigen A is CD19 and antigen B is CD20 and the disease is B cell lymphoma or leukemia. In an exemplary embodiment, antigen A is CD19 and antigen B is BCMA and the disease is B cell lymphoma or leukemia. In an exemplary embodiment, antigen A is CD19 and antigen B is CD38 and the disease is B cell lymphoma or leukemia. In an exemplary embodiment, antigen A is BCMA and antigen B is CD38 and the disease is a plasma cell disorder or primary effusion lymphoma (PEL). In an exemplary embodiment, antigen A is BCMA and antigen B is CS1/SLAMF7 and the disease is a plasma cell disorder or primary effusion lymphoma (PEL). In an exemplary embodiment, antigen A is CD123 and antigen B is MPL and the disease is acute myeloid leukemia, chronic myeloid leukemia, myeloproliferative disorder or myelofibrosis. In an exemplary embodiment, antigen A is CD123 and antigen B is CD33 and the disease is acute myeloid leukemia, chronic myeloid leukemia, or myeloproliferative disorder.

In an exemplary embodiment, both antigen A and B are expressed on blood cells. In an embodiment, one antigen is expressed preferentially or exclusively on blood lineage cells *(e.g.,* normal B cells or lymphoma cells) while the other antigen is expressed preferentially or exclusively on non-blood cells (*e.g.*, prostate cancer cells). In an embodiment, one antigen is expressed preferentially or exclusively on normal blood lineage cells *(e.g.,* normal B cells) while the other antigen is expressed preferentially or exclusively on tumor cells (*e.g*., prostate cancer cells). In an exemplary embodiment, antigen A is CD19 and antigen B is PSMA and the disease is prostate cancer. In such a construct targeting of CD19 provides proliferative signal to the SAR cells by targeting of CD19 expressed on the normal B cells while targeting of PSMA induces killing of prostate cancer cells. In another exemplary embodiment, antigen A is CD20 and antigen B is PSMA and the disease is prostate cancer. In another exemplary embodiment, antigen A is BCMA and antigen B is PSMA and the disease is prostate cancer. Other examples of antigens that are preferentially expressed on tumor cells *(e.g.,* solid tumor cells) are known in the art, including but not limited to: Her2, Her3, Mesothelin, CEACAM5, EGFR, EGFRviii, IL13Ra2, TAJ, ROR1, CD70, DLL3, Muc16, Muc1, GD2, GD3, CDH6, CDH17, EpCAM, LiCAM, Folate receptor 1, GCC, NY-ESO, AFP, MAGE and WT1.

In another embodiment, the disclosure provides methods of treating or preventing a disease by providing to the subject in need thereof immune effector cells *(e.g.,* T or NK cells) or stem cells that can give rise to immune effector cells that are engineered to express a CD19xC20 bispecific SAR, wherein the disease causing or disease associated cells express CD19 and CD20. In one embodiment, the disease to be treated or prevented is a cancer or immune disease. In one embodiment, the cancer to be treated or prevented is Acute B cell leukemia, chronic B cell leukemia, or B cell lymphoma.

In another embodiment, the disclosure provides methods of treating or preventing a disease by providing to the subject in need thereof immune effector cells *(e.g.,* T or NK cells) or stem cells that can give rise to immune effector cells that are engineered to express a CD19xC22 bispecific SAR, wherein the disease causing or disease associated cells express CD19 and CD22. In one embodiment, the disease to be treated or prevented is a cancer or immune disease. In one embodiment, the cancer to be treated or prevented is Acute B cell leukemia, chronic B cell leukemia, or B cell lymphoma.

In another embodiment, the disclosure provides methods of treating or preventing a disease by providing to the subject in need thereof immune effector cells *(e.g.,* T cells) or stem cells that can give rise to immune effector cells that are engineered to express a CD19xC22xCD20 trispecific SAR, wherein the disease causing or disease associated cells express CD19, CD22 and CD20. In one embodiment, the disease to be treated or prevented is a cancer or immune disease. In one embodiment, the cancer to be treated or prevented is Acute B cell leukemia, chronic B cell leukemia, or B cell lymphoma.

In another embodiment, the disclosure provides methods of treating or preventing a disease by providing to the subject in need thereof immune effector cells *(e.g.,* T cells) or stem cells that can give rise to immune effector cells that are engineered to express a BCMAxCD38 bispecific SAR, wherein the disease causing or disease associated cells express BCMA and CD38. In one embodiment, the disease to be treated or prevented is a cancer or immune disease. In one embodiment, the cancer to be treated or prevented is plasma cell disorder (*e.g*., plasma cell leukemia, myeloma.

In another embodiment, the disclosure provides methods of treating or preventing a disease by providing to the subject in need thereof immune effector cells *(e.g.,* T cells) or stem cells that can give rise to immune effector cells that are engineered to express TCRB1-SAR, wherein the disease causing or disease associated cells express TCRB1 (T cell receptor Beta1 chain). In one embodiment, the disease to be treated or prevented is a cancer or immune disease. In one embodiment, the cancer to be treated or prevented is T cell leukemia or T cell lymphoma. In one embodiment, the immune disorder to be treated or prevented is multiple sclerosis, rheumatoid arthritis, ankylosing spondylitis, inflammatory Bowel Disease, Diabetes Mellitus, Graft vs host disease or autoimmune Thyroiditis.

In another embodiment, the disclosure provides methods of treating or preventing a disease by providing to the subject in need thereof immune effector cells *(e.g.,* T cells) or stem cells that can give rise to immune effector cells that are engineered to express TCRB2-SIR, wherein the disease causing or disease associated cells express TCRB2 (T cell receptor Beta2 SAR). In one embodiment, the disease to be treated or prevented is a cancer or immune disorder. In one embodiment, the cancer to be treated or prevented is T cell leukemia or T cell lymphoma. In one embodiment, the immune disorder to be treated or prevented is multiple sclerosis, rheumatoid arthritis, ankylosing spondylitis, inflammatory Bowel Disease, Diabetes Mellitus, Graft vs host disease or autoimmune thyroiditis.

In another embodiment, the disclosure provides methods of treating or preventing a disease by providing to the subject in need thereof immune effector cells (*e.g.*, T cells) or stem cells that can give rise to immune effector cells that are engineered to express T cell receptor gamma-delta -SIR, wherein the disease-causing or disease associated cells express T cell receptor gamma-delta. In one embodiment, the disease to be treated or prevented is a cancer or immune disorder. In one embodiment, the cancer to be treated or prevented is T cell leukemia or T cell lymphoma. In one embodiment, the immune disorder to be treated or prevented is multiple sclerosis, rheumatoid arthritis, ankylosing spondylitis, inflammatory bowel disease, diabetes mellitus, Graft vs host disease or autoimmune Thyroiditis.

In another embodiment, the disclosure provides methods of treating or preventing a disease by providing to the subject in need thereof immune effector cells (*e.g.*, T cells) or stem cells that can give rise to immune effector cells that are engineered to express a SAR encoding CD4-DC-SIGN. In one embodiment, the disease to be treated or prevented is HIV1/AIDS.

In another embodiment, the disclosure provides methods of treating or preventing an autoimmune-diseases by providing to the subject in need thereof immune effector cells (*e.g.*, T cells) or stem cells that can give rise to immune effector cells that are engineered to express a SAR encoding the autoantigen or a fragment thereof. In one embodiment, the autoimmune disease is diabetes mellitus, rheumatoid arthritis, multiple sclerosis, pemphigus vulgaris, paraneoplastic pemphigous, glomerulonephritis, ankylosing spondylitis, Ulcerative Colitis or Crohn's disease. In one aspect, the disease is pemphigus vulgaris, and the antigen binding domain of the SAR comprises of extracellular domain of Desmoglein 3 (Dsg3)

In another embodiment, the disclosure provides methods of treating or preventing a cancer, infection, autoimmune or allergic diseases by providing to the subject in need thereof immune effector cells (*e.g.*, T cells) or stem cells that can give rise to immune effector cells that are engineered to express a universal SAR encoding CD16 or a deletion- or point-mutant fragment thereof along with an antibody or an antibody fragment that binds to the CD16 domain of the SAR and an antigen expressed on the disease associated cells. In one aspect the disease associated cell is a cancer cell, an infected cell, or a plasma cell or a B cell or a T cell.

In another embodiment, the disclosure provides methods of treating or preventing a cancer, infection, autoimmune or allergic diseases by providing to the subject in need thereof immune effector cells (*e.g.,* T cells) or stem cells that can give rise to immune effector cells that are engineered to express a universal SAR encoding an immunoglobulin binding receptor or a deletion- or point-mutant fragment thereof. The SAR-expressing immune effector cells are administered to the patient along with one or more antibodies or antibody fragments that bind to the immunoglobulin binding domain of the SAR receptor and with one or more antigens expressed on the disease associated cells. In one aspect the disease associated cell is a cancer cell, an infected cell, or a plasma cell or a B cell or a T cell.

In another embodiment, the disclosure provides methods of treating or preventing a cancer, infection, autoimmune or allergic diseases by providing to the subject in need thereof immune effector cells (*e.g.*, T cells) or stem cells that can give rise to immune effector cells that are engineered to express both a universal SAR encoding an immunoglobulin binding receptor or a deletion- or point-mutant fragment thereof joined to a T cell receptor constant chain (*e.g*., constant chain of TCRα) and an antigen binding domain (*e.g.,* a scFv, vHH, vL, vH, or a non-immunoglobulin antigen binding domain) joined to a T cell receptor constant chain (*e.g*., constant chain of TCRβ). The SARs-expressing immune effector cells are administered to the patient along with one or more antibodies or antibody fragments that bind to the immunoglobulin binding domain of the first SAR receptor with one or more antigens expressed on the disease associated cells. In one aspect the disease associated cell is a cancer cell, an infected cell, or a plasma cell or a B cell or a T cell.

In another embodiment, the disclosure provides methods of treating or preventing a cancer, infection, autoimmune or allergic diseases by providing to the subject in need thereof immune effector cells (*e.g.*, T cells) or stem cells that can give rise to immune effector cells that are engineered to express a universal SAR encoding an immunoglobulin receptor or a deletion- or point-mutant fragment thereof thereof along with one or more antibodies or an antibody fragments that bind to the above receptor and one or more antigens expressed on the disease associated cells.

In another embodiment, the disclosure provides methods of treating or preventing a cancer, infection, autoimmune or allergic diseases by providing to the subject in need thereof immune effector cells (*e.g.*, T cells) or stem cells that can give rise to immune effector cells that are engineered to express both a universal SAR encoding CD16 or a deletion- or point-mutant (*e.g*., V158 mutant) fragment thereof joined to a T cell receptor constant chain and a SAR encoding an antigen binding domain (*e.g.*, a scFv, vHH, vL, vH, or a non-immunoglobulin antigen binding domain) joined to a T cell receptor constant chain. The SARs-expressing immune effector cells are administered to the patient along with one or more antibody or an antibody fragments that binds to the CD16 domain of the SAR and one or more antigens expressed on the disease associated cells. In one aspect the disease associated cell is a cancer cell, an infected cell, or a plasma cell or a B cell or a T cell.

In another embodiment, the disclosure provides methods of treating or preventing a cancer, infection, autoimmune or allergic diseases by providing to the subject in need thereof immune effector cells (*e.g.*, T cells) or stem cells that can give rise to immune effector cells that are engineered to express a universal SAR encoding CD16 or a deletion- or point-mutant fragment (*e.g.*, V158 mutant) thereof along with one or more antibody or an antibody fragments that binds to the CD16 domain of the SAR and one or more antigens expressed on the disease associated cells. In one aspect the disease associated cell is a cancer cell, an infected cell, or a plasma cell or a B cell or a T cell.

In another embodiment, the disclosure provides methods of treating or preventing a cancer, infection, autoimmune or allergic diseases by providing to the subject in need thereof immune effector cells (*e.g.*, T cells) that are engineered to express a SAR encoding NKG2D receptor or a deletion- or point-mutant fragment thereof. In one aspect the disease associated cell is a cancer cell, an infected cell, or a plasma cell or a B cell or a T cell.

In another embodiment, the disclosure provides methods of treating or preventing a disease by providing to the subject in need thereof immune effector cells (*e.g.*, T cells) or stem cells that can give rise to immune effector cells that are engineered to express CD19SAR. In one aspect the disease is an immune or allergic disease.

In another embodiment, the disclosure provides methods of treating or preventing a disease by providing to the subject in need thereof immune effector cells (*e.g.*, T cells) that are engineered to express CD20SAR. In one aspect the disease is an immune or allergic disease.

In another embodiment, the disclosure provides methods of treating or preventing a disease by providing to the subject in need thereof immune effector cells (*e.g.*, T cells) that are engineered to express CD22SAR. In one aspect the disease is an immune or allergic disease.

In another embodiment, the disclosure provides methods of treating or preventing a cancer, infection, autoimmune or allergic diseases by providing to the subject in need thereof immune effector cells (*e.g.*, T cells) or stem cells that can give rise to immune effector cells that are engineered to express a FITC-SIR along with a FITC-labelled antibody or an antibody fragment or an antibody fragment or a receptor or a ligand or a non-Immunoglobulin scaffold that binds to an antigen expressed on the disease associated cells. In one aspect the disease associated cell is a cancer cell, an infected cell, or a plasma cell or a B cell or a T cell.

In another embodiment, the disclosure provides methods of treating or preventing a cancer, infection, autoimmune or allergic diseases by providing to the subject in need thereof immune effector cells (*e.g.*, T cells) or stem cells that can give rise to immune effector cells that are engineered to express an avidin-SAR along with a Biotin-labelled antibody or an antibody fragment or an antibody fragment or a receptor or a ligand or a non-Immunoglobulin scaffold that binds to an antigen expressed on the disease associated cells. In one aspect the disease associated cell is a cancer cell, an infected cell, or a plasma cell.

In another embodiment, the disclosure provides methods of treating or preventing a cancer, infection, autoimmune or allergic diseases by providing to the subject in need thereof immune effector cells (*e.g.*, T cells) or stem cells that can give rise to immune effector cells that are engineered to express an Streptag-SAR along with a Streptag-containing antibody or an antibody fragment or a receptor or a ligand or a non-Immunoglobulin scaffold that binds to an antigen expressed on the disease associated cells. In one aspect the disease associated cell is a cancer cell, an infected cell, or a plasma cell.

In another embodiment, the disclosure provides methods of treating or preventing a disease by providing to the subject in need thereof immune effector cells (*e.g.*, T cells) that are engineered to express IgE-SAR whose antigen binding domain comprises of an antibody or antibody fragment that binds to IgE. In one aspect the disease is an immune or allergic disease.

In another embodiment, the disclosure relates to treatment of a subject *in vivo* using a PD1SAR (*i.e.*, a SAR containing the extracellular domain of PD1 as its antigen binding domain) such that growth of cancerous tumors is inhibited. A PD1SAR may be used alone to inhibit the growth of cancerous tumors.

In another aspect of the disclosure, there is provided a pharmaceutical composition comprising a SAR or an isolated cell or cell population comprising a SAR according to the present disclosure and optionally a pharmaceutically acceptable carrier.

The genetically modified cells or pharmaceutical composition of the present disclosure can be administered by any convenient route, including parenteral administration. Parenteral administration includes, for example, intravenous, intramuscular, intraarterial, intraperitoneal, intranasal, rectal, intravesical, intradermal, topical or subcutaneous administration. Compositions can take the form of one or more dosage units.

The composition of the disclosure can be in the form of a liquid, *e.g.,* , a solution, emulsion or suspension. The liquid can be useful for delivery by injection, infusion (*e.g.*, IV infusion) or sub-cutaneously. The liquid compositions of the invention, whether they are solutions, suspensions or other like form, can also include one or more of the following: sterile diluents such as water, saline solution, typically physiological saline, Ringer's solution, isotonic sodium chloride, fixed oils such as synthetic mono or diglycerides, polyethylene glycols, glycerin, or other solvents; antibacterial agents such as benzyl alcohol or methyl paraben; and agents for the adjustment of tonicity such as sodium chloride or dextrose. A composition can be enclosed in an ampoule, a disposable syringe or a multiple-dose vial made of glass, plastic or other material.

The amount of the pharmaceutical composition of the present disclosure that is effective/active in the treatment of a particular disorder or condition will depend on the nature of the disorder or condition and can be determined by standard clinical techniques. In addition, *in vitro* or *in vivo* assays can optionally be employed to help identify optimal dosage ranges. The precise dose to be employed in the compositions will also depend on the route of administration, and the seriousness of the disease or disorder, and should be decided according to the judgment of the practitioner and each patient's circumstances.

The compositions of the disclosure comprise an effective amount of a binding molecule of the present disclosure such that a suitable dosage will be obtained. The correct dosage of the compounds will vary according to the particular formulation, the mode of application, and its particular site, host and the disease being treated. Other factors like age, body weight, sex, diet, time of administration, rate of excretion, condition of the host, drug combinations, reaction sensitivities and severity of the disease shall be taken into account. Administration can be carried out continuously or periodically within the maximum tolerated dose.

Typically, this amount is at least about 0.01 % of a binding molecule of the present disclosure by weight of the composition.

Preferred compositions of the present disclosure are prepared so that a parenteral dosage unit contains from about 0.01 % to about 2% by weight of the binding molecule of the disclosure.

For intravenous administration, the composition can comprise from about typically about 0.1 mg/kg to about 250 mg/kg of the animal's body weight, preferably, between about 0.1 mg/kg and about 20 mg/kg of the animal's body weight, and more typically about 1 mg/kg to about 10 mg/kg of the animal's body weight.

The present compositions can take the form of suitable carriers, such aerosols, sprays, suspensions, or any other form suitable for use. Other examples of suitable pharmaceutical carriers are described in "Remington's Pharmaceutical Sciences" by E. W. Martin.

The pharmaceutical compositions can be prepared using methodology well known in the pharmaceutical art. For example, a composition intended to be administered by injection can be prepared by combining a binding molecule of the present disclosure with water so as to form a solution. A surfactant can be added to facilitate the formation of a homogeneous solution or suspension.

The pharmaceutical composition of the disclosure can be co-administered with other therapeutics, for example anti-cancer agents.

In one embodiment, the cancer is selected from a haematological cancer or malignancy or a solid tumor. Hematologic cancers are cancers of the blood or bone marrow. Solid tumors are abnormal masses of tissue that usually do not contain cysts or liquid areas.

In one embodiment, the cancer is metastatic.

Cancers that may treated by methods, uses and compositions described herein include, but are not limited to, cancer cells from the bladder, blood, bone, bone marrow, brain, breast, colon, esophagus, gastrointestine, gum, head, kidney, liver, lung, nasopharynx, neck, ovary, prostate, skin, stomach, testis, tongue, or uterus. In addition, the cancer may specifically be of the following histological type, though it is not limited to these: neoplasm, malignant; carcinoma; carcinoma, undifferentiated; giant and spindle cell carcinoma; small cell carcinoma; papillary carcinoma; squamous cell carcinoma; lymphoepithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumor, malignant; branchiolo-alveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary and follicular adenocarcinoma; nonencapsulating sclerosing carcinoma; adrenal cortical carcinoma; endometroid carcinoma; skin appendage carcinoma; apocrine adenocarcinoma; sebaceous adenocarcinoma; ceruminous adenocarcinoma; mucoepidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; paget's disease, mammary; acinar cell carcinoma; adenosquamous carcinoma; adenocarcinoma w/squamous metaplasia; thymoma, malignant; ovarian stromal tumor, malignant; thecoma, malignant; granulosa cell tumor, malignant; and roblastoma, malignant; Sertoli cell carcinoma; leydig cell tumor, malignant; lipid cell tumor, malignant; paraganglioma, malignant; extra-mammary paraganglioma, malignant; pheochromocytoma;glomangiosarcoma; malignant melanoma; amelanotic melanoma; superficial spreading melanoma; malig melanoma in giant pigmented nevus; epithelioid cell melanoma; blue nevus, malignant; sarcoma; fibrosarcoma; fibrous histiocytoma, malignant; myxosarcoma; liposarcoma; leiomyosarcoma; rhabdomyosarcoma; embryonal rhabdomyosarcoma; alveolar rhabdomyosarcoma; stromal sarcoma; mixed tumor, malignant; mullerian mixed tumor; nephroblastoma; hepatoblastoma; carcinosarcoma; mesenchymoma, malignant; brenner tumor, malignant; phyllodes tumor, malignant; synovial sarcoma; mesothelioma, malignant; dysgerminoma; embryonal carcinoma; teratoma, malignant; struma ovarii, malignant; choriocarcinoma; mesonephroma, malignant; hemangiosarcoma; hemangioendothelioma, malignant; kaposi's sarcoma; hemangiopericytoma, malignant; lymphangiosarcoma; osteosarcoma; juxtacortical osteosarcoma; chondrosarcoma; chondroblastoma, malignant; mesenchymal chondrosarcoma; giant cell tumor of bone; ewing's sarcoma; odontogenic tumor, malignant; ameloblastic odontosarcoma; ameloblastoma, malignant; ameloblastic fibrosarcoma; pinealoma, malignant; chordoma; glioma, malignant; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumor; meningioma, malignant; neurofibrosarcoma; neurilemmoma, malignant; granular cell tumor, malignant; malignant lymphoma; Hodgkin's disease; Hodgkin's lymphoma; paragranuloma; malignant lymphoma, small lymphocytic; malignant lymphoma, large cell, diffuse; malignant lymphoma, follicular; mycosis fungoides; other specified non-Hodgkin's lymphomas; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; myeloid leukemia; basophilic leukemia; eosinophilic leukemia; monocytic leukemia; mast cell leukemia; megakaryoblastic leukemia; myeloid sarcoma; and hairy cell leukemia.

In one embodiment, the SAR is used to target BCMA and CD38 and is used to treat plasma cell and autoimmune disorders, such as multiple myeloma, plasma cell leukemia, primary effusion lymphoma and systemic lupus erythematosus (SLE). Such a SAR includes antigen binding domains specific to BCMA and CD38 as described herein. Exemplary such SARs are provided in **Table 34** (SEQ ID NO:17638-177810, 17884-17956).

In one embodiment, the SAR is used to target BCMA and CD22 and is used to treat lymphoid disorders and plasma cell disorders, such as lymphoma, acute lymphocytic leukemia, multiple myeloma, plasma cell leukemia primary effusion lymphoma and systemic lupus erythematosus (SLE). Such a SAR includes antigen binding domains specific to BCMA and CD22 as described herein. Exemplary such SARs are provided in **Table 34.**

In one embodiment, the SAR is used to target BCMA and CD19 and is used to treat lymphoid disorders and plasma cell disorders, such as lymphoma, acute lymphocytic leukemia, multiple myeloma, plasma cell leukemia primary effusion lymphoma and systemic lupus erythematosus (SLE). Such a SAR includes antigen binding domains specific to BCMA and CD19 as described herein. Exemplary such SARs are provided in **Table 34** (SEQ ID NO: 17665-17737).

In one embodiment, the SAR is used to target BCMA, CD38 and CD22 and is used to treat lymphoid, plasma cell and autoimmune disorders, such as multiple myeloma, plasma cell leukemia, primary effusion lymphoma, diffuse large B cell lymphoma, B-ALL, chronic lymphocytic leukemia and systemic lupus erythematosus (SLE). Such a SAR includes antigen binding domains specific to BCMA, CD38 and CD22 as described herein.

In one embodiment, the SAR is used to target BCMA, CD38 and CD19 and is used to treat lymphoid, plasma cell and autoimmune disorders, such as multiple myeloma, plasma cell leukemia, primary effusion lymphoma, diffuse large B cell lymphoma, B-ALL, chronic lymphocytic leukemia and systemic lupus erythematosus (SLE). Such a SAR includes antigen binding domains specific to BCMA, CD38 and CD19 as described herein.

In one embodiment, the SAR is used to target BCMA, CD38, CD22 and CD19 and is used to treat lymphoid, plasma cell and autoimmune disorders, such as multiple myeloma, plasma cell leukemia, primary effusion lymphoma, diffuse large B cell lymphoma, B-ALL, chronic lymphocytic leukemia and systemic lupus erythematosus (SLE). Such a SAR includes antigen binding domains specific to BCMA, CD38, CD22 and/or CD19 as described herein. Exemplary such SARs are provided in **Table 32.**

In one embodiment, the SAR is used to target BCMA, CD38, CD22, CD20 and CD19 and is used to treat lymphoid, plasma cell and autoimmune disorders, such as multiple myeloma, plasma cell leukemia, primary effusion lymphoma, diffuse large B cell lymphoma, B-ALL, chronic lymphocytic leukemia and systemic lupus erythematosus (SLE). Such a SAR includes antigen binding domains specific to BCMA, CD38, CD22, CD20 and/or CD19 as described herein. Exemplary such SARs are provided in Table 32.

In one embodiment, the SAR is used to target CD19 and CD22 and is used to treat lymphoid and autoimmune disorders, such as lymphoma, acute lymphocytic leukemia, chronic lymphocytic leukemia and systemic lupus erythematosus (SLE). Such a SAR includes antigen binding domains specific to CD19 and CD22 as described herein.

In one embodiment, the SAR is used to target CD19 and CD20 and is used to treat lymphoid and autoimmune disorders, such as lymphoma, acute lymphocytic leukemia, chronic lymphocytic leukemia and systemic lupus erythematosus (SLE). Such a SAR includes antigen binding domains specific to CD19 and CD22 as described herein.

In one embodiment, the SAR is used to target CD19, CD22 and CD20 and is used to treat lymphoid and autoimmune disorders, such as lymphoma, acute lymphocytic leukemia, chronic lymphocytic leukemia and systemic lupus erythematosus (SLE). Such a SAR includes antigen binding domains specific to CD19, CD20 and CD22 as described herein.

In one embodiment, the SAR is used to target CD19, CD22, BCMA and/or CD20 and is used to treat plasma cell, lymphoid and autoimmune disorders, such as multiple myeloma, plasma cell leukemia, primary effusion lymphoma, diffuse large B cell lymphoma, acute lymphocytic leukemia, chronic lymphocytic leukemia and systemic lupus erythematosus (SLE). Such a SAR includes antigen binding domains specific to CD19, CD22, BCMA and/or CD20 as described herein. Exemplary such SARs are provided in **Table 31.**

In one embodiment, the SAR is used to target CD123 and CD33 and is used to treat myeloid and myeloproliferative disorders such as acute myeloid leukemia and myelodysplasia. Such a SAR includes antigen binding domains specific to CD123 and CD33 as described herein. Exemplary such SARs are provided in SEQ ID NO (PRT): 22479-22484.

In one embodiment, the SAR is used to target CD123 and FLT3 and is used to treat lymphoid, myeloid and myeloproliferative disorders such as acute myeloid leukemia, acute lymphoid leukemia and myelodysplasia. Such a SAR includes antigen binding domains specific to CD123 and FLT3 as described herein. Exemplary such SARs are provided in SEQ ID NO (PRT): 22485-22487.

In one embodiment, the SAR is used to target CD123 and MPL (TPO-R) and is used to treat lymphoid, myeloid and myeloproliferative disorders such as acute myeloid leukemia, acute lymphoid leukemia and myelodysplasia. Such a SAR includes antigen binding domains specific to CD123 and MPL as described herein. Exemplary such SARs are provided in SEQ ID NO (PRT): 22488-22490.

In one embodiment, the SAR is used to target PSMA and treat prostate cancer. Such a SAR includes an antigen binding domain specific to PSMA as described herein.

In one embodiment, the SAR is used to target PSMA and CD19 and treat prostate cancer. Such a SAR includes antigen binding domains specific to PSMA and CD19 as described herein. The CD19 specific antigen binding domain is used to primarily stimulate activation, proliferation and expansion of the SAR.

In one embodiment, the SAR is used to target PSMA and CD22 and treat prostate cancer. Such a SAR includes antigen binding domains specific to PSMA and CD22 as described herein. The CD22 specific antigen binding domain is used to primarily stimulate activation, proliferation and expansion of the SAR.

In one embodiment, the SAR is used to target PSMA and CD20 and treat prostate cancer. Such a SAR includes antigen binding domains specific to PSMA and CD20 as described herein. The CD20 specific antigen binding domain is used to primarily stimulate activation, proliferation and expansion of the SAR.

In one embodiment, the SAR is used to target PSMA and BCMA and treat prostate cancer. Such a SAR includes antigen binding domains specific to PSMA and BCMA as described herein. The BCMA specific antigen binding domain is used to primarily stimulate activation, proliferation and expansion of the SAR.

In one embodiment, the SAR is used to target Her2, IL13Ra2 and/or CD19 and treat breast cancer, gastric cancer or brain cancer. Such a SAR includes antigen binding domains specific to Her2, IL13Ra2 and/or CD19 as described herein. Exemplary such SARs are provided in SEQ ID NO (PRT):22465-22473.

In one embodiment, the SAR is used to target Her2, IL13Ra2, low conductance chloride channel and/or CD19 and treat breast cancer, gastric cancer or brain cancer. Such a SAR includes antigen binding domains specific to Her2, IL13Ra2, low conductance chloride channel and/or CD19 as described herein. Exemplary such SARs are provided in SEQ ID NO (PRT):22474-22478, 22491-22495.

In one embodiment, the disclosure provides SARs targeting Her2, Her3, CD22, EGFR and/or CD19. Exemplary such SARs are provided in **Table 32.**

In one embodiment, the disclosure provides SARs targeting Her2, IL13Ra2, CD22, and/or CD19. Exemplary such SARs are provided in **Table 32.**

In one embodiment, the disclosure provides UNICON SAR targeting different antigens and comprising an adaptor binding domain. Exemplary such SARs are provided in **Table 30.**

Pharmaceutical compositions of the disclosure may be administered in a manner appropriate to the disease to be treated (or prevented). The quantity and frequency of administration will be determined by such factors as the condition of the patient, and the type and severity of the patient's disease. When "an immunologically effective amount," "an anti-tumor effective amount," "a tumor-inhibiting effective amount," or "therapeutic amount" or "anti-infective" is indicated, the amount of the compositions of the disclosure to be administered can be determined by a physician with consideration of individual differences in age, weight, tumor size, extent of infection or metastasis, and condition of the patient (subject) as the case may be. It can generally be stated that a pharmaceutical composition comprising the immune effector cells (*e.g.,* T cells, NK cells) described herein may be administered at a dosage of 10⁴ to 10⁹ cells/kg body weight, in some instances 10⁵ to 10⁶ cells/kg body weight, including all integer values within those ranges. T or NK cell compositions may also be administered multiple times at these dosages. The cells can be administered by using infusion techniques that are commonly known in immunotherapy (see, *e.g.,* Rosenberg et al., New Eng. J. of Med. 319:1676, 1988) and cellular therapies.

The dosage ranges for the agent, including SAR adaptors and antibodies, depend upon the potency, and encompass amounts large enough to produce the desired effect *e.g*., slowing of tumor growth or a reduction in tumor size. The dosage should not be so large as to cause unacceptable adverse side effects. Generally, the dosage will vary with the age, condition, and sex of the patient and can be determined by one of skill in the art. The dosage can also be adjusted by the individual physician in the event of any complication. In some embodiments, the dosage ranges from 0.001 mg/kg body weight to 0.5 mg/kg body weight. In some embodiments, the dose range is from 5 µg/kg body weight to 100 µg/kg body weight. Alternatively, the dose range can be titrated to maintain serum levels between 1 µg/mL and 1000 µg/mL. For systemic administration, subjects can be administered a therapeutic amount, such as, *e.g.,* 0.1 mg/kg, 0.5 mg/kg, 1.0 mg/kg, 2.0 mg/kg, 2.5 mg/kg, 5 mg/kg, 10 mg/kg, 15 mg/kg, 20 mg/kg, 25 mg/kg, 30 mg/kg, 40 mg/kg, 50 mg/kg, or more.

Administration of the doses recited above can be repeated. In some embodiments, the doses are given once a day, or multiple times a day, for example but not limited to three times a day. In some embodiments, the doses recited above are administered daily for several weeks or months. The duration of treatment depends upon the subject's clinical progress and responsiveness to therapy.

In some embodiments, the dose can be from about 2 mg/kg to about 15 mg/kg. In some embodiments, the dose can be about 2 mg/kg. In some embodiments, the dose can be about 4 mg/kg. In some embodiments, the dose can be about 5 mg/kg. In some embodiments, the dose can be about 6 mg/kg. In some embodiments, the dose can be about 8 mg/kg. In some embodiments, the dose can be about 10 mg/kg. In some embodiments, the dose can be about 15 mg/kg. In some embodiments, the dose can be from about 100 mg/m2 to about 700 mg/m2. In some embodiments, the dose can be about 250 mg/m2. In some embodiments, the dose can be about 375 mg/m2. In some embodiments, the dose can be about 400 mg/m2. In some embodiments, the dose can be about 500 mg/m2.

In some embodiments, the dose can be administered intravenously. In some embodiments, the intravenous administration can be an infusion occurring over a period of from about 10 minute to about 3 hours. In some embodiments, the intravenous administration can be an infusion occurring over a period of from about 30 minutes to about 90 minutes.

In some embodiments the dose can be administered about weekly. In some embodiments, the dose can be administered weekly. In some embodiments, the dose can be administered weekly for from about 12 weeks to about 18 weeks. In some embodiments the dose can be administered about every 2 weeks. In some embodiments the dose can be administered about every 3 weeks. In some embodiments, the dose can be from about 2 mg/kg to about 15 mg/kg administered about every 2 weeks. In some embodiments, the dose can be from about 200 mg/m² to about 400 mg/m² administered intravenously about every 2 weeks. In some embodiments, a total of from about 2 to about 10 doses are administered. In some embodiments, a total of 4-8 doses are administered. In some embodiments, a total of 5 doses are administered. In some embodiments, a total of 6 doses are administered. In some embodiments, the administration occurs for a total of from about 4 weeks to about 12 weeks. In some embodiments, the initial dose can be from about 1.5 to about 2.5-fold greater than subsequent doses.

A therapeutically effective amount is an amount of an agent that is sufficient to produce a statistically significant, measurable change in tumor size, tumor growth *etc.* (efficacy measurements are described below herein). Such effective amounts can be gauged in clinical trials as well as animal studies.

An agent can be administered intravenously by injection or by gradual infusion over time. Given an appropriate formulation for a given route, for example, agents useful in the methods and compositions described herein can be administered intravenously, intranasally, by inhalation, intraperitoneally, intramuscularly, subcutaneously, intracavity, and can be delivered by peristaltic means, if desired, or by other means known by those skilled in the art. It is preferred that the compounds used herein are administered orally, intravenously or intramuscularly to a patient having cancer. Local administration directly to a tumor mass is also specifically contemplated.

In embodiments where the subject is administered a UNISAR or UNICON-SAR, the UNISAR/UNICON-SAR cell and the SAR adaptor can be administered together or separately. In embodiments where the subject is separately administered UNISAR/UNICON-SAR cell and the SAR adaptor each of the compositions can be administered, separately, according to any of the dosages and administration routes/routines described herein.

Precise amounts of active ingredient required to be administered depend on the judgment of the practitioner and are particular to each individual. However, suitable dosage ranges for systemic application are disclosed herein and depend on the route of administration. Suitable regimes for administration are also variable but are typified by an initial administration followed by repeated doses at one or more hour intervals by a subsequent injection or other administration. Alternatively, continuous intravenous infusion sufficient to maintain concentrations in the blood in the ranges specified for *in vivo* therapies are contemplated.

In one embodiment, a SAR or cell comprising a SAR of the disclosure is used in combination with an existing therapy or therapeutic agent, for example an anti-cancer therapy. Thus, in another aspect, the disclosure also relates to a combination therapy comprising administration of a SAR-T or pharmaceutical composition of the disclosure and an anti-cancer therapy. The anti-cancer therapy may include a therapeutic agent or radiation therapy and includes gene therapy, viral therapy, RNA therapy bone marrow transplantation, nanotherapy, targeted anti-cancer therapies or oncolytic drugs. Examples of other therapeutic agents include other checkpoint inhibitors, antineoplastic agents, immunogenic agents, attenuated cancerous cells, tumor antigens, antigen presenting cells such as dendritic cells pulsed with tumor-derived antigen or nucleic acids, immune stimulating cytokines (*e.g*., IL-2, IFNa2, GM-CSF), targeted small molecules and biological molecules (such as components of signal transduction pathways, *e.g*., modulators of tyrosine kinases and inhibitors of receptor tyrosine kinases, and agents that bind to tumor-specific antigens, including EGFR antagonists), an anti-inflammatory agent, a cytotoxic agent, a radiotoxic agent, or an immunosuppressive agent and cells transfected with a gene encoding an immune stimulating cytokine (*e.g*., GM-CSF), chemotherapy. In one embodiment, the SAR-T or pharmaceutical composition of the disclosure is used in combination with surgery. The SAR-T or pharmaceutical composition of the disclosure may be administered at the same time or at a different time as the other therapy, *e.g*., simultaneously, separately or sequentially.

In one embodiment, the method includes administering a cell expressing the SAR molecule, as described herein, in combination with an agent which enhances the activity of a SAR-expressing cell, wherein the agent is a cytokine, *e.g.,* IL-2, IL-7, IL-15, IL-21, or a combination thereof.

In other embodiments, the cells expressing a SIR molecule are administered in combination with an agent that ameliorates one or more side effects associated with administration of a cell expressing a SIR molecule. Side effects associated with the SIR-expressing cell can be chosen from cytokine release syndrome (CRS), hemophagocytic lymphohistiocytosis (HLH) or neurological complications. Examples of such agents include steroids (*e.g.* prednisone, dexamethasone), IL6R antagonists (*e.g.,* tocilizumab), src kinase inhibitors (*e.g.*, dasatinib), a kinase inhibitor (*e.g.*, Ibrutinib), calcineurin inhibitors (*e.g.*, tacrolimus or cyclosporine A) or chemotherapy drugs (*e.g*., cyclophosphamide, methotrexate or vincristine).

In embodiments of any of the aforeseaid methods or uses, the cells expressing the SIR molecule are administered in combination with an agent that treats the disease associated with expression of the target antigen, *e.g*., any of the second or third therapies disclosed herein. Additional exemplary combinations include one or more of the following.

In another embodiment, the cell expressing the SIR molecule, *e.g.,* as described herein, can be administered in combination with another agent that increases the expression of the target antigen against which the SAR is directed.

In another embodiment, the SIR-expressing immune effector cell of the disclosure, *e.g*., T cell, NK cell or hematopoietic stem cell, is administered to a subject along with an agent that disrupt the immunosuppressive pathways in the tumor microenvironment in certain cancers.

In one embodiment, the SAR-expressing immune effector cell of the disclosure, *e.g*., T cell, NK cell or hematopoietic stem cell, is administered to a subject that has received a previous stem cell transplantation, *e.g*., autologous stem cell transplantation or an allogenic stem cell transplanation.

In one embodiment, the SIR-expressing immune effector cell of the disclosure, *e.g.,* T cell, NK cells or hematopoietic stem cells, is administered to a subject that has received a previous dose of chemotherapy, such as melphalan, fludarabine, etoposide or cylophosphamide.

In one embodiment, the SAR-expressing immune effector cell of the disclosure, *e.g*., T cell, NK cells or hematopoietic stem cells, is administered to a subject that has received a previous dose of a drug that enhances the expression of the target antigen of SAR, such as compounds 27, 40 and 49 (Du, J et al, Blood, Prepublished online October 12, 2016) Arsenic trixoxide or ATRA. Similarly, BCMA-targeted SAR can be used in combination with γ-secretase inhibitors.

In one embodiment, the cell expressing a SAR molecule, *e.g.,* a SAR molecule described herein, is administered in combination with an agent that increases the efficacy of a cell expressing a SIR molecule, *e.g.,* an agent described herein. In one embodiment, the cells expressing a SAR molecule, *e.g.,* a SAR molecule described herein, are administered in combination with a low, immune enhancing dose of an mTOR inhibitor.

In an embodiment, the SAR expressing cells are used in combination with an agent that control the activity of the SAR-expressing cells. In an embodiment, the agent is a SAR adaptor.

In some embodiments, provided are methods for depleting SAR-expressing engineered immune cells from a subject adminstered with said cells. Depletion can be by inhibition or elimination.

In one aspect, a method for depleting engineered immune cells expressing a SAR comprising an epitope specific for a monoclonal antibody comprises contacting said engineered immune cell with a monoclonal antibody specific for the epitope.

In some embodiments, a method for depleting from a subject administered with engineered immune cells expressing a SAR comprising an epitope specific for a monoclonal antibody comprises administering to the subject a monoclonal antibody specific for the epitope. In these embodiments, administration of the monoclonal antibody specific for the epitope present in the extracellular domain of the SAR to the subject eliminates or inhibits the activity of engineered SAR-expressing immune cells from the subject. In one aspect, depletion of engineered CAR expressing immune cells allows for recovery of an endogenous population of SAR-expressing cells.

In one aspect, the disclosure relates to a method for promoting recovery of endogenous antigen-expressing cells in a subject administered with engineered immune cells expressing at cell surface a SAR that target one or more antigens expressed on endogenously expressing cells comprising an epitope specific for an antigen binding agent (*e.g.*, monoclonal antibody, vHH domain, antibody fragment, non-immunoglobulin antigen binding scaffold *etc.*), the method comprising administering a an antigen binding agent (*e.g.*, monoclonal antibody, vHH domain, antibody fragment, non-immunoglobulin antigen binding scaffold *etc.*) specific for the epitope to the subject. The term "endogenous antigen expressing cells" as used herein refer to normal healthy cells that express the antigen targeted by the SAR. In an exemplary embodiment, endogenous antigen expressing cells refer to normal bone marrow cells that express CD33 in a subject administered CD33-specific SAR-T cells. Other examples of endogenous antigen expressing cells include cells expressing CD34, CD123, CD70, CD19 *etc.*

In some embodiments, endogenous antigen-expressing cells are endogenous antigen-expressing bone marrow cells. In one aspect, the term "recovery" refers to increasing the number of endogenous antigen-expressing cells. The number of endogenous antigen-expressing cells may increase due to increase in proliferation of endogenous antigen-expressing cells and/or due to reduction in elimination of endogenous antigen-expressing cells by SAR expressing engineered immune cells. In some embodiments, administration of the antigen binding agent (*e.g*., monoclonal antibody) to the subject depletes the antigen expressing engineered immune cells and increases the number of endogenous antigen-expressing cells, e.g, endogenous antigen-expressing bone marrow progenitor cells, in the subject. In one embodiment, administration of the antigen binding agent (*e.g*., monoclonal antibody) to the subject increases the number of endogenous antigen-expressing cells by at least about 10%, 15%, 20%, 25%, 30%, 35%, 40%, 45%, 50%, 55%, 60%, 65%, 70%, 75%, 80%, 85%, 90%, or 95%, compared to the number of endogenous antigen-expressing cells prior to administration of the antigen binding agent.

In one aspect, provided is a method for treating an Antigen X-mediated condition in a subject, the method comprising: (a) administering to the subject engineered immune cells expressing at cell surface X-specific SARs comprising one or more epitopes specific for one or more monoclonal antibodies; and (b) subsequently depleting the engineered immune cells from the subject by administering one or more monoclonal antibodies specific for the epitope to the subject. In an embodiment, the X-specific SAR may be a bispecific or a multispecific SAR which targets antigens in addition to antigen X.

In some embodiments, the antigen binding agent used in the method for depleting SAR-expressing engineered immune cells are selected from alemtuzumab, ibritumomab tiuxetan, muromonab-CD3, tositumomab, abciximab, basiliximab, brentuximab vedotin, cetuximab, infliximab, rituximab, bevacizumab, certolizumab pegol, daclizumab, eculizumab, efalizumab, gemtuzumab, natalizumab, omalizumab, palivizumab, ranibizumab, tocilizumab, trastuzumab, vedolizumab, adalimumab, belimumab, canakinumab, denosumab, golimumab, ipilimumab, ofatumumab, panitumumab, QBEND-10, ustekinumab, and combinations thereof.

In some embodiments, said epitope specific for a monoclonal antibody (mAb-specific epitope) is a CD20 epitope or mimotope (SEQ ID NO: 22418) and the antigen binding agent specific for the epitope is rituximab. In some embodiments, said epitope specific for a monoclonal antibody (mAb-specific epitope) is a CD20 epitope (SEQ ID NO: 22412) and the antigen binding agent specific for the epitope is Obinutuzumab. In some embodiments, said epitope specific for a monoclonal antibody (mAb-specific epitope) is a Her2 epitope, *e.g.,* SEQ ID NO: 22432, and the antigen binding agent specific for the epitope is Herceptin (Trastuzumab), Kadcyla (Ado-trastuzumab), or ENHERTU (fam-trastuzumab deruxtecan-nxki). In some embodiments, said epitope specific for an antigen binding agent (*e.g.,* mAb-specific epitope) is an EGFR epitope, *e.g.,* SEQ ID NO: 22421-22425, and the antigen binding agent specific for the epitope is cetuximab. In some embodiments, said epitope specific for a monoclonal antibody (mAb-specific epitope) is an EGFR epitope, *e.g.,* SEQ ID NO: 22426-22431, and the antigen binding agent (*e.g*., mAb) specific for the epitope is Panitumumab.

In some embodiments, the step of administering a monoclonal antibody to the subject comprises infusing the subject with the monoclonal antibody. In some embodiments, the amount of epitope-specific mAb administered to the subject is sufficient to eliminate at least 20%, 30%, 40%, 50%, 60%, 70%, 80% or 90% of the CAR-expressing immune cell in the subject.

In some embodiments, the step of administering a monoclonal antibody to the subject comprises infusing the subject with 375 mg/m² of rituximab, once or several times weekly. In some embodiments, the step of administering a monoclonal antibody to the subject comprises infusing the subject with 6 mg/kg of Panitumumab by intravenous infusion over 60 minutes, once or several times weekly or every 14 days. In some embodiments, the step of administering a monoclonal antibody to the subject comprises infusing the subject with 8 mg/kg of Herceptin by intravenous infusion over 60 minutes, once or several times weekly or every 14 days. In some embodiments, the step of administering a monoclonal antibody to the subject comprises infusing the subject with 400 mg/m² of cetuximab, once or several times weekly. In some embodiments, the step of administering a monoclonal antibody to the subject comprises infusing the subject with 3.6 mg/kg of Kadcyla, once or several times every 3 weeks. In some embodiments, the step of administering a monoclonal antibody to the subject comprises infusing the subject with 5.4 mg/kg of ENHERTU, once or several times every 3 weeks.

In some embodiments, when immune cells expressing a SAR comprising an mAb-specific epitope (SAR-expressing immune cells) are depleted in a CDC assay using epitope-specific mAb, the amount of viable SAR-expressing immune cells decreases, *e.g*., by at least 10%, 20%, 30%, 40%, 50%, 60%, 70%, 80% or 90%.

In some embodiments, a cytotoxic drug is coupled to the epitope-specific antigen binding agent (*e.g*., mAbs) which are used to deplete SAR-expressing immune cells. By combining targeting capabilities of monoclonal antibodies with the cell-killing ability of cytotoxic drugs, antibody-drug conjugate (ADC) allows a sensitive discrimination between healthy and diseased tissue when compared to the use of the drug alone.

In some embodiments, the epitope-specific mAb to be infused is conjugated beforehand with a molecule able to promote complement dependent cytotoxicity (CDC).

Therefore, the complement system helps or complements the ability of antibodies to clear pathogens from the organism. When stimulated by one of several, is triggered an activation cascade as a massive amplification of the response and activation of the cell-killing membrane attack complex. Different molecule may be used to conjugate the mAb, such as glycans [Courtois, A, Gac-Breton, S., Berthou, C, Guezennec, I, Bordron, A. and Boisset, C. (2012), Complement dependent cytotoxicity activity of therapeutic antibody fragments is acquired by immunogenic glycan coupling, Electronic Journal of Biotechnology ISSN: 0717-3458; http://www.ejbiotechnology.info DOI: l0.2225/voll5-issue5).

The disclosure also provides kits for use in the instant methods. Kits of the disclosure include one or more containers comprising a polynucleotide encoding a SAR, or an engineered immune cell comprising a polynucleotide encoding a SAR as described herein, and instructions for use in accordance with any of the methods of the disclosure described herein. The SARs and other compositions and methods the disclosure can be used for diagnostic, research and development and therapeutic applications. Generally, these instructions comprise a description of administration of the engineered immune cell for the above described therapeutic treatments. The SARs and other compositions and methods the dsclosure can be used for diagnostic, therapeutic research and development, laboratory research and therapeutic applications. Therefore, the kits and the accompanying instructions may comprise description of how to manufacture and use the various compositions and methods of the disclosure for diagnostic, therapeutic research and development, laboratory research and therapeutic applications.

The kit may include one or more agents for lymphodepletion and/or myelodepletion (*e.g*., alemtuzumab, cytoxan, fludarabine, cyclophosphamide, etoposide or temozolomide). The kit may include one or more adaptors or antibodies that can be used in combination with the SARs of the disclosure. The kit may include drugs (*e.g*., Dasatinib or Tocilizumab) or SAR adaptors to control the activity of SAR expressing cells. The kit may include the map and sequence of the SAR and vector encoding the SAR and the SAR adaptors. The kit may include instruction about how to generate vectors expressing the SAR, how to introduce SAR into immune cells (*e.g.*, T or NK cells), how to expand SAR-expressing cells and how to test the activity of SAR-expressing cells.

The kits may include instructions how to generate novel SARs, SAR adaptors, antibodies, including bispecific antibodies based on the novel antigen binding domains described in this disclosure and instructions regarding methods of use of such reagents.

The instructions relating to the use of the engineered immune cells as described herein generally include information as to dosage, dosing schedule, and route of administration for the intended treatment. The containers may be unit doses, bulk packages (*e.g*., multi-dose packages) or sub-unit doses. Instructions supplied in the kits of the disclosure are typically written instructions on a label or package insert (e.g, a paper sheet included in the kit), but machine-readable instructions (e.g, instructions carried on a magnetic or optical storage disk) are also acceptable. In some embodiments, the containers are identifiable (e.g, by label, barcode, or radio-frequency identification (RFID)), trackable, or imprinted with a machine-readable container identifier.

The kits of this disclosure are in suitable packaging. Suitable packaging includes, but is not limited to, vials, bottles, jars, flexible packaging (*e.g*., sealed Mylar or plastic bags), and the like. In some embodiments, the container (*e.g*., plastic bag) is suitable for intravenous infusion. Also contemplated are packages for use in combination with a specific device, such as an inhaler, nasal administration device (e.g, an atomizer) or an infusion device such as a minipump. A kit may have a sterile access port (for example the container may be an intravenous solution bag or a vial having a stopper pierceable by a hypodermic injection needle). The container may also have a sterile access port (for example the container may be an intravenous solution bag or a vial having a stopper pierceable by a hypodermic injection needle). At least one active agent in the composition is a SAR. The container may further comprise a second pharmaceutically active agent.

Kits may optionally provide additional components such as buffers and interpretive information. Normally, the kit comprises a container and a label or package insert(s) on or associated with the container.

Unless otherwise defined herein, scientific and technical terms used in connection with the present disclosure shall have the meanings that are commonly understood by those of ordinary skill in the art. While the foregoing disclosure provides a general description of the subject matter encompassed within the scope of the present invention, including methods, as well as the best mode thereof, of making and using this invention, the following examples are provided to further enable those skilled in the art to practice this disclosure and to provide a complete written description thereof. However, those skilled in the art will appreciate that the specifics of these examples should not be read as limiting on the invention, the scope of which should be apprehended from the claims and equivalents thereof appended to this disclosure. Various further aspects and embodiments of the present disclosure will be apparent to those skilled in the art in view of the present disclosure.

Animal models can also be used to measure SAR activity. For example, xenograft model using human cancer associated antigen described herein-specific SAR⁺ T cells to treat a primary human pre-B-ALL in immunodeficient mice can be used. Dose dependent SAR treatment response can be evaluated. See, *e.g.,* Milone et al., Molecular Therapy 17(S): 1453-1464 (2009). Assessment of cell proliferation and cytokine production has been previously described, *e.g.,* at Milone et al., Molecular Therapy 17(S): 1453-1464 (2009). Cytotoxicity can be assessed by a standard ⁵¹Cr-release assay or Matador assay. Imaging technologies can be used to evaluate specific trafficking and proliferation of SIRs in tumor-bearing animal models.

Other assays, including those described in the Example section herein as well as those that are known in the art can also be used to evaluate the SARs described herein.

All documents and sequence database identifiers mentioned in this specification are incorporated herein by reference in their entirety. "and/or" where used herein is to be taken as specific disclosure of each of the two specified features or components with or without the other. For example, "A and/or B" is to be taken as specific disclosure of each of (i) A, (ii) B and (iii) A and B, just as if each is set out individually herein. Unless context dictates otherwise, the descriptions and definitions of the features set out above are not limited to any particular aspect or embodiment of the disclosure and apply equally to all aspects and embodiments which are described.

The disclosure is further described in the non-limiting examples.

### EXAMPLES

Cell lines engineered to express luciferases (*e.g*., GLuc or NLuc) for measuring cytotoxicity of different constructs targeting different cell surface and intracellular antigens are provided in **Table C-1.** Cell lines used in these experiments, target antigens on the cells lines and their growth media are shown in the following **Table C-1.** The relative level of expression of the commonly used blood lineage antigens in several target cell lines is provided in **Table C-2.** Cells were cultured at 37°C, in a 5% CO₂ humidified incubator. The cell lines were obtained from ATCC, NIH AIDS reagent program or were available in the laboratory.

**Table C-1:**

| **Cell line** | **Culture Conditions** | **Exemplary SAR Target Antigens Expressed** |
|---|---|---|
| BC-1 | RPMI, 20% FCS | BCMA, GPRC, CD138 |
| BC-3 | RPMI, 20% FCS | BCMA, GPRC, CD138 |
| BCBL-1 | RPMI, 20% FCS | GPRC, CD138 |
| JSC-1 | RPMI, 20% FCS | GPRC, CD138 |
| MM1S | RPMI, 10% FCS | CD38, GPRC, CD44, CD200R |
| U266 | RPMI, 10% FCS | BCMA, WT1/HLA-A2+, CS1, CLL1, CD138, c-MET, IL6R, CD179b, NY-ESO/HLA-A2, NYBR, LAMP1 |
| L363 | RPMI, 10% FCS | BCMA, GPRC, WT1/HLA-A2+, CS1, CLL1, CD138, NY-ESO/HLA-A2, NYBR, LAMP1 |
| K562 | RPMI, 10% FCS | CD33, IL1Ra, TnAg |
| BV173 | RPMI, 10% FCS | CD123, CD179b, IL1Ra, WT1/HLA-A2+,CXCR4, FLT3, CD179a |
| Nalm6 | RPMI, 10% FCS | CD19, CD20, CD22, CD179b, CD179a |
| HL60 | RPMI, 10% FCS | CD33, CD34, CLL1, IL6R, CD32, CD179 |
| U937 | RPMI, 10% FCS | CD4, CLL1 |
| RS:411 (RS411) | RPMI, 20% FCS | CD19, Folate Receptor beta (FRbeta), TGFbeta, CD179b, NKG2D, FLT3, CD179a |
| MV:411 | RPMI, 10% FCS | FLT3, CD123, FRbeta |
| Raji | RPMI, 10% FCS | CD19, CD20, CD22, BCMA, CD38, CD70, CD79, Folate Receptor beta, CLL1 |
| HEL-92.1.7 (HEL) | RPMI, 10% FCS | MPL, CD33, CD32, CD200R |
| Jurkat | RPMI, 10% FCS | TnAg, TSLRP, TSHR, CD4, CD38 |
| Daudi | RPMI, 10% FCS | BCMA, FRbeta |
| REC-1 | RPMI, 10% FCS | NKG2D, ROR1 |
| KG-1 | RPMI, 20% FCS | CD33, CD34, CD123, TSLRP |
| CEM | RPMI, 10% FCS | CD5, CD43 |
| U937 | RPMI, 10% FCS | CD4, CLL1 |
| LAMA5 | RPMI, 10% FCS | WT1/HLA-A2 |
| A549 | DMEM,10% FCS | ROR1, CD22, TIM1, CDH17 |
| HT29 | DMEM,10% FCS | EGFR, SLEA, c-MET |
| Molm-13 | RPMI, 20% FCS | FLT3, IL6R, LAMP1, TSLRP, CD4, CSF2RA, CXCR4, IL6R, CSF2RA, GPC3 |
| A431 | DMEM,10% FCS | EGFR, Folate Receptor Alpha, Her3 |
| P19 | DMEM,10% FCS | SSEA |
| THP-1 | RPMI, 10% FCS | CD32, CD33,CXCR4, CD123, CD44, IL6R, Folate Receptor beta, CD70, LAMP1, FLT3, CSF2RA |
| U87MG | DMEM,10% FCS | CD276, gpNMB, IL13RA2 |
| LoVo | DMEM,10% FCS | Tissue Factor, CDH17, EGFR |
| SKOV-3 | DMEM,10% FCS | Folate Receptor alpha (FR1), FSHR, Her2, Her3, LHR, MSLN, TIM1, EPCAM |
| NCI-H1993 | DMEM,10% FCS | EGFR |
| Kasumi-1 | RPMI, 20% FCS | CLEC5A, PR1/HLA-A2, TGFbeta, |
| Jeko-1 | RPMI, 20% FCS | BCMA, ROR1 |
| PC-3 | DMEM,10% FCS | CGH, TROP2, PSCA, PSMA. EPCAM, FSHR, CLD18A2 (CLDN18.2) |
| HeLa | DMEM,10% FCS | EGFR, FR1, MSLN, TSHR |
| LnCap | DMEM,10% FCS | EGFR, FSHR, PSCA, PSMA, CD22, Her3, CD22, LHR, CLD18A2 (CLDN18.2) |
| OVCAR-3 | DMEM,10% FCS | B7H4, CDH6, DLL3, FR1, FSH, LHR, MSLN, PTK7, TnAg, TSHR, L1CAM |
| MEL-624 | DMEM,10% FCS | CDH19, GD2, GD3, gp100/HLA-A2, gpNMB, HMWMAA, NYESO/HLA-A2, MART1/HLA-A2 |
| LS174-T | DMEM,10% FCS | CEA |
| MEL-526 | DMEM,10% FCS | GD2 |
| MDA-MB231 | DMEM,10% FCS | CD324, Muc1 |
| L1236 | RPMI, 20% FCS | CD30, CD23, PDL1 |
| L428 | RPMI, 20% FCS | CD30, CD123, CCR4, PDL1 |
| L540 | RPMI, 20% FCS | CD30, CCR4, PDL1 |
| Molt-16 | RPMI, 20% FCS | IL1ra, NKG2D |
| CEM | RPMI, 10% FCS | CD5 |
| MG-63 | DMEM,10% FCS | IL13RA2 |
| Karpass-299 | RPMI, 20% FCS | Alk, GPRC, PDL 1 |
| MCF7 | DMEM,10% FCS | B7D4, CD276, TROP2, Her3, Muc1, LewisY, LHR |
| AA-2 | RPMI, 10% FCS | HIV1 env glycoprotein (gp120) |
| HL2/3 | DMEM,10% FCS | HIV1 env glycoprotein (gp120) |
| TF228.1.16 | DMEM,10% FCS | HIV1 env glycoprotein (gp120), CCR4 |
| TT | DMEM,10% FCS | TGF-Beta, TSHR, GFRalpha4 |
| DMS79 | RPMI, 10% FCS | Fucosyl-GM1, Slea (CA19.9; Sialyl Lewis Antigen) |
| LAN-5 | DMEM,10% FCS | ALK, DLL3, GFRalpha4, FUCOSYL-GM1 |
| PEER1 | RPMI, 10% FCS | TSHR |
| SK-MEL-37 | DMEM,10% FCS | DLL3, GD2 |
| F9 | DMEM,10% FCS | SSEA |
| HepG2 | DMEM,10% FBS | GPC3, AFP/HLA-A2, TAJ |

**TABLE C-2: Relative expression of different antigens in blood cancer cell lines on a scale of 1-5 with 5 being the highest.**

| | **RAJI** | **RAJI-CD19-KO** | **NLAM6** | **RS;411** | **Daudi** | **L363** | **U266** | **THP** |
|---|---|---|---|---|---|---|---|---|
| **CD19** | 5 | 0 | 4 | 3 | 4.5 | 0 | 0 | 0 |
| **CD22** | 2 | 2 | 2 | 3 | 3 | 0 | 0 | 0 |
| **CD20** | 4 | 4 | 0.2 | 0 | 5 | 0 | 0 | 0 |
| **BCMA** | 4 | 4 | 0.2 | 0 | 4 | 4 | 4 | 0 |
| **CD38** | 4 | 4 | 4 | 4 | 5 | 5 | 3 | 3 |

Jurkat cell line (clone E6-1) engineered with a NFAT-dependent EGFP (or GFP) reporter gene was a gift from Dr. Arthur Weiss at University of California San Francisco and have been described to study CAR-signaling ((Wu, CY *et al.,* Science 350:293-302,2015). Jurkat cells were maintained in RPMI-1640 medium supplemented with 10% FBS, penicillin and streptomycin.

### Generation of lentiviral vectors encoding SARs

The lentiviral vectors pLenti-EF1a or pLenti-EF1α (SEQ ID NO: 1), pLenti-EF1a-DWPRE [SEQ ID NO: 2] and pCCLc-MNDU3 Vector (SEQ ID NO: 3) were used. The psPAX2 vector was a gift from Didier Trono (Addgene plasmid # 12260). The pLP/VSVG envelope plasmid and 293FT cells were obtained from Invitrogen (ThermoFisher Scientific).

The generation of Chimeric antigen receptor (*e.g.*, 2^{nd} generation CARs, SIRs, Ab-TCR and TFP *etc.*), the generation and use of GGS-NLuc fusion proteins (Topanga and Malibu-Glo assays), and the generation and use of luciferase (*e.g*., GLuc) reporter cell lines for measurement of cellular cytotoxicity using the Matador assays have been described (PCT/US2017/024843, PCT/US2017/025602, PCT/US2017/052344, PCT/US2017/064379 and PCT/US2018/53247).

### Lentivirus and retrovirus vectors

Lentiviruses were generated by calcium phosphate or PI based transfection in 293FT cells essentially as described previously (Matta H et al, Cancer biology and therapy. 2(2):206-10. 2003). The virus was either used fresh to infect the target cells or stored frozen in aliquots at -80°C.

### Infection of T cells and PBMC

Buffy coat cells were obtained from healthy de-identified adult donors from the Blood Bank at Children Hospital of Los Angeles and used to isolate peripheral blood mononuclear cells (PBMC) by Ficoll-Hypaque gradient centrifugation. PBMC were either used as such or used to isolate T cells using CD3 magnetic microbeads (Miltenyi Biotech). PBMC or isolated T cells were re-suspended in XVIVO medium (Lonza) supplanted with 10 ng/ml CD3 antibody, 10ng/ml CD28 antibody and 100 IU recombinant human-IL2. Cells were cultured at 37°C, in a 5% CO2 humidified incubator. Cells were activated in the above medium for 1 day prior to infection with lentiviral vectors. In general, primary cells (*e.g.*, T cells) were infected in the morning using spin-infection (1800 rpm for 90 minutes at 37°C with 300µl of concentrated virus that had been re-suspended in XVIVO medium in the presence of 8 µg/ml of Polybrene^{®} (Sigma, Catalog no. H9268). The media was changed in the evening and the infection was repeated for two more days for a total of 3 infections. After the 3rd infection, the cells were pelleted and resuspended in fresh XVIVO media containing 10ng/ml CD3 antibody, 10ng/ml CD28 antibody and 100 IU recombinant human-IL2 and supplemented with respective antibiotics (if indicated) and place in the cell culture flask for selection, unless indicated otherwise. Cells were cultured in the above medium for 10-15 days in case no drug selection was used and for 20-30 days in case drug-selection was used. In cases, where cells were infected with a lentivirus expressing EGFP, they were expanded without drug-selection or flow-sorted to enrich for EGFP-expressing cells. For infection of cancer cell lines, approximately 500,000 cells were infected with 2 ml of the unconcentrated viral supernatant in a total volume of 3 ml with Polybrene^{®} (Sigma, Catalog no. H9268). Then next morning, the cells were pelleted and resuspended in the media with respective antibiotics and place in the cell culture flask for selection.

### FACS analysis for detecting expression of SAR

For detection of SARs using Protein L staining, 1 × 10⁶ cells were harvested and washed three times with 3 ml of ice-cold 1 × PBS containing 4% bovine serum albumin (BSA) wash buffer. After wash, cells were resuspended in 0.1 ml of the ice-cold wash buffer containing 1 µg of protein L at 4°C for 1 hour. Cells were washed three times with ice-cold wash buffer, and then incubated (in the dark) with 10µl of APC-conjugated streptavidin in 0.1 ml of the wash buffer for 30 minutes followed by two washings with ice cold wash buffer. FACS was done using FACSVerse analyzer from BD Biosciences.

### Assay to detect the expression of antigens on target cells and to determine the antigen binding activity of various of antigen binding moieties used in the construction of the SARs and BiTes

The expression of antigens on target cells was determined by bioinformatics approaches in combination with immunostaining with antigen specific antibodies or a highly sensitive antigen detection assay as described in PCT/US2017/025602 and incorporated herein in its entirety by reference. This assay involves the fusion of a GLuc or NLuc reporter fragment to the antigen binding domain of an antibody, a scFv, a vHH or any other antigen binding fragment or any receptor and ligand. The SEQ ID NOs of the exemplary soluble forms of several antigens containing their extracellular domains in fusion with Luc are provided in **Table 23.** These constructs also carry a puromycin resistance gene (PAC), which is optional and not needed for the functionality of the soluble proteins. The SEQ ID Nos of the exemplary scFv in fusion with Luc are provided in **Table 21.** The SEQ ID Nos of the exemplary SVH in fusion with Luc are provided in **Table 22.** These constructs also carry a puromycin resistance gene (PAC), which is optional and not needed for the functionality of the scFv luciferase fusion proteins.

### The immune effector cells expressing SAR are tested in the following assays to identify the functional SAR.

**(A) Topanga Assay (NLuc binding assay):** The control vector- and SAR-expressing Jurkat-NFAT-GFP or T cells are stained with the target Antigen-Nluc fusion protein (as described above) and their ability to bind to the target antigen is assayed by measuring Nluc activity. For example, Jurkat-NFAT-GFP cells expressing FMC63 based CAR targeting CD19 show increased binding to CD19-NLuc fusion protein as compared to control vector expressing Jurkat-NFAT-GFP cells or parental Jurkat-NFAT-GFP cells.

**(B) Assay for cytokine production**: The control vector- and SAR-expressing Jurkat-NFAT-GFP or T cells are cocultured with the target cell lines for 4-96 hours and supernatant examined for induction of cytokines (*e.g.*, IL2, IFNγ, TNFα *etc.*) expresson using ELISA.

**(C) Assay for Cytotoxic Activity *in vitro* and *in vivo*:** The uninfected T cells or those expressing a control vector or SAR are cocultured with the target cell lines expressing a non-secretory form of a luciferase (such as GLuc, NLuc, Turboluc 16 *etc.*) for 4-96 hours and induction of cell lysis examined by measuring the luciferase activity as described in PCT/US17/52344.

**Induction of NFAT promoter driven GFP expression.** The control vector- and SAR-expressing Jurkat-NFAT-GFP cocultured for 4-24 hours with the target antigen-expressing cell line (described above) and their ability to bind to the target antigen is assayed by measuring induction of GFP expression using Flow Cytometry. Cellular supernatant is collected and assayed for the induction of cytokines (*e.g*., IL2).

### Induction of NFAT promoter driven GFP expression.

A number of different single chain and multi chain SAR constructs on different backgrounds (e.g., 2^{nd} generation CAR, double chain SIR, one and half chain SIR etc) targeting different antigens were constructs. While some constructs targeted a single antigen, others targeted more than one antigen or two different epitopes of a single antigen.

The control vector- and SAR-expressing Jurkat-NFAT-GFP cocultured for 4-24 hours with the target antigen-expressing cell line (described above) and their ability to bind to the target antigen is assayed by measuring induction of GFP expression using Flow Cytometry. Cellular supernatant is collected and assayed for the induction of cytokines (*e.g*., IL2).

JNG (Jurkat cells expressing EGFP under an NFAT promoter) were infected with lentiviral vectors encoding different single chain and multi-chain SARs by spinfection in the presence of 8 µg/ml of Polybrene^{®} (Sigma, Catalog no. H9268). The Jurkat-NFAT-GFP cells are engineered in such a way that the IL-2 promoter, which carries NFAT binding sites, is cloned upstream of the GFP gene. These cells have been used to study signaling via TCR and CAR. The different SARs were stably expressed in Jurkat NFAT-GFP cells by lentiviral mediated gene transfer, followed by selection with puromycin or blasticidin. No drug selection was used in case the construct lacked an antibiotic resistance gene. The SAR-expressing Jurkat-NFAT-GFP cells were cocultured with the target cells at E:T ratio of approximately 1:2 for approximately 4 hours to 18 hours. When the interaction between SIRs and their target antigen results in activation of the NFAT pathway, GFP expression is induced. Hence Jurkat-NFAT-GFP cells expressing SAR show increased levels of GFP expression when they interact with target cell lines expressing the receptor for the SIR. Induction of GFP expression by coculturing of Jurkat-NFAT-GFP cells expressing different SIR constructs and the different target cells was studied essentially as described previously (Wu, Roybal, Puchner, Onuffer, & Lim, 2015). GFP expression was monitored by FACS analysis. The Jurkat-NFAT-GFP (parental) cells were used as control. The results with different SARs are summarized in the following summary **Tables.** The structure of different constructs can be determined based on their names. For example, the construct CD8SP-CD19-hu-mROO5-1-vL-[hTCRa-CSDVP]-F-F2A-**Spe-IgHSP-Apa-CD20-vHH-2HCD25-G4S**-CD19-hu-mROO5-1-vH-Mlu-[hTCRb-KACIAH]-F-P2A-PAC has the following modules from 5' to 3' direction: a CD8 signal peptide (CD8SP), the CD19 targeting hu-mROO5-1 vL fragment (CD19-hu-mROO5-1), Xho1 cloning site, human TCRa constant chain containing CSDVP mutations (hTCRa-CSDVP), Furine cleavage site (F), F2A ribosomal skip sequence (F2A), SpeI site, human IgH signal peptide (IgHSP), Apa I restriction enzyme site, camelid CD20-2HCD25-VHH module targeting CD20, BamHI restriction enzyme site, Glycinex4-serine linker (G4S), BstI restriction enzyme site, CD19-hu-mROO5-1-vH fragment targeting CD19, Mlu I restriction enzyme site, human TCRb constant chain containing KACIAH mutations ([hTCRb-KACIAH]), Furine cleavage site (F), P2A ribosomal skip sequence and Puromycin resistance gene (PAC). The sequence of the other constructs in the table can be similarly determined from their names and by referring to the different Tables of this disclosure that provide the names and SEQ ID NO of the different modules. Alternatively, the composition and modular architecture of the different SAR constructs can be determined from their sequences by bioinformatics tools, including using nucleic acid and amino acids homology searches. A SAR is considered positive in the assay in case the SAR expressing Jurkat-NFAT-GFP cells show greater % GFP positive cells when cultured with the target cell line as compared to parental Jurkat-NFAT-GFP cells. The signs +/-, +, 2+ *etc.* after the name of the cell lines indicate the relative degree of positivity on the Jurkat-NFAT-GFP assay as measured by the % GFP positive cells after culture of the SAR expressing Jurkat-NFAT-GFP cells with that cell line above the % GFP cells observed in parental Jurkat NFAT-GFP cells serving as controls. In general, the number +/-, +, 2+, 3+, 4+, 5+ refer to approximately 5%, 10%, 20%, 30%, 40% and 50% GFP positive cells. The % of GFP induction in the above assay is dependent not only the the relative strength of the SAR but also, among other factors, on the relative level of expression of the SAR in the JNG cells and the relative level of expression of the target antigen in the target cell line. To control for the difference in gene transduction efficiency of the different SAR constructs, the experiment was conducted with JNG cells transduced with SAR constructs co-expressing a drug resistance gene (e.g., puromycin or PAC) followed by drug selection to generate a stable population of SAR-expressing cells. It is to be noted that the drug resistance gene (e.g., PAC) in the SAR constructs is optional and in vivo studies were conducted with SAR constructs lacking the PAC gene.

### UNISPECIFIC, BISPECIFIC AND BIPARATOPIC SINGLE CHAIN SARs on 2nd GENERATION CAR BACKBONE

A number of unispecific, bispecific and biparatopic constructs on the 2^{nd} generation CAR backbone (BBz) with 4-1BB (BB) costimulatory domain and CD3z (z) activation domain were generated. These constructs, in general comprised a CD8 signal peptide (CD8SP) and CD8 hinge and transmembrane domain (CD8TM). They also comprised a Myc epitope tag, which was inserted upstream of the CD8TM region. The antigen binding domain(s) of these constructs comprised one or more AABDs based on single vH domains (SVH), including FHVH, aVH, chVH domains. The antigen binding domains of other construct comprised AABDs based on camelid derived vHH domains. An exemplary construct with a single AABD based on FHVH is represented by SEQ ID NO: 21907. An exemplary construct with a single AABD based on vHH is represented by SEQ ID NO:21908. An exemplary biparatopic construct with two tandem FHVH targeting two epitopes of BCMA is represented by SEQ ID NO: 21904. An exemplary construct with two tandem FHVH targeting the same epitopes of BCMA is represented by SEQ ID NO: 21903. An exemplaryi construct targeting two different antigens (BCMA and CD22) is represented by SEQ ID NO: 21910. An exemplary construct comprising a single human vH domain on chVH backbone is represented by SEQ ID NO: 21918. An exemplary bispecific construct comprising a vHH domain and a FVHV domain is represented by SEQ ID NO: 21912.

An exemplary construct comprising a BCMA-FHVH-93 attached to the N-terminus of a CD19-targeted scFv (MOR0028-scFv) via a G4s linker is CD8SP-BCMA-FHVH-93-G4S-CD19-MOR0028-scFv-hCD8TM-BBZ-T2A-PAC (SEQ ID NO: 21946). Similarly, the construct CD8SP-BCMA-FHVH-93-G4S-Her2-huMab-4D5-H91A-D98A-F100A-Y102V-hCD8TM-BBZ-T2A-PAC (SEQ ID NO: 21947) comprises BCMA-FHVH-93 attached to the N-terminus of a Her2 targeted scFv via a G4s linker.

The constructs were tested in JNG assay. As shown in **Table 41,** almost all constructs induced GFP expression when co-cultured with cell lines expressing their target antigen(s) but did not show GFP induction spontaneously or upon co-culture with cell lines that lacked the expression of their target antigens. These results demonstrate that functional unispecific, bispecific and biparatopic SAR constructs can be constructed with one or more AABD that are joined via optional short G4S linkers. The results further demonstrate lack of tonic signaling resulting in self-activation or compromised signaling when two or more AABD are joined in frame to make SAR constructs. Some of the CD38 targeted constructs did show GFP induction upon expression in JNG cells (i.e., self activation) without exposure to target cell lines. However, this was probably due to expression of CD38 antigen in JNG cells. The results further demonstrate that AABD belonging to different class or backbones (e.g., vHH, FHVH, chVH, aVH etc.) can be combined in a single SAR construct without compromising their antigen binding capabilities due to non-specific aggregation and/or steric hinderance between the different domains. Furthermore, the bispecific constructs comprising two AABD belonging to same class or different classes showed no significant decline in their ability to induce GFP in response to either of the single antigen as compared to unispecific constructs. Thus, the bispcific construct CD8SP-CD22-FHVH-24-PSMA71v2-chVH-Myc-CD8TM-BBZ-T2A-PAC (SEQ ID NO: 21917) was slightly more effective in inducing GFP (+5.5) when exposed to PSMA-expressing LNCaP cells as compared to the unispecific SAR construct (+4.5) represented by CD8SP-PSMA71-chVH-Myc-CD8TM-BBZ-T2A-PAC (SEQ ID NO: 21939). The SAR with SEQ ID NO: 21917 was, however, able to effectively induce GFP upon co-culure with RAJI cells that lack PSMA but express CD22.

Furthermore, effective GFP induction against both antigen targets were seen in the SAR constructs (SEQ ID NO: 21946-21947) in which an AABD is attached to the N-terminus of an scFv. For example, the construct with SEQ ID NO: 21946 showed effective GFP induction upon exposure to both RAJI (CD19⁺BCMA⁺) and RAJI-CD19-KO (CD19⁻BCMA⁺) cells as it was able to respond to the latter cells via BCMA-FHVH-93 domain. These results demonstrated that SAR constructs comprising an scFv and an AABD (e.g., FHVH, vHH, etc.) can be generated which show good expression, antigen binding and signaling abilities. An advantage of such SAR constructs is that a large number of monoclonal antibodies against different cancer antigens are available that can be used to generate scFv for incorporation into SARs. The use of two scFv to make bispecific or biparatopic SARs, however, results in poor expression and compromised signaling due to non-specific aggregation and tonic signaling induced by aggregations of the vL/vH fragments of the two scFv. This disclosure provides a solution to this problem by using one scFv in combination with one or more AABD to generate bispecific, biparatopic and multispecific single chain SAR constructs. Thus, these results demonstrate that functional single chain SARs with the backbones of a conventional (e.g., 2^{nd} or 3^{rd} generation) single chain CAR can be generated by joining in frame an AABD to the N-terminus or near the N-terminus of an scFv. The order of the vL and vH fragments in an scFv in such constructs can be vL-vH or vH-vL.

Additional exemplary SAR constructs comprising one or more AABD targeting different antigens that are joined in frame to the polynucleotide encoding the N-terminus of a CD19 targeting hu-mROO5-1 scFv via an optional linker are provided in SEQ ID NOs: 4087, 4118-4119, 4214 (NKG2D), 4245-4246, 4341 (RZip), 4595 (E4), 4753-4754 (CD16-V158), 4976 (CD19-vHH-048), 5070-5071 (BCMA-FHVH-33 x CD19-vHH-048), 5265 (CD20-vHH-2HCD25), 5472 (CD123-vHH-2), 5720 (Her2-vHH-2D3), 5899 (CD22-FHVH-158 x Her3-Affi) and 6009 (IL13Ra2-huvHH-USC1-C1), respectively. The description of the AABD in the above constructs is provided in paranthesis after their respective SEQ ID NOs. These SAR constructs are expressed in JNG cells and are shown to induce GFP upon co-culture with their target antigen(s) expressing cell lines. The experiment is conducted in the absence and presence of SAR adaptors for constructs encoding an adaptor binding domain as their AABD (*e.g.*, SEQ ID NO: 4214, 4341, 4595, 4753-4754 etc.). Additonal SAR constructs comprising different AABD fused to scFv can be constructed by substituting the AABD of the above constructs and tested using the assays described herein.

The above SAR constructs are also expressed in JNG cells in which the expression of constant chains of TCRα, TCRβ or both has been eliminated by CRISPR/Cas9 mediated gene knock-out. It is observed that the SAR constructs are expressed on the surface of TCRα-/-, TCRβ-/- and TCRα-/-β-/- cells at similar levels as the wild-type JNG cells and have similar signaling ability when exposed to their target antigen expressing cells. Essentially similar results are obtained when the SAR constructs are inserted at the TRAC or TRBC genomic loci so that it is expressed under the promoter and regulatory elements of TCRα or TCRβ genes. The insertion of SAR constructs at the TRAC and TRBC loci, which results in simultaneous disruption of these genes, is achieved using gene targeting as described in described in Knipping F et al, Molecular Therapy: Methods & Clinical Development, Vol 4, 2017 and Eyquem J et al Nature, 543(7643):113-117, 2017, respectively. Similarly, the endogenous TCRα, TCRβ, TCRγ or TCRδ genes are disrupted in αβ T cells or γδ T cells followed by the expression of the SARs with the conventional 2^{nd} generation CAR backbones. It is observed that the SARs on the conventional 2^{nd} generation backbone are still expressed and functional in the cells with elimination of TCRα, β, γ and/or δ chains.

**Table 41** demonstrate effective signaling via a number of unispecific and bispecific SAR construct targeting novel antigens. Exemplary unispecific constructs targeting CEA, CSPG4 and PSMA are represented by SEQ ID NO: 21927, 21945 and 21939, respectively. These SAR constructs can be used for the treatment of cancers expressing these antigens. Similarlly, bispecific SAR constructs targeting CD20 and CD22, BCMA and CD20, BCMA and CD22 etc can be used to overcome disease relapse due to loss of single antigen (antigen escape) on tumor cells.

The bispecific constructs targeting a solid tumor antigen and an antigen expressed on blood cells (e.g., PSMA with CD20, PSMA and CD22, or PSMA and BCMA) have the advantage of providing adequate proliferative signal to the SAR-T cells upon administration to the patient due to high level expression of hematopoietic antigens on blood cells. This can potentially overcome the problem of lack of expansion of solid tumor targeted unispecific SAR constructs.

**TABLE 41: UNISPECIFIC, BISPECIFIC AND BIPARATOPIC SINGLE CHAIN SARs on 2nd GENERATION CAR BACKBONE**

| TARG ET ANTIG EN | SE Q ID NO (D NA ) | SE Q ID NO (PR T) | Constructs Name | NFAT-GFP ASSAY (JNG) Assay |
|---|---|---|---|---|
| BCMA, CD19 | 219 01 | 227 16 | CD8SP-BCMA-FHVH-33-huCD19USC3- W103R-Myc-CD8TM-BBZ-T2A-PAC | Raji (3+), Raji-CD19-KO (+/-), Nalm6 (+), L363 (3+), U266 (3+). RS411 (-) |
| BCMA, CD19 | 219 02 | 227 17 | CD8SP-BCMA-FHVH-74-huCD19USC3- W103R-Myc-CD8TM-BBZ-T2A-PAC | Raji (+), Raji-CD19-KO (+), Nalm6 (-), L363 (4+), U266 (4+). RS411 (-) |
| BCMA | 219 03 | 227 18 | CD8SP-BCMA-FHVH-33-BCMA-FHVH- 33-Myc-CD8TM-BBZ-T2A-PAC | L363 (4+), U266 (3.5+) |
| BCMA | 219 04 | 227 19 | CD8SP-BCMA-FHVH-33-BCMA-FHVH- 74-Myc-CD8TM-BBZ-T2A-PAC | L363 (4.5+), U266 (4+) |
| BCMA | 219 05 | 227 20 | CD8SP-BCMA-FHVH-74-BCMA-FHVH- 33-Myc-CD8TM-BBZ-T2A-PAC | L363 (4.5+), U266 (3.5+) |
| BCMA | 219 06 | 227 21 | CD8SP-BCMA-FHVH-74-BCMA-FHVH- 74-Myc-CD8TM-BBZ-T2A-PAC | Raji (1.5+), Raji-CD19-KO (1.5+), Nalm6 (+/-), L363 (5+), U266 (4.5+). RS411 (-) |
| BCMA | 219 07 | 227 22 | CD8SP-BCMA-FHVH-74-CD8TM-BBZ | L363 (+/-), U266 (+/-) |
| CD20 | 219 08 | 227 23 | CD8SP-CD20vHH-CD8TM-BBZ | Raji (+/-), Nalm6 (+/-) |
| CD22, BCMA | 219 09 | 227 24 | CD8SP-CD22-FHVH-24-BCMA-FHVH- 33-CD8TM-BBZ | Raji (2+), Nalm6 (+), L363 (3+), U266 (1.5+). Daudi (1.5) |
| CD22, BCMA | 219 10 | 227 25 | CD8SP-CD22-FHVH-24-BCMA-FHVH- 74-CD8TM-BBZ | Raji (+/-), Nalm6 (+/-), Daudi (+/-), L363 (+/-), U266 (+/-) |
| CD22, BCMA | 219 11 | 227 26 | CD8SP-CD22-FHVH-24-BCMA-FHVH- 93-CD8TM-BBZ | Raji (+/-), Nalm6 (+/-), Daudi (+/-), L363 (+/-), U266 (+/-) |
| CD20, CD22 | 219 12 | 227 27 | CD8SP-CD20vHH-G4S-CD22FHVH- CD8TM-BBZ | Raji (1.5+), Nalm6 (+/-), Daudi (+) |
| CEA | 219 13 | 227 28 | CD8SP-CEA300-3001-aVH-CD8TM-BBZ | HL60 (-), LoVo (+/-) |
| CD22, CEA | 219 14 | 227 29 | CD8SP-CD22-FHVH-24-CEA3001-aVH- Myc-CD8TM-BBZ-T2A-PAC | Raji (2+), LoVo (4+) |
| CD22 | 219 15 | 227 30 | CD8SP-CD22-FHVH-24-CD22-FHVH- 158-Myc-CD8TM-BBZ-T2A-PAC | Raji (2+) |
| CD22, PSMA | 219 16 | 227 31 | CD8SP-CD22-FHVH-24-PSMA28-chVH- Myc-CD8TM-BBZ-T2A-PAC | Raji (2+), LNCaP (5+) |
| CD22, PSMA | 219 17 | 227 32 | CD8SP-CD22-FHVH-24-PSMA71v2- chVH-Myc-CD8TM-BBZ-T2A-PAC | Raji (2.5+), LNCaP (5.5+) |
| CD22, PSMA | 219 18 | 227 33 | CD8SP-CD22-FHVH-24-PSMA28v2- chVH-Myc-CD8TM-BBZ-T2A-PAC | Raji (2.5+), LNCaP (6+) |
| CEA, BCMA | 219 19 | 227 34 | CD8SP-CEA300-aVH-BCMA-FHVH-93- Myc-CD8TM-BBZ-T2A-PAC | LoVo (4+), L363 (5+) |
| CD20, BCMA | 219 20 | 227 35 | CD8SP-CD20vHH-C07-BCMA-FHVH-93- Myc-CD8TM-BBZ-T2A-PAC | Raji (2.5+), L363 (5+) |
| PSMA, BCMA | 219 21 | 227 36 | CD8SP-PSMA71-chVH-BCMA-FHVH-93- Myc-CD8TM-BBZ-T2A-PAC | LNCaP (4+), L363 (5+) |
| PSMA, BCMA | 219 22 | 227 37 | IgHSP-PSMA28v2-chVH-BCMA-FHVH- 93-Myc-CD8TM-BBZ-T2A-PAC | LNCaP (4+), L363 (4+) |
| BCMA | 219 23 | 227 38 | CD8SP-BCMA-FHVH-74-Myc-CD8TM- BBZ-T2A-PAC | L363 (5+) |
| CD20 | 219 24 | 227 39 | CD8SP-CD20vHH-C07-Myc-CD8TM- BBZ-T2A-PAC | Raji (3+) |
| PSMA | 219 25 | 227 40 | CD8SP-PSMA71-chVH-Myc-CD8TM- BBZ-T2A-PAC | LNCaP (4.5+) |
| CD22, BCMA | 219 26 | 227 41 | CD8SP-CD22-FHVH-24-BCMA-FHVH- 33-hCD8TM-BBZ-T2A-PAC | Raji (2+), L363 (3.5+) |
| CEA | 219 27 | 227 42 | CD8SP-CEA300-aVH-Myc-CD8TM-BBZ- T2A-PAC | LoVo (5+) |
| BCMA, CD38 | 219 28 | 227 43 | CD8SP-BCMA-FHVH-33-CD38-309407- FHVH-Myc-CD8TM-BBZ-T2A-PAC | L363 (+/-) |
| BCMA, CD38 | 219 29 | 227 44 | CD8SP-BCMA-FHVH-74-CD38-309021- FHVH-Myc-CD8TM-BBZ-T2A-PAC | L363 (-) |
| BCMA, CD38 | 219 30 | 227 45 | CD8SP-BCMA-FHVH-33-CD38-986- FHVH-Myc-CD8TM-BBZ-T2A-PAC | L363 (-) |
| BCMA, CD38 | 219 31 | 227 46 | CD8SP-BCMA-FHVH-74-CD38-986- FHVH-Myc-CD8TM-BBZ-T2A-PAC | Self activation |
| BCMA | 219 32 | 227 47 | CD8SP-BCMA-FHVH-33-BCMA-FHVH- 93-Myc-CD8TM-BBZ-T2A-PAC | L363 (5+), U266 (4+), Nalm6 (+/-), RS411 (-) |
| CD22, BCMA | 219 33 | 227 48 | CD8SP-CD22-FHVH-24-BCMA-FHVH- 74-Myc-CD8TM-BBZ-T2A-PAC | L363 (4+), U266 (4+), Nalm6 (+), RS411 (+) |
| CD22, BCMA | 219 34 | 227 49 | CD8SP-CD22-FHVH-24-BCMA-FHVH- 93-Myc-CD8TM-BBZ-T2A-PAC | L363 (4+), Nalm6 (+), RS411 (+/-) |
| CD22, BCMA | 219 35 | 227 50 | CD8SP-CD22-FHVH-24-BCMA-FHVH- 33-Myc-CD8TM-BBZ-T2A-PAC | L363 (5+), Nalm6 (+), RS411 (+) |
| BCMA | 219 36 | 227 51 | CD8SP-BCMA-FHVH-74-BCMA-FHVH- 93-Myc-CD8TM-BBZ-T2A-PAC | L363 (5+), Nalm6 (-), RS411 (-) |
| BCMA | 219 37 | 227 52 | CD8SP-BCMA-FHVH-74-Myc-CD8TM- BBZ-T2A-PAC | L363 (5+) |
| CD20 | 219 38 | 227 53 | CD8SP-CD20vHH-C07-Myc-CD8TM- BBZ-T2A-PAC | Raji (3+) |
| PSMA | 219 39 | 227 54 | CD8SP-PSMA71-chVH-Myc-CD8TM- BBZ-T2A-PAC | LNCaP (4.5+) |
| BCMA, CD38 | 219 40 | 227 55 | CD8SP-BCMA-FHVH-33-CD38-309021- FHVH-Myc-CD8TM-BBZ-TA2-PAC | Self activation |
| CD22 | 219 41 | 227 56 | CD8SP-CD22-FHVH-24-G4S-CD22- FHVH-158-Myc-CD8TM-BBZ-T2A-PAC | Raji (1.5+), Nalm6 (+), RS411 (-) |
| BCMA | 219 42 | 227 57 | CD8SP-BCMA-FHVH-33-Myc-CD8TM- BBZ-T2A-PAC | L363 (5.5+), RS411 (-) |
| BCMA, CD19 | 219 43 | 227 58 | CD8SP-BCMA-FHVH-74-CD19- MOR0chVH-Myc-CD8TM-BBZ-T2A-PAC | Raji (1.5+), Nalm6 (-), L363 (4+) |
| CSPG4 | 219 44 | 227 59 | CD8SP-MCSP-aVH-58-Myc-CD8TM- BBZ-TA2-PAC | SK-MEL-28 (+) |
| CSPG4 | 219 45 | 227 60 | CD8SP-MCSP-aVH-56-Myc-CD8TM- BBZ-TA2-PAC | SK-MEL-28 (1.5+) |
| BCMA, CD19 | 219 46 | 227 61 | CD8SP-BCMA-FHVH-93-G4S-CD19- MOR0028-scFv-hCD8TM-BBZ-T2A-PAC | Raji (4+), Raji-CD19-KO (3+), Nalm6 (3.5+), RS411 (5+) |
| BCMA, HER2 | 219 47 | 227 62 | CD8SP-BCMA-FHVH-93-G4S-Her2- huMab-4D5-H91A-D98A-F100A-Y102V- hCD8TM-BBZ-T2A-PAC | Raji (3+), Nalm6 (+), L363 (4+), RS411 (-), SKOV-3 (7+), MCF-7 (2.5+), PC-3 (-) |

### UNISPECIFIC, BISPECIFIC AND BIPARATOPIC SINGLE CHAIN SARs ON TFP BACKBONE

Single chain receptors comprising an scFv fused to CD3ε, CD3γ, CD3ε chains have been described and are known as TFPε, TFPγ and TFPε respectively. To generate SAR constructs comprising AABDs that are fused to the extracellular, transmembrane and cytoplasmic domains (ECDTMCP) of CD3ε, CD3γ, CD3ε, a number of bispecific and biparatopic constructs were constructed. The names, component AABD modules and SEQ ID NOs of these constructs are provided in **Table 42.** The AABD components of these constructs belong to different classes, such as FHVH (e.g., SEQ ID NO: 21949-21955), avH (SEQ ID NO: 21951) and vHH (SEQ ID NO: 21963) frameworks. Representative bispecific constructs are represented by SEQ ID NO: 21950-21953 and represented biparatopic constructs are represented by SEQ ID NO: 21949 and 21952. The constructs were cloned in lentiviral vectors and stably expressed in JNG cells as described above. The JNG cells expressing the SARs were tested in NFAT-GFP assay upon co-culture with the target antigen expressing cell lines. **Table 42** shows robust induction of GFP by nearly all SAR constructs. Further, the % GFP induction by the bispecific constructs against a specific antigen (e.g., CD20) is noted to be similar to the % GFP observed using a unispecific construct targeting that antigen and comprising the same antigen binding domain, thereby demonstrating lack of steric hinderance by the presence of more than one antigen binding domains. These results demonstrate that functional single chain SAR constructs with the backbones of TFPε, TFPδ and TFPγ can be generated using AABDs with the framework of vHH and single vH domains, including fully human single vH domains. The results further demonstrate that functional single chain bispecific and biparatopic SAR with the backbones TFPε, TFPδ and TFPγ can be generated using AABDs comprising single vH domains, including fully human vH domains. The results further demonstrate that functional bispecific and biparatopic SAR with the backbones TFPε, TFPδ and TFPγ can be generated using a combination of AABD belonging to different classes (e.g., FHVH and vHH etc.).

Additional exemplary SAR constructs with the backbones of TFPs and comprising one or more AABD are represented by SEQ ID NO: 6495-6497 (CD22-FHVH-24 x PSMA-chVH-71v2), 6641-6643 (CD22-FHVH-24 x CD19-FHVH-354), 6714-6716 (CD22-FHVH-24 x BCMA-FHVH-74), 6933-6935 (CEA-aVH-300 x BCMA-FHVH-93). Exemplary bispecific, biparatopic and multispecific SAR constructs with the backbones of TFPs and comprising one or more AABDs that are joined in frame to the N-terminus of the encoded CD19 targeted humanized hu-mROO5-1 scFv are represented by SEQ ID NO: 5004-5006 (CD19-vHH-048), 5252-5254 (CD38-FHVH-309021), 5346-5348 (BCMA-FHVH-33 x CD38-FHVH-309021), 5594-5596 (BCMA-FHVH-33 x CD123-vHH-2), 4237-4239 (NKG2D x NKG2D), 4364-4367 (RZip), 4491-4493 (E4), and 4745-4747 (CD16A-V158), respectively. The names of the AABD(s) are shown in parenethesis after their SEQ ID NOs. These SAR constructs can be expressed in JNG cells and shown to induce GFP upon co-culture with their target antigen(s) expressing cell lines. The experiment is conducted in the absence and presence of SAR adaptors for constructs encoding an adaptor binding domain as one of their AABDs (*e.g*., SEQ ID NO: 4364, 4341, 4491, 4745 etc.). Additonal SAR constructs comprising different AABD fused to scFv can be constructed by substituting the AABD of the above constructs and tested using the assays described herein. These results can be used to support the argument that functional single chain SARs with the backbones of TFPε, γ and δ can be generated by joining in frame an AABD to the N-terminus or near the N-terminus of an scFv. The order of the vL and vH fragments in an scFv in such constructs can be vL-vH or vH-vL.

The above SAR constructs on TFP backbones are also expressed in JNG cells in which the expression of TCRα, TCRβ or both has been eliminated by CRISPR/Cas9 mediated gene knock-out. In contrast to the results with SARs on the conventional 2^{nd} generation CAR backbone, it is observed that the SAR constructs on the TFP backbones are not effectively expressed on the surface of TCRα-/-, TCRβ-/- and TCRα-/-β-/- cells at similar levels as observed in the wild-type JNG cells and have poor signaling ability when exposed to their target antigen expressing cells. It is observed that co-expression of the constant chain of TCRα can rescue the expression of the SARs on the TFP backbone in cells in which the expression of endogenous TCRα chain has been impaired or eliminated. An exemplary construct to co-express an exogenous TCRα constant chain is presented in SEQ ID NO: 23331. Similary, co-expression of TCRβ constant chain is observed to rescue the expression of SARs on the TFP backbone in cells in which the endogenous TCRβ chain has been eliminated or impaired. An exemplary construct to co-express an exogenous TCRβ constant chain is presented in SEQ ID NO: 23332. Finally, coexpression of both TCRα and TCRβ constant chains is observed to be sufficient to rescue the expression of SARs on TFP backbone in the αβ T cells in which both TCRα and TCRβ have been knocked out. Alternatively, it is observed that co-expression of the constant chains of both TCRγ and TCRδ can also rescue the expresson of SARs on TFP backbone in αβ T cells in which the expression of endogeneous TCRα and/or TCRβ constant chains has been eliminated. Exemplary constructs to express TCRγ and TCRδ chains are provided in SEQ ID NO: 23333 and 23334, respectively. Similarly, the coexpression of constant chains TCRα and TCRβ can also rescue the expresson of SARs on TFP backbone in γδ T cells in which the expression of endogeneous TCRγ and/or TCRδ constant chains has been eliminated.

Essentially similar results are obtained when the SAR constructs are inserted at the TRAC or TRBC genomic loci so that they are expressed under the promoter and regulatory elements of endogenous TCRα or TCRβ genes. The insertion of SAR constructs at the TRAC and TRBC loci, which results in simultaneous disruption of these genes, is achieved using gene targeting as described in described in Knipping F et al, Molecular Therapy: Methods & Clinical Development, Vol 4, 2017 and Eyquem J et al Nature, 543(7643):113-117, 2017, respectively. An exemplary targeting construct is represented by SEQ ID NO: 23329. SARs on the TFP backbone show impaired expression on T cells surface and reduced or absent activity (e.g., T cell activation, proliferation, cytokine production and cytotoxicity etc.) in T cell when their expression is directed to the TRAC locus as compared to when they are expressed using lentiviral vectors. It is next tested if expression of TCRα constant chain (TRAC chain) can be used to restore the expression and signaling activity of SARs on TFP backbone in T cells in which the endogenous TRAC genomic locus has been disrupted by the SARS expression cassette. For this purpose, a targeting construct is constructed that coexpress a TFP with an accessory module encoding a TCRα constant chain with an amino terminal signal peptide (IgSP). The accessory module is separated from the SARs encoding sequence by a Furine-SGSG-P2A linker. The nucleotide sequence of an exemplary targeting construct coexpressing a TCRα constant chain with an exemplary SAR constructs on TFP backbone targeting BCMA and CD22 is shown in SEQ ID NOs: 23330. The nucleotide sequence of the TCRα constant chain in this construct is codon optimized and differs from the endogenous TCRα constant chain in its nucleotide sequence. In an alternate embodiment, the TRAC chain is codon optimized and carries amino acid substitutions (e.g., SEQ ID NO: 1044, 1047) that are known to enhance the expression of human TCRα constant chain or its base pairing with exogenously expressed TCRβ chian. The exogenous TRAC can be expressed in T cells either by itself (SEQ ID NO: 23331) or it can be co-expressed with the TFP expression cassettes using a single vector.

The cells expressing the SAR constructs are exposed to RAJI and L363 target cells and tested in functional assays as described above. T cells in which the targeting construct includes a SAR on TFP backbone co-expressing a TCRα constant chain (SEQ ID NO: 23330) show better expression of the SAR on cell surface as compared to T cells in which the SAR construct on the TFP backbone alone (SEQ ID NO: 23329) (*i.e.*, without coexpression of the exogenous TRAC chain) is directed to the TRAC locus. In addition, T cells targeted with the targeting construct with SEQ ID NO: 23330 show greater proliferation, activation, cytokine (e.g., IL2 and TNFα) production, cytotoxicity, in vivo expansion, in vivo anti-tumor activity against the target cells as compared to T cells targeted with the construct with SEQ ID NO: 23329. It is also observed that co-expression of the constant chains of both TCRγ and TCRδ can also rescue the expresson of SARs on TFP backbone in αβ T cells in which the expression of endogeneous TCRα and/or TCRβ constant chains has been eliminated by insertion of the SAR expresson cassette at the endogenous TRAC or/and TRBC loci. Exemplary constructs to express TCRγ and TCRδ chains are provided in SEQ ID NO: 23333 and 23334, respectively.

| **TABLE 42: UNISPECIFIC, BISPECIFIC AND BIPARATOPIC SINGLE CHAIN SARs ON TFP BACKBONE** | | | | |
|---|---|---|---|---|
| **TARGE T ANTIGE N** | **SEQ ID NO (DN A)** | **SEQ ID NO (PR T)** | **Constructs Name** | **NFAT-GFP ASSAY** |
| CD22 | 2194 9 | 2276 4 | CD8SP-CD22-FHVH-24-G4S-CD22- FHVH-158-G4S-CD3dECDTMCP-2A-Pac | Raji (1.5+), Nalm6 (+/-) |
| CD22, BCMA | 2195 0 | 2276 5 | CD8SP-CD22-FHVH-24-BCMA-FHVH- 33-G4S-CD3dECDTMCP-2A-Pac | Raji (1.5+), L363 (1.5+) |
| CEA | 2195 1 | 2276 6 | CD8SP-CEA300-aVH-G4S- CD3dECDTMCP-2A-Pac | LoVo (2+) |
| CD22 | 2195 2 | 2276 7 | SP-CD22-FHVH-24-G4S-CD22-FHVH- 158-G4S-CD3eECDTMCP-2A-Pac | Raji (+), Nalm6 (+/-) |
| CD22, BCMA | 2195 3 | 2276 8 | SP-CD22-FHVH-24-BCMA-FHVH-33- G4S-CD3eECDTMCP-2A-Pac | Raji (+), L363 (+/-) |
| CD22 | 2195 4 | 2276 9 | SP-CD22-FHVH-24-CD22-FHVH-158- G4S-CD3dECDTMCP-2A-Pac | Raji (+), Nalm6 (+/-) |
| CD22, BCMA | 2195 5 | 2277 0 | SP-CD22-FHVH-24-BCMA-FHVH-74- G4S-CD3dECDTMCP-2A-Pac | Raji (1.5+), L363 (2+) |
| CEA | 2195 6 | 2277 1 | SP-CEA300-aVH-G4S-CD3eECDTMCP- 2A-Pac | LoVo (2.5+) |
| CD22 | 2195 7 | 2277 2 | SP-CD22-FHVH-24-CD22-FHVH-158- G4S-CD3eECDTMCP-2A-Pac | Raji (+), Nalm6 (+/-) |
| CD22, BCMA | 2195 8 | 2277 3 | SP-CD22-FHVH-24-BCMA-FHVH-74- G4S-CD3eECDTMCP-2A-Pac | Raji (1.5+), L363 (+) |
| BCMA, CD38 | 2195 9 | 2277 4 | SP-BCMA-FHVH-74-CD38-309021- FHVH-G4S-CD3eECDTMCP-2A-Pac | L363 (2.5+) |
| BCMA, CD38 | 2196 0 | 2277 5 | SP-BCMA-FHVH-33-CD38-986-FHVH- G4S-CD3eECDTMCP-2A-Pac | L363 (2.5+) |
| BCMA, CD38 | 2196 1 | 2277 6 | SP-BCMA-FHVH-74-CD38-986-FHVH- G4S-CD3eECDTMCP-2A-Pac | L363 (2.5+) |
| BCMA, CD38 | 2196 2 | 2277 7 | SP-BCMA-FHVH-33-CD38-309021- FHVH-G4S-CD3eECDTMCP-2A-Pac | L363 (2.5+) |
| CD20, CD22 | 2196 3 | 2277 8 | CD20-vHH-C07-G4S-CD22-FHVH-158- G4S-CD3eECDTMCP-2A-Pac | Raji (2.5+), Nalm6 (+/-) |

### GENERATION OF UNISPECIFIC, BISPECIFIC AND BIPARATOPIC SARs ON ONE-AND-A-HALF AND DOUBLE CHAIN SIR BACKBONES

Synthetic Immune Recepor (SIR) is a next generaton CAR platform described in WO2018102795 that provides physiological T cell signaling by engaging the endogeneous TCR signaling complex. In one configuration, a double chain SIR typicaly comprises two polypeptides each of which comprises an antigen binding domain (e.g., a vL or a vH etc.) attached to a TCR constant chain (e.g., Cα and Cβ or Cγ and Cδ). The vL and vH domains of a double chain SIR join to form an Fv that can bind to the target antigen. A one and a half chain SIR comprises an antigen binding domain (*e.g*., an scFV) attached in frame to one TCR constant chain which is coexpressed with the complementary second TCR constant chain that lacks an antigen binding domain. Several bispecific SIR constructs were generated in which nucleotides encoding two scFv were attached to the two TCR constant chains. For example, in the SAR construct with SEQ ID NO (DNA): 21974 and SEQ ID NO (PRT): 22789, the encoded scFv targeting CD19 derived from a humanized CD19 directed monoclonal antibody (hCD19-Bu12) is fused in frame to the mutated TCRβ chain (hTCRβ-KACIAH) and the encoded scFv targeting CD123 (CD123-CSL362) is attached to the complementary mutated human TCRα chain (hTCRα-CSDVP). The resulting construct was expressed in JNG cells but was found to be unresponsive against target cells expressing CD19 (e.g., Raji) and CD123 (Bv173) **(Table 43).** Similarly, the bispecific SIR construct represented by SEQ ID NO: 21975 encodes an MPL targeting scFv (MPL-175) attached in frame via an intervening linker to hCD19-Bu12 scFv which in turn is attached to the mutated human TCRα chain (hTCRα-CSDVP) via a MYC tag linker. The encoded TCRβ chain (hTCRβ-KACIAH) in this construct lacks an antigen binding domain but carries a V5 tag. This construct was also found to show only weak activity against CD19 expressing Raji and Nalm6 cells as measured by JNG assay. JNG cells expressing this construct also showed marginal GFP induction when co-cultured with MPL expressing HEL 92.1.7 cells (**Table 43**). In the constructs represented by SEQ ID NO: 21981 and 21982, the encoded two vL fragments are joined in tandem via an E-coil linker and are then attached in frame to one TCR constant chain whereas two vH fragments are joined in tandem via a K-coil linker and then attached to the complementary TCR chain. The construct with SEQ ID NO: 21981 showed no GFP induction activity in JNG assay while the construct with SEQ ID NO: 21982 showed mild to moderate activity. Several additional bispecific and biparatopic constructs on double chain or one and a half chain SIR backbones were generated encoding two or more vL, vH or scFv fragments joined via a variety of linkers and in different configurations and tested in the JNG assay. These constructs, in general, showed weak and/or inconsistent GFP induction when expressed in JNG cells as compared the corresponding double chain (DC) or one-and-half-chain (OAH) unispecific constructs encoding a single vL, vH or scFv per construct. Results with several exemplary constructs are provided in **Table 43.** These results demonstrate that presence of more than one vL, vH or scFv in a DC or OAH chain SIR backbone results in poor and/or inconsistent expression and/or signaling activity.

| **TABLE 43: UNISPECIFIC, BISPECIFIC AND BIPARATOPIC SARs ON ONE-AND-A-HALF AND DOUBLE CHAIN SIR BACKBONE** | | | | |
|---|---|---|---|---|
| **ANTIGEN** | **SEQ ID NO (DNA)** | **SEQ ID NO (PRT)** | **Constructs Name** | **NFAT-GFP ASSAY** |
| CD19, CD123 | 21974 | 22789 | hCD19-Bu12(vL-vH)-V5-[hTCRβ-KACIAH]- F-P2A-CD123-CSL362-(vL-vH)-MYC- [hTCRα-CSDVP]-F-F2A-PAC | Raji (-), Bv173 (-) |
| MPL, CD19 | 21975 | 22790 | SP-V5-[hTCRβ-KACIAH]-F-P2A-MPL-175- scFv-EAAAK-hCD19-Bu12-scFv-MYC- [hTCRα-CSDVP]-F-F2A-PAC | Raji (+), Nalm6 (+), HEL 92.1.7 (+/-) |
| CS1, CD19 | 21976 | 22791 | SP-V5-[hTCRβ-KACIAH]-F-P2A-HuLuc64- EAAAK-hCD19-Bu12-scFv-MYC-[hTCRα- CSDVP]-F-F2A-PAC | Raji (2+), Nalm6 (2+), L363 (+), U266 (1.5+) |
| CD19, CD20 | 21977 | 22792 | SP-V5-[hTCRβ-KACIAH]-F-P2A-hCD19- Bu12-EAAAK-CD20-2F2-scFv-MYC-[hTCRα- CSDVP]-F-F2A-PAC | Raji (+), Nalm6 (+) |
| CD19, CD20 | 21978 | 22793 | SP-V5-[hTCRβ-KACIAH]-F-P2A-huFMC63- 11-EAAAK-CD20-2F2-scFv-MYC-[hTCRα- CSDVP]-F-F2A-PAC | Raji (2+), Nalm6 (2+) |
| CS1, CD19 | 21979 | 22794 | SP-V5-[hTCRβ-KACIAH]-F-P2A-HuLuc64- EAAAK-FMC64-scFv-MYC-[hTCRα- CSDVP]-F-F2A-PAC | Raji (+), Nalm6 (+), L363 (+), U266 (1.5+) |
| CD19, CS1 | 21980 | 22795 | hCD19-Bu12-V5-[hTCRβ-KACIAH]-F-P2A- huLuc64-scFV-MYC-[hTCRα-CSDVP]-F-F2A- PAC | Raji (+), Nalm6 (+/-), L363 (+/-), U266 (+) |
| CD19, CD19 | 21981 | 22796 | SP-FMC63-vL-Ecoil-MOR0028-vL-[hTCRβ- KACIAH]-F-P2A-IgH-SP-FMC63-vH-Kcoil- MOR0028-vH-[hTCRα-CSDVP]-F-F2A-PAC | Raji (-), Nalm6 (-) |
| CD19, CD22 | 21982 | 22797 | SP-FMC63-vL-Ecoil-CD22-5-vL-[hTCRα- CSDVP]-F-F2A-SP-FMC63-vH-Kcoil-CD22-5- vH-[hTCRβ-KACIAH]-F-P2A-PAC | Raji (2+), Nalm6 (+), Bv173 (+) |

### GENERATON AND TESTING OF UNISPECIFIC, BISPECIFIC AND BIPARATOPIC DOUBLE CHAIN CONSTRUCTS COMPRISING AABDs

Next, approximately 100 double chain constructs encoding AABD were generated and expressed in JNG cells. These constructs encoded a variety of antigen binding domains. The SEQ ID (DNA) and SEQ ID (PRT) are provided in Table 44. In the constructs with SEQ ID NO: 21985-21997, 22007-22008, 22011-22016, the encoded one FVHV is attached to TCRβ constant chain (hTCRβ-S57C) and the encoded second FHVH is attached to a the complementary TCRα constant chain [hTCRα-T48C-opt]. For example, in the exemplary construct with SEQ ID NO: 21985, the encoded CD22-FHVH-24 is attached to hTCRβ-S57C and BCMA-FHVH-93 is attached to hTCRα-T48C-opt. All these constructs failed to show significant activity in the JNG assay when expressed in JNG cells and co-cultured with their target antigen expressing cell lines (**Table 44**). Replacement of FHVH domain with other AABD (e.g., vHH or aVH) did not result in improvement in activity. Several unispecific and bispecific one-and-half chain constructs were also generated and are represented by SEQ ID NO: 21999-22000, 22002-22006 (**Table 44**). A representative construct of this series is CD8SP-V5-[hTCRβ-KACIAH]-F-P2A-CD8SP-BCMA-FHVH-74-MYC-[hTCRα-CSDVP]-F-F2A-PAC (SEQ ID NO: 22002). In this construct, an encoded BCMA-FHVH-74 is attached via a MYC linker to [hTCRα-CSDVP] chain while a V5 linker is attached to the encoded complementary [hTCRβ-KACIAH] chain. These constructs also showed only weak and inconsistent activtiy when expressed in JNG cells. Another design tested involved attachement of two AABD in tandem to one of the two TCR constant chains and is represented by SEQ ID NO: 22009 and 22010. The constructs with this design also showed only weak and inconsistent activity (**Table 44**). Next, constructs were generated in which one or both the TCR constant chains were truncated. Additionally, a variety of linker domains between the AABD and the TCR constant chain domains were tested. Exemplary linkers included Gly-Ser linkers (GS), E Coil, PG4SP and Ig Hinge domains (**Table 17**). Exemplary constructs are represented by SEQ ID NO:22025-22085. These constructs were expressed in JNG cells and tested in JNG assay. The constructs with this design also, in general, showed only weak and/or inconsistent activity as compared to the corresponding single chain constructs on the 2nd generation CAR or TFP backbones (**Table 44**).

| **TABLE 44: BISPECIFIC AND BIPARATOPIC DOUBLE CHAIN CONSTRUCTS** | | | |
|---|---|---|---|
| **ANTIGEN** | **SEQ ID NO (DNA)** | **SEQ ID NO (PRT)** | **NFAT-GFP ASSAY** |
| CD22, BCMA | 21985 | 22800 | L363 (1.5+), Nalm6 (+/-), RS411 (-) |
| CD22, BCMA | 21986 | 22801 | L363 (+), Nalm6 (+/-), RS411 (-) |
| CD22, BCMA | 21987 | 22802 | L363 (+), Nalm6 (+/-), RS411 (-) |
| BCMA, BCMA | 21988 | 22803 | L363 (+), Nalm6 (-), RS411 (-) |
| BCMA, BCMA | 21989 | 22804 | L363 (+), Nalm6 (-), RS411 (-) |
| BCMA, BCMA | 21990 | 22805 | L363 (+), Nalm6 (-), RS411 (-) |
| BCMA, BCMA | 21991 | 22806 | L363 (1.5+), Nalm6 (-), RS411 (-) |
| CD22, PSMA | 21992 | 22807 | Raji (+/-), Nalm6 (-), LNCaP (2+) |
| CD22, CD22 | 21993 | 22808 | Raji (+/-) |
| BCMA, CD38 | 21994 | 22809 | L363 (+) |
| CD22, CD38 | 21995 | 22810 | Raji (+), L363 (-) |
| BCMA, CD38 | 21996 | 22811 | L363 (+) |
| CD38, BCMA | 21997 | 22812 | Self Activation (SA), L363 (+), RS411 (-) |
| CD38, BCMA | 21998 | 22813 | L363 (+) |
| CD38, CD38 | 21999 | 22814 | L363 (-), RS411 (-) |
| CD38, CD38 | 22000 | 22815 | L363 (-), RS411 (-) |
| CD22, BCMA | 22001 | 22816 | Raji (+/-), L363 (-) |
| BCMA | 22002 | 22817 | L363 (+), U266 (+) |
| CD22, BCMA | 22003 | 22818 | Raji (+), Nalm6 (+/-), Daudi (+/-), L363 (+), |
| CD22, BCMA | 22004 | 22819 | Raji (+), Nalm6 (+/-), Daudi (+/-), L363 (+) |
| CEA | 22005 | 22820 | HL60 (-), LoVo (+/-) |
| CD20 | 22006 | 22821 | Raji (+), Nalm6 (-) |
| CD22, CEA | 22007 | 22822 | Raji (+/-), Nalm6 (-), RS411 (-), LoVo (+/-) |
| CD22, CD38 | 22008 | 22823 | Raji (+/-), Nalm6 (-), L363 (-), RS411 (-) |
| CD38, CD38 | 22009 | 22824 | L363 (+/-) |
| CD38, CD38 | 22010 | 22825 | L363 (-) |
| CD22, CD22 | 22011 | 22826 | Raji (+/-), Nalm6 (-), RS411 (-) |
| CD38, BCMA | 22012 | 22827 | L363 (+), RS411 (+/-) |
| CD22, CD38 | 22013 | 22828 | Raji (-), Nalm6 (-), L363 (-), RS411 (-) |
| BCMA, CD38 | 22014 | 22829 | L363 (+/-), RS411 (-) |
| CD38, BCMA | 22015 | 22830 | L363 (-), RS411 (-) |
| CD38, BCMA | 22016 | 22831 | L363 (+/-), RS411 (-) |
| CD22, PSMA | 22017 | 22832 | Raji (+/-), Nalm6 (-), RS411 (-), LNCaP (-) |
| CEA, PSMA | 22018 | 22833 | LNCaP (+/-), LoVo (+/-) |
| CD22, BCMA | 22019 | 22834 | Raji (+), Nalm6 (+/-), L363 (+), RS411 (-) |
| CD22, CD22 | 22020 | 22835 | Raji (+/-), Nalm6 (+/-), L363 (-) |
| CD22, BCMA | 22021 | 22836 | Raji (+/-), Nalm6 (+/-), L363 (+/-) |
| PSMA, PSMA | 22022 | 22837 | LNCaP (1.5+) |
| CEA | 22023 | 22838 | LoVo (-) |
| CD22, CD22 | 22024 | 22839 | Raji (-), Nalm6 (-), L363 (-) |
| CD22, BCMA | 22025 | 22840 | Raji (+/-), Nalm6 (-), L363 (+) |
| CD22, PSMA | 22026 | 22841 | Raji (-), Nalm6 (-), L363 (-), LNCaP (-) |
| CD22, PSMA | 22027 | 22842 | Raji (-), Nalm6 (-), L363 (-), LNCaP (-) |
| CD22, PSMA | 22028 | 22843 | Raji (-), Nalm6 (-), L363 (-), LNCaP (-) |
| CD22, PSMA | 22029 | 22844 | Raji (+/-), Nalm6 (-), L363 (+), LNCaP (-) |
| CD22, PSMA | 22030 | 22845 | Raji (-), Nalm6 (-), L363 (-), LNCaP (-) |
| CD22, PSMA | 22031 | 22846 | Raji (+/-), Nalm6 (-), L363 (-), LNCaP (-) |
| CD22, PSMA | 22032 | 22847 | Raji (+/-), Nalm6 (-), L363 (-), LNCaP (-) |
| CD22, PSMA | 22033 | 22848 | Raji (+/-), Nalm6 (-), L363 (-), LNCaP (-) |
| CD22, BCMA | 22034 | 22849 | Raji (+/-), Nalm6 (-), L363 (+/-) |
| CD22, CD22 | 22035 | 22850 | Raji (+/-), Nalm6 (+/-), L363 (+/-) |
| CD22 | 22036 | 22851 | Raji (-), Nalm6 (-), Daudi (-) |
| CD22, BCMA | 22037 | 22852 | Raji (-), Nalm6 (-), L363 (-) |
| CD22, BCMA | 22038 | 22853 | Raji (-), Nalm6 (-), L363 (-) |
| CD22, BCMA | 22039 | 22854 | Raji (-), Nalm6 (-), L363 (-) |
| CD22, BCMA | 22040 | 22855 | Raji (-), Nalm6 (-), L363 (-) |
| CD22, BCMA | 22041 | 22856 | Raji (+), Nalm6 (-), L363 (+) |
| CD22, PSMA | 22042 | 22857 | Raji (-), Nalm6 (-), LNCaP (+/-) |
| CD22, PSMA | 22043 | 22858 | Raji (+/-), Nalm6 (-), LNCaP (+) |
| CD22, PSMA | 22044 | 22859 | Raji (-), Nalm6 (-), LNCaP (+/-) |
| CD22, PSMA | 22045 | 22860 | Raji (-), Nalm6 (-), LNCaP (+/-) |
| CD22, BCMA | 22046 | 22861 | Raji (2+), Nalm6 (+/-), L363 (+) |
| CD22, BCMA | 22047 | 22862 | Raji (1.5+), Nalm6 (+/-), L363 (+/-) |
| BCMA, BCMA | 22048 | 22863 | Raji (+), Nalm6 (-), L363 (+/-) |
| CD22, BCMA | 22049 | 22864 | Raji (1.5+), Nalm6 (+/-), L363 (+/-) |
| BCMA, BCMA | 22050 | 22865 | Raji (+), Nalm6 (-), L363 (+/-) |
| CD22, BCMA | 22051 | 22866 | Raji (+/-), Nalm6 (-), L363 (+/-) |
| CD22, BCMA | 22052 | 22867 | Raji (+/-), Nalm6 (-), L363 (+/-) |
| CD22, PSMA | 22053 | 22868 | Raji (-), Nalm6 (-), LNCaP (+) |
| CD22, PSMA | 22054 | 22869 | Raji (-), Nalm6 (-), LNCaP (-) |
| CD22, PSMA | 22055 | 22870 | Raji (+/-), Nalm6 (-), LNCaP (+) |
| CD22, PSMA | 22056 | 22871 | Raji (+/-), Nalm6 (-), LNCaP (-) |
| CD22, PSMA | 22057 | 22872 | Raji (+/-), Nalm6 (-), LNCaP (+) |
| CD22, PSMA | 22058 | 22873 | Raji (+/-), LNCaP (+) |
| CD22, PSMA | 22059 | 22874 | Raji (+/-), LNCaP (+) |
| CD22, BCMA | 22060 | 22875 | Raji (2+), Nalm6 (+/-), L363 (2+) |
| BCMA, BCMA | 22061 | 22876 | L363 (2+) |
| BCMA, BCMA | 22062 | 22877 | L363 (+/-) |
| BCMA, BCMA | 22063 | 22878 | L363 (+/-) |
| BCMA, BCMA | 22064 | 22879 | L363 (+/-) |
| BCMA, BCMA | 22065 | 22880 | L363 (-) |
| CD22, BCMA | 22066 | 22881 | Raji (+/-), Nalm6 (+/-), L363 (+/-), RS411 (-) |
| CD22, BCMA | 22067 | 22882 | Raji (+/-), Nalm6 (+/-), L363 (+), RS411 (-) |
| CD22, BCMA | 22068 | 22883 | Raji (+), Nalm6 (+/-), L363 (+), RS411 (+/-) |
| CD22, BCMA | 22069 | 22884 | Raji (+), Nalm6 (-), L363 (-), RS411 (-) |
| BCMA, CD19 | 22070 | 22885 | Raji (+/-), Nalm6 (+/-), L363 (+/-), RS411 (-) |
| BCMA, BCMA | 22071 | 22886 | L363 (2.5+), RS411 (-) |
| CD22, BCMA | 22072 | 22887 | Raji (+), Nalm6 (+/-), L363 (+), RS411 (-) |
| CD22, BCMA | 22073 | 22888 | Raji (+/-), Nalm6 (-), L363 (+/-), RS411 (-) |
| BCMA, BCMA | 22074 | 22889 | L363 (2.5+), RS411 (-) |
| BCMA, BCMA | 22075 | 22890 | L363 (+/-), RS411 (-) |
| CD22, BCMA | 22076 | 22891 | Raji (+/-), Nalm6 (-), L363 (+) |
| CD22, BCMA | 22077 | 22892 | Raji (-), Nalm6 (-), L363 (-) |
| CD22, BCMA | 22078 | 22893 | Raji (+/-), Nalm6 (-), L363 (+/-) |
| PSMA, PSMA | 22079 | 22894 | LNCaP (+/-) |
| CD22, BCMA | 22080 | 22895 | Raji (+), Nalm6 (-), L363 (1.5+) |
| PSMA, PSMA | 22081 | 22896 | LNCaP (3.5+) |
| CD22, CD22 | 22082 | 22897 | Raji (+/-), Nalm6 (-), L363 (-) |
| CD22, BCMA | 22083 | 22898 | Raji (+/-), Nalm6 (-), L363 (+/-) |
| CD22, CD22 | 22084 | 22899 | Raji (+/-), Nalm6 (-), L363 (-) |
| CD22, BCMA | 22085 | 22900 | Raji (+/-), Nalm6 (-), L363 (1.5+) |

### BISPECIFIC AND BIPARATOPIC SARs ON SIR, AABD-TCR and TCR BACKBONES

Another panel of constructs were generated comprising different antigen binding domain, linkers and TCR constant chains and tested in JNG assay (**Table 45**). The constructs represented by SEQ ID NO: 22096 and 22098 showed the best activity in this group. Both these constructs encoded an FHVH attached to a truncated TCRg chain (TCRg-6MD; SEQ ID NO: 1132) via an IgCL linker (SEQ ID NO: 1142) and a second FHVH attached to a truncated TCRd chain (TCRd-6MD; SEQ ID NO: 1139) via an IgG1-CH1 linker (SEQ ID NO: 1143). The construct with SEQ ID NO: 22099 also showed good activity. This construct comprises an encoded FHVH attached to a truncated TCRa chain (TCRa-wt2-opt-6MD; SEQ ID NO: 1112) via an IgCL linker (SEQ ID NO: 1142) and a second FHVH attached to a truncated TCRd chain (TCRb-wt2-opt-6MD; SEQ ID NO: 1126) via an IgG1-CH1 linker (SEQ ID NO: 1143).

The construct in which the vHH or FHVH or Centyrin or affibody or other non-immunoglobulin antigen binding domain are operably linked to the vL and the vH fragment which are then operably linked via Ig linkers (e.g., IgCL1 and IgG1-CH1) to the truncated TCR constant chains belonging to TCRα, β, γ or δ (i.e., TCRg-6MD, TCRd-6MD, TCRa-wt2-opt-6MD and TCRb-wt2-opt-6MD) retained their activity against two or more antigen targets.

The constructs in which the encoded IgG1-CH1 linker is replaced by other Ig linkers listed in **Table 13** are also active. The constructs in which the encoded FHVH domains are switched for other AABD (e.g., vHH, centyrin, Rzip, E4, CD16 V158 etc.) are also active. The constructs in which encoded multiple AABD are joined in tandem via short optional linkes and then joined in frame to the truncated TCR constant chains (e.g., SEQ ID NO: 1126, 1132, 1139, 1112) via Ig linkers are also tested in JNG cells and show good signaling activity. The constructs with long linker domains (i.e., encoding >25 amino acid residues) between the AABD and the truncated TCR constant chains show stronger signaling as compared to the constructs with short linkers. The constructs that encode for long linkers that are between 50-150 amino acids in length show superior activity. The constructs in which an AABD is attached to a truncated TCR chain (e.g., TCRb-wt2-opt-6MD, TCRa-wt2-opt-6MD, TCRg-6MD, TCRd-6MD etc.) via an Ig linker or a long linker are collectively designated AABD-TCR.

The above results demonstrate that attachment of an AABD does not interfere with the ability of the vL fragment to combine with its complementary vH fragment to form a Fv that can recognize the target antigen and induce cell signaling when the vL and vH fragments are each attached to a truncated TCR constant chain via an Ig linker or a long linker. The above results further demonstrate that an AABD (e.g., a vHH or SVH) which is operably linked to the N-terminus or near the N-terminus of a vL or vH fragment retains its ability to bind to its target antigen and induce T cell signaling when expressed as part of a SAR. Taken collectively, these surprising results demonstrate that attachment of an AABD to the encoded N-terminus of vL or vH fragment of a SAR does not interfere with the assembly of a functional Fv capable of binding the target antigen and inducing T cell signaling.

**TABLE 45: BISPECIFIC AND BIPARATOPIC SARs ON SIR AND AABD, TCR BACKBONE**

| **ANTIGEN** | **SEQ ID NO (DNA)** | **SEQ ID NO (PRT)** | **Constructs Name** | **NFAT-GFP ASSAY** |
|---|---|---|---|---|
| BCMA | 22087 | 22902 | CD8SP-BCMA-FHVH-74-G4S-[hTCRβ-S57C]- F-P2A-IgHSP-BCMA-FHVH-33-GS-[TCRα- wt2-opt-6MD] | Raji (+), Nalm6 (-), L363 (2+) |
| CD19, BCMA | 22088 | 22903 | CD8SP-FMC63-scFv-[hTCRβ-S57C]-F-P2A- SP-BCMA-FHVH-93-GS-[TCRα-wt2-opt- 6MD] | Raji (2+), Nalm6 (2.5+), L363 (3+) |
| CD22, BCMA | 22089 | 22904 | CD8SP-CD22-FHVH-24-GS-[TCRβ-wt2-opt- 6MD]-F-P2A-SP-BCMA-FHVH-93-[hTCRa- T48C-opt]-F-F2A-PAC | Raji (+), Nalm6 (-), L363 (2+) |
| CD22, BCMA | 22090 | 22905 | CD8SP-CD22-FHVH-24-IgG1-Hinge-[TCRβ- wt2-opt-6MD]-F-P2A-SP-BCMA-FHVH-93- [hTCRa-T48C-opt]-F-F2A-PAC | Raji (1.5+), Nalm6 (-), L363 (3+) |
| CD22, BCMA | 22091 | 22906 | CD8SP-CD22-FHVH-24-[hTCRβ-KACIAH]-F- P2A-SP-BCMA-FHVH-93-[hTCRa-T48C-opt]- F-F2A-PAC | Raji (+), Nalm6 (+/-), L363 (1.5+) |
| BCMA | 22092 | 22907 | CD8SP-BCMA-FHVH-74-[hTCRβ-KACIAH]- F-P2A-SP-BCMA-FHVH-33-[hTCRa-T48C- opt]-F-F2A-PAC | Raji (1.5+), Nalm6 (-), L363 (1.5+) |
| CEA, CD19 | 22093 | 22908 | CD8SP-CEA300-aVH-V5-[hTCRβ-KACIAH]- F-P2A-CD8SP-CD19-hu-mROO5-1-MYC- [hTCRa-CSDVP]-F-F2A-PAC | Raji (2+), Nalm6 (+), L363 (-), LoVo (+/-) |
| CD19, PSMA | 22094 | 22909 | CD8SP-FMC63-scFv-V5-[hTCRβ-KACIAH]-F- P2A-IgHSP-PSMA28v2-ChVH-G4Sx2- PSMA71v2-ChVH-MYC-[hTCRa-CSDVP]-F- F2A-PAC | Raji (1.5+), Nalm6 (+), L363 (+/-), LNCaP (+/-) |
| CD19, BCMA | 22095 | 22910 | CD8SP-FMC63-scFv-[hTCRβ-S57C]-F-P2A- SP-BCMA-FHVH-93-[hTCRa-T48C-opt]-F- F2A-PAC | Raji (2+), Nalm6 (2+), L363 (2+) |
| CD22, BCMA | 22096 | 22911 | CD8SP-CD22-FHVH-24-IgCL-TCRg-6MD-F- P2A-IgHSP-BCMA-FHVH-93-IgG1-CH1- TCRd-6MD-F-F2A-PAC | Raji (2+), Nalm6 (+), L363 (3.5+) |
| CD22, BCMA | 22097 | 22912 | CD8SP-CD22-FHVH-24-IgCL-TCRb-wt2-opt- 6MD-F-P2A-Bcu-IgHSP-BCMA-FHVH-93- IgG1-CH1-TCRa-wt2-opt-6MD-F-F2A-PAC | Raji (+), Nalm6 (-), L363 (1.5+) |
| BCMA | 22098 | 22913 | CD8SP-BCMA-FHVH-74-IgCL-TCRg-6MD-F- P2A-Bcu-IgHSP-BCMA-FHVH-33-IgG1-CH1- TCRd-6MD-F-F2A-PAC | L363 (4+) |
| BCMA | 22099 | 22914 | CD8SP-BCMA-FHVH-74-IgCL-TCRb-wt2- opt-6MD-F-P2A-Bcu-IgHSP-BCMA-FHVH- 33-IgG1-CH1-TCRa-wt2-opt-6MD-F-F2A-PAC | L363 (2+) |

### GENERATION OF SAR CONSTRUCTS ON SIR AND Ab-TCR BACKBONES

Another panel of SAR construct are generated on SIR and ab-TCR backbone **(Table 46).** An exemplary double chain bispecific SAR on SIR backbone comprising two chains is CD8SP-CD19-hu-mROO5-1-vL-[hTCRb-S57C]-F-P2A-IgHSP-CD20-vHH2-HCD25-G4S-CD19-hu-mROO5-1-vH-[hTCRa-T48C-opt]-F-F2A-PAC and is represented by SEQ ID NO (DNA): 22104 and SEQ ID NO (PRT): 22919. This SAR construct has the backbone of a SIR, which has been described in WO2018102795 and is incorporated in its entirety by reference herein. One chain of the SAR construct encodes the humanized hu-mROO5-1 vL fragment fused to the constant chain of human TCRb carrying S57C mutation (hTCRb-S57C) and the other chain of the SAR encodes for the humanized hu-mROO5-1 vH fragment fused to the constant chain of human TCRa carrying the T48C mutation (hTCRa-T48C). The hu-mROO5-1 vL and hu-mROO5-1 vH fragments of the SAR together form a Fv targeting CD19. A vHH fragment targeting CD20 (CD20-vHH2-HCD25) is fused to the N-terminus of the hu-mROO5-1 vH fragment via a Glycine-Serine linker. Thus, the SAR targets CD19 via the hu-mROO5-1 Fv and targets CD20 via CD20-USC1-vHH-2HCD26. This construct showed strong activity against RAJI (CD19+/CD20+), RAJI-CD19-KO (CD19+/CD20-), DAUDI (CD19+/CD20+) and RS;411 (CD19+/CD20-) cell lines when expressed in JNG cells and tested using JNG assay. A large panel of additional bispecific SAR constructs were generated in which encoded one AABD is attached to the hu-mROO5-1 vL or the hu-mROO5-1 vH fragments of a double chain SIR and are represented by SEQ ID NO:22104-22119 **(Table 46).** These constructs vary in the type of AABD (e.g., FHVH, vHH, Centyrin etc.), the type of TCR constant chain (e.g., hTCRb-S57C, hTCRb-KACIAH, hTCRa-CSDVP, hTCRa-T48C-opt, hTCRg-d5, hTCRd-d2 etc.), and the nature of the target antigen targeted by the AABD and the Fv (**Table 46**). The exemplary construct with SEQ ID NO: 22109 encodes a BCMA targeted Centyrin as the AABD while the exemplary construct with SEQ ID NO: 22118 encodes a CD20 vHH domain as the AABD. These constructs showed robust activity against a panel of cell lines expressing one or both of their target antigen(s) while showing lack of non-specific activity against cell lines lacking the expression of both of their target antigens (**Table 46**).

The above results demonstrate that attachment of an AABD does not interfere with the ability of the vL fragment to combine with its complementary vH fragment to form a Fv that can recognize the target antigen and induce cell signaling when the vL and vH fragments are each attached to a TCR constant chain. The above results further demonstrate that an AABD (e.g., a vHH or SVH) which is operably linked to the N-terminus or near the N-terminus of a vL or vH fragment retains its ability to bind to its target antigen and induce T cell signaling when expressed as part of a SAR with the backbone of a SIR. Taken collectively, these surprising results demonstrate that attachment of an AABD to the encoded N-terminus of vL or vH fragment of a SAR does not interfere with the assembly of a functional Fv capable of binding the target antigen and inducing T cell signaling.

A panel of trispecific SAR constructs on the hu-mROO5-1 SIR backbone were next generated and are represented by SEQ ID NO: 22123-22139. These constructs vary in the type of encoded AABD (e.g., FHVH, vHH, Centyrin etc.), the type of TCR constant chain (e.g., hTCRb-S57C, hTCRb-KACIAH, hTCRa-CSDVP, hTCRa-T48C-opt, hTCRg-d5, hTCRd-d2 etc.), and the nature of the target antigen targeted by the AABD and the Fv (**Table 46**).

An exemplary double chain trispecific SAR comprising two chains is CD8SP-CD20-VHH-2HCD26-USC1-G4S-hu-mROO5-vL-[hTCRb-S57C]-F-P2A-IgHSP-CD20-vHH2-HCD25-G4S-CD19-hu-mROO5-1-vH-[hTCRa-T48C-opt]-F-F2A-PAC and is represented by DNA SEQ ID NO: 22136 and PRT SEQ ID NO: 22951. Another exemplary double chain trispecific SAR with this design is CD8SP-BCMA-FHVH-93-G4S-hu-mROO5-vL-[hTCRb-KACIAH]-F-P2A-IgHSP-CD20-vHH2-HCD25-G4S-CD19-hu-mROQO5-1-vH-[hTCRa-CSDVP]-F-F2A-PAC (SEQ ID NO: 22123). This SAR construct has the backbone of a SIR. The encoded hu-mROO5-1 vL and hu-mROO5-1 vH fragments of the SIR dimerize to form a Fv targeting CD19. A FHVH fragment targeting BCMA is fused to the N-terminus of the hu-mROO5-1 vL fragment via a Glycine-Serine (G4S) linker. A vHH fragment targeting CD20 (CD20-vHH2-HCD25) is fused to the N-terminus of the hu-mROO5-1 vH fragment via a Glycine-Serine (GS) linker. Thus, the SAR targets CD19 via the hu-mROO5-1 Fv, targets BCMA via BCMA FHVH-93 and targets CD20 via CD20-vHH2-HCD25. These constructs showed robust activity against a panel of cell lines expressing one or both of their target antigen(s) while showing lack of non-specific activity against cell lines lacking the expression of both of their target antigens (**Table 46**).

A panel of double chain bispecific and trispecific constructs were also constructed on the backbone of double chain Ab-TCR and are represented by SEQ ID NO: 22120-22122 and 22140-22141, respectively. A representative SAR with the backbone of an Ab-TCR is CD8SP-CD19-hu-mRO05-1-vL-IgCL-TCRb-wt2-opt-6NM-F-P2A-IgHSP-CD20-vHH2-HCD25-G4S-CD19-hu-mROO5-1-vH-IgG1-CH1-TCRa-wt2-opt-6MD-F-F2A-PAC (SEQ ID NO: 22120). One chain of this SAR construct encodes the humanized hu-mROO5-1 vL fragment fused to the constant chain of truncatetd TCRb (TCRb-wt2-opt-6MD) via an IgCL linker and the other chain of the SAR encodes the humanized hu-mROO5-1 vH fragment fused to the constant chain of truncated human TCRa chain (TCRa-wt2-opt-6MD) via an IgG1-CH1 linker. The encoded hu-mROO5-1 vL and hu-mROO5-1 vH fragments of the SAR together form a Fv targeting CD19. A vHH fragment targeting CD20 (CD20-vHH2-HCD25) is fused to the N-terminus of the hu-mROO5-1 vH fragment via a Glycine-Serine linker. Thus, the SAR targets CD19 via the hu-mROO5-1 Fv and targets CD20 via CD20-USC1-vHH-2HCD26. These constructs showed robust activity against a panel of cell lines expressing one, two or three of their target antigen(s) while showing lack of non-specific activity against cell lines lacking the expression of either of their target antigens (**Table 46**).

The expression of different antigens on the panel of cell lines can be determined by reference to **Table C-1 and C-2.**

Another panel of bispecific and trispecific SAR constructs were generated on the backbone of double chain SIRs or double chain Ab-TCR comprising vL and vH fragments derived from a CD22 targeted humanized antibody huRFB4, a MPL targeted humanized antibody hu-161-2, a BCMA targeted humanized and optimized antibody BCMA-hu72, and a CD20 targeted antibody CD20-2F2. These constructs and are represented by SEQ ID NO: 22144-22147, 22148-22150, 22151-22155, 22156-22159. These constructs showed robust activity against a panel of cell lines expressing one, two or all three of their target antigen(s) while showing lack of non-specific activity against cell lines lacking the expression of either of their target antigens (**Table 46**).

Another panel of bispecific and trispecific SAR constructs were generated on the backbone of CD19-targeted hu-mROO5-1 based double chain SIR targeting additional antigens expressed on blood cancers (e.g., CD123) and solid tumors (e.g., IL13Ra2, Muc16 etc.). These constructs are represented by SEQ ID NO: 22164-22172 and showed showed robust activity against a panel of cell lines expressing their target antigen(s) while showing lack of non-specific activity against cell lines lacking the expression of either of their target antigens (**Table 46**).

Another bispecific SAR construct was generating targeting CD19 and PD1 (SEQ ID NO: 22162) and showed robust activity against Raji, Nalm6 and RS;411 cell lines (**Table 46**).

Another bispecific SAR construct was generating targeting CD19 and human serum albumn (SEQ ID NO: 22163). In this construct a vHH targeting human serum albumin (HAS) is attached to vL fragment of hu-mROO5-1 via a MMP14X3 protease cleavable linker. This construct showed a dose dependent reduction in activity upon addition of human serum albumin (**Table 46**).

Another panel of SAR construct was generated comprising the Fc binding domain of high affinity variant of CD16A, the human Fc receptor (SEQ ID NO: 22170-22173). The constructs were expressed in JNG cells and tested for GFP induction upon co-culture with their target antigen expressing cell lines. In addition, the experiment was repeated in the presence and absence of Herpceptin (a Her2 antibody) and/or Rituximab (a CD20 antibody). The construct with SEQ ID NO: 22170 showed robust GFP induction upon co-culture with RAJI cells and lack of non-specific GFP induction upon co-culture with RAJI-CD19-KO cells. Furthermore, JNG cells expressing this SAR construct showed robust GFP induction when co-cultured with SKOV-3 (Her2+/CD19-) cells in the presence of Hercepin (1µg/ml) **(Table 46).** Essentially similar results were obtained with JNG cells expressing the SAR constructs with SEQ ID NO: 22173-22176. JNG cells expressing the CD19 and CD16 V158 expressing SAR construct with SEQ ID NO: 22172 showed robust GFP induction with CD19-expressing Raji cells but lack of GFP induction with RAJI-CD19-KO (CD19-ve) cells. However, it showed robust GFP induction when co-cultured with RAJI-CD19-KO cells in the presence of Rituximab which targets CD20 antigen **(Table 46).** Taken together the above results demonstrate the ability of the SAR constructs to have bispecific, trispecific and universal capabilities. The UNICON constructs (e.g., SEQ ID NO: 22170-22176) can target some antigens constitutively and other antigens conditionally only in the presence of added antibodies, thereby providing a highly flexible and adaptable platform for adoptive cell therapy that combines convenience and reduced cost with flexibility and improved safety and efficacy.

An exemplary trispecific SAR construct targeting CD19 and CD20 and coexpressing the ligand binding domain of NKG2D is represented by SEQ ID NO: 22177 and showed robust activity against CD19 expressing Raji and Nalm6 cells as well as against NKG2D-ligand expressing SKOV-3 cells **(Table 6).**

Exemplary bispecific and trispecific constructs targeting PSMA along with one or more hematopoietic antigens (e.g., CD19, CD20 etc.) are represented by SEQ ID NO: 22178-22190 and showed robust GFP induction activity against Raji (CD19⁺/CD20⁺/PSMA⁻) and LNCaP (CD19⁻/CD20⁻/PSMA⁺) cells.

Exemplary bispecific and trispecific SAR constructs comprising Cltx (chlorotoxin) attached to the backbone of a CD19 targeted SIR are represented by SEQ ID NO: 22191-22193. Cltx targets small conductance chloride channel expressed on certain cancer cells. These constructs showed robust GFP induction upon co-culture with Raji, Nalm6, RS;411 and other cell lines but lack of non-specific activity against Raji-CD19-KO or L363 cells.

Exemplary bispecific SAR constructs targeting CD19 and Her2 are represented by SEQ ID NO: 22195-22197 and showed robust GFP induction activity against Raji (CD19⁺) and SKOV-3 (Her2⁺) cell lines (**Table 46**). The constructs with SEQ ID NO: 22195 and 22197 encodes a Her2 targeting DARPIN as the AABD, while the construct with SEQ ID NO: 22196 encodes a Her2 targeting vHH domain.

An exemplary bispecific SAR constructs targeting CD19 and Her3 are represented by SEQ ID NO: 22198 and showed robust GFP induction activity against Raji (CD19⁺), MCF-7 (Her3⁺) and SKOV-3 (Her3⁺) cell lines (**Table 46**)

Exemplary bispecific and trispecific constructs on the backbone of a γδ TCR are represented by SEQ ID NO: 22201 and 22202. In the construct CD8SP-BCMA-FHVH-93-G4S-TCR-Vg9-F-P2A-IgHSP-CD20-vHH2-HCD25-G4S-TCR-Vd2-F-F2A-PAC (SEQ ID NO: 22201), an encoded BCMA-FHVH is fused in frame to the N-terminus end of Vg9 variable domain via a G4S linker and an encoded CD20-vHH2-HCD25 fragment is attached to the N-terminus of Vd2 variable domain via a G4S linker. This construct showed robust induction of GFP upon exposure to Raji, Raji-CD19-KO (CD19⁻/BCAM⁺) and L363 (BCMA⁺) cells, thereby demonstrating the ability to generate trispecific SAR on the backbone of a TCR. Similar results were obtained with the bispecific SAR represented by SEQ ID NO: 22202 that targets CD20 as one of its antigens.

Several SAR constructs with hybrid chains also showed activity and are represented by SEQ ID NO: 22203-22207. The construct CD8SP-BCMA-FHVH-93-G4S-hu-mROO5-vL-[hTCRb-KACIAH]-F-P2A-IgHSP-CD20-vHH2-HCD25-G4S-Her2-huMab4D5-D98W-vH-[hTCRa-CSDVP]-F-F2A-PAC with SEQ ID NO: 22203 has the backbone of a SIR and encodes for a vL fragment from CD19 targeted hu-mROO5-1 and a vH fragment from Her2 specific Her2-huMab4D5-D98W. The JNG cells expressing this construct were able to respond to Raji and L363 cells via GFP induction through its BCMA-FHVH-93 and CD20-vHH2-HCD25 domains although they failed to respond to Her2-expressing SKOV-3 cells. These results demonstrate that a functional Fv fragment targeting CD19 and/or Her2 is not being formed as the complementary hu-mROO5-1 vH and Her2-huMab4D5-D98W-vL chains are missing in this construct. Nevertheless, the expressed hu-mROO5-vL and Her2-huMab4D5-D98W vH fragments serve as scaffold for the attachment of the BCMA-FHVH-93 and CD20-vHH2-HCD25 domains. Thus, a funcational bispecific and/or trispecific SAR on the backbone of a double chain SIR can be generated with vL and vH fragments that are derived from different antibodies.

Similarly, the SAR construct MC7G5-TCRb-Vb-[hTCRb-S57C]-F-P2A-IgHSP-CD20-vHH2-HCD25-G4S-CD19-hu-mROO5-1-vH-[hTCRa-T48C-opt]-F-F2A-PAC (SEQ ID NO: 22205) comprised a Vb fragment from MC.7.G5 TCR and a vH fragment from hu-mROO5-1 antibody but was able to show robust GFP induction activity (Table 46) when expressed in JNG cells upon exposure to Raji cells which were bound by the CD20-vHH2-HCD25 domain. Thus, a funcational bispecific and/or trispecific SAR on the backbone of a double chain SIR can be generated with one variable fragment (e.g., Va, Vb, Vg or Vd) derived from a TCR and a second variable fragment (e.g., vL or vH) derived form an antibody.

Similarly, the SAR construct CD8SP-BCMA-FHVH-93-G4S-hu-mROO5-vL-[hTCRb-S57C]-F-P2A-IgHSP-CD20-VHH-USC1-2HCD26-G4S-AFP-Va-14-3-AI-[hTCRa-T48C]-F-F2A-PAC (SEQ ID NO: 22206) encodes for a hu-mROO5-vL fragment from a CD19-targeted antibody and an AFP-targeted variable fragment (Va) from a TCR. This construct showed robust GFP induction activity **(Table 46)** upon exposure to Raji cells which were bound by the CD20-vHH2-HCD25 domain. These results further demonstrate that a funcational bispecific and/or trispecific SAR on the backbone of a double chain SIR can be constructed with one variable fragment derived from a TCR and a second variable fragment derived form an antibody.

The construct CD8SP-CD19-hu-mROO5-1-vL-IgCL-TCRg-6MD-F-P2A-SP-CD19-hu-mROO5-1-vH-hTCR-delta-F-F2A-PAC (SEQ ID NO: 22207) encodes for hu-mROO5-1 attached to a truncated TCRg-6MD fragment via an IgCL linker and a hu-mROO5-1-vH fragment attached to a full-length hTCR-delta constant chain without an intervening linker. This SAR construct showed robust GFP induction in response to Raji cells (Table 46) thereby demonstrating that a TCR Ig domain can substitute for an Ig domain (i.e., IgCL, IgG1-CH1 etc.) derived from an antibody for the generation of a functional SAR. This SAR construct represents a hybrid between a SIR and an Ab-TCR.

**TABLE 46: Jurkat NFAT GFP ASSAY USING, BISPECIFIC AND MULTISPECIFIC SAR CONSTRUCTS**

| **.Target** | **SE Q ID N O (D N A)** | **SE Q ID NO (PR T)** | **Name of Construct** | **Jurkat-NFAT-GFP (JNG) ASSAY** |
|---|---|---|---|---|
| CD19 | 22 10 1 | 229 16 | CD8SP-FMC63-vL-hTCRb-S57C-opt-F-P2A- SP-FMC63-vH-[hTCRa-T48C-opt]-F-F2A- PAC | Raji (2. 5+), Raji-CD19-KO (-), Nalm6 (3+) |
| CD19 | 22 10 2 | 229 17 | CD8SP-FMC63-vL-[hTCRb-KACIAH]-F- P2A-IgHSP-FMC63-vH-[hTCRa-CSDVP]-F- F2A-PAC | Raji (3.5+), Raji-CD19-KO (-) |
| CD19 | 22 10 3 | 229 18 | CD8SP-CD19-hu-mROO5-1-vL-IgCL-TCRg- 6MD-F-P2A-SP-CD19-hu-mROO5-1-vH- IgG1-CH1-TCRd-6MD-F-F2A-PAC | Raji (4+), Raji-CD19-KO (-) |
| CD19, CD22 | 22 10 4 | 229 19 | CD8SP-CD19-hu-mROO5-1-vL-[hTCRb- S57C]-F-P2A-IgHSP-CD20-vHH2-HCD25- G4S-CD19-hu-mROO5-1-vH-[hTCRa-T48C- opt]-F-F2A-PAC | Raji (5+), Raji-CD19-KO (5+), Daudi (6+), RS411 (5+) |
| CD19, CD20 | 22 10 5 | 229 20 | CD8SP-CD19-hu-mROO5-1-vL-[hTCRb- S57C]-F-P2A-IgHSP-CD20-vHH2-HCD25- G4S-CD19-hu-mROO5-1-vH-[hTCRa-T48C- opt]-F-F2A-PAC | Raji (2.5+), Raji-CD19-KO (4+), Nalm6 (5+) |
| CD19, CD20 | 22 10 6 | 229 21 | CD8SP-CD19-hu-mROO5-1-vL-[hTCRb- KACIAH]-F-P2A-IgHSP-CD20-vHH2- HCD25-G4S-CD19-hu-mROO5-1-vH- [hTCRa-CSDVP]-F-F2A-PAC | Raji (5+), Raji-CD19-KO (6+), L363 (-), RS411 (+) |
| CD19, CD20 | 22 10 7 | 229 22 | CD8SP-CD19-hu-mROO5-1-vL-[hTCRa- CSDVP]-F-F2A-IgHSP-CD20-vHH2-HCD25- G4S-CD19-hu-mROO5-1-vH-[hTCRb- KACIAH]-F-P2A-PAC | Raji (4+), Raji-CD19-KO (5+), L363 (-), RS411 (+) |
| BCMA , CD19 | 22 10 8 | 229 23 | CD8SP-BCMA-FHVH-74-G4S-hu-mROO5- vL-[hTCRb-S57C]-F-P2A-SP-CD19-hu- mROO5-1-vH-[hTCRa-T48C-opt]-F-F2A- PAC | Raji (4+), Raji-CD19-KO (3+), Nalm6 (7+), L363 (3.5+), RS411 (5+), HL60 (-), MOLM-13 (-), THP-1 (-) |
| BCMA , CD19 | 22 10 9 | 229 24 | CD8SP-BCMA-Centyrin-G4S-hu-mROO5- vL-[hTCRb-S57C]-F-P2A-SP-CD19-hu- mROO5-1-vH-[hTCRa-T48C-opt]-F-F2A- PAC | Raji (2+), Raji-CD19-KO (+/-), Nalm6 (2+), L363 (+/-), RS411 (4+) |
| CD22, CD19 | 22 11 0 | 229 25 | CD8SP-CD22-FHVH-24-G4S-hu-mROO5- vL-[hTCRb-S57C]-F-P2A-SP-CD19-hu- mROO5-1-vH-[hTCRa-T48C-opt]-F-F2A- PAC | Raji (5+), Raji-CD19-KO (4+), Nalm6 (-), L363 (-), U266 (-), RS411 (4+) |
| BCMA , CD19 | 22 11 1 | 229 26 | CD8SP-BCMA-FHVH-33-G4S-hu-mROO5- vL-[hTCRb-S57C]-F-P2A-SP-CD19-hu- mROO5-1-vH-[hTCRa-T48C-opt]-F-F2A- PAC | Raji (4+), Raji-CD19-KO (3+), Nalm6 (2+), L363 (3+), U266 (-), RS411 (4+), HL60 (-), MOLM-13 (-), THP-1 (-) |
| CD19, BCMA | 22 11 2 | 229 27 | CD8SP-CD19-hu-mROO5-1-vL-[hTCRb- S57C]-F-P2A-IgHSP-BCMA-917-vHH-E59D- G4S-CD19-hu-mROO5-1-vH-[hTCRa-T48C- opt]-F-F2A-PAC | Raji (6.5+), Raji-CD19-KO (5+), Nalm6 (6+), L363 (4.5+), RS411 (4.5+) |
| CD19, CD20 | 22 11 3 | 229 28 | CD8SP-CD19-hu-mROO5-1-vL-[hTCRb- KACIAH]-F-P2A-IgHSP-CD20-vHH2- HCD25-G4S-CD19-hu-mROO5-1-vH- [hTCRa-CSDVP]-F-F2A-PAC | Raji (5+), Raji-CD19-KO (4+), Nalm6 (4.5+), Daudi (4.5+), RS411 (4.5+) |
| CD19, CD20 | 22 11 4 | 229 29 | CD8SP-CD19-hu-mROO5-1-vL-[hTCRa- CSDVP]-F-F2A-IgHSP-CD20-vHH2-HCD25- G4S-CD19-hu-mROO5-1-vH-[hTCRb- KACIAH]-F-P2A-PAC | Raji (6+), Raji-CD19-KO (5+), Nalm6 (6+), Daudi (6+), RS411 (5+) |
| CD19, BCMA | 22 11 5 | 229 30 | CD8SP-CD19-hu-mROO5-1-vL-[hTCRβ- KACIAH]-F-P2A-CD8SP-BCMA-FHVH-74- CD19-hu-mROO5-1-vH-[hTCRa-CSDVP]-F- F2A-PAC | Raji (1.5+), Raji-CD19-KO (1.5+), Nalm6 (2.5+), L363 (2.5+), RS411 (2+) |
| CD19, BCMA | 22 11 6 | 229 31 | CD8SP-CD19-hu-mROO5-1-vL-[hTCRa- CSDVP]-F-F2A-CD8SP-BCMA-FHVH-74- CD19-hu-mROO5-1-vH-[hTCRβ-KACIAH]- F-P2A-PAC | Raji (3+), Raji-CD19-KO (2+), Nalm6 (3+), L363 (3.5+), RS411 (2+) |
| CD19, CD22 | 22 11 7 | 229 32 | CD8SP-CD19-hu-mROO5-1-vL-[hTCRa- CSDVP]-F-F2A-CD8SP-CD22-FHVH-24-SP- CD19-hu-mROO5-1-vH-[hTCRβ-KACIAH]- F-P2A-PAC | Raji (3+), Raji-CD19-KO (2.5+), Nalm6 (1.5+), L363 (-) |
| CD20-CD19 | 22 11 8 | 229 33 | CD8SP-CD20-vHH-C07-G4S-CD19- huCD19USC3-vL-[hTCRβ-S57C]-F-P2A-SP- CD19USC3-vH-[hTCRa-T48C-opt]-F-F2A- PAC | Raji (3+), Raji-CD19-KO (2+), Nalm6 (3+), Daudi (3+), RS411 (3+) |
| CD19, CD20 | 22 11 9 | 229 34 | CD8SP-CD19-hu-mROO5-1-vL-huTCRg-d5- F-P2A-IgHSP-CD20-vHH2-HCD25-G4S- | Raji (4+), Raji-CD19-KO (3+), Nalm6 (5.5+), L363 (-), RS411 (3+) |
| | | | CD19-hu-mROO5-1-vH-huTCRd-d2-F-F2A- PAC | |
| CD19, CD20 | 22 12 0 | 229 35 | CD8SP-CD19-hu-mROO5-1-vL-IgCL-TCRb- wt2-opt-6MD-F-P2A-IgHSP-CD20-vHH2- HCD25-G4S-CD19-hu-mROO5-1-vH-IgG1- CH1-TCRa-wt2-opt-6MD-F-F2A-PAC | Raji (4+), Raji-CD19-KO (3+), Nalm6 (3.5+), L363 (-), RS411 (2.5+) |
| CD19, CD20 | 22 12 1 | 229 36 | CD8SP-CD19-hu-mROO5-1-vL-IgCL-TCRg- 6MD-F-P2A-IgHSP-CD20-vHH2-HCD25- G4S-CD19-hu-mROO5-1-vH-IgG1-CH1- TCRd-6MD-F-F2A-PAC | Raji (5+), Raji-CD19-KO (4+), Nalm6 (7+), L363 (-), RS411 (5.5+) |
| CD22-BCMA | 22 12 2 | 229 37 | CD8SP-CD22-FHVH-24-IgCL-TCRg-6MD- F-P2A-Bcu-IgHSP-BCMA-FHVH-93-IgG1- CH1-TCRd-6MD-F-F2A-PAC | Raji (3.5+), Nalm6 (1.5+), L363 (2.5+) |
| CD19, BCMA , CD20 | 22 12 3 | 229 38 | CD8SP-BCMA-FHVH-93-G4S-hu-mROO5- vL-[hTCRb-KACIAH]-F-P2A-IgHSP-CD20- vHH2-HCD25-G4S-CD19-hu-mROO5-1-vH- [hTCRa-CSDVP]-F-F2A-PAC | Raji (4.5+), Nalm6 (5+), L363 (3+), RS411 (4+), HL60 (-), MOLM-13 (-), THP-1 (-) |
| CD19, CD20, CD22 | 22 12 4 | 229 39 | CD8SP-CD20-VHH-USC1-G4S-CD22-USC1- 160-G4S-hu-mROO5-vL-[hTCRa-CSDVP]-F- F2A-IgHSP-CD20-vHH2-HCD25-G4S-CD19- hu-mROO5-1-vH-[hTCRb-KACIAH]-F-P2A- PAC | Raji (2.5+), Raji-CD19-KO (1.5+), Nalm6 (2+), RS411 (2+) |
| CD20, CD20, CD19 | 22 12 5 | 229 40 | CD8SP-CD20-VHH-2HCD26-USC1-G4S-hu- mROO5-vL-[hTCRb-KACIAH]-F-P2A- IgHSP-CD20-vHH2-HCD25-G4S-CD19-hu- mROO5-1-vH-[hTCRa-CSDVP]-F-F2A-PAC | Raji (4+), Raji-CD19-KO (5+), Nalm6 (2.5+), L363 (-), RS411 (+/-) |
| CD19, CD19, CD20 | 22 12 6 | 229 41 | CD8SP-CD19-FHVH-354-G4S-hu-mROO5- vL-[hTCRb-KACIAH]-F-P2A-IgHSP-CD20- vHH2-HCD25-G4S-CD19-hu-mROO5-1-vH- [hTCRa-CSDVP]-F-F2A-PAC | Raji (5+), Raji-CD19-KO (6+), Nalm6 (5+), L363 (-), RS411 (+/-) |
| BCMA , CD20, CD19 | 22 12 7 | 229 42 | CD8SP-BCMA-Centyrin-G4S-hu-mROO5- vL-[hTCRa-CSDVP]-F-F2A-IgHSP-CD20- vHH2-HCD25-G4S-CD19-hu-mROO5-1-vH- [hTCRb-KACIAH]-F-P2A-PAC | Raji (+), Raji-CD19-KO (+), Nalm6 (+), L363 (+), RS411 (+) |
| BCMA , CD38, CD19 | 22 12 8 | 229 43 | CD8SP-BCMA-FHVH-74-G4Sx2-IE-G4Sx2- CD38-309021-FHVH-G4S-hu-mROO5-vL- [hTCRb-S57C]-F-P2A-SP-CD19-hu-mROO5- 1-vH-[hTCRa-T48C-opt]-F-F2A-PAC | Raji (3+), Raji-CD19-KO (2+), L363 (2+), L363-CD38-KO (2+), RS411 (2+) |
| CD38, CD19, CD20 | 22 12 9 | 229 44 | CD8SP-CD38-USC1-FHVH-32184-G4S-hu- mROO5-vL-[hTCRa-CSDVP]-F-F2A-IgHSP- CD20-vHH2-HCD25-G4S-CD19-hu-mROO5- 1-vH-[hTCRb-KACIAH]-F-P2A-PAC | Raji (+), Raji-CD19-KO (+), Nalm6 (+), L363 (+), RS411 (+) |
| BCMA , CD19, CD20 | 22 13 0 | 229 45 | CD8SP-BCMA-FHVH-93-G4S-hu-mROO5- vL-[hTCRb-KACIAH]-F-P2A-IgHSP-CD20- vHH2-HCD25-G4S-CD19-hu-mROO5-1-vH- [hTCRa-CSDVP]-F-F2A-PAC | Raji (4+), Raji-CD19-KO (5+), Nalm6 (5.5+), L363 (5+), RS411 (4+) |
| BCMA , CD19, CD20 | 22 13 1 | 229 46 | CD8SP-BCMA-FHVH-93-G4S-hu-mROO5- vL-[hTCRb-KACIAH]-F-P2A-IgHSP-CD20- vHH2-HCD25-G4S-CD19-hu-mROO5-1-vH- [hTCRa-CSDVP]-F-F2A-PAC | Raji (4+), Raji-CD19-KO (5+), Nalm6 (6+), L363 (5+), RS411 (3.5+) |
| BCMA , CD19, CD20 | 22 13 2 | 229 47 | CD8SP-BCMA-FHVH-93-G4S-hu-mROO5- vL-[hTCRb-KACIAH]-F-P2A-IgHSP-CD20- VHH-USC1-2HCD26-G4S-CD19-hu- mROO5-1-vH-[hTCRa-CSDVP]-F-F2A-PAC | Raji (4.5+), Raji-CD19-KO (6+), Nalm6 (5+), L363 (4+), RS411 (4+) |
| CD19, CD19, CD20 | 22 13 3 | 229 48 | CD8SP-CD19-FHVH-354-G4S-hu-mROO5- vL-[hTCRb-KACIAH]-F-P2A-IgHSP-CD20- vHH2-HCD25-G4S-CD19-hu-mROO5-1-vH- [hTCRa-CSDVP]-F-F2A-PAC | Raji (2.5+), Raji-CD19-KO (5+), Nalm6 (5.5+) |
| CD38, CD19, CD20 | 22 13 4 | 229 49 | CD8SP-CD38-USC1-FHVH-32184-G4S-hu- mROO5-vL-[hTCRb-KACIAH]-F-P2A- IgHSP-CD20-vHH2-HCD25-G4S-CD19-hu- mROO5-1-vH-[hTCRa-CSDVP]-F-F2A-PAC | Raji (+/-), Raji-CD19-KO (+/-), Raji-CD22-KO (+/), Nalm6 (-), L363 (-), U266 (-), RS411 (-) |
| BCMA , CD19, CD20 | 22 13 5 | 229 50 | CD8SP-BCMA-FHVH-93-G4S-hu-mROO5- vL-[hTCRb-S57C]-F-P2A-IgHSP-CD20- VHH-USC1-2HCD26-G4S-CD19-hu- mROO5-1-vH-[hTCRa-T48C-opt]-F-F2A- PAC | Raji (3.5+), Raji-CD19-KO (2.5+), Nalm6 (2.5+), L363 (2+), U266 (3+), RS411 (2+) |
| CD20, CD19, CD20 | 22 13 6 | 229 51 | CD8SP-CD20-VHH-2HCD26-USC1-G4S-hu- mROO5-vL-[hTCRb-S57C]-F-P2A-IgHSP- CD20-vHH2-HCD25-G4S-CD19-hu-mROO5- 1-vH-[hTCRa-T48C-opt]-F-F2A-PAC | Raji (3+), Raji-CD19-KO (2.5+), Nalm6 (+), L363 (-), U266 (-), RS411 (+) |
| CD38, CD19, CD20 | 22 13 7 | 229 52 | CD8SP-CD38-331-vHH-D64E-G4S-hu- mROO5-vL-[hTCRb-KACIAH]-F-P2A- IgHSP-CD20-vHH2-HCD25-G4S-CD19-hu- mROO5-1-vH-[hTCRa-CSDVP]-F-F2A-PAC | Raji (-), Raji-CD19-KO (-), Nalm6 (-), L363 (-), U266 (-), RS411 (-) |
| CD38, CD19, CD20 | 22 13 8 | 229 53 | CD8SP-CD38-331-vHH-S53E-G4S-hu- mROO5-vL-[hTCRb-KACIAH]-F-P2A- IgHSP-CD20-vHH2-HCD25-G4S-CD19-hu- mROO5-1-vH-[hTCRa-CSDVP]-F-F2A-PAC | Raji (-), Raji-CD19-KO (-), Nalm6 (-), L363 (-), U266 (-), RS411 (-) |
| BCMA , CD20, CD19 | 22 13 9 | 229 54 | CD8SP-BCMA-FHVH-93-G4S-hu-mROO5- vL-huTCRg-d5-F-P2A-IgHSP-CD20-vHH2- HCD25-G4S-CD19-hu-mROO5-1-vH- huTCRd-d2-F-F2A-PAC | Raji (2+), Raji-CD19-KO (1.5+), Nalm6 (1.5+), L363 (1.5+), RS411 (2+) |
| BCMA , CD20, CD19 | 22 14 0 | 229 55 | CD8SP-BCMA-FHVH-93-G4S-hu-mROO5- vL-IgCL-TCRb-wt2-opt-6MD-F-P2A-IgHSP- CD20-vHH2-HCD25-G4S-CD19-hu-mROO5- 1-vH-IgG1-CH1-TCRa-wt2-opt-6MD-F-F2A- PAC | Raji (5+), Raji-CD19-KO (4+), Nalm6 (5+), L363 (2+), RS411 (4+) |
| BCMA , CD19, CD20 | 22 14 1 | 229 56 | CD8SP-BCMA-FHVH-93-G4S-hu-mROO5- vL-IgCL-TCRg-6MD-F-P2A-IgHSP-CD20- vHH2-HCD25-G4S-CD19-hu-mROO5-1-vH- IgG1-CH1-TCRd-6MD-F-F2A-PAC | Raji (4+), Raji-CD19-KO (3.5+), Nalm6 (5.5+), L363 (2.5+), RS411 (4+) |
| BCMA , CD38, CD20, CD19 | 22 14 2 | 229 57 | CD8SP-BCMA-FHVH-74-G4Sx2-IE-G4Sx2- CD38-309021-FHVH-G4S-hu-mROO5-vL- [hTCRa-CSDVP]-F-F2A-IgHSP-CD20-vHH2- HCD25-G4S-CD19-hu-mROO5-1-vH- [hTCRb-KACIAH]-F-P2A-PAC | Raji (+), Raji-CD19-KO (+), Nalm6 (+), L363 (+/), RS411 (+/-) |
| BCMA , CD38, CD19, CD20 | 22 14 3 | 229 58 | CD8SP-BCMA-FHVH-74-G4Sx2-IE-G4Sx2- CD38-309021-FHVH-G4S-hu-mROO5-vL- [hTCRb-KACIAH]-F-P2A-IgHSP-CD20- vHH2-HCD25-G4S-CD19-hu-mROO5-1-vH- [hTCRa-CSDVP]-F-F2A-PAC | Raji (2+), Raji-CD19-KO (1.5+), L363 (2+), L363-CD38-KO (1.5+), RS411 (2+) |
| BCMA , CD22 | 22 14 4 | 229 59 | CD8SP-BCMA-Centyrin-G4S-huRFB4-vL- [hTCRb-S57C]-F-P2A-SP-huRFB4-vH- [hTCRa-T48C-opt]-F-F2A-PAC | Raji (+), Raji-CD22-KO (+/-), Nalm6 (+/-), L363 (+), U266 (3+), RS411 (-) |
| BCMA , CD20, CD22 | 22 14 5 | 229 60 | CD8SP-BCMA-FHVH-93-G4S-huRFB4-vL- IgCL-TCRg-6MD-F-P2A-IgHSP-CD20- vHH2-HCD25-G4S-huRFB4-vH-IgG1-CH1- TCRd-6MD-F-F2A-PAC | Raji (+/-), Raji-CD19-KO (1.5+), Raji-CD22-KO (2.5+), Nalm6 (+), L363 (1.5+), U266 (2+), RS411 (+) |
| BCMA , CD20, CD22 | 22 14 6 | 229 61 | CD8SP-BCMA-FHVH-93-G4S-huRFB4-vL- IgCL-TCRb-wt2-opt-6MD-F-P2A-IgHSP- CD20-vHH2-HCD25-G4S-huRFB4-vH-IgG1- CH1-TCRa-wt2-opt-6MD-F-F2A-PAC | Raji (2+), Raji-CD19-KO (1.5+), Raji-CD22-KO (3+), Nalm6 (+/-), L363 (+), U266 (2+), RS411 (-) |
| BCMA , CD20, CD22 | 22 14 7 | 229 62 | CD8SP-BCMA-FHVH-93-G4S-huRFB4-vL- huTCRg-d5-F-P2A-IgHSP-CD20-vHH2- HCD25-G4S-huRFB4-vH-huTCRd-d2-F-F2A- PAC | Raji (+), Raji-CD22-KO (1.5+), Nalm6 (-), L363 (+), U266 (1.5+), RS411 (-) |
| BCMA , CD20, MPL | 22 14 8 | 229 63 | CD8SP-BCMA-FHVH-93-G4S-hu-161-2-vL- IgCL-TCRg-6MD-F-P2A-IgHSP-CD20- vHH2-HCD25-G4S-hu-161-2-vH-IgG1-CH1- TCRd-6MD-F-F2A-PAC | Raji (3+), Nalm6 (-), L363 (1.5+), U266 (2.5+), RS411 (-), HEL 92.1.7 (+) |
| BCMA , CD20, MPL | 22 14 9 | 229 64 | CD8SP-BCMA-FHVH-93-G4S-hu-161-2-vL- IgCL-TCRb-wt2-opt-6MD-F-P2A-IgHSP- CD20-vHH2-HCD25-G4S-hu-161-2-vH- IgG1-CH1-TCRa-wt2-opt-6MD-F-F2A-PAC | Raji (3+), Nalm6 (-), L363 (+), U266 (3+), RS411 (-), HEL 92.1.7 (+/-) |
| BCMA , CD20, MPL | 22 15 0 | 229 65 | CD8SP-BCMA-FHVH-93-G4S-hu-161-2-vL- huTCRg-d5-F-P2A-IgHSP-CD20-vHH2- HCD25-G4S-hu-161-2-vH-huTCRd-d2-F- F2A-PAC | Raji (+), Nalm6 (-), L363 (+/-), U266 (+), RS411 (-), HEL 92.1.7 (-) |
| BCMA , BCMA | 22 15 1 | 229 66 | CD8SP-BCMA-Centyrin-G4S-BCMA-hu72- vL-S29G-[hTCRb-S57C]-F-P2A-SP-BCMA- hu72-vH-G32S-[hTCRa-T48C-opt]-F-F2A- PAC | L363 (2+), U266 (4+), RS411 (-) |
| BCMA , BCMA | 22 15 2 | 229 67 | CD8SP-BCMA-FHVH-33-G4S-BCMA-hu72- vL-S29G-[hTCRb-S57C]-F-P2A-SP-BCMA- hu72-vH-G32S-[hTCRa-T48C-opt]-F-F2A- PAC | L363 (2+), U266 (4.5+), RS411 (-) |
| BCMA , CD20 | 22 15 3 | 229 68 | CD8SP-BCMA-FHVH-93-G4S-BCMA-hu72- vL-S29G-IgCL-TCRg-6MD-F-P2A-IgHSP- CD20-vHH2-HCD25-G4S-BCMA-hu72-vH- G32S-IgG1-CH1-TCRd-6MD-F-F2A-PAC | Raji (2.5+), Nalm6 (1.5+), L363 (2+), RS411 (-) |
| BCMA , CD20 | 22 15 4 | 229 69 | CD8SP-BCMA-FHVH-93-G4S-BCMA-hu72- vL-S29G-IgCL-TCRb-wt2-opt-6MD-F-P2A- IgHSP-CD20-vHH2-HCD25-G4S-BCMA- hu72-vH-G32S-IgG1-CH1-TCRa-wt2-opt- 6MD-F-F2A-PAC | Raji (4+), Nalm6 (+), L363 (2+), RS411 (-) |
| BCMA , CD20 | 22 15 5 | 229 70 | CD8SP-BCMA-FHVH-93-G4S-BCMA-hu72- vL-S29G-huTCRg-d5-F-P2A-IgHSP-CD20- vHH2-HCD25-G4S-BCMA-hu72-vH-G32S- huTCRd-d2-F-F2A-PAC | Raji (2+), Nalm6 (1.5+), L363 (1.5+), RS411 (-) |
| BCMA , CD20 | 22 15 6 | 229 71 | CD8SP-BCMA-Centyrin-G4S-CD20-2F2-vL- [hTCRb-S57C]-F-P2A-SP-CD20-2F2-vH- [hTCRa-T48C-opt]-F-F2A-PAC | Raji (1.5+), Nalm6 (-), L363 (+), RS411 (-) |
| BCMA , CD20 | 22 15 7 | 229 72 | CD8SP-BCMA-FHVH-33-G4S-CD20-2F2- vL-[hTCRb-S57C]-F-P2A-SP-CD20-2F2-vH- [hTCRa-T48C-opt]-F-F2A-PAC | Raji (+), Nalm6 (+/-), L363 (1.5+), RS411 (-) |
| BCMA , CD20 | 22 15 8 | 229 73 | CD8SP-BCMA-FHVH-93-G4S-CD20-2F2- vL-IgCL-TCRb-wt2-opt-6MD-F-P2A-IgHSP- CD20-vHH2-HCD25-G4S-CD20-2F2-vH- IgG1-CH1-TCRa-wt2-opt-6MD-F-F2A-PAC | Raji (3+), Nalm6 (+), L363 (1.5+), RS411 (-) |
| BCMA , CD20 | 22 15 9 | 229 74 | CD8SP-BCMA-FHVH-93-G4S-CD20-2F2- vL-huTCRg-d5-F-P2A-IgHSP-CD20-vHH2- HCD25-G4S-CD20-2F2-vH-huTCRd-d2-F- F2A-PAC | Raji (3+), Nalm6 (+/-), L363 (+/-), RS411 (-) |
| BCMA , CD20 | 22 16 0 | 229 75 | CD8SP-BCMA-FHVH-93-G4S-HuLuc64-vL- huTCRg-d5-F-P2A-IgHSP-CD20-vHH2- HCD25-G4S-CD19-hu-mROO5-1-vH- huTCRd-d2-F-F2A-PAC | Raji (2+), Raji-CD19-KO (1.5+), Nalm6 (+), L363 (+), U266 (3+), RS411 (-) |
| BCMA , CD20, CD19 | 22 16 1 | 229 76 | CD8SP-BCMA-FHVH-93-G4S-hu-mROO5- vL-[hTCRb-S57C]-F-P2A-IgHSP-CD20- VHH-USC1-2HCD26-G4S-CD19-hu- mROO5-1-vH-[hTCRa-T48C-opt]-F-F2A- PAC | Raji (+/-), Raji-CD19-KO (+/-), Nalm6 (+/-), L363 (-), RS411 (-) |
| CD19, PD1 | 22 16 2 | 229 77 | CD8SP-CD19-hu-mROO5-1-vL-[hTCRb- S57C]-F-P2A-IgHSP-PD1-vHH-USC-707- G4S-CD19-hu-mROO5-1-vH-[hTCRa-T48C- opt]-F-F2A-PAC | Raji (5.5+), Nalm6 (5+), RS411 (2.5+) |
| CD19, CD20, Album in | 22 16 3 | 229 78 | CD8SP-Alb8-vHH-MMP14X3-hu-mROO5- vL-[hTCRb-S57C]-F-P2A-IgHSP-CD20- VHH-USC1-2HCD26-G4S-CD19-hu- mROO5-1-vH-[hTCRa-T48C-opt]-F-F2A- PAC | Raji (4+), Raji-CD19-KO (4.5+), Raji + 5% Human Serum Albumin (2+), Raji + 10% Human Serum Albumin (3+), Raji + 25% Human Serum Albumin (+), |
| CD19, CD123 | 22 16 4 | 229 79 | CD8SP-CD19-hu-mROO5-1-vL-[hTCRb- S57C]-F-P2A-IgHSP-CD123-USC-3-vHH- G4S-CD19-hu-mROO5-1-vH-[hTCRa-T48C- opt]-F-F2A-PAC | **Raji** (6+), L428 (3+) |
| BCMA , CD20, CD19 | 22 16 5 | 229 80 | CD8SP-BCMA-FHVH-93-G4S-hu-mROO5- vL-[hTCRb-S57C]-F-P2A-IgHSP-CD20- VHH-USC1-2HCD26-G4S-CD19-hu- mROO5-1-vH-[hTCRa-T48C-opt]-F-F2A- PAC | **Raji** (+), L363 (+) |
| MUC1 6, CD19, CD20 | 22 16 6 | 229 81 | CD8SP-MUC16-VHH-USC1-R3MU5-G4S- hu-mROO5-vL-[hTCRa-CSDVP]-F-F2A- IgHSP-CD20-vHH2-HCD25-G4S-CD19-hu- mROO5-1-vH-[hTCRb-KACIAH]-F-P2A- PAC | SKOV-3 (2+), Raji (2+), Raji-CD19-KO (2+), Nalm6 (3+), RS411 (2.5+) |
| MUC1 6, CD19, CD20 | 22 16 7 | 229 82 | CD8SP-MUC16-VHH-USC2-R3MU5-G4S- hu-mROO5-vL-[hTCRa-CSDVP]-F-F2A- IgHSP-CD20-vHH2-HCD25-G4S-CD19-hu- mROO5-1-vH-[hTCRb-KACIAH]-F-P2A- PAC | SKOV-3 (1.5+), Raji (+), Raji-CD19-KO (+), Nalm6 (1.5+), RS411 (1.5+) |
| IL13R a2, CD19, CD20 | 22 16 8 | 229 83 | CD8SP-IL13Ra2-vHH-USC2-Cl3-G4S-hu- mROO5-vL-[hTCRa-CSDVP]-F-F2A-IgHSP- CD20-vHH2-HCD25-G4S-CD19-hu-mROO5- 1-vH-[hTCRb-KACIAH]-F-P2A-PAC | U-87 MG (3.5+), Raji (3+), Raji-CD19-KO (2+), Nalm6 (6+), RS411 (5.5+) |
| IL13R A, CD19, CD20 | 22 16 9 | 229 84 | CD8SP-IL13Ra2-vHH-USC1-Cl-G4S-hu- mROO5-vL-[hTCRa-CSDVP]-F-F2A-IgHSP- CD20-vHH2-HCD25-G4S-CD19-hu-mROO5- 1-vH-[hTCRb-KACIAH]-F-P2A-PAC | Raji (3+), Raji-CD19-KO (4+), Nalm6 (4.5+), L363 (-), RS411 (2+), U-87 MG (3+) |
| CD19, Fc | 22 17 0 | 229 85 | CD8SP-CD19-hu-mROO5-1-vL-[hTCRb- S57C]-F-P2A-IgHSP-CD16A-V158-FL-v2- G4S-CD19-hu-mROO5-1-vH-[hTCRa-T48C- opt]-F-F2A-PAC | Raji (6+), Raji-CD19-KO (-), SKOV-3 (-), SKOV-3 + 1µg/mL Herceptin (5.5+) |
| CD19, CD20, Fc | 22 17 1 | 229 86 | CD8SP-CD16A-V158-G4S-hu-mROO5-vL- [hTCRb-KACIAH]-F-P2A-IgHSP-CD20- vHH2-HCD25-G4S-CD19-hu-mROO5-1-vH- [hTCRa-CSDVP]-F-F2A-PAC | Raji (3.5+), Raji-CD19-KO (3+), Nalm6 (5+), RS411 (4+) |
| CD19, Fc | 22 17 2 | 229 87 | CD8SP-CD16A-V158-G4S-hu-mROO5-vL- [hTCRb-S57C]-F-P2A-SP-CD19-hu-mROO5- 1-vH-[hTCRa-T48C-opt]-F-F2A-PAC | Raji (4.5+), Raji-CD19-KO (-), Raji-CD19-KO+ Rituximab (2.5+) |
| CD22, CD20, Fc | 22 17 3 | 229 88 | CD8SP-CD16A-V158-G4S-huRFB4-vL- [hTCRb-KACIAH]-F-P2A-IgHSP-CD20- vHH2-HCD25-G4S-huRFB4-vH-[hTCRa- CSDVP]-F-F2A-PAC | Raji (3+), Raji-CD19-KO (2+), Raji-CD22-KO (3+), Nalm6 (+), L363 (-), SKOV-3 (-), Raji-CD19-KO + Rituximab (2+), SKOV-3 + Herceptin (4+) |
| BCMA , CD19, Fc | 22 17 4 | 229 89 | CD8SP-BCMA-FHVH-93-G4S-hu-mROO5- vL-[hTCRb-S57C]-F-P2A-IgHSP-CD16A- V158-FL-v2-G4S-CD19-hu-mROO5-1-vH- [hTCRa-T48C-opt]-F-F2A-PAC | Raji (3.5+), Raji-CD19-KO (1.5+), Nalm6 (3+), L363 (2.5+), U266 (3+), RS411 (2+), SKOV-3 (-), Raji-CD19-KO + Rituximab (2+), Raji-CD19-KO Herceptin (1.5+), SKOV-3 + Herceptin (4+) |
| CD20, CD19, Fc | 22 17 5 | 229 90 | CD8SP-CD20-VHH-2HCD26-USC1-G4S-hu- mROO5-vL-[hTCRb-S57C]-F-P2A-IgHSP- CD16A-V158-FL-v2-G4S-CD19-hu-mROO5- 1-vH-[hTCRa-T48C-opt]-F-F2A-PAC | Raji (5+), Raji-CD19-KO (4.5+), Nalm6 (4+), L363 (-), RS411 (2.5+), SKOV-3 (-), Raji-CD19-KO + Rituximab (3.5+), Raji-CD19-KO Herceptin (4+), SKOV-3 + Herceptin (4+) |
| CD20, Fc | 22 17 6 | 229 91 | CD8SP-CD16A-V158-G4S-CD20-2F2-vL- [hTCRb-KACIAH]-F-P2A-IgHSP-CD20- vHH2-HCD25-G4S-CD20-2F2-vH-[hTCRa- CSDVP]-F-F2A-PAC | Raji (3+), Nalm6 (+), SKOV-3 (-), SKOV-3 + Herceptin (4+) |
| CD19, CD20, NKG2 D-L | 22 17 7 | 229 92 | CD8SP-NKG2D-G4Sx3-NKG2D-G4S-hu- mROO5-vL-[hTCRb-KACIAH]-F-P2A- IgHSP-CD20-vHH2-HCD25-G4S-CD19-hu- mROO5-1-vH-[hTCRa-CSDVP]-F-F2A-PAC | Raji (2.5+), Raji-CD19-KO (2+), Nalm6 (3+), L363 (+), SKOV-3 (2+) |
| PSMA, CD20, CD19 | 22 17 8 | 229 93 | CD8SP-PSMA71-chVH-G4S-hu-mROO5-vL- [hTCRb-KACIAH]-F-P2A-IgHSP-CD20- vHH2-HCD25-G4S-CD19-hu-mROO5-1-vH- [hTCRa-CSDVP]-F-F2A-PAC | Raji (4.5+), Raji-CD19-KO (5+), Nalm6 (5+), L363 (+/-), RS411 (+), LNCaP (5+) |
| CD19, PSMA | 22 17 9 | 229 94 | CD8SP-CD19-hu-mROO5-1-vL-[hTCRa- CSDVP]-F-F2A-PSMA28v2-chVH-G4S- CD19-hu-mROO5-1-vH-[hTCRb-KACIAH]- F-P2A-PAC | Raji (5+), Raji-CD19-KO (-), L363 (+/-), RS411 (+), LNCaP (6+) |
| CD19, PSMA | 22 18 0 | 229 95 | CD8SP-CD19-hu-mROO5-1-vL-[hTCRb- KACIAH]-F-P2A-IgHSP-PSMA28v2-chVH- G4S-CD19-hu-mROO5-1-vH-[hTCRa- CSDVP]-F-F2A-PAC | Raji (5+), Raji-CD19-KO (+/-), L363 (+/-), RS411 (+), LNCaP (7+) |
| CD19, PSMA | 22 18 1 | 229 96 | CD8SP-CD19-hu-mROO5-1-vL-[hTCRb- S57C]-F-P2A-PSMA28v2-chVH-G4S-CD19- hu-mROO5-1-vH-[hTCRa-T48C-opt]-F-F2A- PAC | Raji (5+), Raji-CD19-KO (-), LNCaP (4+) |
| PSMA, CD19 | 22 18 2 | 229 97 | CD8SP-PSMA-USC76-chVH-G4S-hu- mROO5-vL-[hTCRb-S57C]-F-P2A-SP-CD19- hu-mROO5-1-vH-[hTCRa-T48C-opt]-F-F2A- PAC | Raji (5+), LNCaP (3.5+) |
| PSMA, CD19 | 22 18 3 | 229 98 | CD8SP-PSMA-USC104-chVH-G4S-hu- mROO5-vL-[hTCRb-S57C]-F-P2A-SP-CD19- hu-mROO5-1-vH-[hTCRa-T48C-opt]-F-F2A- PAC | Raji (5+), LNCaP (3.5+) |
| PSMA, CD19 | 22 18 4 | 229 99 | CD8SP-PSMA-USC101-chVH-G4S-hu- mROO5-vL-[hTCRb-S57C]-F-P2A-SP-CD19- hu-mROO5-1-vH-[hTCRa-T48C-opt]-F-F2A- PAC | Raji (3.5+), LNCaP (3.5+) |
| CD19, PSMA | 22 18 5 | 230 00 | CD8SP-CD19-hu-mROO5-1-vL-[hTCRb- S57C]-F-P2A-IgHSP-PSMA-USC-30-chVH- G4S-CD19-hu-mROO5-1-vH-[hTCRa-T48C- opt]-F-F2A-PAC | Raji (5+), LNCaP (5+), Huh 7 (+), KMH2 (+/-) |
| CD19, PSMA | 22 18 6 | 230 01 | CD8SP-CD19-hu-mROO5-1-vL-[hTCRb- S57C]-F-P2A-IgHSP-PSMA-USC-29-chVH- G4S-CD19-hu-mROO5-1-vH-[hTCRa-T48C- opt]-F-F2A-PAC | Raji (2+), LNCaP (5+), Huh 7 (2+), KMH2 (2+) |
| CD19, PSMA | 22 18 7 | 230 02 | CD8SP-CD19-hu-mROO5-1-vL-IgCL-TCRb- wt2-opt-6MD-F-P2A-PSMA28v2-chVH-G4S- CD19-hu-mROO5-1-vH-IgG1-CH1-TCRa- wt2-opt-6MD-F-F2A-PAC | Raji (4+), Raji-CD19-KO (4.5+), LNCaP (4+) |
| CD19, PSMA | 22 18 8 | 230 03 | CD8SP-CD19-hu-mROO5-1-vL-huTCRg-d5- F-P2A-PSMA28v2-chVH-G4S-CD19-hu- mROO5-1-vH-huTCRd-d2-F-F2A-PAC | Raji (2.5+), Raji-CD19-KO (-), Nalm6 (2+), LNCaP (3+) |
| CD19, PSMA | 22 18 9 | 230 04 | CD8SP-CD19-hu-mROO5-1-vL-[hTCRb- S57C-opt]-F-P2A-PSMA28v2-chVH-G4S- CD19-hu-mROO5-1-vH-IgG1-CH1-TCRa- wt2-opt-6MD-F-F2A-PAC | Raji (1.5+), Raji-CD19-KO (-), Nalm6 (+), LNCaP (+) |
| CD19, PSMA | 22 19 0 | 230 05 | CD8SP-CD19-hu-mROO5-1-vL-[hTCRb- S57C-opt]-F-P2A-IgHSP-PSMA28v2-chVH- G4S-CD19-hu-mROO5-1-vH-IgG1-CH1- TCRa-wt2-opt-6MD-F-F2A-PAC | Raji (3+), Raji-CD19-KO (2+), Nalm6 (+), LNCaP (5+) |
| CD19, Cl channe l | 22 19 1 | 230 06 | CD8SP-CD19-hu-mROO5-1-vL-[hTCRb- S57C]-F-P2A-IgHSP-CLTX23-G4S-CD19- hu-mROO5-1-vH-[hTCRa-T48C-opt]-F-F2A- PAC | Raji (3+), Raji-CD19-KO (-), Nalm6 (4+), L363 (-), RS411 (2+) |
| CD19, CD20, Cl Chann el | 22 19 2 | 230 07 | CD8SP-CLTX-G4S-hu-mROO5-vL-[hTCRa- CSDVP]-F-F2A-IgHSP-CD20-vHH2-HCD25- G4S-CD19-hu-mROO5-1-vH-[hTCRb- KACIAH]-F-P2A-PAC | Raji (4+), Raji-CD19-KO (2.5+), Nalm6 (+), L363 (-), RS411 (3+) |
| CD19, Cl Chann el | 22 19 3 | 230 08 | CD8SP-CD19-hu-mROO5-1-vL-[hTCRb- S57C]-F-P2A-IgHSP-CLTX23-G4S-CD19- hu-mROO5-1-vH-[hTCRa-T48C-opt]-F-F2A- PAC | Raji (+), Raji-CD19-KO (-), Nalm6 (+), L363 (-), RS411 (-) |
| CD22, HER2 | 22 19 4 | 230 09 | CD8SP-CD22-FHVH-24-IgCL-TCRg-6MD- F-P2A-Her2-affi-IgG1-CH1-TCRd-6MD-F- F2A-PAC | SKOV-3 (-), MCF-7 (-), Raji (4+) |
| CD19, HER2 | 22 19 5 | 230 10 | CD8SP-CD19-hu-mROO5-1-vL-[hTCRb- S57C]-F-P2A-IgHSP-Her2-Dar1-G4S-CD19- hu-mROO5-1-vH-[hTCRa-T48C-opt]-F-F2A- PAC | Raji (4+), Raji-CD19-KO (-), Nalm6 (7+), L363 (-), SKOV-3 (4+), MCF-7 (5.5+), MDA-MB-231 (5.5+), PC-3 (6+) |
| CD19, HER2 | 22 19 6 | 230 11 | CD8SP-CD19-hu-mROO5-1-vL-[hTCRb- S57C]-F-P2A-IgHSP-Her2-47D5-vHH-G4S- CD19-hu-mROO5-1-vH-[hTCRa-T48C-opt]- F-F2A-PAC | Raji (3.5+), Raji-CD19-KO (-), SKOV-3 (7+) |
| CD19, HER2 | 22 19 7 | 230 12 | CD8SP-CD19-hu-mROO5-1-vL-[hTCRb- S57C]-F-P2A-IgHSP-Her2-Dar1-G4S-CD19- hu-mROO5-1-vH-[hTCRa-T48C-opt]-F-F2A- PAC | Raji (2+), Raji-CD19-KO (-), SKOV-3 (5+) |
| HER3, BCMA | 22 19 8 | 230 13 | Her3-affi-IgCL-TCRg-6MD-F-P2A-Bcu- IgHSP-BCMA-FHVH-93-IgG1-CH1-TCRd- 6MD-F-F2A-PAC | SKOV-3 (3+), MCF-7 (5.5+), L363 (2.5+) |
| CD19, HER3 | 22 19 9 | 230 14 | CD8SP-CD19-hu-mROO5-1-vL-[hTCRb- S57C]-F-P2A-IgHSP-Her3-AFFI-G4S-CD19- hu-mROO5-1-vH-[hTCRa-T48C-opt]-F-F2A- PAC | Raji (5+), Raji-CD19-KO (-), Nalm6 (7+), L363 (-), SKOV-3 (-) |
| MIR1, CD20 | 22 20 0 | 230 15 | MC7G5-TCRb-Vb-[hTCRb-S57C]-F-P2A- IgHSP-CD20-vHH2-HCD25-G4S-MC7G6- TCRa-Va-[hTCRa-T48C-opt]-F-F2A-PAC | Raji (+), Nalm6 (-), L363 (-), RS411 (-) |
| BCMA , CD20, HMB-PP | 22 20 1 | 230 16 | CD8SP-BCMA-FHVH-93-G4S-TCR-Vg9-F- P2A-IgHSP-CD20-vHH2-HCD25-G4S-TCR- Vd2-F-F2A-PAC | Raji (2.5+), Raji-CD19-KO (2+), Nalm6 (+/-), L363 (1.5+), RS411 (-) |
| CD20, HMB-PP | 22 20 2 | 230 17 | CD8SP-TCR-Vg9-F-P2A-IgHSP-CD20- vHH2-HCD25-G4S-TCR-Vd2-F-F2A-PAC | Raji (3+) |
| BCMA , CD20 | 22 20 3 | 230 18 | CD8SP-BCMA-FHVH-93-G4S-hu-mROO5- vL-[hTCRb-KACIAH]-F-P2A-IgHSP-CD20- vHH2-HCD25-G4S-Her2-huMab4D5-D98W- vH-[hTCRa-CSDVP]-F-F2A-PAC | SKOV-3 (-), Raji (4.5+), L363 (4+) |
| CD20 | 22 20 4 | 230 19 | CD8SP-CD19-hu-mROO5-1-vL-[hTCRa- CSDVP]-F-F2A-IgHSP-CD20-vHH2-HCD25- G4S-Her2-huMab4D5-D98W-vH-[hTCRb- KACIAH]-F-P2A-PAC | SKOV-3 (-), Raji (3+) |
| CD20 | 22 20 5 | 230 20 | MC7G5-TCRb-Vb-[hTCRb-S57C]-F-P2A- IgHSP-CD20-vHH2-HCD25-G4S-CD19-hu- mROO5-1-vH-[hTCRa-T48C-opt]-F-F2A- PAC | Raji (5+) |
| BCMA , CD20 | 22 20 6 | 230 21 | CD8SP-BCMA-FHVH-93-G4S-hu-mROO5- vL-[hTCRb-S57C]-F-P2A-IgHSP-CD20- VHH-USC1-2HCD26-G4S-AFP-Va-14-3-AI- [hTCRa-T48C]-F-F2A-PAC | Raji (6+) |
| CD19 | 22 20 7 | 230 22 | CD8SP-CD19-hu-mROO5-1-vL-IgCL-TCRg- 6MD-F-P2A-SP-CD19-hu-mROO5-1-vH- huTCR-delta-F-F2A-PAC | Raji (5+) |

### SAR constructs comprising epitopes

A panel of unispecific, bispecific and multispecific SAR constructs comprising one or more epitope tags joined to the N-terminus domain of the encoded antigen binding domains (e.g., vL, vH, AABD etc.) via a short flexible Gly-Ser linker or protease cleavable linkers **(Table 19)** were generated. The SEQ ID of these exemplary constructs are providedd in the **Table 47.** The constructs were expressed in JNG cells and showed significant activity upon exposure to their target antigen expressing cells, thereby demonstrating that the attachment of the epitope tag does not interfere with antigen binding ability of the antigen binding domains. More importantly many constructs showed binding to Rituximab as measured by flow cytometry and reduced survival when cultured with Rituximab in the absence and in the presence of complement. Similarly, the construct with SEQ ID NO: 22247 and 22248 and showed binding to Herceptin as measured by flow cytometry. JNG cells expressing the constructs with SEQ ID NO: 22247 and 22248 showed cytotoxicity when exposed to Herceptin (Trastuzumab), T-DM1 (Kadcyla. Trastuzumab emtansine) and Enhertu. The immune cells expressing Ritx2 epitope tagged SARs (SEQ ID NO: 22209) are purified using flow cytometry and or MACS using Rituximab. The immune cells expressing Herceptin mimotag tagged SARs (SEQ ID NO: 22247 and 22248) are purified using flow cytometry and or MACS using Herceptin. Furthermore, immune cells (e.g., T cells) expressing SARs (SEQ ID NO: 22247 and 22248) are depleted upon administration of Herceptin, T-DM1 or Enhertu to the subjects reconstituted with these SAR-expressing cells. The dose and frequency of administration of these antibodies is known in the art

| **TABLE 47: UNISPECIFIC, BISPECIFIC AND BIPARATOPIC SARs WITH EPITOPE TAGS AND PROTEASE CLEAVABLE LINKERS** | | | | |
|---|---|---|---|---|
| **ANTIGEN** | **SEQ ID NO (DNA)** | **SEQ ID NO (PRT)** | **Constructs Name** | **NFAT-GFP ASSAY** |
| CD19, CD20 | 22209 | 23024 | CD8SP-RitX2-G4S-hu-mROO5-vL-[hTCRb- KACIAH]-F-P2A-IgHSP-CD20-vHH2-HCD25- G4S-CD19-hu-mROO5-1-vH-[hTCRa-CSDVP]- F-F2A-PAC | Raji (2+), Raji-CD19-KO (1.5+),Nalm6 (2+) |
| CD19, CD20 | 22210 | 23025 | CD8SP-Herceptin-Mimotag-G4S-hu-mROO5- vL-[hTCRb-KACIAH]-F-P2A-IgHSP-CD20- vHH2-HCD25-G4S-CD19-hu-mROO5-1-vH- [hTCRa-CSDVP]-F-F2A-PAC | Raji (2+), Raji-CD19-KO (1.5+),Nalm6 (+) |
| CD19 | 22211 | 23026 | CD8SP-CD19-hu-mROO5-1-vL-[hTCRb- S57C]-F-P2A-IgHSP-Herceptin-Mimotag-G4S- CD19-hu-mROO5-1-vH-[hTCRa-T48C-opt]-F- F2A-PAC | Raji (2+), Raji-CD19-KO (-) |
| CD19, CD20 | 22212 | 23027 | CD8SP-R8-G4S-hu-mROO5-vL-[hTCRb- KACIAH]-F-P2A-IgHSP-CD20-vHH2-HCD25- G4S-CD19-hu-mROO5-1-vH-[hTCRa-CSDVP]- F-F2A-PAC | Raji (6+), Raji-CD19-KO (6+) |
| CD19, CD20 | 22213 | 23028 | CD8SP-RitX2-MMP14-1-hu-mROO5-vL- [hTCRb-KACIAH]-F-P2A-IgHSP-CD20-vHH2- HCD25-G4S-CD19-hu-mROO5-1-vH-[hTCRa- CSDVP]-F-F2A-PAC | Raji (1.5+) |
| CD19, CD20 | 22214 | 23029 | CD8SP-RitX2-MMP9x2-hu-mROO5-vL- [hTCRb-KACIAH]-F-P2A-IgHSP-CD20-vHH2- HCD25-G4S-CD19-hu-mROO5-1-vH-[hTCRa- CSDVP]-F-F2A-PAC | Raji (+) |
| CD19, CD20 | 22215 | 23030 | CD8SP-RitX2-Cathepsin-S-hu-mROO5-vL- [hTCRb-KACIAH]-F-P2A-IgHSP-CD20-vHH2- HCD25-G4S-CD19-hu-mROO5-1-vH-[hTCRa- CSDVP]-F-F2A-PAC | Raji (3+), Raji-CD19-KO (3+) |
| CD19, CD20 | 22216 | 23031 | CD8SP-Herceptin-Mimotag-MMP9x2-hu- mROO5-vL-[hTCRb-KACIAH]-F-P2A-IgHSP- CD20-vHH2-HCD25-G4S-CD19-hu-mROO5-1- vH-[hTCRa-CSDVP]-F-F2A-PAC | Raji (4.5+) |
| BCMA, CD19, CD20 | 22217 | 23032 | CD8SP-BCMA-FHVH-93-Cathepsin-S-hu- mROO5-vL-[hTCRb-KACIAH]-F-P2A-IgHSP- CD20-VHH-USC1-2HCD26-G4S-CD19-hu- mROO5-1-vH-[hTCRa-T48C-opt]-F-F2A-PAC | Raji (+), Raji-CD19-KO (3+), Nalm6 (4+), L363 (3+), U266 (4+), RS411 (1.5+) |
| CD19, CD20 | 22218 | 23033 | CD8SP-R5pLtag-G4S-hu-mROO5-vL-[hTCRb- KACIAH]-F-P2A-IgHSP-CD20-vHH2-HCD25- G4S-CD19-hu-mROO5-1-vH-[hTCRa-CSDVP]- F-F2A-PAC | Raji (3+) |
| CD19 | 22219 | 23034 | CD8SP-CD19-hu-mROO5-1-vL-[hTCRb- S57C]-F-P2A-IgHSP-R8-G4S-CD19-hu- mROO5-1-vH-[hTCRa-T48C-opt]-F-F2A-PAC | Raji (+/-) |
| BCMA, CD19, CD20 | 22220 | 23035 | CD8SP-BCMA-FHVH-93-MMP14-1-hu- mROO5-vL-[hTCRb-KACIAH]-F-P2A-IgHSP- CD20-VHH-USC1-2HCD26-G4S-CD19-hu- mROO5-1-vH-[hTCRa-T48C-opt]-F-F2A-PAC | Raji (-), Raji-CD19-KO (+), Nalm6 (+/-), L363 (+/-), U266 (+), RS411 (-) |
| BCMA, CD20, CD19 | 22221 | 23036 | CD8SP-BCMA-FHVH-93-MMP9x2-hu- mROO5-vL-[hTCRb-KACIAH]-F-P2A-IgHSP- CD20-VHH-USC1-2HCD26-G4S-CD19-hu- mROO5-1-vH-[hTCRa-T48C-opt]-F-F2A-PAC | Raji (-), Raji-CD19-KO (1.5+), Nalm6 (+), L363 (+), U266 (2+), RS411 (+) |
| CD19, CD20 | 22222 | 23037 | CD8SP-Herceptin-Mimotag-Cathepsin-S-hu- mROO5-vL-[hTCRb-KACIAH]-F-P2A-IgHSP- CD20-vHH2-HCD25-G4S-CD19-hu-mROO5-1- vH-[hTCRa-CSDVP]-F-F2A-PAC | Raji (2+) |
| CD19 | 22223 | 23038 | CD8SP-CD19-hu-mROO5-1-vL-[hTCRb- S57C]-F-P2A-IgHSP-Herceptin-Mimotag- MMP1-CD19-hu-mROO5-1-vH-[hTCRa-T48C- opt]-F-F2A-PAC | Raji (3+) |
| CD19, BCMA | 22224 | 23039 | CD8SP-CD19-hu-mROO5-1-vL-[hTCRb- S57C]-F-P2A-IgHSP-BCMA917-vHH-E59D- MMP14-2-CD19-hu-mROO5-1-vH-[hTCRa- T48C-opt]-F-F2A-PAC | Raji (+), Raji-CD19-KO (2+), Nalm6 (2.5+), L363 (2+), U266 (4+), RS411 (1.5+) |
| CD19, BCMA | 22225 | 23040 | CD8SP-CD19-hu-mROO5-1-vL-[hTCRb- S57C]-F-P2A-IgHSP-BCMA917-vHH-E59D- MMP1-CD19-hu-mROO5-1-vH-[hTCRa-T48C- opt]-F-F2A-PAC | Raji (1.5+), Raji-CD19-KO (4+), Nalm6 (3+), L363 (3+), U266 (5+), RS411 (2+) |
| BCMA, CD20, CD19 | 22226 | 23041 | CD8SP-BCMA-FHVH-93-MMP9x2-hu- mROO5-vL-[hTCRb-KACIAH]-F-P2A-IgHSP- CD20-VHH-USC1-2HCD26-G4S-CD19-hu- mROO5-1-vH-[hTCRa-T48C-opt]-F-F2A-PAC | Raji (+/-), Raji-CD19-KO (1.5+), Nalm6 (+), L363 (+), U266 (2.5+), RS411 (+/-) |
| CD19 | 22227 | 23042 | CD8SP-CD19-hu-mROO5-1-vL-[hTCRb- S57C]-F-P2A-IgHSP-Herceptin-Mimotag- MMP14-2-CD19-hu-mROO5-1-vH-[hTCRa- T48C-opt]-F-F2A-PAC | Raji (4+) |
| CD19 | 22228 | 23043 | CD8SP-CD19-hu-mROO5-1-vL-[hTCRb- S57C]-F-P2A-IgHSP-R5pL-tag-G4S-CD19-hu- mROO5-1-vH-[hTCRa-T48C-opt]-F-F2A-PAC | Raji (2+) |
| BCMA, CD19, CD20 | 22229 | 23044 | CD8SP-BCMA-FHVH-93-MMP14-1-hu- mROO5-vL-[hTCRb-KACIAH]-F-P2A-IgHSP- CD20-VHH-USC1-2HCD26-G4S-CD19-hu- mROO5-1-vH-[hTCRa-T48C-opt]-F-F2A-PAC | Raji (3+), Raji-CD19-KO (5+), Nalm6 (3.5+), L363 (3.5+), U266 (6+), RS411 (2+) |
| CD19 | 22230 | 23045 | CD8SP-CD19-hu-mROO5-1-vL-[hTCRb- S57C]-F-P2A-IgHSP-R5pLx2-Qbend-G4S- CD19-hu-mROO5-1-vH-[hTCRa-T48C-opt]-F- F2A-PAC | Raji (4+), Raji-CD19-KO (-) |
| CD19, CD20 | 22231 | 23046 | CD8SP-Herceptin-Mimotag-MMP14-1-hu- mROO5-vL-[hTCRb-KACIAH]-F-P2A-IgHSP- CD20-vHH2-HCD25-G4S-CD19-hu-mROO5-1- vH-[hTCRa-CSDVP]-F-F2A-PAC | Raji (3.5+), Raji-CD19-KO (4.5+) |
| CD19 | 22233 | 23048 | CD8SP-CD19-hu-mROO5-1-vL-[hTCRb- S57C]-F-P2A-IgHSP-RitX2-G4S-CD19-hu- mROO5-1-vH-[hTCRa-T48C-opt]-F-F2A-PAC | Raji (2.5+) |
| CD19 | 22234 | 23049 | CD8SP-CD19-hu-mROO5-1-vL-[hTCRb- S57C]-F-P2A-IgHSP-HercepMimotopx3-citux- Mimotope1-G4S-CD19-hu-mROO5-1-vH- [hTCRa-T48C-opt]-F-F2A-PAC | Raji (+/-) |
| CD19 | 22235 | 23050 | CD8SP-CD19-hu-mROO5-1-vL-[hTCRb- S57C]-F-P2A-IgHSP-Panitumab-tag1-G4S- CD19-hu-mROO5-1-vH-[hTCRa-T48C-opt]-F- F2A-PAC | Raji (6+) |
| CD19 | 22236 | 23051 | CD8SP-CD19-hu-mROO5-1-vL-[hTCRb- S57C]-F-P2A-IgHSP-Panitumab-tag2-citux- Mimotope1-G4S-CD19-hu-mROO5-1-vH- [hTCRa-T48C-opt]-F-F2A-PAC | Raji (4+) |
| CD19 | 22237 | 23052 | CD8SP-CD19-hu-mROO5-1-vL-[hTCRb- S57C]-F-P2A-IgHSP-Panitumab-tag3-citux- Mimotope1-G4S-CD19-hu-mROO5-1-vH- [hTCRa-T48C-opt]-F-F2A-PAC | Raji (5+) |
| CD19, CD20 | 22238 | 23053 | CD8SP-CD19-hu-mROO5-1-vL-[hTCRb- S57C]-F-P2A-IgH-SP-IgSP-HercepMimotopx3- CD20-VHH-USC1-2HCD26-G4S-CD19-hu- mROO5-1-vH-[hTCRa-T48C-opt]-F-F2A-PAC | Raji (+) |
| CD19 | 22239 | 23054 | CD8SP-RitX2-G4S-hu-mROO5-1-vL-[hTCRb- S57C]-F-P2A-IgHSP-Herceptin-Mimotag-G4S- CD19-hu-mROO5-1-vH-[hTCRa-T48C-opt]-F- F2A-PAC | Raji (+) |
| CD19 | 22240 | 23055 | CD8SP-CD19-hu-mROO5-1-vL-[hTCRb- S57C]-F-P2A-IgSP-RituxGAtag-Herceptin- Mimotag-G4S-CD19-hu-mROO5-1-vH-[hTCRa- T48C-opt]-F-F2A-PAC | Raji (3+) |
| CD19 | 22241 | 23056 | CD8SP-CD19-hu-mROO5-1-vL-[hTCRb- S57C]-F-P2A-IgHSP-RitxGA4-G4S-CD19-hu- mROO5-1-vH-[hTCRa-T48C-opt]-F-F2A-PAC | Raji (4+) |
| CD19 | 22242 | 23057 | CD8SP-CD19-hu-mROO5-1-vL-[hTCRb- S57C]-F-P2A-IgHSP-Cituximab-mimotopx2- G4S-CD19-hu-mROO5-1-vH-[hTCRa-T48C- opt]-F-F2A-PAC | Raji (5.5+) |
| CD19-CD20-Rituximab | 22243 | 23058 | CD8SP-R5pL-tag-G4S-hu-mROO5-vL-[hTCRb- KACIAH]-F-P2A-IgHSP-CD20-vHH2-HCD25- G4S-CD19-hu-mROO5-1-vH-[hTCRa-CSDVP]- F-F2A-PAC | Raji (3+) |
| CD19-Rituximab | 22244 | 23059 | CD8SP-CD19-hu-mROO5-1-vL-[hTCRb- S57C]-F-P2A-IgHSP-R8-G4S-CD19-hu- mROO5-1-vH-[hTCRa-T48C-opt]-F-F2A-PAC | Raji (+/-) |
| BCMA-CD19 | 22245 | 23060 | CD8SP-BCMA-FHVH-93-MMP14-1-hu- mROO5-vL-[hTCRb-KACIAH]-F-P2A-IgHSP- CD20-VHH-USC1-2HCD26-G4S-CD19-hu- mROO5-1-vH-[hTCRa-T48C-opt]-F-F2A-PAC | Raji (-), Raji-CD19-KO (+), Nalm6 (+/-), L363 (+/-), U266 (+), |
| BCMA-CD20 | 22246 | 23061 | CD8SP-BCMA-FHVH-93-MMP9x2-hu- mROO5-vL-[hTCRb-KACIAH]-F-P2A-IgHSP- CD20-VHH-USC1-2HCD26-G4S-CD19-hu- mROO5-1-vH-[hTCRa-T48C-opt]-F-F2A-PAC | Raji (-), Raji-CD19-KO (1.5+), Nalm6 (+), L363 (+), U266 (2+) |
| CD19-Herceptin | 22247 | 23062 | CD8SP-Herceptin-Mimotag-Cathepsin-S-hu- mROO5-vL-[hTCRb-KACIAH]-F-P2A-IgHSP- CD20-vHH2-HCD25-G4S-CD19-hu-mROO5-1- vH-[hTCRa-CSDVP]-F-F2A-PAC | Raji (2+) |
| CD19-HER2 | 22248 | 23063 | CD8SP-CD19-hu-mROO5-1-vL-[hTCRb- S57C]-F-P2A-IgHSP-Herceptin-Mimotag- MMP1-CD19-hu-mROO5-1-vH-[hTCRa-T48C- opt]-F-F2A-PAC | Raji (3+) |
| CD19-BCMA | 22249 | 23064 | CD8SP-CD19-hu-mROO5-1-vL-[hTCRb- S57C]-F-P2A-IgHSP-BCMA917-vHH-E59D- MMP14-2-CD19-hu-mROO5-1-vH-[hTCRa- T48C-opt]-F-F2A-PAC | Raji (+), Raji-CD19-KO (2+), Nalm6 (2.5+), L363 (2+), U266 (4+), RS411 (+) |
| CD19-BCMA | 22250 | 23065 | CD8SP-CD19-hu-mROO5-1-vL-[hTCRb- S57C]-F-P2A-IgHSP-BCMA917-vHH-E59D- MMP1-CD19-hu-mROO5-1-vH-[hTCRa-T48C- opt]-F-F2A-PAC | Raji (1.5+), Raji-CD19-KO (4+), Nalm6 (3+), L363 (3+), U266 (5+), RS411 (2+) |

### SARS WITH ADAPTOR BINDING DOMAINS

A panel of double chain SARs on the backbone of SIR and Ab-TCR constructs were generated comprising different adaptor binding domains. An exemplary SAR construct with SEQ ID NO: 22286 comprised an engineered NKG2D-YA domain attached to the N-terminus of the encoded IgCL fragment of a SAR with the backbone of an Ab-TCR **(Table 48).** The construct with SEQ ID NO: 22287 encodes an **NKG2D-YA-G4Sx3-NKG2D-YA** domain attached to the encoded hu-mROO5-1 vH fragment of an Ab-TCR via a G4S linker. The constructs were expressed in JNG cells and showed robust induction of GFP upon exposure to CD19+ and BCMA+ Raji cells. More importantly, JNG cells showed induction of GFP when exposed to LNCaP cells only in the presence of a SAR-adaptor PSMA-USC76-chVH-G4S-ULBP2-S3 (SEQ ID NO: 23114) that comprises a PSMA targeted chVH fused via a G4S linker to a non-natural ULBP2-S3 ligand that binds specifically to NKG2D-YA **(Table 48).** Essentially similar results were obtained with construct with SEQ ID NO: 22288 **(Table 48).** However, the construct with SEQ ID NO: 22289 which encodes for NKG2D-YA that is fused directly to TCRb constant chain without an Ig linker failed to show GFP induction in response to PSMA-USC76-chVH-G4S-ULBP2-S3.

The SAR construct represented by SEQ ID NO: 22292 cncodes for a D domain (Afo3) capable of binding to a p26 domain attached to the N-terminus of the encoded vL fragment of a hu-mROO5-1 based SIR. The JNG cells expressing this construct showed robust induction of GFP when exposed to L428 cells (CD123⁺) but only in the presence of SAR adaptor bc40-p26-CD123-cg06 (SEQ ID NO: 23120) that comprises a p26 domain fused to a D domain targeting CD123 **(Table 48).**

The bispecific CD19xCD20 SAR construct represented by SEQ ID NO: 22293 also encodes a RZIP leucine zipper domain, which binds to the leucine zippe domain EZIP, that is attached to the N-terminus of the encoded vL fragment of a hu-mROO5-1-based CD19-targeted SIR. The construct also encodes for a CD20-VHH-USC1-2HCD26 domain attached to the hu-mROO5-1 vH domain. The JNG cells expressing this construct showed robust induction of GFP when exposed to L363 cells (BCMA⁺) but only in the presence of SAR adaptor BCMA-FHVH-74-EZIP (SEQ ID NO: 23119) that comprises a BCMA-FHVH-74 domain fused to a leucine zipper EZIP domain **(Table 48).** Furthermore, the JNG cells could be redirected to LNCaP (PSMA+) cells in the presence of the SAR adaptor CD8SP-PSMA-USC76-chVH-EZIP (SEQ ID NO: 23118) **(Table 48).** Weaker results were obtained with the construct with SEQ ID NO: 22294 in which the RZIP domain is attached directly to a truncated TCR chain [hTCRb-wt-opt2-6MD] with a short GS linker and without an Ig linker or a long linker. These results again demonstrate the requirement for an Ig linker, a variable domain (e.g., vL, vH, Va, Vb, Vg, Vd) or a long linker (>25 amino acids) when an AABD is operationally linked to a TCR constant chain in a SAR.

The SAR adaptors for the preceding experiments were generated by transient transfection of their respective expression constructs in 293FT cells and supernatants containing the secreted adaptor proteins were collected 48-72 hours post-transfection and frozen until use.

Additional SAR constructs are generated comprising two or more adaptors that are either attached to two separate chains of a double chain SAR or attached in tandem to one of the chains via an optional short linker (e.g., G4S linker). These constructs are also shown to bind to and respond to their respective target antigen expressing cells in the presence of the appropriate SAR adaptor.

Additional constructs are generated by fusing one or more adaptors to the N-terminus of an encoded scFv of a 2^{nd} generation CAR, TFPε, TFPγ or TFPδ. These constructs are also shown to bind to and respond to their respective target antigen expressing cells in the presence of their SAR adaptors. These results demonstrate that bispecific and multispecific SARs encoding one or more adaptors (e.g., leucine zipper domains, D domains etc.) can be generated by fusing the adaptor to the N-terminus or near the N-terminus of the vL, vH, Va, Vb, Vg, Vd, scFv, or AABD domains comprising a CAR, SIR, Ab-TCR, AABD-TCR, TFP or TCR.

| **TABLE 48: SARS WITH ADAPTOR BINDING DOMAINS** | | | | |
|---|---|---|---|---|
| **ANTIGEN** | **SEQ ID NO (DNA)** | **SEQ ID NO (PRT)** | **Constructs Name** | **NFAT-GFP ASSAY** |
| CD20-CD19, ULBP2-S3 | 22286 | 23101 | CD8SP-NKG2D-YA-IgCL- TCRg-6MD-F-P2A-IgHSP-CD20- vHH2-HCD25-G4S-CD19-hu- mROO5-1-vH-IgG1-CH1-TCRd- 6MD-F-F2A-PAC | Raji (+), LNCaP (-), LNCaP with PSMA-USC76-chVH-G4S-ULBP2-S3 (+) |
| BCMA-CD19, ULBP2-S3 | 22287 | 23102 | CD8SP-BCMA-FHVH-93-G4S- hu-mROO5-vL-IgCL-TCRb-wt2- opt-6MD-F-P2A-IgH-SP- NKG2D-YA-G4Sx3-NKG2D- YA-G4S-CD19-hu-mROO5-1- vH-IgG1-CH1-TCRa-wt2-opt- 6MD-F-F2A-PAC | Raji (3+), L363 (+/-), LNCaP (-), LNCaP with PSMA-USC76-chVH-G4S-ULBP2-S3 (+) |
| CD20, ULBP2-S3 | 22288 | 23103 | CD8SP-**NKG2D**-YA-IgCL- TCRg-6MD-F-P2A-IgHSP-CD20- vHH2-HCD25-G4S-**NKG2D-AF**- Y119F-IgG1-CH1-TCRd-6MD-F- F2A-PAC | Raji (3+), LNCaP (-), LNCaP with PSMA-USC76-chVH-G4S-ULBP2-S3 (+), |
| ULBP2-S3 | 22289 | 23104 | CD8SP-NKG2D-YA-[hTCRb- S57C]-F-P2A-SP-NKG2D-AF- [hTCRa-T48C-opt]-F-F2A-PAC | Raji (-), LNCaP (-), LNCaP + PSMA-USC76-chVH-G4S-ULBP2-S3 (-), |
| CD19, p26 | 22292 | 23107 | CD8SP-Af03-G4S-hu-mROO5- vL-[hTCRb-S57C]-F-P2A-SP- CD19-hu-mROO5-1-vH-[hTCRa- T48C-opt]-F-F2A-PAC | L428 (-), L428 with bc40-p26-CD123-cg06 (+3) |
| CD19, CD20, RZIP | 22293 | 23108 | CD8SP-CD8SP-R1-Rzip-G4S-hu- mROO5-vL-[hTCRb-S57C]-F- P2A-IgHSP-CD20-VHH-USC1- 2HCD26-G4S-CD19-hu-mROO5- 1-vH-[hTCRa-T48C-opt]-F-F2A- PAC | LnCAP (-), LnCAP with PSMA-USC76-chVH-EZIP (+5.5), L363 (-), L363 with BCMA-FHVH-74-EZIP (+3) |
| CD22, RZIP | 22294 | 23109 | CD8SP-CD22-FHVH-24-GS- [TCRa-wt-opt2-6MD]-F-F2A- CD8SP-R1-RZip-GS-[hTCRb-wt- opt2-6MD]-F-P2A-PAC | L363 (-), LNCaP (-), LNCaP with PSMA-USC76-chVH-EZIP (+1) |

### SARS comprising autoantigens

An exemplary SAR comprising the autoantigen Dsg3 is CD8SP-Dsg3-EC1-EC2-L693-IgCL-TCRb-wt2-opt-6MD-F-P2A-IgHSP-CD20-vHH2-HCD25-G4S-CD19-hu-mROO5-1-vH-IgG1-CH1-TCRa-wt2-opt-6MD-F-F2A-PAC (SEQ ID NO: 22307). This JNG expressing this SAR construct showed increased GFP induction when exposed to Raji cells expressing a membrane anchored scFv targeting Dsg3 as compared to GFP induction observed with the parental Raji cells. Additional exemplary SAR comprising different domains of Dsg3 are represented by SEQ ID NO: 22308-22312. T cells expressing these SARs are used to deplete Dsg3 autoantibody producing B cells and plasma cells in a patient with pemphigus vulgaris. Similarly, T cells expressing SARs targeting Dsg1 can be generated using the design of the above constructs and can be used to deplete Dsg1 autoantibodies producing B and plasma cells in a subject with pemphigus foliaceus (PF).

### SARs comprising ligands

Exemplary bispecific SARs on the backbone of CD19 targeted SIR and comprising the receptor binding domain of the ligands 4-1BB (SEQ ID NO: 22252) and APRIL (SEQ ID NO: 22253-22254) were generated and expressed in JNG cells. The cells retained the ability to respond to Raji cells (CD19+) upon co-culture as measured by GFP induction.

### SARs comprising single variable domain of TCR (svd-TCR)

Exemplary bispecific SARs on the backbone of CD19 targeted SIR and comprising svd-TCR targeting an HLA-A*02:01 in complex with a human NY-ESO-1 157 peptide (SEQ ID NO: 21461) and a MAGE-A2 peptide (SEQ ID NO: 21464) were generated and expressed in JNG cells by transient infection with the corresponding lentiviruses and without further drug selection. The SEQ ID NO of these constructs is represented by SEQ ID NO: 22256-22257 and 22258-22262, respectively. The cells showed the ability to respond to Raji cells (CD19⁺/CD20⁺) and HepG2 cells (HLA-A*02:01/MAGE-A3⁺ and HLA-A*02:01/NY-ESO-1⁺) upon co-culture as measured by GFP induction **(Table 49).** The cells are also tested for binding to NY-ESO-1 peptide/HLA-A02:01 tetramers and MAGE-A02:01 peptide/HLA-A02:01 tetramers by flow cytometry.

Additional SAR constructs are generated comprising two or more svd-TCR that are either attached to two separate chains of a double chain SAR or attached in tandem to one of the chains via an optional short linker (e.g., G4S linker). These constructs are also shown to bind to and respond to their respective target antigen expressing cells.

Additional constructs are generated by fusing one or more svd-TCRs to the N-terminus of an encoded scFv of a 2^{nd} generation CAR, TFPε, TFPγ or TFPδ. These constructs are also shown to bind to and respond to their respective target antigen expressing cells. These results demonstrate that bispecific and multispecific SARs encoding one or more svd-TCR can be generated by fusing the svd-TCR to the N-terminus or near the N-terminus of the vL, vH, Va, Vb, Vg, Vd, scFv, AABD domains comprising a CAR, SIR, Ab-TCR, AABD-TCR, TFP or TCR.

| **TABLE 49: SARs comprising svd-TCR** | | | | |
|---|---|---|---|---|
| **Antigen** | **SEQ ID NO (DNA)** | **SEQ ID NO (PRT)** | **Constructs Name** | **NFAT-GFP ASSAY** |
| CD19, CD20, NY-ESO-1/HLA.A02:01 | 22256 | 23071 | NY-ESO-sVb-TCR-G4Sv2-hu-mROO5-vL- [hTCRb-S57C]-F-P2A-IgHSP-CD20-VHH- USC1-2HCD26-G4S-CD19-hu-mROO5-1- vH-[hTCRa-T48C-opt]-F-F2A-PAC | RAJI (+/-), HepG2 (-/+) |
| CD19, CD20, NY-ESO-1/HLA.A02 | 22257 | 23072 | NY-ESO-sVb-TCR-G4Sv2-hu-mROO5-vL- IgCL-TCRb-wt2-opt-6MD-F-P2A-IgHSP- CD20-vHH2-HCD25-G4S-CD19-hu- mROO5-1-vH-IgG1-CH1-TCRa-wt2-opt- 6MD-F-F2A-PAC | RAJI (2+), HepG2 (-/+) |
| CD19, CD20, MAGE-A3/HLA.A02 | 22258 | 23073 | MAGE-A3-sVb-7-G4Sv3-hu-mROO5-vL- [hTCRb-KACIAH]-F-P2A-IgHSP-CD20- vHH2-HCD25-G4S-CD19-hu-mROO5-1- vH-[hTCRa-CSDVP]-F-F2A-PAC | RAJI (2.5+), HepG2 (2+) |
| CD19, CD20, MAGE-A3/HLA.A02 | 22259 | 23074 | MAGE-A3-sVb-7-G4Sv3-hu-mROO5-vL- [hTCRb-S57C]-F-P2A-IgHSP-CD20-VHH- USC1-2HCD26-G4S-CD19-hu-mROO5-1- vH-[hTCRa-T48C-opt]-F-F2A-PAC | RAJI (-/+), HepG2 (2+) |
| CD19, CD20, MAGE-A3/HLA.A02 | 22260 | 23075 | MAGE-A3-sVb-7-G4Sv3-hu-mROO5-vL- IgCL-TCRg-6MD-F-P2A-IgHSP-CD20- vHH2-HCD25-G4S-CD19-hu-mROO5-1- vH-IgG1-CH1-TCRd-6MD-F-F2A-PAC | RAJI (-/+), HepG2 (1.5+) |
| CD19, CD20, MAGE-A3/HLA.A02:01 | 22261 | 23076 | MAGE-A3-sVb-7-G4Sv3-hu-mROO5-vL- IgCL-TCRb-wt2-opt-6MD-F-P2A-IgHSP- CD20-vHH2-HCD25-G4S-CD19-hu- mROO5-1-vH-IgG1-CH1-TCRa-wt2-opt- 6MD-F-F2A-PAC | RAJI (+), HepG2 (1+) |
| CD19, CD20, MAGE-A3/HLA.A02:01 | 22262 | 23077 | MAGE-A3-sVb-7-G4Sv3-hu-mROO5-vL- huTCRg-d5-F-P2A-IgHSP-CD20-vHH2- HCD25-G4S-CD19-hu-mROO5-1-vH- huTCRd-d2-F-F2A-Pac | RAJI (+), HepG2 (1.5+) |

### Expression of bispecific and multispecific SAR on the backbones of SIR, cTCR, AABD-TCR, Ab-TCR and TFPαβ in T cells with impaired TCRα or TCRβ expression.

The SAR constructs on SIR, AABD-TCR and Ab-TCR backbones are also expressed in JNG cells in which the expression of TCRα, TCRβ or both has been eliminated by CRISPR/Cas9 mediated gene knock-out. In contrast to the results with SARs on the TFP backbone, it is observed that the SAR constructs on the above backbones are effectively expressed on the surface of TCRα-/-, TCRβ-/- and TCRα-/-β-/- cells at similar or higher levels as observed in the wild-type JNG cells and have robust signaling ability when exposed to their target antigen expressing cells. Essentially similar results are obtained when the SAR constructs on the SIR, cTCR, AABD-TCR, Ab-TCR and TFPαβ backbones are inserted at the TRAC or TRBC genomic loci so that they are expressed under the promoter and regulatory elements of endogenous TCRα or TCRβ genes. The insertion of SAR constructs at the TRAC and TRBC loci, which results in simultaneous disruption of these genes, is achieved using gene targeting as described in described in Knipping F et al, Molecular Therapy: Methods & Clinical Development, Vol 4, 2017 and Eyquem J et al Nature, 543(7643):113-117, 2017, respectively. An exemplary targeting construct to direct the SAR to the TRAC locus is represented by SEQ ID NO: 23. Additional targeting constructs can be generated by replacing the module encoding the SAR in this construct with module encoding a different SAR on the SIR, cTCR, Ab-TCR, AABD-TCR, TFPαβ, TFPγδ and TCR backbones using techniques known in the art.

### In vitro and in vivo analysis of Bispecific and multispecifc

### In vitro and in vivo analysis of Bispecific and multispecifc SARs

A panel of bispecific and multispecific SAR constructs (SEQ ID NO: 22098, 22104-22108, 22165) are expressed in T cells and tested for in vitro cytotoxicity against RAJI, RAJI-CD19-KO, NALM6 and HL60 cells. The unispecific SAR constructs with SEQ ID NO: 22101-22103 are used as controls. The bispecific and multispecific constructs are shown to possess robust cytotoxicity against both CD19+ RAJI and RAJI-CD19-KO cells as well as against NALM6 cells while the unispecific constructs are inactive against RAJI-CD19-KO cells. The SAR-T cells are tested in an in vivo model of RAJI and RAJI-CD19-KO cells xenografted in NSG mice. The bispecific and multispecific SAR-T cells show robust antitumor activity against both cell lines while the unispecific constructs are inactive against RAJI-CD19-KO cells

### Miscellaneous SAR constructs

The bispecific constructs in which the vHH or FHVH or Centyrin or affibody or other non-immunoglobulin antigen binding domain is operably linked to the N-terminus of the vL or the vH fragment of a double chain SIR retain their activity against two or more antigens. Thus, the following bispecific constructs were positive in JNG-GFP induction assays against both RAJI WT cells that express CD19 and RAJI CD19 KO A7 cells that lack CD19 expression. These clones were also highly positive against DAUDI and RS;411cells. In contrast the corresponding unispecific constructs were negative against RAJI CD19 KO A7 cells suggesting that they are unable to recognize and signal in response to CD20 antigen that is also expressed on RAJI wild-type and RAJI CD19 KO A7 cells. Such bispecific constructs are represented by the following sequence and SEQ ID Nos. In the constructs represented by (SEQ ID NO: 4090 and SEQ ID NO: 15711), (SEQ ID NO: 4088 and SEQ ID NO: 15709) and (SEQ ID NO: 4089 and SEQ ID NO: 15710), the CD20-vHH-2HCD25 binding domain is operably linked to the N-terminus of hu-mROO5-1-vH which in turn is operably linked to [hTCRa-T48C], [hTCRb-KACIAH] or [hTCRa-CSDVP] TCR constant chains, respectively. The hu-mROO5-1-vL chain in these constructs is operably linked to [hTCRb-S57C], [hTCRa-CSDVP] or [hTCRb-KACIAH] chains, respectively. Thus, in these three constructs the hu-mROO5-1-vL and hu-mROO5-1-vH combine to form a Fv that targets CD19, while the AABD represented by CD20-vHH-2HCD25 targets CD20. The above results demonstrate that attachment of CD20-vHH-2HCD25 to the N-terminus of hu-mROO5-1-vH does not interfere with the ability of hu-mROO5-1-vH to combine with the hu-mROO5-1-vL chain to form the Fv fragment. The above results further demonstrate that resulting Fv fragment in which the CD20-vHH-2HCD25 is operably linked to the N-terminus of hu-mROO5-1-vH still retains its ability to recognize and bind to CD19 and induce T cell signal upon binding to CD19-expresing cells.

In contrast to the above constructs, the bispecific SAR represented by CD8SP-CD20-vHH-C07-G4S-CD19-huCD19USC3-vL-[hTCRb-S57C-opt]-F-P2A-SP-huCD19USC3-vH-[hTCRa-T48C-opt]-F-F2A-PAC (SEQ ID NO: 1330 and SEQ ID NO: 12020) has the backbone of a SIR comprising the CD19-huCD19U5C3-vL and CD19-huCD19USC3-vH fragments which combine to form a Fv which targets CD19. In this SAR, the AABD targeting CD20 and represented by CD20-vHH-C07 is operably linked to the N-terminus of CD19-huCD19USC3-vL chain. This SAR construct is able to induce T cell signaling and GFP expression when co-cultured with RAJI (CD19+/CD20+), RAJI CD19 KO A7 (CD19-/CD20+), DAUDI (CD20+) and RS411 (CD19+) cells. In comparison the unispecific construct CD8SP-huCD19-USC3-vL-[hTCRb-S57C]-F-P2A-SP-huCD19-USC3-vH-[hTCRa-T48C]-F-F2A-PAC (SEQ ID NO: 1244 and SEQ ID NO: 11934) is not able to induce GFP expression when co-cultured with RAJI CD19 KO A7 (CD19-/CD20+) cells. The above results demonstrate that attachment of an AABD (i.e., CD20-vHH-C07) to the N-terminus of CD19-huCD19USC3-vL via a Gly-Ser linker does not interfere with the ability of the vL fragment to combine with its complementary vH fragment (i.e., huCD19-USC3-vH) to form a Fv that can recognize the target antigen (i.e., CD19) and induce cell signaling. The above results further demonstrate that an AABD (e.g., a vHH or SVH) which is operably linked to the N-terminus of a vL fragment retains its ability to bind to its target antigen and induce T cell signaling when expressed as part of a SAR.

It was observed that JNG cells expressing the construct CD8SP-CD22-FHVH-24-[IgCL-TCRg-6MD]-F-P2A-SP-BCMA-FHVH-93-[IgG1-CH1-TCRd-6MD]-F-F2A-PAC which is represented by SEQ ID NO: 6821 and SEQ ID NO: 17511 strongly induced GFP expression when co-cultured with RAJI (CD19+) and L363 (BCMA+) suggesting that they can recognize and respond to both CD22 and BCMA. Essentially, the same results were obtained when the experiment was repeated with JNG cells expressing the construct CD8SP-CD22-FHVH-24-[IgCL-TCRb-wt-opt2-6MD]-F-P2A-SP-BCMA-FHVH-93-[IgG1-CH1-TCRa-wt-opt2-6MD]-F-F2A-PAC which has the SEQ ID NO: 6812 and SEQ ID NO:17502 respectively.

As the SARs are modular in design the sequences of different SARs described in this disclosure can be determined from their names which describe the orders of their various component modules.

### USE OF SMAC MIMETICS FOR GENERATION OF SAR-T CELLS.

T cells were infected with a SAR (SEQ ID NO: 7348) with the backbone of a SIR targeting CD19 and then expanded in T cell culture medium (XVIVO medium supplanted with 10 ng/ml CD3 antibody, 10ng/ml CD28 antibody and 100 IU recombinant human-IL2 at 37°C in a 5% CO2 humidified incubator in the absence and presence of two different concentrations of a SMAC mimetic Birinapant (100nM or 1µM) for 16 days. Uninfected T cells (T-UI) and SIR-T cells were tested for cytotoxicity against RAJI target cells expressing Gluc using MATADOR Assay at E:T ratios 0.25:1 and 0.5:1 for 4h in 384-well plate. Matador assay was performed by adding 1:100 CTZ in PBS 15µl/well in well mode using injectors. Expansion of SAR-T cells in the presence of SMAC mimetic resulted in dose dependent increase in cell killing of RAJI cells while it has no effect on non-specific killing by the uninfected control T cells.

T cells were activated overnight with CD3/CD28 beads followed by three infections with CD19-targeted 2^{nd} generation CAR (SEQ ID No: 7343) and a CD19 targeted SIR CD8SP-hu-mROO5-1-vL-[hTCRb-S57C]-F-P2A-SP-hu-mROO5-1-vH-[hTCRa-T48C]-E02 (SEQ ID NO: 7348). Cells were expanded in XVIVO medium under standard conditions in the presence of IL2 and CD3/CD28 beads in the absence or presence of Birinapant 1µM for 7 days and. After 7 days, cells were analyzed by flow cytometry for expression of different cell surface markers. In vitro expansion of CAR-T and SIR-T cells in the presence of Birinapant was shown to result in increase in proportion of CD8 cells, a decline in CD4 cells and increase in CD62L cells, which is a marker of naive T cells with stem like properties.

1 × 10⁵ T cells expanded for 7 days in the presence of Birinapant were co-cultured with 1 × 10⁵ Nalm6 cells for 48h in 96-well plate. After 48h, supernatant was collected and used for measurement of IFNγ, TNFα and IL-2 by ELISA. There was no significant decrease in IFNγ, TNFα or IL2 secretion following treatment with Birianapant.

To test the *in vivo* efficacy of the different CAR-T cells, NSG mice were injected via tail vein with 0.5 × 10⁶ Nalm6 cells stably expressing heat stable luciferase LucPPE (NALM6-LucPPE) and three days later injected with 5 × 10⁶ control T cells and T cells expressing the 2^{nd} generation CAR constructs represented by SEQ ID No: 7343 and a SIR represented by SEQ ID NO: 7348. Animals were imaged weekly by bioluminescence imaging following injection of D-luciferin. There was significant tumor growth in animals given control T cells and they all died by day 40. The animals given T cells expressing SEQ ID NO: 7343 and 7348 that had been expanded for 7 days in the absence or presence of Birinapant (1µM) cleared the disease and remained disease free until day 100 with the exception of one animal given T cells expressing SEQ ID NO 7343 that had been expanded in the presence of Birinapant. Overall, the above results demonstrate that T cells expressing SIR retain their in vitro and in vivo efficacy when expanded in the presence of Birinapant. The results further demonstrated that cells expressing LucPPE can be used for monitoring in vivo growth of cancer cells via bioluminescence imaging.

### Use of Gas-permeable Rapid Expansion G-Rex) Cultureware for expansion of SIR-T cells

Primary human T cells were infected three times with CD19-targeted 2^{nd} generation CAR (SEQ ID No: 7343) and a CD19 targeted SIR (SEQ ID NO: 7348). One set of T-CAR cells was cultured in G-Rex 6-well plates (Cat. # 80240M, Wilson Wolf Corporation), where 1.25 million T cells were plated in each well containing 35 ml of T cell culture medium.

Fresh media was changed in G-Rex plates after every 5 days and after 3 days in cells cultured in regular culture flasks under normoxic conditions.

Fresh media was changed in G-Rex plates after every 7 days in Hypoxia experiment and after 3 days in cells cultured in regular culture flasks.

For hypoxic conditions a 4% O2 humidified incubator was used instead of 5% CO2 humidified incubator for normal conditions.

After 3 infections, cells were analyzed by flow cytometry on Day 5 with Protein L and approximately 45% of cells were found to express SIR on cell surface. Cells were pelleted and re-suspended in XVIVO-15 (Lonza) T-cell medium supplemented with PSA, 100 IU/ml recombinant human IL2 and Dynabeads Human T-Activator CD3/CD28 and 5% hAB serum. One set of SIR-T cells was cultured in G-Rex 6-well plates (Cat. # 80240M, Wilson Wolf Corporation), where 1.25 million T cells were plated in each well containing 35 ml of T cell culture medium. Fresh media was changed in G-Rex plates after every 5 days. The other set of SIR-T cells were cultured in 10 ml of medium in a regular tissue culture flask. Fresh media was changed in G-Rex plates after every 5 days and after 3 days in cells cultured in regular culture flasks. After 14 days expansion, cells were tested for cytotoxicity against Nalm6-Gluc cells using Matador assay. SIR-T cells retained their cytotoxicity against target cells when grown in GRex flasks.

The cells were analyzed by flow cytometery for expresson of SIR by staining with Protein L and for presence of different T cell markers. SIR-T cells expanded in a G-Rex flask showed significantly higher expression of SIR as determined by Protein L staining (53.7% vs 46.53%) and higher proportion of CD8 cells (80.34% vs 68.37%), CCR7 cells (65.69% vs 36.34%), CD62L (62.87% vs 51.99%) and CD45RA (91.94% vs 45.91%). As CCR7+/CD45RA+/CD62L+ cells are known to possess features of stem like T cells, the above results indicate that culture of SIR-T in GRex flasks results in expansion of cells with immunophenotype of stem like T cells (Tscm) and prevent their terminal differentiation. The results further demonstrate that expansion of SIR-T cells in GRex flasks results in preferential expansion of CD8 cells.

Control T cells or SIR-T cells grown in regular flask or G-Rex 6-well plate were cultured with Nalm6-hGLuc 100K/well/100ul in 96-well plate U-bottom in XVIVO medium with no additional component. After 48h co-culture, supernatant was collected and used for ELISA. SIR-T cells expanded in GRex plates were shown to produce more IFNγ, TNFα and IL2 when cultured with NALM6 cells expressing their target antigen (e.g., CD19) as compared to SIR-T cells expanded in regular flask. In addition, the baseline (i.e., in the absence of NALM6 cells) production of IFNγ and TNFα was lower in SIR-T cells expanded in GREx flask suggesting low tonic signaling.

### In vivo comparison of SIR-T cells expanded in regular flask and GRex Flasks

SIR-T cells from the preceding experiment that were expanded in regular T75 flask or GRex flask were compared for in vivo efficacy using a Nalm6-LucPPe xenograft model in NSG mice. For this purpose, 0.5 × 10⁶ Nalm6 cells expressing LucPPe were injected into NSG mice via tail vein injection. Two days later, animals received injection of increasing number of SIR-T cells (2 × 10⁶, 4 × 10⁶, 8 × 10⁶) per animal. Animals were imaged using bioluminescence imaging following injection of D-Luciferin. The animals given SIR-T cells expanded in GRex flask showed superior control of leukemia as determined by bioluminescence imaging which also resulted in improvement in survival. This experiment also demonstrate that LucPPe and other thermostable beetle luciferases can be used for in vivo monitoring of tumor growth by bioluminescence imaging following injection of D-luciferin.

The experiment is repeated by expansion of Ab-TCR-T cells, TFP-T, TAC-T cells targeting CD19. It is observed that expansion of Ab-TCR-T and TFP-T cells under GRex flasks confer superior expansion, retention of T stem cell phenotype and superior control of tumor in vivo. Finally, the experiment is repeated by expansion of bispecific and multispecific SIR-T, Ab-TCR-T and TFP-T and again expansion using GRex flasks is shown to result in not only superior expansion but also superior in vitro and in vivo characteristics.

### Expansion in G-Rex plates under hypoxic conditions

The experiment in the preceding section was repeated using a CD19-targeted 2^{nd} generation CAR (SEQ ID No: 7343) and a CD19 targeted SIR (SEQ ID NO: 7348). T cells were infected three times with the lentiviral vectors encoding the SIR and CAR and then 1 million cells infected with each SIR/CAR construct were cultured in regular T75 flask or GRex flask as described in the preceding section. One set of each flasks were cultured under normal oxygenation conditions while the other set was cultured under hypoxic conditions. For hypoxic conditions a 4% O2 humidified incubator was used instead of 5% CO2 humidified incubator for normal conditions.

After 13 days of culture, cells were counted and analyzed by flow cytometry. Expansion of cells under hypoxic conditions in normal tissue culture flasks led to lower level of expansion. Thus, the uninfected T cells (T-UI), CAR-T and SIR-T cultured in regular flask expanded from 1 million to 6, 6 and 9 million cells, respectively, under normoxic conditions but expanded to 4.4, 5.2 and 6 million cells. respectively under hypoxic conditions. In contrast, expansion in GRex flasks led to 80, 85, 84 million cells, respectively, under normoxic conditions and 70, 70 and 72 million cells, respectively, under hypoxic conditions. Thus, expansion in GREx flasks not only leads to much greater expansion of CAR-T and SIR-T cells but also results in significant expansion even under hypoxic conditions thereby allowing production of sufficient cells for clinical administration. This point is important as failure to produce sufficient number of CAR-T and SIR-T cells is a major cause of manufacturing failure in CAR-T and SIR-T field

Further expansion of CAR-T and SIR-T in GREX flask under hypoxia resulted in higher proportion of T cells expressing the CAR and SIR, higher percentage of CD8+ cells and higher percentage of cells expressing the markers associated with stem like T cells (i.e., CD45RA, CCR7 and CD62L).

The cells expanded under different conditions were tested for cytotoxicity against NALM6-cells expressing Gluc using the Matador assay. All cell populations were shown to demonstrate cytotoxicity. The supernatant from the different cell populations were tested for cytokine production upon co-culture with NALM6 cells. All cell populations were shown to produce IFNγ, TNFα and IL2 upon co-culture with NALM6 cells.

Finally, the different cell populations were compared for in vivo efficacy in a NALM6 xenograft model using NSG mice. The experiment was performed as in the preceding example by injecting 0.5 million NALM6-LucPPE cells via tail vein followed 2 days later with 2 million CAR-T or SIR-T cells grown under normoxic or hypoxic conditions in GREX flasks. The results showed that SIR-T cells expanded in GREX flask under hypoxic conditions performed equally well as those expanded under normal conditions while CAR-T cells expanded under hypoxic conditions performed worse than those expanded under normoxic conditions.

### Comparative analysis of Mesothelin (MSLN) SARs with different antigen binding domains

Mesothelin is highly expressed in several cancers but also shows low level expression on normal healthy tissues. To reduce affinity tuned SARs targeting MSLN, several mutants of MSLN specific humanized antibody MSLN-hu22A10 and MSLN-7D9 were generated. The SEQ ID NO of the vL, vH fragments and their CDR regions are provided in **Table 39.** The different mutant and wild-type vL and vH fragments were assembled to make SAR constructs on the backbone of double chain SIR. The name, chain composition and SEQ ID of the different SAR constructs are provided in **Table 50.** JNG cells were generating expressing the different SAR constructs targeting MSLN cloned in the pCCLc-MNDU3-WPRE vector (SEQ ID NO: 4). In case of constructs co-expressing PAC, cells were also selected with Puromycin. Cells were then tested for GFP transduction following incubation with and SKOV3 (MSLN-high), MCF7 (MSLN-low), PC3 (MSLN-medium), MDA-MB-231 (MSLN low), LNCaP (MSLN low), Huh-7 (MSLN-low) and RAJI (MSLN-ve) cell lines, respectively. As shown in the **Table 50,** different MSLN SAR cells showed varying level of GFP induction upon co-culture with the target cell lines with different levels of MSLN expression. The JNG cells expressing the SAR with SEQ ID NO(DNA): 21873 and SEQ ID NO (PRT): 22688 showed the strongest GFP induction in response to SKOV-3 and MDA-MB-231 cell lines that express high levels of MSLN but also showed modest GFP induction when co-cultured PC3 cells that express low to moderate amounts of MSLN but did not show GFP induction on co-culture with MCF7 cells that express low levels of MSLN. This SAR comprises hu22A10-N31S-vL and hu22A10-F27Y-vH fragments. The SEQ ID NO of these fragments and their CDR1-3 are provided in **Table 39.** The SAR with SEQ ID NO: 22691 with similar but slightly weaker. This SAR also showed significant GFP induction when co-cultured with PC3 cells showing moderate levels of MSLN. This SAR comprise hu22A10-N31S-vL and wild-type hu22A10-vH fragments. The SAR with SEQ ID NO (PRT): 22687, 22692 showed weaker but sufficient GFP induction with SKOV3 and none to negligible GFP induction with PC3 cell line. The SAR with SEQ ID NO:22694 showed the lowest GFP induction among the panel against SKOV-3 cells. This SAR comprises MSLN-hu22A10-Y96W-vL and wild-type MSLN-hu22A10-vH fragments.

These results demonstrate that it is possible to generate affinity tuned and sensitivity modified MSLN targeted SARs by incorporating different mutations in the vL and vH fragments. The SARs with activity against moderate MSLN expressing cells (e.g., PC3) may be more suitable if disease relapse due to antigen escape is a concern. In contrast, the MSLN SAR with negligible or no activity against moderate MSLN expressing cells (e.g., PC3 cells) may be more suitable for debilitated patients or where safety is the primary concern.

These vL and vH fragments are used to generate a diverse panel of MSLN targeting unispecific, bispecific and multispecific SARs whose SEQ ID NO: are provided in SEQ ID NO: 22540-22581. Additional mutants of MSLN-hu22A10 can be generated and can be used in alternate embodiment of the disclosure. The SEQ ID NO of other MSLN targeted vL, vH, scFv fragments baseds are also provided in Table 3 and 39. These vL, vH and scFv fragments are used to generate a diverse panel of MSLN targeting unispecific, bispecific and multispecific SARs.

In one embodiment, the disclosure provides a MSLN-targeted SAR, which when expressed in JNG cells causes less than 10% induction of GFP when the JNG cells expressing the SAR are co-cultured for 24 hours with PC3 cells. In one embodiment, the disclosure provides a MSLN-targeted SAR, which when expressed in JNG cells causes less than 10% induction of GFP when co-cultured for 24 hours with a MSLN negative cell line. In one embodiment, the disclosure provides a MSLN-targeted SAR, which when expressed in JNG cells cause more than 20% induction of GFP when co-cultured for 24 hours with SKOV3 cells.

T cells expressing the different SAR constructs targeting MSLN are generated and demonstrate in vitro cytotoxicity against PC3 and SKOV3 cells using Matador assay. The SAR-T cells are also shown to induce cytokine production (TNFα, IFNγ, and IL2) when exposed to target cells. The T cells expressing the SAR with SEQ ID NO: 22686-22695 are tested for in vivo anti-tumor activity against a SKOV-3 xenograft model using NSG immunodeficient mice. The corresponding 2^{nd} generation CARs on the BBz background are used as controls. T cells expressing the SAR with SEQ ID NO: 22685-22695 show effective tumor control as measured by bioluminescence imaging and result in improved animal survival. The T cells expressing the SARs with SEQ ID NO: 22685-22695 also show superior in vivo activity as compared to the 2^{nd} generation CARs comprising the 4-1BB costimulatory domain and CD3z activation domain.

| **TABLE 50: MSLN targeting SARs** | | | |
|---|---|---|---|
| **SEQ ID NO (DNA)** | **SEQ ID NO (PRT)** | **Constructs Name** | **NFAT-GFP ASSAY** |
| 21871 | 22686 | CD8SP-MSLN-hu22A10-vL-[hTCRb-S57C]-F- P2A-SP-MSLN-hu22A10-vH-[hTCRa-T48C- opt]-F-F2A-PAC | SKOV-3 (2+) |
| 21872 | 22687 | CD8SP-MSLN-hu22A10-vL-Y96W-[hTCRb- S57C]-F-P2A-SP-MSLN-hu22A10-F27Y-vH- [hTCRa-T48C-opt]-F-F2A-PAC | SKOV-3 (1.5+), MCF-7 (-), PC-3 (-), MDA-MB-231 (2+) |
| 21873 | 22688 | CD8SP-MSLN-hu22A10-vL-N31S-[hTCRb- S57C]-F-P2A-SP-MSLN-hu22A10-F27Y-vH- [hTCRa-T48C-opt]-F-F2A-PAC | SKOV-3 (5+), MCF-7 (-), PC-3 (3+), MDA-MB-231 (5+) |
| 21874 | 22689 | CD8SP-MSLN-hu22A10-vL-[hTCRb-S57C]-F- P2A-SP-MSLN-hu22A10-F27Y-vH-[hTCRa- T48C-opt]-F-F2A-PAC | SKOV-3 (3+) |
| 21876 | 22691 | CD8SP-MSLN-hu22A10-vL-N31S-[hTCRβ- S57C]-F-P2A-SP-MSLN-hu22A10-vH-[hTCRα- T48C-opt]-F-F2A-PAC | Raji (-), SKOV-3 (2.5+), MDA-MB-231 (2.5+), MCF-7 (-), PC-3 (2+), LNCaP (-), Huh-7 (-) |
| 21877 | 22692 | CD8SP-MSLN-hu22A10-vL-Y96W-[hTCRβ- S57C]-F-P2A-SP-MSLN-hu22A10-vH-[hTCRα- T48C-opt]-F-F2A-PAC | Raji (-), SKOV-3 (1.5+), MDA-MB-231 (+), MCF-7 (-), PC-3 (+), LNCaP (-), Huh-7 (-) |
| 21878 | 22693 | CD8SP-MSLN-hu22A10-vL-N31S-[hTCRβ- S57C]-F-P2A-SP-MSLN-hu22A10-vH-[hTCRα- T48C-opt]-F-F2A-PAC | SKOV-3 (2.5+) |
| 21879 | 22694 | CD8SP-MSLN-hu22A10-vL-Y96W-[hTCRβ- S57C]-F-P2A-SP-MSLN-hu22A10-vH-[hTCRα- T48C-opt]-F-F2A-PAC | SKOV-3 (+) |
| 21880 | 22695 | CD8SP-MSLN-7D9-vH-[hTCRβ-S57C]-F-P2A- SP-MSLN-7D9-vL-[hTCRα-T48C-opt]-F-F2A- PAC | Raji (-), SKOV-3 (1.5+), MCF-7 (-), PC-3 (+), LNCaP (-), MDA-MB-231 (-), Huh-7 (-) |

T cells expressing the above MSLN SARs are shown to induce cytotoxicity in MSLN-expressing SKOV3 cells using Matador assay and induce cytokine production (e.g., TNFα, IFNγ, IL-2 etc.).

Next, the SAR-T cells are tested in a SKOV3 xenograft model in NSG mice. For this purpose, 1 × 10⁶ SKOV3-Luc cells are injected subcutaneously followed a week later by injection of intravenous injection of 3 × 10⁶ SAR-expressing T cells. Mice given SAR-T cells show varying level of tumor inhibition and improvement in survival. Mice given SAR-T with SEQ ID NO: 10315, 10384, 10453,10522, 10591 and 10660 show tumor inhibition and improvement in survival. Mice given SAR-T with SEQ ID NO: 10311, 10380, 10449, 10518, 10587 and 10656 show weak protection.

### Comparison of BCMA SARs

The BCMA SAR CD8SP-BCMA-hu72-vL-[hTCRb-S57C]-F-P2A-SP-BCMA-hu72-vH-[hTCRa-T48C-opt] (SEQ ID NO: 1473) with the backbone of double chain SIR was selected for further optimization by mutagenesis of its vL and vH chains. The mutant constructs were cloned in the lentiviral vector with or without a puromycin resistance gene. The nucleic acid and amino acid SEQ ID NO: of the different constructs are represented by SEQ ID NO (DNA): 21843-21870 and SEQ ID NO (PRT): 22658-22685, respectively. The constructs were expressed in JNG cells using lentiviral mediated gene transfer. In the case of constructs carrying puromycin resistance gene, the JNG cells were selected with puromycin to generate stably transduced cells. The JNG cells expressing the different SAR constructs were compared for their ability to induce GFP expression upon overnight co-culture with L363 cells which express BCMA. The different constructs showed varying level of NFAT driven GFP induction. The SAR constructs represented by SEQ ID NOs: 21843-21853, 21861, 21862 and 21870 showed consistently higher GFP induction as compared to constructs represented by SEQ ID NO: 21854-21860, 21863-21869. The highest GFP induction was seen with the constructs SP-BCMA-hu72-vL-S29G-[hTCRb-S57C]-F-P2A-SP-BCMA-hu72-vH-G32S-[hTCRa-T48C-opt] (SEQ ID NO: 21853) and SP-BCMA-hu72-vL-I27L-[hTCRb-S57C]-F-P2A-SP-BCMA-hu72-vH-S64T-[hTCRa-T48C-opt] (SEQ ID NO: 21852), respectively. JNG cells expressing these constructs also showed no background GFP induction reflecting lack of tonic signaling. In addition, JNG cells expressing these constructs showed no GFP induction upon co-culture with HL60 and THP-1 cell lines that do not express BCMA. These constructs have S29G (SEQ ID NO: 10934) and I27L (SEQ ID NO: 10933) mutations in their vL regions and G32S (SEQ ID NO: 11179) and S64T (SEQ ID NO: 11173) mutations in their vH regions. The scFv comprising S29G and G32S is represented by SEQ ID NO: 11422.

A number of unispecific and bispecific SARs constructs on different backbones (e.g., 2^{nd} generaiton CAR, SIR, Ab-TCR, zSIR, TFPε, TFPγ, TFPδ etc) comprising the above mutations are represented by SEQ ID NO: 7409-7753, 7892-8374 and can be used to generate a diverse immune response.

T cells are generated to express the following BCMA SARs constructs and are shown to induce cytotoxicity in BCMA-expressing L363 cells using Matador assay and to induce cytokine production (e.g., TNFα, IFNγ, IL-2 etc.) when co-cultured with L363 cells.

Next, the SAR-T cells are tested in a L363 xenograft model in NSG mice. For this purpose, 0.5 × 10⁶ L363-Luc cells are injected i.v. via tail vein followed 3 days later by intravenous injection of 2 × 10⁶ SAR-expressing T cells. Mice given SAR-T cells show varying level of tumor inhibition and improvement in survival. Mice given SAR-T with SEQ ID NO: 21852 and 21853 show superior disease control and prolongation of survival as compared to mice given control T cells.

Bispecific and biparatopic SAR constructs targeting BCMA and CD20 were constructed on the backbone of SAR with SEQ ID NO: 21853. These constructs are represented by SEQ ID NO (DNA): 22151-22155 and SEQ ID NO (PRT): 22966-22970. These SAR constructs were expressed in JNG cells and tested for induction of GFP upon overnight co-culture with a panel of cell lines expressing BCMA or CD20 or both. The bispecific and biparatopic constructs with SEQ ID NO: 22151-22152 showed GFP induction when co-cultured with the BCMA-expressing L363 (BCMA+) and U266 cells but did not show GFP induction when co-cultured with NALM6 cells that lack BCMA but express low level of CD20 **(Table C2).** In contrast, the bispecific SAR constructs with SEQ ID NO: 22153-22155 showed GFP induction with L363, U266, NALM6 and RAJI cells but showed negletible non-specific GFP induction when co-cultued with RS:411 cells that lack both BCMA and CD20 expression. Thus, T cells expressing the SAR constructs with SEQ ID NO: 22153-22155 can recognize more than one antigen and respond robustly with induction of NFAT signaling pathway.

The T cells expressing the bispecific and multispecific SAR constructs are tested for in vivo efficacy using a panel of cell lines expressing different antigens using xenograft model in NSG mice. The T cells expressing the bispecific and multispecific SAR constructs are shown to possess in vivo activity against a diverse panel of cell lines that express one or more of the antigens targeted by them. The T cells expressing the bispecific and multispecific SAR constructs are also shown to possess activity against cell lines that have down-regulated or lost the expression of one or more of their target antigens as long as they continue to express at least one of the antigens targeted by the SAR.

### Comparison of Her2 SARs

JNG cells were generating expressing the different SAR constructs targeting Her2 cloned in the pCCLc-MNDU3-WPRE vector (SEQ ID NO: 4). JNG cells were infected with lentiviral vectors encoding the different SAR constructs targeting Her2 with the backbone of double chain SIRs. The vL and vH fragments of these constructs were derived from Herceptin or one of its variants. In case of constructs co-expressing PAC, cells were also selected with Puromycin. Cells were then tested for GFP transduction following incubation with and SKOV3 (Her2-high), MCF7 (Her2-medium), PC3 (Her2-low) and MDA-MB-231 (Her2 low) cell lines, respectively. As shown in the **Table** 51, different Her2 SAR constructs showed varying level of GFP induction. The SAR constructs with SEQ ID NO: 21895 and 21897 in which the vL fragment of huMab4D5 or its mutant is attached to TCRb constant chain or one of its variants (KACIAH), showed strong GFP induction when co-cultured with Her2-high and Her2-low expressing cell lines. In contrast, the SAR constructs with SEQ ID NO: 21894, 21896, 21898 and 21899 showed modest activity against Her2 high expressing cell lines but low to negligible activity against Her2 low expressing cell lines. These results demonstrate that it is possible to modify the sensitivity of Her targeted SARs by incorporating different mutations in the vL and vH fragments and by attaching them to different TCR constant chains.

The SEQ ID NO of the exemplary vL, vH and scFv fragments based on humanized Her2 4D5 antibody and its variants are provided in **Table 3.** These vL and vH fragments are used to generate a diverse panel of Her2 targeting unispecific, bispecific and multispecific SARs whose SEQ ID NO: are provided in SEQ ID NO: 8858-8926 and 21683-21703. Additional mutants of 4D5 are known in the art and can be used in alternate embodiment of the disclosure. The SEQ ID NO of other Her2 targeted vL, vH, scFv fragments based on Her2-USC-1516 and Her2 FRP5 antibodies are also provided in **Table 3.** These vL, vH and scFv fragments are used to generate a diverse panel of Her2 targeting unispecific, bispecific and multispecific SARs provided in **Table 36** (SEQ ID NO: 8927-8995 and 8651-8719).

In one embodiment, the disclosure provides a Her2-targeted SAR, which when expressed in JNG cells causes less than 10% induction of GFP when the JNG cells expressing the SAR are co-cultured for 24 hours with MDA-MB-231. In one embodiment, the disclosure provides a Her2-targeted SAR, which when expressed in JNG cells causes less than 10% induction of GFP when co-cultured for 24 hours with a Her2 negative cell line. In one embodiment, the disclosure provides a Her2-targeted SAR, which when expressed in JNG cells cause more than 20% induction of GFP when co-cultured for 24 hours with SKOV3 cells.

T cells expressing the different SAR constructs targeting Her2 are generated and demonstrate in vitro cytotoxicity against MCF7 and SKOV3 cells using Matador assay. The SAR-T cells are also shown to induce cytokine production (TNFα, IFNγ, and IL2) when exposed to target cells. The SAR-T cells also show in vivo anti-tumor activity when tested in a xenograft model using NSG immunodeficient mice.

**TABLE 51: Her2 targeting SARs**

| **SEQ ID NO (DNA)** | **SEQ ID NO (PRT)** | **Constructs Name** | **NFAT-GFP ASSAY** |
|---|---|---|---|
| 21894 | 22709 | CD8SP-Her2-huMab4D5-H91A-vL-[hTCRa- T48C]-F-F2A-SP-Her2-huMab4D5-D98W-vH- [hTCRb-S57C-opt1] | SKOV-3 (5.5+), MCF-7 (3.5+), PC-3 (+/-), MDA-MB-231 (-) |
| 21895 | 22710 | CD8SP-Her2-huMab4D5-H91A-vL-V5-[hTCRβ- KACIAH]-F-P2A-IgHSP-Hercep-Hu4D5_vH- MYC-[hTCRa-CSDVP]-F-F2A-PAC | SKOV-3 (7+), MCF-7 (7+), PC-3 (8+), MDA-MB-231 (7+) |
| 21896 | 22711 | CD8SP-Her2-huMab4D5-D98W-vL-[hTCRa- T48C]-F-F2A-SP-Her2-huMab4D5-D98A- F100A-Y102V-vH-[hTCRb-S57C-opt1] | SKOV-3 (7+), MCF-7 (5+), PC-3 (+/-), MDA-MB-231 (-), SKOV-3+ Herceptin (+/-) |
| 21897 | 22712 | Hercep-Hu4D5_vL-V5-[hTCRβ-KACIAH]-F- P2A-IgHSP-Her2-huMab4D5-D98A-F100A- Y102V-vH-MYC-[hTCRa-CSDVP]-F-F2A-PAC | SKOV-3 (4.5+), MCF-7 (4.5+), PC-3 (6+), MDA-MB-231 (5+) |
| 21898 | 22713 | CD8SP-Hercep-Hu4D5-vL-[hTCRa-T48C]-F- F2A-SP-Her2-huMab4D5-D98A-F100A-Y102V- vH-[hTCRb-S57C-opt1] | SKOV-3 (3.5+) |

### PSMA SARs

JNG cells were generating expressing the different SAR constructs targeting PSMA cloned in the pCCLc-MNDU3-WPRE vector (SEQ ID NO: 4). The cells were tested for binding to PSMA-ECD-GGS-NLuc (SEQ ID NO: 11995) and JNG cells expressing all the SAR constructs showed binding to PSMA-ECD-GGS-NLuc (SEQ ID NO: 11995). The cells also showed binding to Protein L by flow cytometry. The JNG cells expressing the SAR constructs were then tested for induction of GFP expression upon over-night co-culture with LNCaP cells. All the constructs showed GFP robust induction.

T cells were generated expressing the following SAR constructs by lentiviral mediated gene transduction. T cells showed expression of the SAR constructs on cell surface as measured by flow cytometry. The ability of SAR-T cells to induced cytotoxicy of the LNCaP-LucPPe target cells was demonstrated using Matador assay. For this purpose, LNCaP cells were stably transduced with a thermostable variant of bettle luciferase (LucPPe-146-1H2; SEQ ID NO: 17) using lentiviral mediated gene transfer. The T cells were tested *in vivo* using an LNCaP-LucPPE xenograft model in NSG mice. LNCaP-LucPPe cells 1 × 10⁶ in 100 µL of Matrigel and culture medium mixture (1:1, v/v) were implanted subcutaneously into the right thigh of 5-week-old male NSG mice (n =6). Seventeen days later, 3 × 10⁶ of PSMA-SAR T cells were injected intravenously via tail vein injection. Tumor growth was measured by *in vivo* bioluminescence imaging (BLI) following injection of D-luciferin. The T cells expressing SARs with SEQ ID NO: 9694, 9763, 9832 and 9901 showed effective control of tumor as measured by *in vivo* bioluminescence imaging and tumor volume measurement using a caliper. This resulted in improvement in survival. In particular, animals given 2^{nd} generation CAR-T with SEQ ID NO: 9758 showed minimum control of tumor while animals given T-cells given SAR with the backbone of double chain SIR (e.g., SEQ ID NO:9762, 9901 and 9970) showed statistically significant and effective tumor control. Effective tumor control was also seen using SAR-T constructs in which the [hTCRb-S57C] and [hTCRa-T48C] modules in the constructs with SEQ ID NO:9762, 9901 and 9970 were replaced by [hTCRb-KACIAH] and [hTCRa-CSDVP] modules, respectively. Further, effective control was seen in SAR-T cells expressing the construct CD8SP-hu-PSMA-J591-vL-[hTCRb-KACIAH]-F-P2A-SP-hu-PSMA-J591-vH-[hTCRa-CSDVP]-F-F2A-K13-opt (SEQ ID NO: 21485) which coexpressed the K13 module (SEQ ID NO: 1317).

Approximately 197 days after the injection of SAR-T cells, blood samples were collected from surviving mice. The presence of circulating human T cells was confirmed by staining with an antibody against human CD3 and flow cytometry. Expression of SAR on T cells was confirmed by staining with APC-conjugated Protein-L and flow cytometry. Surviving animals were rechallenged with subcutaneous injecton of fresh LNCaP cancer cells as before. The mice given SAR-T cells were able to reject LNCaP cells when rechallenged even 200 days later, suggesting long-term immunity. Furthermore, there was an increase in the percentage of SAR-T cells, as measured by flow cytometry, in the animals upon rechallenge with LNCaP cells. In contrast, mice which had not been administered SAR-T cells were used as controls and showed progressive tumor growth.

In addition to its expression on cancer cells, PSMA is also expressed at low level on normal cells. Therefore, to develop SARs that can recognize and repond to target cells with different levels of PSMA. a panel of SARs on different backbones and with different vL and vH fragments are generated and stably expressed in JNG cells. The JNG cells expressing the different SAR constructs are tested against LNCaP (PSMA-Hi), KMH2 (PSMA-low) and Huh-7 (PSMA-low) cell lines in co-culture assay. The results are shown in **Table 52.** The SAR constructs with SEQ ID NO: 21882, 21884, 21886, 21887, and 21892 showed modest GFP induction when cultured with LNCaP cells that express high levels of PSMA but showed negligible GFP induction with KMH2 and Huh-7 cells that show low levels of PSMA. SAR constructs with SEQ ID NO: 21888-21891 show weak activity even against LNCaP cells. The SAR construct with SEQ ID NO: 21885 with shorter TCRγ (huTCRg-d7) and TCRδ (huTCRd-d2) chains showed high NFAT-driven GFPactivity against LNCaP cells but was also active against KMH2 and Huh-7 cells that express low levels of PSMA. The SAR construct with SEQ ID NO: 21884 with huTCRg-d12 chain showed weaker activity as compared to SAR with SEQ ID NO: 21885. The SAR construct with SEQ ID NO: 21886 with deleted TCRa (hTCRa-d17) and TCRb (hTCRb-d17) constant chains showed relatively high GFP induction when co-cultured with LNCaP cells while having negligible activity against Huh-7 and KMH2 cell lines. Thus, depending on the level of PSMA expression in a patient's cancer, different SAR constructs with different backbones, constant chains and antigen binding domains can be selected to maximize efficacy and safety. Thus, the SAR with SEQ ID NO: 21885 may have activity against PSMA-low expressing tumors, it may also have on-target off-tumor toxicity against normal healthy tissues. In contrast, SAR with SEQ ID NO: 21886 and 21892 may miss some low PSMA expressing tumors but may offer better safety profile. Furthermore, the SAR constructs with shorter TCR constant chains not only retain their signaling activity but, in addition, may possess higher signaling activity. The SAR constructs with the shorter TCR constant chains also have the advantage of smaller size which in turn results in higher titer of the viral vector (e.g., lentiviral vector) encoding these constructs.

Athough the above experiments are conducted with SARs with puromycin resistance gene (PAC), the results are also applicable to constructs lacking this optional module. Finally, the vL and vH fragments used in the construction of the above constructs can be also used to generate additional SAR constructs with different backbones and with additional binding modules (e.g., AABD) to generate a diverse panel of unispecific, bispecific, multispecific and universal SAR constructs. The SEQ ID NO of the vL, vH and scFv fragments targeting PSMA are provided in **Table 3.** The SEQ ID NO of exemplary exemplary unispecific, bispecific and multispecific SAR constructs targeting PSMA alone or in combination with other antigens are provided in SEQ ID NO (DNA): 9686-10030 (Table 36) and in SEQ ID NO (DNA): 21662-21682. These SAR constructs can be tested further using assays described in this disclosure and can be used to generate a diverse immune response against PSMA expressing cancers.

The disclosure provides SARs on the backbones of 2^{nd} generation CAR, SIR, cTCR, AbTCR, TFPs, which when expressed in immune cells (e.g., T cells or NK cells etc.) can selectively target PSMA-high expressing cancer cells while sparing the PSMA-low expressing normal cells. The disclosure also provides a convenient method for screening of SARs with differential sensitivity towards tumors with different level of expression of PSMA (e.g., PSMA high and PSMA low tumors). In one embodiment, the disclosure provides a PSMA-targeted SAR, which when expressed in JNG cells causes less than 10% induction of GFP when the JNG cells expressing the SAR are co-cultured for 24 hours with Huh-7 cells. In one embodiment, the disclosure provides a PSMA-targeted SAR, which when expressed in JNG cells cause less than 10% induction of GFP when co-cultured for 24 hours with a PSMA negative cell line (e.g., a cell line in which both PSMA alleles are knocked out using CRISPR/Cas9). In one embodiment, the disclosure provides a PSMA-targeted SAR, which when expressed in JNG cells cause more than 20% induction of GFP when co-cultured for 24 hours with LNCaP cells.

**TABLE 52: GENERATING A DIVERSE PANEL OF SARS AGAINST PSMA**

| **SEQ ID NO (DNA)** | **SEQ ID NO (PRT)** | **SAR constructs Name** | **NFAT-GFP ASSAY** |
|---|---|---|---|
| 21882 | 22697 | CD8SP-hu-PSMA-2-J591-vL-[hTCRb-S57C]-F-P2A-SP- PSMA-3-J591-Y27F-E31D-vH-[hTCRα-T48C-opt]-F-F2A- PAC | LNCaP (3+), PC-3 (-), KMH2 (-), Huh-7 (-) |
| 21883 | 22698 | CD8SP-hu-PSMA-J591-vL-[hTCRb-S57C]-F-P2A-SP- PSMA-3-J591-Y27F-E31D-vH-[hTCRα-T48C-opt]-F-F2A- PAC | LNCaP (6+), PC-3 (+/-), KMH2 (-), Huh-7 (-) |
| 21884 | 22699 | CD8SP-hu-PSMA-J591-vL-huTCRg-d12-F-P2A-SP-hu- PSMA-J591-vH-huTCRd-d2-F-F2A-PAC | LNCaP (2+), Huh-7 (-), KMH2 (-) |
| 21885 | 22700 | CD8SP-hu-PSMA-J591-vL-huTCRg-d7-F-P2A-SP-hu- PSMA-J591-vH-huTCRd-d2-F-F2A-PAC | LNCaP (6+), Huh-7 (2+), KMH2 (2+) |
| 21886 | 22701 | CD8SP-hu-PSMA-J591-vL-hu-TCRb-d17-F-P2A-IgHSP-hu- PSMA-J591-vH-hu-TCRa-d17-F-F2A-PAC | LNCaP (4+), Huh-7 (-), KMH2 (-) |
| 21887 | 22702 | CD8SP-hu-PSMA-J591-vL-hu-TCRb-S57C-d14-F-P2A-SP- hu-PSMA-J591-vH-hu-TCRa-d17-F-F2A-PAC | LNCaP (3+), Huh-7 (-), KMH2 (-) |
| 21888 | 22703 | CD8SP-hu-PSMA-3-J591-Y91A-vL-[hTCRb-S57C]-F-P2A- SP-PSMA-3-J591-Y27F-E31D-vH-[hTCRα-T48C-opt]-F- F2A-PAC | LNCaP (-), Huh-7 (-), KMH2 (-) |
| 21889 | 22704 | CD8SP-RitX2-G4S-hu-PSMA-J591-vL-[hTCRb-S57C]-F- P2A-SP-PSMA-3-J591-Y27F-E31D-vH-[hTCRa-T48C-opt]- F-F2A-PAC | LNCaP (+/-) |
| 21890 | 22705 | CD8SP-RitX2-G4S-hu-PSMA-J591-vL-[hTCRb-S57C]-F- P2A-SP-PSMA-3-J591-Y27F-E31D-vH-[hTCRa-T48C-opt]- F-F2A-PAC | LNCaP (-) |
| 21891 | 22706 | CD8SP-RitxGAx2-RitX2-G4S-hu-PSMA-J591-vL-[hTCRb- S57C]-F-P2A-SP-hu-PSMA-J591-vH-[hTCRa-T48C-opt]-F- F2A-PAC | LNCaP (+/-) |
| 21892 | 22707 | CD8SP-hu-PSMA-J591-vL-[hTCRb-S57C]-F-P2A-SP-hu- PSMA-J591-vH-[hTCRa-T48C-opt]-F-F2A-PAC | LNCaP (3+), Huh-7 (-), KMH2 (-) |

### Comparison of SIR with different deletion mutants of TCR chains

JNG cells were infected with lentiviral vectors encoding the following deletion constructs targeting CD19 and based on hu-mROO5-1 vL and vH fragments that are linked to wild type and different deletion mutants of TCRa and TCRb chains and TCRg and TCRd chains. The cells were incubated with RAJI cells and induction of GFP expression was measured by flow cytometry (Table 53). However, constructs with deletion mutants of TCRα, β, γ and δ chains showed significant GFP induction upon coculture with RAJI cells. In particular, the construct with SEQ ID NO: 22641 with deletion of both TCRα and TCRβ showed significant GFP induction. Similarly, the constructs deletion of TCRγ and TCRδ chains with SEQ ID NO: 22651 and 22652 showed significant residual activity. These deletion mutants can be used to generate SAR with varying levels of strength and activity. These mutants can also result in higher titer of the viral vectors.

**TABLE 53 DELETION MUTAGENESIS OF TCR CONSTANT CHAINS**

| **SEQ ID NO (DNA)** | **SEQ ID NO (PRT)** | **Name of Construct** | **Jurkat-NFAT ASSA Y** |
|---|---|---|---|
| 21813 | 22628 | CD19-hu-mROO5-1-vL-hTCRb-d6-F-P2A-SP-CD19-hu- mROO5-1-vH-hTCRa-T48C-opt-F-F2A-PAC | Raji (5+) |
| 21814 | 22629 | CD19-hu-mROO5-1-vL-hTCRb-d2-F-P2A-SP-CD19-hu- mROO5-1-vH-hTCRa-T48C-opt-F-F2A-PAC | Raji (6+) |
| 21815 | 22630 | CD19-hu-mROO5-1-vL-hTCRb-d17-F-P2A-SP-CD19-hu- mROO5-1-vH-hTCRa-T48C-opt-F-F2A-PAC | Raji (4+) |
| 21816 | 22631 | CD19-hu-mROO5-1-vL-hTCRb-d4-F-P2A-SP-CD19-hu- mROO5-1-vH-hTCRa-T48C-opt-F-F2A-PAC | Raji (6+) |
| 21817 | 22632 | CD19-hu-mROO5-1-vL-hTCRb-d9-F-P2A-SP-CD19-hu- mROO5-1-vH-hTCRa-T48C-opt-F-F2A-PAC | Raji (5+) |
| 21818 | 22633 | CD19-hu-mROO5-1-vL-hTCRb-d14-F-P2A-SP-CD19-hu- mROO5-1-vH-hTCRa-T48C-opt-F-F2A-PAC | Raji (4+) |
| 21819 | 22634 | CD19-hu-mROO5-1-vL-hTCRb-S57C-F-P2A-SP-CD19-hu- mROO5-1-vH-hTCRa-T48C-opt-d17-F-F2A-PAC | Raji (4+) |
| 21820 | 22635 | CD19-hu-mROO5-1-vL-hTCRb-S57C-F-P2A-SP-CD19-hu- mROO5-1-vH-hTCRa-d6-F-F2A-PAC | Raji (2.5+) |
| 21821 | 22636 | CD19-hu-mROO5-1-vL-hTCRb-S57C-F-P2A-SP-CD19-hu- mROO5-1-vH-hTCRa-d13-T48C-optF-F2A-PAC | Raji (4+) |
| 21822 | 22637 | CD19-hu-mROO5-1-vL-hTCRb-d9-F-P2A-SP-CD19-hu- mROO5-1-vH-hTCRa-d10-F-F2A-PAC | Raji (6.5+) |
| 21823 | 22638 | CD19-hu-mROO5-1-vL-hTCRb-d2-F-P2A-SP-CD19-hu- mROO5-1-vH-hTCRa-d6-F-F2A-PAC | Raji (6.5+) |
| 21824 | 22639 | CD19-hu-mROO5-1-vL-hTCRb-d4-F-P2A-SP-CD19-hu- mROO5-1-vH-hTCRa-d6-F-F2A-PAC | Raji (6+) |
| 21825 | 22640 | CD19-hu-mROO5-1-vL-hTCRb-d6-F-P2A-SP-CD19-hu- mROO5-1-vH-hTCRa-d6-F-F2A-PAC | Raji (6+) |
| 21826 | 22641 | CD19-hu-mROO5-1-vL-hTCRb-d17-F-P2A-SP-CD19-hu- mROO5-1-vH-hTCRa-d17-F-F2A-PAC | Raji (6.5+) |
| 21827 | 22642 | CD19-hu-mROO5-1-vL-hTCRb-d17-F-P2A-SP-CD19-hu- mROO5-1-vH-hTCRa-d26-T48C-opt-F-F2A-PAC | Raji (+) |
| 21828 | 22643 | CD19-hu-mROO5-1-vL-hTCRb-d17-F-P2A-SP-CD19-hu- mROO5-1-vH-hTCRa-d32-T48C-opt-F-F2A-PAC | Raji (2+) |
| 21829 | 22644 | CD19-hu-mROO5-1-vL-hTCRb-d17-F-P2A-SP-CD19-hu- mROO5-1-vH-hTCRa-d37-T48C-opt-F-F2A-PAC | Raji (+/-) |
| 21830 | 22645 | CD19-hu-mROO5-1-vL-hTCRb-d17-F-P2A-SP-CD19-hu- mROO5-1-vH-hTCRa-d46-T48C-opt-F-F2A-PAC | Raji (2+) |
| 21831 | 22646 | CD19-hu-mROO5-1-vL-hTCRb-d17-F-P2A-SP-CD19-hu- mROO5-1-vH-hTCRa-d52-F-F2A-PAC | Raji (2+) |
| 21832 | 22647 | CD19-hu-mROO5-1-vL-hTCRb-d41-S57C-F-P2A-SP-CD19- hu-mROO5-1-vH-hTCRa-d32-T48C-opt-F-F2A-PAC | Raji (+) |
| 21833 | 22648 | CD19-hu-mROO5-1-vL-hTCRb-d50-S57C-F-P2A-SP-CD19- hu-mROO5-1-vH-hTCRa-d37-T48C-opt-F-F2A-PAC | Raji (-) |
| 21834 | 22649 | CD19-hu-mROO5-1-vL-hTCRb-d55-S57C-F-P2A-SP-CD19- hu-mROO5-1-vH-hTCRa-d46-T48C-opt-F-F2A-PAC | Raji (-) |
| 21835 | 22650 | CD19-hu-mROO5-1-vL-hTCRb-d63-F-P2A-SP-CD19-hu- mROO5-1-vH-hTCRa-d52-F-F2A-PAC | Raji (-) |
| 21836 | 22651 | CD19-hu-mROO5-1-vL-huTCRg-d5-F-P2A-SP-CD19-hu- mROO5-1-vH-huTCRd-d2-F-F2A-PAC | Raji (6+) |
| 21837 | 22652 | CD19-hu-mROO5-1-vL-huTCRg-d7-F-P2A-SP-CD19-hu- mROO5-1-vH-huTCRd-d2-F-F2A-PAC | Raji (5+) |
| 21838 | 22653 | CD8SP-CD19-hu-mROO5-1-vL-huTCRg-d12-F-P2A-SP- CD19-hu-mROO5-1-vH-huTCRd-d2-F-F2A-PAC | Raji (+) |
| 21839 | 22654 | CD19-hu-mROO5-1-vL-huTCRg-d7-F-P2A-SP-CD19-hu- mROO5-1-vH-huTCRd-d8-F-F2A-PAC | Raji (+/-) |
| 21840 | 22655 | CD8SP-CD19-hu-mROO5-1-vL-huTCRg-d12-F-P2A-SP- CD19-hu-mROO5-1-vH-huTCRd-d13-F-F2A-PAC | Raji (-) |

Use **of universal SAR-T cells expressing CD16 as the antigen binding domain.** Daudi cells expressing luciferase are injected intraperitoneally (i.p.; 0.3 × 10⁶ cells/mouse) in NSG mice (Jackson Laboratory). Some mice receive rituximab (150 mg) i.p. Human T cells (1 × 10⁷) expressing CD8SP-hu-mROO5-1-vL-[hTCRb-S57C]-F-P2A-IgSP-Apa-CD16-V158-v2-G4Sx3v2-hu-mROO5-1-vH-[hTCRa-T48C] (SEQ ID NO: 4725) SAR are injected four days after Daudi inoculation. Control mice receive tissue culture medium instead of rituximab or T cells. Rituximab injection is repeated weekly for 4 weeks, with no further T lymphocyte injection. All mice receive intraperitoneal injections of 1,000-2,000 IU of IL-2 twice a week for 4 weeks. Mice receiving rituximab plus SAR-T cells show reduced tumor growth.

### Use of SARs containing RZiP domain

T cells expressing an exemplary SAR containing RZIP CD8SP-hu-mROO5-1-vL-[hTCRb-S57C]-F-P2A-IgSP-Apa-RZIP-G4Sx3v2-hu-mROO5-1-vH-[hTCRa-T48C] (SEQ ID NO: 4345) are generated and administered to a patient with B-cell lymphoma at dose of 5 million cells/Kg. The patient is also administered purified CD8SP-CD20-2F2-vL-Gly-Ser-Linker-CD20-2F2-vH-G4S-EZIP (SEQ ID NO: 12033) and CD8SP-CD22-5-(vH-vL)-G4S-EZIP (SEQ ID NO:12146) targeting CD20 and CD22 by intravenous continuous infusion at rate of 1-100 µg/day. The dose is started at 1 µg/day and then adjusted based on tolerability and development of symptoms of CRS.

### Use of SARs containing E4 domain

T cells expressing an exemplary SAR containing E4 CD8SP-hu-mROO5-1-vL-[hTCRb-S57C]-F-P2A-IgSP-Apa-E4-G4Sx3v2-hu-mROO5-1-vH-[hTCRa-T48C] (SEQ ID NO: 4472) are generated and administered to a patient with B-cell lymphoma at dose of 5 million cells/Kg. The patient is also administered purified CD8SP-CD20-2F2-vL-Gly-Ser-Linker-CD20-2F2-vH-G4S-K4 (SEQ ID NO: 12291) and CD8SP-CD22-h10F4v2-vL-Gly-Ser-Linker-CD22-h10F4v2-vH-G4S-K4 (SEQ ID NO:12293) targeting CD20 and CD22 by intravenous continuous infusion at rate of 1-100 µg/day. The dose is started at 1 µg/day and then adjusted based on tolerability and development of symptoms of CRS.

**Use of *in vitro* and vivo selection to select SARs with desired properties.** A pool of SARs targeting CD19 listed in **Table 35** are targeted to the TRAC locus in T cells using TRAC gRNA essentially as described by Eyquem J et al (Nature, 543(7643):113-117, 2017). The targeting vector also carry DNA barcodes located downstream of the stop codon of the SAR inserts. An exemplary construct for targeting a SAR to TRAC locus is TRAC-5-SA-F2A-CD8SP-CD19-vHH-048-G4Sx3-R1-hu-mROO5-1-vL-[hTCRb-S57C]-F-P2A-IgSP-Apa-CD20-vHH-2HCD25-G4Sx3v2-hu-mROO5-1-vH-[hTCRa-T48C]-STOP-pA-Del-Exon1-TRAC-3 (SEQ ID NO: 23). Other SAR constructs based on Ab-TCR, cTCR and TFP backbone can be designed to target the TRAC locus as described in PCT/US2018/053247, which is incorporated in its entirety herein by reference. The TRAC-targeting construct encoding a SAR based on TFP backbones also coexpress the constant chains of TCRα or functional variant thereof as described in PCT/US2018/053247, which is incorporated in its entirety herein by reference. T cells can be derived from peripheral blood. In an alternate embodiment, T cells are derived from a single clone of iPSC or hematopoietic stem cells using techniques known in the art. T cells expressing the pool of SARs are co-cultured with RAJI cells *in vitro* for 1 to 21 days. Aliquotes of the SAR-T cell pools are collected before the culture with the target cells and on different days after co-culture. Samples are subjected to next generation sequencing to determine the relative frequency of different SARs following exposure to the target cells. Bioinformatics analyses is used to determine the SARs that are associated with better proliferative response following co-culture with the target cells. Essentially a similar approach is used to determine the SARs that confer higher proliferative potential on T cells *in vivo* and/or persist long term *in vivo* and/or are present at higher frequency when normalized for their frequency in the starting T cell population in surviving animals as compared to animals that succumb to tumor challenge. In alternate embodimentof the disclosure essentially a similar approach is used on human clinical samples to identify SARs that are associated with different properties and/or outcomes including but not limited to better long term survival, lower incidence of cytokine release syndrome, lower neurotoxicity and/or higher long term persistence. Such SARs can be subsequently used, either singly or in various combinations, to develop different SAR subpools, containing SARs targeting the same or different antigen binding domains, with diverse properties for the treatment of different disease conditions and different patients. In other enablements, the SAR-T cells are exposed to their target cell line and then sorted into different sets based on the degree of intracellular IFNγ as determined by flow cytometry. The frequency of different SARs in the low vs high IFNγ population is determined by next generation sequencing and normalized to their frequency in the control SAR-T cell population, *i.e.,* SAR-T cells that have not been exposed to the target cell line or are exposed to a cell line that does not express the targe of SARs. From this analysis, SARs that are associated with different levels of IFNγ production can be determined. A similar approach is used to screen for and select SARs with any or a combination of desired properties or attributes including but not limited to, lower TNFα production, lower expression of exhaustion markers, lower expression of markers of terminal differentiation and/or higher expression of markers of cytotoxicity.

**Use of autologous SAR-T cells for adoptive cell therapy. SAR-T** cells of the disclosure can be used for adoptive cell therapy. As an example, patients with relapsed Acute Lymphocytic Leukemia (ALL), Chronic Lymphocytic Leukemia (CLL), or high-risk intermediate grade B-cell lymphomas may receive immunotherapy with adoptively transferred autologous SAR-T cells targeting CD19. A leukapheresis product collected from each patient undergoes selection of CD3-positive T lymphocytes using the CliniMACS Prodigy^{®} System from Miltenyi Biotec and following the manufacturer's recommendations. The T lymphocytes are optionally enriched for Pgp-positive T cells using Flow sorting following staining with Pgp antibodies, MACS following staining with Pgp antibodies or Photodynamic selection following exposure to TH9402 plus light. Cells are transduced with clinical grade CD8SP-hu-mROO5-1-vL-[hTCRb-S57C]-F-P2A-IgSP-Apa-CD20-vHH-2HCD25-G4Sx3v2-hu-mROO5-1-vH-[hTCRa-T48C] (SEQ ID NO: 4091) and then selection and expansion of the SAR-T cells occur in a GRex flask under hypoxic conditions. After the resulting cell products have undergone quality control testing (including sterility and tumor specific cytotoxicity tests), they are cryopreserved. Meanwhile, following leukapheresis, study participants commence with lymphodepletive chemotherapy (30 mg/m²/day fludarabine plus 500 mg/m²/day cyclophosphamide × 3 days). One day after completion of their lymphodepleting regimen, the previously stored SAR-T cell product is transported, thawed and infused at the patient's bedside. The study participant receives SAR-transduced lymphocytes infused intravenously followed by high-dose (720,000 IU/kg) IL-2 (Aldesleukin; Prometheus, San Diego, CA) every 8 hours to tolerance. The dose of SAR-T product varies from 1 × 10⁴ SAR+ve CD3 cells/kg to 5 × 10⁹ SAR+ve CD3 cells/kg as per the study protocol. The SAR-T product may be administered in a single infusion or split infusions. Research participants can be pre-medicated at least 30 minutes prior to T cell infusion with 15 mg/kg of acetaminophen P.O. (max. 650 mg.) and diphenhydramine 0.5-1 mg/kg I.V. (max dose 50 mg). The study participant may optionally receive dailiy injections of human IL-2. Clinical and laboratory correlative follow-up studies can then be performed at the physician's discretion, and may include quantitative RT-PCR studies for the presence of CD19-expressing ALL/lymphoma cells and/or the adoptively transferred T cells; FDG-PET and/or CT scans; bone marrow examination for disease specific pathologic evaluation; lymph node biopsy; and/or long-term follow up per the guidelines set forth by the FDA's Biologic Response Modifiers Advisory Committee that apply to gene transfer studies. Essentially a similar approach can be used to treat other diseases using autologous immune cells *(e.g.,* T cells) that have been engineered to express the SAR of the disclosure where the SAR targets an antigen or antigens expressed on the disease causing or disease-associated cells.

**Use of autologous SAR-T cells targeting multiple antigens for adoptive cell therapy.** Patients many different diseases, including infectious diseases (e.g., HIV1, EBV, CMV, HTLV1, etc), degenerative diseases (e.g., Alzheimer's disease), autoimmune disease *(e.g.,* pemphigous vulgaris), allergic diseases *(e.g.,* chronic idiopathic urticarial) and multiple cancers are enrolled in an IRB approved phase I clinical trial of immunotherapy with adoptively transferred autologous SAR-T cells targeting different disease causing or disease associated antigens. The SAR for different diseases are selected based on the known expression of their target antigen in the disease causing or disease associated cells. Where possible, the expression of the SAR target on the disease causing or disease associated cells is confirmed by binding with ABD-GGS-NLuc fusion protein in which the antigen binding domain of SAR fused to non-secretory form of NLuc protein via a flexible linker. Alternatively, immunohistochemistry or flow cytometry using commercially available antibodies is used to confirm the expression of the SAR target on disease causing or disease associated cells. T cells are collected from the subject using leukopheresis, transduced with the appropriate SAR encoding lentivirus vector and expanded ex vivo using CD3/CD28 beads in a GRex flask with Birinapat. After the resulting cell products have undergone quality control testing (including sterility and tumor specific cytotoxicity tests), they are cryopreserved. Meanwhile, study participants commence with lymphodepletive chemotherapy (30 mg/m²/day fludarabine plus 500 mg/m²/day cyclophosphamide x 3 days). One day after completion of their lymphodepleting regimen, the study participant receives transduced lymphocytes infused intravenously followed by high -dose (720,000 IU/kg) IL-2 (Aldesleukin; Prometheus, San Diego, CA) every 8 hours to tolerance. The previously stored SAR-T cell product is transported, thawed and infused at the patient's bedside. The dose of SAR-T product varies from 1 × 10⁴ SAR+ve CD3 cells/kg to 5 × 10⁹ SAR+ve CD3 cells/kg as per the study protocol. The SAR-T product may be administered in a single infusion or split infusions. Research participants can be pre-medicated at least 30 minutes prior to T cell infusion with 15 mg/kg of acetaminophen P.O. (max. 650 mg.) and diphenhydramine 0.5-1 mg/kg I.V. (max dose 50 mg). The study participant may optionally receive dailiy injections of human IL-2. Clinical and laboratory correlative follow-up studies can then be performed at the physician's discretion.

**Use of allogeneic SAR-T cells for adoptive cells therapy.** Patients with relapsed Acute Lymphocytic Leukemia or high-risk intermediate grade B-cell lymphomas who have undergone an allogeneic bone marrow transplant may receive immunotherapy with adoptively transferred allogeneic SAR-T cells. A leukapheresis product collected from the donor (same donor as used for the allogeneic transplant) undergoes selection of CD3 positive T lymphocytes using the CliniMACS Prodigy^{®} System from Miltenyi Biotec and following the manufacturer's recommendations. The expression of TRAC and β2M is eliminated by CRISP9 mediated knock-out using techniques known in the art and T cells lacking cell surface expression of TCR/CD3 complex and HLA are selected. Cells are activate using a CD3 and CD28 magnetic bead-based artificial antigen presenting cells and transduced with clinical grade CD20-SIR virus (*e.g.*, CD8SP-MC7G5-Vb-[hTCRb-S57C]-F-P2A-IgSP-Apa-CD20-vHH-2HCD25-G4Sx3v2-MC7G5-Va-[hTCRa-T48C](SEQ ID NO: 3503]. Cells are expanded for 9-12 days in a closed system. After the resulting cell products have undergone quality control testing (including sterility and tumor specific cytotoxicity tests), they are cryopreserved. Meanwhile, study participants commence with lymphodepletive chemotherapy (30 mg/m²/day fludarabine plus 500 mg/m²/day cyclophosphamide x 3 days). One day after completion of their lymphodepleting regimen, the study participant receives transduced lymphocytes infused intravenously followed by high -dose (720,000 IU/kg) IL-2 (Aldesleukin; Prometheus, San Diego, CA) every 8 hours to tolerance. The SAR-T cell product is transported, thawed and infused at the patient's bedside. The dose of SAR-T product may vary from 1 × 10⁴ SAR+ve CD3 cells/kg to 5 × 10⁹ SAR+ve CD3 cells/kg as per the study protocol. The SAR product may be administered in a single infusion or split infusions. Research participants can be pre-medicated at least 30 minutes prior to SAR-T cell infusion with 15 mg/kg of acetaminophen P.O. (max. 650 mg.) and diphenhydramine 0.5-1 mg/kg I.V. (max dose 50 mg). Clinical and laboratory correlative follow-up studies can then be performed at the physician's discretion, and may include quantitative RT-PCR studies for the presence of CD20-expressing ALL/lymphoma cells and/or the adoptively transferred T cells; FDG-PET and/or CT scans; bone marrow examination for disease specific pathologic evaluation; lymph node biopsy; and/or long-term follow up per the guidelines set forth by the FDA's Biologic Response Modifiers Advisory Committee that apply to gene transfer studies. Use of immunosuppressive drugs is also at the discretion of the physician. Essentially a similar approach can be used to treat other diseases using allogeneic immune cells *(e.g.,* T cells) expressing the SAR of the disclosure where the SAR targets an antigen or antigens expressed on the disease causing or disease-associated cells. Essentially a similar protocol is used to test other SAR constructs listed in Tables 25-36.

**SAR-T Cell Hepatic Arterial Infusion.** In addition to intravenous infusion, SAR-T cells can be infused intra-arterially to provide high concentration of SAR-T cells in a local area or organ involved with a disease.

**Intraperitoneal administration of SAR-T cells. SAR-T** cells can also be administered intraperitoneally, essentially as described in Koneru M et al (Journal of Translational Medicine; 2015; 13:102).

**Use of SAR-T cells for intratumoral injection. SAR-T** cells can also be administered intra-tumorally, essentially as described in Brown CE, et al, Clin Cancer Res. 2015 September 15; 21(18): 4062-4072.

**Use of SAR-T cells for ex-vivo purging of bone marrow or peripheral blood stem cell preparation prior to transplant. SAR** T cells can be used to purge the bone marrow or peripheral blood stem cell preparation of cancer cells prior to stem cell transplant.

### Use of SAR targeting PSMA for treatment of a patient with prostate cancer.

A patient with prostate cancer is administered Mozibil to mobilize lymphocytes and then undergoes apheresis. The T cells are purified and used to manufacture SAR-T cell product in GRex flask after transduction with a lentiviral vector expressing the SAR construct CD8SP-hu-PSMA-J591-vL-[hTCRb-S57C]-F-P2A-SP-hu-PSMA-J591-vH-[hTCRa-T48C] (SEQ ID NO: 9763). The subject receives lymphodepleting chemotherapy followed by infusion of the SAR-T cell product at dose of 5 million SAR-T cells/Kg.

### Use of SAR targeting BCMA for treatment of a patient with myeloma.

A patient with myeloma undergoes apheresis. The T cells are purified and used to manufacture SAR-T cell product in GRex flask under hypoxic conditions after transduction with a lentiviral vector expressing the SAR construct CD8SP-BCMA-hu-USC82-S29G-vL-[hTCRb-S57C]-F-P2A-SP-BCMA-hu-USC73-G32S-vH-[hTCRa-T48C] (SEQ ID NO: 8314). The subject receives lymphodepleting chemotherapy followed by infusion of the SAR-T cell product at dose of 5 million SAR-T cells/Kg. Essentially similar procedure is used to administer other SAR-T cell products targeting BCMA (e.g., SEQ ID NO: 7415-7418, 7427.

### Use of allogeneic SAR-T cell product

An allogeneic SAR-T product is generated by targeting the SAR construct TRAC-5-SA-F2A-CD8SP-CD19-vHH-048-G4Sx3-R1-hu-mROO5-1-vL-[hTCRb-S57C]-F-P2A-IgSP-Apa-CD20-vHH-2HCD25-G4Sx3v2-hu-mROO5-1-vH-[hTCRa-T48C]-STOP-pA-Del-Exon1-TRAC-3 (SEQ ID NO: 23) to the TRAC locus using techniques known in the arta. The expression of β2M is also eliminated using Cas9 mediated gene disruption using techniques known in the art. The allogeneic product is administered to a patient with B-cell ALL after lymphodepleting chemotherapy.

### Use of SAR targeting MSLN for treatment of a patient with ovarian cancer.

A patient with ovarian cancer undergoes apheresis. The T cells are purified and used to manufacture SAR-T cell product in GRex flask under hypoxic conditions after transduction with a lentiviral vector expressing the SAR construct CD8SP-MSLN-hu22A10-vL-Y96W-[hTCRb-S57C]-F-P2A-SP-MSLN-hu22A10-vH-[hTCRa-T48C] (SEQ ID NO:10660). The subject receives lymphodepleting chemotherapy followed by infusion of the SAR-T cell product at dose of 5 million SAR-T cells/Kg. Essentially similar procedure is used to administer other SAR-T cell products targeting MSLN (SEQ ID NO: 10591, 10453, 10384-85 etc.).

**T cells expressing ROR1 SARs induce cytotoxicity in ROR1-expressing cells.** Human peripheral blood T cells isolated using CD3 magnetic beads are infected with lentiviruses expressing the SAR constructs (SEQ ID NO: 19065-19271) targeting ROR1. SAR-T cells are expanded in vitro for 10-14 days. A549 cells stably expressing GLuc are cocultured with T cells expressing the different CARs at an E:T ratio of 10:1 for 4 hours. SAR-T cells mediated induction of lysis of target cells is assayed using the Matador assay by measurement of GLuc activity. The in vivo activity of the SARs is demonstrated using a xenograft model in NSG mice.

**T cells expressing CD229 SARs induce cytotoxicity in CD229-expressing cells.** Human peripheral blood T cells isolated using CD3 magnetic beads are infected with lentiviruses expressing the SAR constructs (SEQ ID NO: 19883-20091) targeting CD229. SAR-T cells are expanded in vitro for 10-14 days. RAJI target cells stably expressing GLuc are cocultured with T cells expressing the different CARs at an E:T ratio of 10:1 for 4 hours. SAR-T cells mediated induction of lysis of target cells is assayed using the Matador assay by measurement of GLuc activity. The in vivo activity of the SARs is demonstrated using a xenograft model in NSG mice.

**T cells expressing Toso SARs induce cytotoxicity in Toso-expressing cells.** Human peripheral blood T cells isolated using CD3 magnetic beads are infected with lentiviruses expressing the SAR constructs (SEQ ID NO: 19686-19720) targeting Toso. SAR-T cells are expanded in vitro for 10-14 days. Target cells stably expressing GLuc are cocultured with T cells expressing the different CARs at an E:T ratio of 10:1 for 4 hours. SAR-T cells mediated induction of lysis of target cells is assayed using the Matador assay by measurement of GLuc activity. The in vivo activity of the SARs is demonstrated using a xenograft model in NSG mice.

**T cells expressing SARS-CoV2 SARs induce cytotoxicity in Toso-expressing cells.** Human peripheral blood T cells isolated using CD3 magnetic beads are infected with lentiviruses expressing the SAR constructs targeting SARS-Cov2 RBD. SAR-T cells are expanded in vitro for 10-14 days. K562 cells stably expressing GLuc and membrane bound form of RBD are cocultured with T cells expressing the different CARs at an E:T ratio of 10:1 for 4 hours. SAR-T cells mediated induction of lysis of target cells is assayed using the Matador assay by measurement of GLuc activity.

Essentially a similar procedure is used to demonstrate the in vitro and in vivo activity of other SARs of the disclosure targeting other antigens.

### Embodiments:

1. At least one recombinant polynucleotide encoding at least one synthetic antigen receptor (SAR) polypeptide, the at least one SAR polypeptide comprising:
   a) a first module comprising one or more autonomous antigen binding domains (AABDs) or fragments thereof selected from the group of:
      i) a single vH domain (SVH) or a fragment thereof;
      ii) a single vL domain (SVL) or a fragment thereof;
      iii) a vHH domain or a fragment thereof;
      iv) a single domain antibody or a fragment thereof;
      v) a single variable domain of a TCR (svd-TCR) or a fragment thereof;
      vi) a non-immunoglobulin antigen binding scaffold selected from a DARPIN, an affibody, an affilis, an adnectin, an affitin, an obody, an repebody, an fynomer, an alphabody, an avimer, an atrimer, an centyrin, an pronecti, an anticalins, an kunitz domain, an Armadillo repeat protein and a D domain, or a fragment of any of the foregoing;
      vii) a ligand-binding domain of a receptor or a fragment thereof;
      viii) a receptor-binding domain of a ligand;
      ix) an autoantigen or a fragment thereof;
      x) an adaptor binding domain or a fragment thereof; and
      xi) an Fc binding domain or a fragment thereof;
   b) a second module comprising vL (V_{L}), vH (V_{H}), Va/Vα, Vb1 (Vβ1), Vb2 (Vβ2), Vg (Vγ), Vd (Vδ), scFv, scTCR, Ig linker domain or a combination thereof;
   c) an optional third module comprising an extracellular domain, a connecting peptide or a hinge domain;
   d) a fourth module that comprises a transmembrane domain; and
   e) an optional fifth module comprising one or more intracellular signaling domains;
   where the first, second, optional third, fourth and the optional fifth modules are operationally linked via one or more optional linkers.
2. A recombinant polynucleotide encoding a SAR polypeptide of embodiment 1, where the one or more AABDs are attached via an optional linker to the N-terminus or near the N-terminus of
   1) a first polypeptide chain comprising a vH, Va, Vg or an Ig linker domain fragment operably linked via an optional linker to a first T cell receptor constant chain fragment comprising a first connecting peptide operably linked to a first transmembrane domain of a first TCR subunit; and
   2) a second polypeptide chain comprising a vL, Vb, Vd or an Ig linker domain operably linked via an optional linker to a second T cell receptor constant chain fragment comprising a second connecting peptide operably linked to a second transmembrane domain of a second TCR subunit;
   wherein the first TCR constant chain fragment and the second TCR constant chain fragment form a T cell receptor module (TCRM) that is capable of recruiting at least one TCR-associated signaling module when expressed in a T cell.
3. The at least one recombinant polynucleotide encoding at least one SAR polypeptide of embodiment 2, wherein the encoded vH, Va, or Vg of the first antigen-binding domain and the vL, Vb or Vd of the second antigen-binding domain form an antigen-binding module that specifically binds to a target antigen.
4. The at least one recombinant polynucleotide encoding at least one SAR polypeptide of embodiment 1 or 2, wherein the AABD, vL, vH, Va, Vb, Vg, Vd and/or Ig linker domain is a fully human, humanized, chimeric or a non-human domain.
5. The at least one recombinant polynucleotide encoding at least one SAR polypeptide of embodiment 1 or 2, wherein the encoded Ig linker domain comprises a polypeptide with SEQ ID NO: 11832-11865 or a fragment or variant thereof with at least 70% sequence homology to a polypeptide having the sequence of SEQ ID NO: 11832-11865.
6. The at least one recombinant polynucleotide encoding at least one SAR polypeptide of embodiment 1 or 2, wherein the encoded optional linker domain comprises a polypeptide with SEQ ID NO: 11832-11865, 11714-11730 or a fragment or a variant thereof with at least 70% sequence homology to a polypeptide having the sequence of SEQ ID NO: 11832-11865, 11714-11730 or a domain that is between 25 to 500 amino acids in length.
7. The at least one recombinant polynucleotide encoding at least one SAR polypeptide of embodiment 1, comprising a partial or entire extacellular, transmbrane and intracellular domains of a polypeptide capable of being recruited to a T cell receptor module (TCRM).
8. The at least one recombinant polynucleotide encoding at least one SAR polypeptide of embodiment 1, comprising the extacellular, transmbrane and intracellular domains of a polypeptide selected from the group of CD3δ, CD3ε, CD3γ and CD3ζ or a fragment thereof with at leasst 70% sequence homology to a polypeptide having the sequence of any one of SEQ ID NO: 11903 to 11906.
9. The at least one recombinant polynucleotide encoding at least one SAR polypeptide of embodiment 2, wherein the encoded T cell receptor constant chain fragment comprises a polypeptide or a fragment thereof having at least 70% sequence homology to a polypeptide having the sequence of any one of SEQ ID NO: 11732-11830.
10. The at least one recombinant polynucleotide encoding at least one SAR of embodiment 2, wherein the encoded T cell receptor constant chain comprises a connecting peptide or a fragment thereof having at least 70% sequence homology to a connecting peptide having the sequence of any one of SEQ ID NO: 11867-11875.
11. The at least one recombinant polynucleotide encoding at least one SAR polypeptide of embodiments 1 or 2, comprising a transmembrane domain or a fragment thereof having at least 70% sequence homology to a transmembrane domain having a sequence of any one of SEQ ID NO: 11877-11881 or 23332-23334.
12. The at least one recombinant polynucleotide encoding at least one SAR polypeptide of embodiment 1, wherein the intracellular domain contains a cytosolic domain or a fragment thereof with at least 70% sequence homology to a polypeptide having the sequence of any one of SEQ ID NO: 11883-11886, 11785, or 23335-23337.
13. The at least one recombinant polynucleotide encoding at least one SAR polypeptide of embodiment 1 which has the backbone of a first generation CAR, a second generation CAR, a third generation CAR, a single chain SIR, a one and a half chain SIR, a double chain SIR, a zSIR, a single chain cTCR, a one and a half chain cTCR, a double chain cTCR, an Ab-TCR, an AABD-TCR, a TFPε, a TFPy, a TFPδ, a TFPαβ, a TFPγδ, a Tri-TAC, a single chain TCR, a double chain TCR or an HLA-independent TCR.
14. The at least one recombinant polynucleotide encoding at least one SAR polypetpide of embodiment 1 or 2, wherein the SAR can bind to:
   i) at least one antigen; and/or
   ii) at least one epitope of one or more than antigen(s).
15. The at least one recombinant polynucleotide encoding at least one SAR polypeptide of embodiment 1, where the encoded two or more AABDs bind to:
   i) at least one antigen; and/or
   ii) at least one epitope of one or more than antigen(s).
16. The at least one recombinant polynucleotide encoding at least one SAR polypeptide of embodiment 1 or 2, wherein the encoded one or more antigen binding domains bind to at least one antigen selected from the group of CD5; CD19; CD123; CD22; CD30; CD171; CS1 (also referred to as CD2 subset 1, CRACC, SLAMF7, CD319, and 19A24); C-type lectin-like molecule-1 (CLL-1 or CLECL1); CD33; epidermal growth factor receptor variant III (EGFRviii); ganglioside G2 (GD2); ganglioside GD3 (aNeu5Ac(2-8)aNeu5Ac(2-3)bDGalp(l-4 )bDGlcp(l-l)Cer); TNF receptor family member B cell maturation (BCMA); Tn antigen ((Tn Ag) or (GalNAcα-Ser/Thr)); prostate-specific membrane antigen (PSMA); Receptor tyrosine kinase-like orphan receptor 1 (ROR1); Fms Like Tyrosine Kinase 3 (FLT3); Tumor-associated glycoprotein 72 (TAG72); CD38; CD44v6; a glycosylated CD43 epitope expressed on acute leukemia or lymphoma but not on hematopoietic progenitors, a glycosylated CD43 epitope expressed on non-hematopoietic cancers, Carcinoembryonic antigen (CEA); Epithelial cell adhesion molecule (EPCAM); B7H3 (CD276); KIT (CD117); Interleukin-13 receptor subunit alpha-2 (IL-13Ra2 or CD213A2); Mesothelin; Interleukin 11 receptor alpha (IL-llRa); prostate stem cell antigen (PSCA); Protease Serine 21 (Testisin or PRSS21); vascular endothelial growth factor receptor 2 (VEGFR2); Lewis(Y) antigen; CD24; Platelet-derived growth factor receptor beta (PDGFR-beta); Stage-specific embryonic antigen-4 (SSEA-4); CD20; Folate receptor alpha (FRa or FR1); Folate receptor beta (FRb); Receptor tyrosine-protein kinase ERBB2 (Her2/neu); Mucin 1, cell surface associated (MUC1); epidermal growth factor receptor (EGFR); neural cell adhesion molecule (NCAM); Prostase; prostatic acid phosphatase (PAP); elongation factor 2 mutated (ELF2M); Ephrin B2; fibroblast activation protein alpha (FAP); insulin-like growth factor 1 receptor (IGF-I receptor); carbonic anhydrase IX (CAlX); Proteasome (Prosome, Macropain) Subunit, Beta Type, 9 (LMP2); glycoprotein 100 (gpl00); oncogene fusion protein consisting of breakpoint cluster region (BCR); Abelson murine leukemia viral oncogene homolog 1 (Abl) (bcr-abl); tyrosinase; ephrin type-A receptor 2 (EphA2); sialyl Lewis adhesion molecule (sLe); ganglioside GM3 (aNeu5Ac(2-3)bDClalp(l- 4)bDGlcp(l-1)Cer); transglutaminase 5 (TGS5); high molecular weight-melanoma associated antigen (HMWMAA); o-acetyl-GD2 ganglioside (OAcGD2); tumor endothelial marker 1 (TEM1/CD248); tumor endothelial marker 7-related (TEM7R); claudin 6 (CLDN6); thyroid stimulating hormone receptor (TSHR); G protein coupled receptor class C group 5, member D (GPRC5D); chromosome X open reading frame 61 (CXORF61); CD97; CD179a; anaplastic lymphoma kinase (ALK); Polysialic acid; placenta-specific 1 (PLAC1); hexasaccharide portion of globoH glycoceramide (GloboH); mammary gland differentiation antigen (NY-BR-1); uroplakin 2 (UPK2); Hepatitis A virus cellular receptor 1 (HAVCR1); adrenoceptor beta 3 (ADRB3); pannexin 3 (PANX3); G protein-coupled receptor 20 (GPR20); lymphocyte antigen 6 complex, locus K 9 (LY6K); Olfactory receptor 51E2 (OR51E2); TCR Gamma Alternate Reading Frame Protein (TARP); Wilms tumor protein (WT1); Cancer/testis antigen 1 (NY-ESO-1); Cancer/testis antigen 2 (LAGE-1a); Melanoma-associated antigen 1 (MAGE-A1); ETS translocation-variant gene 6, located on chromosome 12p (ETV6-AML); sperm protein 17 (SPA17); X Antigen Family, Member 1A (XAGEl); angiopoietin-binding cell surface receptor 2 (Tie 2); melanoma cancer testis antigen-1 (MAD-CT-1); melanoma cancer testis antigen-2 (MAD-CT-2); Fos-related antigen 1; tumor protein p53 (p53); p53 mutant; prostein; survivin; telomerase; prostate carcinoma tumor antigen-1 (PCT A-1 or Galectin 8), melanoma antigen recognized by T cells 1 (MelanA or MARTI); Rat sarcoma (Ras) mutant; human Telomerase reverse transcriptase (hTERT); sarcoma translocation breakpoints; melanoma inhibitor of apoptosis (ML-IAP); ERG (transmembrane protease, serine 2 (TMPRSS2) ETS fusion gene); N-Acetyl glucosaminyl-transferase V (NA17); paired box protein Pax-3 (PAX3); Androgen receptor; Cyclin Bl; v-myc avian myelocytomatosis viral oncogene neuroblastoma derived homolog (MYCN); Ras Homolog Family Member C (RhoC); Tyrosinase-related protein 2 (TRP-2); Cytochrome P450 lB 1 (CYPlB 1); CCCTC-Binding Factor (Zinc Finger Protein)-Like (BORIS or Brother of the Regulator oflmprinted Sites), Squamous Cell Carcinoma Antigen Recognized By T Cells 3 (SART3); Paired box protein Pax-5 (PAX5); proacrosin binding protein sp32 (OY-TESl); lymphocyte-specific protein tyrosine kinase (LCK); A kinase anchor protein 4 (AKAP-4); synovial sarcoma, X breakpoint 2 (SSX2); Receptor for Advanced Glycation Endproducts (RAGE-1); renal ubiquitous 1 (RUl); renal ubiquitous 2 (RU2); legumain; human papilloma virus E6 (HPV E6); human papilloma virus E7 (HPV E7); intestinal carboxyl esterase; heat shock protein 70-2 mutated (mut hsp70-2); CD79a; CD79b; CD72; Leukocyte-associated immunoglobulin-like receptor 1 (LAIRl); Fc fragment of IgA receptor (FCAR or CD89); Leukocyte immunoglobulin-like receptor subfamily A member 2 (LILRA2); CD300 molecule-like family member f (CD300LF); C-type lectin domain family 12 member A (CLEC12A); bone marrow stromal cell antigen 2 (BST2); EGF-like module-containing mucin-like hormone receptor-like 2 (EMR2); lymphocyte antigen 75 (LY75); Glypican-3 (GPC3); Fc receptor-like 5 (FCRL5); immunoglobulin lambda-like polypeptide 1 (IGLLl); MPL; Biotin; c-MYC epitope Tag; CD34; LAMP1 TROP2; GFRalpha4; CDH17; CDH6; NYBR1; CDH19; CD200R,; Slea (CA19.9; Sialyl Lewis Antigen); Fucosyl-GM1; PTK7; gpNMB; CDH1-CD324; DLL3; CD276/B7H3; IL11Ra; IL13Ra2; CD179b-IGLl1; TCRgamma-delta; NKG2D; CD32 (FCGR2A),; Tn ag; Tim1-/HVCR1; CSF2RA (GM-CSFR-alpha); TGFbetaR2; Lews Ag; TCR-beta1 chain; TCR-beta2 chain; TCR-gamma chain; TCR-delta chain; FITC; Leutenizing hormone receptor (LHR); Follicle stimulating hormone receptor (FSHR); Gonadotropin Hormone receptor (CGHR or GR); CCR4; GD3; SLAMF6; SLAMF4; HIV1 envelope glycoprotein; HTLV1-Tax; CMV pp65; EBV-EBNA3c; KSHV K8.1; KSHV-gH; influenza A hemagglutinin (HA); GAD; PDL1; Guanylyl cyclase C (GCC); auto antibody to desmoglein 3 (Dsg3); auto antibody to desmoglein 1 (Dsg1); HLA; HLA-A; HLA-A2; HLA-B; HLA-C; HLA-DP; HLA-DM; HLA-DOA; HLA-DOB; HLA-DQ; HLA-DR; HLA-G; IgE, CD99; Ras G12V; Tissue Factor 1 (TF1); AFP; GPRC5D; Claudin18.2 (CLD18A2 or CLDN18A.2); P-glycoprotein; STEAP1; Liv1; Nectin-4; Cripto; gpA33; BST1/CD157; low conductance chloride channel (LCCC); TAJ/TROY; MPL (TPO-R); KIR3DL2; CD32b; CD229; Toso; GPC3; BAFF-R; MR1; CMV-pp65/MHC complex; and NYESO-1/MHC complex.
17. The at least one recombinant polynucleotide encoding at least one SAR of embodiment 1 or 2, wherein the encoded SAR polypeptide comprises one or more antigen binding domains selected from the group consisting of:
   (i) a heavy chain variable region (vH) comprising a sequence as set forth in any of SEQ ID Nos: 10978-11214 or sequences with at least 70% identity thereto or sequences with at least 70% identity in the three complementarity determining regions (CDRs) to the sequences set forth in any one or more of SEQ ID NOS: 10978-11214 and 23148-23161 or sequences with less than 3 substitutions in the three CDRs of the sequences set forth in any one or more of SEQ ID NOS: 10978-11214 and 23148-23161 or sequences that bind to the same target antigens or the same epitopes on the target antigens as the sequences set forth in any one or more of SEQ ID NOS: 10978-11214 and 23148-23161 and which encodes a polypeptide that binds to its antigen;
   (ii) a light chain variable region (vL) comprising a sequence as set forth in any one of SEQ ID NO: 10736-10972 or sequences with at least 70% identity to sequences set forth in any one or more of SEQ ID NOS: 10736-10972 and 23136-23147 or sequences with at least 70% identity in the three complementarity determining regions (CDRs) to the sequences set forth in any one or more of SEQ ID NOS: 10736-10972 and 23136-23147 or sequences with less than 3 substitutions in the three CDRs of the sequences set forth in any one or more of SEQ ID NOS: 10736-10972 and 23136-23147 or sequences that bind to the same target antigens or the same epitopes on the target antigens as the sequences set forth in any one or more of SEQ ID NOS: 10736-10972 and 23136-23147 and which encodes a polypeptide that binds to its antigen;
   (iii) a single chain variable fragment (scFv) comprising a sequence as set forth in any one SEQ ID NO: 11220-11456 or sequences with at least 70% identity thereto and which encodes a polypeptide that binds to its antigen;
   (iv) a camelid VHH domain comprising a sequence as set forth in any one of SEQ ID NO: SEQ ID NOS: 11524-11525, 11530-11531, 11549-11575, 11576-11592 and 23163-23173 or sequences with at least 70% identity to sequences set forth in any one or more of SEQ ID NOS: 11520-11592 and 23163-23173 and or sequences with at least 70% identity in the three complementarity determining regions (CDRs) to the sequences set forth in any one or more of SEQ ID NOS: 11524-11525, 11530-11531, 11549-11575, 11576-11592 and 23163-23173 or sequences with less than 3 substitutions in the three CDRs of the sequences set forth in any one or more of SEQ ID NOS: 11524-11525, 11530-11531, 11549-11575, 11576-11592 and 23163-23173 or sequences that bind to the same target antigens or the same epitopes on the target antigens as the sequences set forth in any one or more of SEQ ID NOS: 11524-11525, 11530-11531, 11549-11575, 11576-11592 and 23163-23173 and which encodes a polypeptide that binds to its antigen;
   (v) a non-immunoglobulin scaffold encoded by a polynucleotide of any one of SEQ ID NOS: 11662-11673 or sequences with at least 70% identity to sequences set forth in any one or more of SEQ ID NOS: 11662-11673 or sequences that bind to the same target antigens or the same epitopes on the target antigens as the sequences set forth in any one or more of SEQ ID NOS: 11662-11673;
   (vi) the ligand binding domain of a receptor comprising a sequence as set forth in any one of SEQ ID NO:11674-11691 or sequences with at least 70% identity thereto and which encodes a polypeptide that binds to its cognate;
   (vii) the receptor binding domain of a ligand comprising a sequence as set forth in any one of SEQ ID NO 11692 to 11702, 22391-22392, 22402-22404 or sequences with at least 70% identity thereto and which encodes a polypeptide that binds to its cognate;
   (viii) a single vH domain comprising a sequence as set forth in any one of SEQ ID NO: SEQ ID NOS: 11519-11523, 11526-11529, 11532-11548, 11644-11645 and 23174 or sequences with at least 70% identity to sequences set forth in any one or more of SEQ ID NOS: 11519-11523, 11526-11529, 11532-11548, 11644-11645 and 23174 and or sequences with 70% identity in the three complementarity determining regions (CDRs) to the sequences set forth in any one or more of SEQ ID NOS: 11519-11523, 11526-11529, 11532-11548, 11644-11645 and 23174 or sequences with less than 3 substitutions in the three CDRs of the sequences set forth in any one or more of SEQ ID NOS: 11519-11523, 11526-11529, 11532-11548, 11644-11645 and 23174 or sequences that bind to the same target antigens or the same epitopes on the target antigens as the sequences set forth in any one or more of SEQ ID NOS: 11519-11523, 11526-11529, 11532-11548, 11644-11645 and 23174 and which encodes a polypeptide that binds to its antigen;
   (ix) an adaptor binding domain comprising a sequence as set forth in any one of SEQ ID NO 11704 to 11712, or 22383 or sequences with at least 70% identity thereto and which encodes a polypeptide that binds to its adaptor;
   (x) an autoantigen comprising a sequence as set forth in any one of SEQ ID NO 11687, 22406-22407 to 11712, or 22383 or sequences with at least 70% identity thereto and which encodes a polypeptide that binds to its autoantibody or autoantibody producing cells;
   (xi) a TCR variable region (Va, Vb, Vg or Vd) comprising a sequence as set forth in any of SEQ ID NOs: 22396-2239 and 11653-11660 or sequences with at least 70% identity thereto or sequences with at least 70% identity in the three complementarity determining regions (CDRs) to the sequences set forth in any one or more of SEQ ID NOS: 22396-22397 and 11653-11660 or sequences with less than 3 substitutions in the three CDRs of the sequences set forth in any one or more of SEQ ID NOS: 22396-22397 and 11653-11660 or sequences that bind to the same target antigens or the same epitopes on the target antigens as the sequences set forth in any one or more of SEQ ID NOS: 22396-22397 and 11653-11660 and which encodes a polypeptide that binds to its antigen; and
   (xii) a single variable TCR domain (svd-TCR) comprising a sequence as set forth in any of SEQ ID NOs: 22399-22400 or sequences with at least 70% identity thereto or sequences with 70-99% identity in the three complementarity determining regions (CDRs) to the sequences set forth in any one or more of SEQ ID NOS: 22399-22400 or sequences with less than 3 substitutions in the three CDRs of the sequences set forth in any one or more of SEQ ID NOS: 22399-22400 or sequences that bind to the same target antigens or the same epitopes on the target antigens as the sequences set forth in any one or more of SEQ ID NOS: 22399-22400 and which encodes a polypeptide that binds to its antigen.
18. The at least one recombinant polynucleotide encoding at least one SAR polypeptide of embodiment 1, wherein at least one of the target antigens is expressed on blood lineage cells while at least one of the target antigens is expressed on solid tumor cells.
19. A recombinant expression system comprising the recombinant polynucleotide of embodiment 1 which is co-expressed with a therapeutic control, wherein the therapeutic control is selected from the group consisting of a truncated epidermal growth factor receptor (tEGFR), truncated epidermal growth factor receptor viii (tEGFRviii), truncated CD30 (tCD30), truncated BCMA (tBCMA), truncated CD19 (tCD19), CD34, thymidine kinase, cytosine deaminase, nitroreductase, xanthine-guanine phosphoribosyl transferase, human caspase 8, human caspase 9, inducible caspase 9 (icaspase9), purine nucleoside phosphorylase, linamarase/linamarin/glucose oxidase, deoxyribonucleoside kinase, horseradish peroxidase (HRP)/indole-3-acetic (IAA), Gamma-glutamylcysteine synthetase, CD20/alphaCD20, CD34/thymidine kinase chimera, dox-dependent caspase-2, mutant thymidine kinase (HSV-TKSR39), AP1903/Fas system, a chimeric cytokine receptor (CCR), 41BBL, CD40L, K13, MC159, cFLIP-L/MRITα, cFLIP-p22, HTLV1 Tax, HTLV2 Tax, HTLV2 Tax-RS mutant, FKBPx2-K13, FKBPx2-HTLV2-Tax, FKBPx2-HTLV2-Tax-RS, IL6R-304-vHH-Alb8-vHH, IL12f, PD1-4H1 scFV, PD1-5C4 scFV, PD1-4H1-Alb8-vHH, PD1-5C4-Alb8-vHH, CTLA4-Ipilimumab-scFv, CTLA4-Ipilimumab-Alb8-vHH, IL6-19A-scFV, IL6-19A-scFV-Alb8-vHH, sHVEM, sHVEM-Alb8-vHH, hTERT, Fx06, CD3z, CD3z-GGGS-41BB, CD3-BBz, CD3-CD28z, CD3-CD28-Lck fusion protein, shRNA targeting Brd4, chimeric antigen receptor (CAR), hTERT, heparinase, a CAR, an inhibitory CAR and combination thereof and combinations thereof.
20. At least one vector comprising the recombinant polynucleotide of embodiment 1, wherein the vector is selected from the group consisting of a DNA vector, an RNA vector, a plasmid, a lentivirus vector, an adenoviral vector, a retrovirus vector, a baculovirus vector, a sleeping beauty transposon vector, and a piggybac transposon vector.
21. At least one SAR polypeptide encoded by the at least one recombinant polynucleotide of embodiment 1.
22. A recombinant cell or a cell population that expresses the at least one recombinant polynucleotide of embodiment 1.
23. The cell or cell population of embodiment 22, wherein the cell is an immune effector cell or a stem cell that can give rise to an immune effector cell, or an induced pluripotent stem cell (iPSC) that can give rise to an immune effector cell.
24. The cell or cell population of embodiment 22, where the cell is an autologous cell or an allogeneic cell.
25. The cell or cell population of embodiment 22 with impaired or abolished functional expression of an endogenous TCR and with functional expression of a SAR polypeptide.
26. The cell or cell population of embodiment 22, where the SAR polypeptide is expressed from an expression cassette placed in the locus of an endogenous T cell gene.
27. The cell or cell population of embodiment 22, where the at least one polynucleotide is under the control of the promoter and/or regulatory elements for an endogenous T cell gene.
28. A cell or cell population of embodiments 26 or 27, where the endogenous T cell gene locus is TRAC locus, TRBC locus, TRGC locus and/or TRDC locus.
29. The cell or cell population of embodiments 26 and/or 27, wherein the placement of the SAR polynucleotide disrupts or abolishes the endogenous expression of a TCR comprising an endogenous TCRα chain and/or an endogenous TCRβ chain, or an endogenous TCRγ chain and/or endogenous TCRδ chain in the T cell.
30. The cell or cell population of embodiments 26 and 27, wherein the disruption or abolished expression of an endogenous TCR results in enhanced expression and/or activity of the non-naturally occurring immune receptor as compared to its expression and/or activity in T cells with wild-type endogenous TCR.
31. The cell or cell population of embodiment 30, wherein the SAR has the backbone of one or more of a SIR, a cTCR and/or an Ab-TCR.
32. The cell or cell population of embodiments 25 or 29, wherein the SAR polypeptide has the backbone of a TFP and the disruption or abolished expression of an endogenous TCR results in impaired expression and/or activity of the SAR as compared to its expression and/or activity in T cells with wild-type endogenous TCR.
33. The cell or cell population of embodiments 32, wherein the SAR polypeptide has the backbone of a TFP and the cell or cell population further expresses a sequence encoding a TCR constant chain, wherein the TCR constant chain is
   i) a TCR alpha constant chain or fragment thereof, or
   ii) a TCR beta constant chain or a fragment thereof, or
   iii) a TCR gamma constant chain or fragment thereof, or
   iv) a TCR delta constant chain or a fragment thereof, or
   v) a combination of i) and ii) or iii) and iv)
34. The cell or cell population of embodiments 33, wherein the expression of a sequence encoding a TCR constant chain restores the expression and/or activity of the SAR polypeptide.
35. A method of making a SAR-expressing immune effector cell, comprising introducing at least one vector of embodiment 20 or at least one recombinant polynucleotide of embodiment 1 into an immune effector cell or a hematopoietic stem cell or progenitor cell that can give rise to an immune effector cell, under conditions such that the SAR polypeptide is expressed.
36. A method of expanding the SAR-expressing immune effector cells of embodiment 22 in a gas permeable flask under normoxic or hypoxic conditions.
37. A method of embodiment 36, where the SAR-expressing immune effector cells are expanded in the presence of a SMAC mimetic or a NIK agonist compound.
38. A method of providing anti-disease immunity in a subject comprising administering to the subject an effective amount of the immune effector cell or a stem cell that can give rise to an immune effector cell of any one of embodiments 22-35, wherein the cell is an autologous T cell or an allogeneic T cell, or an autologous NKT cell or an allogeneic NKT cell or an autologous or an allogeneic hematopoietic stem cell or an autologous or an allogeneic iPSC that can give rise to an immune effector cell.
39. A method of treating or preventing a disease associated with expression of a disease-associated antigen in a subject, comprising administering to the subject an effective amount of an immune effector cell comprising a synthetic antigen receptor (SAR) polypeptide, wherein the SAR polypeptide bind to one or more disease-associated antigens selected from a group consisting of: CD5, CD19; CD123; CD22; CD23, CD30; CD171; CS-1 (also referred to as CD2 subset 1, CRACC, SLAMF7, CD319, and 19A24); C-type lectin-like molecule-1 (CLL-1 or CLECL1); CD33; epidermal growth factor receptor variant III (EGFRviii); ganglioside G2 (GD2); ganglioside GD3 (aNeu5Ac(2-8)aNeu5Ac(2-3)bDGalp(l-4 )bDGlcp(l-l)Cer); TNF receptor family member B cell maturation (BCMA); Tn antigen (Tn Ag) or (GalNAcα-Ser/Thr)); prostate-specific membrane antigen (PSMA); Receptor tyrosine kinase-like orphan receptor 1 (ROR1); Fms Like Tyrosine Kinase 3 (FLT3); Tumor-associated glycoprotein 72 (TAG72); CD38; CD44v6; a glycosylated CD43 epitope expressed on acute leukemia or lymphoma but not on hematopoietic progenitors, a glycosylated CD43 epitope expressed on non-hematopoietic cancers, Carcinoembryonic antigen (CEA); Epithelial cell adhesion molecule (EPCAM); B7H3 (CD276); KIT (CD117); Interleukin-13 receptor subunit alpha-2 (IL-13Ra2 or CD213A2); Mesothelin; Interleukin 11 receptor alpha (IL-llRa); prostate stem cell antigen (PSCA); Protease Serine 21 (Testisin or PRSS21); vascular endothelial growth factor receptor 2 (VEGFR2); Lewis(Y) antigen; CD24; Platelet-derived growth factor receptor beta (PDGFR-beta); Stage-specific embryonic antigen-4 (SSEA-4); CD20; Folate receptor alpha (FRa or FR1); Folate receptor beta (FRb); Receptor tyrosine-protein kinase ERBB2 (Her2/neu); Mucin 1, cell surface associated (MUC1); epidermal growth factor receptor (EGFR); neural cell adhesion molecule (NCAM); Prostase; prostatic acid phosphatase (PAP); elongation factor 2 mutated (ELF2M); Ephrin B2; fibroblast activation protein alpha (FAP); insulin-like growth factor 1 receptor (IGF-I receptor), carbonic anhydrase IX (CAlX); Proteasome (Prosome, Macropain) Subunit, Beta Type, 9 (LMP2); glycoprotein 100 (gpl00); oncogene fusion protein consisting of breakpoint cluster region (BCR) and Abelson murine leukemia viral oncogene homolog 1 (Abl) (bcr-abl); tyrosinase; ephrin type-A receptor 2 (EphA2); Fucosyl GM1; sialyl Lewis adhesion molecule (sLe); ganglioside GM3 (aNeu5Ac(2-3)bDClalp(l- 4)bDGlcp(l-1)Cer); transglutaminase 5 (TGS5); high molecular weight-melanoma associated antigen (HMWMAA); o-acetyl-GD2 ganglioside (OAcGD2); \tumor endothelial marker 1 (TEM1/CD248); tumor endothelial marker 7-related (TEM7R); claudin 6 (CLDN6); thyroid stimulating hormone receptor (TSHR); G protein coupled receptor class C group 5, member D (GPRC5D); chromosome X open reading frame 61 (CXORF61); CD97; CD179a; anaplastic lymphoma kinase (ALK); Polysialic acid; placenta-specific 1 (PLAC1); hexasaccharide portion of globoH glycoceramide (GloboH); mammary gland differentiation antigen (NY-BR-1); uroplakin 2 (UPK2); Hepatitis A virus cellular receptor 1 (HAVCR1); adrenoceptor beta 3 (ADRB3); pannexin 3 (PANX3); G protein-coupled receptor 20 (GPR20); lymphocyte antigen 6 complex, locus K 9 (LY6K); Olfactory receptor 51E2 (OR51E2); TCR Gamma Alternate Reading Frame Protein (TARP); Wilms tumor protein (WT1); Cancer/testis antigen 1 (NY-ES0-1); Cancer/testis antigen 2 (LAGE-1a); Melanoma-associated antigen 1 (MAGE-A1); ETS translocation-variant gene 6, located on chromosome 12p (ETV6-AML); sperm protein 17 (SPA17); X Antigen Family, Member 1A (XAGEl); angiopoietin-binding cell surface receptor 2 (Tie 2); melanoma cancer testis antigen-1 (MAD-CT-1); melanoma cancer testis antigen-2 (MAD-CT-2); Fos-related antigen 1; tumor protein p53 (p53); p53 mutant; prostein; surviving; telomerase; prostate carcinoma tumor antigen-1 (PCT A-1 or Galectin 8), melanoma antigen recognized by T cells 1 (MelanA or MARTI); Rat sarcoma (Ras) mutant; human Telomerase reverse transcriptase (hTERT); sarcoma translocation breakpoints; melanoma inhibitor of apoptosis (ML-IAP); ERG (transmembrane protease, serine 2 (TMPRSS2) ETS fusion gene); N-Acetyl glucosaminyl-transferase V (NA17); paired box protein Pax-3 (PAX3); Androgen receptor; Cyclin Bl; v-myc avian myelocytomatosis viral oncogene neuroblastoma derived homolog (MYCN); Ras Homolog Family Member C (RhoC); Tyrosinase-related protein 2 (TRP-2); Cytochrome P450 lB 1 (CYPlB 1 ); CCCTC-Binding Factor (Zinc Finger Protein)-Like (BORIS or Brother of the Regulator oflmprinted Sites), Squamous Cell Carcinoma Antigen Recognized By T Cells 3 (SART3); Paired box protein Pax-5 (PAX5); proacrosin binding protein sp32 (OY-TESl); lymphocyte-specific protein tyrosine kinase (LCK); A kinase anchor protein 4 (AKAP-4); synovial sarcoma, X breakpoint 2 (SSX2); Receptor for Advanced Glycation End products (RAGE-1); renal ubiquitous 1 (RUl); renal ubiquitous 2 (RU2); legumain; human papilloma virus E6 (HPV E6); human papilloma virus E7 (HPV E7); intestinal carboxyl esterase; heat shock protein 70-2 mutated (mut hsp70-2); CD79a; CD79b; CD72; Leukocyte-associated immunoglobulin-like receptor 1 (LAIRl); Fc fragment of IgA receptor (FCAR or CD89); Leukocyte immunoglobulin-like receptor subfamily A member 2 (LILRA2); CD300 molecule-like family member f (CD300LF); C-type lectin domain family 12 member A (CLEC12A); bone marrow stromal cell antigen 2 (BST2); EGF-like module-containing mucin-like hormone receptor-like 2 (EMR2); lymphocyte antigen 75 (LY75); Glypican-3 (GPC3); Fc receptor-like 5 (FCRL5); and immunoglobulin lambda-like polypeptide 1 (IGLLI), MPL, Biotin, c-MYC epitope Tag, CD34, LAMP1 TROP2, GFRalpha4, CDH17, CDH6, NYBR1, CDH19, CD200R, Slea (CA19.9; Sialyl Lewis Antigen); Fucosyl-GM1, PTK7, gpNMB, CDH1-CD324, DLL3, CD276/B7H3, IL11Ra, IL13Ra2, CD179b-IGLl1, TCRgamma-delta, NKG2D, CD32 (FCGR2A), Tim1-/HVCR1, CSF2RA (GM-CSFR-alpha), TGFbetaR2, , Lews Ag, TCR-beta1 chain, TCR-beta2 chain, TCR-gamma chain, TCR-delta chain, FITC, Leutenizing hormone receptor (LHR), Follicle stimulating hormone receptor (FSHR), Chorionic Gonadotropin Hormone receptor (CGHR), CCR4, SLAMF6, SLAMF4, HIV1 envelope glycoprotein, HTLV1-Tax, CMV pp65, EBV-EBNA3c, KSHV K8.1, KSHV-gH, influenza A hemagglutinin (HA), GAD, PDL1, Guanylyl cyclase C (GCC), auto antibody to desmoglein 3 (Dsg3) and desmoglein 1 (Dsg1), HLA, HLA-A, HLA-A2, HLA-B, HLA-C, HLA-DP, HLA-DM, HLA-DOA, HLA-DOB, HLA-DQ, HLA-DR, HLA-G, IGE, CD99, RAS G12V, Tissue Factor 1 (TF1), AFP, GPRC5D, claudin18.2 (CLD18A2 OR CLDN18A.2)), P-glycoprotein, STEAP1, LIV1, NECTIN-4, CRIPTO, GPA33, BST1/CD157, low conductance chloride channel, TAJ/TROY, MPL (TPO-R), KIR3DL2, CD32b, CD229, Toso, GPC3, MR1, NYESO-1/MHC complex, CMVpp65/MHC complex.
40. The \ method of embodiment 39, wherein the disease associated with expression of the disease associated antigen is selected from the group consisting of a proliferative disease, a precancerous condition, a cancer, and a non-cancer related indication associated with expression of the disease-associated antigen.
41. A recombinant polynucleotide encoding a SAR polypeptide, the polynucleotide comprising a sequence selected from the group consisting of SEQ ID NO: 1849-10720, 21609-21625, 21626-21659, 21662-21792. 21808, 21813-21840, 21843-21879, 21882-21892, 21894-21899, 21901-21947, 21949-21963, 21967-21972, 21985-22085, 22087-22099, 22104-22207, 22209-22250, 22252-22254, 22256-22262, 22264-22273, 22275-22284, 22286-22296 and 22307-22312 or a sequence with at least 75% identity to the nucleotide sequence of the above.
42. An amino acid sequence encoding a synthetic antigen receptor (SAR) polypeptide selected from the group consisting of SEQ ID NO: 12539-21410, 22445-22460, 22462-22495, 22498-22614, 22624, 22626-22655, 22658-22685, 22687-22694, 22697-22707, 22709-22714, 22716-22762, 22764-22778, 22782-22787, 22800-22900, 22902-22914, 22919-23022, 23024-23065, 23067-23069, 23071-23077, 23090-23099, 23101-23111 and 23122-23127 or a sequence with at least 75% identity to an amino acid sequences of the forgoing.
43. A composition comprising at least one polynucleotide of embodiments 1 or 41, a SAR polypeptide molecule of embodiments 21 or 42, a vector of embodiment 20 or the cell of any one of embodiments 22-34 and a pharmaceutically acceptable excipient.
44. A kit comprising at least one polynucleotide of embodiments 1 or 41, a SAR polypeptide of embodiments 21 or 42, a vector of embodiment 20 or the cell of any one of embodiments 22-34

## Claims

1. A synthetic antigen receptor (SAR) polypeptide comprising a first and a second polypeptide chain, the first and/or the second polypeptide chains comprise a first module comprising one or more non-scFv antigen binding domains or fragments thereof; and wherein
(I) the first polypeptide chain further comprises:
a) a second module comprising a light chain variable (vL) or Ig linker domain;
b) an optional third module comprising an extracellular domain, a connecting peptide or a hinge domain; and
c) a fourth module that comprises a transmembrane domain;
and
(II) the second polypeptide chain further comprises:
a) a second module comprising a heavy chain variable (vH) or Ig linker domain;
b) an optional third module comprising an extracellular domain, a connecting peptide or a hinge domain; and
c) a fourth module that comprises a transmembrane domain;
wherein the first module is operably linked to the N-terminus or near the N-terminus of the second module, and the optional third module is located between and operably linked to the second module and the fourth module.

2. The SAR polypeptide of claim 1, wherein the first and/or second polypeptide chain comprises a fifth module comprising one or more intracellular signaling domains, optionally wherein the one or more intracellular signaling domains comprise:
a) a primary activation domain,
b) one or more co-stimulatory domains, or
c) a primary activation domain and one or more co-stimulatory domains.

3. The SAR polypeptide of claim 2, wherein
a) the first, second, third, fourth and/or fifth modules are operationally linked via one or more linkers; and/or
b) the one or more non-scFv antigen binding domains or fragment thereof present in the first modules are operationally linked via one or more linkers.

4. The SAR polypeptide of any one of claims 1-3, wherein the one or more non-scFv antigen binding domains or fragment thereof, the vL domain, and/or the vH fragment is a fully human, humanized, chimeric or a non-human domain,
optionally wherein the one or more non-scFv antigen binding domains or fragments thereof are selected from the group consisting of:
a) a single domain antibody or a fragment thereof, optionally wherein the single domain antibody or fragment thereof is selected from a single vL domain (SVL), a single vH domain (SVH), a vHH domain or a fragment thereof;
b) a non-immunoglobulin antigen binding scaffold or a fragment thereof;
c) a single variable domain of a TCR (svd-TCR) or a fragment thereof;
d) a ligand-binding domain of a receptor or a fragment thereof;
e) a receptor-binding domain of a ligand;
f) an autoantigen or a fragment thereof;
g) an adaptor binding domain or a fragment thereof; and
h) an Fc binding domain or a fragment thereof;
preferably wherein the one or more non-scFv antigen binding domains or fragments thereof are:
(i) a single vH domain (SVH), a single vL domain (SVL), a vHH domain or a fragment thereof; or
(ii) a non-immunoglobulin antigen binding scaffold or fragment thereof selected from a DARPIN, an affibody, an affilin, an adnectin, an affitin, an obody, a repebody, a fynomer, an alphabody, an avimer, an atrimer, a centyrin, a pronecti, an anticalins, a kunitz domain, an Armadillo repeat protein and a D domain, or a fragment of any of the foregoing.

5. The SAR polypeptide of claim 4, wherein the one or more non-scFv antigen binding domains or fragments thereof are attached to the N-terminus or near the N-terminus of
(i) the second module of the first polypeptide chain, comprising a vH domain that is operably linked to a) a first T cell receptor (TCR) constant chain fragment, wherein said first TCR constant chain fragment comprises a first connecting peptide operably linked to a first transmembrane domain of a first TCR subunit or b) a first CD3 chain fragment; and
(ii) the second module of the second polypeptide chain, comprising a vL domain that is operably linked to a) a second T cell receptor (TCR) constant chain fragment, wherein said second TCR constant chain fragment comprises a second connecting peptide operably linked to a second transmembrane domain of a second TCR subunit or b) a second CD3 chain fragment.

6. The SAR polypeptide of claim 5, wherein
a) the vH domain is operably linked to the first TCR constant chain fragment or the first CD3 chain fragment via an optional linker; and/or
b) the vL domain is operably linked to the second TCR constant chain fragment or the second CD3 chain fragment via an optional linker;
optionally wherein the linker comprises a polypeptide with SEQ ID NOs: 11832-11865, 11714-11730 or a fragment or a variant thereof with at least 70% sequence identity to a polypeptide having the sequence of SEQ ID NOs: 11832-11865, 11714-11730 or a domain that is between 25 to 500 amino acids in length.

7. The SAR polypeptide of any one of claims 1-6, wherein
(i) the first TCR constant chain fragment and the second TCR constant chain fragment form a T cell receptor module (TCRM) that is capable of recruiting at least one TCR-associated signaling module when expressed in a T cell; and/or
(ii) the vL domain of the second module of the first polypeptide chain and the vH domain of the second module of the second polypeptide chain form an antigen-binding module that specifically binds to a target antigen.

8. The SAR polypeptide of any one of claims 4-7, wherein
a) the TCR constant chain fragment comprises one or more features selected from the group consisting of:
i) a polypeptide or a fragment thereof having at least 70% sequence identity to a polypeptide having the sequence of any one of SEQ ID NOs: 11732-11742, 11744-11766, 11768-11777, 11793-11830;
ii) an extracellular domain or a fragment thereof having at least 70% sequence homology to an extracellular domain having the sequence of any one of SEQ ID NOs:11848-11865;iii) a connecting peptide or a fragment thereof having at least 70% sequence identity to a connecting peptide having the sequence of any one of SEQ ID NOs: 11867-11874;
iv) a transmembrane domain or a fragment thereof having at least 70% sequence identity to a transmembrane domain having a sequence of any one of SEQ ID NOs: 11877-11880; and
v) an intracellular signaling domain containing a cytosolic domain or a fragment thereof with at least 70% sequence identity to a polypeptide having the sequence of any one of SEQ ID NOs: 11883-11885; or
b) the CD3 chain fragment comprises one or more features selected from the group consisting of:
i) a polypeptide or a fragment thereof having at least 70% sequence homology to a polypeptide having the sequence of any one of SEQ ID NOs:11778-11779, 11903 to 11906, 11789-11790;
ii) a connecting peptide or a fragment thereof having at least 70% sequence homology to a connecting peptide having the sequence of any one of SEQ ID NOs: 11875;
iii) the transmembrane domain or a fragment thereof has at least 70% sequence homology to a transmembrane domain having a sequence of any one of SEQ ID NOs: 11881, 23332-23334;
iv) a cytosolic domain or a fragment thereof comprising a primary signaling domain with at least 70% sequence homology to a polypeptide having the sequence of any one of SEQ ID NOs: 11886; 23335-23337; and
v) one or more optional co-stimulatory domains, wherein the one or more optional co-stimulatory domains comprise a sequence with at least 70% sequence homology to a polypeptide having the sequence of any one of SEQ ID NOs: 11781-11782.

9. The SAR polypeptide of any one of claims 1-8, wherein the one or more non-scFv and/or one or more vL/vH antigen binding domains bind to at least one antigen selected from the group of CD5; CD19; CD123; CD22; CD30; CD171; CS1 (also referred to as CD2 subset 1, CRACC, SLAMF7, CD319, and 19A24); C-type lectin-like molecule-1 (CLL-1 or CLECL1); CD33; epidermal growth factor receptor variant III (EGFRviii); ganglioside G2 (GD2); ganglioside GD3 (aNeu5Ac(2-8)aNeu5Ac(2-3)bDGalp(1-4)bDGlcp(1-1)Cer); TNF receptor family member B cell maturation (BCMA); Tn antigen ((Tn Ag) or (GalNAcα-Ser/Thr)); prostate-specific membrane antigen (PSMA); Receptor tyrosine kinase-like orphan receptor 1 (ROR1); Fms Like Tyrosine Kinase 3 (FLT3); Tumor-associated glycoprotein 72 (TAG72); CD38; CD44v6; a glycosylated CD43 epitope expressed on acute leukemia or lymphoma but not on hematopoietic progenitors, a glycosylated CD43 epitope expressed on non-hematopoietic cancers, Carcinoembryonic antigen (CEA); Epithelial cell adhesion molecule (EPCAM); B7H3 (CD276); KIT (CD117); Interleukin-13 receptor subunit alpha-2 (IL-13Ra2 or CD213A2); Mesothelin; Interleukin 11 receptor alpha (IL-IIRa); prostate stem cell antigen (PSCA); Protease Serine 21 (Testisin or PRSS21); vascular endothelial growth factor receptor 2 (VEGFR2); Lewis(Y) antigen; CD24; Platelet-derived growth factor receptor beta (PDGFR-beta); Stage-specific embryonic antigen-4 (SSEA-4); CD20; Folate receptor alpha (FRa or FR1); Folate receptor beta (FRb); Receptor tyrosine-protein kinase ERBB2 (Her2/neu); Mucin 1, cell surface associated (MUC1); epidermal growth factor receptor (EGFR); neural cell adhesion molecule (NCAM); Prostase; prostatic acid phosphatase (PAP); elongation factor 2 mutated (ELF2M); Ephrin B2; fibroblast activation protein alpha (FAP); insulin-like growth factor 1 receptor (IGF-I receptor); carbonic anhydrase IX (CAIX); Proteasome (Prosome, Macropain) Subunit, Beta Type, 9 (LMP2); glycoprotein 100 (gp100); oncogene fusion protein consisting of breakpoint cluster region (BCR) and Abelson murine leukemia viral oncogene homolog 1 (Abl) (bcr-abl); tyrosinase; ephrin type-A receptor 2 (EphA2); sialyl Lewis adhesion molecule (sLe); ganglioside GM3 (aNeu5Ac(2-3)bDClalp(I-4)bDGlcp(I-1)Cer); transglutaminase 5 (TGS5); high molecular weight-melanoma associated antigen (HMWMAA); o-acetyl-GD2 ganglioside (OAcGD2); tumor endothelial marker 1 (TEM1/CD248); tumor endothelial marker 7-related (TEM7R); claudin 6 (CLDN6); thyroid stimulating hormone receptor (TSHR); G protein coupled receptor class C group 5, member D (GPRC5D); chromosome X open reading frame 61 (CXORF61); CD97; CD179a; anaplastic lymphoma kinase (ALK); Polysialic acid; placenta-specific 1 (PLAC1); hexasaccharide portion of globoH glycoceramide (GloboH); mammary gland differentiation antigen (NY-BR-1); uroplakin 2 (UPK2); Hepatitis A virus cellular receptor 1 (HAVCR1); adrenoceptor beta 3 (ADRB3); pannexin 3 (PANX3); G protein-coupled receptor 20 (GPR20); lymphocyte antigen 6 complex, locus K 9 (LY6K); Olfactory receptor 51E2 (OR51E2); TCR Gamma Alternate Reading Frame Protein (TARP); Wilms tumor protein (WT1); Cancer/testis antigen 1 (NY-ESO-1); Cancer/testis antigen 2 (LAGE-1a); Melanoma-associated antigen 1 (MAGE-A1); ETS translocation-variant gene 6, located on chromosome 12p (ETV6-AML); sperm protein 17 (SPA17); X Antigen Family, Member 1A (XAGE1); angiopoietin-binding cell surface receptor 2 (Tie 2); melanoma cancer testis antigen-1 (MAD-CT-1); melanoma cancer testis antigen-2 (MAD-CT-2); Fos-related antigen 1; tumor protein p53 (p53); p53 mutant; prostein; survivin; telomerase; prostate carcinoma tumor antigen-1 (PCT A-1 or Galectin 8), melanoma antigen recognized by T cells 1 (MelanA or MARTI); Rat sarcoma (Ras) mutant; human Telomerase reverse transcriptase (hTERT); sarcoma translocation breakpoints; melanoma inhibitor of apoptosis (ML-IAP); ERG (transmembrane protease, serine 2 (TMPRSS2) ETS fusion gene); N-Acetyl glucosaminyl-transferase V (NA17); paired box protein Pax-3 (PAX3); Androgen receptor; Cyclin B1; v-myc avian myelocytomatosis viral oncogene neuroblastoma derived homolog (MYCN); Ras Homolog Family Member C (RhoC); Tyrosinase-related protein 2 (TRP-2); Cytochrome P450 1B 1 (CYP1B 1); CCCTC-Binding Factor (Zinc Finger Protein)-Like (BORIS or Brother of the Regulator of Imprinted Sites), Squamous Cell Carcinoma Antigen Recognized By T Cells 3 (SART3); Paired box protein Pax-5 (PAX5); proacrosin binding protein sp32 (OY-TES1); lymphocyte-specific protein tyrosine kinase (LCK); A kinase anchor protein 4 (AKAP-4); synovial sarcoma, X breakpoint 2 (SSX2); Receptor for Advanced Glycation End products (RAGE-1); renal ubiquitous 1 (RU1); renal ubiquitous 2 (RU2); legumain; human papilloma virus E6 (HPV E6); human papilloma virus E7 (HPV E7); intestinal carboxyl esterase; heat shock protein 70-2 mutated (mut hsp70-2); CD79a; CD79b; CD72; Leukocyte-associated immunoglobulin-like receptor 1 (LAIR1); Fc fragment of IgA receptor (FCAR or CD89); Leukocyte immunoglobulin-like receptor subfamily A member 2 (LILRA2); CD300 molecule-like family member f (CD300LF); C-type lectin domain family 12 member A (CLEC12A); bone marrow stromal cell antigen 2 (BST2); EGF-like module-containing mucin-like hormone receptor-like 2 (EMR2); lymphocyte antigen 75 (LY75); Glypican-3 (GPC3); Fc receptor-like 5 (FCRL5); immunoglobulin lambda-like polypeptide 1 (IGLL1); MPL; Biotin; c-MYC epitope Tag; CD34; LAMP1 TROP2; GFRalpha4; CDH17; CDH6; NYBR1; CDH19; CD200R; Slea (CA19.9; Sialyl Lewis Antigen); Fucosyl-GM1; PTK7; gpNMB; CDH1-CD324; DLL3; CD276/B7H3; IL11Ra; IL13Ra2; CD179b-IGLI1; TCR gamma-delta; NKG2D; CD32 (FCGR2A); Tn ag; Tim1-/HVCR1; CSF2RA (GM-CSFR-alpha); TGFbetaR2; Lewis Ag; TCR-beta1 chain; TCR-beta2 chain; TCR-gamma chain; TCR-delta chain; FITC; Luteinizing hormone receptor (LHR); Follicle stimulating hormone receptor (FSHR); Gonadotropin Hormone receptor (CGHR or GR); CCR4; GD3; SLAMF6; SLAMF4; HIV1 envelope glycoprotein; HTLV1-Tax; CMV pp65; EBV-EBNA3c; KSHV K8.1; KSHV-gH; influenza A hemagglutinin (HA); GAD; PDL1; Guanylyl cyclase C (GCC); auto antibody to desmoglein 3 (Dsg3); auto antibody to desmoglein 1 (Dsg1); HLA; HLA-A; HLA-A2; HLA-B; HLA-C; HLA-DP; HLA-DM; HLA-DOA; HLA-DOB; HLA-DQ; HLA-DR; HLA-G; IgE, CD99; Ras G12V; Tissue Factor 1 (TF1); AFP; GPRC5D; Claudin18.2 (CLD18A2 or CLDN18A.2); P-glycoprotein; STEAP1; Liv1; Nectin-4; Cripto; gpA33; BST1/CD157; low conductance chloride channel (LCCC); TAJ/TROY; MPL (TPO-R); KIR3DL2; CD32b; CD229; Toso; GPC3; BAFF-R; MR1; CMV-pp65/MHC complex; and NYESO-1/MHC complex.

10. The SAR polypeptide of any one of claims 1-9, wherein
(i) the heavy chain variable region (vH) comprises a sequence as set forth in any of SEQ ID NOs:11161, 10978-11160; 11162-11214, and 23148-23161 or sequences with at least 70% identity thereto or sequences with at least 70% identity in the three complementarity determining regions (CDRs) to the sequences set forth in any one or more of SEQ ID NOs: 11161, 10978-11160; 11162-11214, and 23148-23161 or sequences with less than 3 substitutions in the three CDRs of the sequences set forth in any one or more of SEQ ID NOs: 11161, 10978-11160; 11162-11214, and 23148-23161 or sequences that bind to the same target antigens or the same epitopes on the target antigens as the sequences set forth in any one or more of SEQ ID NOs: 11161, 10978-11160; 11162-11214, and 23148-23161 and which encodes a polypeptide that binds to its antigen;
(ii) the light chain variable region (vL) comprises a sequence as set forth in any one of SEQ ID NOs: 10919, 10736-10918, 10920-10972 and 23136-23147 or sequences with at least 70% identity to sequences set forth in any one or more of SEQ ID NOs: 10919, 10736-10918, 10920-10972 and 23136-23147 or sequences with at least 70% identity in the three complementarity determining regions (CDRs) to the sequences set forth in any one or more of SEQ ID NOs: 10919, 10736-10918, 10920-10972 and 23136-23147 or sequences with less than 3 substitutions in the three CDRs of the sequences set forth in any one or more of SEQ ID NOs: 10919, 10736-10918, 10920-10972 and 23136-23147 or sequences that bind to the same target antigens or the same epitopes on the target antigens as the sequences set forth in any one or more of SEQ ID NOs: 10919, 10736-10918, 10920-10972 and 23136-23147 and which encodes a polypeptide that binds to its antigen;
(iii) the VHH domain comprises a sequence as set forth in any one of SEQ ID NOs: SEQ ID NOs: 11531, 11524-11525, 11530, 11532-11531, 11549-11575, 11576-11592 and 23163-23173 or sequences with at least 70% identity to sequences set forth in any one or more of SEQ ID NOs: 11531, 11524-11525, 11530, 11532-11531, 11549-11575, 11576-11592 and 23163-23173 and or sequences with at least 70% identity in the three complementarity determining regions (CDRs) to the sequences set forth in any one or more of SEQ ID NOs: 11531, 11524-11525, 11530, 11532-11531, 11549-11575, 11576-11592 and 23163-23173 or sequences with less than 3 substitutions in the three CDRs of the sequences set forth in any one or more of SEQ ID NOs: 11531, 11524-11525, 11530, 11532-11531, 11549-11575, 11576-11592 and 23163-23173 or sequences that bind to the same target antigens or the same epitopes on the target antigens as the sequences set forth in any one or more of SEQ ID NOs: 11531, 11524-11525, 11530, 11532-11531, 11549-11575, 11576-11592 and 23163-23173 and which encodes a polypeptide that binds to its antigen;
(iv) a non-immunoglobulin antigen binding scaffold is encoded by a polynucleotide of any one of SEQ ID NOs: 11662-11673 or sequences with at least 70% identity to sequences set forth in any one or more of SEQ ID NOs: 11662-11673 or sequences that bind to the same target antigens or the same epitopes on the target antigens as the sequences set forth in any one or more of SEQ ID NOs: 11662-11673;
(v) the ligand-binding domain of a receptor comprises a sequence as set forth in any one of SEQ ID NOs:11674-11691 or sequences with at least 70% identity thereto and which encodes a polypeptide that binds to its cognate;
(vi) the receptor-binding domain of a ligand comprises a sequence as set forth in any one of SEQ ID NOs 11692 to 11702, 22391-22392, 22402-22404 or sequences with at least 70% identity thereto and which encodes a polypeptide that binds to its cognate;
(vii) the single vH domain comprises a sequence as set forth in any one of SEQ ID NOs: 11519-11523, 11526-11529, 11532-11548, 11644-11645 and 23174 or sequences with at least 70% identity to sequences set forth in any one or more of SEQ ID NOs: 11519-11523, 11526-11529, 11532-11548, 11644-11645 and 23174 or sequences with 70% identity in the three complementarity determining regions (CDRs) to the sequences set forth in any one or more of SEQ ID NOs: 11519-11523, 11526-11529, 11532-11548, 11644-11645 and 23174 or sequences with less than 3 substitutions in the three CDRs of the sequences set forth in any one or more of SEQ ID NOs: 11519-11523, 11526-11529, 11532-11548, 11644-11645 and 23174 or sequences that bind to the same target antigens or the same epitopes on the target antigens as the sequences set forth in any one or more of SEQ ID NOs: 11519-11523, 11526-11529, 11532-11548, 11644-11645 and 23174 and which encodes a polypeptide that binds to its antigen;
(viii) an adaptor binding domain comprising a sequence as set forth in any one of SEQ ID NOs: 11704 to 11712, or 22383 or sequences with at least 70% identity thereto and which encodes a polypeptide that binds to its adaptor;
(ix) an autoantigen comprising a sequence as set forth in any one of SEQ ID NOs: 11687, 22406-22407 to 11712, or 22383 or sequences with at least 70% identity thereto and which encodes a polypeptide that binds to its autoantibody or autoantibody producing cells;
(x) a single variable TCR domain (svd-TCR) comprising a sequence as set forth in any of SEQ ID NOs: 22399-22400 or sequences with at least 70% identity thereto or sequences with 70-99% identity in the three complementarity determining regions (CDRs) to the sequences set forth in any one or more of SEQ ID NOs: 22399-22400 or sequences with less than 3 substitutions in the three CDRs of the sequences set forth in any one or more of SEQ ID NOs: 22399-22400 or sequences that bind to the same target antigens or the same epitopes on the target antigens as the sequences set forth in any one or more of SEQ ID NOs: 22399-22400 and which encodes a polypeptide that binds to its antigen.

11. The SAR polypeptide of any one of claims 1-10, comprising an amino acid sequence contained in the first or the second polypeptide chain of the SAR selected from the group consisting of SEQ ID NOs: 16951, 12539-14188, 14778-14799, 14811-14835, 14853-14899, 14905-14926, 14938-14962, 14978-15026, 15032-15053, 15065-15090, 15105-15153, 15159-15180, 15192-15216, 15234-15280, 15286-15307, 15319-15343, 15361-15407, 15413-15434, 15446-15470, 15488-15534, 15540-15561, 15573-15597, 15615-15661, 15669-15693, 15708-15729, 15738-15759, 15763-15787, 15801-15842, 15846-15867, 15876-15911, 15669-15759, 15763-15911, 15917-15941, 15956-15977, 15986-16007, 16011-16035, 16049-16091, 16094-16115, 16124-16159, 16165-16189, 16204-16225, 16234-16255, 16259-16283, 16297-16338, 16342-16363, 16372-16407, 16413-16437, 16452-16473, 16507-16531, 16545-16586, 16590-16611, 16661-16685, 16700-16721, 16730-16751, 16755-16779, 16793-16834, 16838-16859, 16909-16933, 16948-16950, 16952-16969, 16978-16999, 17003-17027, 17041-17083, 17086-17107, 17116-17151, 17160-17184, 17234-17257, 17306-17330, 17379-17403, 17452-17476, 17525-17549, 22445-22460, or a sequence with at least 95% identity to an amino acid sequences of the forgoing.

12. A polynucleotide encoding the SAR polypeptide of any one of claims 1-11.

13. A recombinant cell comprising or expressing a SAR polypeptide of any one of claims 1-11 or comprising a polynucleotide of claim 12, optionally wherein the cell is an immune effector cell or a stem cell that can give rise to an immune effector cell, preferably wherein the cell is selected from one or more of the following:
a) an autologous T cell, an allogeneic T cell, an autologous natural killer T (NKT) cell, an allogeneic NKT cell, an autologous hematopoietic stem cell, an allogeneic hematopoietic stem cell, an autologous induced pluripotent stem cell (iPSC) or an allogeneic iPSC that can give rise to an immune effector cell; and/or
b) an alpha/beta T cell, gamma/delta T cell, regulatory T cell (TREG), CD8+ T cell, and CD4+ T cell, B cell, natural killer (NK) cell, natural killer T (NKT) cell, naïve T cell, central memory T cell, effector memory T cell, stem memory T cell (Tscm), iPSC-derived T cell, synthetic T cell, a myeloid-derived phagocyte or an immune effector cell derived from a stem cell; or
c) a hematopoietic stem cell, a peripheral blood stem cell, a bone marrow derived stem cell, an immune stem cell, embryonic stem cell, an induced pluripotent stem cell (iPSC).

14. A SAR polypeptide of any one of claims 1-11, a polynucleotide of claims 12, or a recombinant cell of claim 13 for use in therapy, optionally,
wherein the subject suffers or has an increased risk of suffering from a disease or disorder selected from the group consisting of:
a proliferative disease, a precancerous condition, a cancer, optionally acute B cell leukemia, chronic B cell leukemia, B cell lymphoma, T cell leukemia or T cell lymphoma, myeloma, myelodysplastic syndrome, myeloproliferative disorders, skin cancer, breast cancer, colon cancer, rectal cancer, esophageal cancer, anal cancer, cancer of unknown primary site, endocrine cancer, testicular cancer, lung cancer, hepatocellular cancer, gastric cancer, pancreatic cancer, cervical cancer, ovarian cancer, liver cancer, bladder cancer, cancer of the urinary tract, cancer of reproductive organs thyroid cancer, renal cancer, melanoma, head and neck cancer, brain cancer, and prostate cancer; infectious disease; allergic disease; degenerative disease; or immune disorder, optionally multiple sclerosis, rheumatoid arthritis, ankylosing spondylitis, inflammatory bowel disease, diabetes mellitus, graft vs host disease or autoimmune thyroiditis, and a non-cancer related indication associated with expression of a disease-associated antigen, wherein the disease-associated antigen is selected from the group consisting of:
CD5; CD19; CD123; CD22; CD30; CD171; CS1 (also referred to as CD2 subset 1, CRACC, SLAMF7, CD319, and 19A24); C-type lectin-like molecule-1 (CLL-1 or CLECL1); CD33; epidermal growth factor receptor variant III (EGFRviii); ganglioside G2 (GD2); ganglioside GD3 (aNeu5Ac(2-8)aNeu5Ac(2-3)bDGalp(1-4)bDGlcp(1-1)Cer); TNF receptor family member B cell maturation (BCMA); Tn antigen ((Tn Ag) or (GalNAcα-Ser/Thr)); prostate-specific membrane antigen (PSMA); Receptor tyrosine kinase-like orphan receptor 1 (ROR1); Fms Like Tyrosine Kinase 3 (FLT3); Tumor-associated glycoprotein 72 (TAG72); CD38; CD44v6; a glycosylated CD43 epitope expressed on acute leukemia or lymphoma but not on hematopoietic progenitors, a glycosylated CD43 epitope expressed on non-hematopoietic cancers, Carcinoembryonic antigen (CEA); Epithelial cell adhesion molecule (EPCAM); B7H3 (CD276); KIT (CD117); Interleukin-13 receptor subunit alpha-2 (IL-13Ra2 or CD213A2); Mesothelin; Interleukin 11 receptor alpha (IL-IIRa); prostate stem cell antigen (PSCA); Protease Serine 21 (Testisin or PRSS21); vascular endothelial growth factor receptor 2 (VEGFR2); Lewis(Y) antigen; CD24; Platelet-derived growth factor receptor beta (PDGFR-beta); Stage-specific embryonic antigen-4 (SSEA-4); CD20; Folate receptor alpha (FRa or FR1); Folate receptor beta (FRb); Receptor tyrosine-protein kinase ERBB2 (Her2/neu); Mucin 1, cell surface associated (MUC1); epidermal growth factor receptor (EGFR); neural cell adhesion molecule (NCAM); Prostase; prostatic acid phosphatase (PAP); elongation factor 2 mutated (ELF2M); Ephrin B2; fibroblast activation protein alpha (FAP); insulin-like growth factor 1 receptor (IGF-I receptor); carbonic anhydrase IX (CAIX); Proteasome (Prosome, Macropain) Subunit, Beta Type, 9 (LMP2); glycoprotein 100 (gp100); oncogene fusion protein consisting of breakpoint cluster region (BCR) and Abelson murine leukemia viral oncogene homolog 1 (Abl) (bcr-abl); tyrosinase; ephrin type-A receptor 2 (EphA2); sialyl Lewis adhesion molecule (sLe); ganglioside GM3 (aNeu5Ac(2-3)bDClalp(I-4)bDGlcp(I-1)Cer); transglutaminase 5 (TGS5); high molecular weight-melanoma associated antigen (HMWMAA); o-acetyl-GD2 ganglioside (OAcGD2); tumor endothelial marker 1 (TEM1/CD248); tumor endothelial marker 7-related (TEM7R); claudin 6 (CLDN6); thyroid stimulating hormone receptor (TSHR); G protein coupled receptor class C group 5, member D (GPRC5D); chromosome X open reading frame 61 (CXORF61); CD97; CD179a; anaplastic lymphoma kinase (ALK); Polysialic acid; placenta-specific 1 (PLAC1); hexasaccharide portion of globoH glycoceramide (GloboH); mammary gland differentiation antigen (NY-BR-1); uroplakin 2 (UPK2); Hepatitis A virus cellular receptor 1 (HAVCR1); adrenoceptor beta 3 (ADRB3); pannexin 3 (PANX3); G protein-coupled receptor 20 (GPR20); lymphocyte antigen 6 complex, locus K 9 (LY6K); Olfactory receptor 51E2 (OR51E2); TCR Gamma Alternate Reading Frame Protein (TARP); Wilms tumor protein (WT1); Cancer/testis antigen 1 (NY-ESO-1); Cancer/testis antigen 2 (LAGE-1a); Melanoma-associated antigen 1 (MAGE-A1); ETS translocation-variant gene 6, located on chromosome 12p (ETV6-AML); sperm protein 17 (SPA17); X Antigen Family, Member 1A (XAGE1); angiopoietin-binding cell surface receptor 2 (Tie 2); melanoma cancer testis antigen-1 (MAD-CT-1); melanoma cancer testis antigen-2 (MAD-CT-2); Fos-related antigen 1; tumor protein p53 (p53); p53 mutant; prostein; survivin; telomerase; prostate carcinoma tumor antigen-1 (PCT A-1 or Galectin 8), melanoma antigen recognized by T cells 1 (MelanA or MARTI); Rat sarcoma (Ras) mutant; human Telomerase reverse transcriptase (hTERT); sarcoma translocation breakpoints; melanoma inhibitor of apoptosis (ML-IAP); ERG (transmembrane protease, serine 2 (TMPRSS2) ETS fusion gene); N-Acetyl glucosaminyl-transferase V (NA17); paired box protein Pax-3 (PAX3); Androgen receptor; Cyclin B1; v-myc avian myelocytomatosis viral oncogene neuroblastoma derived homolog (MYCN); Ras Homolog Family Member C (RhoC); Tyrosinase-related protein 2 (TRP-2); Cytochrome P450 1B 1 (CYP1B 1); CCCTC-Binding Factor (Zinc Finger Protein)-Like (BORIS or Brother of the Regulator of Imprinted Sites), Squamous Cell Carcinoma Antigen Recognized By T Cells 3 (SART3); Paired box protein Pax-5 (PAX5); proacrosin binding protein sp32 (OY-TES1); lymphocyte-specific protein tyrosine kinase (LCK); A kinase anchor protein 4 (AKAP-4); synovial sarcoma, X breakpoint 2 (SSX2); Receptor for Advanced Glycation End products (RAGE-1); renal ubiquitous 1 (RU1); renal ubiquitous 2 (RU2); legumain; human papilloma virus E6 (HPV E6); human papilloma virus E7 (HPV E7); intestinal carboxyl esterase; heat shock protein 70-2 mutated (mut hsp70-2); CD79a; CD79b; CD72; Leukocyte-associated immunoglobulin-like receptor 1 (LAIR1); Fc fragment of IgA receptor (FCAR or CD89); Leukocyte immunoglobulin-like receptor subfamily A member 2 (LILRA2); CD300 molecule-like family member f (CD300LF); C-type lectin domain family 12 member A (CLEC12A); bone marrow stromal cell antigen 2 (BST2); EGF-like module-containing mucin-like hormone receptor-like 2 (EMR2); lymphocyte antigen 75 (LY75); Glypican-3 (GPC3); Fc receptor-like 5 (FCRL5); immunoglobulin lambda-like polypeptide 1 (IGLL1); MPL; Biotin; c-MYC epitope Tag; CD34; LAMP1 TROP2; GFRalpha4; CDH17; CDH6; NYBR1; CDH19; CD200R; Slea (CA19.9; Sialyl Lewis Antigen); Fucosyl-GM1; PTK7; gpNMB; CDH1-CD324; DLL3; CD276/B7H3; IL11Ra; IL13Ra2; CD179b-IGLI1; TCR gamma-delta; NKG2D; CD32 (FCGR2A); Tn ag; Tim1-/HVCR1; CSF2RA (GM-CSFR-alpha); TGFbetaR2; Lewis Ag; TCR-beta1 chain; TCR-beta2 chain; TCR-gamma chain; TCR-delta chain; FITC; Luteinizing hormone receptor (LHR); Follicle stimulating hormone receptor (FSHR); Gonadotropin Hormone receptor (CGHR or GR); CCR4; GD3; SLAMF6; SLAMF4; HIV1 envelope glycoprotein; HTLV1-Tax; CMV pp65; EBV-EBNA3c; KSHV K8.1; KSHV-gH; influenza A hemagglutinin (HA); GAD; PDL1; Guanylyl cyclase C (GCC); auto antibody to desmoglein 3 (Dsg3); auto antibody to desmoglein 1 (Dsg1); HLA; HLA-A; HLA-A2; HLA-B; HLA-C; HLA-DP; HLA-DM; HLA-DOA; HLA-DOB; HLA-DQ; HLA-DR; HLA-G; IgE, CD99; Ras G12V; Tissue Factor 1 (TF1); AFP; GPRC5D; Claudin18.2 (CLD18A2 or CLDN18A.2); P-glycoprotein; STEAP1; Liv1; Nectin-4; Cripto; gpA33; BST1/CD157; low conductance chloride channel (LCCC); TAJ/TROY; MPL (TPO-R); KIR3DL2; CD32b; CD229; Toso; GPC3; BAFF-R; MR1; CMV-pp65/MHC complex; and NYESO-1/MHC complex.

15. An *in vitro* method of making a recombinant cell of claim 13, the method comprising:
a) introducing at least one recombinant polynucleotide of claim 12 into an immune effector cell or a hematopoietic stem cell or progenitor cell that can give rise to an immune effector cell, under conditions such that the SAR polypeptide is expressed; and/or
b) introducing *in vitro* transcribed RNA(s) or synthetic RNA(s) into a cell or population of cells, where the RNA(s) comprises a recombinant polynucleotide of claim 12;
optionally wherein
i) the SAR is expressed from an expression cassette placed in the locus of an endogenous T cell gene; and/or
ii) the SAR is under the control of the promoter and/or regulatory elements for an endogenous T cell gene,
optionally wherein the endogenous T cell gene locus is TRAC locus, TRBC locus, TRGC locus and/or TRDC locus.
